# EUROPEAN PATENT SPECIFICATION

(11) **EP 2 058 400 B1**
(45) Date of publication and mention of the grant of the patent: **18.05.2016**
(21) Application number: 08006358.9
(22) Date of filing: 19.08.1996
(51) Int. Cl.: C12N 15/73, C12N 15/63, C12N 15/13, C12N 15/10, C12N 15/62, C12N 15/70, C12N 1/21, C07K 16/00, C07K 1/04

(54) **Vectors/DNA-Sequences from Human Combinatorial Antibody Libraries**
Vektoren/DNA-Sequenzen für kombinatorische Bibliotheken menschlicher Antikörper
Vecteurs/séquences ADN pour des librairies combinatoires d'anticorps humains

(43) Date of publication of application: 13.05.2009
(62) Divisional of application: 01110609.3
(73) Proprietor: MorphoSys AG, 82152 Planegg-Martinsried (DE)
(72) Inventor: Knappik, Achim, 82418 Hofheim (DE); Pack, Peter, 68526 Ladenburg (DE); Ge, Liming, 85653 Aying (DE)
(74) Representative: Hutter, Bernd

(56) References cited:
- WO-A-91/19818
- WO-A-94/18219
- KRUIF DE J ET AL: "SELECTION AND APPLICATION OF HUMAN SINGLE CHAIN FV ANTIBODY FRAGMENTS FROM A SEMI-SYNTHETIC PHAGE ANTIBODY DISPLAY LIBRARY WITHDESIGNED CDR3 REGIONS" JOURNAL OF MOLECULAR BIOLOGY, LONDON, GB, vol. 248, 1995, pages 97-105, XP000984404 ISSN: 0022-2836
- WINTER G ET AL: "MAKING ANTIBODIES BY PHAGE DISPLAY TECHNOLOGY" ANNUAL REVIEW OF IMMUNOLOGY, ANNUAL REVIEWS INC, US, vol. 12, 1994, pages 433-455, XP000564245 ISSN: 0732-0582
- KNAPPIK A ET AL: "FULLY SYNTHETIC HUMAN COMBINATORIAL ANTIBODY LIBRARIES (HUCAL) BASED ON MODULAR CONSENSUS FRAMEWORKS AND CDRS RANDOMIZED WITH TRINUCLEOTIDES" JOURNAL OF MOLECULAR BIOLOGY, LONDON, GB, vol. 296, no. 1, 1 February 2000 (2000-02-01), pages 57-86, XP002941688 ISSN: 0022-2836

### SEQUENCE LISTING

(1) GENERAL INFORMATION:
   (i) APPLICANT:
      (A) NAME: Morphosys Gesellschaft fuer
         Proteinoptimierung mbH
      (B) STREET: Frankfurter Ring 193a
      (C) CITY: Muenchen
      (E) COUNTRY: DE
      (F) POSTAL CODE (ZIP): 80807

      (A) NAME: Knappik, Achim
      (B) STREET: Killerstr. 16
      (C) CITY: Graefelfing
      (E) COUNTRY: DE
      (F) POSTAL CODE (ZIP): 82166

      (A) NAME: Pack, Peter
      (B) STREET: Franz-Wolter-Str. 4
      (C) CITY: Muenchen
      (E) COUNTRY: DE
      (F) POSTAL CODE (ZIP): 81925

      (A) NAME: Ilag, Vic
      (B) STREET: Knorrstr. 85
      (C) CITY: Muenchen
      (E) COUNTRY: DE
      (F) POSTAL CODE (ZIP): 80807

      (A) NAME: Ge, Liming
      (B) STREET: Nestroystr. 17
      (C) CITY: Muenchen
      (E) COUNTRY: DE
      (F) POSTAL CODE (ZIP): 81373

      (A) NAME: Moroney, Simon
      (B) STREET: Osterwalcstr. 44
      (C) CITY: Muenchen
      (E) COUNTRY: DE
      (F) POSTAL CODE (ZIP): 80805

      (A) NAME: Plueckthun, Andreas
      (B) STREET: Moehrlistr. 97
      (C) CITY: Zuerich
      (E) COUNTRY: CH
      (F) POSTAL CODE (ZIP): 8006
   (ii) TITLE OF INVENTION: Protein/(Poly)peptide libraries
   (iii) NUMBER OF SEQUENCES: 371
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (v) CURRENT APPLICATION DATA:
      APPLICATION NUMBER: WO PCT/EP96/03647
   (vi) PRIOR APPLICATION DATA:
      (A) APPLICATION NUMBER: EP 95 11 3021.0
      (B) FILING DATE: 18-AUG-1995
(2) INFORMATION FOR SEQ ID NO: 1:
   (i) SEQUENCE CHARACTERISTICS:
      (A) LENGTH: 20 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 1:
   Ala Gly Gly Gly Ser Gly Gly Gly Gly Ser Gly Gly Gly Gly Ser Gly
   1               5                   10                  15
   Gly Gly Gly Ser
               20
(2) INFORMATION FOR SEQ ID NO: 2:
   (i) SEQUENCE CHARACTERISTICS:
      (A) LENGTH: 82 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 2:
   TCAGCGGGTG GCGGTTCTGG CGGCGGTGGG AGCGGTGGCG GTGGTTCTGG CGGTGGTGGT    60
   TCCGATATCG GTCCACGTAC GG                                             82
(2) INFORMATION FOR SEQ ID NO: 3:
   (i) SEQUENCE CHARACTERISTICS:
      (A) LENGTH: 83 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
      (xi) SEQUENCE DESCRIPTION: SEQ ID NO: 3:
   AATTCCGTAC GTGGACCGAT ATCGGAACCA CCACCGCCAG AACCACCGCC ACCGCTCCCA    60
   CCGCCGCCAG AACCGCCACC CGC                                            83
(2) INFORMATION FOR SEQ ID NO: 4:
   (i) SEQUENCE CHARACTERISTICS:
      (A) LENGTH: 69 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide library"
   (ix) FEATURE:
      (A) NAME/KEY: misc_feature
      (B) LOCATION:28..45
      (D) OTHER INFORMATION:/product= "6 random codons by trinucleotide mutagenesis (19aa, no Cys)"
   (xi) SEQUENCE DESCRIPTION: SEQ ID NO: 4:
   GATACGGCCG TGTATTATTG CGCGCGTNNK NNKNNKNNKN NKNNKGATTA TTGGGGCCAA 60
   GGCACCCTG 69
(2) INFORMATION FOR SEQ ID NO: 5:
   (i) SEQUENCE CHARACTERISTICS:
      (A) LENGTH: 84 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide library"
   (ix) FEATURE:
      (A) NAME/KEY: misc_feature
      (B) LOCATION:28..57
      (D) OTHER INFORMATION:/product= "10 random codons by trinucleotide mutagenesis (19aa, no Cys)"
   (ix) FEATURE:
      (A) NAME/KEY: misc_feature
      (B) LOCATION:58..60
      (D) OTHER INFORMATION:/product= "random codon by trinucleotide mutagenesis (TTT/ATG)"
   (ix) FEATURE:
      (A) NAME/KEY: misc_feature
      (B) LOCATION:64..66
      (D) OTHER INFORMATION:/product= "random codon by trinucleotide mutagenesis (GTT/TAT)"
   (xi) SEQUENCE DESCRIPTION: SEQ ID NO: 5:
   GATACGGCCG TGTATTATTG CGCGCGTNNK NNKNNKNNKN NKNNKNNKNN KNNKNNKWTK 60
   GATKWTTGGG GCCAAGGCAC CCTG 84
(2) INFORMATION FOR SEQ ID NO: 6:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 6:
      GATACGGCCG TGTATTATTG C 21
(2) INFORMATION FOR SEQ ID NO: 7:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 7:
      CAGGGTGCCT TGGCCCC 17
(2) INFORMATION FOR SEQ ID NO: 8:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 8:
      GCAGAAGGCG AACGTCC 17
(2) INFORMATION FOR SEQ ID NO: 9:
   (i) SEQUENCE CHARACTERISTICS:
      (A) LENGTH: 80 base pairs
      (C) STRANDEDNESS: single
      (D) TOPOLOGY: linear
   (ii) MOLECULE TYPE: other nucleic acid
      (A) DESCRIPTION: /desc = "synthetic oligonucleotide library"
   (ix) FEATURE:
      (A) NAME/KEY: misc_feature
      (B) LOCATION:39..41
      (D) OTHER INFORMATION:/product= "random codon (mixture of GCT, CGT, CAT, TCT, TAT)"
   (ix) FEATURE:
      (A) NAME/KEY: misc_feature
      (B) LOCATION:42..53
      (D) OTHER INFORMATION:/product= "random codons by trinucleotide mutagenesis (19 aa, no Cys)"
   (ix) FEATURE:
      (A) NAME/KEY: misc_feature
      (B) LOCATION:57..59
      (D) OTHER INFORMATION:/product= "random codon by trinucleotide mutagenesis (19 aa, no Cys)"
   (xi) SEQUENCE DESCRIPTION: SEQ ID NO: 9:
   TGGAAGCTGA AGACGTGGGC GTGTATTATT GCCAGCAGBV TNNKNNKNNK NNKCCGNNKT 60
   TTGGCCAGGG TACGAAAGTT 80
(2) INFORMATION FOR SEQ ID NO: 10:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 10:
      AACTTTCGTA CCCTGGCC 18
(2) INFORMATION FOR SEQ ID NO: 11:
   (i) SEQUENCE CHARACTERISTICS:
      (A) LENGTH: 108 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide library"
   (ix) FEATURE:
      (A) NAME/KEY: misc_feature
      (B) LOCATION:21..23
      (D) OTHER INFORMATION:/product= "random codon by trinucleotide mutagenesis (19aa, no Cys)"
   (ix) FEATURE:
      (A) NAME/KEY: misc_feature
      (B) LOCATION:27..35
      (D) OTHER INFORMATION:/product= "random codons by trinucleotide mutagenesis (19 aa, no Cys)"
   (ix) FEATURE:
      (A) NAME/KEY: misc_feature
      (B) LOCATION:36..41
      (D) OTHER INFORMATION:/product= "random codons by mixed monomers (A/G A/C/G T)"
   (ix) FEATURE: (A) NAME/KEY: misc_feature (B) LOCATION:42..44 (D) OTHER INFORMATION:/product= "random codon by trinucleotide mutagenesis (19aa, no Cys)"
   (ix) FEATURE:
      (A) NAME/KEY: misc_feature
      (B) LOCATION:48..50
      (D) OTHER INFORMATION:/product= "random codon by trinucleotide mutagenesis (19aa, no Cys)"
   (xi) SEQUENCE DESCRIPTION: SEQ ID NO: 11:
   AGGGTCTCGA GTGGGTGAGC NNKATTNNKN NKNNKRVTRV TNNKACCNNK TATGCGGATA      60
   GCGTGAAAGG CCGTTTTACC ATTTCACGTG ATAATTCGAA AAACACCA                  108
(2) INFORMATION FOR SEQ ID NO: 12:
   (i) SEQUENCE CHARACTERISTICS:
      (A) LENGTH: 105 base pairs
      (B) TYPE: nucleic acid
      (C) STRANDEDNESS: single
      (D) TOPOLOGY: linear

      (A) DESCRIPTION: /desc = "synthetic oligonucleotide library"
   (ix) FEATURE:
      (A) NAME/KEY: misc_feature
      (B) LOCATION:21..23
      (D) OTHER INFORMATION:/product= "random codon by trinucleotide mutagenesis (19aa, no Cys)"
   (ix) FEATURE:
      (A) NAME/KEY: misc_feature
      (B) LOCATION:27..32
      (D) OTHER INFORMATION:/product= "random codons by trinucleotide mutagenesis (19aa, no Cys)"
   (ix) FEATURE:
      (A) NAME/KEY: misc_feature
      (B) LOCATION:33..38
      (D) OTHER INFORMATION:/product= "random codons by mixed monomers (A/G A/C/G T)"
   (ix) FEATURE:
      (A) NAME/KEY: misc_feature
      (B) LOCATION:39..41
      (D) OTHER INFORMATION:/product= "random codon by trinucleotide mutagenesis (19aa, no Cys)"
   (ix) FEATURE:
      (A) NAME/KEY: misc_feature
      (B) LOCATION:45..47
      (D) OTHER INFORMATION:/product= "random codon by trinucleotide mutagenesis (19aa, no Cys)"
   (xi) SEQUENCE DESCRIPTION: SEQ ID NO: 12:
   AGGGTCTCGA GTGGGTGAGC NNKATTNNKN NKRVTRVTNN KACCNNKTAT GCGGATAGCG 60
   TGAAAGGCCG TTTTACCATT TCACGTGATA ATTCGAAAAA CACCA 105
(2) INFORMATION FOR SEQ ID NO: 13:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 13:
      TGGTGTTTTT CGAATTATCA 20
(2) INFORMATION FOR SEQ ID NO: 14:
   (i) SEQUENCE CHARACTERISTICS:
      (A) LENGTH: 108 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 14:
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Asn Tyr
               20                  25                  30
   Leu Asn Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Tyr Ser Thr Pro Leu
                   85                  90                  95
   Thr Phe Gly Gly Gly Thr Lys Val Glu Ile Lys Arg
               100                 105
(2) INFORMATION FOR SEQ ID NO: 15:
   (i) SEQUENCE CHARACTERISTICS:
      (A) LENGTH: 113 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 15:
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Tyr Thr Phe Gly Gln Gly Thr Lys Leu Glu Ile Lys
               100                 105                 110
   Arg
(2) INFORMATION FOR SEQ ID NO: 16:
   (i) SEQUENCE CHARACTERISTICS:
      (A) LENGTH: 109 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 16:
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Asn Ser Pro
                   85                  90                  95
   Tyr Thr Phe Gly Gln Gly Thr Lys Val Glu Ile Lys Arg
               100                 105
(2) INFORMATION FOR SEQ ID NO: 17:
   (i) SEQUENCE CHARACTERISTICS:
      (A) LENGTH: 114 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 17:
   Asp Ile Val Met Thr Gln Ser Pro Asp Ser Leu Ala Val Ser Leu Gly
   1               5                   10                  15
   Glu Arg Ala Thr Ile Asn Cys Lys Ser Ser Gln Ser Val Leu Tyr Ser
               20                  25                  30
   Ser Asn Asn Lys Asn Tyr Leu Ala Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Tyr Tyr Ser Thr Pro Pro Thr Phe Gly Gln Gly Thr Lys Val Glu Ile
               100                 105                 110
   Lys Arg
(2) INFORMATION FOR SEQ ID NO: 18:
   (i) SEQUENCE CHARACTERISTICS:
      (A) LENGTH: 112 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 18:
   Gln Ser Val Leu Thr Gln Pro Pro Ser Val Ser Gly Ala Pro Gly Gln
   1               5                   10                  15
   Arg Val Thr Ile Ser Cys Ser Gly Ser Ser Ser Asn Ile Gly Asn Asn
               20                  25                  30
   Tyr Val Ser Trp Tyr Gln Gln Leu Pro Gly Thr Ala Pro Lys Leu Leu
           35                  40                  45
   Ile Tyr Asp Asn Asn Lys Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Ala Thr Trp Asp Asp Ser Leu
                   85                  90                  95
   Ser Gly Val Val Phe Gly Gly Gly Thr Lys Leu Thr Val Leu Gly Gln
               100                 105                 110
(2) INFORMATION FOR SEQ ID NO: 19:
   (i) SEQUENCE CHARACTERISTICS:
      (A) LENGTH: 112 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 19:
   Gln Ser Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Gly Tyr
               20                  25                  30
   Asn Tyr Val Ser Trp Tyr Gln Gln His Pro Gly Lys Ala Pro Lys Leu
           35                  40                  45
   Met Ile Tyr Asp Val Ser Lys Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Ser Tyr Ala Gly Ser
                   85                  90                  95
   Ser Thr Val Val Phe Gly Gly Gly Thr Lys Leu Thr Val Leu Gly Gln
               100                 105                 110
(2) INFORMATION FOR SEQ ID NO: 20:
   (i) SEQUENCE CHARACTERISTICS:
      (A) LENGTH: 108 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 20:
   Tyr Glu Leu Thr Gln Pro Pro Ser Val Ser Val Ala Pro Gly Gln Thr
   1               5                   10                  15
   Ala Arg Ile Thr Cys Ser Gly Asp Ser Leu Gly Ser Lys Tyr Ala Ser
               20                  25                  30
   Trp Tyr Gln Gln Lys Pro Gly Gln Ala Pro Val Leu Val Ile Tyr Asp
           35                  40                  45
   Asp Asn Lys Arg Pro Ser Gly Ile Pro Glu Arg Phe Ser Gly Ser Asn
       50                  55                  60
   Ser Gly Asn Thr Ala Thr Leu Thr Ile Ser Gly Val Gln Ala Glu Asp
   65                  70                  75                  80
   Glu Ala Asp Tyr Tyr Cys Gln Ser Trp Asp Ser Ser Gly Asn Val Val
                   85                  90                  95
   Phe Gly Gly Gly Thr Lys Leu Thr Val Leu Gly Gln
               100                 105
(2) INFORMATION FOR SEQ ID NO: 21:
   (i) SEQUENCE CHARACTERISTICS:
      (A) LENGTH: 119 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 21:
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Ala Ile Ser Trp Val Arg Gln Ala Pro Gly Gln Gly Leu Glu Trp Met
           35                  40                  45
   Gly Gly Ile Ile Pro Ile Phe Gly Thr Ala Asn Tyr Ala Gln Lys Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Ala Pro Gly Tyr Cys Ser Gly Phe Asp Tyr Trp Gly Gln Gly
               100                 105                 110
   Thr Leu Val Thr Val Ser Ser
           115
(2) INFORMATION FOR SEQ ID NO: 22:
   (i) SEQUENCE CHARACTERISTICS:
      (A) LENGTH: 117 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 22:
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Ser Tyr
               20                  25                  30
   Tyr Met His Trp Val Arg Gln Ala Pro Gly Gln Gly Leu Glu Trp Met
           35                  40                  45
   Gly Trp Ile Asn Pro Asn Ser Gly Asn Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Ser Leu Arg Ser Asp Asp Thr Ala Val Tyr Tyr Cys
                   85                  90                  95
   Ala Arg Asp Gly Asp Gly Gly Phe Asp Tyr Trp Gly Gln Gly Thr Leu
               100                 105                 110
   Val Thr Val Ser Ser
           115
(2) INFORMATION FOR SEQ ID NO: 23:
   (i) SEQUENCE CHARACTERISTICS:
      (A) LENGTH: 120 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 23:
(2) INFORMATION FOR SEQ ID NO: 24:
   (i) SEQUENCE CHARACTERISTICS:
      (A) LENGTH: 117 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 24:
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met His Trp Val Arg Gln Ala Pro Gly Lys Gly Leu Glu Trp Val
           35                  40                  45
   Ser Val Ile Ser Tyr Asp Gly Gly Asn Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Arg Gly Gly Ser Gly Asp Tyr Trp Gly Gln Gly Thr Leu
               100                 105                 110
   Val Thr Val Ser Ser
           115
(2) INFORMATION FOR SEQ ID NO: 25:
   (i) SEQUENCE CHARACTERISTICS:
      (A) LENGTH: 118 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 25:
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Tyr
               20                  25                  30
   Tyr Trp Ser Trp Ile Arg Gln Pro Pro Gly Lys Gly Leu Glu Trp Ile
           35                  40                  45
   Gly Glu Ile Tyr His Ser Gly Ser Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Arg Gly Arg Gly Gly Gly Gly Val Phe Asp Tyr Trp Gly Gln Gly Thr
               100                 105                 110
   Leu Val Thr Val Ser Ser
           115
(2) INFORMATION FOR SEQ ID NO: 26:
   (i) SEQUENCE CHARACTERISTICS:
      (A) LENGTH: 119 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 26:
   Glu Val Gln Leu Val Gln Ser Gly Ala Glu Val Lys Lys Pro Gly Glu
   1               5                   10                  15
   Ser Leu Lys Ile Ser Cys Lys Gly Ser Gly Tyr Ser Phe Thr Ser Tyr
               20                  25                  30
   Trp Ile Gly Trp Val Arg Gln Met Pro Gly Lys Gly Leu Glu Trp Met
           35                  40                  45
   Gly Ile Ile Tyr Pro Gly Asp Ser Asp Thr Arg Tyr Ser Pro Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Leu Gly Gly Gly Gly Tyr Tyr Phe Asp Tyr Trp Gly Gln Gly
   100 105 110
   Thr Leu Val Thr Val Ser Ser
   115
(2) INFORMATION FOR SEQ ID NO: 27:
   (i) SEQUENCE CHARACTERISTICS:
      (A) LENGTH: 119 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 27:
   Gln Val Gln Leu Gln Gln Ser Gly Pro Gly Leu Val Lys Pro Ser Gln
   1 5 10 15
   Thr Leu Ser Leu Thr Cys Ala Ile Ser Gly Asp Ser Val Ser Ser Asn
   20 25 30
   Ser Ala Ala Trp Asn Trp Ile Arg Gln Ser Pro Ser Arg Gly Leu Glu
   35 40 45
   Trp Leu Gly Arg Thr Tyr Tyr Arg Ser Lys Trp Tyr Asn Asp Tyr Ala
   50 55 60
   Val Ser Val Lys Ser Arg Ile Thr Ile Asn Pro Asp Thr Ser Lys Asn
   65 70 75 80
   Gln Phe Ser Leu Gln Leu Asn Ser Val Thr Pro Glu Asp Thr Ala Val
   85 90 95
   Tyr Tyr Cys Ala Arg Asp Pro Gly Gly Phe Asp Val Trp Gly Gln Gly
   100 105 110
   Thr Leu Val Thr Val Ser Ser
   115
(2) INFORMATION FOR SEQ ID NO: 28:
   (i) SEQUENCE CHARACTERISTICS:
      (A) LENGTH: 109 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 28:
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Ser Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln His Tyr Thr Thr Pro Pro
                   85                  90                  95
   Thr Phe Gly Gln Gly Thr Lys Val Glu Ile Lys Arg Thr
               100                 105
(2) INFORMATION FOR SEQ ID NO: 29:
   (i) SEQUENCE CHARACTERISTICS:
      (A) LENGTH: 114 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 29:
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Gln Gln His
                   85                  90                  95
   Tyr Thr Thr Pro Pro Thr Phe Gly Gln Gly Thr Lys Val Glu Ile Lys
               100                 105                 110
   Arg Thr
(2) INFORMATION FOR SEQ ID NO: 30:
   (i) SEQUENCE CHARACTERISTICS:
      (A) LENGTH: 110 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 30:
   Asp Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Val Pro Ala Arg Phe Ser
       50                  55                  60
   Gly Ser Gly Ser Gly Thr Asp Phe Thr Leu Thr Ile Ser Ser Leu Glu
   65                  70                  75                  80
   Pro Glu Asp Phe Ala Val Tyr Tyr Cys Gln Gln His Tyr Thr Thr Pro
                   85                  90                  95
   Pro Thr Phe Gly Gln Gly Thr Lys Val Glu Ile Lys Arg Thr
               100                 105                 110
(2) INFORMATION FOR SEQ ID NO: 31:
   (i) SEQUENCE CHARACTERISTICS:
      (A) LENGTH: 115 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 31:
   Asp Ile Val Met Thr Gln Ser Pro Asp Ser Leu Ala Val Ser Leu Gly
   1               5                   10                  15
   Glu Arg Ala Thr Ile Asn Cys Arg Ser Ser Gln Ser Val Leu Tyr Ser
               20                  25                  30
   Ser Asn Asn Lys Asn Tyr Leu Ala Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   His Tyr Thr Thr Pro Pro Thr Phe Gly Gln Gly Thr Lys Val Glu Ile
               100                 105                 110
   Lys Arg Thr
           115
(2) INFORMATION FOR SEQ ID NO: 32:
   (i) SEQUENCE CHARACTERISTICS:
      (A) LENGTH: 109 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 32:
   Gln Ser Val Leu Thr Gln Pro Pro Ser Val Ser Gly Ala Pro Gly Gln
   1               5                   10                  15
   Arg Val Thr Ile Ser Cys Ser Gly Ser Ser Ser Asn Ile Gly Ser Asn
               20                  25                  30
   Tyr Val Ser Trp Tyr Gln Gln Leu Pro Gly Thr Ala Pro Lys Leu Leu
           35                  40                  45
   Ile Tyr Asp Asn Asn Gln Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Gln Gln His Tyr Thr Thr Pro
                   85                  90                  95
   Pro Val Phe Gly Gly Gly Thr Lys Leu Thr Val Leu Gly
               100                 105
(2) INFORMATION FOR SEQ ID NO: 33:
   (i) SEQUENCE CHARACTERISTICS:
      (A) LENGTH: 110 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ.ID NO: 33:
   Gln Ser Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Gly Tyr
               20                  25                  30
   Asn Tyr Val Ser Trp Tyr Gln Gln His Pro Gly Lys Ala Pro Lys Leu
           35                  40                  45
   Met Ile Tyr Asp Val Ser Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Gln Gln His Tyr Thr Thr
                   85                  90                  95
   Pro Pro Val Phe Gly Gly Gly Thr Lys Leu Thr Val Leu Gly
               100                 105                 110
(2) INFORMATION FOR SEQ ID NO: 34:
   (i) SEQUENCE CHARACTERISTICS:
      (A) LENGTH: 107 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 34:
   Ser Tyr Glu Leu Thr Gln Pro Pro Ser Val Ser Val Ala Pro Gly Gln
   1               5                   10                  15
   Thr Ala Arg Ile Ser Cys Ser Gly Asp Ala Leu Gly Asp Lys Tyr Ala
               20                  25                  30
   Ser Trp Tyr Gln Gln Lys Pro Gly Gln Ala Pro Val Leu Val Ile Tyr
           35                  40                  45
   Asp Asp Ser Asp Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Gln Gln His Tyr Thr Thr Pro Pro Val
                   85                  90                  95
   Phe Gly Gly Gly Thr Lys Leu Thr Val Leu Gly
               100                 105
(2) INFORMATION FOR SEQ ID NO: 35:
   (i) SEQUENCE CHARACTERISTICS:
      (A) LENGTH: 120 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 35:
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Ala Ile Ser Trp Val Arg Gln Ala Pro Gly Gln Gly Leu Glu Trp Met
           35                  40                  45
   Gly Gly Ile Ile Pro Ile Phe Gly Thr Ala Asn Tyr Ala Gln Lys Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Trp Gly Gly Asp Gly Phe Tyr Ala Met Asp Tyr Trp Gly Gln
               100                 105                 110
   Gly Thr Leu Val Thr Val Ser Ser
           115                 120
(2) INFORMATION FOR SEQ ID NO: 36:
   (i) SEQUENCE CHARACTERISTICS:
      (A) LENGTH: 120 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 36:
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Ser Tyr
               20                  25                  30
   Tyr Met His Trp Val Arg Gln Ala Pro Gly Gln Gly Leu Glu Trp Met
           35                  40                  45
   Gly Trp Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Trp Gly Gly Asp Gly Phe Tyr Ala Met Asp Tyr Trp Gly Gln
               100                 105                 110
   Gly Thr Leu Val Thr Val Ser Ser
           115                 120
(2) INFORMATION FOR SEQ ID NO: 37:
   (i) SEQUENCE CHARACTERISTICS:
      (A) LENGTH: 121 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 37:
   Gln Val Gln Leu Lys Glu Ser Gly Pro Ala Leu Val Lys Pro Thr Gln
   1               5                   10                  15
   Thr Leu Thr Leu Thr Cys Thr Phe Ser Gly Phe Ser Leu Ser Thr Ser
               20                  25                  30
   Gly Val Gly Val Gly Trp Ile Arg Gln Pro Pro Gly Lys Ala Leu Glu
           35                  40                  45
   Trp Leu Ala Leu Ile Asp Trp Asp Asp Asp Lys Tyr Tyr Ser Thr Ser
       50                  55                  60
   Leu Lys Thr Arg Leu Thr Ile Ser Lys Asp Thr Ser Lys Asn Gln Val
   65                  70                  75                  80
   Val Leu Thr Met Thr Asn Met Asp Pro Val Asp Thr Ala Thr Tyr Tyr
                   85                  90                  95
   Cys Ala Arg Trp Gly Gly Asp Gly Phe Tyr Ala Met Asp Tyr Trp Gly
               100                 105                 110
   Gln Gly Thr Leu Val Thr Val Ser Ser
           115                 120
(2) INFORMATION FOR SEQ ID NO: 38:
   (i) SEQUENCE CHARACTERISTICS:
      (A) LENGTH: 120 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 38:
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Gly Asp Gly Phe Tyr Ala Met Asp Tyr Trp Gly Gln
               100                 105                 110
   Gly Thr Leu Val Thr Val Ser Ser
           115                 120
(2) INFORMATION FOR SEQ ID NO: 39:
   (i) SEQUENCE CHARACTERISTICS:
      (A) LENGTH: 119 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 39:
(2) INFORMATION FOR SEQ ID NO: 40:
   (i) SEQUENCE CHARACTERISTICS:
      (A) LENGTH: 120 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 40:
   Glu Val Gln Leu Val Gln Ser Gly Ala Glu Val Lys Lys Pro Gly Glu
   1               5                   10                  15
   Ser Leu Lys Ile Ser Cys Lys Gly Ser Gly Tyr Ser Phe Thr Ser Tyr
               20                  25                  30
   Trp Ile Gly Trp Val Arg Gln Met Pro Gly Lys Gly Leu Glu Trp Met
           35                  40                  45
   Gly Ile Ile Tyr Pro Gly Asp Ser Asp Thr Arg Tyr Ser Pro Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Trp Gly Gly Asp Gly Phe Tyr Ala Met Asp Tyr Trp Gly Gln
               100                 105                 110
   Gly Thr Leu Val Thr Val Ser Ser
           115                 120
(2) INFORMATION FOR SEQ ID NO: 41:
   (i) SEQUENCE CHARACTERISTICS:
      (A) LENGTH: 123 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 41:
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Asn
               20                  25                  30
   Ser Ala Ala Trp Asn Trp Ile Arg Gln Ser Pro Gly Arg Gly Leu Glu
           35                  40                  45
   Trp Leu Gly Arg Thr Tyr Tyr Arg Ser Lys Trp Tyr Asn Asp Tyr Ala
       50                  55                  60
   Val Ser Val Lys Ser Arg Ile Thr Ile Asn Pro Asp Thr Ser Lys Asn
   65                  70                  75                  80
   Gln Phe Ser Leu Gln Leu Asn Ser Val Thr Pro Glu Asp Thr Ala Val
                   85                  90                  95
   Tyr Tyr Cys Ala Arg Trp Gly Gly Asp Gly Phe Tyr Ala Met Asp Tyr
               100                 105                 110
   Trp Gly Gln Gly Thr Leu Val Thr Val Ser Ser
           115                 120
(2) INFORMATION FOR SEQ ID NO: 42:
   (i) SEQUENCE CHARACTERISTICS:
      (A) LENGTH: 327 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene"
   (ix) FEATURE:
      (A) NAME/KEY: CDS
      (B) LOCATION:1..327
      (D) OTHER INFORMATION:/product= "V kappa 1"
   (xi) SEQUENCE DESCRIPTION: SEQ ID NO: 42:
   GAT ATC CAG ATG ACC CAG AGC CCG TCT AGC CTG AGC GCG AGC GTG GGT      48
   Asp Ile Gln Met Thr Gln Ser Pro Ser Ser Leu Ser Ala Ser Val Gly
    1               5                  10                  15
   GAT CGT GTG ACC ATT ACC TGC AGA GCG AGC CAG GGC ATT AGC AGC TAT      96
   Asp Arg Val Thr Ile Thr Cys Arg Ala Ser Gln Gly Ile Ser Ser Tyr
               20                  25                  30
   CTG GCG TGG TAC CAG CAG AAA CCA GGT AAA GCA CCG AAA CTA TTA ATT        144
   Leu Ala Trp Tyr Gln Gln Lys Pro Gly Lys Ala Pro Lys Leu Leu Ile
           35                  40                  45
   TAT GCA GCC AGC AGC TTG CAA AGC GGG GTC CCG TCC CGT TTT AGC GGC        192
   Tyr Ala Ala Ser Ser Leu Gln Ser Gly Val Pro Ser Arg Phe Ser Gly
       50                  55                  60
   TCT GGA TCC GGC ACT GAT TTT ACC CTG ACC ATT AGC AGC CTG CAA CCT        240
   Ser Gly Ser Gly Thr Asp Phe Thr Leu Thr Ile Ser Ser Leu Gln Pro
   65                  70                  75                  80
   GAA GAC TTT GCG ACC TAT TAT TGC CAG CAG CAT TAT ACC ACC CCG CCG        288
   Glu Asp Phe Ala Thr Tyr Tyr Cys Gln Gln His Tyr Thr Thr Pro Pro
                   85                  90                  95
   ACC TTT GGC CAG GGT ACG AAA GTT GAA ATT AAA CGT ACG                    327
   Thr Phe Gly Gln Gly Thr Lys Val Glu Ile Lys Arg Thr
               100                 105
(2) INFORMATION FOR SEQ ID NO: 43:
   (i) SEQUENCE CHARACTERISTICS:
      (A) LENGTH: 109 amino acids
      (B) TYPE: amino acid
      (D) TOPOLOGY: linear
   (ii) MOLECULE TYPE: protein
   (xi) SEQUENCE DESCRIPTION: SEQ ID NO: 43:
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Ser Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln His Tyr Thr Thr Pro Pro
                   85                  90                  95
   Thr Phe Gly Gln Gly Thr Lys Val Glu Ile Lys Arg Thr
               100                 105
(2) INFORMATION FOR SEQ ID NO: 44:
   (i) SEQUENCE CHARACTERISTICS:
      (A) LENGTH: 342 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene"
   (ix) FEATURE:
      (A) NAME/KEY: CDS
      (B) LOCATION:1..342
      (D) OTHER INFORMATION:/product= "V kappa 2"
   (xi) SEQUENCE DESCRIPTION: SEQ ID NO: 44:
(2) INFORMATION FOR SEQ ID NO: 45:
   (i) SEQUENCE CHARACTERISTICS:
      (A) LENGTH: 114 amino acids
      (B) TYPE: amino acid
      (D) TOPOLOGY: linear
   (ii) MOLECULE TYPE: protein
   (xi) SEQUENCE DESCRIPTION: SEQ ID NO: 45:
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Gln Gln His
                   85                  90                  95
   Tyr Thr Thr Pro Pro Thr Phe Gly Gln Gly Thr Lys Val Glu Ile Lys
               100                 105                 110
   Arg Thr
(2) INFORMATION FOR SEQ ID NO: 46:
   (i) SEQUENCE CHARACTERISTICS:
      (A) LENGTH: 330 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene"
   (ix) FEATURE:
      (A) NAME/KEY: CDS
      (B) LOCATION:1..330
      (D) OTHER INFORMATION:/product= "V kappa 3"
   (xi) SEQUENCE DESCRIPTION: SEQ ID NO: 46:
(2) INFORMATION FOR SEQ ID NO: 47:
   (i) SEQUENCE CHARACTERISTICS:
      (A) LENGTH: 110 amino acids
      (B) TYPE: amino acid
      (D) TOPOLOGY: linear
   (ii) MOLECULE TYPE: protein
   (xi) SEQUENCE DESCRIPTION: SEQ ID NO: 47:
   Asp Ile Val Leu Thr Gln Ser Pro Ala Thr Leu Ser Leu Ser Pro Gly
    1               5                  10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Val Pro Ala Arg Phe Ser
       50                  55                  60
   Gly Ser Gly Ser Gly Thr Asp Phe Thr Leu Thr Ile Ser Ser Leu Glu
   65                  70                  75                  80
   Pro Glu Asp Phe Ala Val Tyr Tyr Cys Gln Gln His Tyr Thr Thr Pro
                   85                  90                  95
   Pro Thr Phe Gly Gln Gly Thr Lys Val Glu Ile Lys Arg Thr
               100                 105                 110
(2) INFORMATION FOR SEQ ID NO: 48:
   (i) SEQUENCE CHARACTERISTICS:
      (A) LENGTH: 345 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene"
   (ix) FEATURE:
      (A) NAME/KEY: CDS
      (B) LOCATION:1..345
      (D) OTHER INFORMATION:/product= "V kappa 4"
   (xi) SEQUENCE DESCRIPTION: SEQ ID NO: 48:
   GAT ATC GTG ATG ACC CAG AGC CCG GAT AGC CTG GCG GTG AGC CTG GGC     48
   Asp Ile Val Met Thr Gln Ser Pro Asp Ser Leu Ala Val Ser Leu Gly
                   115                 120                 125
   GAA CGT GCG ACC ATT AAC TGC AGA AGC AGC CAG AGC GTG CTG TAT AGC     96
   Glu Arg Ala Thr Ile Asn Cys Arg Ser Ser Gln Ser Val Leu Tyr Ser
               130                 135                 140
   AGC AAC AAC AAA AAC TAT CTG GCG TGG TAC CAG CAG AAA CCA GGT CAG    144
   Ser Asn Asn Lys Asn Tyr Leu Ala Trp Tyr Gln Gln Lys Pro Gly Gln
           145                 150                 155
   CCG CCG AAA CTA TTA ATT TAT TGG GCA TCC ACC CGT GAA AGC GGG GTC    192
   Pro Pro Lys Leu Leu Ile Tyr Trp Ala Ser Thr Arg Glu Ser Gly Val
       160                 165                 170
   CCG GAT CGT TTT AGC GGC TCT GGA TCC GGC ACT GAT TTT ACC CTG ACC    240
   Pro Asp Arg Phe Ser Gly Ser Gly Ser Gly Thr Asp Phe Thr Leu Thr
   175                 180                 185                 190
   ATT TCG TCC CTG CAA GCT GAA GAC GTG GCG GTG TAT TAT TGC CAG CAG    288
   Ile Ser Ser Leu Gln Ala Glu Asp Val Ala Val Tyr Tyr Cys Gln Gln
                   195                 200                 205
   CAT TAT ACC ACC CCG CCG ACC TTT GGC CAG GGT ACG AAA GTT GAA ATT    336
   His Tyr Thr Thr Pro Pro Thr Phe Gly Gln Gly Thr Lys Val Glu Ile
               210                 215                 220
   AAA CGT ACG                                                        345
   Lys Arg Thr
           225
(2) INFORMATION FOR SEQ ID NO: 49:
   (i) SEQUENCE CHARACTERISTICS:
      (A) LENGTH: 115 amino acids
      (B) TYPE: amino acid
      (D) TOPOLOGY: linear
   (ii) MOLECULE TYPE: protein
   (xi) SEQUENCE DESCRIPTION: SEQ ID NO: 49:
   Asp Ile Val Met Thr Gln Ser Pro Asp Ser Leu Ala Val Ser Leu Gly
    1               5                  10                  15
   Glu Arg Ala Thr Ile Asn Cys Arg Ser Ser Gln Ser Val Leu Tyr Ser
               20                  25                  30
   Ser Asn Asn Lys Asn Tyr Leu Ala Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   His Tyr Thr Thr Pro Pro Thr Phe Gly Gln Gly Thr Lys Val Glu Ile
               100                 105                 110
   Lys Arg Thr
           115
(2) INFORMATION FOR SEQ ID NO: 50:
   (i) SEQUENCE CHARACTERISTICS:
      (A) LENGTH: 327 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene"
   (ix) FEATURE:
      (A) NAME/KEY: CDS
      (B) LOCATION:1..327
      (D) OTHER INFORMATION:/product= "V lambda 1"
   (xi) SEQUENCE DESCRIPTION: SEQ ID NO: 50:
   CAG AGC GTG CTG ACC CAG CCG CCT TCA GTG AGT GGC GCA CCA GGT CAG        48
   Gln Ser Val Leu Thr Gln Pro Pro Ser Val Ser Gly Ala Pro Gly Gln
                   120                 125                 130
   CGT GTG ACC ATC TCG TGT AGC GGC AGC AGC AGC AAC ATT GGC AGC AAC        96
   Arg Val Thr Ile Ser Cys Ser Gly Ser Ser Ser Asn Ile Gly Ser Asn
               135                 140                 145
   TAT GTG AGC TGG TAC CAG CAG TTG CCC GGG ACG GCG CCG AAA CTG CTG       144
   Tyr Val Ser Trp Tyr Gln Gln Leu Pro Gly Thr Ala Pro Lys Leu Leu
           150                 155                 160
   ATT TAT GAT AAC AAC CAG CGT CCC TCA GGC GTG CCG GAT CGT TTT AGC       192
   Ile Tyr Asp Asn Asn Gln Arg Pro Ser Gly Val Pro Asp Arg Phe Ser
       165                 170                 175
   GGA TCC AAA AGC GGC ACC AGC GCG AGC CTT GCG ATT ACG GGC CTG CAA       240
   Gly Ser Lys Ser Gly Thr Ser Ala Ser Leu Ala Ile Thr Gly Leu Gln
   180                 185                 190                 195
   AGC GAA GAC GAA GCG GAT TAT TAT TGC CAG CAG CAT TAT ACC ACC CCG       288
   Ser Glu Asp Glu Ala Asp Tyr Tyr Cys Gln Gln His Tyr Thr Thr Pro
                   200                 205                 210
   CCT GTG TTT GGC GGC GGC ACG AAG TTA ACC GTT CTT GGC                   327
   Pro Val Phe Gly Gly Gly Thr Lys Leu Thr Val Leu Gly
               215                 220
(2) INFORMATION FOR SEQ ID NO: 51:
   (i) SEQUENCE CHARACTERISTICS:
      (A) LENGTH: 109 amino acids
      (B) TYPE: amino acid
      (D) TOPOLOGY: linear
   (ii) MOLECULE TYPE: protein
   (xi) SEQUENCE DESCRIPTION: SEQ ID NO: 51:
   Gln Ser Val Leu Thr Gln Pro Pro Ser Val Ser Gly Ala Pro Gly Gln
    1               5                  10                  15
   Arg Val Thr Ile Ser Cys Ser Gly Ser Ser Ser Asn Ile Gly Ser Asn
               20                  25                  30
   Tyr Val Ser Trp Tyr Gln Gln Leu Pro Gly Thr Ala Pro Lys Leu Leu
           35                  40                  45
   Ile Tyr Asp Asn Asn Gln Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Gln Gln His Tyr Thr Thr Pro
                   85                  90                  95
   Pro Val Phe Gly Gly Gly Thr Lys Leu Thr Val Leu Gly
               100                 105
(2) INFORMATION FOR SEQ ID NO: 52:
   (i) SEQUENCE CHARACTERISTICS:
      (A) LENGTH: 330 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene"
   (ix) FEATURE:
      (A) NAME/KEY: CDS
      (D) OTHER INFORMATION:/product= "V lambda 2"
   (xi) SEQUENCE DESCRIPTION: SEQ ID NO: 52:
   CAG AGC GCA CTG ACC CAG CCA GCT TCA GTG AGC GGC TCA CCA GGT CAG     48
   Gln Ser Ala Leu Thr Gln Pro Ala Ser Val Ser Gly Ser Pro Gly Gln
   110                 115                 120                 125
   AGC ATT ACC ATC TCG TGT ACG GGT ACT AGC AGC GAT GTG GGC GGC TAT     96
   Ser Ile Thr Ile Ser Cys Thr Gly Thr Ser Ser Asp Val Gly Gly Tyr
                   130                 135                 140
   AAC TAT GTG AGC TGG TAC CAG CAG CAT CCC GGG AAG GCG CCG AAA CTG    144
   Asn Tyr Val Ser Trp Tyr Gln Gln His Pro Gly Lys Ala Pro Lys Leu
               145                 150                 155
   ATG ATT TAT GAT GTG AGC AAC CGT CCC TCA GGC GTG AGC AAC CGT TTT    192
   Met Ile Tyr Asp Val Ser Asn Arg Pro Ser Gly Val Ser Asn Arg Phe
           160                 165                 170
   AGC GGA TCC AAA AGC GGC AAC ACC GCG AGC CTG ACC ATT AGC GGC CTG    240
   Ser Gly Ser Lys Ser Gly Asn Thr Ala Ser Leu Thr Ile Ser Gly Leu
       175                 180                 185
   CAA GCG GAA GAC GAA GCG GAT TAT TAT TGC CAG CAG CAT TAT ACC ACC    288
   Gln Ala Glu Asp Glu Ala Asp Tyr Tyr Cys Gln Gln His Tyr Thr Thr
   190                 195                 200                 205
   CCG CCT GTG TTT GGC GGC GGC ACG AAG TTA ACC GTT CTT GGC            330
   Pro Pro Val Phe Gly Gly Gly Thr Lys Leu Thr Val Leu Gly
                   210                 215
(2) INFORMATION FOR SEQ ID NO: 53:
   (i) SEQUENCE CHARACTERISTICS:
      (A) LENGTH: 110 amino acids
      (B) TYPE: amino acid
      (D) TOPOLOGY: linear
   (ii) MOLECULE TYPE: protein
   (xi) SEQUENCE DESCRIPTION: SEQ ID NO: 53:
   Gln Ser Ala Leu Thr Gln Pro Ala Ser Val Ser Gly Ser Pro Gly Gln
    1               5                  10                  15
   Ser Ile Thr Ile Ser Cys Thr Gly Thr Ser Ser Asp Val Gly Gly Tyr
               20                  25                  30
   Asn Tyr Val Ser Trp Tyr Gln Gln His Pro Gly Lys Ala Pro Lys Leu
           35                  40                  45
   Met Ile Tyr Asp Val Ser Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Gln Gln His Tyr Thr Thr
                   85                  90                  95
   Pro Pro Val Phe Gly Gly Gly Thr Lys Leu Thr Val Leu Gly
               100                 105                 110
(2) INFORMATION FOR SEQ ID NO: 54:
   (i) SEQUENCE CHARACTERISTICS:
      (A) LENGTH: 321 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene"
   (ix) FEATURE:
      (A) NAME/KEY: CDS
      (B) LOCATION:1..321
      (D) OTHER INFORMATION:/product= "V lambda 3"
   (xi) SEQUENCE DESCRIPTION: SEQ ID NO: 54:ly
(2) INFORMATION FOR SEQ ID NO: 55:
   (i) SEQUENCE CHARACTERISTICS:
      (A) LENGTH: 107 amino acids
      (B) TYPE: amino acid
      (D) TOPOLOGY: linear
   (ii) MOLECULE TYPE: protein
   (xi) SEQUENCE DESCRIPTION: SEQ ID NO: 55:
   Ser Tyr Glu Leu Thr Gln Pro Pro Ser Val Ser Val Ala Pro Gly Gln
   1               5                   10                  15
   Thr Ala Arg Ile Ser Cys Ser Gly Asp Ala Leu Gly Asp Lys Tyr Ala
               20                  25                  30
   Ser Trp Tyr Gln Gln Lys Pro Gly Gln Ala Pro Val Leu Val Ile Tyr
           35                  40                  45
   Asp Asp Ser Asp Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Gln Gln His Tyr Thr Thr Pro Pro Val
                   85                  90                  95
   Phe Gly Gly Gly Thr Lys Leu Thr Val Leu Gly
               100                 105
(2) INFORMATION FOR SEQ ID NO: 56:
   (i) SEQUENCE CHARACTERISTICS:
      (A) LENGTH: 361 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene"
   (ix) FEATURE:
      (A) NAME/KEY: CDS
      (B) LOCATION:1..360
      (D) OTHER INFORMATION:/product= "VH1A"
   (xi) SEQUENCE DESCRIPTION: SEQ ID NO: 56:
   CAG GTG CAA TTG GTT CAG TCT GGC GCG GAA GTG AAA AAA CCG GGC AGC      48
   Gln Val Gln Leu Val Gln Ser Gly Ala Glu Val Lys Lys Pro Gly Ser
           110                 115                 120
   AGC GTG AAA GTG AGC TGC AAA GCC TCC GGA GGC ACT TTT AGC AGC TAT      96
   Ser Val Lys Val Ser Cys Lys Ala Ser Gly Gly Thr Phe Ser Ser Tyr
       125                 130                 135
   GCG ATT AGC TGG GTG CGC CAA GCC CCT GGG CAG GGT CTC GAG TGG ATG     144
   Ala Ile Ser Trp Val Arg Gln Ala Pro Gly Gln Gly Leu Glu Trp Met
   140                 145                 150                 155
   GGC GGC ATT ATT CCG ATT TTT GGC ACG GCG AAC TAC GCG CAG AAG TTT     192
   Gly Gly Ile Ile Pro Ile Phe Gly Thr Ala Asn Tyr Ala Gln Lys Phe
                   160                 165                 170
   CAG GGC CGG GTG ACC ATT ACC GCG GAT GAA AGC ACC AGC ACC GCG TAT     240
   Gln Gly Arg Val Thr Ile Thr Ala Asp Glu Ser Thr Ser Thr Ala Tyr
               175                 180                 185
   ATG GAA CTG AGC AGC CTG CGT AGC GAA GAT ACG GCC GTG TAT TAT TGC     288
   Met Glu Leu Ser Ser Leu Arg Ser Glu Asp Thr Ala Val Tyr Tyr Cys
           190                 195                 200
   GCG CGT TGG GGC GGC GAT GGC TTT TAT GCG ATG GAT TAT TGG GGC CAA     336
   Ala Arg Trp Gly Gly Asp Gly Phe Tyr Ala Met Asp Tyr Trp Gly Gln
       205                 210                 215
   GGC ACC CTG GTG ACG GTT AGC TCA G                                   361
   Gly Thr Leu Val Thr Val Ser Ser
   220                 225
(2) INFORMATION FOR SEQ ID NO: 57:
   (i) SEQUENCE CHARACTERISTICS:
      (A) LENGTH: 120 amino acids
      (B) TYPE: amino acid
      (D) TOPOLOGY: linear
   (ii) MOLECULE TYPE: protein
   (xi) SEQUENCE DESCRIPTION: SEQ ID NO: 57:
   Gln Val Gln Leu Val Gln Ser Gly Ala Glu Val Lys Lys Pro Gly Ser
    1               5                  10                  15
   Ser Val Lys Val Ser Cys Lys Ala Ser Gly Gly Thr Phe Ser Ser Tyr
               20                  25                  30
   Ala Ile Ser Trp Val Arg Gln Ala Pro Gly Gln Gly Leu Glu Trp Met
           35                  40                  45
   Gly Gly Ile Ile Pro Ile Phe Gly Thr Ala Asn Tyr Ala Gln Lys Phe
       50                  55                  60
   Gln Gly Arg Val Thr Ile Thr Ala Asp Glu Ser Thr Ser Thr Ala Tyr
    65                  70                  75                  80
   Met Glu Leu Ser Ser Leu Arg Ser Glu Asp Thr Ala Val Tyr Tyr Cys
                   85                  90                  95
   Ala Arg Trp Gly Gly Asp Gly Phe Tyr Ala Met Asp Tyr Trp Gly Gln
               100                 105                 110
   Gly Thr Leu Val Thr Val Ser Ser
           115                 120
(2) INFORMATION FOR SEQ ID NO: 58:
   (i) SEQUENCE CHARACTERISTICS:
      (A) LENGTH: 361 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene"
   (ix) FEATURE:
      (A) NAME/KEY: CDS
      (B) LOCATION:1..360
      (D) OTHER INFORMATION:/product= "VH1B"
   (xi) SEQUENCE DESCRIPTION: SEQ ID NO: 58:
   CAG GTG CAA TTG GTT CAG AGC GGC GCG GAA GTG AAA AAA CCG GGC GCG      48
   Gln Val Gln Leu Val Gln Ser Gly Ala Glu Val Lys Lys Pro Gly Ala
                   125                 130                 135
   AGC GTG AAA GTG AGC TGC AAA GCC TCC GGA TAT ACC TTT ACC AGC TAT      96
   Ser Val Lys Val Ser Cys Lys Ala Ser Gly Tyr Thr Phe Thr Ser Tyr
               140                 145                 150
   TAT ATG CAC TGG GTC CGC CAA GCC CCT GGG CAG GGT CTC GAG TGG ATG     144
   Tyr Met His Trp Val Arg Gln Ala Pro Gly Gln Gly Leu Glu Trp Met
           155                 160                 165
   GGC TGG ATT AAC CCG AAT AGC GGC GGC ACG AAC TAC GCG CAG AAG TTT     192
   Gly Trp Ile Asn Pro Asn Ser Gly Gly Thr Asn Tyr Ala Gln Lys Phe
       170                 175                 180
   CAG GGC CGG GTG ACC ATG ACC CGT GAT ACC AGC ATT AGC ACC GCG TAT     240
   Gln Gly Arg Val Thr Met Thr Arg Asp Thr Ser Ile Ser Thr Ala Tyr
   185                 190                 195                 200
   ATG GAA CTG AGC AGC CTG CGT AGC GAA GAT ACG GCC GTG TAT TAT TGC     288
   Met Glu Leu Ser Ser Leu Arg Ser Glu Asp Thr Ala Val Tyr Tyr Cys
                   205                 210                 215
   GCG CGT TGG GGC GGC GAT GGC TTT TAT GCG ATG GAT TAT TGG GGC CAA     336
   Ala Arg Trp Gly Gly Asp Gly Phe Tyr Ala Met Asp Tyr Trp Gly Gln
               220                 225                 230
   GGC ACC CTG GTG ACG GTT AGC TCA G                                   361
   Gly Thr Leu Val Thr Val Ser Ser
           235                 240
(2) INFORMATION FOR SEQ ID NO: 59:
   (i) SEQUENCE CHARACTERISTICS:
      (A) LENGTH: 120 amino acids
      (B) TYPE: amino acid
      (D) TOPOLOGY: linear
   (ii) MOLECULE TYPE: protein
   (xi) SEQUENCE DESCRIPTION: SEQ ID NO: 59:
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Ser Tyr
               20                  25                  30
   Tyr Met His Trp Val Arg Gln Ala Pro Gly Gln Gly Leu Glu Trp Met
           35                  40                  45
   Gly Trp Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Trp Gly Gly Asp Gly Phe Tyr Ala Met Asp Tyr Trp Gly Gln
               100                 105                 110
   Gly Thr Leu Val Thr Val Ser Ser
           115                 120
(2) INFORMATION FOR SEQ ID NO: 60:
   (i) SEQUENCE CHARACTERISTICS:
      (A) LENGTH: 364 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene"
   (ix) FEATURE:
      (A) NAME/KEY: CDS
      (B) LOCATION:1..363
      (D) OTHER INFORMATION:/product= "VH2"
   (xi) SEQUENCE DESCRIPTION: SEQ ID NO: 60:
   CAG GTG CAA TTG AAA GAA AGC GGC CCG GCC CTG GTG AAA CCG ACC CAA      48
   Gln Val Gln Leu Lys Glu Ser Gly Pro Ala Leu Val Lys Pro Thr Gln
                   125                 130                 135
   ACC CTG ACC CTG ACC TGT ACC TTT TCC GGA TTT AGC CTG TCC ACG TCT      96
   Thr Leu Thr Leu Thr Cys Thr Phe Ser Gly Phe Ser Leu Ser Thr Ser
               140                 145                 150
   GGC GTT GGC GTG GGC TGG ATT CGC CAG CCG CCT GGG AAA GCC CTC GAG     144
   Gly Val Gly Val Gly Trp Ile Arg Gln Pro Pro Gly Lys Ala Leu Glu
           155                 160                 165
   TGG CTG GCT CTG ATT GAT TGG GAT GAT GAT AAG TAT TAT AGC ACC AGC     192
   Trp Leu Ala Leu Ile Asp Trp Asp Asp Asp Lys Tyr Tyr Ser Thr Ser
       170                 175                 180
   CTG AAA ACG CGT CTG ACC ATT AGC AAA GAT ACT TCG AAA AAT CAG GTG     240
   Leu Lys Thr Arg Leu Thr Ile Ser Lys Asp Thr Ser Lys Asn Gln Val
   185                 190                 195                 200
   GTG CTG ACT ATG ACC AAC ATG GAC CCG GTG GAT ACG GCC ACC TAT TAT     288
   Val Leu Thr Met Thr Asn Met Asp Pro Val Asp Thr Ala Thr Tyr Tyr
                   205                 210                 215
   TGC GCG CGT TGG GGC GGC GAT GGC TTT TAT GCG ATG GAT TAT TGG GGC     336
   Cys Ala Arg Trp Gly Gly Asp Gly Phe Tyr Ala Met Asp Tyr Trp Gly
               220                 225                 230
   CAA GGC ACC CTG GTG ACG GTT AGC TCA G                               364
   Gln Gly Thr Leu Val Thr Val Ser Ser
           235                 240
(2) INFORMATION FOR SEQ ID NO: 61:
   (i) SEQUENCE CHARACTERISTICS:
      (A) LENGTH: 121 amino acids
      (B) TYPE: amino acid
      (D) TOPOLOGY: linear
   (ii) MOLECULE TYPE: protein
   (xi) SEQUENCE DESCRIPTION: SEQ ID NO: 61:
   Gln Val Gln Leu Lys Glu Ser Gly Pro Ala Leu Val Lys Pro Thr Gln
    1               5                  10                  15
   Thr Leu Thr Leu Thr Cys Thr Phe Ser Gly Phe Ser Leu Ser Thr Ser
               20                  25                  30
   Gly Val Gly Val Gly Trp Ile Arg Gln Pro Pro Gly Lys Ala Leu Glu
           35                  40                  45
   Trp Leu Ala Leu Ile Asp Trp Asp Asp Asp Lys Tyr Tyr Ser Thr Ser
       50                  55                  60
   Leu Lys Thr Arg Leu Thr Ile Ser Lys Asp Thr Ser Lys Asn Gln Val
   65                  70                  75                  80
   Val Leu Thr Met Thr Asn Met Asp Pro Val Asp Thr Ala Thr Tyr Tyr
                   85                  90                  95
   Cys Ala Arg Trp Gly Gly Asp Gly Phe Tyr Ala Met Asp Tyr Trp Gly
               100                 105                 110
   Gln Gly Thr Leu Val Thr Val Ser Ser
           115                 120
(2) INFORMATION FOR SEQ ID NO: 62:
   (i) SEQUENCE CHARACTERISTICS:
      (A) LENGTH: 361 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene"
   (ix) FEATURE:
      (A) NAME/KEY: CDS
      (B) LOCATION:1..360
      (D) OTHER INFORMATION:/product= "VH3"
   (xi) SEQUENCE DESCRIPTION: SEQ ID NO: 62:
   GAA GTG CAA TTG GTG GAA AGC GGC GGC GGC CTG GTG CAA CCG GGC GGC      48
   Glu Val Gln Leu Val Glu Ser Gly Gly Gly Leu Val Gln Pro Gly Gly
               125                 130                 135
   AGC CTG CGT CTG AGC TGC GCG GCC TCC GGA TTT ACC TTT AGC AGC TAT      96
   Ser Leu Arg Leu Ser Cys Ala Ala Ser Gly Phe Thr Phe Ser Ser Tyr
           140                 145                 150
   GCG ATG AGC TGG GTG CGC CAA GCC CCT GGG AAG GGT CTC GAG TGG GTG     144
   Ala Met Ser Trp Val Arg Gln Ala Pro Gly Lys Gly Leu Glu Trp Val
       155                 160                 165
   AGC GCG ATT AGC GGT AGC GGC GGC AGC ACC TAT TAT GCG GAT AGC GTG     192
   Ser Ala Ile Ser Gly Ser Gly Gly Ser Thr Tyr Tyr Ala Asp Ser Val
   170                 175                 180                 185
   AAA GGC CGT TTT ACC ATT TCA CGT GAT AAT TCG AAA AAC ACC CTG TAT     240
   Lys Gly Arg Phe Thr Ile Ser Arg Asp Asn Ser Lys Asn Thr Leu Tyr
                   190                 195                 200
   CTG CAA ATG AAC AGC CTG CGT GCG GAA GAT ACG GCC GTG TAT TAT TGC     288
   Leu Gln Met Asn Ser Leu Arg Ala Glu Asp Thr Ala Val Tyr Tyr Cys
               205                 210                 215
   GCG CGT TGG GGC GGC GAT GGC TTT TAT GCG ATG GAT TAT TGG GGC CAA     336
   Ala Arg Trp Gly Gly Asp Gly Phe Tyr Ala Met Asp Tyr Trp Gly Gln
           220                 225                 230
   GGC ACC CTG GTG ACG GTT AGC TCA G                                   361
   Gly Thr Leu Val Thr Val Ser Ser
       235                 240
(2) INFORMATION FOR SEQ ID NO: 63:
   (i) SEQUENCE CHARACTERISTICS:
      (A) LENGTH: 120 amino acids
      (B) TYPE: amino acid
      (D) TOPOLOGY: linear
   (ii) MOLECULE TYPE: protein
   (xi) SEQUENCE DESCRIPTION: SEQ ID NO: 63:
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Trp Gly Gly Asp Gly Phe Tyr Ala Met Asp Tyr Trp Gly Gln
               100                 105                 110
   Gly Thr Leu Val Thr Val Ser Ser
           115                 120
(2) INFORMATION FOR SEQ ID NO: 64:
   (i) SEQUENCE CHARACTERISTICS:
      (A) LENGTH: 358 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene"
   (ix) FEATURE:
      (A) NAME/KEY: CDS
      (B) LOCATION:1..357
      (D) OTHER INFORMATION:/product= "VH4"
   (xi) SEQUENCE DESCRIPTION: SEQ ID NO: 64:
   CAG GTG CAA TTG CAA GAA AGT GGT CCG GGC CTG GTG AAA CCG AGC GAA        48
   Gln Val Gln Leu Gln Glu Ser Gly Pro Gly Leu Val Lys Pro Ser Glu
                   125                     130                 135
   ACC CTG AGC CTG ACC TGC ACC GTT TCC GGA GGC AGC ATT AGC AGC TAT        96
   Thr Leu Ser Leu Thr Cys Thr Val Ser Gly Gly Ser Ile Ser Ser Tyr
               140                 145                 150
   TAT TGG AGC TGG ATT CGC CAG CCG CCT GGG AAG GGT CTC GAG TGG ATT       144
   Tyr Trp Ser Trp Ile Arg Gln Pro Pro Gly Lys Gly Leu Glu Trp Ile
           155                 160                 165
   GGC TAT ATT TAT TAT AGC GGC AGC ACC AAC TAT AAT CCG AGC CTG AAA       192
   Gly Tyr Ile Tyr Tyr Ser Gly Ser Thr Asn Tyr Asn Pro Ser Leu Lys
       170                 175                 180
   AGC CGG GTG ACC ATT AGC GTT GAT ACT TCG AAA AAC CAG TTT AGC CTG       240
   Ser Arg Val Thr Ile Ser Val Asp Thr Ser Lys Asn Gln Phe Ser Leu
   185                 190                 195                 200
   AAA CTG AGC AGC GTG ACG GCG GCG GAT ACG GCC GTG TAT TAT TGC GCG       288
   Lys Leu Ser Ser Val Thr Ala Ala Asp Thr Ala Val Tyr Tyr Cys Ala
                   205                 210                 215
   CGT TGG GGC GGC GAT GGC TTT TAT GCG ATG GAT TAT TGG GGC CAA GGC       336
   Arg Trp Gly Gly Asp Gly Phe Tyr Ala Met Asp Tyr Trp Gly Gln Gly
               220                 225                 230
   ACC CTG GTG ACG GTT AGC TCA G                                         358
   Thr Leu Val Thr Val Ser Ser
           235
(2) INFORMATION FOR SEQ ID NO: 65:
   (i) SEQUENCE CHARACTERISTICS:
      (A) LENGTH: 119 amino acids
      (B) TYPE: amino acid
      (D) TOPOLOGY: linear
   (ii) MOLECULE TYPE: protein
   (xi) SEQUENCE DESCRIPTION: SEQ ID NO: 65:
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Ile Ser Ser Tyr
               20                  25                  30
   Tyr Trp Ser Trp Ile Arg Gln Pro Pro Gly Lys Gly Leu Glu Trp Ile
           35                  40                  45
   Gly Tyr Ile Tyr Tyr Ser Gly Ser Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Arg Trp Gly Gly Asp Gly Phe Tyr Ala Met Asp Tyr Trp Gly Gln Gly
               100                 105                 110
   Thr Leu Val Thr Val Ser Ser
           115
(2) INFORMATION FOR SEQ ID NO: 66:
   (i) SEQUENCE CHARACTERISTICS:
      (A) LENGTH: 361 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene"
   (ix) FEATURE:
      (A) NAME/KEY: CDS
      (B) LOCATION:1..360
      (D) OTHER INFORMATION:/product= "VH5"
   (xi) SEQUENCE DESCRIPTION: SEQ ID NO: 66:
   GAA GTG CAA TTG GTT CAG AGC GGC GCG GAA GTG AAA AAA CCG GGC GAA          48
   Glu Val Gln Leu Val Gln Ser Gly Ala Glu Val Lys Lys Pro Gly Glu
   120                 125                 130                 135
   AGC CTG AAA ATT AGC TGC AAA GGT TCC GGA TAT TCC TTT ACG AGC TAT          96
   Ser Leu Lys Ile Ser Cys Lys Gly Ser Gly Tyr Ser Phe Thr Ser Tyr
                   140                 145                 150
   TGG ATT GGC TGG GTG CGC CAG ATG CCT GGG AAG GGT CTC GAG TGG ATG         144
   Trp Ile Gly Trp Val Arg Gln Met Pro Gly Lys Gly Leu Glu Trp Met
               155                 160                 165
   GGC ATT ATT TAT CCG GGC GAT AGC GAT ACC CGT TAT TCT CCG AGC TTT         192
   Gly Ile Ile Tyr Pro Gly Asp Ser Asp Thr Arg Tyr Ser Pro Ser Phe
           170                 175                 180
   CAG GGC CAG GTG ACC ATT AGC GCG GAT AAA AGC ATT AGC ACC GCG TAT         240
   Gln Gly Gln Val Thr Ile Ser Ala Asp Lys Ser Ile Ser Thr Ala Tyr
       185                 190                 195
   CTT CAA TGG AGC AGC CTG AAA GCG AGC GAT ACG GCC ATG TAT TAT TGC         288
   Leu Gln Trp Ser Ser Leu Lys Ala Ser Asp Thr Ala Met Tyr Tyr Cys
   200                 205                 210                 215
   GCG CGT TGG GGC GGC GAT GGC TTT TAT GCG ATG GAT TAT TGG GGC CAA         336
   Ala Arg Trp Gly Gly Asp Gly Phe Tyr Ala Met Asp Tyr Trp Gly Gln
   GGC ACC CTG GTG ACG GTT AGC TCA G                                       361
   Gly Thr Leu Val Thr Val Ser Ser
               235
(2) INFORMATION FOR SEQ ID NO: 67:
   (i) SEQUENCE CHARACTERISTICS:
      (A) LENGTH: 120 amino acids
      (B) TYPE: amino acid
      (D) TOPOLOGY: linear
   (ii) MOLECULE TYPE: protein
   (xi) SEQUENCE DESCRIPTION: SEQ ID NO: 67:
   Glu Val Gln Leu Val Gln Ser Gly Ala Glu Val Lys Lys Pro Gly Glu
    1               5                  10                  15
   Ser Leu Lys Ile Ser Cys Lys Gly Ser Gly Tyr Ser Phe Thr Ser Tyr
               20                  25                  30
   Trp Ile Gly Trp Val Arg Gln Met Pro Gly Lys Gly Leu Glu Trp Met
           35                  40                  45
   Gly Ile Ile Tyr Pro Gly Asp Ser Asp Thr Arg Tyr Ser Pro Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Trp Gly Gly Asp Gly Phe Tyr Ala Met Asp Tyr Trp Gly Gln
               100                 105                 110
   Gly Thr Leu Val Thr Val Ser Ser
           115                 120
(2) INFORMATION FOR SEQ ID NO: 68:
   (i) SEQUENCE CHARACTERISTICS:
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene"
   (ix) FEATURE:
      (A) NAME/KEY: CDS
      (B) LOCATION:1..369
      (D) OTHER INFORMATION:/product= "VH6"
   (xi) SEQUENCE DESCRIPTION: SEQ ID NO: 68:
(2) INFORMATION FOR SEQ ID NO: 69:
   (i) SEQUENCE CHARACTERISTICS:
      (A) LENGTH: 123 amino acids
      (B) TYPE: amino acid
      (D) TOPOLOGY: linear
   (ii) MOLECULE TYPE: protein
   (xi) SEQUENCE DESCRIPTION: SEQ ID NO: 69:
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Asn
               20                  25                  30
   Ser Ala Ala Trp Asn Trp Ile Arg Gln Ser Pro Gly Arg Gly Leu Glu
           35                  40                  45
   Trp Leu Gly Arg Thr Tyr Tyr Arg Ser Lys Trp Tyr Asn Asp Tyr Ala
       50                  55                  60
   Val Ser Val Lys Ser Arg Ile Thr Ile Asn Pro Asp Thr Ser Lys Asn
   65                  70                  75                  80
   Gln Phe Ser Leu Gln Leu Asn Ser Val Thr Pro Glu Asp Thr Ala Val
                   85                  90                  95
   Tyr Tyr Cys Ala Arg Trp Gly Gly Asp Gly Phe Tyr Ala Met Asp Tyr
               100                 105                 110
   Trp Gly Gln Gly Thr Leu Val Thr Val Ser Ser
           115                 120
(2) INFORMATION FOR SEQ ID NO: 70:
   (i) SEQUENCE CHARACTERISTICS:
      (A) LENGTH: 49 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 70:
      GAATGCATAC GCTGATATCC AGATGACCCA GAGCCCGTCT AGCCTGAGC 49
(2) INFORMATION FOR SEQ ID NO: 71:
   (i) SEQUENCE CHARACTERISTICS:
      (A) LENGTH: 56 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 71:
      CGCTCTGCAG GTAATGGTCA CACGATCACC CACGCTCGCG CTCAGGCTAG ACGGGC 56
(2) INFORMATION FOR SEQ ID NO: 72:
   (i) SEQUENCE CHARACTERISTICS:
      (A) LENGTH: 58 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 72:
      GACCATTACC TGCAGAGCGA GCCAGGGCAT TAGCAGCTAT CTGGCGTGGT ACCAGCAG 58
(2) INFORMATION FOR SEQ ID NO: 73:
   (i) SEQUENCE CHARACTERISTICS:
      (A) LENGTH: 71 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 73:
   CTTTGCAAGC TGCTGGCTGC ATAAATTAAT AGTTTCGGTG CTTTACCTGG TTTCTGCTGG 60
   TACCACGCCA G 71
(2) INFORMATION FOR SEQ ID NO: 74:
   (i) SEQUENCE CHARACTERISTICS:
      (A) LENGTH: 67 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 74:
   CAGCCAGCAG CTTGCAAAGC GGGGTCCCGT CCCGTTTTAG CGGCTCTGGA TCCGGCACTG 60
   ATTTTAC 67
(2) INFORMATION FOR SEQ ID NO: 75:
   (i) SEQUENCE CHARACTERISTICS:
      (A) LENGTH: 67 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 75:
   GATAATAGGT CGCAAAGTCT TCAGGTTGCA GGCTGCTAAT GGTCAGGGTA AAATCAGTGC 60
   CGGATCC 67
(2) INFORMATION FOR SEQ ID NO: 76:
   (i) SEQUENCE CHARACTERISTICS:
      (A) LENGTH: 54 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 76:
      CGATATCGTG ATGACCCAGA GCCCACTGAG CCTGCCAGTG ACTCCGGGCG AGCC 54
(2) INFORMATION FOR SEQ ID NO: 77:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 77:
   GCCGTTGCTA TGCAGCAGGC TTTGGCTGCT TCTGCAGCTA ATGCTCGCAG GCTCGCCCGG 60
   AGTCAC 66
(2) INFORMATION FOR SEQ ID NO: 78:
   (i) SEQUENCE CHARACTERISTICS:
      (A) LENGTH: 62 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 78:
   CTGCTGCATA GCAACGGCTA TAACTATCTG GATTGGTACC TTCAAAAACC AGGTCAAAGC 60
   CC 62
(2) INFORMATION FOR SEQ ID NO: 79:
   (i) SEQUENCE CHARACTERISTICS:
      (A) LENGTH: 71 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 79:
   CGATCCGGGA CCCCACTGGC ACGGTTGCTG CCCAGATAAA TTAATAGCTG CGGGCTTTGA 60
   CCTGGTTTTT G 71
(2) INFORMATION FOR SEQ ID NO: 80:
   (i) SEQUENCE CHARACTERISTICS:
      (A) LENGTH: 69 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 80:
   AGTGGGGTCC CGGATCGTTT TAGCGGCTCT GGATCCGGCA CCGATTTTAC CCTGAAAATT 60
   AGCCGTGTG 69
(2) INFORMATION FOR SEQ ID NO: 81:
   (i) SEQUENCE CHARACTERISTICS:
      (A) LENGTH: 54 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 81:
      CCATGCAATA ATACACGCCC ACGTCTTCAG CTTCCACACG GCTAATTTTC AGGG 54
(2) INFORMATION FOR SEQ ID NO: 82:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 82:
      GAATGCATAC GCTGATATCG TGCTGACCCA GAGCCCGG 38
(2) INFORMATION FOR SEQ ID NO: 83:
   (i) SEQUENCE CHARACTERISTICS:
      (A) LENGTH: 67 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 83:
   CGCTCTGCAG CTCAGGGTCG CACGTTCGCC CGGAGACAGG CTCAGGGTCG CCGGGCTCTG 60
   GGTCAGC 67
(2) INFORMATION FOR SEQ ID NO: 84:
   (i) SEQUENCE CHARACTERISTICS:
      (A) LENGTH: 56 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 84:
      CCCTGAGCTG CAGAGCGAGC CAGAGCGTGA GCAGCAGCTA TCTGGCGTGG TACCAG 56
(2) INFORMATION FOR SEQ ID NO: 85:
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 85:
   GCACGGCTGC TCGCGCCATA AATTAATAGA CGCGGTGCTT GACCTGGTTT CTGCTGGTAC 60
   CACGCCAGAT AG 72
(2) INFORMATION FOR SEQ ID NO: 86:
   (i) SEQUENCE CHARACTERISTICS:
      (A) LENGTH: 67 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 86:
   GCGCGAGCAG CCGTGCAACT GGGGTCCCGG CGCGTTTTAG CGGCTCTGGA TCCGGCACGG 60
   ATTTTAC 67
(2) INFORMATION FOR SEQ ID NO: 87:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 87:
   GATAATACAC CGCAAAGTCT TCAGGTTCCA GGCTGCTAAT GGTCAGGGTA AAATCCGTGC 60
   CGGATC 66
(2) INFORMATION FOR SEQ ID NO: 88:
   (i) SEQUENCE CHARACTERISTICS:
      (A) LENGTH: 49 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 88:
      GAATGCATAC GCTGATATCG TGATGACCCA GAGCCCGGAT AGCCTGGCG 49
(2) INFORMATION FOR SEQ ID NO: 89:
   (i) SEQUENCE CHARACTERISTICS:
      (A) LENGTH: 56 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 89:
      GCTTCTGCAG TTAATGGTCG CACGTTCGCC CAGGCTCACC GCCAGGCTAT CCGGGC 56
(2) INFORMATION FOR SEQ ID NO: 90:
   (i) SEQUENCE CHARACTERISTICS:
      (A) LENGTH: 74 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 90:
   CGACCATTAA CTGCAGAAGC AGCCAGAGCG TGCTGTATAG CAGCAACAAC AAAAACTATC 60
   TGGCGTGGTA CCAG 74
(2) INFORMATION FOR SEQ ID NO: 91:
   (i) SEQUENCE CHARACTERISTICS:
      (A) LENGTH: 63 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 91:
   GATGCCCAAT AAATTAATAG TTTCGGCGGC TGACCTGGTT TCTGCTGGTA CCACGCCAGA 60
   TAG 63
(2) INFORMATION FOR SEQ ID NO: 92:
   (i) SEQUENCE CHARACTERISTICS:
      (A) LENGTH: 74 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 92:
   AAACTATTAA TTTATTGGGC ATCCACCCGT GAAAGCGGGG TCCCGGATCG TTTTAGCGGC 60
   TCTGGATCCG GCAC 74
(2) INFORMATION FOR SEQ ID NO: 93:
   (i) SEQUENCE CHARACTERISTICS:
      (A) LENGTH: 73 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 93:
   GATAATACAC CGCCACGTCT TCAGCTTGCA GGGACGAAAT GGTCAGGGTA AAATCAGTGC 60
   CGGATCCAGA GCC 73
(2) INFORMATION FOR SEQ ID NO: 94:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 94:
      GAATGCATAC GCTCAGAGCG TGCTGACCCA GCCGCCTTCA GTGAGTGG 48
(2) INFORMATION FOR SEQ ID NO: 95:
   (i) SEQUENCE CHARACTERISTICS:
      (A) LENGTH: 71 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 95:
   CAATGTTGCT GCTGCTGCCG CTACACGAGA TGGTCACACG CTGACCTGGT GCGCCACTCA 60
   CTGAAGGCGG C 71
(2) INFORMATION FOR SEQ ID NO: 96:
   (i) SEQUENCE CHARACTERISTICS:
      (A) LENGTH: 59 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 96:
      GGCAGCAGCA GCAACATTGG CAGCAACTAT GTGAGCTGGT ACCAGCAGTT GCCCGGGAC 59
(2) INFORMATION FOR SEQ ID NO: 97:
   (i) SEQUENCE CHARACTERISTICS:
      (A) LENGTH: 68 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 97:
   CCGGCACGCC TGAGGGACGC TGGTTGTTAT CATAAATCAG CAGTTTCGGC GCCGTCCCGG 60
   GCAACTGC 68
(2) INFORMATION FOR SEQ ID NO: 98:
   (i) SEQUENCE CHARACTERISTICS:
      (A) LENGTH: 60 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 98:
      CCCTCAGGCG TGCCGGATCG TTTTAGCGGA TCCAAAAGCG GCACCAGCGC GAGCCTTGCG 60
(2) INFORMATION FOR SEQ ID NO: 99:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 99:
      CCGCTTCGTC TTCGCTTTGC AGGCCCGTAA TCGCAAGGCT CGCGCTGG 48
(2) INFORMATION FOR SEQ ID NO: 100:
   (i) SEQUENCE CHARACTERISTICS:
      (A) LENGTH: 49 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 100:
      GAATGCATAC GCTCAGAGCG CACTGACCCA GCCAGCTTCA GTGAGCGGC 49
(2) INFORMATION FOR SEQ ID NO: 101:
   (i) SEQUENCE CHARACTERISTICS:
      (A) LENGTH: 64 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 101:
   CGCTGCTAGT ACCCGTACAC GAGATGGTAA TGCTCTGACC TGGTGAGCCG CTCACTGAAG 60
   CTGG 64
(2) INFORMATION FOR SEQ ID NO: 102:
   (i) SEQUENCE CHARACTERISTICS:
      (A) LENGTH: 64 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 102:
   GTACGGGTAC TAGCAGCGAT GTGGGCGGCT ATAACTATGT GAGCTGGTAC CAGCAGCATC 60
   CCGG 64
(2) INFORMATION FOR SEQ ID NO: 103:
   (i) SEQUENCE CHARACTERISTICS:
      (A) LENGTH: 68 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 103:
   CGCCTGAGGG ACGGTTGCTC ACATCATAAA TCATCAGTTT CGGCGCCTTC CCGGGATGCT 60
   GCTGGTAC 68
(2) INFORMATION FOR SEQ ID NO: 104:
   (i) SEQUENCE CHARACTERISTICS:
      (A) LENGTH: 62 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 104:
      CAACCGTCCC TCAGGCGTGA GCAACCGTTT TAGCGGATCC AAAAGCGGCA ACACCGCGAG 60
(2) INFORMATION FOR SEQ ID NO: 105:
   (i) SEQUENCE CHARACTERISTICS:
      (A) LENGTH: 53 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 105:
      CCGCTTCGTC TTCCGCTTGC AGGCCGCTAA TGGTCAGGCT CGCGGTGTTG CCG 53
(2) INFORMATION FOR SEQ ID NO: 106:
   (i) SEQUENCE CHARACTERISTICS:
      (A) LENGTH: 47 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 106:
      GAATGCATAC GCTAGCTATG AACTGACCCA GCCGCCTTCA GTGAGCG 47
(2) INFORMATION FOR SEQ ID NO: 107:
   (i) SEQUENCE CHARACTERISTICS:
      (A) LENGTH: 68 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 107:
   CGCCCAGCGC ATCGCCGCTA CACGAGATAC GCGCGGTCTG ACCTGGTGCA ACGCTCACTG 60
   AAGGCGGC 68
(2) INFORMATION FOR SEQ ID NO: 108:
   (i) SEQUENCE CHARACTERISTICS:
      (A) LENGTH: 58 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 108:
      GGCGATGCGC TGGGCGATAA ATACGCGAGC TGGTACCAGC AGAAACCCGG GCAGGCGC 58
(2) INFORMATION FOR SEQ ID NO: 109:
   (i) SEQUENCE CHARACTERISTICS:
      (A) LENGTH: 70 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 109:
   GCGTTCCGGG ATGCCTGAGG GACGGTCAGA ATCATCATAA ATCACCAGAA CTGGCGCCTG 60
   CCCGGGTTTC 70
(2) INFORMATION FOR SEQ ID NO: 110:
   (i) SEQUENCE CHARACTERISTICS:
      (A) LENGTH: 64 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 110:
   CAGGCATCCC GGAACGCTTT AGCGGATCCA ACAGCGGCAA CACCGCGACC CTGACCATTA 60
   GCGG 64
(2) INFORMATION FOR SEQ ID NO: 111:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 111:
      CCGCTTCGTC TTCCGCCTGA GTGCCGCTAA TGGTCAGGGT C 41
(2) INFORMATION FOR SEQ ID NO: 112:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 112:
      GCTCTTCACC CCTGTTACCA AAGCCCAGGT GCAATTG 37
(2) INFORMATION FOR SEQ ID NO: 113:
   (i) SEQUENCE CHARACTERISTICS:
      (A) LENGTH: 79 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 113:
   GGCTTTGCAG CTCACTTTCA CGCTGCTGCC CGGTTTTTTC ACTTCCGCGC CAGACTGAAC 60
   CAATTGCACC TGGGCTTTG 79
(2) INFORMATION FOR SEQ ID NO: 114:
   (i) SEQUENCE CHARACTERISTICS:
      (A) LENGTH: 80 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 114:
   GAAAGTGAGC TGCAAAGCCT CCGGAGGCAC TTTTAGCAGC TATGCGATTA GCTGGGTGCG 60
   CCAAGCCCCT GGGCAGGGTC 80
(2) INFORMATION FOR SEQ ID NO: 115:
   (i) SEQUENCE CHARACTERISTICS:
      (A) LENGTH: 81 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 115:
   GCCCTGAAAC TTCTGCGCGT AGTTCGCCGT GCCAAAAATC GGAATAATGC CGCCCATCCA 60
   CTCGAGACCC TGCCCAGGGG C 81
(2) INFORMATION FOR SEQ ID NO: 116:
   (i) SEQUENCE CHARACTERISTICS:
      (A) LENGTH: 80 base pairs
      (B) TYPE: nucleic acid
      (C) STRANDEDNESS: single
      (D) TOPOLOGY: linear
   (ii) MOLECULE TYPE: other nucleic acid (̅
      A) DESCRIPTION: /desc = "synthetic oligonucleotide"
   (xi) SEQUENCE DESCRIPTION: SEQ ID NO: 116:
   GCGCAGAAGT TTCAGGGCCG GGTGACCATT ACCGCGGATG AAAGCACCAG CACCGCGTAT 60
   ATGGAACTGA GCAGCCTGCG 80
(2) INFORMATION FOR SEQ ID NO: 117:
   (i) SEQUENCE CHARACTERISTICS:
      (A) LENGTH: 50 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 117:
      GCGCGCAATA ATACACGGCC GTATCTTCGC TACGCAGGCT GCTCAGTTCC 50
(2) INFORMATION FOR SEQ ID NO: 118:
   (i) SEQUENCE CHARACTERISTICS:
      (A) LENGTH: 79 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 118:
   GGCTTTGCAG CTCACTTTCA CGCTCGCGCC CGGTTTTTTC ACTTCCGCGC CGCTCTGAAC 60
   CAATTGCACC TGGGCTTTG 79
(2) INFORMATION FOR SEQ ID NO: 119:
   (i) SEQUENCE CHARACTERISTICS:
      (A) LENGTH: 80 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 119:
   GAAAGTGAGC TGCAAAGCCT CCGGATATAC CTTTACCAGC TATTATATGC ACTGGGTCCG 60
   CCAAGCCCCT GGGCAGGGTC 80
(2) INFORMATION FOR SEQ ID NO: 120:
   (i) SEQUENCE CHARACTERISTICS:
      (A) LENGTH: 81 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 120:
   GCCCTGAAAC TTCTGCGCGT AGTTCGTGCC GCCGCTATTC GGGTTAATCC AGCCCATCCA 60
   CTCGAGACCC TGCCCAGGGG C 81
(2) INFORMATION FOR SEQ ID NO: 121:
   (i) SEQUENCE CHARACTERISTICS:
      (A) LENGTH: 80 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 121:
   GCGCAGAAGT TTCAGGGCCG GGTGACCATG ACCCGTGATA CCAGCATTAG CACCGCGTAT 60
   ATGGAACTGA GCAGCCTGCG 80
(2) INFORMATION FOR SEQ ID NO: 122:
   (i) SEQUENCE CHARACTERISTICS:
      (A) LENGTH: 76 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 122:
   GGTACAGGTC AGGGTCAGGG TTTGGGTCGG TTTCACCAGG GCCGGGCCGC TTTCTTTCAA    60
   TTGCACCTGG GCTTTG                                                    76
(2) INFORMATION FOR SEQ ID NO: 123:
   (i) SEQUENCE CHARACTERISTICS:
      (A) LENGTH: 85 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 123:
   CTGACCCTGA CCTGTACCTT TTCCGGATTT AGCCTGTCCA CGTCTGGCGT TGGCGTGGGC    60
   TGGATTCGCC AGCCGCCTGG GAAAG                                          85
(2) INFORMATION FOR SEQ ID NO: 124:
   (i) SEQUENCE CHARACTERISTICS:
      (A) LENGTH: 83 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 124:
   GCGTTTTCAG GCTGGTGCTA TAATACTTAT CATCATCCCA ATCAATCAGA GCCAGCCACT    60
   CGAGGGCTTT CCCAGGCGGC TGG                                            83
(2) INFORMATION FOR SEQ ID NO: 125:
   (i) SEQUENCE CHARACTERISTICS:
      (A) LENGTH: 78 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 125:
   GCACCAGCCT GAAAACGCGT CTGACCATTA GCAAAGATAC TTCGAAAAAT CAGGTGGTGC 60
   TGACTATGAC CAACATGG 78
(2) INFORMATION FOR SEQ ID NO: 126:
   (i) SEQUENCE CHARACTERISTICS:
      (A) LENGTH: 53 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 126:
      GCGCGCAATA ATAGGTGGCC GTATCCACCG GGTCCATGTT GGTCATAGTC AGC 53
(2) INFORMATION FOR SEQ ID NO: 127:
   (i) SEQUENCE CHARACTERISTICS:
      (A) LENGTH: 51 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 127:
      CGAAGTGCAA TTGGTGGAAA GCGGCGGCGG CCTGGTGCAA CCGGGCGGCA G 51
(2) INFORMATION FOR SEQ ID NO: 128:
   (i) SEQUENCE CHARACTERISTICS:
      (A) LENGTH: 64 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 128:
   CATAGCTGCT AAAGGTAAAT CCGGAGGCCG CGCAGCTCAG ACGCAGGCTG CCGCCCGGTT 60
   GCAC 64
(2) INFORMATION FOR SEQ ID NO: 129:
   (i) SEQUENCE CHARACTERISTICS:
      (A) LENGTH: 70 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 129:
   GATTTACCTT TAGCAGCTAT GCGATGAGCT GGGTGCGCCA AGCCCCTGGG AAGGGTCTCG 60
   AGTGGGTGAG 70
(2) INFORMATION FOR SEQ ID NO: 130:
   (i) SEQUENCE CHARACTERISTICS:
      (A) LENGTH: 71 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 130:
   GGCCTTTCAC GCTATCCGCA TAATAGGTGC TGCCGCCGCT ACCGCTAATC GCGCTCACCC 60
   ACTCGAGACC C 71
(2) INFORMATION FOR SEQ ID NO: 131:
   (i) SEQUENCE CHARACTERISTICS:
      (A) LENGTH: 73 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 131:
   CGGATAGCGT GAAAGGCCGT TTTACCATTT CACGTGATAA TTCGAAAAAC ACCCTGTATC 60
   TGCAAATGAA CAG 73
(2) INFORMATION FOR SEQ ID NO: 132:
   (i) SEQUENCE CHARACTERISTICS:
      (A) LENGTH: 62 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 132:
   CACGCGCGCA ATAATACACG GCCGTATCTT CCGCACGCAG GCTGTTCATT TGCAGATACA 60
   GG 62
(2) INFORMATION FOR SEQ ID NO: 133:
   (i) SEQUENCE CHARACTERISTICS:
      (A) LENGTH: 70 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 133:
   GGTCAGGCTC AGGGTTTCGC TCGGTTTCAC CAGGCCCGGA CCACTTTCTT GCAATTGCAC 60
   CTGGGCTTTG 70
(2) INFORMATION FOR SEQ ID NO: 134:
   (i) SEQUENCE CHARACTERISTICS:
      (A) LENGTH: 76 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 134:
   GAAACCCTGA GCCTGACCTG CACCGTTTCC GGAGGCAGCA TTAGCAGCTA TTATTGGAGC 60
   TGGATTCGCC AGCCGC 76
(2) INFORMATION FOR SEQ ID NO: 135:
   (i) SEQUENCE CHARACTERISTICS:
      (A) LENGTH: 77 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 135:
   GATTATAGTT GGTGCTGCCG CTATAATAAA TATAGCCAAT CCACTCGAGA CCCTTCCCAG 60
   GCGGCTGGCG AATCCAG 77
(2) INFORMATION FOR SEQ ID NO: 136:
   (i) SEQUENCE CHARACTERISTICS:
      (A) LENGTH: 79 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 136:
   CGGCAGCACC AACTATAATC CGAGCCTGAA AAGCCGGGTG ACCATTAGCG TTGATACTTC 60
   GAAAAACCAG TTTAGCCTG 79
(2) INFORMATION FOR SEQ ID NO: 137:
   (i) SEQUENCE CHARACTERISTICS:
      (A) LENGTH: 69 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 137:
   GCGCGCAATA ATACACGGCC GTATCCGCCG CCGTCACGCT GCTCAGTTTC AGGCTAAACT 60
   GGTTTTTCG 69
(2) INFORMATION FOR SEQ ID NO: 138:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 138:
      GCTCTTCACC CCTGTTACCA AAGCCGAAGT GCAATTG 37
(2) INFORMATION FOR SEQ ID NO: 139:
   (i) SEQUENCE CHARACTERISTICS:
      (A) LENGTH: 79 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 139:
   CCTTTGCAGC TAATTTTCAG GCTTTCGCCC GGTTTTTTCA CTTCCGCGCC GCTCTGAACC 60
   AATTGCACTT CGGCTTTGG 79
(2) INFORMATION FOR SEQ ID NO: 140:
   (i) SEQUENCE CHARACTERISTICS:
      (A) LENGTH: 75 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 140:
   CCTGAAAATT AGCTGCAAAG GTTCCGGATA TTCCTTTACG AGCTATTGGA TTGGCTGGGT 60
   GCGCCAGATG CCTGG 75
(2) INFORMATION FOR SEQ ID NO: 141:
   (i) SEQUENCE CHARACTERISTICS:
      (A) LENGTH: 78 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 141:
   CGGAGAATAA CGGGTATCGC TATCGCCCGG ATAAATAATG CCCATCCACT CGAGACCCTT 60
   CCCAGGCATC TGGCGCAC 78
(2) INFORMATION FOR SEQ ID NO: 142:
   (i) SEQUENCE CHARACTERISTICS:
      (A) LENGTH: 77 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 142:
   CGATACCCGT TATTCTCCGA GCTTTCAGGG CCAGGTGACC ATTAGCGCGG ATAAAAGCAT 60
   TAGCACCGCG TATCTTC 77
(2) INFORMATION FOR SEQ ID NO: 143:
   (i) SEQUENCE CHARACTERISTICS:
      (A) LENGTH: 68 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 143:
   GCGCGCAATA ATACATGGCC GTATCGCTCG CTTTCAGGCT GCTCCATTGA AGATACGCGG 60
   TGCTAATG 68
(2) INFORMATION FOR SEQ ID NO: 144:
   (i) SEQUENCE CHARACTERISTICS:
      (A) LENGTH: 81 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 144:
   GAAATCGCAC AGGTCAGGCT CAGGGTTTGG CTCGGTTTCA CCAGGCCCGG ACCAGACTGT 60
   TGCAATTGCA CCTGGGCTTT G 81
(2) INFORMATION FOR SEQ ID NO: 145:
   (i) SEQUENCE CHARACTERISTICS:
      (A) LENGTH: 79 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 145:
   GCCTGACCTG TGCGATTTCC GGAGATAGCG TGAGCAGCAA CAGCGCGGCG TGGAACTGGA 60
   TTCGCCAGTC TCCTGGGCG 79
(2) INFORMATION FOR SEQ ID NO: 146:
   (i) SEQUENCE CHARACTERISTICS:
      (A) LENGTH: 78 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 146:
   CACCGCATAA TCGTTATACC ATTTGCTACG ATAATAGGTA CGGCCCAGCC ACTCGAGGCC 60
   ACGCCCAGGA GACTGGCG 78
(2) INFORMATION FOR SEQ ID NO: 147:
   (i) SEQUENCE CHARACTERISTICS:
      (A) LENGTH: 78 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 147:
   GGTATAACGA TTATGCGGTG AGCGTGAAAA GCCGGATTAC CATCAACCCG GATACTTCGA 60
   AAAACCAGTT TAGCCTGC 78
(2) INFORMATION FOR SEQ ID NO: 148:
   (i) SEQUENCE CHARACTERISTICS:
      (A) LENGTH: 68 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 148:
   GCGCGCAATA ATACACGGCC GTATCTTCCG GGGTCACGCT GTTCAGTTGC AGGCTAAACT 60
   GGTTTTTC 68
(2) INFORMATION FOR SEQ ID NO: 149:
   (i) SEQUENCE CHARACTERISTICS:
      (A) LENGTH: 69 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 149:
   GGCTGAAGAC GTGGGCGTGT ATTATTGCCA GCAGCATTAT ACCACCCCGC CGACCTTTGG 60
   CCAGGGTAC 69
(2) INFORMATION FOR SEQ ID NO: 150:
   (i) SEQUENCE CHARACTERISTICS:
      (A) LENGTH: 71 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 150:
   GCGGAAAAAT AAACACGCTC GGAGCAGCCA CCGTACGTTT AATTTCAACT TTCGTACCCT 60
   GGCCAAAGGT C 71
(2) INFORMATION FOR SEQ ID NO: 151:
   (i) SEQUENCE CHARACTERISTICS:
      (A) LENGTH: 70 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 151:
   GAGCGTGTTT ATTTTTCCGC CGAGCGATGA ACAACTGAAA AGCGGCACGG CGAGCGTGGT 60
   GTGCCTGCTG 70
(2) INFORMATION FOR SEQ ID NO: 152:
   (i) SEQUENCE CHARACTERISTICS:
      (A) LENGTH: 71 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 152:
   CAGCGCGTTG TCTACTTTCC ACTGAACTTT CGCTTCACGC GGATAAAAGT TGTTCAGCAG 60
   GCACACCACG C 71
(2) INFORMATION FOR SEQ ID NO: 153:
   (i) SEQUENCE CHARACTERISTICS:
      (A) LENGTH: 69 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 153:
   GAAAGTAGAC AACGCGCTGC AAAGCGGCAA CAGCCAGGAA AGCGTGACCG AACAGGATAG 60
   CAAAGATAG 69
(2) INFORMATION FOR SEQ ID NO: 154:
   (i) SEQUENCE CHARACTERISTICS:
      (A) LENGTH: 74 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 154:
   GTTTTTCATA ATCCGCTTTG CTCAGGGTCA GGGTGCTGCT CAGAGAATAG GTGCTATCTT 60
   TGCTATCCTG TTCG 74
(2) INFORMATION FOR SEQ ID NO: 155:
   (i) SEQUENCE CHARACTERISTICS:
      (A) LENGTH: 71 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 155:
   GCAAAGCGGA TTATGAAAAA CATAAAGTGT ATGCGTGCGA AGTGACCCAT CAAGGTCTGA 60
   GCAGCCCGGT G 71
(2) INFORMATION FOR SEQ ID NO: 156:
   (i) SEQUENCE CHARACTERISTICS:
      (A) LENGTH: 57 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 156:
      GGCATGCTTA TCAGGCCTCG CCACGATTAA AAGATTTAGT CACCGGGCTG CTCAGAC 57
(2) INFORMATION FOR SEQ. ID NO: 157:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 157:
      GGCGTCTAGA GGCCAAGGCA CCCTGGTGAC GGTTAGCTCA GCGTCGAC 48
(2) INFORMATION FOR SEQ ID NO: 158:
   (i) SEQUENCE CHARACTERISTICS:
      (A) LENGTH: 63 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 158:
   GTGCTTTTGC TGCTCGGAGC CAGCGGAAAC ACGCTTGGAC CTTTGGTCGA CGCTGAGCTA 60
   ACC 63
(2) INFORMATION FOR SEQ ID NO: 159:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 159:
   CTCCGAGCAG CAAAAGCACC AGCGGCGGCA CGGCTGCCCT GGGCTGCCTG GTTAAAGATT 60
   ATTTCC 66
(2) INFORMATION FOR SEQ ID NO: 160:
   (i) SEQUENCE CHARACTERISTICS:
      (A) LENGTH: 65 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 160:
   CTGGTCAGCG CCCCGCTGTT CCAGCTCACG GTGACTGGTT CCGGGAAATA ATCTTTAACC 60
   AGGCA 65
(2) INFORMATION FOR SEQ ID NO: 161:
   (i) SEQUENCE CHARACTERISTICS:
      (A) LENGTH: 60 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 161:
      AGCGGGGCGC TGACCAGCGG CGTGCATACC TTTCCGGCGG TGCTGCAAAG CAGCGGCCTG 60
(2) INFORMATION FOR SEQ ID NO: 162:
   (i) SEQUENCE CHARACTERISTICS:
      (A) LENGTH: 65 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 162:
   GTGCCTAAGC TGCTGCTCGG CACGGTCACA ACGCTGCTCA GGCTATACAG GCCGCTGCTT 60
   TGCAG 65
(2) INFORMATION FOR SEQ ID NO: 163:
   (i) SEQUENCE CHARACTERISTICS:
      (A) LENGTH: 61 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 163:
   GAGCAGCAGC TTAGGCACTC AGACCTATAT TTGCAACGTG AACCATAAAC CGAGCAACAC        60
   C                                                                        61
(2) INFORMATION FOR SEQ ID NO: 164:
   (i) SEQUENCE CHARACTERISTICS:
      (A) LENGTH: 59 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 164:
      GCGCGAATTC GCTTTTCGGT TCCACTTTTT TATCCACTTT GGTGTTGCTC GGTTTATGG 59
(2) INFORMATION FOR SEQ ID NO: 165:
   (i) SEQUENCE CHARACTERISTICS:
      (A) LENGTH: 333 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene"
   (ix) FEATURE:
      (A) NAME/KEY: CDS
      (B) LOCATION:7..321
      (D) OTHER INFORMATION:/product= "C kappa"
   (xi) SEQUENCE DESCRIPTION: SEQ ID NO: 165:
   CGTACG GTG GCT GCT CCG AGC GTG TTT ATT TTT CCG CCG AGC GAT GAA          48
          Val Ala Ala Pro Ser Val Phe Ile Phe Pro Pro Ser Asp Glu
              125                 130                 135
   CAA CTG AAA AGC GGC ACG GCG AGC GTG GTG TGC CTG CTG AAC AAC TTT         96
   Gln Leu Lys Ser Gly Thr Ala Ser Val Val Cys Leu Leu Asn Asn Phe
           140                 145                 150
   TAT CCG CGT GAA GCG AAA GTT CAG TGG AAA GTA GAC AAC GCG CTG CAA        144
   Tyr Pro Arg Glu Ala Lys Val Gln Trp Lys Val Asp Asn Ala Leu Gln
       155                 160                 165
   AGC GGC AAC AGC CAG GAA AGC GTG ACC GAA CAG GAT AGC AAA GAT AGC        192
   Ser Gly Asn Ser Gln Glu Ser Val Thr Glu Gln Asp Ser Lys Asp Ser
   170                 175                 180                 185
   ACC TAT TCT CTG AGC AGC ACC CTG ACC CTG AGC AAA GCG GAT TAT GAA        240
   Thr Tyr Ser Leu Ser Ser Thr Leu Thr Leu Ser Lys Ala Asp Tyr Glu
                   190                 195                 200
   AAA CAT AAA GTG TAT GCG TGC GAA GTG ACC CAT CAA GGT CTG AGC AGC        288
   Lys His Lys Val Tyr Ala Cys Glu Val Thr His Gln Gly Leu Ser Ser
               205                 210                 215
   CCG GTG ACT AAA TCT TTT AAT CGT GGC GAG GCC TGATAAGCAT GC              333
   Pro Val Thr Lys Ser Phe Asn Arg Gly Glu Ala
           220                 225
(2) INFORMATION FOR SEQ ID NO: 166:
   (i) SEQUENCE CHARACTERISTICS:
      (A) LENGTH: 105 amino acids
      (B) TYPE: amino acid
      (D) TOPOLOGY: linear
   (ii) MOLECULE TYPE: protein
   (xi) SEQUENCE DESCRIPTION: SEQ ID NO: 166:
   Val Ala Ala Pro Ser Val Phe Ile Phe Pro Pro Ser Asp Glu Gln Leu
    1               5                  10                  15
   Lys Ser Gly Thr Ala Ser Val Val Cys Leu Leu Asn Asn Phe Tyr Pro
               20                  25                  30
   Arg Glu Ala Lys Val Gln Trp Lys Val Asp Asn Ala Leu Gln Ser Gly
                   35                  40                  45
   Asn Ser Gln Glu Ser Val Thr Glu Gln Asp Ser Lys Asp Ser Thr Tyr
       50                  55                  60
   Ser Leu Ser Ser Thr Leu Thr Leu Ser Lys Ala Asp Tyr Glu Lys His
   65                  70                  75                  80
   Lys Val Tyr Ala Cys Glu Val Thr His Gln Gly Leu Ser Ser Pro Val
                   85                  90                  95
   Thr Lys Ser Phe Asn Arg Gly Glu Ala
               100                 105
(2) INFORMATION FOR SEQ ID NO: 167:
   (i) SEQUENCE CHARACTERISTICS:
      (A) LENGTH: 327 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene"
   (ix) FEATURE:
      (A) NAME/KEY: CDS
      (B) LOCATION:6..317
      (D) OTHER INFORMATION:/product= "CH1"
   (xi) SEQUENCE DESCRIPTION: SEQ ID NO: 167:
   GCTCA GCG TCG ACC AAA GGT CCA AGC GTG TTT CCG CTG GCT CCG AGC         47
         Ala Ser Thr Lys Gly Pro Ser Val Phe Pro Leu Ala Pro Ser
                             110                 115
   AGC AAA AGC ACC AGC GGC GGC ACG GCT GCC CTG GGC TGC CTG GTT AAA       95
   Ser Lys Ser Thr Ser Gly Gly Thr Ala Ala Leu Gly Cys Leu Val Lys
   120                 125                 130                 135
   GAT TAT TTC CCG GAA CCA GTC ACC GTG AGC TGG AAC AGC GGG GCG CTG      143
   Asp Tyr Phe Pro Glu Pro Val Thr Val Ser Trp Asn Ser Gly Ala Leu
                   140                 145                 150
   ACC AGC GGC GTG CAT ACC TTT CCG GCG GTG CTG CAA AGC AGC GGC CTG      191
   Thr Ser Gly Val His Thr Phe Pro Ala Val Leu Gln Ser Ser Gly Leu
               155                 160                 165
   TAT AGC CTG AGC AGC GTT GTG ACC GTG CCG AGC AGC AGC TTA GGC ACT      239
   Tyr Ser Leu Ser Ser Val Val Thr Val Pro Ser Ser Ser Leu Gly Thr
           170                 175                 180
   CAG ACC TAT ATT TGC AAC GTG AAC CAT AAA CCG AGC AAC ACC AAA GTG      287
   Gln Thr Tyr Ile Cys Asn Val Asn His Lys Pro Ser Asn Thr Lys Val
       185                 190                 195
   GAT AAA AAA GTG GAA CCG AAA AGC GAA TTC TGATAAGCTT                   327
   Asp Lys Lys Val Glu Pro Lys Ser Glu Phe
                           200                 205
(2) INFORMATION FOR SEQ ID NO: 168:
   (i) SEQUENCE CHARACTERISTICS:
      (A) LENGTH: 104 amino acids
      (B) TYPE: amino acid
      (D) TOPOLOGY: linear
   (ii) MOLECULE TYPE: protein
   (xi) SEQUENCE DESCRIPTION: SEQ ID NO: 168:
   Ala Ser Thr Lys Gly Pro Ser Val Phe Pro Leu Ala Pro Ser Ser Lys
    1               5                  10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Glu Phe
               100
(2) INFORMATION FOR SEQ ID NO: 169:
   (i) SEQUENCE CHARACTERISTICS:
      (A) LENGTH: 408 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene"
   (ix) FEATURE:
      (A) NAME/KEY: CDS
      (B) LOCATION:85..396
      (D) OTHER INFORMATION:/product= "C lambda"
   (xi) SEQUENCE DESCRIPTION: SEQ ID NO: 169:
   GAAGACGAAG CGGATTATTA TTGCCAGCAG CATTATACCA CCCCGCCTGT GTTTGGCGGC      60
   GGCACGAAGT TAACCGTTCT TGGC CAG CCG AAA GCC GCA CCG AGT GTG ACG        111
                              Gln Pro Lys Ala Ala Pro Ser Val Thr
                              105                 110
   CTG TTT CCG CCG AGC AGC GAA GAA TTG CAG GCG AAC AAA GCG ACC CTG        159
   Leu Phe Pro Pro Ser Ser Glu Glu Leu Gln Ala Asn Lys Ala Thr Leu
       115                 120                 125
   GTG TGC CTG ATT AGC GAC TTT TAT CCG GGA GCC GTG ACA GTG GCC TGG        207
   Val Cys Leu Ile Ser Asp Phe Tyr Pro Gly Ala Val Thr Val Ala Trp
   130                 135                 140                 145
   AAG GCA GAT AGC AGC CCC GTC AAG GCG GGA GTG GAG ACC ACC ACA CCC        255
   Lys Ala Asp Ser Ser Pro Val Lys Ala Gly Val Glu Thr Thr Thr Pro
                   150                 155                 160
   TCC AAA CAA AGC AAC AAC AAG TAC GCG GCC AGC AGC TAT CTG AGC CTG        303
   Ser Lys Gln Ser Asn Asn Lys Tyr Ala Ala Ser Ser Tyr Leu Ser Leu
               165                 170                 175
   ACG CCT GAG CAG TGG AAG TCC CAC AGA AGC TAC AGC TGC CAG GTC ACG        351
   Thr Pro Glu Gln Trp Lys Ser His Arg Ser Tyr Ser Cys Gln Val Thr
           180                 185                 190
   CAT GAG GGG AGC ACC GTG GAA AAA ACC GTT GCG CCG ACT GAG GCC            396
   His Glu Gly Ser Thr Val Glu Lys Thr Val Ala Pro Thr Glu Ala
       195                 200                 205
   TGATAAGCAT GC                                                          408
(2) INFORMATION FOR SEQ ID NO: 170:
   (i) SEQUENCE CHARACTERISTICS:
      (A) LENGTH: 104 amino acids
      (B) TYPE: amino acid
      (D) TOPOLOGY: linear
   (ii) MOLECULE TYPE: protein
   (xi) SEQUENCE DESCRIPTION: SEQ ID NO: 170:
   Gln Pro Lys Ala Ala Pro Ser Val Thr Leu Phe Pro Pro Ser Ser Glu
    1               5                  10                  15
   Glu Leu Gln Ala Asn Lys Ala Thr Leu Val Cys Leu Ile Ser Asp Phe
               20                  25                  30
   Tyr Pro Gly Ala Val Thr Val Ala Trp Lys Ala Asp Ser Ser Pro Val
           35                  40                  45
   Lys Ala Gly Val Glu Thr Thr Thr Pro Ser Lys Gln Ser Asn Asn Lys
       50                  55                  60
   Tyr Ala Ala Ser Ser Tyr Leu Ser Leu Thr Pro Glu Gln Trp Lys Ser
   65                  70                  75                  80
   His Arg Ser Tyr Ser Cys Gln Val Thr His Glu Gly Ser Thr Val Glu
                   85                  90                  95
   Lys Thr Val Ala Pro Thr Glu Ala
               100
(2) INFORMATION FOR SEQ ID NO: 171:
   (i) SEQUENCE CHARACTERISTICS:
      (A) LENGTH: 78 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 171:
   GAAGACAAGC GGATTATTAT TGCCAGCAGC ATTATACCAC CCCGCCTGTG TTTGGCGGCG    60
   GCACGAAGTT AACCGTTC                                                  78
(2) INFORMATION FOR SEQ ID NO: 172:
   (i) SEQUENCE CHARACTERISTICS:
      (A) LENGTH: 80 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 172:
   CAATTCTTCG CTGCTCGGCG GAAACAGCGT CACACTCGGT GCGGCTTTCG GCTGGCCAAG    60
   AACGGTTAAC TTCGTGCCGC                                                80
(2) INFORMATION FOR SEQ ID NO: 173:
   (i) SEQUENCE CHARACTERISTICS:
      (A) LENGTH: 80 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 173:
   CGCCGAGCAG CGAAGAATTG CAGGCGAACA AAGCGACCCT GGTGTGCCTG ATTAGCGACT    60
   TTTATCCGGG AGCCGTGACA                                                80
(2) INFORMATION FOR SEQ ID NO: 174:
   (i) SEQUENCE CHARACTERISTICS:
      (A) LENGTH: 80 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 174:
   TGTTTGGAGG GTGTGGTGGT CTCCACTCCC GCCTTGACGG GGCTGCTATC TGCCTTCCAG 60
   GCCACTGTCA CGGCTCCCGG 80
(2) INFORMATION FOR SEQ ID NO: 175:
   (i) SEQUENCE CHARACTERISTICS:
      (A) LENGTH: 94 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 175:
   CCACACCCTC CAAACAAAGC AACAACAAGT ACGCGGCCAG CAGCTATCTG AGCCTGACGC 60
   CTGAGCAGTG GAAGTCCCAC AGAAGCTACA GCTG 94
(2) INFORMATION FOR SEQ ID NO: 176:
   (i) SEQUENCE CHARACTERISTICS:
      (A) LENGTH: 80 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 176:
   GCATGCTTAT CAGGCCTCAG TCGGCGCAAC GGTTTTTTCC ACGGTGCTCC CCTCATGCGT 60
   GACCTGGCAG CTGTAGCTTC 80
(2) INFORMATION FOR SEQ ID NO: 177:
   (i) SEQUENCE CHARACTERISTICS:
      (A) LENGTH: 843 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene"
   (ix) FEATURE:
      (A) NAME/KEY: CDS
      (B) LOCATION:1..843
      (D) OTHER INFORMATION:/product= "VH3-Vk2"
   (xi) SEQUENCE DESCRIPTION: SEQ ID NO: 177:
   ATG AAA CAA AGC ACT ATT GCA CTG GCA CTC TTA CCG TTG CTC TTC ACC     48
   Met Lys Gln Ser Thr Ile Ala Leu Ala Leu Leu Pro Leu Leu Phe Thr
   105                 110                 115                 120
   CCT GTT ACC AAA GCC GAC TAC AAA GAT GAA GTG CAA TTG GTG GAA AGC     96
   Pro Val Thr Lys Ala Asp Tyr Lys Asp Glu Val Gln Leu Val Glu Ser
                   125                 130                 135
   GGC GGC GGC CTG GTG CAA CCG GGC GGC AGC CTG CGT CTG AGC TGC GCG    144
   Gly Gly Gly Leu Val Gln Pro Gly Gly Ser Leu Arg Leu Ser Cys Ala
               140                 145                 150
   GCC TCC GGA TTT ACC TTT AGC AGC TAT GCG ATG AGC TGG GTG CGC CAA    192
   Ala Ser Gly Phe Thr Phe Ser Ser Tyr Ala Met Ser Trp Val Arg Gln
           155                 160                 165
   GCC CCT GGG AAG GGT CTC GAG TGG GTG AGC GCG ATT AGC GGT AGC GGC    240
   Ala Pro Gly Lys Gly Leu Glu Trp Val Ser Ala Ile Ser Gly Ser Gly
       170                 175                 180
   GGC AGC ACC TAT TAT GCG GAT AGC GTG AAA GGC CGT TTT ACC ATT TCA    288
   Gly Ser Thr Tyr Tyr Ala Asp Ser Val Lys Gly Arg Phe Thr Ile Ser
   185                 190                 195                 200
   CGT GAT AAT TCG AAA AAC ACC CTG TAT CTG CAA ATG AAC AGC CTG CGT    336
   Arg Asp Asn Ser Lys Asn Thr Leu Tyr Leu Gln Met Asn Ser Leu Arg
                   205                 210                 215
   GCG GAA GAT ACG GCC GTG TAT TAT TGC GCG CGT TGG GGC GGC GAT GGC    384
   Ala Glu Asp Thr Ala Val Tyr Tyr Cys Ala Arg Trp Gly Gly Asp Gly
               220                 225                 230
   TTT TAT GCG ATG GAT TAT TGG GGC CAA GGC ACC CTG GTG ACG GTT AGC    432
   Phe Tyr Ala Met Asp Tyr Trp Gly Gln Gly Thr Leu Val Thr Val Ser
           235                 240                 245
   TCA GCG GGT GGC GGT TCT GGC GGC GGT GGG AGC GGT GGC GGT GGT TCT    480
   Ser Ala Gly Gly Gly Ser Gly Gly Gly Gly Ser Gly Gly Gly Gly Ser
       250                 255                 260
   GGC GGT GGT GGT TCC GAT ATC GTG ATG ACC CAG AGC CCA CTG AGC CTG    528
   Gly Gly Gly Gly Ser Asp Ile Val Met Thr Gln Ser Pro Leu Ser Leu
   265                 270                 275                 280
   CCA GTG ACT CCG GGC GAG CCT GCG AGC ATT AGC TGC AGA AGC AGC CAA        576
   Pro Val Thr Pro Gly Glu Pro Ala Ser Ile Ser Cys Arg Ser Ser Gln
                   285                 290                 295
   AGC CTG CTG CAT AGC AAC GGC TAT AAC TAT CTG GAT TGG TAC CTT CAA        624
   Ser Leu Leu His Ser Asn Gly Tyr Asn Tyr Leu Asp Trp Tyr Leu Gln
               300                 305                 310
   AAA CCA GGT CAA AGC CCG CAG CTA TTA ATT TAT CTG GGC AGC AAC CGT        672
   Lys Pro Gly Gln Ser Pro Gln Leu Leu Ile Tyr Leu Gly Ser Asn Arg
           315                 320                 325
   GCC AGT GGG GTC CCG GAT CGT TTT AGC GGC TCT GGA TCC GGC ACC GAT        720
   Ala Ser Gly Val Pro Asp Arg Phe Ser Gly Ser Gly Ser Gly Thr Asp
       330                 335                 340
   TTT ACC CTG AAA ATT AGC CGT GTG GAA GCT GAA GAC GTG GGC GTG TAT        768
   Phe Thr Leu Lys Ile Ser Arg Val Glu Ala Glu Asp Val Gly Val Tyr
   345                 350                 355                 360
   TAT TGC CAG CAG CAT TAT ACC ACC CCG CCG ACC TTT GGC CAG GGT ACG        816
   Tyr Cys Gln Gln His Tyr Thr Thr Pro Pro Thr Phe Gly Gln Gly Thr
                   365                 370                 375
   AAA GTT GAA ATT AAA CGT ACG GAA TTC                                    843
   Lys Val Glu Ile Lys Arg Thr Glu Phe
               380                 385
(2) INFORMATION FOR SEQ ID NO: 178:
   (i) SEQUENCE CHARACTERISTICS:
      (A) LENGTH: 281 amino acids
      (B) TYPE: amino acid
      (D) TOPOLOGY: linear
   (ii) MOLECULE TYPE: protein
   (xi) SEQUENCE DESCRIPTION: SEQ ID NO: 178:
   Met Lys Gln Ser Thr Ile Ala Leu Ala Leu Leu Pro Leu Leu Phe Thr
    1               5                  10                  15
   Pro Val Thr Lys Ala Asp Tyr Lys Asp Glu Val Gln Leu Val Glu Ser
                   20                  25                  30
   Gly Gly Gly Leu Val Gln Pro Gly Gly Ser Leu Arg Leu Ser Cys Ala
               35                  40                  45
   Ala Ser Gly Phe Thr Phe Ser Ser Tyr Ala Met Ser Trp Val Arg Gln
           50                  55                  60
   Ala Pro Gly Lys Gly Leu Glu Trp Val Ser Ala Ile Ser Gly Ser Gly
   65                  70                  75                  80
   Gly Ser Thr Tyr Tyr Ala Asp Ser Val Lys Gly Arg Phe Thr Ile Ser
                   85                  90                  95
   Arg Asp Asn Ser Lys Asn Thr Leu Tyr Leu Gln Met Asn Ser Leu Arg
               100                 105                 110
   Ala Glu Asp Thr Ala Val Tyr Tyr Cys Ala Arg Trp Gly Gly Asp Gly
           115                 120                 125
   Phe Tyr Ala Met Asp Tyr Trp Gly Gln Gly Thr Leu Val Thr Val Ser
       130                 135                 140
   Ser Ala Gly Gly Gly Ser Gly Gly Gly Gly Ser Gly Gly Gly Gly Ser
   145                 150                 155                 160
   Gly Gly Gly Gly Ser Asp Ile Val Met Thr Gln Ser Pro Leu Ser Leu
                   165                 170                 175
   Pro Val Thr Pro Gly Glu Pro Ala Ser Ile Ser Cys Arg Ser Ser Gln
               180                 185                 190
   Ser Leu Leu His Ser Asn Gly Tyr Asn Tyr Leu Asp Trp Tyr Leu Gln
           195                 200                 205
   Lys Pro Gly Gln Ser Pro Gln Leu Leu Ile Tyr Leu Gly Ser Asn Arg
       210                 215                 220
   Ala Ser Gly Val Pro Asp Arg Phe Ser Gly Ser Gly Ser Gly Thr Asp
   225                 230                 235                 240
   Phe Thr Leu Lys Ile Ser Arg Val Glu Ala Glu Asp Val Gly Val Tyr
                   245                 250                 255
   Tyr Cys Gln Gln His Tyr Thr Thr Pro Pro Thr Phe Gly Gln Gly Thr
               260                 265                 270
   Lys Val Glu Ile Lys Arg Thr Glu Phe
           275                 280
(2) INFORMATION FOR SEQ ID NO: 179:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 179:
   Cys Ala Arg Trp Gly Gly Asp Gly Phe Tyr Ala Met Asp Tyr Trp
   1               5                   10                  15
(2) INFORMATION FOR SEQ ID NO: 180:
   (i) SEQUENCE CHARACTERISTICS:
      (A) LENGTH: 12 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 180:
   Cys Ala Arg Phe Gly Lys Met Asn Tyr Asp Tyr Trp
   1 5 10
(2) INFORMATION FOR SEQ ID NO: 181:
   (i) SEQUENCE CHARACTERISTICS:
      (A) LENGTH: 12 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 181:
   Cys Ala Arg His Arg Thr Glu Trp His Asp Tyr Trp
   1 5 10
(2) INFORMATION FOR SEQ ID NO: 182:
   (i) SEQUENCE CHARACTERISTICS:
      (A) LENGTH: 12 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 182:
   Cys Ala Arg Val Arg Glu Leu Tyr His Asp Tyr Trp
   1 5 10
(2) INFORMATION FOR SEQ ID NO: 183:
   (i) SEQUENCE CHARACTERISTICS:
      (A) LENGTH: 12 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 183:
   Cys Ala Arg Lys Phe Leu Lys Ala Arg Asp Tyr Trp
   1 5 10
(2) INFORMATION FOR SEQ ID NO: 184:
   (i) SEQUENCE CHARACTERISTICS:
      (A) LENGTH: 12 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 184:
   Cys Ala Arg Trp Asn Thr Thr Gly Tyr Asp Tyr Trp
   1               5                   10
(2) INFORMATION FOR SEQ ID NO: 185:
   (i) SEQUENCE CHARACTERISTICS:
      (A) LENGTH: 12 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 185:
   Cys Ala Arg Ile Asn Glu Ala Gln Pro Asp Tyr Trp
   1               5                   10
(2) INFORMATION FOR SEQ ID NO: 186:
   (i) SEQUENCE CHARACTERISTICS:
      (A) LENGTH: 11 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 186:
   Cys Ala Arg Thr Ala Ile Thr Arg Asp Tyr Trp
   1               5                   10
(2) INFORMATION FOR SEQ ID NO: 187:
   (i) SEQUENCE CHARACTERISTICS:
      (A) LENGTH: 12 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 187:
   Cys Ala Arg Trp Tyr Asn Arg Asn Ser Asp Tyr Trp
   1               5                   10
(2) INFORMATION FOR SEQ ID NO: 188:
   (i) SEQUENCE CHARACTERISTICS:
      (A) LENGTH: 12 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 188:
   Cys Ala Arg Ser Val Gly Asp Ser Lys Asp Tyr Trp
   1               5                   10
(2) INFORMATION FOR SEQ ID NO: 189:
   (i) SEQUENCE CHARACTERISTICS:
      (A) LENGTH: 12 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 189:
   Cys Ala Arg Ser Lys Thr Phe Ala Ala Asp Tyr Trp
   1               5                   10
(2) INFORMATION FOR SEQ ID NO: 190:
   (i) SEQUENCE CHARACTERISTICS:
      (A) LENGTH: 12 amino acids
      (B) TYPE: amino acid
      (C) STRANDEDNESS:
      (D) TOPOLOGY: linear
   (ii) MOLECULE TYPE: protein
   (v) FRAGMENT TYPE: internal
(2) INFORMATION FOR SEQ ID NO: 191:
   (i) SEQUENCE CHARACTERISTICS:
      (A) LENGTH: 12 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 191:
   Cys Ala Arg Met Gln Ser Glu Trp Met Asp Tyr Trp
   1 5 10
(2) INFORMATION FOR SEQ ID NO: 192:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 192:
   Cys Ala Arg Tyr Phe Val His Phe Leu Tyr Thr Met Val Met Asp Val
   1 5 10 15
   Trp
(2) INFORMATION FOR SEQ ID NO: 193:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 193:
   Cys Ala Arg Met Ala Leu Arg Ala Ser Gly Lys Tyr Ile Met Asp Val
   1 5 10 15
   Trp
(2) INFORMATION FOR SEQ ID NO: 194:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 194:
   Cys Ala Arg Lys Asn Gln Met Val Phe His Ala Arg Lys Phe Asp Val
   Trp
(2) INFORMATION FOR SEQ ID NO: 195:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 195:
   Cys Ala Arg Thr Gln Ser Phe Trp Glu Gln Gln Lys Val Met Asp Tyr
   1               5                   10                  15
   Trp
(2) INFORMATION FOR SEQ ID NO: 196:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 196:
   Cys Ala Arg Tyr Pro Tyr Arg Ser Asn Phe Phe Met Pro Met Asp Val
   1               5                   10                  15
   Trp
(2) INFORMATION FOR SEQ ID NO: 197:
   (i) SEQUENCE CHARACTERISTICS:
      (A) LENGTH: 16 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (ix) FEATURE:
      (A) NAME/KEY: Modified-site
      (B) LOCATION:3..4
      (D) OTHER INFORMATION:/product= "see Figure 10C"
         /label= R*G
         /note= "* denotes codon with one-base deletion, causes shift of reading fr..."
   (xi) SEQUENCE DESCRIPTION: SEQ ID NO: 197:
   Cys Ala Arg Gly Ser Gly Ser Glu His Trp Ser Ile Phe Asp Val Trp
   1               5                   10                  15
(2) INFORMATION FOR SEQ ID NO: 198:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 198:
   Cys Ala Arg Arg Asn Pro Trp Asn Val Asn Tyr Leu His Phe Asp Val
   1               5                   10                  15
   Trp
(2) INFORMATION FOR SEQ ID NO: 199:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 199:
   Cys Ala Arg Met Lys Pro Met Leu Asn Arg Asp Gly Thr Met Asp Val
   1 5 10 15
   Trp
(2) INFORMATION FOR SEQ ID NO: 200:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 200:
   Cys Ala Arg Lys Gly Ser Glu Phe Leu Glu Thr Asp Val Met Asp Tyr
   1 5 10 15
   Trp
(2) INFORMATION FOR SEQ ID NO: 201:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 201:
   Cys Ala Arg Ser Trp Thr Asn Asp Lys Pro Asn Phe Ile Met Asp Val
   1 5 10 15
   Trp
(2) INFORMATION FOR SEQ ID NO: 202:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 202:
   Cys Ala Arg Tyr Ala Gly Thr Thr Phe Lys Gln Gly Pro Met Asp Tyr
   1 5 10 15
   Trp
(2) INFORMATION FOR SEQ ID NO: 203:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 203:
   Cys Ala Arg Lys Arg Met Met Gln Asn Pro Arg Phe Arg Phe Asp Val
   1 5 10 15
   Trp
(2) INFORMATION FOR SEQ ID NO: 204:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 204:
   Cys Ala Arg Arg Ser Lys Gln Lys Arg Lys Met Arg Arg Phe Asp Val
   1 5 10 15
   Trp
(2) INFORMATION FOR SEQ ID NO: 205:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 205:
   Cys Ala Arg Arg Asn Gly Lys Arg His Leu Arg His Arg Phe Asp Val
   1               5                   10                  15
   Trp
(2) INFORMATION FOR SEQ ID NO: 206:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 206:
   Cys Ala Arg Arg Lys Met Arg Lys Arg Ile Lys Arg Arg Phe Asp Val
   1               5                   10                  15
   Trp
(2) INFORMATION FOR SEQ ID NO: 207:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 207:
   Cys Ala Arg Tyr Arg Lys Ile Met Lys Trp Lys Asn Ser Phe Asp Val
   1               5                   10                  15
   Trp
(2) INFORMATION FOR SEQ ID NO: 208:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 208:
   Cys Ala Arg Leu Ile Glu Val His Pro Ser Phe Asp Gln Met Asp Val
   1 5 10 15
   Trp
(2) INFORMATION FOR SEQ ID NO: 209:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 209:
   Cys Ala Arg Arg Lys Pro Met Phe Leu Lys Lys Ala Val Phe Asp Val
   1 5 10 15
   Trp
(2) INFORMATION FOR SEQ ID NO: 210:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 210:
   Cys Ala Arg Arg Lys Phe His Arg Tyr Ser Thr Val Lys Phe Asp Tyr
   1 5 10 15
   Trp
(2) INFORMATION FOR SEQ ID NO: 211:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 211:
   Cys Ala Arg Arg Lys Thr Met Arg Ser Arg Val Lys Tyr Phe Asp Tyr
   1               5                   10                  15
   Trp
(2) INFORMATION FOR SEQ ID NO: 212:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 212:
   Cys Ala Arg Lys Lys Arg Ser Trp Arg Arg Met Asp Arg Phe Asp Val
   1               5                   10                  15
   Trp
(2) INFORMATION FOR SEQ ID NO: 213:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 213:
   Cys Ala Arg Arg Asn Pro Arg Arg Gly Arg Met Asn Arg Phe Asp Val
   1               5                   10                  15
   Trp
(2) INFORMATION FOR SEQ ID NO: 214:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 214:
   Cys Ala Arg Lys Gly Lys Lys Lys Phe Ala Arg Pro Arg Phe Asp Val
   1               5                   10                  15
   Trp
(2) INFORMATION FOR SEQ ID NO: 215:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 215:
   Cys Ala Arg Arg Met Val His Lys Gly Lys Arg Lys Ile Phe Asp Val
   1               5                   10                  15
   Trp
(2) INFORMATION FOR SEQ ID NO: 216:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 216:
   Cys Ala Arg Arg Lys His Ile Thr Tyr Pro Arg Lys Gln Phe Asp Val
   1               5                   10                  15
   Trp
(2) INFORMATION FOR SEQ ID NO: 217:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 217:
   Cys Ala Arg Arg Trp Thr Lys Arg Arg Ser Phe Ala Arg Phe Asp Val
   1               5                   10                  15
   Trp
(2) INFORMATION FOR SEQ ID NO: 218:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 218:
   Cys Ala Arg Lys Lys Leu Lys Gln Tyr Thr Phe Ser Arg Phe Asp Tyr
   1               5                   10                  15
   Trp
(2) INFORMATION FOR SEQ ID NO: 219:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 219:
   Cys Ala Arg Thr Arg Pro Trp Gln Ala Thr Arg Lys Gly Phe Asp Val
   1               5                   10                  15
   Trp
(2) INFORMATION FOR SEQ ID NO: 220:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 220:
   Cys Ala Arg Asn Gln Trp Glu Phe Lys Asn Arg Arg Lys Met Asp Tyr
   1 5 10 15
   Trp
(2) INFORMATION FOR SEQ ID NO: 221:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 221:
   Cys Ala Arg Lys Arg Trp Met Trp Pro Ile Gly Lys Arg Phe Asp Tyr
   1 5 10 15
   Trp
(2) INFORMATION FOR SEQ ID NO: 222:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 222:
   Cys Ala Arg Tyr Ser Leu Trp Arg Leu Asp Glu Tyr Phe Phe Asp Tyr
   1 5 10 15
   Trp
(2) INFORMATION FOR SEQ ID NO: 223:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 223:
   Cys Ala Arg Val Pro Trp Gly Asp Phe Trp Ser Trp His Met Asp Val
   1               5                   10                  15
   Trp
(2) INFORMATION FOR SEQ ID NO: 224:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 224:
   Cys Ala Arg Asn Gly Leu Glu Pro Arg His Arg Lys Met Met Asp Tyr
   1               5                   10                  15
   Trp
(2) INFORMATION FOR SEQ ID NO: 225:
   (i) SEQUENCE CHARACTERISTICS:
      (A) LENGTH: 12 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 225:
   Cys Ala Arg Ile Met Lys Ala Pro Pro Asp Tyr Trp
   1               5                   10
(2) INFORMATION FOR SEQ ID NO: 226:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 226:
   Cys Ala Arg Arg Lys Thr Trp His Trp Phe Tyr Lys Arg Met Asp Tyr
   1               5                   10                  15
   Trp
(2) INFORMATION FOR SEQ ID NO: 227:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 227:
   Cys Ala Arg Trp Lys Asp Met Trp Ser Gln Val Tyr Val Met Asp Tyr
   1               5                   10                  15
   Trp
(2) INFORMATION FOR SEQ ID NO: 228:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 228:
   Cys Ala Arg Asn Lys Gln Gln Met Arg Phe Arg Arg Phe Met Asp Tyr
   1               5                   10                  15
   Trp
(2) INFORMATION FOR SEQ ID NO: 229:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 229:
   Cys Ala Arg Asn Met Leu Ala Leu Ser Arg Gly Lys Glu Met Asp Val
   1 5 10 15
   Trp
(2) INFORMATION FOR SEQ ID NO: 230:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 230:
   Cys Ala Arg Asn Met Arg Leu Met Arg Met Arg Lys Asn Phe Asp Val
   1 5 10 15
   Trp
(2) INFORMATION FOR SEQ ID NO: 231:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 231:
   Cys Ala Arg Tyr Ile Lys Gln Ala Lys Arg Lys Leu Ala Phe Asp Tyr
   1 5 10 15
   Trp
(2) INFORMATION FOR SEQ ID NO: 232:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 232:
   Cys Ala Arg Tyr Asn Arg His Ala Trp Gln Lys Met Gln Phe Asp Tyr
   1 5 10 15
   Trp
(2) INFORMATION FOR SEQ ID NO: 233:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 233:
   Cys Ala Arg Tyr Val Lys Tyr Ala Arg Asn Lys Met Gln Phe Asp Tyr
   1 5 10 15
   Trp
(2) INFORMATION FOR SEQ ID NO: 234:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 234:
   Cys Ala Arg Tyr Lys Arg Gly Ala Trp Met Lys Thr Met Phe Asp Val
   1 5 10 15
   Trp
(2) INFORMATION FOR SEQ ID NO: 235:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 235:
   Cys Ala Arg Arg Lys Pro Leu Arg Arg Ile Met Lys Trp Phe Asp Tyr
   1 5 10 15
   Trp
(2) INFORMATION FOR SEQ ID NO: 236:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 236:
   Cys Ala Arg Tyr Arg Lys Arg Ala Ser Arg Gln Met Gln Phe Asp Tyr
   1 5 10 15
   Trp
(2) INFORMATION FOR SEQ ID NO: 237:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 237:
   Cys Ala Arg Gln Arg Tyr Arg Ser Lys Ile Lys Gly His Phe Asp Val
   1 5 10 15
   Trp
(2) INFORMATION FOR SEQ ID NO: 238:
   (i) SEQUENCE CHARACTERISTICS:
      (A) LENGTH: 16 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 238:
   Cys Ala Arg Trp Arg Asp Phe Asn Ser Tyr Asp Pro Met Asp Tyr Trp
   1 5 10 15
(2) INFORMATION FOR SEQ ID NO: 239:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 239:
   Cys Ala Arg Met Ala Asp Leu Asp Asn Tyr Trp Val Gln Phe Asp Tyr
   1 5 10 15
   Trp
(2) INFORMATION FOR SEQ ID NO: 240:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 240:
   Cys Ala Arg Leu Gln Ala Tyr Leu Lys Pro His His Trp Met Asp Tyr
   1 5 10 15
   Trp
(2) INFORMATION FOR SEQ ID NO: 241:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 241:
   Cys Ala Arg Arg Leu Ile Glu Gln Ala Arg Asp His Val Met Asp Tyr
   1               5                   10                  15
   Trp
(2) INFORMATION FOR SEQ ID NO: 242:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 242:
   Cys Ala Arg Ser Trp His Asn Ser Gln Phe Thr Gln Ser Phe Asp Val
   1               5                   10                  15
   Trp
(2) INFORMATION FOR SEQ ID NO: 243:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 243:
   Cys Ala Arg Val Asp His Phe Gln Thr Glu Asn Glu Trp Met Asp Tyr
   1               5                   10                  15
   Trp
(2) INFORMATION FOR SEQ ID NO: 244:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 244:
   Cys Ala Arg Asp Trp Pro Thr Leu Ile Phe Trp Tyr Trp Phe Asp Tyr
   1 5 10 15
   Trp
(2) INFORMATION FOR SEQ ID NO: 245:
   (i) SEQUENCE CHARACTERISTICS:
      (A) LENGTH: 12 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 245:
   Cys Ala Arg Gly Phe Gly Phe Thr Glu Asp Tyr Trp
   1 5 10
(2) INFORMATION FOR SEQ ID NO: 246:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 246:
   Cys Ala Arg Gln Phe Asp Glu Asp Ser Phe Val Arg Arg Phe Asp Val
   1 5 10 15
   Trp
(2) INFORMATION FOR SEQ ID NO: 247:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 247:
   Cys Ala Arg Ile Leu Lys Glu Ser Ser Lys Ser Arg Gln Met Asp Val
   1 5 10 15
   Trp
(2) INFORMATION FOR SEQ ID NO: 248:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 248:
   Cys Ala Arg Glu Gln Asp Glu Tyr Gly Ala Ile Arg Ile Met Asp Tyr
   1 5 10 15
   Trp
(2) INFORMATION FOR SEQ ID NO: 249:
   (i) SEQUENCE CHARACTERISTICS:
      (A) LENGTH: 18 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 249:
   Cys Ala Arg Asn His Phe Glu Ala Ser Trp Pro Arg Arg Gln Met Asp
   1 5 10 15
   Val Trp
(2) INFORMATION FOR SEQ ID NO: 250:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 250:
   Cys Ala Arg Glu Asn Glu Trp Val Asp Met Ile Leu Asp Met Asp Tyr
   1 5 10 15
   Trp
(2) INFORMATION FOR SEQ ID NO: 251:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO: 251:
   Cys Ala Arg Gln Tyr Ser Glu Thr Arg Trp Val Arg Lys Phe Asp Tyr
   1 5 10 15
   Trp
(2) INFORMATION FOR SEQ ID NO: 252:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 252:
   Cys Ala Arg Gln Phe Lys Glu Ser Lys Thr Arg Arg Lys Phe Asp Val
   1 5 10 15
   Trp
(2) INFORMATION FOR SEQ ID NO: 253:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 253:
   Cys Ala Arg Lys Lys Thr Gln Tyr Val His Asp Trp Arg Met Asp Val
   1               5                   10                  15
   Trp
(2) INFORMATION FOR SEQ ID NO: 254:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 254:
   Cys Ala Arg Arg Trp Arg Glu Thr Lys Ser Lys Arg Phe Phe Asp Val
   1               5                   10                  15
   Trp
(2) INFORMATION FOR SEQ ID NO: 255:
   (i) SEQUENCE CHARACTERISTICS:
      (A) LENGTH: 12 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 255:
   Cys Ala Arg Asp Tyr Ile Met Glu Phe Asp Tyr Trp
   1               5                   10
(2) INFORMATION FOR SEQ ID NO: 256:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 256:
   Cys Ala Arg Gln Phe Glu Glu Thr Lys Gln Arg Arg Leu Met Asp Tyr
   1               5                   10                  15
   Trp
(2) INFORMATION FOR SEQ ID NO: 257:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 257:
   Cys Ala Arg Asp Gln Gly Phe Tyr Ala Ile Asp Tyr Val Met Asp Tyr
   1               5                   10                  15
   Trp
(2) INFORMATION FOR SEQ ID NO: 258:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 258:
   Cys Ala Arg Val Phe Thr Tyr Met Tyr Asn Tyr Phe Arg Phe Asp Val
   1               5                   10                  15
   Trp
(2) INFORMATION FOR SEQ ID NO: 259:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 259:
   Cys Ala Arg Val Phe Phe Glu Gln Met Glu Val Val Arg Met Asp Val
   1 5 10 15
   Trp
(2) INFORMATION FOR SEQ ID NO: 260:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 260:
   Cys Ala Arg Glu Lys Glu Tyr Arg Leu Ser Trp Ser Gln Met Asp Tyr
   1 5 10 15
   Trp
(2) INFORMATION FOR SEQ ID NO: 261:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 261:
   Cys Ala Arg Tyr Pro Ser Arg Trp Ala Pro Asn Trp Tyr Met Asp Tyr
   1 5 10 15
   Trp
(2) INFORMATION FOR SEQ ID NO: 262:
   (i) SEQUENCE CHARACTERISTICS:
      (A) LENGTH: 17 amino acids
      (B) TYPE: amino acid
      (C) STRANDEDNESS:
      (D) TOPOLOGY: linear
   (ii) MOLECULE TYPE: protein
   (v) FRAGMENT TYPE: internal
   (xi) SEQUENCE DESCRIPTION: SEQ ID NO: 262:
   Cys Ala Arg Asp Gly Gly Phe Lys Pro Leu Thr His Phe Phe Asp Val
   1               5                   10                  15
   Trp
(2) INFORMATION FOR SEQ ID NO: 263:
   (i) SEQUENCE CHARACTERISTICS:
      (A) LENGTH: 143 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic DNA cassette"
   (xi) SEQUENCE DESCRIPTION: SEQ ID NO: 263:
   ACATGTAAGC TTCCCCCCCC CCTTAATTAA CCCCCCCCCC TGTACACCCC CCCCCCGCTA      60
   GCCCCCCCCC CCAGATCTCC CCCCCCCCGA CGTCCCCCCT CTAGACCCCC CCCCCGCATG     120
   CCCCCCCCCC CGAATTCGAC GTC                                             143
(2) INFORMATION FOR SEQ ID NO: 264:
   (i) SEQUENCE CHARACTERISTICS:
      (A) LENGTH: 1947 base pairs
      (B) TYPE: nucleic acid
      (C) STRANDEDNESS: double
      (D) TOPOLOGY: circular
   (ii) MOLECULE TYPE: other nucleic acid
      (A) DESCRIPTION: /desc = "synthetic vector"
   (ix) FEATURE:
      (A) NAME/KEY: CDS
      (B) LOCATION:132..989
      (D) OTHER INFORMATION:/product= "Amp resistance"
   (xi) SEQUENCE DESCRIPTION: SEQ ID NO: 264:
   CAGGTGGCAC TTTTCGGGGA AATGTGCGCG GAACCCCTAT TTGTTTATTT TTCTAAATAC    60
   ATTCAAATAT GTATCCGCTC ATGAGACAAT AACCCTGATA AATGCTTCAA TAATATTGAA   120
   AAAGGAAGAG T ATG AGT ATT CAA CAT TTC CGT GTC GCC CTT ATT CCC TTT     170
                Met Ser Ile Gln His Phe Arg Val Ala Leu Ile Pro Phe
                              285                 290
   TTT GCG GCA TTT TGC CTT CCT GTT TTT GCT CAC CCA GAA ACG CTG GTG     218
   Phe Ala Ala Phe Cys Leu Pro Val Phe Ala His Pro Glu Thr Leu Val
   295                 300                 305                 310
   AAA GTA AAA GAT GCT GAA GAT CAG TTG GGT GCA CGA GTG GGT TAC ATC     266
   Lys Val Lys Asp Ala Glu Asp Gln Leu Gly Ala Arg Val Gly Tyr Ile
                   315                 320                 325
   GAA CTG GAT CTC AAC AGC GGT AAG ATC CTT GAG AGT TTT CGC CCC GAA     314
   Glu Leu Asp Leu Asn Ser Gly Lys Ile Leu Glu Ser Phe Arg Pro Glu
               330                 335                 340
   GAA CGT TTT CCA ATG ATG AGC ACT TTT AAA GTT CTG CTA TGT GGC GCG     362
   Glu Arg Phe Pro Met Met Ser Thr Phe Lys Val Leu Leu Cys Gly Ala
           345                 350                 355
   GTA TTA TCC CGT ATT GAC GCC GGG CAA GAG CAA CTC GGT CGC CGC ATA     410
   Val Leu Ser Arg Ile Asp Ala Gly Gln Glu Gln Leu Gly Arg Arg Ile
       360                 365                 370
   CAC TAT TCT CAG AAT GAC TTG GTT GAG TAC TCA CCA GTC ACA GAA AAG      458
   His Tyr Ser Gln Asn Asp Leu Val Glu Tyr Ser Pro Val Thr Glu Lys
   375                 380                 385                 390
   CAT CTT ACG GAT GGC ATG ACA GTA AGA GAA TTA TGC AGT GCT GCC ATA      506
   His Leu Thr Asp Gly Met Thr Val Arg Glu Leu Cys Ser Ala Ala Ile
               395                 400                 405
   ACC ATG AGT GAT AAC ACT GCG GCC AAC TTA CTT CTG ACA ACG ATC GGA      554
   Thr Met Ser Asp Asn Thr Ala Ala Asn Leu Leu Leu Thr Thr Ile Gly
           410                 415                 420
   GGA CCG AAG GAG CTA ACC GCT TTT TTG CAC AAC ATG GGG GAT CAT GTA      602
   Gly Pro Lys Glu Leu Thr Ala Phe Leu His Asn Met Gly Asp His Val
       425                 430                 435
   ACT CGC CTT GAT CGT TGG GAA CCG GAG CTG AAT GAA GCC ATA CCA AAC      650
   Thr Arg Leu Asp Arg Trp Glu Pro Glu Leu Asn Glu Ala Ile Pro Asn
   440                 445                 450
   GAC GAG CGT GAC ACC ACG ATG CCT GTA GCA ATG GCA ACA ACG TTG CGC      698
   Asp Glu Arg Asp Thr Thr Met Pro Val Ala Met Ala Thr Thr Leu Arg
   455                 460                 465                 470
   AAA CTA TTA ACT GGC GAA CTA CTT ACT CTA GCT TCC CGG CAA CAA TTA      746
   Lys Leu Leu Thr Gly Glu Leu Leu Thr Leu Ala Ser Arg Gln Gln Leu
               475                 480                 485
   ATA GAC TGG ATG GAG GCG GAT AAA GTT GCA GGA CCA CTT CTG CGC TCG      794
   Ile Asp Trp Met Glu Ala Asp Lys Val Ala Gly Pro Leu Leu Arg Ser
           490                 495                 500
   GCC CTT CCG GCT GGC TGG TTT ATT GCT GAT AAA TCT GGA GCC GGT GAG      842
   Ala Leu Pro Ala Gly Trp Phe Ile Ala Asp Lys Ser Gly Ala Gly Glu
       505                 510                 515
   CGT GGG TCT CGC GGT ATC ATT GCA GCA CTG GGG CCA GAT GGT AAG CCC      890
   Arg Gly Ser Arg Gly Ile Ile Ala Ala Leu Gly Pro Asp Gly Lys Pro
   520                 525                 530
   TCC CGT ATC GTA GTT ATC TAC ACG ACG GGG AGT CAG GCA ACT ATG GAT      938
   Ser Arg Ile Val Val Ile Tyr Thr Thr Gly Ser Gln Ala Thr Met Asp
   535                 540                 545                 550
   GAA CGA AAT AGA CAG ATC GCT GAG ATA GGT GCC TCA CTG ATT AAG CAT      986
   Glu Arg Asn Arg Gln Ile Ala Glu Ile Gly Ala Ser Leu Ile Lys His
               555                 560                 565
   TGG TAACTGTCAG ACCAAGTTTA CTCATATATA CTTTAGATTG ATTTAAAACT          1039
   Trp
   TCATTTTTAA TTTAAAAGGA TCTAGGTGAA GATCCTTTTT GATAATCTCA TGACCAAAAT   1099
   CCCTTAACGT GAGTTTTCGT TCCACTGAGC GTCAGACCCC GTAGAAAAGA TCAAAGGATC   1159
   TTCTTGAGAT CCTTTTTTTC TGCGCGTAAT CTGCTGCTTG CAAACAAAAA AACCACCGCT   1219
   ACCAGCGGTG GTTTGTTTGC CGGATCAAGA GCTACCAACT CTTTTTCCGA AGGTAACTGG   1279
   CTTCAGCAGA GCGCAGATAC CAAATACTGT CCTTCTAGTG TAGCCGTAGT TAGGCCACCA    1339
   CTTCAAGAAC TCTGTAGCAC CGCCTACATA CCTCGCTCTG CTAATCCTGT TACCAGTGGC    1399
   TGCTGCCAGT GGCGATAAGT CGTGTCTTAC CGGGTTGGAC TCAAGACGAT AGTTACCGGA    1459
   TAAGGCGCAG CGGTCGGGCT GAACGGGGGG TTCGTGCACA CAGCCCAGCT TGGAGCGAAC    1519
   GACCTACACC GAACTGAGAT ACCTACAGCG TGAGCTATGA GAAAGCGCCA CGCTTCCCGA    1579
   AGGGAGAAAG GCGGACAGGT ATCCGGTAAG CGGCAGGGTC GGAACAGGAG AGCGCACGAG    1639
   GGAGCTTCCA GGGGGAAACG CCTGGTATCT TTATAGTCCT GTCGGGTTTC GCCACCTCTG    1699
   ACTTGAGCGT CGATTTTTGT GATGCTCGTC AGGGGGGCGG AGCCTATGGA AAAACGCCAG    1759
   CAACGCGGCC TTTTTACGGT TCCTGGCCTT TTGCTGGCCT TTTGCTCACA TGTAAGCTTC    1819
   CCCCCCCCCT TAATTAACCC CCCCCCCTGT ACACCCCCCC CCCGCTAGCC CCCCCCCCCA    1879
   GATCTCCCCC CCCCCGACGT CCCCCCTCTA GACCCCCCCC CCGCATGCCC CCCCCCCCGA    1939
   ATTCACGT                                                             1947
(2) INFORMATION FOR SEQ ID NO: 265:
   (i) SEQUENCE CHARACTERISTICS:
      (A) LENGTH: 286 amino acids
      (B) TYPE: amino acid
      (D) TOPOLOGY: linear
   (ii) MOLECULE TYPE: protein
   (xi) SEQUENCE DESCRIPTION: SEQ ID NO: 265:
   Met Ser Ile Gln His Phe Arg Val Ala Leu Ile Pro Phe Phe Ala Ala
   1               5                   10                  15
   Phe Cys Leu Pro Val Phe Ala His Pro Glu Thr Leu Val Lys Val Lys
               20                  25                  30
   Asp Ala Glu Asp Gln Leu Gly Ala Arg Val Gly Tyr Ile Glu Leu Asp
           35                  40                  45
   Leu Asn Ser Gly Lys Ile Leu Glu Ser Phe Arg Pro Glu Glu Arg Phe
       50                  55                  60
   Pro Met Met Ser Thr Phe Lys Val Leu Leu Cys Gly Ala Val Leu Ser
   65                  70                  75                  80
   Arg Ile Asp Ala Gly Gln Glu Gln Leu Gly Arg Arg Ile His Tyr Ser
                   85                  90                  95
   Gln Asn Asp Leu Val Glu Tyr Ser Pro Val Thr Glu Lys His Leu Thr
               100                 105                 110
   Asp Gly Met Thr Val Arg Glu Leu Cys Ser Ala Ala Ile Thr Met Ser
           115                 120                 125
   Asp Asn Thr Ala Ala Asn Leu Leu Leu Thr Thr Ile Gly Gly Pro Lys
       130                 135                 140
   Glu Leu Thr Ala Phe Leu His Asn Met Gly Asp His Val Thr Arg Leu
   145                 150                 155                 160
   Asp Arg Trp Glu Pro Glu Leu Asn Glu Ala Ile Pro Asn Asp Glu Arg
                   165                 170                 175
   Asp Thr Thr Met Pro Val Ala Met Ala Thr Thr Leu Arg Lys Leu Leu
               180                 185                 190
   Thr Gly Glu Leu Leu Thr Leu Ala Ser Arg Gln Gln Leu Ile Asp Trp
           195                 200                 205
   Met Glu Ala Asp Lys Val Ala Gly Pro Leu Leu Arg Ser Ala Leu Pro
       210                 215                 220
   Ala Gly Trp Phe Ile Ala Asp Lys Ser Gly Ala Gly Glu Arg Gly Ser
   225                 230                 235                 240
   Arg Gly Ile Ile Ala Ala Leu Gly Pro Asp Gly Lys Pro Ser Arg Ile
                   245                 250                 255
   Val Val Ile Tyr Thr Thr Gly Ser Gln Ala Thr Met Asp Glu Arg Asn
               260                 265                 270
   Arg Gln Ile Ala Glu Ile Gly Ala Ser Leu Ile Lys His Trp
           275                 280                 285
(2) INFORMATION FOR SEQ ID NO: 266:
   (i) SEQUENCE CHARACTERISTICS:
      (A) LENGTH: 142 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic DNA cassette"
   (xi) SEQUENCE DESCRIPTION: SEQ ID NO: 266:
   GACGTCTTAA TGTGAGTTAG CTCACTCATT AGGCACCCCA GGCTTTACAC TTTATGCTTC    60
   CGGCTCGTAT GTTGTGTGGA ATTGTGAGCG GATAACAATT TCACACAGGA AACAGCTATG   120
   ACCATGATTA CGAATTTCTA GA                                            142
(2) INFORMATION FOR SEQ ID NO: 267:
   (i) SEQUENCE CHARACTERISTICS:
      (A) LENGTH: 520 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene cassette"
   (ix) FEATURE:
      (A) NAME/KEY: CDS
      (B) LOCATION:1..510
      (D) OTHER INFORMATION:/product= "gIIIp ss with myc-tag, amber codon"
   (xi) SEQUENCE DESCRIPTION: SEQ ID NO: 267:
(2) INFORMATION FOR SEQ ID NO: 268:
   (i) SEQUENCE CHARACTERISTICS:
      (A) LENGTH: 170 amino acids
      (B) TYPE: amino acid
      (D) TOPOLOGY: linear
   (ii) MOLECULE TYPE: protein
   (xi) SEQUENCE DESCRIPTION: SEQ ID NO: 268:
   Glu Phe Glu Gln Lys Leu Ile Ser Glu Glu Asp Leu  *  Gly Gly Gly
    1               5                  10                  15
   Ser Gly Ser Gly Asp Phe Asp Tyr Glu Lys Met Ala Asn Ala Asn Lys
               20                  25                  30
   Gly Ala Met Thr Glu Asn Ala Asp Glu Asn Ala Leu Gln Ser Asp Ala
           35                  40                  45
   Lys Gly Lys Leu Asp Ser Val Ala Thr Asp Tyr Gly Ala Ala Ile Asp
       50                  55                  60
   Gly Phe Ile Gly Asp Val Ser Gly Leu Ala Asn Gly Asn Gly Ala Thr
   65                  70                  75                  80
   Gly Asp Phe Ala Gly Ser Asn Ser Gln Met Ala Gln Val Gly Asp Gly
                   85                  90                  95
   Asp Asn Ser Pro Leu Met Asn Asn Phe Arg Gln Tyr Leu Pro Ser Leu
               100                 105                 110
   Pro Gln Ser Val Glu Cys Arg Pro Phe Val Phe Gly Ala Gly Lys Pro
           115                 120                 125
   Tyr Glu Phe Ser Ile Asp Cys Asp Lys Ile Asn Leu Phe Arg Gly Val
       130                 135                 140
   Phe Ala Phe Leu Leu Tyr Val Ala Thr Phe Met Tyr Val Phe Ser Thr
   145                 150                 155                 160
   Phe Ala Asn Ile Leu Arg Asn Lys Glu Ser
                   165                 170
(2) INFORMATION FOR SEQ ID NO: 269:
   (i) SEQUENCE CHARACTERISTICS:
      (A) LENGTH: 123 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic DNA cassette"
   (xi) SEQUENCE DESCRIPTION: SEQ ID NO: 269:
   GGGGGGGGGG AAGCTTGACC TGTGAAGTGA AAAATGGCGC AGATTGTGCG ACATTTTTTT     60
   TGTCTGCCGT TTAATTAAAG GGGGGGGGGG GCCGGCCTGG GGGGGGGTGT ACAGGGGGGG    120
   GGG 123
(2) INFORMATION FOR SEQ ID NO: 270:
   (i) SEQUENCE CHARACTERISTICS:
      (A) LENGTH: 470 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic DNA cassette"
   (xi) SEQUENCE DESCRIPTION: SEQ ID NO: 270:
   GCTAGCACGC GCCCTGTAGC GGCGCATTAA GCGCGGCGGG TGTGGTGGTT ACGCGCAGCG 60
   TGACCGCTAC ACTTGCCAGC GCCCTAGCGC CCGCTCCTTT CGCTTTCTTC CCTTCCTTTC 120
   TCGCCACGTT CGCCGGCTTT CCCCGTCAAG CTCTAAATCG GGGCATCCCT TTAGGGTTCC 180
   GATTTAGTGC TTTACGGCAC CTCGACCCCA AAAAACTTGA TTAGGGTGAT GGTTCTCGTA 240
   GTGGGCCATC GCCCTGATAG ACGGTTTTTC GCCCTTTGAC GTTGGAGTCC ACGTTCTTTA 300
   ATAGTGGACT CTTGTTCCAA ACTGGAACAA CACTCAACCC TATCTCGGTC TATTCTTTTG 360
   ATTTATAAGG GATTTTGCCG ATTTCGGCCT ATTGGTTAAA AAATGAGCTG ATTTAACAAA 420
   AATTTAACGC GAATTTTAAC AAAATATTAA CGTTTACAAT TTCATGTACA 470
(2) INFORMATION FOR SEQ ID NO: 271:
   (i) SEQUENCE CHARACTERISTICS:
      (A) LENGTH: 733 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic DNA cassette"
   (xi) SEQUENCE DESCRIPTION: SEQ ID NO: 271:
   AGATCTGACC AAAATCCCTT AACGTGAGTT TTCGTTCCAC TGAGCGTCAG ACCCCGTAGA    60
   AAAGATCAAA GGATCTTCTT GAGATCCTTT TTTTCTGCGC GTAATCTGCT GCTTGCAAAC   120
   AAAAAAACCA CCGCTACCAG CGGTGGTTTG TTTGCCGGAT CAAGAGCTAC CAACTCTTTT   180
   TCCGAAGGTA ACTGGCTACA GCAGAGCGCA GATACCAAAT ACTGTTCTTC TAGTGTAGCC   240
   GTAGTTAGGC CACCACTTCA AGAACTCTGT AGCACCGCCT ACATACCTCG CTCTGCTAAT   300
   CCTGTTACCA GTGGCTGCTG CCAGTGGCGA TAAGTCGTGT CTTACCGGGT TGGACTCAAG   360
   ACGATAGTTA CCGGATAAGG CGCAGCGGTC GGGCTGAACG GGGGGTTCGT GCACACAGCC   420
   CAGCTTGGAG CGAACGACCT ACACCGAACT GAGATACCTA CAGCGTGAGC TATGAGAAAG   480
   CGCCACGCTT CCCGAAGGGA GAAAGGCGGA CAGGTATCCG GTAAGCGGCA GGGTCGGAAC   540
   AGGAGAGCGC ACGAGGGAGC TTCCAGGGGG AAACGCCTGG TATCTTTATA GTCCTGTCGG   600
   GTTTCGCCAC CTCTGACTTG AGCGTCGATT TTTGTGATGC TCGTCAGGGG GGCGGAGCCT   660
   ATGGAAAAAC GCCAGCAACG CGGCCTTTTT ACGGTTCCTG GCCTTTTGCT GGCCTTTTGC   720
   TCACATGGCT AGC                                                      733
(2) INFORMATION FOR SEQ ID NO: 272:
   (i) SEQUENCE CHARACTERISTICS:
      (A) LENGTH: 813 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene cassette"
   (ix) FEATURE:
      (A) NAME/KEY: CDS
      (B) LOCATION:102..758
      (D) OTHER INFORMATION:/product= "cat resistance"
   (xi) SEQUENCE DESCRIPTION: SEQ ID NO: 272:
   GGGACGTCGG GTGAGGTTCC AACTTTCACC ATAATGAAAT AAGATCACTA CCGGGCGTAT      60
   TTTTTGAGTT ATCGAGATTT TCAGGAGCTA AGGAAGCTAA A ATG GAG AAA AAA         113
                                                 Met Glu Lys Lys
   ATC ACT GGA TAT ACC ACC GTT GAT ATA TCC CAA TGG CAT CGT AAA GAA       161
   Ile Thr Gly Tyr Thr Thr Val Asp Ile Ser Gln Trp His Arg Lys Glu
   175                 180                 185                 190
   CAT TTT GAG GCA TTT CAG TCA GTT GCT CAA TGT ACC TAT AAC CAG ACC       209
   His Phe Glu Ala Phe Gln Ser Val Ala Gln Cys Thr Tyr Asn Gln Thr
                   195                 200                 205
   GTT CAG CTG GAT ATT ACG GCC TTT TTA AAG ACC GTA AAG AAA AAT AAG       257
   Val Gln Leu Asp Ile Thr Ala Phe Leu Lys Thr Val Lys Lys Asn Lys
               210                 215                 220
   CAC AAG TTT TAT CCG GCC TTT ATT CAC ATT CTT GCC CGC CTG ATG AAT       305
   His Lys Phe Tyr Pro Ala Phe Ile His Ile Leu Ala Arg Leu Met Asn
           225                 230                 235
   GCT CAC CCG GAG TTC CGT ATG GCA ATG AAA GAC GGT GAG CTG GTG ATA       353
   Ala His Pro Glu Phe Arg Met Ala Met Lys Asp Gly Glu Leu Val Ile
       240                 245                 250
   TGG GAT AGT GTT CAC CCT TGT TAC ACC GTT TTC CAT GAG CAA ACT GAA        401
   Trp Asp Ser Val His Pro Cys Tyr Thr Val Phe His Glu Gln Thr Glu
   255                 260                 265                 270
   ACG TTT TCA TCG CTC TGG AGT GAA TAC CAC GAC GAT TTC CGG CAG TTT        449
   Thr Phe Ser Ser Leu Trp Ser Glu Tyr His Asp Asp Phe Arg Gln Phe
                   275                 280                 285
   CTA CAC ATA TAT TCG CAA GAT GTG GCG TGT TAC GGT GAA AAC CTG GCC        497
   Leu His Ile Tyr Ser Gln Asp Val Ala Cys Tyr Gly Glu Asn Leu Ala
               290                 295                 300
   TAT TTC CCT AAA GGG TTT ATT GAG AAT ATG TTT TTC GTC TCA GCC AAT        545
   Tyr Phe Pro Lys Gly Phe Ile Glu Asn Met Phe Phe Val Ser Ala Asn
           305                 310                 315
   CCC TGG GTG AGT TTC ACC AGT TTT GAT TTA AAC GTA GCC AAT ATG GAC        593
   Pro Trp Val Ser Phe Thr Ser Phe Asp Leu Asn Val Ala Asn Met Asp
       320                 325                 330
   AAC TTC TTC GCC CCC GTT TTC ACT ATG GGC AAA TAT TAT ACG CAA GGC        641
   Asn Phe Phe Ala Pro Val Phe Thr Met Gly Lys Tyr Tyr Thr Gln Gly
   335                 340                 345                 350
   GAC AAG GTG CTG ATG CCG CTG GCG ATT CAG GTT CAT CAT GCC GTT TGT        689
   Asp Lys Val Leu Met Pro Leu Ala Ile Gln Val His His Ala Val Cys
                   355                 360                 365
   GAT GGC TTC CAT GTC GGC AGA ATG CTT AAT GAA TTA CAA CAG TAC TGC        737
   Asp Gly Phe His Val Gly Arg Met Leu Asn Glu Leu Gln Gln Tyr Cys
               370                 375                 380
   GAT GAG TGG CAG GGC GGG GCG TAATTTTTTT AAGGCAGTTA TTGGGTGCCC           788
   Asp Glu Trp Gln Gly Gly Ala
           385
   TTAAACGCCT GGTGCTAGAT CTTCC                                           813
(2) INFORMATION FOR SEQ ID NO: 273:
   (i) SEQUENCE CHARACTERISTICS:
      (A) LENGTH: 219 amino acids
      (B) TYPE: amino acid
      (D) TOPOLOGY: linear
   (ii) MOLECULE TYPE: protein
   (xi) SEQUENCE DESCRIPTION: SEQ ID NO: 273:
(2) INFORMATION FOR SEQ ID NO: 274:
   (i) SEQUENCE CHARACTERISTICS:
      (A) LENGTH: 2755 base pairs
      (B) TYPE: nucleic acid
      (C) STRANDEDNESS: double
      (D) TOPOLOGY: circular
   (ii) MOLECULE TYPE: other nucleic acid
      (A) DESCRIPTION: /desc = "synthetic vector"
   (ix) FEATURE:
      (A) NAME/KEY: CDS
      (B) LOCATION:3..509
      (D) OTHER INFORMATION:/product= "gIIIp ss, myc tag, amber codon"
   (ix) FEATURE:
      (A) NAME/KEY: CDS
      (B) LOCATION:complement (1853..2509)
      (D) OTHER INFORMATION:/product= "cat resistance"
   (xi) SEQUENCE DESCRIPTION: SEQ ID NO: 274:
   AA TTC GAG CAG AAG CTG ATC TCT GAG GAG GAT CTG TAG GGT GGT GGC      47
      Phe Glu Gln Lys Leu Ile Ser Glu Glu Asp Leu  *  Gly Gly Gly
      220                225                 230
   TCT GGT TCC GGT GAT TTT GAT TAT GAA AAG ATG GCA AAC GCT AAT AAG        95
   Ser Gly Ser Gly Asp Phe Asp Tyr Glu Lys Met Ala Asn Ala Asn Lys
   235                 240                 245                 250
   GGG GCT ATG ACC GAA AAT GCC GAT GAA AAC GCG CTA CAG TCT GAC GCT       143
   Gly Ala Met Thr Glu Asn Ala Asp Glu Asn Ala Leu Gln Ser Asp Ala
                   255                 260                 265
   AAA GGC AAA CTT GAT TCT GTC GCT ACT GAT TAC GGT GCT GCT ATC GAT       191
   Lys Gly Lys Leu Asp Ser Val Ala Thr Asp Tyr Gly Ala Ala Ile Asp
               270                 275                 280
   GGT TTC ATT GGT GAC GTT TCC GGC CTT GCT AAT GGT AAT GGT GCT ACT       239
   Gly Phe Ile Gly Asp Val Ser Gly Leu Ala Asn Gly Asn Gly Ala Thr
           285                 290                 295
   GGT GAT TTT GCT GGC TCT AAT TCC CAA ATG GCT CAA GTC GGT GAC GGT       287
   Gly Asp Phe Ala Gly Ser Asn Ser Gln Met Ala Gln Val Gly Asp Gly
       300                 305                 310
   GAT AAT TCA CCT TTA ATG AAT AAT TTC CGT CAA TAT TTA CCT TCC CTC       335
   Asp Asn Ser Pro Leu Met Asn Asn Phe Arg Gln Tyr Leu Pro Ser Leu
   315                 320                 325                 330
   CCT CAA TCG GTT GAA TGT CGC CCT TTT GTC TTT GGC GCT GGT AAA CCA       383
   Pro Gln Ser Val Glu Cys Arg Pro Phe Val Phe Gly Ala Gly Lys Pro
                   335                 340                 345
   TAT GAA TTT TCT ATT GAT TGT GAC AAA ATA AAC TTA TTC CGT GGT GTC       431
   Tyr Glu Phe Ser Ile Asp Cys Asp Lys Ile Asn Leu Phe Arg Gly Val
               350                 355                 360
   TTT GCG TTT CTT TTA TAT GTT GCC ACC TTT ATG TAT GTA TTT TCT ACG       479
   Phe Ala Phe Leu Leu Tyr Val Ala Thr Phe Met Tyr Val Phe Ser Thr
           365                 370                 375
   TTT GCT AAC ATA CTG CGT AAT AAG GAG TCT TGATAAGCTT GACCTGTGAA         529
   Phe Ala Asn Ile Leu Arg Asn Lys Glu Ser
       380                 385
   GTGAAAAATG GCGCAGATTG TGCGACATTT TTTTTGTCTG CCGTTTAATT AAAGGGGGGG      589
   GGGGGCCGGC CTGGGGGGGG GTGTACATGA AATTGTAAAC GTTAATATTT TGTTAAAATT      649
   CGCGTTAAAT TTTTGTTAAA TCAGCTCATT TTTTAACCAA TAGGCCGAAA TCGGCAAAAT      709
   CCCTTATAAA TCAAAAGAAT AGACCGAGAT AGGGTTGAGT GTTGTTCCAG TTTGGAACAA      769
   GAGTCCACTA TTAAAGAACG TGGACTCCAA CGTCAAAGGG CGAAAAACCG TCTATCAGGG      829
   CGATGGCCCA CTACGAGAAC CATCACCCTA ATCAAGTTTT TTGGGGTCGA GGTGCCGTAA      889
   AGCACTAAAT CGGAACCCTA AAGGGAGCCC CCGATTTAGA GCTTGACGGG GAAAGCCGGC      949
   GAACGTGGCG AGAAAGGAAG GGAAGAAAGC GAAAGGAGCG GGCGCTAGGG CGCTGGCAAG     1009
   TGTAGCGGTC ACGCTGCGCG TAACCACCAC ACCCGCCGCG CTTAATGCGC CGCTACAGGG     1069
   CGCGTGCTAG CCATGTGAGC AAAAGGCCAG CAAAAGGCCA GGAACCGTAA AAAGGCCGCG     1129
   TTGCTGGCGT TTTTCCATAG GCTCCGCCCC CCTGACGAGC ATCACAAAAA TCGACGCTCA 1189
   AGTCAGAGGT GGCGAAACCC GACAGGACTA TAAAGATACC AGGCGTTTCC CCCTGGAAGC 1249
   TCCCTCGTGC GCTCTCCTGT TCCGACCCTG CCGCTTACCG GATACCTGTC CGCCTTTCTC 1309
   CCTTCGGGAA GCGTGGCGCT TTCTCATAGC TCACGCTGTA GGTATCTCAG TTCGGTGTAG 1369
   GTCGTTCGCT CCAAGCTGGG CTGTGTGCAC GAACCCCCCG TTCAGCCCGA CCGCTGCGCC 1429
   TTATCCGGTA ACTATCGTCT TGAGTCCAAC CCGGTAAGAC ACGACTTATC GCCACTGGCA 1489
   GCAGCCACTG GTAACAGGAT TAGCAGAGCG AGGTATGTAG GCGGTGCTAC AGAGTTCTTG 1549
   AAGTGGTGGC CTAACTACGG CTACACTAGA AGAACAGTAT TTGGTATCTG CGCTCTGCTG 1609
   TAGCCAGTTA CCTTCGGAAA AAGAGTTGGT AGCTCTTGAT CCGGCAAACA AACCACCGCT 1669
   GGTAGCGGTG GTTTTTTTGT TTGCAAGCAG CAGATTACGC GCAGAAAAAA AGGATCTCAA 1729
   GAAGATCCTT TGATCTTTTC TACGGGGTCT GACGCTCAGT GGAACGAAAA CTCACGTTAA 1789
   GGGATTTTGG TCAGATCTAG CACCAGGCGT TTAAGGGCAC CAATAACTGC CTTAAAAAAA 1849
   TTACGCCCCG CCCTGCCACT CATCGCAGTA CTGTTGTAAT TCATTAAGCA TTCTGCCGAC 1909
   ATGGAAGCCA TCACAAACGG CATGATGAAC CTGAATCGCC AGCGGCATCA GCACCTTGTC 1969
   GCCTTGCGTA TAATATTTGC CCATAGTGAA AACGGGGGCG AAGAAGTTGT CCATATTGGC 2029
   TACGTTTAAA TCAAAACTGG TGAAACTCAC CCAGGGATTG GCTGAGACGA AAAACATATT 2089
   CTCAATAAAC CCTTTAGGGA AATAGGCCAG GTTTTCACCG TAACACGCCA CATCTTGCGA 2149
   ATATATGTGT AGAAACTGCC GGAAATCGTC GTGGTATTCA CTCCAGAGCG ATGAAAACGT 2209
   TTCAGTTTGC TCATGGAAAA CGGTGTAACA AGGGTGAACA CTATCCCATA TCACCAGCTC 2269
   ACCGTCTTTC ATTGCCATAC GGAACTCCGG GTGAGCATTC ATCAGGCGGG CAAGAATGTG 2329
   AATAAAGGCC GGATAAAACT TGTGCTTATT TTTCTTTACG GTCTTTAAAA AGGCCGTAAT 2389
   ATCCAGCTGA ACGGTCTGGT TATAGGTACA TTGAGCAACT GACTGAAATG CCTCAAAATG 2449
   TTCTTTACGA TGCCATTGGG ATATATCAAC GGTGGTATAT CCAGTGATTT TTTTCTCCAT 2509
   TTTAGCTTCC TTAGCTCCTG AAAATCTCGA TAACTCAAAA AATACGCCCG GTAGTGATCT 2569
   TATTTCATTA TGGTGAAAGT TGGAACCTCA CCCGACGTCT AATGTGAGTT AGCTCACTCA 2629
   TTAGGCACCC CAGGCTTTAC ACTTTATGCT TCCGGCTCGT ATGTTGTGTG GAATTGTGAG 2689
   CGGATAACAA TTTCACACAG GAAACAGCTA TGACCATGAT TACGAATTTC TAGAGCATGC 2749
   GGGGGG 2755
(2) INFORMATION FOR SEQ ID NO: 275:
   (i) SEQUENCE CHARACTERISTICS:
      (A) LENGTH: 169 amino acids
      (B) TYPE: amino acid
      (D) TOPOLOGY: linear
   (ii) MOLECULE TYPE: protein
   (xi) SEQUENCE DESCRIPTION: SEQ ID NO: 275:
   Phe Glu Gln Lys Leu Ile Ser Glu Glu Asp Leu  *  Gly Gly Gly Ser
   Gly Ser Gly Asp Phe Asp Tyr Glu Lys Met Ala Asn Ala Asn Lys Gly
               20                  25                  30
   Ala Met Thr Glu Asn Ala Asp Glu Asn Ala Leu Gln Ser Asp Ala Lys
           35                  40                  45
   Gly Lys Leu Asp Ser Val Ala Thr Asp Tyr Gly Ala Ala Ile Asp Gly
       50                  55                  60
   Phe Ile Gly Asp Val Ser Gly Leu Ala Asn Gly Asn Gly Ala Thr Gly
   65                  70                  75                  80
   Asp Phe Ala Gly Ser Asn Ser Gln Met Ala Gln Val Gly Asp Gly Asp
                   85                  90                  95
   Asn Ser Pro Leu Met Asn Asn Phe Arg Gln Tyr Leu Pro Ser Leu Pro
               100                 105                 110
   Gln Ser Val Glu Cys Arg Pro Phe Val Phe Gly Ala Gly Lys Pro Tyr
           115                 120                 125
   Glu Phe Ser Ile Asp Cys Asp Lys Ile Asn Leu Phe Arg Gly Val Phe
       130                 135                 140
   Ala Phe Leu Leu Tyr Val Ala Thr Phe Met Tyr Val Phe Ser Thr Phe
   145                 150                 155                 160
   Ala Asn Ile Leu Arg Asn Lys Glu Ser
                   165
(2) INFORMATION FOR SEQ ID NO: 276:
   (i) SEQUENCE CHARACTERISTICS:
      (A) LENGTH: 219 amino acids
      (B) TYPE: amino acid
      (D) TOPOLOGY: linear
   (ii) MOLECULE TYPE: protein
   (xi) SEQUENCE DESCRIPTION: SEQ ID NO: 276:
   Met Glu Lys Lys Ile Thr Gly Tyr Thr Thr Val Asp Ile Ser Gln Trp
   1               5                   10                  15
   His Arg Lys Glu His Phe Glu Ala Phe Gln Ser Val Ala Gln Cys Thr
               20                  25                  30
   Tyr Asn Gln Thr Val Gln Leu Asp Ile Thr Ala Phe Leu Lys Thr Val
           35                  40                  45
   Lys Lys Asn Lys His Lys Phe Tyr Pro Ala Phe Ile His Ile Leu Ala
   50 55 60
   Arg Leu Met Asn Ala His Pro Glu Phe Arg Met Ala Met Lys Asp Gly
   65 70 75 80
   Glu Leu Val Ile Trp Asp Ser Val His Pro Cys Tyr Thr Val Phe His
   85 90 95
   Glu Gln Thr Glu Thr Phe Ser Ser Leu Trp Ser Glu Tyr His Asp Asp
   100 105 110
   Phe Arg Gln Phe Leu His Ile Tyr Ser Gln Asp Val Ala Cys Tyr Gly
   115 120 125
   Glu Asn Leu Ala Tyr Phe Pro Lys Gly Phe Ile Glu Asn Met Phe Phe
   130 135 140
   Val Ser Ala Asn Pro Trp Val Ser Phe Thr Ser Phe Asp Leu Asn Val
   145 150 155 160
   Ala Asn Met Asp Asn Phe Phe Ala Pro Val Phe Thr Met Gly Lys Tyr
   165 170 175
   Tyr Thr Gln Gly Asp Lys Val Leu Met Pro Leu Ala Ile Gln Val His
   180 185 190
   His Ala Val Cys Asp Gly Phe His Val Gly Arg Met Leu Asn Glu Leu
   195 200 205
   Gln Gln Tyr Cys Asp Glu Trp Gln Gly Gly Ala
   210 215
(2) INFORMATION FOR SEQ ID NO: 277:
   (i) SEQUENCE CHARACTERISTICS:
      (A) LENGTH: 173 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic DNA cassette"
   (xi) SEQUENCE DESCRIPTION: SEQ ID NO: 277:
   GACGTCTTAA TGTGAGTTAG CTCACTCATT AGGCACCCCA GGCTTTACAC TTTATGCTTC 60
   CGGCTCGTAT GTTGTGTGGA ATTGTGAGCG GATAACAATT TCACACAGGA AACAGCTATG 120
   ACCATGTCTA GAATAACTTC GTATAATGTA CGCTATACGA AGTTATCGCA TGC 173
(2) INFORMATION FOR SEQ ID NO: 278:
   (i) SEQUENCE CHARACTERISTICS:
      (A) LENGTH: 47 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic DNA cassette"
   (xi) SEQUENCE DESCRIPTION: SEQ ID NO: 278:
      AGATCTCATA ACTTCGTATA ATGTATGCTA TACGAAGTTA TGACGTC 47
(2) INFORMATION FOR SEQ ID NO: 279:
   (i) SEQUENCE CHARACTERISTICS:
      (A) LENGTH: 1255 base pairs
      (C) STRANDEDNESS: double
      (D) TOPOLOGY: linear
   (ii) MOLECULE TYPE: other nucleic acid
      (A) DESCRIPTION: /desc = "synthetic gene cassette"
   (ix) FEATURE:
      (A) NAME/KEY: CDS
      (B) LOCATION:1..1245
      (D) OTHER INFORMATION:/product= "gIIIp, GGGGS linker"
   (xi) SEQUENCE DESCRIPTION: SEQ ID NO: 279:
   GAA TTC GGT GGT GGT GGA TCT GCG TGC GCT GAA ACG GTT GAA AGT TGT       48
   Glu Phe Gly Gly Gly Gly Ser Ala Cys Ala Glu Thr Val Glu Ser Cys
   220                 225                 230                 235
   TTA GCA AAA TCC CAT ACA GAA AAT TCA TTT ACT AAC GTC TGG AAA GAC       96
   Leu Ala Lys Ser His Thr Glu Asn Ser Phe Thr Asn Val Trp Lys Asp
                   240                 245                 250
   GAC AAA ACT TTA GAT CGT TAC GCT AAC TAT GAG GGC TGT CTG TGG AAT      144
   Asp Lys Thr Leu Asp Arg Tyr Ala Asn Tyr Glu Gly Cys Leu Trp Asn
               255                 260                 265
   GCT ACA GGC GTT GTA GTT TGT ACT GGT GAC GAA ACT CAG TGT TAC GGT      192
   Ala Thr Gly Val Val Val Cys Thr Gly Asp Glu Thr Gln Cys Tyr Gly
           270                 275                 280
   ACA TGG GTT CCT ATT GGG CTT GCT ATC CCT GAA AAT GAG GGT GGT GGC      240
   Thr Trp Val Pro Ile Gly Leu Ala Ile Pro Glu Asn Glu Gly Gly Gly
       285                 290                 295
   TCT GAG GGT GGC GGT TCT GAG GGT GGC GGT TCT GAG GGT GGC GGT ACT      288
   Ser Glu Gly Gly Gly Ser Glu Gly Gly Gly Ser Glu Gly Gly Gly Thr
   300                 305                 310                 315
   AAA CCT CCT GAG TAC GGT GAT ACA CCT ATT CCG GGC TAT ACT TAT ATC      336
   Lys Pro Pro Glu Tyr Gly Asp Thr Pro Ile Pro Gly Tyr Thr Tyr Ile
                   320                 325                 330
   AAC CCT CTC GAC GGC ACT TAT CCG CCT GGT ACT GAG CAA AAC CCC GCT     384
   Asn Pro Leu Asp Gly Thr Tyr Pro Pro Gly Thr Glu Gln Asn Pro Ala
               335                 340                 345
   AAT CCT AAT CCT TCT CTT GAG GAG TCT CAG CCT CTT AAT ACT TTC ATG     432
   Asn Pro Asn Pro Ser Leu Glu Glu Ser Gln Pro Leu Asn Thr Phe Met
           350                 355                 360
   TTT CAG AAT AAT AGG TTC CGA AAT AGG CAG GGG GCA TTA ACT GTT TAT     480
   Phe Gln Asn Asn Arg Phe Arg Asn Arg Gln Gly Ala Leu Thr Val Tyr
       365                 370                 375
   ACG GGC ACT GTT ACT CAA GGC ACT GAC CCC GTT AAA ACT TAT TAC CAG     528
   Thr Gly Thr Val Thr Gln Gly Thr Asp Pro Val Lys Thr Tyr Tyr Gln
   380                 385                 390                 395
   TAC ACT CCT GTA TCA TCA AAA GCC ATG TAT GAC GCT TAC TGG AAC GGT     576
   Tyr Thr Pro Val Ser Ser Lys Ala Met Tyr Asp Ala Tyr Trp Asn Gly
                   400                 405                 410
   AAA TTC AGA GAC TGC GCT TTC CAT TCT GGC TTT AAT GAG GAT TTA TTT     624
   Lys Phe Arg Asp Cys Ala Phe His Ser Gly Phe Asn Glu Asp Leu Phe
               415                 420                 425
   GTT TGT GAA TAT CAA GGC CAA TCG TCT GAC CTG CCT CAA CCT CCT GTC     672
   Val Cys Glu Tyr Gln Gly Gln Ser Ser Asp Leu Pro Gln Pro Pro Val
           430                 435                 440
   AAT GCT GGC GGC GGC TCT GGT GGT GGT TCT GGT GGC GGC TCT GAG GGT     720
   Asn Ala Gly Gly Gly Ser Gly Gly Gly Ser Gly Gly Gly Ser Glu Gly
       445                 450                 455
   GGT GGC TCT GAG GGT GGC GGT TCT GAG GGT GGC GGC TCT GAG GGA GGC     768
   Gly Gly Ser Glu Gly Gly Gly Ser Glu Gly Gly Gly Ser Glu Gly Gly
   460                 465                 470                 475
   GGT TCC GGT GGT GGC TCT GGT TCC GGT GAT TTT GAT TAT GAA AAG ATG     816
   Gly Ser Gly Gly Gly Ser Gly Ser Gly Asp Phe Asp Tyr Glu Lys Met
                   480                 485                 490
   GCA AAC GCT AAT AAG GGG GCT ATG ACC GAA AAT GCC GAT GAA AAC GCG     864
   Ala Asn Ala Asn Lys Gly Ala Met Thr Glu Asn Ala Asp Glu Asn Ala
               495                 500                 505
   CTA CAG TCT GAC GCT AAA GGC AAA CTT GAT TCT GTC GCT ACT GAT TAC     912
   Leu Gln Ser Asp Ala Lys Gly Lys Leu Asp Ser Val Ala Thr Asp Tyr
           510                 515                 520
   GGT GCT GCT ATC GAT GGT TTC ATT GGT GAC GTT TCC GGC CTT GCT AAT     960
   Gly Ala Ala Ile Asp Gly Phe Ile Gly Asp Val Ser Gly Leu Ala Asn
       525                 530                 535
   GGT AAT GGT GCT ACT GGT GAT TTT GCT GGC TCT AAT TCC CAA ATG GCT     1008
   Gly Asn Gly Ala Thr Gly Asp Phe Ala Gly Ser Asn Ser Gln Met Ala
   540                 545                 550                 555
   CAA GTC GGT GAA GGT GAT AAT TCA CCT TTA ATG AAT AAT TTC CGT CAA     1056
   Gln Val Gly Glu Gly Asp Asn Ser Pro Leu Met Asn Asn Phe Arg Gln
                   560                 565                 570
   TAT TTA CCT TCC ATC CCT CAA TCG GTT GAA TGT CGC CCT TTT GTC TTT    1104
   Tyr Leu Pro Ser Ile Pro Gln Ser Val Glu Cys Arg Pro Phe Val Phe
               575                 580                 585
   GGC GCT GGT AAA CCC TAT GAA TTT TCT ATT GAT TGT GAC AAA ATA AAC    1152
   Gly Ala Gly Lys Pro Tyr Glu Phe Ser Ile Asp Cys Asp Lys Ile Asn
           590                 595                 600
   TTA TTC CGT GGT GTC TTT GCG TTT CTT TTA TAT GTT GCC ACC TTT ATG    1200
   Leu Phe Arg Gly Val Phe Ala Phe Leu Leu Tyr Val Ala Thr Phe Met
       605                 610                 615
   TAT GTA TTT TCT ACG TTT GCT AAC ATA CTG CGT AAT AAG GAG TCT        1245
   Tyr Val Phe Ser Thr Phe Ala Asn Ile Leu Arg Asn Lys Glu Ser
   620                 625                 630
   TGATAAGCTT                                                         1255
(2) INFORMATION FOR SEQ ID NO: 280:
   (i) SEQUENCE CHARACTERISTICS:
      (A) LENGTH: 415 amino acids
      (B) TYPE: amino acid
      (D) TOPOLOGY: linear
   (ii) MOLECULE TYPE: protein
   (xi) SEQUENCE DESCRIPTION: SEQ ID NO: 280:
(2) INFORMATION FOR SEQ ID NO: 281:
   (i) SEQUENCE CHARACTERISTICS:
      (A) LENGTH: 502 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene cassette"
   (ix) FEATURE:
      (A) NAME/KEY: CDS
      (B) LOCUTION:9..492
      (D) OTHER INFORMATION:/product= "gIIIp ss"
   (xi) SEQUENCE DESCRIPTION: SEQ ID NO: 281:
   CGG GAA TTC GGA GGC GGT TCC GGT GGT GGC TCT GGT TCC GGT GAT TTT      48
       Glu Phe Gly Gly Gly Ser Gly Gly Gly Ser Gly Ser Gly Asp Phe
                   420                 425                 430
   GAT TAT GAA AAG ATG GCA AAC GCT AAT AAG GGG GCT ATG ACC GAA AAT      96
   Asp Tyr Glu Lys Met Ala Asn Ala Asn Lys Gly Ala Met Thr Glu Asn
               435                 440                 445
   GCC GAT GAA AAC GCG CTA CAG TCT GAC GCT AAA GGC AAA CTT GAT TCT     144
   Ala Asp Glu Asn Ala Leu Gln Ser Asp Ala Lys Gly Lys Leu Asp Ser
           450                 455                 460
   GTC GCT ACT GAT TAC GGT GCT GCT ATC GAT GGT TTC ATT GGT GAC GTT     192
   Val Ala Thr Asp Tyr Gly Ala Ala Ile Asp Gly Phe Ile Gly Asp Val
       465                 470                 475
   TCC GGC CTT GCT AAT GGT AAT GGT GCT ACT GGT GAT TTT GCT GGC TCT     240
   Ser Gly Leu Ala Asn Gly Asn Gly Ala Thr Gly Asp Phe Ala Gly Ser
   480                 485                 490
   AAT TCC CAA ATG GCT CAA GTC GGT GAC GGT GAT AAT TCA CCT TTA ATG     288
   Asn Ser Gln Met Ala Gln Val Gly Asp Gly Asp Asn Ser Pro Leu Met
   495                 500                 505                 510
   AAT AAT TTC CGT CAA TAT TTA CCT TCC CTC CCT CAA TCG GTT GAA TGT     336
   Asn Asn Phe Arg Gln Tyr Leu Pro Ser Leu Pro Gln Ser Val Glu Cys
                   515                 520                 525
   CGC CCT TTT GTC TTT GGC GCT GGT AAA CCA TAT GAA TTT TCT ATT GAT     384
   Arg Pro Phe Val Phe Gly Ala Gly Lys Pro Tyr Glu Phe Ser Ile Asp
               530                 535                 540
   TGT GAC AAA ATA AAC TTA TTC CGT GGT GTC TTT GCG TTT CTT TTA TAT     432
   Cys Asp Lys Ile Asn Leu Phe Arg Gly Val Phe Ala Phe Leu Leu Tyr
           545                 550                 555
   GTT GCC ACC TTT ATG TAT GTA TTT TCT ACG TTT GCT AAC ATA CTG CGT     480
   Val Ala Thr Phe Met Tyr Val Phe Ser Thr Phe Ala Asn Ile Leu Arg
       560                 565                 570
   AAT AAG GAG TCT TGATAAGCTT                                          502
   Asn Lys Glu Ser
   575
(2) INFORMATION FOR SEQ ID NO: 282:
   (i) SEQUENCE CHARACTERISTICS:
      (A) LENGTH: 163 amino acids
      (B) TYPE: amino acid
      (D) TOPOLOGY: linear
   (ii) MOLECULE TYPE: protein
   (xi) SEQUENCE DESCRIPTION: SEQ ID NO: 282:
   Glu Phe Gly Gly Gly Ser Gly Gly Gly Ser Gly Ser Gly Asp Phe Asp
    1               5                  10                  15
   Tyr Glu Lys Met Ala Asn Ala Asn Lys Gly Ala Met Thr Glu Asn Ala
               20                  25                  30
   Asp Glu Asn Ala Leu Gln Ser Asp Ala Lys Gly Lys Leu Asp Ser Val
           35                  40                  45
   Ala Thr Asp Tyr Gly Ala Ala Ile Asp Gly Phe Ile Gly Asp Val Ser
       50                  55                  60
   Gly Leu Ala Asn Gly Asn Gly Ala Thr Gly Asp Phe Ala Gly Ser Asn
   65                  70                  75                  80
   Ser Gln Met Ala Gln Val Gly Asp Gly Asp Asn Ser Pro Leu Met Asn
                   85                  90                  95
   Asn Phe Arg Gln Tyr Leu Pro Ser Leu Pro Gln Ser Val Glu Cys Arg
               100                 105                 110
   Pro Phe Val Phe Gly Ala Gly Lys Pro Tyr Glu Phe Ser Ile Asp Cys
           115                 120                 125
   Asp Lys Ile Asn Leu Phe Arg Gly Val Phe Ala Phe Leu Leu Tyr Val
       130                 135                 140
   Ala Thr Phe Met Tyr Val Phe Ser Thr Phe Ala Asn Ile Leu Arg Asn
   145                 150                 155                 160
   Lys Glu Ser
(2) INFORMATION FOR SEQ ID NO: 283:
   (i) SEQUENCE CHARACTERISTICS:
      (A) LENGTH: 47 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic DNA cassette"
   (xi) SEQUENCE DESCRIPTION: SEQ ID NO: 283:
      GCATGCCATA ACTTCGTATA ATGTACGCTA TACGAAGTTA TAAGCTT 47
(2) INFORMATION FOR SEQ ID NO: 284:
   (i) SEQUENCE CHARACTERISTICS:
      (A) LENGTH: 1163 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene cassette"
   (ix) FEATURE:
      (A) NAME/KEY: CDS
      (B) LOCATION:82..978
      (D) OTHER INFORMATION:/product= "bla resistance"
   (xi) SEQUENCE DESCRIPTION: SEQ ID NO: 284:
   GGGGGTGTAC ATTCAAATAT GTATCCGCTC ATGAGACAAT AACCCTGATA AATGCTTCAA        60
   TAATATTGAA AAAGGAAGAG T ATG AGT ATT CAA CAT TTC CGT GTC GCC CTT        111
                           Met Ser Ile Gln His Phe Arg Val Ala Leu
                                   165                 170
   ATT CCC TTT TTT GCG GCA TTT TGC CTT CCT GTT TTT GCT CAC CCA GAA        159
   Ile Pro Phe Phe Ala Ala Phe Cys Leu Pro Val Phe Ala His Pro Glu
       175                 180                 185
   ACG CTG GTG AAA GTA AAA GAT GCT GAG GAT CAG TTG GGT GCG CGA GTG        207
   Thr Leu Val Lys Val Lys Asp Ala Glu Asp Gln Leu Gly Ala Arg Val
   190                 195                 200                 205
   GGT TAC ATC GAA CTG GAT CTC AAC AGC GGT AAG ATC CTT GAG AGT TTT        255
   Gly Tyr Ile Glu Leu Asp Leu Asn Ser Gly Lys Ile Leu Glu Ser Phe
                   210                 215                 220
   CGC CCC GAA GAA CGT TTT CCA ATG ATG AGC ACT TTT AAA GTT CTG CTA        303
   Arg Pro Glu Glu Arg Phe Pro Met Met Ser Thr Phe Lys Val Leu Leu
               225                 230                 235
   TGT GGC GCG GTA TTA TCC CGT ATT GAC GCC GGG CAA GAG CAA CTC GGT        351
   Cys Gly Ala Val Leu Ser Arg Ile Asp Ala Gly Gln Glu Gln Leu Gly
           240                 245                 250
   CGC CGC ATA CAC TAT TCT CAG AAT GAC TTG GTT GAG TAC TCA CCA GTC        399
   Arg Arg Ile His Tyr Ser Gln Asn Asp Leu Val Glu Tyr Ser Pro Val
       255                 260                 265
   ACA GAA AAG CAT CTT ACG GAT GGC ATG ACA GTA AGA GAA TTA TGC AGT        447
   Thr Glu Lys His Leu Thr Asp Gly Met Thr Val Arg Glu Leu Cys Ser
   270                 275                 280                 285
   GCT GCC ATA ACC ATG AGT GAT AAC ACT GCG GCC AAC TTA CTT CTG ACA        495
   Ala Ala Ile Thr Met Ser Asp Asn Thr Ala Ala Asn Leu Leu Leu Thr
                   290                 295                 300
   ACG ATC GGA GGA CCG AAG GAG CTA ACC GCT TTT TTG CAC AAC ATG GGG        543
   Thr Ile Gly Gly Pro Lys Glu Leu Thr Ala Phe Leu His Asn Met Gly
               305                 310                 315
   GAT CAT GTA ACT CGC CTT GAT CGT TGG GAA CCG GAG CTG AAT GAA GCC        591
   Asp His Val Thr Arg Leu Asp Arg Trp Glu Pro Glu Leu Asn Glu Ala
           320                 325                 330
   ATA CCA AAC GAC GAG CGT GAC ACC ACG ATG CCT GTA GCA ATG GCA ACA        639
   Ile Pro Asn Asp Glu Arg Asp Thr Thr Met Pro Val Ala Met Ala Thr
       335                 340                 345
   ACG TTG CGC AAA CTA TTA ACT GGC GAA CTA CTT ACT CTA GCT TCC CGG       687
   Thr Leu Arg Lys Leu Leu Thr Gly Glu Leu Leu Thr Leu Ala Ser Arg
   350                 355                 360                 365
   CAA CAG TTA ATA GAC TGG ATG GAG GCG GAT AAA GTT GCA GGA CCA CTT       735
   Gln Gln Leu Ile Asp Trp Met Glu Ala Asp Lys Val Ala Gly Pro Leu
                   370                 375                 380
   CTG CGC TCG GCC CTT CCG GCT GGC TGG TTT ATT GCT GAT AAA TCT GGA       783
   Leu Arg Ser Ala Leu Pro Ala Gly Trp Phe Ile Ala Asp Lys Ser Gly
               385                 390                 395
   GCC GGT GAG CGT GGG TCT CGC GGT ATC ATT GCA GCA CTG GGG CCA GAT       831
   Ala Gly Glu Arg Gly Ser Arg Gly Ile Ile Ala Ala Leu Gly Pro Asp
           400                 405                 410
   GGT AAG CCC TCC CGT ATC GTA GTT ATC TAC ACG ACG GGG AGT CAG GCA       879
   Gly Lys Pro Ser Arg Ile Val Val Ile Tyr Thr Thr Gly Ser Gln Ala
       415                 420                 425
   ACT ATG GAT GAA CGA AAT AGA CAG ATC GCT GAG ATA GGT GCC TCA CTG       927
   Thr Met Asp Glu Arg Asn Arg Gln Ile Ala Glu Ile Gly Ala Ser Leu
   430                 435                 440                 445
   ATT AAG CAT TGG GTA ACT GTC AGA CCA AGT TTA CTC ATA TAT ACT TTA       975
   Ile Lys His Trp Val Thr Val Arg Pro Ser Leu Leu Ile Tyr Thr Leu
                   450                 455                 460
   GAT TGATTTAAAA CTTCATTTTT AATTTAAAAG GATCTAGGTG AAGATCCTTT           1028
   Asp
   TTGATAATCT CATGACCAAA ATCCCTTAAC GTGAGTTTTC GTTCCACTGA GCGTCAGACC    1088
   CCGTAGAAAA GATCAAAGGA TCTTCTTGAG ATCCTTTTTG ATAATGGCCG GCCCCCCCCC    1148
   TTAATTAAGG GGGGG                                                      1163
(2) INFORMATION FOR SEQ ID NO: 285:
   (i) SEQUENCE CHARACTERISTICS:
      (A) LENGTH: 299 amino acids
      (B) TYPE: amino acid
      (D) TOPOLOGY: linear
   (ii) MOLECULE TYPE: protein
   (xi) SEQUENCE DESCRIPTION: SEQ ID NO: 285:
   Met Ser Ile Gln His Phe Arg Val Ala Leu Ile Pro Phe Phe Ala Ala
    1               5                  10                  15
   Phe Cys Leu Pro Val Phe Ala His Pro Glu Thr Leu Val Lys Val Lys
               20                  25                  30
   Asp Ala Glu Asp Gln Leu Gly Ala Arg Val Gly Tyr Ile Glu Leu Asp
           35                  40                  45
   Leu Asn Ser Gly Lys Ile Leu Glu Ser Phe Arg Pro Glu Glu Arg Phe
        50                  55                  60
   Pro Met Met Ser Thr Phe Lys Val Leu Leu Cys Gly Ala Val Leu Ser
    65                  70                  75                  80
   Arg Ile Asp Ala Gly Gln Glu Gln Leu Gly Arg Arg Ile His Tyr Ser
                    85                  90                  95
   Gln Asn Asp Leu Val Glu Tyr Ser Pro Val Thr Glu Lys His Leu Thr
               100                 105                 110
   Asp Gly Met Thr Val Arg Glu Leu Cys Ser Ala Ala Ile Thr Met Ser
           115                 120                 125
   Asp Asn Thr Ala Ala Asn Leu Leu Leu Thr Thr Ile Gly Gly Pro Lys
       130                 135                 140
   Glu Leu Thr Ala Phe Leu His Asn Met Gly Asp His Val Thr Arg Leu
   145                 150                 155                 160
   Asp Arg Trp Glu Pro Glu Leu Asn Glu Ala Ile Pro Asn Asp Glu Arg
                   165                 170                 175
   Asp Thr Thr Met Pro Val Ala Met Ala Thr Thr Leu Arg Lys Leu Leu
               180                 185                 190
   Thr Gly Glu Leu Leu Thr Leu Ala Ser Arg Gln Gln Leu Ile Asp Trp
           195                 200                 205
   Met Glu Ala Asp Lys Val Ala Gly Pro Leu Leu Arg Ser Ala Leu Pro
       210                 215                 220
   Ala Gly Trp Phe Ile Ala Asp Lys Ser Gly Ala Gly Glu Arg Gly Ser
   225                 230                 235                 240
   Arg Gly Ile Ile Ala Ala Leu Gly Pro Asp Gly Lys Pro Ser Arg Ile
                   245                 250                 255
   Val Val Ile Tyr Thr Thr Gly Ser Gln Ala Thr Met Asp Glu Arg Asn
               260                 265                 270
   Arg Gln Ile Ala Glu Ile Gly Ala Ser Leu Ile Lys His Trp Val Thr
           275                 280                 285
   Val Arg Pro Ser Leu Leu Ile Tyr Thr Leu Asp
       290                 295
(2) INFORMATION FOR SEQ ID NO: 286:
   (i) SEQUENCE CHARACTERISTICS:
      (A) LENGTH: 470 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic DNA cassette"
   (xi) SEQUENCE DESCRIPTION: SEQ ID NO: 286:
   GCTAGCACGC GCCCTGTAGC GGCGCATTAA GCGCGGCGGG TGTGGTGGTT ACGCGCAGCG 60
   TGACCGCTAC ACTTGCCAGC GCCCTAGCGC CCGCTCCTTT CGCTTTCTTC CCTTCCTTTC 120
   TCGCCACGTT CGCCGGCTTT CCCCGTCAAG CTCTAAATCG GGGGCTCCCT TTAGGGTTCC 180
   GATTTAGTGC TTTACGGCAC CTCGACCCCA AAAAACTTGA TTAGGGTGAT GGTTCTCGTA 240
   GTGGGCCATC GCCCTGATAG ACGGTTTTTC GCCCTTTGAC GTTGGAGTCC ACGTTCTTTA 300
   ATAGTGGACT CTTGTTCCAA ACTGGAACAA CACTCAACCC TATCTCGGTC TATTCTTTTG 360
   ATTTATAAGG GATTTTGCCG ATTTCGGCCT ATTGGTTAAA AAATGAGCTG ATTTAACAAA 420
   AATTTAACGC GAATTTTAAC AAAATATTAA CGTTTACAAT TTCATGTACA 470
(2) INFORMATION FOR SEQ ID NO: 287:
   (i) SEQUENCE CHARACTERISTICS:
      (A) LENGTH: 832 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic DNA cassette"
   (xi) SEQUENCE DESCRIPTION: SEQ ID NO: 287:
   AGATCTAATA AGATGATCTT CTTGAGATCG TTTTGGTCTG CGCGTAATCT CTTGCTCTGA 60
   AAACGAAAAA ACCGCCTTGC AGGGCGGTTT TTCGTAGGTT CTCTGAGCTA CCAACTCTTT 120
   GAACCGAGGT AACTGGCTTG GAGGAGCGCA GTCACTAAAA CTTGTCCTTT CAGTTTAGCC 180
   TTAACCGGCG CATGACTTCA AGACTAACTC CTCTAAATCA ATTACCAGTG GCTGCTGCCA 240
   GTGGTGCTTT TGCATGTCTT TCCGGGTTGG ACTCAAGACG ATAGTTACCG GATAAGGCGC 300
   AGCGGTCGGA CTGAACGGGG GGTTCGTGCA TACAGTCCAG CTTGGAGCGA ACTGCCTACC 360
   CGGAACTGAG TGTCAGGCGT GGAATGAGAC AAACGCGGCC ATAACAGCGG AATGACACCG 420
   GTAAACCGAA AGGCAGGAAC AGGAGAGCGC AGGAGGGAGC CGCCAGGGGG AAACGCCTGG 480
   TATCTTTATA GTCCTGTCGG GTTTCGCCAC CACTGATTTG AGCGTCAGAT TTCGTGATGC 540
   TTGTCAGGGG GGCGGAGCCT ATGGAAAAAC GGCTTTGCCG CGGCCCTCTC ACTTCCCTGT 600
   TAAGTATCTT CCTGGCATCT TCCAGGAAAT CTCCGCCCCG TTCGTAAGCC ATTTCCGCTC 660
   GCCGCAGTCG AACGACCGAG CGTAGCGAGT CAGTGAGCGA GGAAGCGGAA TATATCCTGT 720
   ATCACATATT CTGCTGACGC ACCGGTGCAG CCTTTTTTCT CCTGCCACAT GAAGCACTTC 780
   ACTGACACCC TCATCAGTGC CAACATAGTA AGCCAGTATA CACTCCGCTA GC 832
(2) INFORMATION FOR SEQ ID NO: 288:
   (i) SEQUENCE CHARACTERISTICS:
      (A) LENGTH: 49 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic DNA cassette"
   (xi) SEQUENCE DESCRIPTION: SEQ ID NO: 288:
      AGATCTCATA ACTTCGTATA ATGTATGCTA TACGAAGTTA TTCAGATCT 49
(2) INFORMATION FOR SEQ ID NO: 289:
   (i) SEQUENCE CHARACTERISTICS:
      (A) LENGTH: 96 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic DNA cassette"
   (xi) SEQUENCE DESCRIPTION: SEQ ID NO: 289:
   TCTAGAGCAT GCGTAGGAGA AAATAAAATG AAACAAAGCA CTATTGCACT GGCACTCTTA 60
   CCGTTGCTCT TCACCCCTGT TACCAAAGCC GAATTC 96
(2) INFORMATION FOR SEQ ID NO: 290:
   (i) SEQUENCE CHARACTERISTICS:
      (A) LENGTH: 120 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic DNA cassette"
   (xi) SEQUENCE DESCRIPTION: SEQ ID NO: 290:
   TCTAGAGCAT GCGTAGGAGA AAATAAAATG AAACAAAGCA CTATTGCACT GGCACTCTTA 60
   CCGTTGCTCT TCACCCCTGT TACCAAAGCC GACTACAAAG ATGAAGTGCA ATTGGAATTC 120
(2) INFORMATION FOR SEQ ID NO: 291:
   (i) SEQUENCE CHARACTERISTICS:
      (A) LENGTH: 96 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic DNA cassette"
   (xi) SEQUENCE DESCRIPTION: SEQ ID NO: 291:
   TCTAGAGGTT GAGGTGATTT TATGAAAAAG AATATCGCAT TTCTTCTTGC ATCTATGTTC 60
   GTTTTTTCTA TTGCTACAAA TGCATACGCT GAATTC 96
(2) INFORMATION FOR SEQ ID NO: 292:
   (i) SEQUENCE CHARACTERISTICS:
      (A) LENGTH: 1221 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene cassette"
   (ix) FEATURE:
      (A) NAME/KEY: CDS
      (B) LOCATION:79..1158
      (D) OTHER INFORMATION:/product= "lacI"
   (xi) SEQUENCE DESCRIPTION: SEQ ID NO: 292:
(2) INFORMATION FOR SEQ ID NO: 293:
   (i) SEQUENCE CHARACTERISTICS:
      (A) LENGTH: 360 amino acids
      (B) TYPE: amino acid
      (D) TOPOLOGY: linear
   (ii) MOLECULE TYPE: protein
   (xi) SEQUENCE DESCRIPTION: SEQ ID NO: 293:
   Val Lys Pro Val Thr Leu Tyr Asp Val Ala Glu Tyr Ala Gly Val Ser
   1               5                   10                  15
   Tyr Gln Thr Val Ser Arg Val Val Asn Gln Ala Ser His Val Ser Ala
               20                  25                  30
   Lys Thr Arg Glu Lys Val Glu Ala Ala Met Ala Glu Leu Asn Tyr Ile
           35                  40                  45
   Pro Asn Arg Val Ala Gln Gln Leu Ala Gly Lys Gln Ser Leu Leu Ile
       50                  55                  60
   Gly Val Ala Thr Ser Ser Leu Ala Leu His Ala Pro Ser Gln Ile Val
   65                  70                  75                  80
   Ala Ala Ile Lys Ser Arg Ala Asp Gln Leu Gly Ala Ser Val Val Val
                   85                  90                  95
   Ser Met Val Glu Arg Ser Gly Val Glu Ala Cys Lys Ala Ala Val His
               100                 105                 110
   Asn Leu Leu Ala Gln Arg Val Ser Gly Leu Ile Ile Asn Tyr Pro Leu
           115                 120                 125
   Asp Asp Gln Asp Ala Ile Ala Val Glu Ala Ala Cys Thr Asn Val Pro
       130                 135                 140
   Ala Leu Phe Leu Asp Val Ser Asp Gln Thr Pro Ile Asn Ser Ile Ile
   145                 150                 155                 160
   Phe Ser His Glu Asp Gly Thr Arg Leu Gly Val Glu His Leu Val Ala
                           165                 170                 175
   Leu Gly His Gln Gln Ile Ala Leu Leu Ala Gly Pro Leu Ser Ser Val
               180                 185                 190
   Ser Ala Arg Leu Arg Leu Ala Gly Trp His Lys Tyr Leu Thr Arg Asn
           195                 200                 205
   Gln Ile Gln Pro Ile Ala Glu Arg Glu Gly Asp Trp Ser Ala Met Ser
       210                 215                 220
   Gly Phe Gln Gln Thr Met Gln Met Leu Asn Glu Gly Ile Val Pro Thr
   225                 230                 235                 240
   Ala Met Leu Val Ala Asn Asp Gln Met Ala Leu Gly Ala Met Arg Ala
                   245                 250                 255
   Ile Thr Glu Ser Gly Leu Arg Val Gly Ala Asp Ile Ser Val Val Gly
               260                 265                 270
   Tyr Asp Asp Thr Glu Asp Ser Ser Cys Tyr Ile Pro Pro Leu Thr Thr
           275                 280                 285
   Ile Lys Gln Asp Phe Arg Leu Leu Gly Gln Thr Ser Val Asp Arg Leu
       290                 295                 300
   Leu Gln Leu Ser Gln Gly Gln Ala Val Lys Gly Asn Gln Leu Leu Pro
   305                 310                 315                 320
   Val Ser Leu Val Lys Arg Lys Thr Thr Leu Ala Pro Asn Thr Gln Thr
                   325                 330                 335
   Ala Ser Pro Arg Ala Leu Ala Asp Ser Leu Met Gln Leu Ala Arg Gln
               340                 345                 350
   Val Ser Arg Leu Glu Ser Gly Gln
           355                 360
(2) INFORMATION FOR SEQ ID NO: 294:
   (i) SEQUENCE CHARACTERISTICS:
      (A) LENGTH: 2380 base pairs
      (B) TYPE: nucleic acid
      (C) STRANDEDNESS: double
      (D) TOPOLOGY: circular
   (ii) MOLECULE TYPE: other nucleic acid
      (A) DESCRIPTION: /desc = "synthetic vector"
   (ix) FEATURE:
      (A) NAME/KEY: CDS
      (B) LOCATION:complement (51..707)
      (D) OTHER INFORMATION:/product= "cat resistance"
   (xi) SEQUENCE DESCRIPTION: SEQ ID NO: 294:
   GATCTAGCAC CAGGCGTTTA AGGGCACCAA TAACTGCCTT AAAAAAATTA CGCCCCGCCC 60
   TGCCACTCAT CGCAGTACTG TTGTAATTCA TTAAGCATTC TGCCGACATG GAAGCCATCA 120
   CAAACGGCAT GATGAACCTG AATCGCCAGC GGCATCAGCA CCTTGTCGCC TTGCGTATAA 180
   TATTTGCCCA TAGTGAAAAC GGGGGCGAAG AAGTTGTCCA TATTGGCTAC GTTTAAATCA 240
   AAACTGGTGA AACTCACCCA GGGATTGGCT GAGACGAAAA ACATATTCTC AATAAACCCT 300
   TTAGGGAAAT AGGCCAGGTT TTCACCGTAA CACGCCACAT CTTGCGAATA TATGTGTAGA 360
   AACTGCCGGA AATCGTCGTG GTATTCACTC CAGAGCGATG AAAACGTTTC AGTTTGCTCA 420
   TGGAAAACGG TGTAACAAGG GTGAACACTA TCCCATATCA CCAGCTCACC GTCTTTCATT 480
   GCCATACGGA ACTCCGGGTG AGCATTCATC AGGCGGGCAA GAATGTGAAT AAAGGCCGGA 540
   TAAAACTTGT GCTTATTTTT CTTTACGGTC TTTAAAAAGG CCGTAATATC CAGCTGAACG 600
   GTCTGGTTAT AGGTACATTG AGCAACTGAC TGAAATGCCT CAAAATGTTC TTTACGATGC 660
   CATTGGGATA TATCAACGGT GGTATATCCA GTGATTTTTT TCTCCATTTT AGCTTCCTTA 720
   GCTCCTGAAA ATCTCGATAA CTCAAAAAAT ACGCCCGGTA GTGATCTTAT TTCATTATGG 780
   TGAAAGTTGG AACCTCACCC GACGTCTAAT GTGAGTTAGC TCACTCATTA GGCACCCCAG 840
   GCTTTACACT TTATGCTTCC GGCTCGTATG TTGTGTGGAA TTGTGAGCGG ATAACAATTT 900
   CACACAGGAA ACAGCTATGA CCATGATTAC GAATTTCTAG ACCCCCCCCC CGCATGCCAT 960
   AACTTCGTAT AATGTACGCT ATACGAAGTT ATAAGCTTGA CCTGTGAAGT GAAAAATGGC 1020
   GCAGATTGTG CGACATTTTT TTTGTCTGCC GTTTAATTAA AGGGGGGGGG GGGCCGGCCT 1080
   GGGGGGGGGT GTACATGAAA TTGTAAACGT TAATATTTTG TTAAAATTCG CGTTAAATTT 1140
   TTGTTAAATC AGCTCATTTT TTAACCAATA GGCCGAAATC GGCAAAATCC CTTATAAATC 1200
   AAAAGAATAG ACCGAGATAG GGTTGAGTGT TGTTCCAGTT TGGAACAAGA GTCCACTATT 1260
   AAAGAACGTG GACTCCAACG TCAAAGGGCG AAAAACCGTC TATCAGGGCG ATGGCCCACT 1320
   ACGAGAACCA TCACCCTAAT CAAGTTTTTT GGGGTCGAGG TGCCGTAAAG CACTAAATCG 1380
   GAACCCTAAA GGGAGCCCCC GATTTAGAGC TTGACGGGGA AAGCCGGCGA ACGTGGCGAG 1440
   AAAGGAAGGG AAGAAAGCGA AAGGAGCGGG CGCTAGGGCG CTGGCAAGTG TAGCGGTCAC 1500
   GCTGCGCGTA ACCACCACAC CCGCCGCGCT TAATGCGCCG CTACAGGGCG CGTGCTAGCG 1560
   GAGTGTATAC TGGCTTACTA TGTTGGCACT GATGAGGGTG TCAGTGAAGT GCTTCATGTG 1620
   GCAGGAGAAA AAAGGCTGCA CCGGTGCGTC AGCAGAATAT GTGATACAGG ATATATTCCG 1680
   CTTCCTCGCT CACTGACTCG CTACGCTCGG TCGTTCGACT GCGGCGAGCG GAAATGGCTT 1740
   ACGAACGGGG CGGAGATTTC CTGGAAGATG CCAGGAAGAT ACTTAACAGG GAAGTGAGAG 1800
   GGCCGCGGCA AAGCCGTTTT TCCATAGGCT CCGCCCCCCT GACAAGCATC ACGAAATCTG 1860
   ACGCTCAAAT CAGTGGTGGC GAAACCCGAC AGGACTATAA AGATACCAGG CGTTTCCCCC    1920
   TGGCGGCTCC CTCCTGCGCT CTCCTGTTCC TGCCTTTCGG TTTACCGGTG TCATTCCGCT    1980
   GTTATGGCCG CGTTTGTCTC ATTCCACGCC TGACACTCAG TTCCGGGTAG GCAGTTCGCT    2040
   CCAAGCTGGA CTGTATGCAC GAACCCCCCG TTCAGTCCGA CCGCTGCGCC TTATCCGGTA    2100
   ACTATCGTCT TGAGTCCAAC CCGGAAAGAC ATGCAAAAGC ACCACTGGCA GCAGCCACTG    2160
   GTAATTGATT TAGAGGAGTT AGTCTTGAAG TCATGCGCCG GTTAAGGCTA AACTGAAAGG    2220
   ACAAGTTTTA GTGACTGCGC TCCTCCAAGC CAGTTACCTC GGTTCAAAGA GTTGGTAGCT    2280
   CAGAGAACCT ACGAAAAACC GCCCTGCAAG GCGGTTTTTT CGTTTTCAGA GCAAGAGATT    2340
   ACGCGCAGAC CAAAACGATC TCAAGAAGAT CATCTTATTA                          2380
(2) INFORMATION FOR SEQ ID NO: 295:
   (i) SEQUENCE CHARACTERISTICS:
      (A) LENGTH: 219 amino acids
      (B) TYPE: amino acid
      (D) TOPOLOGY: linear
   (ii) MOLECULE TYPE: protein
   (xi) SEQUENCE DESCRIPTION: SEQ ID NO: 295:
   Met Glu Lys Lys Ile Thr Gly Tyr Thr Thr Val Asp Ile Ser Gln Trp
   His Arg Lys Glu His Phe Glu Ala Phe Gln Ser Val Ala Gln Cys Thr
               20                  25                  30
   Tyr Asn Gln Thr Val Gln Leu Asp Ile Thr Ala Phe Leu Lys Thr Val
           35                  40                  45
   Lys Lys Asn Lys His Lys Phe Tyr Pro Ala Phe Ile His Ile Leu Ala
       50                  55                  60
   Arg Leu Met Asn Ala His Pro Glu Phe Arg Met Ala Met Lys Asp Gly
   65                  70                  75                  80
   Glu Leu Val Ile Trp Asp Ser Val His Pro Cys Tyr Thr Val Phe His
                   85                  90                  95
   Glu Gln Thr Glu Thr Phe Ser Ser Leu Trp Ser Glu Tyr His Asp Asp
               100                 105                 110
   Phe Arg Gln Phe Leu His Ile Tyr Ser Gln Asp Val Ala Cys Tyr Gly
           115                 120                 125
   Glu Asn Leu Ala Tyr Phe Pro Lys Gly Phe Ile Glu Asn Met Phe Phe
       130                 135                 140
   Val Ser Ala Asn Pro Trp Val Ser Phe Thr Ser Phe Asp Leu Asn Val
   145                 150                 155                 160
   Ala Asn Met Asp Asn Phe Phe Ala Pro Val Phe Thr Met Gly Lys Tyr
                   165                 170                 175
   Tyr Thr Gln Gly Asp Lys Val Leu Met Pro Leu Ala Ile Gln Val His
               180                 185                 190
   His Ala Val Cys Asp Gly Phe His Val Gly Arg Met Leu Asn Glu Leu
           195                 200                 205
   Gln Gln Tyr Cys Asp Glu Trp Gln Gly Gly Ala
       210                 215
(2) INFORMATION FOR SEQ ID NO: 296:
   (i) SEQUENCE CHARACTERISTICS:
      (A) LENGTH: 3488 base pairs
      (B) TYPE: nucleic acid
      (C) STRANDEDNESS: double
      (D) TOPOLOGY: circular
   (ii) MOLECULE TYPE: other nucleic acid
      (A) DESCRIPTION: /desc = "synthetic vector"

      (A) NAME/KEY: CDS
      (B) LOCATION:complement (1341..1997)
      (D) OTHER INFORMATION:/product= "cat resistance"
   (ix) FEATURE:
      (A) NAME/KEY: CDS
      (B) LOCATION:complement (2521..3417)
      (D) OTHER INFORMATION:/product= "bla resistance"
   (xi) SEQUENCE DESCRIPTION: SEQ ID NO: 296:
   GTACATGAAA TTGTAAACGT TAATATTTTG TTAAAATTCG CGTTAAATTT TTGTTAAATC    60
   AGCTCATTTT TTAACCAATA GGCCGAAATC GGCAAAATCC CTTATAAATC AAAAGAATAG   120
   ACCGAGATAG GGTTGAGTGT TGTTCCAGTT TGGAACAAGA GTCCACTATT AAAGAACGTG   180
   GACTCCAACG TCAAAGGGCG AAAAACCGTC TATCAGGGCG ATGGCCCACT ACGAGAACCA   240
   TCACCCTAAT CAAGTTTTTT GGGGTCGAGG TGCCGTAAAG CACTAAATCG GAACCCTAAA   300
   GGGAGCCCCC GATTTAGAGC TTGACGGGGA AAGCCGGCGA ACGTGGCGAG AAAGGAAGGG   360
   AAGAAAGCGA AAGGAGCGGG CGCTAGGGCG CTGGCAAGTG TAGCGGTCAC GCTGCGCGTA   420
   ACCACCACAC CCGCCGCGCT TAATGCGCCG CTACAGGGCG CGTGCTAGCG GAGTGTATAC   480
   TGGCTTACTA TGTTGGCACT GATGAGGGTG TCAGTGAAGT GCTTCATGTG GCAGGAGAAA   540
   AAAGGCTGCA CCGGTGCGTC AGCAGAATAT GTGATACAGG ATATATTCCG CTTCCTCGCT   600
   CACTGACTCG CTACGCTCGG TCGTTCGACT GCGGCGAGCG GAAATGGCTT ACGAACGGGG   660
   CGGAGATTTC CTGGAAGATG CCAGGAAGAT ACTTAACAGG GAAGTGAGAG GGCCGCGGCA   720
   AAGCCGTTTT TCCATAGGCT CCGCCCCCCT GACAAGCATC ACGAAATCTG ACGCTCAAAT   780
   CAGTGGTGGC GAAACCCGAC AGGACTATAA AGATACCAGG CGTTTCCCCC TGGCGGCTCC 840
   CTCCTGCGCT CTCCTGTTCC TGCCTTTCGG TTTACCGGTG TCATTCCGCT GTTATGGCCG 900
   CGTTTGTCTC ATTCCACGCC TGACACTCAG TTCCGGGTAG GCAGTTCGCT CCAAGCTGGA 960
   CTGTATGCAC GAACCCCCCG TTCAGTCCGA CCGCTGCGCC TTATCCGGTA ACTATCGTCT 1020
   TGAGTCCAAC CCGGAAAGAC ATGCAAAAGC ACCACTGGCA GCAGCCACTG GTAATTGATT 1080
   TAGAGGAGTT AGTCTTGAAG TCATGCGCCG GTTAAGGCTA AACTGAAAGG ACAAGTTTTA 1140
   GTGACTGCGC TCCTCCAAGC CAGTTACCTC GGTTCAAAGA GTTGGTAGCT CAGAGAACCT 1200
   ACGAAAAACC GCCCTGCAAG GCGGTTTTTT CGTTTTCAGA GCAAGAGATT ACGCGCAGAC 1260
   CAAAACGATC TCAAGAAGAT CATCTTATTA GATCTAGCAC CAGGCGTTTA AGGGCACCAA 1320
   TAACTGCCTT AAAAAAATTA CGCCCCGCCC TGCCACTCAT CGCAGTACTG TTGTAATTCA 1380
   TTAAGCATTC TGCCGACATG GAAGCCATCA CAAACGGCAT GATGAACCTG AATCGCCAGC 1440
   GGCATCAGCA CCTTGTCGCC TTGCGTATAA TATTTGCCCA TAGTGAAAAC GGGGGCGAAG 1500
   AAGTTGTCCA TATTGGCTAC GTTTAAATCA AAACTGGTGA AACTCACCCA GGGATTGGCT 1560
   GAGACGAAAA ACATATTCTC AATAAACCCT TTAGGGAAAT AGGCCAGGTT TTCACCGTAA 1620
   CACGCCACAT CTTGCGAATA TATGTGTAGA AACTGCCGGA AATCGTCGTG GTATTCACTC 1680
   CAGAGCGATG AAAACGTTTC AGTTTGCTCA TGGAAAACGG TGTAACAAGG GTGAACACTA 1740
   TCCCATATCA CCAGCTCACC GTCTTTCATT GCCATACGGA ACTCCGGGTG AGCATTCATC 1800
   AGGCGGGCAA GAATGTGAAT AAAGGCCGGA TAAAACTTGT GCTTATTTTT CTTTACGGTC 1860
   TTTAAAAAGG CCGTAATATC CAGCTGAACG GTCTGGTTAT AGGTACATTG AGCAACTGAC 1920
   TGAAATGCCT CAAAATGTTC TTTACGATGC CATTGGGATA TATCAACGGT GGTATATCCA 1980
   GTGATTTTTT TCTCCATTTT AGCTTCCTTA GCTCCTGAAA ATCTCGATAA CTCAAAAAAT 2040
   ACGCCCGGTA GTGATCTTAT TTCATTATGG TGAAAGTTGG AACCTCACCC GACGTCTAAT 2100
   GTGAGTTAGC TCACTCATTA GGCACCCCAG GCTTTACACT TTATGCTTCC GGCTCGTATG 2160
   TTGTGTGGAA TTGTGAGCGG ATAACAATTT CACACAGGAA ACAGCTATGA CCATGATTAC 2220
   GAATTTCTAG ACCCCCCCCC CGCATGCCAT AACTTCGTAT AATGTACGCT ATACGAAGTT 2280
   ATAAGCTTGA CCTGTGAAGT GAAAAATGGC GCAGATTGTG CGACATTTTT TTTGTCTGCC 2340
   GTTTAATTAA GGGGGGGGGC CGGCCATTAT CAAAAAGGAT CTCAAGAAGA TCCTTTGATC 2400
   TTTTCTACGG GGTCTGACGC TCAGTGGAAC GAAAACTCAC GTTAAGGGAT TTTGGTCATG 2460
   AGATTATCAA AAAGGATCTT CACCTAGATC CTTTTAAATT AAAAATGAAG TTTTAAATCA 2520
   ATCTAAAGTA TATATGAGTA AACTTGGTCT GACAGTTACC CAATGCTTAA TCAGTGAGGC 2580
   ACCTATCTCA GCGATCTGTC TATTTCGTTC ATCCATAGTT GCCTGACTCC CCGTCGTGTA 2640
   GATAACTACG ATACGGGAGG GCTTACCATC TGGCCCCAGT GCTGCAATGA TACCGCGAGA 2700
   CCCACGCTCA CCGGCTCCAG ATTTATCAGC AATAAACCAG CCAGCCGGAA GGGCCGAGCG 2760
   CAGAAGTGGT CCTGCAACTT TATCCGCCTC CATCCAGTCT ATTAACTGTT GCCGGGAAGC 2820
   TAGAGTAAGT AGTTCGCCAG TTAATAGTTT GCGCAACGTT GTTGCCATTG CTACAGGCAT 2880
   CGTGGTGTCA CGCTCGTCGT TTGGTATGGC TTCATTCAGC TCCGGTTCCC AACGATCAAG 2940
   GCGAGTTACA TGATCCCCCA TGTTGTGCAA AAAAGCGGTT AGCTCCTTCG GTCCTCCGAT 3000
   CGTTGTCAGA AGTAAGTTGG CCGCAGTGTT ATCACTCATG GTTATGGCAG CACTGCATAA 3060
   TTCTCTTACT GTCATGCCAT CCGTAAGATG CTTTTCTGTG ACTGGTGAGT ACTCAACCAA 3120
   GTCATTCTGA GAATAGTGTA TGCGGCGACC GAGTTGCTCT TGCCCGGCGT CAATACGGGA 3180
   TAATACCGCG CCACATAGCA GAACTTTAAA AGTGCTCATC ATTGGAAAAC GTTCTTCGGG 3240
   GCGAAAACTC TCAAGGATCT TACCGCTGTT GAGATCCAGT TCGATGTAAC CCACTCGCGC 3300
   ACCCAACTGA TCCTCAGCAT CTTTTACTTT CACCAGCGTT TCTGGGTGAG CAAAAACAGG 3360
   AAGGCAAAAT GCCGCAAAAA AGGGAATAAG GGCGACACGG AAATGTTGAA TACTCATACT 3420
   CTTCCTTTTT CAATATTATT GAAGCATTTA TCAGGGTTAT TGTCTCATGA GCGGATACAT 3480
   ATTTGAAT 3488
(2) INFORMATION FOR SEQ ID NO: 297:
   (i) SEQUENCE CHARACTERISTICS:
      (A) LENGTH: 219 amino acids
      (B) TYPE: amino acid
      (D) TOPOLOGY: linear
   (ii) MOLECULE TYPE: protein
   (xi) SEQUENCE DESCRIPTION: SEQ ID NO: 297:
   Met Glu Lys Lys Ile Thr Gly Tyr Thr Thr Val Asp Ile Ser Gln Trp
    1               5                  10                  15
   His Arg Lys Glu His Phe Glu Ala Phe Gln Ser Val Ala Gln Cys Thr
               20                  25                  30
   Tyr Asn Gln Thr Val Gln Leu Asp Ile Thr Ala Phe Leu Lys Thr Val
           35                  40                  45
   Lys Lys Asn Lys His Lys Phe Tyr Pro Ala Phe Ile His Ile Leu Ala
       50                  55                  60
   Arg Leu Met Asn Ala His Pro Glu Phe Arg Met Ala Met Lys Asp Gly
   65                  70                  75                  80
   Glu Leu Val Ile Trp Asp Ser Val His Pro Cys Tyr Thr Val Phe His
                   85                  90                  95
   Glu Gln Thr Glu Thr Phe Ser Ser Leu Trp Ser Glu Tyr His Asp Asp
               100                 105                 110
   Phe Arg Gln Phe Leu His Ile Tyr Ser Gln Asp Val Ala Cys Tyr Gly
           115                 120                 125
   Glu Asn Leu Ala Tyr Phe Pro Lys Gly Phe Ile Glu Asn Met Phe Phe
       130                 135                 140
   Val Ser Ala Asn Pro Trp Val Ser Phe Thr Ser Phe Asp Leu Asn Val
   145                 150                 155                 160
   Ala Asn Met Asp Asn Phe Phe Ala Pro Val Phe Thr Met Gly Lys Tyr
                   165                 170                 175
   Tyr Thr Gln Gly Asp Lys Val Leu Met Pro Leu Ala Ile Gln Val His
               180                 185                 190
   His Ala Val Cys Asp Gly Phe His Val Gly Arg Met Leu Asn Glu Leu
           195                 200                 205
   Gln Gln Tyr Cys Asp Glu Trp Gln Gly Gly Ala
       210                 215
(2) INFORMATION FOR SEQ ID NO: 298:
   (i) SEQUENCE CHARACTERISTICS:
      (A) LENGTH: 299 amino acids
      (B) TYPE: amino acid
      (D) TOPOLOGY: linear
   (ii) MOLECULE TYPE: protein
   (xi) SEQUENCE DESCRIPTION: SEQ ID NO: 298:
(2) INFORMATION FOR SEQ ID NO: 299:
   (i) SEQUENCE CHARACTERISTICS:
      (A) LENGTH: 2728 base pairs
      (B) TYPE: nucleic acid
      (C) STRANDEDNESS: double
      (D) TOPOLOGY: circular
   (ii) MOLECULE TYPE: other nucleic acid
      (A) DESCRIPTION: /desc = "synthetic vector"
   (ix) FEATURE:
      (A) NAME/KEY: CDS
      (B) LOCATION:complement (471..1367)
      (D) OTHER INFORMATION:/product= "bla resistance"
   (xi) SEQUENCE DESCRIPTION: SEQ ID NO: 299:
   GATCTCATAA CTTCGTATAA TGTATGCTAT ACGAAGTTAT GACGTCTAAT GTGAGTTAGC    60
   TCACTCATTA GGCACCCCAG GCTTTACACT TTATGCTTCC GGCTCGTATG TTGTGTGGAA   120
   TTGTGAGCGG ATAACAATTT CACACAGGAA ACAGCTATGA CCATGATTAC GAATTTCTAG   180
   ACCCCCCCCC CGCATGCCAT AACTTCGTAT AATGTACGCT ATACGAAGTT ATAAGCTTGA   240
   CCTGTGAAGT GAAAAATGGC GCAGATTGTG CGACATTTTT TTTGTCTGCC GTTTAATTAA 300
   GGGGGGGGGC CGGCCATTAT CAAAAAGGAT CTCAAGAAGA TCCTTTGATC TTTTCTACGG 360
   GGTCTGACGC TCAGTGGAAC GAAAACTCAC GTTAAGGGAT TTTGGTCATG AGATTATCAA 420
   AAAGGATCTT CACCTAGATC CTTTTAAATT AAAAATGAAG TTTTAAATCA ATCTAAAGTA 480
   TATATGAGTA AACTTGGTCT GACAGTTACC CAATGCTTAA TCAGTGAGGC ACCTATCTCA 540
   GCGATCTGTC TATTTCGTTC ATCCATAGTT GCCTGACTCC CCGTCGTGTA GATAACTACG 600
   ATACGGGAGG GCTTACCATC TGGCCCCAGT GCTGCAATGA TACCGCGAGA CCCACGCTCA 660
   CCGGCTCCAG ATTTATCAGC AATAAACCAG CCAGCCGGAA GGGCCGAGCG CAGAAGTGGT 720
   CCTGCAACTT TATCCGCCTC CATCCAGTCT ATTAACTGTT GCCGGGAAGC TAGAGTAAGT 780
   AGTTCGCCAG TTAATAGTTT GCGCAACGTT GTTGCCATTG CTACAGGCAT CGTGGTGTCA 840
   CGCTCGTCGT TTGGTATGGC TTCATTCAGC TCCGGTTCCC AACGATCAAG GCGAGTTACA 900
   TGATCCCCCA TGTTGTGCAA AAAAGCGGTT AGCTCCTTCG GTCCTCCGAT CGTTGTCAGA 960
   AGTAAGTTGG CCGCAGTGTT ATCACTCATG GTTATGGCAG CACTGCATAA TTCTCTTACT 1020
   GTCATGCCAT CCGTAAGATG CTTTTCTGTG ACTGGTGAGT ACTCAACCAA GTCATTCTGA 1080
   GAATAGTGTA TGCGGCGACC GAGTTGCTCT TGCCCGGCGT CAATACGGGA TAATACCGCG 1140
   CCACATAGCA GAACTTTAAA AGTGCTCATC ATTGGAAAAC GTTCTTCGGG GCGAAAACTC 1200
   TCAAGGATCT TACCGCTGTT GAGATCCAGT TCGATGTAAC CCACTCGCGC ACCCAACTGA 1260
   TCCTCAGCAT CTTTTACTTT CACCAGCGTT TCTGGGTGAG CAAAAACAGG AAGGCAAAAT 1320
   GCCGCAAAAA AGGGAATAAG GGCGACACGG AAATGTTGAA TACTCATACT CTTCCTTTTT 1380
   CAATATTATT GAAGCATTTA TCAGGGTTAT TGTCTCATGA GCGGATACAT ATTTGAATGT 1440
   ACATGAAATT GTAAACGTTA ATATTTTGTT AAAATTCGCG TTAAATTTTT GTTAAATCAG 1500
   CTCATTTTTT AACCAATAGG CCGAAATCGG CAAAATCCCT TATAAATCAA AAGAATAGAC 1560
   CGAGATAGGG TTGAGTGTTG TTCCAGTTTG GAACAAGAGT CCACTATTAA AGAACGTGGA 1620
   CTCCAACGTC AAAGGGCGAA AAACCGTCTA TCAGGGCGAT GGCCCACTAC GAGAACCATC 1680
   ACCCTAATCA AGTTTTTTGG GGTCGAGGTG CCGTAAAGCA CTAAATCGGA ACCCTAAAGG 1740
   GAGCCCCCGA TTTAGAGCTT GACGGGGAAA GCCGGCGAAC GTGGCGAGAA AGGAAGGGAA 1800
   GAAAGCGAAA GGAGCGGGCG CTAGGGCGCT GGCAAGTGTA GCGGTCACGC TGCGCGTAAC 1860
   CACCACACCC GCCGCGCTTA ATGCGCCGCT ACAGGGCGCG TGCTAGCGGA GTGTATACTG 1920
   GCTTACTATG TTGGCACTGA TGAGGGTGTC AGTGAAGTGC TTCATGTGGC AGGAGAAAAA 1980
   AGGCTGCACC GGTGCGTCAG CAGAATATGT GATACAGGAT ATATTCCGCT TCCTCGCTCA 2040
   CTGACTCGCT ACGCTCGGTC GTTCGACTGC GGCGAGCGGA AATGGCTTAC GAACGGGGCG 2100
   GAGATTTCCT GGAAGATGCC AGGAAGATAC TTAACAGGGA AGTGAGAGGG CCGCGGCAAA 2160
   GCCGTTTTTC CATAGGCTCC GCCCCCCTGA CAAGCATCAC GAAATCTGAC GCTCAAATCA 2220
   GTGGTGGCGA AACCCGACAG GACTATAAAG ATACCAGGCG TTTCCCCCTG GCGGCTCCCT 2280
   CCTGCGCTCT CCTGTTCCTG CCTTTCGGTT TACCGGTGTC ATTCCGCTGT TATGGCCGCG 2340
   TTTGTCTCAT TCCACGCCTG ACACTCAGTT CCGGGTAGGC AGTTCGCTCC AAGCTGGACT 2400
   GTATGCACGA ACCCCCCGTT CAGTCCGACC GCTGCGCCTT ATCCGGTAAC TATCGTCTTG 2460
   AGTCCAACCC GGAAAGACAT GCAAAAGCAC CACTGGCAGC AGCCACTGGT AATTGATTTA 2520
   GAGGAGTTAG TCTTGAAGTC ATGCGCCGGT TAAGGCTAAA CTGAAAGGAC AAGTTTTAGT 2580
   GACTGCGCTC CTCCAAGCCA GTTACCTCGG TTCAAAGAGT TGGTAGCTCA GAGAACCTAC 2640
   GAAAAACCGC CCTGCAAGGC GGTTTTTTCG TTTTCAGAGC AAGAGATTAC GCGCAGACCA 2700
   AAACGATCTC AAGAAGATCA TCTTATTA 2728
(2) INFORMATION FOR SEQ ID NO: 300:
   (i) SEQUENCE CHARACTERISTICS:
      (A) LENGTH: 299 amino acids
      (B) TYPE: amino acid
      (D) TOPOLOGY: linear
   (ii) MOLECULE TYPE: protein
   (xi) SEQUENCE DESCRIPTION: SEQ ID NO: 300:
   Met Ser Ile Gln His Phe Arg Val Ala Leu Ile Pro Phe Phe Ala Ala
   1 5 10 15
   Phe Cys Leu Pro Val Phe Ala His Pro Glu Thr Leu Val Lys Val Lys
   20 25 30
   Asp Ala Glu Asp Gln Leu Gly Ala Arg Val Gly Tyr Ile Glu Leu Asp
   35 40 45
   Leu Asn Ser Gly Lys Ile Leu Glu Ser Phe Arg Pro Glu Glu Arg Phe
   50 55 60
   Pro Met Met Ser Thr Phe Lys Val Leu Leu Cys Gly Ala Val Leu Ser
   65 70 75 80
   Arg Ile Asp Ala Gly Gln Glu Gln Leu Gly Arg Arg Ile His Tyr Ser
   85 90 95
   Gln Asn Asp Leu Val Glu Tyr Ser Pro Val Thr Glu Lys His Leu Thr
   100 105 110
   Asp Gly Met Thr Val Arg Glu Leu Cys Ser Ala Ala Ile Thr Met Ser
   115 120 125
   Asp Asn Thr Ala Ala Asn Leu Leu Leu Thr Thr Ile Gly Gly Pro Lys
   130 135 140
   Glu Leu Thr Ala Phe Leu His Asn Met Gly Asp His Val Thr Arg Leu
   145 150 155 160
   Asp Arg Trp Glu Pro Glu Leu Asn Glu Ala Ile Pro Asn Asp Glu Arg
   165 170 175
   Asp Thr Thr Met Pro Val Ala Met Ala Thr Thr Leu Arg Lys Leu Leu
   180 185 190
   Thr Gly Glu Leu Leu Thr Leu Ala Ser Arg Gln Gln Leu Ile Asp Trp
   195 200 205
   Met Glu Ala Asp Lys Val Ala Gly Pro Leu Leu Arg Ser Ala Leu Pro
   210 215 220
   Ala Gly Trp Phe Ile Ala Asp Lys Ser Gly Ala Gly Glu Arg Gly Ser
   225 230 235 240
   Arg Gly Ile Ile Ala Ala Leu Gly Pro Asp Gly Lys Pro Ser Arg Ile
   245 250 255
   Val Val Ile Tyr Thr Thr Gly Ser Gln Ala Thr Met Asp Glu Arg Asn
   260 265 270
   Arg Gln Ile Ala Glu Ile Gly Ala Ser Leu Ile Lys His Trp Val Thr
   275 280 285
   Val Arg Pro Ser Leu Leu Ile Tyr Thr Leu Asp
   290 295
(2) INFORMATION FOR SEQ ID NO: 301:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 301:
      TATGAGATCT CATAACTTCG TATAATGTAC GCTATACGAA GTTAT 45
(2) INFORMATION FOR SEQ ID NO: 302:
   (A) LENGTH: 45 base pairs
   (B) TYPE: nucleic acid
   (C) STRANDEDNESS: single
   (D) TOPOLOGY: linear

   (ii) MOLECULE TYPE: other nucleic acid
      (A) DESCRIPTION: /desc = "synthetic oligonucleotide"
   (xi) SEQUENCE DESCRIPTION: SEQ ID NO: 302:
      TAATAACTTC GTATAGCATA CATTATACGA AGTTATGAGA TCTCA 45
(2) INFORMATION FOR SEQ ID NO: 303:
   (i) SEQUENCE CHARACTERISTICS:
      (A) LENGTH: 91 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 303:
   CATTTTTTGC CCTCGTTATC TACGCATGCG ATAACTTCGT ATAGCGTACA TTATACGAAG 60
   TTATTCTAGA CATGGTCATA GCTGTTTCCT G 91
(2) INFORMATION FOR SEQ ID NO: 304:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 304:
      GGGGGGAATT CGGTGGTGGT GGATCTGCGT GCGCTGAAAC GGTTGAAAGT TG 52
(2) INFORMATION FOR SEQ ID NO: 305:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 305:
      CCCCCCCAAG CTTATCAAGA CTCCTTATTA CG 32
(2) INFORMATION FOR SEQ ID NO: 306:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 306:
      GGGGGGGGAA TTCGGAGGCG GTTCCGGTGG TGGC 34
(2) INFORMATION FOR SEQ ID NO: 307:
   (i) SEQUENCE CHARACTERISTICS:
      (A) LENGTH: 74 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 307:
   GGGGGGGGAA TTCGAGCAGA AGCTGATCTC TGAGGAGGAT CTGTAGGGTG GTGGCTCTGG 60
   TTCCGGTGAT TTTG 74
(2) INFORMATION FOR SEQ ID NO: 308:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 308:
      CCATAACTTC GTATAATGTA CGCTATACGA AGTTATA 37
(2) INFORMATION FOR SEQ ID NO: 309:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 309:
      AGCTTATAAC TTCGTATAGC GTACATTATA CGAAGTTATG GCATG 45
(2) INFORMATION FOR SEQ ID NO: 310:
   (i) SEQUENCE CHARACTERISTICS:
      (A) LENGTH: 76 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 310:
   AGCTTGACCT GTGAAGTGAA AAATGGCGCA GATTGTGCGA CATTTTTTTT GTCTGCCGTT 60
   TAATTAAAGG GGGGGT 76
(2) INFORMATION FOR SEQ ID NO: 311:
   (i) SEQUENCE CHARACTERISTICS:
      (A) LENGTH: 75 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 311:
   GTACACCCCC CCCCAGGCCG GCCCCCCCCC CCCTTTAATT AAACGGCAGA CAAAAAAAAT 60
   GTCGCACAAT CTGCG 75
(2) INFORMATION FOR SEQ ID NO: 312:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 312:
      GGGGGGGTGT ACATTCAAAT ATGTATCCGC TCATG 35
(2) INFORMATION FOR SEQ ID NO: 313:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 313:
      GGGTTACATC GAACTGGATC TC 22
(2) INFORMATION FOR SEQ ID NO: 314:
   (i) SEQUENCE CHARACTERISTICS:
      (A) LENGTH: 59 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 314:
      CCAGTTCGAT GTAACCCACT CGCGCACCCA ACTGATCCTC AGCATCTTTT ACTTTCACC 59
(2) INFORMATION FOR SEQ ID NO: 315:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 315:
      ACTCTAGCTT CCCGGCAACA GTTAATAGAC TGGATGGAGG CGG 43
(2) INFORMATION FOR SEQ ID NO: 316:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 316:
      CTGTTGCCGG GAAGCTAGAG TAAG 24
(2) INFORMATION FOR SEQ ID NO: 317:
   (i) SEQUENCE CHARACTERISTICS:
      (A) LENGTH: 58 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 317:
      CCCCCCCTTA ATTAAGGGGG GGGGCCGGCC ATTATCAAAA AGGATCTCAA GAAGATCC 58
(2) INFORMATION FOR SEQ ID NO: 318:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 318:
      GGGGGGGGCT AGCACGCGCC CTGTAGCGGC GCATTAA 37
(2) INFORMATION FOR SEQ ID NO: 319:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 319:
      CCCCCCCTGT ACATGAAATT GTAAACGTTA ATATTTTG 38
(2) INFORMATION FOR SEQ ID NO: 320:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 320:
      GGGCGATGGC CCACTACGAG AACCATCACC CTAATC 36
(2) INFORMATION FOR SEQ ID NO: 321:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 321:
      GGGGGGAGAT CTAATAAGAT GATCTTCTTG AG 32
(2) INFORMATION FOR SEQ ID NO: 322:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 322:
      GAGTTGGTAG CTCAGAGAAC CTACGAAAAA CCGCCCTGCA AGGCG 45
(2) INFORMATION FOR SEQ ID NO: 323:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 323:
      GTAGGTTCTC TGAGCTACCA ACTC 24
(2) INFORMATION FOR SEQ ID NO: 324:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 324:
      GTTTCCCCCT GGCGGCTCCC TCCTGCGCTC TCCTGTTCCT GCC 43
(2) INFORMATION FOR SEQ ID NO: 325:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 325:
      AGGAGGGAGC CGCCAGGGGG AAAC 24
(2) INFORMATION FOR SEQ ID NO: 326:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 326:
      GACATCAGCG CTAGCGGAGT GTATAC 26
(2) INFORMATION FOR SEQ ID NO: 327:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 327:
      GATCTCATAA CTTCGTATAA TGTATGCTAT ACGAAGTTAT TCA 43
(2) INFORMATION FOR SEQ ID NO: 328:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 328:
      GATCTGAATA ACTTCGTATA GCATACATTA TACGAAGTTA TGAGA 45
(2) INFORMATION FOR SEQ ID NO: 329:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 329:
      GGGGGGGAGA TCTGACCAAA ATCCCTTAAC GTGAG 35
(2) INFORMATION FOR SEQ ID NO: 330:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 330:
      GGTATCTGCG CTCTGCTGTA GCCAGTTACC TTCGG 35
(2) INFORMATION FOR SEQ ID NO: 331:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 331:
      CCCCCCCGCT AGCCATGTGA GCAAAAGGCC AGCAA 35
(2) INFORMATION FOR SEQ ID NO: 332:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 332:
      GGGACGTCGG GTGAGGTTCC AAC 23
(2) INFORMATION FOR SEQ ID NO: 333:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 333:
      CCATACGGAA CTCCGGGTGA GCATTCATC 29
(2) INFORMATION FOR SEQ ID NO: 334:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 334:
      CCGGAGTTCC GTATGG 16
(2) INFORMATION FOR SEQ ID NO: 335:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 335:
      ACGTTTAAAT CAAAACTGG 19
(2) INFORMATION FOR SEQ ID NO: 336:
   (i) SEQUENCE CHARACTERISTICS:
      (A) LENGTH: 69 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 336:
   CCAGTTTTGA TTTAAACGTA GCCAATATGG ACAACTTCTT CGCCCCCGTT TTCACTATGG 60
   GCAAATATT 69
(2) INFORMATION FOR SEQ ID NO: 337:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 337:
      GGAAGATCTA GCACCAGGCG TTTAAG 26
(2) INFORMATION FOR SEQ ID NO: 338:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 338:
      GAGGCCGGCC ATCGAATGGC GCAAAAC 27
(2) INFORMATION FOR SEQ ID NO: 339:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 339:
      CGCGTACCGT CCTCATGGGA GAAAATAATA C 31
(2) INFORMATION FOR SEQ ID NO: 340:
   (i) SEQUENCE CHARACTERISTICS:
      (A) LENGTH: 83 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 340:
   CCATGAGGAC GGTACGCGAC TGGGCGTGGA GCATCTGGTC GCATTGGGTC ACCAGCAAAT 60
   CCGCTGTTAG CTGGCCCATT AAG 83
(2) INFORMATION FOR SEQ ID NO: 341:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 341:
      GTCAGCGGCG GGATATAACA TGAGCTGTCC TCGGTATCGT CG 42
(2) INFORMATION FOR SEQ ID NO: 342:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 342:
      GTTATATCCC GCCGCTGACC ACCATCAAAC 30
(2) INFORMATION FOR SEQ ID NO: 343:
   (i) SEQUENCE CHARACTERISTICS:
      (A) LENGTH: 65 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (ix) FEATURE:
      (A) NAME/KEY: conflict
      (B) LOCATION:replace (42..44, "")
      (D) OTHER INFORMATION:/note= "in Fig.35b, M41, LAC6: T4T; but see Fig.35a, M41: LAC6 pos.1055-1119 on complementary strand, 1076 to 1078: TAT"
   (xi) SEQUENCE DESCRIPTION: SEQ ID NO: 343:
   CATCAGTGAA TCGGCCAACG CGCGGGGAGA GGCGGTTTGC GTATTGGGAG CCAGGGTGGT 60
   TTTTC 65
(2) INFORMATION FOR SEQ ID NO: 344:
   (i) SEQUENCE CHARACTERISTICS:
      (A) LENGTH: 73 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 344:
   GGTTAATTAA CCTCACTGCC CGCTTTCCAG TCGGGAAACC TGTCGTGCCA GCTGCATCAG 60
   TGAATCGGCC AAC 73
(2) INFORMATION FOR SEQ ID NO: 345:
   (i) SEQUENCE CHARACTERISTICS:
      (A) LENGTH: 50 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 345:
      CTAGACTAGT GTTTAAACCG GACCGGGGGG GGGCTTAAGG GGGGGGGGGG 50
(2) INFORMATION FOR SEQ ID NO: 346:
   (i) SEQUENCE CHARACTERISTICS:
      (A) LENGTH: 50 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 346:
      CTAGCCCCCC CCCCCCTTAA GCCCCCCCCC GGTCCGGTTT AAACACTAGT 50
(2) INFORMATION FOR SEQ ID NO: 347:
   (i) SEQUENCE CHARACTERISTICS:
      (A) LENGTH: 50 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 347:
      CTAGACTAGT GTTTAAACCG GACCGGGGGG GGGCTTAAGG GGGGGGGGGG 50
(2) INFORMATION FOR SEQ ID NO: 348:
   (i) SEQUENCE CHARACTERISTICS:
      (A) LENGTH: 82 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 348:
   CCCCCCCTTA AGTGGGCTGC AAAACAAAAC GGCCTCCTGT CAGGAAGCCG CTTTTATCGG 60
   GTAGCCTCAC TGCCCGCTTT CC 82
(2) INFORMATION FOR SEQ ID NO: 349:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 349:
      GTTGTTGTGC CACGCGGTTA GGAATGTAAT TCAGCTCCGC 40
(2) INFORMATION FOR SEQ ID NO: 350:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 350:
      AACCGCGTGG CACAACAAC 19
(2) INFORMATION FOR SEQ ID NO: 351:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 351:
      CTTCGTTCTA CCATCGACAC GACCACGCTG GCACCCAGTT G 41
(2) INFORMATION FOR SEQ ID NO: 352:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 352:
      GTGTCGATGG TAGAACGAAG 20
(2) INFORMATION FOR SEQ ID NO: 353:
   (i) SEQUENCE CHARACTERISTICS:
      (A) LENGTH: 67 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 353:
   CCACAGCAAT AGCATCCTGG TCATCCAGCG GATAGTTAAT AATCAGCCCA CTGACACGTT 60
   GCGCGAG 67
(2) INFORMATION FOR SEQ ID NO: 354:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 354:
      GACCAGGATG CTATTGCTGT GG 22
(2) INFORMATION FOR SEQ ID NO: 355:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 355:
      CAGCGCGATT TGCTGGTGGC CCAATGCGAC CAGATGC 37
(2) INFORMATION FOR SEQ ID NO: 356:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 356:
      CACCAGCAAA TCGCGCTG 18
(2) INFORMATION FOR SEQ ID NO: 357:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 357:
      CCCGGACTCG GTAATGGCAC GCATTGCGCC CAGCGCC 37
(2) INFORMATION FOR SEQ ID NO: 358:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 358:
      GCCATTACCG AGTCCGGG 18
(2) INFORMATION FOR SEQ ID NO: 359:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 359:
      AATTCCACCA TCATCACCAT TGACGTCTA 29
(2) INFORMATION FOR SEQ ID NO: 360:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 360:
      AGCTTAGACG TCAATGGTGA TGATGGTGG 29
(2) INFORMATION FOR SEQ ID NO: 361:
   (i) SEQUENCE CHARACTERISTICS:
      (A) LENGTH: 1289 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic gene cassette"
   (ix) FEATURE:
      (A) NAME/KEY: CDS
      (B) LOCATION:complement (280..1137)
      (D) OTHER INFORMATION:/product= "bla resistance"
   (xi) SEQUENCE DESCRIPTION: SEQ ID NO: 361:
   CGCGTTAACC TCAGGTGACC AAGCCCCTGG CCAAGGTCCC GTACGTTCGA AGATTACCAT    60
   CACGTGGATC CGGTACCAGG CCGGCCATTA TCAAAAAGGA TCTCAAGAAG ATCCTTTGAT   120
   CTTTTCTACG GGGTCTGACG CTCAGTGGAA CGAAAACTCA CGTTAAGGGA TTTTGGTCAT   180
   GAGATTATCA AAAAGGATCT TCACCTAGAT CCTTTTAAAT TAAAAATGAA GTTTTAAATC   240
   AATCTAAAGT ATATATGAGT AAACTTGGTC TGACAGTTAC CAATGCTTAA TCAGTGAGGC   300
   ACCTATCTCA GCGATCTGTC TATTTCGTTC ATCCATAGTT GCCTGACTCC CCGTCGTGTA   360
   GATAACTACG ATACGGGAGG GCTTACCATC TGGCCCCAGT GCTGCAATGA TACCGCGAGA   420
   CCCACGCTCA CCGGCTCCAG ATTTATCAGC AATAAACCAG CCAGCCGGAA GGGCCGAGCG   480
   CAGAAGTGGT CCTGCAACTT TATCCGCCTC CATCCAGTCT ATTAACTGTT GCCGGGAAGC   540
   TAGAGTAAGT AGTTCGCCAG TTAATAGTTT GCGCAACGTT GTTGCCATTG CTACAGGCAT   600
   CGTGGTGTCA CGCTCGTCGT TTGGTATGGC TTCATTCAGC TCCGGTTCCC AACGATCAAG   660
   GCGAGTTACA TGATCCCCCA TGTTGTGCAA AAAAGCGGTT AGCTCCTTCG GTCCTCCGAT   720
   CGTTGTCAGA AGTAAGTTGG CCGCAGTGTT ATCACTCATG GTTATGGCAG CACTGCATAA   780
   TTCTCTTACT GTCATGCCAT CCGTAAGATG CTTTTCTGTG ACTGGTGAGT ACTCAACCAA   840
   GTCATTCTGA GAATAGTGTA TGCGGCGACC GAGTTGCTCT TGCCCGGCGT CAATACGGGA   900
   TAATACCGCG CCACATAGCA GAACTTTAAA AGTGCTCATC ATTGGAAAAC GTTCTTCGGG   960
   GCGAAAACTC TCAAGGATCT TACCGCTGTT GAGATCCAGT TCGATGTAAC CCACTCGTGC  1020
   ACCCAACTGA TCTTCAGCAT CTTTTACTTT CACCAGCGTT TCTGGGTGAG CAAAAACAGG  1080
   AAGGCAAAAT GCCGCAAAAA AGGGAATAAG GGCGACACGG AAATGTTGAA TACTCATACT  1140
   CTTCCTTTTT CAATATTATT GAAGCATTTA TCAGGGTTAT TGTCTCATGA GCGGATACAT  1200
   ATTTGAATGT ACTCGGCCGC ACGAGCTGCA GGCGCCATTA ATGGCTCGAG CGCGCTTCAG  1260
   CGCTTTGTCT TCCGGATGTA CATGAAATT                                    1289
(2) INFORMATION FOR SEQ ID NO: 362:
   (i) SEQUENCE CHARACTERISTICS:
      (A) LENGTH: 286 amino acids
      (B) TYPE: amino acid
      (D) TOPOLOGY: linear
   (ii) MOLECULE TYPE: protein
   (xi) SEQUENCE DESCRIPTION: SEQ ID NO: 362:
   Met Ser Ile Gln His Phe Arg Val Ala Leu Ile Pro Phe Phe Ala Ala
   1               5                   10                  15
   Phe Cys Leu Pro Val Phe Ala His Pro Glu Thr Leu Val Lys Val Lys
               20                  25                  30
   Asp Ala Glu Asp Gln Leu Gly Ala Arg Val Gly Tyr Ile Glu Leu Asp
           35                  40                  45
   Leu Asn Ser Gly Lys Ile Leu Glu Ser Phe Arg Pro Glu Glu Arg Phe
       50                  55                  60
   Pro Met Met Ser Thr Phe Lys Val Leu Leu Cys Gly Ala Val Leu Ser
   65                  70                  75                  80
   Arg Ile Asp Ala Gly Gln Glu Gln Leu Gly Arg Arg Ile His Tyr Ser
                   85                  90                  95
   Gln Asn Asp Leu Val Glu Tyr Ser Pro Val Thr Glu Lys His Leu Thr
               100                 105                 110
   Asp Gly Met Thr Val Arg Glu Leu Cys Ser Ala Ala Ile Thr Met Ser
           115                 120                 125
   Asp Asn Thr Ala Ala Asn Leu Leu Leu Thr Thr Ile Gly Gly Pro Lys
       130                 135                 140
   Glu Leu Thr Ala Phe Leu His Asn Met Gly Asp His Val Thr Arg Leu
   145                 150                 155                 160
   Asp Arg Trp Glu Pro Glu Leu Asn Glu Ala Ile Pro Asn Asp Glu Arg
                   165                 170                 175
   Asp Thr Thr Met Pro Val Ala Met Ala Thr Thr Leu Arg Lys Leu Leu
               180                 185                 190
   Thr Gly Glu Leu Leu Thr Leu Ala Ser Arg Gln Gln Leu Ile Asp Trp
           195                 200                 205
   Met Glu Ala Asp Lys Val Ala Gly Pro Leu Leu Arg Ser Ala Leu Pro
       210                 215                 220
   Ala Gly Trp Phe Ile Ala Asp Lys Ser Gly Ala Gly Glu Arg Gly Ser
   225                 230                 235                 240
   Arg Gly Ile Ile Ala Ala Leu Gly Pro Asp Gly Lys Pro Ser Arg Ile
                   245                 250                 255
   Val Val Ile Tyr Thr Thr Gly Ser Gln Ala Thr Met Asp Glu Arg Asn
               260                 265                 270
   Arg Gln Ile Ala Glu Ile Gly Ala Ser Leu Ile Lys His Trp
           275                 280                 285
(2) INFORMATION FOR SEQ ID NO: 363:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 363:
      GCCCTGCAAG CGGAAGAC 18
(2) INFORMATION FOR SEQ ID NO: 364:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO: 364:
      GGCTTTCGAA TGGCCAAAGG 20
(2) INFORMATION FOR SEQ ID NO: 365:
   (i) SEQUENCE CHARACTERISTICS:
      (A) LENGTH: 81 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide library"
   (ix) FEATURE:
      (A) NAME/KEY: misc_feature
      (B) LOCATION:25..27
      (D) OTHER INFORMATION:/product= "random codon by trinucleotide mutagenesis (ACT/GTT)"
   (ix) FEATURE:
      (A) NAME/KEY: misc_feature
      (B) LOCATION:37..39
      (D) OTHER INFORMATION:/product= "random codon by trinucleotides (TTT,CAT,CTT,ATG,CAG)"
   (ix) FEATURE:
      (A) NAME/KEY: misc_feature
      (B) LOCATION:43..45
      (D) OTHER INFORMATION:/product= "random codon by trinucleotides (18 codons, no Pro, no Cys)"
   (ix) FEATURE:
      (A) NAME/KEY: misc_feature
      (B) LOCATION:46..48
      (D) OTHER INFORMATION:/product= "random codon by trinucleotides (GAT, GGT, AAT, TCT, TAT)"
   (ix) FEATURE:
      (A) NAME/KEY: misc_feature
      (B) LOCATION:49..51
      (D) OTHER INFORMATION:/product= "random codon by trinucleotides (GAT, GGT, AAT, TCT)"
   (ix) FEATURE:
      (A) NAME/KEY: misc_feature
      (B) LOCATION:52..54
      (D) OTHER INFORMATION:/product= "random codon by trinucleotide mutagenesis (19aa, no Cys)"
   (ix) FEATURE:
      (A) NAME/KEY: misc_feature
      (B) LOCATION:55..57
      (D) OTHER INFORMATION:/product= "random codon by trinucleotides (CCT/TCT)"
   (ix) FEATURE:
      (A) NAME/KEY: misc_feature
      (B) LOCATION:58..60
      (D) OTHER INFORMATION:/product= "random codon by trinucleotide mutagenesis (19 aa, no Cys)"
   (xi) SEQUENCE DESCRIPTION: SEQ ID NO: 365:
   GCCCTGCAAG CGGAAGACTT TGCGRYTTAT TATTGCHWKC AGNNKDVTDV TNNKYCTNNK 60
   ACCTTTGGCC ATTCGAAAGC C 81
(2) INFORMATION FOR SEQ ID NO: 366:
   (i) SEQUENCE CHARACTERISTICS:
      (A) LENGTH: 81 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide library"
   (ix) FEATURE:
      (A) NAME/KEY: misc_feature
      (B) LOCATION:37..39
      (D) OTHER INFORMATION:/product= "random codon by trinucleotides (TTT,CAT,CTT,ATG,CAG)"
   (ix) FEATURE:
      (A) NAME/KEY: misc_feature
      (B) LOCATION:43..45
      (D) OTHER INFORMATION:/product= "random codon by trinucleotides (18 codons, no Pro, no Cys)"
   (ix) FEATURE:
      (A) NAME/KEY: misc_feature
      (B) LOCATION:46..48
      (D) OTHER INFORMATION:/product= "random codon by trinucleotides (GAT, GGT, AAT, TCT, TAT)"
   (ix) FEATURE:
      (A) NAME/KEY: misc_feature
      (B) LOCATION:49..51
      (D) OTHER INFORMATION:/product= "random codon by trinucleotides (GAT, GGT, AAT, TCT)"
   (ix) FEATURE:
      (A) NAME/KEY: misc_feature
      (B) LOCATION:52..54
      (D) OTHER INFORMATION:/product= "random codon by trinucleotide mutagenesis (19aa, no Cys)"
   (ix) FEATURE:
      (A) NAME/KEY: misc_feature
      (B) LOCATION:55..57
      (D) OTHER INFORMATION:/product= "random codon by trinucleotides (CCT/TCT)"
   (ix) FEATURE:
      (A) NAME/KEY: misc_feature
      (B) LOCATION:5B..60
      (D) OTHER INFORMATION:/product= "random codon by trinucleotide mutagenesis (19 aa, no Cys)"
   (xi) SEQUENCE DESCRIPTION: SEQ ID NO: 366:
   GCCCTGCAAG CGGAAGACGT GGGCGTGTAT TATTGCHWKC AGNNKDVTDV TNNKYCTNNK    60
   ACCTTTGGCC ATTCGAAAGC C                                              81
(2) INFORMATION FOR SEQ ID NO: 367:
   (i) SEQUENCE CHARACTERISTICS:
      (A) LENGTH: 81 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide library"
   (ix) FEATURE:
      (A) NAME/KEY: misc_feature
      (B) LOCATION:37..39
      (D) OTHER INFORMATION:/product= "random codon by trinucleotides (TTT,CAT,CTT,ATG,CAG)"
   (ix) FEATURE:
      (A) NAME/KEY: misc_feature
      (B) LOCATION:43..45
      (D) OTHER INFORMATION:/product= "random codon by trinucleotides (18 codons, no Pro, no Cys)"
   (ix) FEATURE:
      (A) NAME/KEY: misc_feature
      (B) LOCATION:46..48
      (D) OTHER INFORMATION:/product= "random codon by trinucleotides (GAT, GGT, AAT, TCT, TAT)"
   (ix) FEATURE:
      (A) NAME/KEY: misc_feature
      (B) LOCATION:49..51
      (D) OTHER INFORMATION:/product= "random codon by trinucleotides (GAT, GGT, AAT, TCT)"
   (ix) FEATURE:
      (A) NAME/KEY: misc_feature
      (B) LOCATION:52..54
      (D) OTHER INFORMATION:/product= "random codon by trinucleotide mutagenesis (19aa, no Cys)"
   (ix) FEATURE:
      (A) NAME/KEY: misc_feature
      (B) LOCATION:55..57
      (D) OTHER INFORMATION:/product= "random codon by trinucleotides (CCT/TCT)"
   (ix) FEATURE:
      (A) NAME/KEY: misc_feature
      (B) LOCATION:58..60
      (D) OTHER INFORMATION:/product= "random codon by trinucleotide mutagenesis (19 aa, no Cys)"
   (xi) SEQUENCE DESCRIPTION: SEQ ID NO: 367:
   GCCCTGCAAG CGGAAGACGT GGCGGTGTAT TATTGCHWKC AGNNKDVTDV TNNKYCTNNK 60
   ACCTTTGGCC ATTCGAAAGC C 81
(2) INFORMATION FOR SEQ ID NO: 368:
   (i) SEQUENCE CHARACTERISTICS:
      (A) LENGTH: 108 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide library"
   (ix) FEATURE:
      (A) NAME/KEY: misc_feature
      (B) LOCATION:41..43
      (D) OTHER INFORMATION:/product= "random codon by trinucleotides (CGT, TGG, TAT)"
   (ix) FEATURE:
      (A) NAME/KEY: misc_feature
      (B) LOCATION:47..61
      (D) OTHER INFORMATION:/product= "random codons by trinucleotides (18 aa, no Trp, no Cys)"
   (ix) FEATURE:
      (A) NAME/KEY: misc_feature
      (B) LOCATION:62..64
      (D) OTHER INFORMATION:/product= "random codon by trinucleotide mutagenesis (19aa, no Cys)"
   (xi) SEQUENCE DESCRIPTION: SEQ ID NO: 368:
   CCTGCAAGCG GAAGACGAAG CGGATTATTA TTGCCAGAGC YRKGACNNKN NKNNKNNKNN     60
   KNNKGGCGGC GGCACGAAGT TAACCGTTCT TGGCCAGGAA TTCGAGCC                 108
(2) INFORMATION FOR SEQ ID NO: 369:
   (i) SEQUENCE CHARACTERISTICS:
      (A) LENGTH: 105 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide library"
   (ix) FEATURE:
      (A) NAME/KEY: misc_feature
      (B) LOCATION:41..43
      (D) OTHER INFORMATION:/product= "random codon by trinucleotides (CGT, TGG, TAT)"
   (ix) FEATURE:
      (A) NAME/KEY: misc_feature
      (B) LOCATION:47..58
      (D) OTHER INFORMATION:/product= "random codons by trinucleotides (18 aa, no Trp, no Cys)"
   (ix) FEATURE:
      (A) NAME/KEY: misc_feature
      (B) LOCATION:59..61
      (D) OTHER INFORMATION:/product= "random codon by trinucleotide mutagenesis (19aa, no Cys)"
   (xi) SEQUENCE DESCRIPTION: SEQ ID NO: 369:
   CCTGCAAGCG GAAGACGAAG CGGATTATTA TTGCCAGAGC YRKGACNNKN NKNNKNNKNN 60
   KGGCGGCGGC ACGAAGTTAA CCGTTCTTGG CCAGGAATTC GAGCC 105
(2) INFORMATION FOR SEQ ID NO: 370:
   (i) SEQUENCE CHARACTERISTICS:
      (A) LENGTH: 102 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide library"
   (ix) FEATURE:
      (A) NAME/KEY: misc_feature
      (B) LOCATION:41..43
      (D) OTHER INFORMATION:/product= "random codon by trinucleotides (CGT, TGG, TAT)"
   (ix) FEATURE:
      (A) NAME/KEY: misc_feature
      (B) LOCATION:47..55
      (D) OTHER INFORMATION:/product= "random codons by trinucleotides (18 aa, no Trp, no Cys)"
   (ix) FEATURE:
      (A) NAME/KEY: misc_feature
      (B) LOCATION:56..58
      (D) OTHER INFORMATION:/product= "random codon by trinucleotide mutagenesis (19aa, no Cys)"
   (xi) SEQUENCE DESCRIPTION: SEQ ID NO: 370:
   CCTGCAAGCG GAAGACGAAG CGGATTATTA TTGCCAGAGC YRKGACNNKN NKNNKNNKGG 60
   CGGCGGCACG AAGTTAACCG TTCTTGGCCA GGAATTCGAG CC 102
(2) INFORMATION FOR SEQ ID NO: 371:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic oligonucleotide"
   (xi) SEQUENCE DESCRIPTION: SEQ ID NO: 371:
      GGCTCGAATT CCTGGCC 17

## Description

### Field of the Invention

The present invention relates to synthetic DNA sequences which encode one or more collections of homologous proteins/(poly)peptides, and methods for generating and applying libraries of these DNA sequences. In particular, the invention relates to the preparation of a library of human-derived antibody genes by the use of synthetic consensus sequences which cover the structural repertoire of antibodies encoded in the human genome. Furthermore, the invention relates to the use of a single consensus antibody gene as a universal framework for highly diverse antibody libraries. The invention also relates to methods of identifying one or more genes encoding one or more proteins/(poly)peptides having an optimized property selected from the group consisting of optimized affinity for a target molecule, optimized specificity for a target molecule, optimized expression yields, optimized stability and optimized solubility.

### Background to the Invention

All current recombinant methods which use libraries of proteins/(poly)peptides, e.g. antibodies, to screen for members with desired properties, e.g. binding a given ligand, do not provide the possibility to improve the desired properties of the members in an easy and rapid manner. Usually a library is created either by inserting a random oligonucleotide sequence into one or more DNA sequences cloned from an organism, or a family of DNA sequences is cloned and used as the library. The library is then screened, e.g. using phage display, for members which show the desired property. The sequences of one or more of these resulting molecules are then determined. There is no general procedure available to improve these molecules further on.

Winter (EP 0 368 684 B1) has provided a method for amplifying (by PCR), cloning, and expressing antibody variable region genes. Starting with these genes he was able to create libraries of functional antibody fragments by randomizing the CDR3 of the heavy and/or the light chain. This process is functionally equivalent to the natural process of VJ and VDJ recombination which occurs during the development of B-cells in the immune system.

However the Winter invention does not provide a method for optimizing the binding affinities of antibody fragments further on, a process which would be functionally equivalent to the naturally occurring phenomenon of "affinity maturation", which is provided by the present invention. Furthermore, the Winter invention does not provide for artificial variable region genes, which represent a whole family of structurally similar natural genes, and which can be assembled from synthetic DNA oligonucleotides. Additionally, Winter does not enable the combinatorial assembly of portions of antibody variable regions, a feature which is provided by the present disclosure. Furthermore, this approach has the disadvantage that the genes of all antibodies obtained in the screening procedure have to be completely sequenced, since, except for the PCR priming regions, no additional sequence information about the library members is available. This is time and labor intensive and potentially leads to sequencing errors.

The teaching of Winter as well as other approaches have tried to create large antibody libraries having high diversity in the complementarity determining regions (CDRs) as well as in the frameworks to be able to find antibodies against as many different antigens as possible. It has been suggested that a single universal framework may be useful to build antibody libraries, but no approach has yet been successful.

Another problem lies in the production of reagents derived from antibodies. Small antibody fragments show exciting promise for use as therapeutic agents, diagnostic reagents, and for biochemical research. Thus, they are needed in large amounts, and the expression of antibody fragments, e.g. Fv, single-chain Fv (scFv), or Fab in the periplasm of E. coli (Skerra & Plückthun, 1988; Better et al., 1988) is now used routinely in many laboratories. Expression yields vary widely, however. While some fragments yield up to several mg of functional, soluble protein per liter and OD of culture broth in shake flask culture (Carter et al., 1992, Plückthun et al. 1996), other fragments may almost exclusively lead to insoluble material, often found in so-called inclusion bodies. Functional protein may be obtained from the latter in modest yields by a laborious and time-consuming refolding process. The factors influencing antibody expression levels are still only poorly understood. Folding efficiency and stability of the antibody fragments, protease lability and toxicity of the expressed proteins to the host cells often severely limit actual production levels, and several attempts have been tried to increase expression yields. For example, Knappik & Plückthun (1995) could show that expression yield depends on the antibody sequence. They identified key residues in the antibody framework which influence expression yields dramatically. Similarly, Ullrich et al. (1995) found that point mutations in the CDRs can increase the yields in periplasmic antibody fragment expression. Nevertheless, these strategies are only applicable to a few antibodies. Since the Winter invention uses existing repertoires of antibodies, no influence on expressibility of the genes is possible.

Furthermore, the findings of Knappik & Plückthun and Ullrich demonstrate that the knowledge about antibodies, especially about folding and expression is still increasing. The Winter invention does not allow to incorporate such improvements into the library design.

The expressibility of the genes is important for the library quality as well, since the screening procedure relies in most cases on the display of the gene product on a phage surface, and efficient display relies on at least moderate expression of the gene.

These disadvantages of the existing methodologies are overcome by the present invention, which is applicable for all collections of homologous proteins. It has the following novel and useful features illustrated in the following by antibodies as an example:
Artificial antibodies and fragments thereof can be constructed based on known antibody sequences, which reflect the structural properties of a whole group of homologous antibody genes. Therefore it is possible to reduce the number of different genes without any loss in the structural repertoire. This approach leads to a limited set of artificial genes, which can be synthesized de novo, thereby allowing introduction of cleavage sites and removing unwanted cleavages sites. Furthermore, this approach enables (i), adapting the codon usage of the genes to that of highly expressed genes in any desired host cell and (ii), analyzing all possible pairs of antibody light (L) and heavy (H) chains in terms of interaction preference, antigen preference or recombinant expression titer, which is virtually impossible using the complete collection of antibody genes of an organism and all combinations thereof.

The use of a limited set of completely synthetic genes makes it possible to create cleavage sites at the boundaries of encoded structural sub-elements. Therefore, each gene is built up from modules which represent structural sub-elements on the protein/(poly)peptide level. In the case of antibodies, the modules consist of "framework" and "CDR" modules. By creating separate framework and CDR modules, different combinatorial assembly possibilities are enabled. Moreover, if two or more artificial genes carry identical pairs of cleavage sites at the boundaries of each of the genetic sub-elements, pre-built libraries of sub-elements can be inserted in these genes simultaneously, without any additional information related to any particular gene sequence. This strategy enables rapid optimization of, for example, antibody affinity, since DNA cassettes encoding libraries of genetic sub-elements can be (i), pre-built, stored and reused and (ii), inserted in any of these sequences at the right position without knowing the actual sequence or having to determine the sequence of the individual library member.

Additionally, new information about amino acid residues important for binding, stability, or solubility and expression could be integrated into the library design by replacing existing modules with modules modified according to the new observations.

The limited number of consensus sequences used for creating the library allows to speed up the identification of binding antibodies after screening. After having identified the underlying consensus gene sequence, which could be done by sequencing or by using fingerprint restriction sites, just those part(s) comprising the random sequence(s) have to be determined. This reduces the probability of sequencing errors and of false-positive results.

The above mentioned cleavage sites can be used only if they are unique in the vector system where the artificial genes have been inserted. As a result, the vector has to be modified to contain none of these cleavage sites. The construction of a vector consisting of basic elements like resistance gene and origin of replication, where cleavage sites have been removed, is of general interest for many cloning attempts. Additionally, these vector(s) could be part of a kit comprising the above mentioned artificial genes and pre-built libraries.

The collection of artificial genes can be used for a rapid humanization procedure of non-human antibodies, preferably of rodent antibodies. First, the amino acid sequence of the non-human, preferably rodent antibody is compared with the amino acid sequences encoded by the collection of artificial genes to determine the most homologous light and heavy framework regions. These genes are then used for insertion of the genetic sub-elements encoding the CDRs of the non-human, preferably rodent antibody.

Surprisingly, it has been found that with a combination of only one consensus sequence for each of the light and heavy chains of a scFv fragment an antibody repertoire could be created yielding antibodies against virtually every antigen. Therefore, one aspect of the present disclosure is the use of a single consensus sequence as a universal framework for the creation of useful (poly)peptide libraries and antibody consensus sequences useful therefor.

### Detailed Description of the Invention

The present disclosure enables the creation of useful libraries of (poly)peptides. In a first embodiment, the disclosure provides for a method of setting up nucleic acid sequences suitable for the creation of said libraries. In a first step, a collection of at least three homologous proteins is identified and then analyzed. Therefore, a database of the protein sequences is established where the protein sequences are aligned to each other. The database is used to define subgroups of protein sequences which show a high degree of similarity in both the sequence and, if information is available, in the structural arrangement. For each of the subgroups a (poly)peptide sequence comprising at least one consensus sequence is deduced which represents the members of this subgroup; the complete collection of (poly)peptide sequences represent therefore the complete structural repertoire of the collection of homologous proteins. These artificial (poly)peptide sequences are then analyzed, if possible, according to their structural properties to identify unfavorable interactions between amino acids within said (poly)peptide sequences or between said or other (poly)peptide sequences, for example, in multimeric proteins. Such interactions are then removed by changing the consensus sequence accordingly. The (poly)peptide sequences are then analyzed to identify subelements such as domains, loops, helices or CDRs. The amino acid sequence is backtranslated into a corresponding coding nucleic acid sequence which is adapted to the codon usage of the host planned for expressing said nucleic acid sequences. A set of cleavage sites is set up in a way that each of the sub-sequences encoding the sub-elements identified as described above, is flanked by two sites which do not occur a second time within the nucleic acid sequence. This can be achieved by either identifying a cleavage site already flanking a sub-sequence of by changing one or more nucleotides to create the cleavage site, and by removing that site from the remaining part of the gene. The cleavage sites should be common to all corresponding sub-elements or sub-sequences, thus creating a fully modular arrangement of the sub-sequences in the nucleic acid sequence and of the subelements in the corresponding (poly)peptide.

Thus, in light of the above, the disclosure relates to a method of setting up one or more nucleic acid sequences encoding one or more (poly)peptide sequences suitable for the creation of libraries of (poly)peptides said (poly)peptide sequences comprising amino acid consensus sequences, said method comprising the following steps:
(a) deducing from a collection of at least three homologous proteins one or more (poly)peptide sequences comprising at least one amino acid consensus sequence;
(b) optionally, identifying amino acids in said (poly)peptide sequences to be modified so as to remove unfavorable interactions between amino acids within or between said or other (poly)peptide sequences;
(c) identifying at least one structural sub-element within each of said (poly)peptide sequences;
(d) backtranslating each of said (poly)peptide sequences into a corresponding coding nucleic acid sequence;
(e) setting up cleavage sites in regions adjacent to said cleavage sites:
   (ea) being unique within each of said coding nucleic acid sequences;
   (eb) being common to the corresponding sub-sequences of any said coding nucleic acids.

In a further embodiment, the disclosure provides for a method which sets up two or more sets of (poly)peptides, where for each set the method as described above is performed, and where the cleavage sites are not only unique within each set but also between any two sets. This method can be applied for the creation of (poly)peptide libraries comprising for example two a-helical domains from two different proteins, where said library is screened for novel hetero-association domains.

Particularly preferred is a method of setting up two or more sets of one or more nucleic acid sequences comprising executing the steps (a) to € described above for each of said sets with the additional provision that said cleavage sites are unique between said sets.

In yet a further embodiment, at least two of the sets as described above, are derived from the same collection of proteins or at least a part of it. This describes libraries comprising for example, but not limited to, two domains from antibodies such as VH and VL, or two extracellular loops of transmembrane receptors.

In another embodiment, the nucleic acid sequences set up as described above, are synthesized. This can be achieved by any one of several methods well known to the practitioner skilled in the art, for example, by total gene synthesis or by PCR-based approaches.

In one embodiment, the nucleic acid sequences are cloned into a vector. The vector could be a sequencing vector, an expression vector or a display (e.g. phage display) vector, which are well known to those skilled in the art. Any vector could comprise one nucleic acid sequence, or two or more nucleic sequences, either in different or the same operon. In the last case, they could either be cloned separately or as contiguous sequences.

In one embodiment, the removal of unfavorable interactions as described above, leads to enhanced expression of the modified (poly)peptides.

In a preferred embodiment, one or more sub-sequences of the nucleic acid sequences are replaced by different sequences. This can be achieved by excising the sub-sequences using the conditions suitable for cleaving the cleavage sites adjacent to or at the end of the sub-sequence, for example, by using a restriction enzyme at the corresponding restriction site under the conditions well known to those skilled in the art, and replacing the sub-sequence by a different sequence compatible with the cleaved nucleic acid sequence.

In another embodiment of the method of the present disclosure, said different sequences are selected form the group of different sub-sequences encoding the same or different sub-elements derived from the same or different (poly)peptides.

In a further preferred embodiment, the different sequences replacing the initial sub-sequence(s) are genomic or rearranged genomic sequences, for example in grafting CDRs from non-human antibodies onto consensus antibody sequences for rapid humanization of non-human antibodies. In the most preferred embodiment, the different sequences are random sequences, thus replacing the sub-sequence by a collection of sequences to introduce variability and to create a library. The random sequences can be assembled in various ways, for example by using a mixture of mononucleotides or preferably a mixture of trinucleotides (Virnekas et al., 1994) during automated oligonucleotide synthesis, by error-prone PCR or by other methods well known to the practitioner in the art. The random sequences may be completely randomized or biased towards or against certain codons according to the amino acid distribution at certain positions in known protein sequences. Additionally, the collection of random sub-sequences may comprise different numbers of codons, giving rise to a collection of sub-elements having different lengths.

In another embodiment, the disclosure provides for the expression of the nucleic acid sequences from a suitable vector and under suitable conditions well known to those skilled in the art.

In a further preferred embodiment, the (poly)peptides expressed from said nucleic acid sequences are screened and, optionally, optimized. Screening may be performed by using one of the methods well known to the practitioner in the art, such as phage-display, selectively infective phage, polysome technology to screen for binding, assay systems for enzymatic activity or protein stability. (Poly)peptides having the desired property can be identified by sequencing of the corresponding nucleic acid sequence or by amino acid sequencing or mass spectrometry. In the case of subsequent optimization, the nucleic acid sequences encoding the initially selected (poly)peptides can optionally be used without sequencing. Optimization is performed by repeating the replacement of sub-sequences by different sequences, preferably by random sequences, and the screening step one or more times.

The desired property the (poly)peptides are screened for is preferably, but not exclusively, selected from the group of optimized affinity or specificity for a target molecule, optimized enzymatic activity, optimized expression yields, optimized stability and optimized solubility.

In one embodiment, the cleavage sites flanking the sub-sequences are sites recognized and cleaved by restriction enzymes, with recognition and cleavage sequences being either identical or different, the restricted sites either having blunt or sticky ends.

The length of the sub-elements is preferably, but not exclusively ranging between 1 amino acid, such as one residue in the active site of an enzyme or a structure-determining residue, and 150 amino acids, as for whole protein domains. Most preferably, the length ranges between 3 and 25 amino acids, such as most commonly found in CDR loops of antibodies.

The nucleic acid sequences could be RNA or, preferably, DNA.

In one embodiment, the (poly)peptides have an amino acid pattern characteristic of a particular species. This can for example be achieved by deducing the consensus sequences from a collection of homologous proteins of just one species, most preferably from a collection of human proteins. Since the (poly)peptides comprising consensus sequences are artificial, they have to be compared to the protein sequence(s) having the closest similarity to ensure the presence of said characteristic amino acid pattern.

In one embodiment, the disclosure provides for the creation of libraries of (poly)peptides comprising at least part of members or derivatives of the immunoglobulin superfamily, preferably of member or derivatives of the immnoglobulins. Most preferably, the disclosure provides for the creation of libraries of human antibodies, wherein said (poly)peptides are or are derived from heavy or light chain variable regions wherein said structural sub-elements are framework regions (FR) 1, 2, 3, or 4 or complementary determining regions (CDR) 1, 2, or 3. In a first step, a database of published antibody sequences of human origin is established where the antibody sequences are aligned to each other. The database is used to define subgroups of antibody sequences which show a high degree of similarity in both the sequence and the canonical fold of CDR loops (as determined by analysis of antibody structures). For each of the subgroups a consensus sequence is deduced which represents the members of this subgroup; the complete collection of consensus sequences represent therefore the complete structural repertoire of human antibodies.
These artificial genes are then constructed e.g. by total gene synthesis or by the use of synthetic genetic subunits. These genetic subunits correspond to structural subelements on the (poly)peptide level. On the DNA level, these genetic subunits are defined by cleavage sites at the start and the end of each of the sub-elements, which are unique in the vector system. All genes which are members of the collection of consensus sequences are constructed such that they contain a similar pattern of corresponding genetic sub-sequences. Most preferably, said (poly)peptides are or are derived from the HuCAL consensus genes: Vκ1, Vκ2, Vκ3, Vκ4, Vλ1, Vλ2, Vλ3, VH1A, VH1B, VH2, VH3, VH4, VH5, VH6, Cκ, Cλ, CH1 or any combination of said HuCAL consensus genes.
This collection of DNA molecules can then be used to create libraries of antibodies or antibody fragments, preferably Fv, disulphide-linked Fv, single-chain Fv (scFv), or Fab fragments, which may be used as sources of specificities against new target antigens. Moreover, the affinity of the antibodies can be optimized using pre-built library cassettes and a general procedure. The disclosure provides a method for identifying one or more genes encoding one or more antibody fragments which binds to a target, comprising the steps of expressing the antibody fragments, and then screening them to isolate one or more antibody fragments which bind to a given target molecule. Preferably, an scFv fragment library comprising the combination of HuCAL VH3 and HuCAL Vλ2 consensus genes and at least a random sub-sequence encoding the heavy chain CDR3 sub-element is screened for binding antibodies. If necessary, the modular design of the genes can then be used to excise from the genes encoding the antibody fragments one or more genetic sub-sequences encoding structural sub-elements, and replacing them by one or more second sub-sequences encoding structural sub-elements. The expression and screening steps can then be repeated until an antibody having the desired affinity is generated.
Particularly preferred is a method in which one or more of the genetic subunits (e.g. the CDRs) are replaced by a random collection of sequences (the library) using the said cleavage sites. Since these cleavage sites are (i) unique in the vector system and (ii) common to all consensus genes, the same (pre-built) library can be inserted into all artificial antibody genes. The resulting library is then screened against any chosen antigen. Binding antibodies are selected, collected and used as starting material for the next library. Here, one or more of the remaining genetic subunits are randomized as described above.

A further embodiment of the present disclosure relates to fusion proteins by providing for a DNA sequence which encodes both the (poly)peptide, as described above, as well as an additional moiety. Particularly preferred are moieties which have a useful therapeutic function. For example, the additional moiety may be a toxin molecule which is able to kill cells (Vitetta et al., 1993). There are numerous examples of such toxins, well known to the one skilled in the art, such as the bacterial toxins Pseudomonas exotoxin A, and diphtheria toxin, as well as the plant toxins ricin, abrin, modeccin, saporin, and gelonin. By fusing such a toxin for example to an antibody fragment, the toxin can be targeted to, for example, diseased cells, and thereby have a beneficial therapeutic effect. Alternatively, the additional moiety may be a cytokine, such as IL-2 (Rosenberg & Lotze, 1986), which has a particular effect (in this case a T-cell proliferative effect) on a family of cells. In a further embodiment, the additional moiety may confer on its (poly)peptide partner a means of detection and/or purification. For example, the fusion protein could comprise the modified antibody fragment and an enzyme commonly used for detection purposes, such as alkaline phosphatase (Blake et al., 1984). There are numerous other moieties which can be used as detection or purification tags, which are well known to the practitioner skilled in the art. Particularly preferred are peptides comprising at least five histidine residues (Hochuli et al., 1988), which are able to bind to metal ions, and can therefore be used for the purification of the protein to which they are fused (Lindner et al., 1992). Also provided for by the disclosure are additional moieties such as the commonly used C-myc and FLAG tags (Hopp et al., 1988; Knappik & Plückthun, 1994).

Thus in another preferred embodiment, the disclosure relates to a method, wherein at least part of said (poly)peptide sequences or (poly)peptide is connected to a sequence encoding at least one additional moiety or to at least one additional moiety, respectively.

Particularly preferred is a method, wherein said connection is formed via a contiguous nucleic acid sequence or amino acid sequence, respectively.

Most preferably, said additional moiety is a toxin, a cytokine, a reporter enzyme, a moiety being capable of binding a metal ion, a peptide, a tag suitable for detection and/or purification, or a homo- or hetero-association domain.

Particularly preferred is a method of the present disclosure, wherein the expression of said nucleic acid sequences results in the generation of a repertoire of biological activities and/or specificities, preferably the generation of a repertoire based on a universal framework.

By engineering one or more fused additional domains, antibody fragments or any other (poly)peptide can be assembled into larger molecules which also fall under the scope of the present disclosure. For example, mini-antibodies (Pack, 1994) are dimers comprising two antibody fragments, each fused to a self-associating dimerization domain. Dimerization domains which are particularly preferred include those derived from a leucine zipper (Pack & Plückthun, 1992) or helix-turn-helix motif (Pack et al., 1993).

All of the above embodiments of the present disclosure can be effected using standard techniques of molecular biology known to anyone skilled in the art.

In a further embodiment, the random collection of sub-sequences (the library) is inserted into a singular nucleic acid sequence encoding one (poly)peptide, thus creating a (poly)peptide library based on one universal framework. Preferably a random collection of CDR sub-sequences is inserted into a universal antibody framework, for example into the HuCAL H3k2 single-chain Fv fragment described above.

In further embodiments, the disclosure provides for nucleic acid sequence(s), vector(s) containing the nucleic acid sequence(s), host cell(s) containing the vector(s), and (poly)peptides, obtainable according to the methods described above.

The disclosure furthermore relates to a collection of recombinant vectors obtainable by a method according to the present disclosure and to a collection of hast cells transformed with said collection of recombinant vectors.

In another embodiment, the disclosure relates to a method of producing a (poly)peptide or a collection of (poly)peptides as defined above comprising culturing a host cell of the present disclosure or a collection of host cells according to the present disclosure under suitable conditions and isolating said (poly)peptides.

In a still further embodiment, the present disclosure relates to a (poly)peptide devisable by the method according to the present disclosure, encoded by a nucleic acid sequence according to the present disclosure or obtainable by a method according to the present disclosure.

Furthermore, the disclosure relates to a collection of (poly)peptides devisable by a method according to the present disclosure, encoded by a collection of nucleic acid sequences according to the present disclosure or obtainable by a method according to the present disclosure.

In a further preferred embodiment, the disclosure provides for modular vector systems being compatible with the modular nucleic acid sequences encoding the (poly)peptides. The modules of the vectors are flanked by restriction sites unique within the vector system and essentially unique with respect to the restriction sites incorporated into the nucleic acid sequences encoding the (poly)peptides, except for example the restriction sites necessary for cloning the nucleic acid sequences into the vector. The list of vector modules comprises origins of single-stranded replication, origins of double-stranded replication for high- and low copy number plasmids, promotor/operator, repressor or terminator elements, resistance genes, potential recombination sites, gene III for display on filamentous phages, signal sequences, purification and detection tags, and sequences of additional moieties. The vectors are preferably, but not exclusively, expression vectors or vectors suitable for expression and screening of libraries.

In another embodiment, the disclosure provides for a kit, comprising one or more of the list of nucleic acid sequence(s), recombinant vector(s), (poly)peptide(s), and vector(s) according to the methods described above, and suitable host cell(s) for producing the (poly)peptide(s).

In a preferred embodiment, the disclosure provides for the creation of libraries of human antibodies. In a first step, a database of published antibody sequences of human origin is established. The database is used to define subgroups of antibody sequences which show a high degree of similarity in both the sequence and the canonical fold (as determined by analysis of antibody structures). For each of the subgroups a consensus sequence is deduced which represents the members of this subgroup; the complete collection of consensus sequences represent therefore the complete structural repertoire of human antibodies.

These artificial genes are then constructed by the use of synthetic genetic subunits. These genetic subunits correspond to structural sub-elements on the protein level. On the DNA level, these genetic subunits are defined by cleavage sites at the start and the end of each of the subelements, which are unique in the vector system. All genes which are members of the collection of consensus sequences are constructed such that they contain a similar pattern of said genetic subunits.

This collection of DNA molecules can then be used to create libraries of antibodies which may be used as sources of specificities against new target antigens. Moreover, the affinity of the antibodies can be optimised using pre-built library cassettes and a general procedure. The disclosure provides a method for identifying one or more genes encoding one or more antibody fragments which binds to a target, comprising the steps of expressing the antibody fragments, and then screening them to isolate one or more antibody fragments which bind to a given target molecule. If necessary, the modular design of the genes can then be used to excise from the genes encoding the antibody fragments one or more genetic sub-sequences encoding structural sub-elements, and replacing them by one or more second sub-sequences encoding structural sub-elements. The expression and screening steps can then be repeated until an antibody having the desired affinity is generated.

Particularly preferred is a method in which one or more of the genetic subunits (e.g. the CDR's) are replaced by a random collection of sequences (the library) using the said cleavage sites. Since these cleavage sites are (i) unique in the vector system and (ii) common to all consensus genes, the same (pre-built) library can be inserted into all artificial antibody genes. The resulting library is then screened against any chosen antigen. Binding antibodies are eluted, collected and used as starting material for the next library. Here, one or more of the remaining genetic subunits are randomised as described above.

Furthermore, the disclosure relates to a method of designing two or more genes encoding a collection of two or more proteins, comprising the steps of:
(a) either
   (aa) identifying two or more homologous gene sequences, or
   (ab) analyzing at least three homologous genes, and
      deducing two or more consensus gene sequences therefrom,
(b) optionally, modifying codons in said consensus gene sequences to remove unfavorable interactions between amino acids in the resulting proteins,
(c) identifying sub-sequences with encode structural sub-elements in said consensus gene sequences
(d) modifying one or more bases in regions adjacent to or between the ends of said sub-sequences to define one or more cleavage sites, each of which:
   (da) are unique within each consensus gene sequence,
   (db) do not form compatible sites with respect to any single sub-sequence,
   (dc) are common to all homologous sub-sequences.

Particularly preferred is a method of preparing two or more genes encoding a collection of two or more proteins, comprising the steps of:
(a) designing said genes according to the present disclosure, and
(b) synthesizing said genes.

Furthermore, the present disclosure relates to a collection of genes prepared according to the method of the present disclosure.

The disclosure furthermore relates to a collection of two or more genes derived from gene sequences which:
(a) are either homologous, or represent consensus gene sequences derived from at least three homologous genes, and
(b) carry cleavage sites, each of which:
   (ba) lie at or adjacent to the ends of generic sub-sequences which encode structural sub-elements,
   (bb) are unique within each gene sequence,
   (bc) do not form compatible sites with respect to any single sub-sequence, and
   (bd) are common to all homologous sub-sequences.

Particularly preferred is a collection of genes, in which each of said gene sequences has a nucleotide composition characteristic of a particular species.

Most preferably, said species is human.

Yet further preferred is a collection of genes according to the present disclosure, in which one or more of said gene sequences encodes at least part of a member of the immunoglobulin superfamily, preferably of the immunoglobulin family.

Particularly preferred, said structural sub-elements correspond to any combination of framework regions 1, 2, 3, and 4, and/or CDR regions 1, 2, and 3 of antibody heavy chains.

In yet another preferred embodiment, said structural sub-elements correspond to any combination of framework regions 1, 2, 3, and 4, and/or CDR regions 1, 2, and 3 of antibody light chains.

Furthermore, the disclosure relates to a collection of vectors comprising a collection of genes sequences according to the present disclosure.

In another embodiment, the present disclosure, the collection of vectors comprises the additional feature that the vector does not comprise any cleavage site that is contained in the collection of genes according to the present disclosure.

The present invention provides a method of identifying one or more genes encoding one or more proteins having an optimized property, comprising the steps of:
(a) expressing a collection of proteins from a collection of genes;
(b) screening said collection of proteins against at least one antigen to isolate proteins having a desired property;
(c) obtaining a plurality of genes, wherein each of said genes encodes a protein resulting from step (b);
(d) replacing one or more sub-sequence of each of said genes with a different, compatible genetic sub-sequence;
(e) screening the proteins encoded by the genes resulting from step (d) against said antigen(s), wherein the property of one or more proteins encoded by a gene resulting from step (d) is optimized compared to the property of the collection of proteins generated in step (b);
wherein one or more of said genes encode at least part of a member of the immunoglobulin superfamily, wherein the genetic sub-sequence encodes a complementarity determining region, and wherein the optimized property is selected from the group consisting of optimized affinity for a target molecule, optimized specificity for a target molecule, optimized expression yields, optimized stability and optimized solubility.

In another embodiment, the present disclosure relates to a method for identifying one or more genes encoding one or more proteins having a desirable property, comprising the steps of:
(a) expressing from a collection of vectors according to the resent disclosure a collection of proteins.
(b) screening said collection to isolate one or more proteins having a desired property,
(c) identifying the genes encoding the proteins isolates in step (b),
(d) optionally, existing from the genes encoding the proteins isolated in step (b) one or more genetic sub-sequences encoding structural sub-elements, and replacing said sub-sequence(s) by one or more second sub-sequences encoding structural sub-elements, to generate new vectors according to the present disclosure,
(e) optionally, repeating steps (a) to (c).

The disclosure furthermore relates to a method for identifying one or more genes encoding one or more antibody fragments which binds to a target, comprising the steps of:
(a) expressing from the collection of vectors according to the present disclosure a collection of proteins,
(b) screening said collection to isolate one or more antibody fragments which bind to said target,
(c) identifying he genes encoding the proteins isolated in step (b),
(d) optionally, excising from the genes encoding the antibody fragments isolated in step (b) one or more genetic sub-sequences encoding structural sub-elements, and replacing said sub-sequence(s) by one or more second sub-sequences encoding structural sub-generate new vectors according to according to the present disclosure,
(e) optionally, repeating steps (a) to (c).

In yet another embodiment, the present disclosure relates to a kit comprising two or more genes derived from gene sequences which:
(a) are either homologous, or represent consensus gene sequences derived from at least three homologous genes, and
(b) carry cleavage sites, each of which:
   (ba) lie at or adjacent to the ends of genetic sub-sequences which encode structural sub-elements,
   (bb) are unique within each gene sequence,
   (bc) do not form compatible sites with respect to any single sub-sequence, and
   (bd) are common to all homologous sub-sequences.

Furthermore, the disclosure relates to a kit comprising two or more genetic sub-sequences which encode structural sub-elements, which can be assembled to form genes, and which carry cleavage sites, each of which:
(a) lie at or adjacent to the ends of said genetic sub-sequences,
(b) do not form compatible sites with respect of any single sub-sequence, and
(c) are common to all homologous sub-sequences.

### Definitions

### Protein:

The term protein comprises monomeric polypeptide chains as well as homo- or heteromultimeric complexes of two or more polypeptide chains connected either by covalent interactions (such as disulphide bonds) or by non-covalent interactions (such as hydrophobic or electrostatic interactions).

### Analysis of homologous proteins:

The amino acid sequences of three or more proteins are aligned to each other (allowing for introduction of gaps) in a way which maximizes the correspondence between identical or similar amino acid residues at all positions. These aligned sequences are termed homologous if the percentage of the sum of identical and/or similar residues exceeds a defined threshold. This threshold is commonly regarded by those skilled in the art as being exceeded when at least 15% of the amino acids in the aligned genes are identical, and at least 30% are similar. Examples for families of homologous proteins are: immunoglobulin superfamily, scavenger receptor superfamily, fibronectin superfamilies (e.g. type II and III), complement control protein superfamily, cytokine receptor superfamily, cystine knot proteins, tyrosine kinases, and numerous other examples well known to one of ordinary skill in the art.

### Consensus sequence:

Using a matrix of at least three aligned amino acid sequences, and allowing for gaps in the alignment, it is possible to determine the most frequent amino acid residue at each position. The consensus sequence is that sequence which comprises the amino acids which are most frequently represented at each position. In the event that two or more amino acids are equally represented at a single position, the consensus sequence includes both or all of those amino acids.

### Removing unfavorable interactions:

The consensus sequence is per se in most cases artificial and has to be analyzed in order to change amino acid residues which, for example, would prevent the resulting molecule to adapt a functional tertiary structure or which would block the interaction with other (poly)peptide chains in multimeric complexes. This can be done either by (i) building a three-dimensional model of the consensus sequence using known related structures as a template, and identifying amino acid residues within the model which may interact unfavorably with each other, or (ii) analyzing the matrix of aligned amino acid sequences in order to detect combinations of amino acid residues within the sequences which frequently occur together in one sequence and are therefore likely to interact with each other. These probable interaction-pairs are then tabulated and the consensus is compared with these "interaction maps". Missing or wrong interactions in the consensus are repaired accordingly by introducing appropriate changes in amino acids which minimize unfavorable interactions.

### Identification of structural sub-elements:

Structural sub-elements are stretches of amino acid residues within a protein/(poly)peptide which correspond to a defined structural or functional part of the molecule. These can be loops (e.g. CDR loops of an antibody) or any other secondary or functional structure within the protein/(poly)peptide (domains, α-helices, β-sheets, framework regions of antibodies, etc.). A structural sub-element can be identified using known structures of similar or homologous (poly)peptides, or by using the above mentioned matrices of aligned amino acid sequences. Here the variability at each position is the basis for determining stretches of amino acid residues which belong to a structural sub-element (e.g. hypervariable regions of an antibody).

### Sub-sequence:

A sub-sequence is defined as a genetic module which is flanked by unique cleavage sites and encodes at least one structural sub-element. It is not necessarily identical to a structural sub-element.

### Cleavage site:

A short DNA sequence which is used as a specific target for a reagent which cleaves DNA in a sequence-specific manner (e.g. restriction endonucleases).

### Compatible cleavage sites:

Cleavage sites are compatible with each other, if they can be efficiently ligated without modification and, preferably, also without adding an adapter molecule..

### Unique cleavage sites:

A cleavage site is defined as unique if it occurs only once in a vector containing at least one of the genes of interest, or if a vector containing at least one of the genes of interest could be treated in a way that only one of the cleavage sites could be used by the cleaving agent.

### Corresponding (poly)peptide sequences:

Sequences deduced from the same part of one group of homologous proteins are called corresponding (poly)peptide sequences.

### Common cleavage sites:

A cleavage site in at least two corresponding sequences, which occurs at the same functional position (i.e. which flanks a defined sub-sequence), which can be hydrolyzed by the same cleavage tool and which yields identical compatible ends is termed a common cleavage site.

### Excising genetic sub-sequences:

A method which uses the unique cleavage sites and the corresponding cleavage reagents to cleave the target DNA at the specified positions in order to isolate, remove or replace the genetic sub-sequence flanked by these unique cleavage sites.

### Exchanging genetic sub-sequences:

A method by which an existing sub-sequence is removed using the flanking cleavage sites of this sub-sequence, and a new sub-sequence or a collection of sub-sequences, which contain ends compatible with the cleavage sites thus created, is inserted.

### Expression of genes:

The term expression refers to in vivo or in vitro processes, by which the information of a gene is transcribed into mRNA and then translated into a protein/(poly)peptide. Thus, the term expression refers to a process which occurs inside cells, by which the information of a gene is transcribed into mRNA and then into a protein. The term expression also includes all events of post-translational modification and transport, which are necessary for the (poly)peptide to be functional.

### Screening of protein/(poly)peptide libraries:

Any method which allows isolation of one or more proteins/(poly)peptides having a desired property from other proteins/(poly)peptides within a library.

### Amino acid pattern characteristic for a species:

A (poly)peptide sequence is assumed to exhibit an amino acid pattern characteristic for a species if it is deduced from a collection of homologous proteins from just this species.

### Immunoglobulin superfamily (IgSF):

The IgSF is a family of proteins comprising domains being characterized by the immunoglobulin fold. The IgSF comprises for example T-cell receptors and the immunoglobulins (antibodies).

### Antibody framework:

A framework of an antibody variable domain is defined by Kabat et al. (1991) as the part of the variable domain which serves as a scaffold for the antigen binding loops of this variable domain.

### Antibody CDR:

The CDRs (complementarity determining regions) of an antibody consist of the antigen binding loops, as defined by Kabat et al. (1991). Each of the two variable domains of an antibody Fv fragment contain three CDRs.

### HuCAL:

Acronym for Human Combinatorial Antibody Library. Antibody Library based on modular consensus genes according to the disclosure (see Example 1).

### Antibody fragment:

Any portion of an antibody which has a particular function, e.g. binding of antigen. Usually, antibody fragments are smaller than whole antibodies. Examples are Fv, disulphide-linked Fv, single-chain Fv (scFv), or Fab fragments. Additionally, antibody fragments are often engineered to include new functions or properties.

### Universal framework:

One single framework which can be used to create the full variability of functions, specificities or properties which is originally sustained by a large collection of different frameworks, is called universal framework.

### Binding of an antibody to its target:

The process which leads to a tight and specific association between an antibody and a corresponding molecule or ligand is called binding. A molecule or ligand or any part of a molecukle or ligand which is recognized by an antibody is called the target.

### Replacing genetic sub-sequences

A method by which an existing sub-sequence is removed using the flanking cleavage sites of this sub-sequence, and a new sub-sequence or collection of sub-sequences, which contains ends compatible with the cleavage sites thus created, is inserted.

### Assembling of genetic sequences:

Any process which is used to combine synthetic or natural genetic sequences in a specific manner in order to get longer genetic sequences which contain at least parts of the used synthetic or natural genetic sequences.

### Analysis of homologous genes:

The corresponding amino acid sequences of two or more genes are aligned to each other in a way which maximizes the correspondence between identical or similar amino acid residues at all positions. These aligned sequences are termed homologous if the percentage of the sum of identical and/or similar residues exceeds a defined threshold. This threshold is commonly regarded by those skilled in the art as being exceeded when at least 15 per cent of the amino acids in the aligned genes are identical, and at least 30 per cent are similar.

### Legends to Figures and Tables

- **Fig. 1:**: Flow chart outlining the process of construction of a synthetic human antibody library based on consensus sequences.
- **Fig. 2:**: Alignment of consensus sequences designed for each subgroup (amino acid residues are shown with their standard one-letter abbreviation). (A) kappa sequences, (B) lambda sequences and (C), heavy chain sequences. The positions are numbered according to Kabat (1991). In order to maximize homology in the alignment, gaps (-) have been introduced in the sequence at certain positions.
- **Fig. 3:**: Gene sequences of the synthetic V kappa consensus genes. The corresponding amino acid sequences (see Fig. 2) as well as the unique cleavage sites are also shown.
- **Fig. 4:**: Gene sequences of the synthetic V lambda consensus genes. The corresponding amino acid sequences (see Fig. 2) as well as the unique cleavage sites are also shown.
- **Fig. 5:**: Gene sequences of the synthetic V heavy chain consensus genes. The corresponding amino acid sequences (see Fig. 2) as well as the unique cleavage sites are also shown.
- **Fig. 6:**: Oligonucleotides used for construction of the consensus genes. The oligos are named according to the corresponding consensus gene, e.g. the gene Vκ1 was constructed using the six oligonucleotides O1K1 to O1K6. The oligonucleotides used for synthesizing the genes encoding the constant domains Cκ (OCLK1 to 8) and CH1 (OCH1 to 8) are also shown.
- **Fig. 7A/B:**: Sequences of the synthetic genes encoding the constant domains Cκ (**A**) and CH1 (**B**). The corresponding amino acid sequences as well as unique cleavage sites introduced in these genes are also shown.
- **Fig. 7C:**: Functional map and sequence of module M24 comprising the synthetic Cλ gene segment (huCL lambda).
- **Fig. 7D:**: Oligonucleotides used for synthesis of module M24.

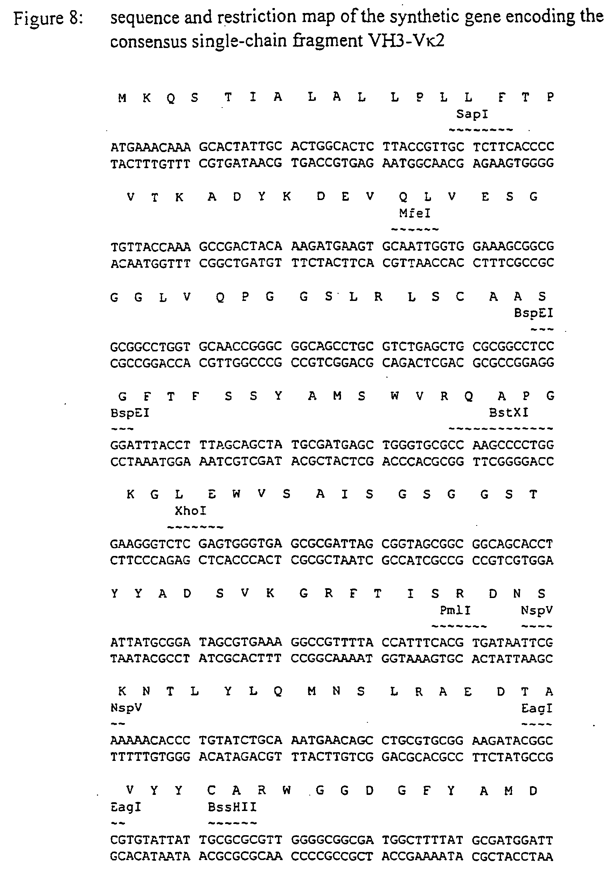

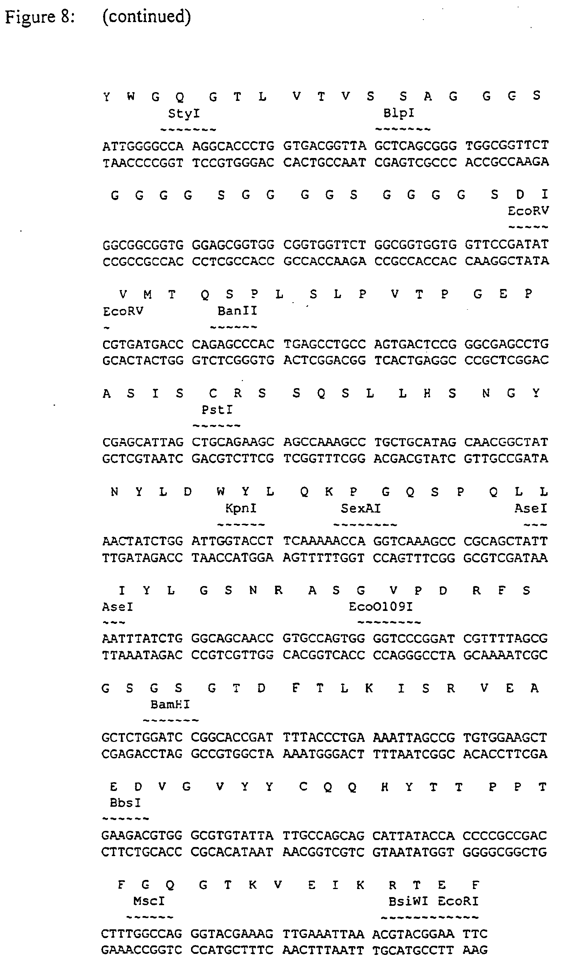

- **Fig. 8:**: Sequence and restriction map of the synthetic gene encoding the consensus single-chain fragment VH3-Vk2. The signal sequence (amino acids 1 to 21) was derived from the *E. coli* phoA gene (Skerra & Plückthun, 1988). Between the phoA signal sequence and the VH3 domain, a short sequence stretch encoding 4 amino acid residues (amino acid 22 to 25) has been inserted in order to allow detection of the single-chain fragment in Western blot or ELISA using the monoclonal antibody M1 (Knappik & Plückthun, 1994). The last 6 basepairs of the sequence were introduced for cloning purposes (EcoRl site).
- **Fig. 9:**: Plasmid map of the vector pIG10.3 used for phage display of the H3κ2 scFv fragment. The vector is derived from pIG10 and contains the gene for the lac operon repressor, lacI, the artificial operon encoding the H3κ2-gene3ss fusion under control of the lac promoter, the Ipp terminator of transcription, the single-strand replication origin of the *E*. *coli* phage f1 (F1_ORI), a gene encoding β-lactamase (bla) and the ColEI derived origin of replication.
- **Fig. 10:**: Sequencing results of independent clones from the initial library, translated into the corresponding amino acid sequences. (**A**) Amino acid sequence of the VH3 consensus heavy chain CDR3 (position 93 to 102, Kabat numbering). (**B**) Amino acid sequences of 12 clones of the 10-mer library. (**C**) Amino acid sequences of 11 clones of the 15-mer library, *: single base deletion.
- **Fig. 11:**: Expression test of individual library members. (**A**) Expression of 9 independent clones of the 10-mer library. (**B**) Expression of 9 independent clones of the 15-mer library. The lane designated with M contains the size marker. Both the gp3-scFv fusion and the scFv monomer are indicated.
- **Fig. 12:**: Enrichment of specific phage antibodies during the panning against FITC-BSA. The initial as well as the subsequent fluorescein-specific sub-libraries were panned against the blocking buffer and the ratio of the phage eluted from the FITC-BSA coated well vs. that from the powder milk coated well from each panning round is presented as the "specificity factor".
- **Fig. 13:**: Phage ELISA of 24 independent clones after the third round of panning tested for binding on FITC-BSA.
- **Fig. 14:**: Competition ELISA of selected FITC-BSA binding clones. The ELISA signals (OD₄₀₅ₙₘ) of scFv binding without inhibition are taken as 100%.
- **Fig. 15:**: Sequencing results of the heavy chain CDR3s of independent clones after 3 rounds of panning against FITC-BSA, translated into the corresponding amino acid sequences (position 93 to 102, Kabat numbering).
- **Fig. 16:**: Coomassie-Blue stained SDS-PAGE of the purified anti-fluorescein scFv fragments: M: molecular weight marker, A: total soluble cell extract after induction, B: fraction of the flow-through, C, D and E: purified scFv fragments 1 HA-3E4, 1 HA-3E5 and 1HA-3E10, respectively.
- **Fig. 17:**: Enrichment of specific phage antibodies during the panning against β-estradiol-BSA, testosterone-BSA, BSA, ESL-1, interleukin-2, lymphotoxin-ß, and LeY-BSA after three rounds of panning.
- **Fig. 18:**: ELISA of selected ESL-1 and β-estradiol binding clones
- **Fig. 19:**: Selectivity and cross-reactivity of HuCAL antibodies: in the diagonal specific binding of HuCAL antibodies can be seen, off-diagonal signals show non-specific cross-reactivity.
- **Fig. 20:**: Sequencing results of the heavy chain CDR3s of independent clones after 3 rounds of panning against β-estradiol-BSA, translated into the corresponding amino acid sequences (position 93 to 102, Kabat numbering). One clone is derived from the 10mer library.
- **Fig. 21:**: Sequencing results of the heavy chain CDR3s of independent clones after 3 rounds of panning against testosterone-BSA, translated into the corresponding amino acid sequences (position 93 to 102, Kabat numbering).
- **Fig. 22:**: Sequencing results of the heavy chain CDR3s of independent clones after 3 rounds of panning against lymphotoxin-β, translated into the corresponding amino acid sequences (position 93 to 102, Kabat numbering). One clone comprises a 14mer CDR, presumably introduced by incomplete coupling of the trinucleotide mixture during oligonucleotide synthesis.
- **Fig. 23:**: Sequencing results of the heavy chain CDR3s of independent clones after 3 rounds of panning against ESL-1, translated into the corresponding amino acid sequences (position 93 to 102, Kabat numbering). Two clones are derived from the 10mer library. One clone comprises a 16mer CDR, presumably introduced by chain elongation during oligonucleotide synthesis using trinucleotides.
- **Fig. 24:**: Sequencing results of the heavy chain CDR3s of independent clones after 3 rounds of panning against BSA, translated into the corresponding amino acid sequences (position 93 to 102, Kabat numbering).
- **Fig. 25:**: Schematic representation of the modular pCAL vector system.
- **Fig. 25a:**: List of restriction sites already used in or suitable for the modular HuCAL genes and pCAL vector system.
- **Fig. 26:**: List of the modular vector elements for the pCAL vector series: shown are only those restriction sites which are part of the modular system.
- **Fig. 27:**: Functional map and sequence of the multi-cloning site module (MCS)
- **Fig. 28:**: Functional map and sequence of the pMCS cloning vector series.
- **Fig. 29:**: Functional map and sequence of the pCAL module M1 (see Fig. 26).
- **Fig. 30:**: Functional map and sequence of the pCAL module M7-III (see Fig. 26).
- **Fig. 31:**: Functional map and sequence of the pCAL module M9-II (see Fig. 26).
- **Fig. 32:**: Functional map and sequence of the pCAL module M11-II (see Fig. 26).
- **Fig. 33:**: Functional map and sequence of the pCAL module M14-Ext2 (see Fig. 26).
- **Fig. 34:**: Functional map and sequence of the pCAL module M17 (see Fig. 26).
- **Fig. 35:**: Functional map and sequence of the modular vector pCAL4.
- **Fig. 35a:**: Functional maps and sequences of additional pCAL modules (M2, M3, M7I, M7II, M8, M10II, M11II, M12, M13, M19, M20, M21, M41) and of low-copy number plasmid vectors (pCALO1 to pCALO3).
- **Fig. 35b:**: List of oligonucleotides and primers used for synthesis of pCAL vector modules.
- **Fig. 36:**: Functional map and sequence of the ß-lactamase cassette for replacement of CDRs for CDR library cloning.
- **Fig. 37:**: Oligo and primer design for Vk CDR3 libraries
- **Fig. 38:**: Oligo and primer design for Vλ CDR3 libraries
- **Fig. 39:**: Functional map of the pBS13 expression vector series.
- **Fig. 40:**: Expression of all 49 HuCAL scFvs obtained by combining each of the 7 VH genes with each of the 7 VL genes (pBS13, 30°C): Values are given for the percentage of soluble vs. insoluble material, the total and the soluble amount compared to the combination H3k2, which was set to 100%. In addition, the corresponding values for the McPC603 scFv are given.

- **Table 1:**: Summary of human immunoglobulin germline sequences used for computing the germline membership of rearranged sequences. (**A**) kappa sequences, (**B**) lambda sequences and (**C**), heavy chain sequences. (1) The germline name used in the various calculations, (2) the references number for the corresponding sequence (see appendix for sequence related citations), (3) the family where each sequence belongs to and (4), the various names found in literature for germline genes with identical amino acid sequences.
- **Table 2:**: Rearranged human sequences used for the calculation of consensus sequences. (**A**) kappa sequences, (**B**) lambda sequences and (**C**), heavy chain sequences. The table summarized the name of the sequence (1), the length of the sequence in amino acids (2), the germline family (3) as well as the computed germline counterpart (4). The number of amino acid exchanges between the rearranged sequence and the germline sequence is tabulated in (5), and the percentage of different amino acids is given in (6). Column (7) gives the references number for the corresponding sequence (see appendix for sequence related citations).
- **Table 3:**: Assignment of rearranged V sequences to their germline counterparts. (**A**) kappa sequences, (**B**) lambda sequences and (**C**), heavy chain sequences. The germline genes are tabulated according to their family (1), and the number of rearranged genes found for every germline gene is given in (2).
- **Table 4:**: Computation of the consensus sequence of the rearranged V kappa sequences. (**A**), V kappa subgroup 1, (**B**), V kappa subgroup 2, (**C**), V kappa subgroup 3 and (**D**), V kappa subgroup 4. The number of each amino acid found at each position is tabulated together with the statistical analysis of the data. (1) Amino acids are given with their standard one-letter abbreviations (and B means D or N, Z means E or Q and X means any amino acid). The statistical analysis summarizes the number of sequences found at each position (2), the number of occurrences of the most common amino acid (3), the amino acid residue which is most common at this position (4), the relative frequency of the occurrence of the most common amino acid (5) and the number of different amino acids found at each position (6).
- **Table 5:**: Computation of the consensus sequence of the rearranged V lambda sequences. (**A**), V lambda subgroup 1, (B**),** V lambda subgroup 2, and (**C**), V lambda subgroup 3. The number of each amino acid found at each position is tabulated together with the statistical analysis of the data. Abbreviations are the same as in Table 4.
- **Table 6:**: Computation of the consensus sequence of the rearranged V heavy chain sequences. (**A**), V heavy chain subgroup 1A, (**B**), V heavy chain subgroup 1 B, (**C**), V heavy chain subgroup 2, (**D**), V heavy chain subgroup 3, (**E**), V heavy chain subgroup 4, (**F**), V heavy chain subgroup 5, and (**G**), V heavy chain subgroup 6. The number of each amino acid found at each position is tabulated together with the statistical analysis of the data. Abbreviations are the same as in Table 4.

### Examples

### Example 1: Design of a Synthetic Human Combinatorial Antibody Library (HuCAL)

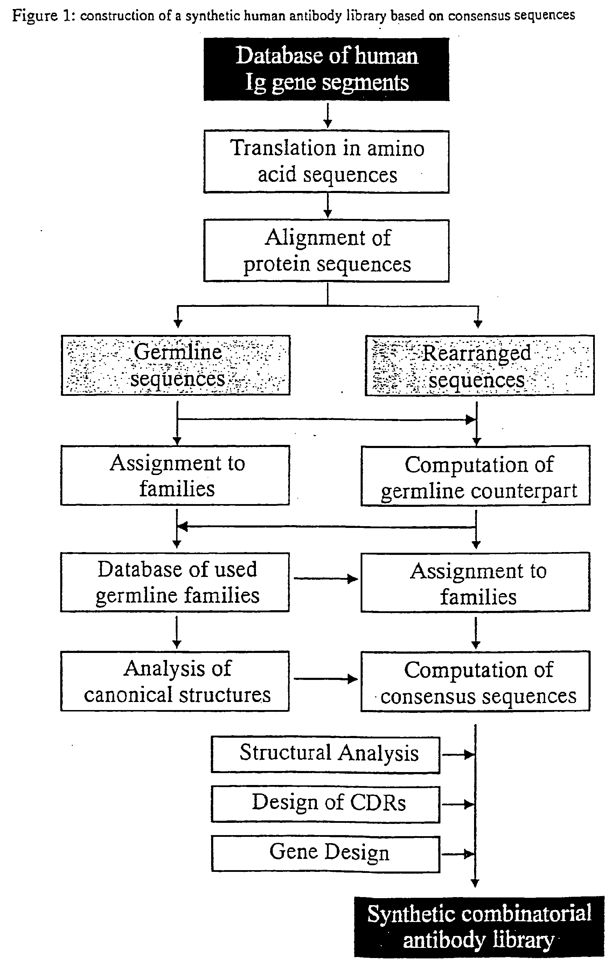

The following example describes the design of a fully synthetic human combinatorial antibody library (HuCAL), based on consensus sequences of the human immunoglobulin repertoire, and the synthesis of the consensus genes. The general procedure is outlined in Fig. 1.

### 1.1 Sequence database

### 1.1.1 Collection and alignment of human immunoglobulin sequences

In a first step, sequences of variable domains of human immunoglobulins have been collected and divided into three sub bases: V heavy chain (VH), V kappa (Vk) and V lambda (VI). For each sequence, the gene sequence was then translated into the corresponding amino acid sequence. Subsequently, all amino acid sequences were aligned according to Kabat et al. (1991). In the case of VI sequences, the numbering system of Chuchana et al. (1990) was used. Each of the three main databases was then divided into two further sub bases: the first sub base contained all sequences derived from rearranged V genes, where more than 70 positions of the sequence were known. The second sub base contained all germline gene segments (without the D- and J- minigenes; pseudogenes with internal stop codons were also removed). In all cases, where germline sequences with identical amino acid sequence but different names were found, only one sequence was used (see Table 1). The final databases of rearranged sequences contained 386, 149 and 674 entries for Vk, Vλ and VH, respectively. The final databases of germline sequences contained 48, 26 and 141 entries for Vk, Vλ and VH, respectively.

### 1.1.2 Assignment of sequences to subgroups

The sequences in the three germline databases where then grouped according to sequence homology (see also Tomlinson et al., 1992, Williams & Winter, 1993, and Cox et al., 1994). In the case of Vk, 7 families could be established. Vλ was divided into 8 families and VH into 6 families. The VH germline genes of the VH7 family (Van Dijk et al., 1993) were grouped into the VH1 family, since the genes of the two families are highly homologous. Each family contained different numbers of germline genes, varying from 1 (for example VH6) to 47 (VH3).

### 1.2 Analysis of sequences

### 1.2.1 Computation of germline membership

For each of the 1209 amino acid sequences in the databases of rearranged genes, the nearest germline counterpart, i.e. the germline sequence with the smallest number of amino acid differences was then calculated. After the germline counterpart was found, the number of somatic mutations which occurred in the rearranged gene and which led to amino acid exchanges could be tabulated. In 140 cases, the germline counterpart could not be calculated exactly, because more than one germline gene was found with an identical number of amino acid exchanges. These rearranged sequences were removed from the database. In a few cases, the number of amino acid exchanges was found to be unusually large (>20 for VL and >25 for VH), indicating either heavily mutated rearranged genes or derivation from germline genes not present in the database. Since it was not possible to distinguish between these two possibilities, these sequences were also removed from the database. Finally, 12 rearranged sequences were removed from the database because they were found to have very unusual CDR lengths and composition or unusual amino acids at canonical positions (see below). In summary, 1023 rearranged sequences out of 1209 (85%) could be clearly assigned to their germline counterparts (see Table 2).

After this calculation, every rearranged gene could be arranged in one of the families established for the germline genes. Now the usage of each germline gene, i.e. the number of rearranged genes which originate from each germline gene, could be calculated (see Table 2). It was found that the usage was strongly biased towards a subset of germline genes, whereas most of the germline genes were not present as rearranged genes in the database and therefore apparently not used in the immune system (Table 3). This observation had already been reported in the case of Vk (Cox, et al., 1994). All germline gene families, where no or only very few rearranged counterparts could be assigned, were removed from the database, leaving 4 Vk, 3 Vλ, and 6 VH families.

### 1.2.2 Analysis of CDR conformations

The conformation of the antigen binding loops of antibody molecules, the CDRs, is strongly dependent on both the length of the CDRs and the amino acid residues located at the so-called canonical positions (Chothia & Lesk, 1987). It has been found that only a few canonical structures exist, which determine the structural repertoire of the immunoglobulin variable domains (Chothia et al., 1989). The canonical amino acid positions can be found in CDR as well as framework regions. The 13 used germline families defined above (7 VL and 6 VH) were now analyzed for their canonical structures in order to define the structural repertoire encoded in these families.

In 3 of the 4 Vk families (Vk1, 2 and 4), one different type of CDR1 conformation could be defined for every family. The family Vk3 showed two types of CDR1 conformation: one type which was identical to Vk1 and one type only found in Vk3. All Vk CDR2s used the same type of canonical structure. The CDR3 conformation is not encoded in the germline gene segments. Therefore, the 4 Vk families defined by sequence homology and usage corresponded also to 4 types of canonical structures found in Vk germline genes.

The 3 Vλ families defined above showed 3 types of CDR1 conformation, each family with one unique type. The Vλ1 family contained 2 different CDR1 lengths (13 and 14 amino acids), but identical canonical residues, and it is thought that both lengths adopt the same canonical conformation (Chothia & Lesk, 1987). In the CDR2 of the used Vλ germlines, only one canonical conformation exists, and the CDR3 conformation is not encoded in the germline gene segments. Therefore, the 3 Vλ families defined by sequence homology and usage corresponded also to 3 types of canonical structures.

The structural repertoire of the human VH sequences was analyzed in detail by Chothia et al., 1992. In total, 3 conformations of CDR1 (H1-1, H1-2 and H1-3) and 6 conformations of CDR2 (H2-1, H2-2, H2-3, H2-4, H2-5 and H2-x) could be defined. Since the CDR3 is encoded in the D- and J-minigene segments, no particular canonical residues are defined for this CDR.
All the members of the VH1 family defined above contained the CDR1 conformation H1-1, but differed in their CDR2 conformation: the H2-2 conformation was found in 6 germline genes, whereas the conformation H2-3 was found in 8 germline genes. Since the two types of CDR2 conformations are defined by different types of amino acid at the framework position 72, the VH1 family was divided into two subfamilies: VH1 A with CDR2 conformation H2-2 and VH1 B with the conformation H2-3. The members of the VH2 family all had the conformations H1-3 and H2-1 in CDR1 and CDR2, respectively. The CDR1 conformation of the VH3 members was found in all cases to be H1-1, but 4 different types were found in CDR2 (H2-1, H2-3, H2-4 and H2-x). In these CDR2 conformations, the canonical framework residue 71 is always defined by an arginine. Therefore, it was not necessary to divide the VH3 family into subfamilies, since the 4 types of CDR2 conformations were defined solely by the CDR2 itself. The same was true for the VH4 family. Here, all 3 types of CDR1 conformations were found, but since the CDR1 conformation was defined by the CDR itself (the canonical framework residue 26 was found to be glycine in all cases), no subdivisions were necessary. The CDR2 conformation of the VH4 members was found to be H2-1 in all cases. All members of the VH5 family were found to have the conformation H1-1 and H2-2, respectively. The single germline gene of the VH6 family had the conformations H1-3 and H2-5 in CDR1 and CDR2, respectively.

In summary, all possible CDR conformations of the Vk and Vλ genes were present in the 7 families defined by sequence comparison. From the 12 different CDR conformations found in the used VH germline genes, 7 could be covered by dividing the family VH1 into two subfamilies, thereby creating 7 VH families. The remaining 5 CDR conformations (3 in the VH3 and 2 in the VH4 family) were defined by the CDRs themselves and could be created during the construction of CDR libraries. Therefore, the structural repertoire of the used human V genes could be covered by 49 (7 x 7) different frameworks.

### 1.2.3 Computation of consensus sequences

The 14 databases of rearranged sequences (4 Vk, 3 Vλ and 7 VH) were used to compute the HuCAL consensus sequences of each subgroup (4 HuCAL- Vk, 3 HuCAL- Vλ, 7 HuCAL- VH, see Table 4, 5 and 6). This was done by counting the number of amino acid residues used at each position (position variability) and subsequently identifying the amino acid residue most frequently used at each position. By using the rearranged sequences instead of the used germline sequences for the calculation of the consensus, the consensus was weighted according to the frequency of usage. Additionally, frequently mutated and highly conserved positions could be identified. The consensus sequences were cross-checked with the consensus of the germline families to see whether the rearranged sequences were biased at certain positions towards amino acid residues which do not occur in the collected germline sequences, but this was found not to be the case. Subsequently, the number of differences of each of the 14 consensus sequences to each of the germline sequences found in each specific family was calculated. The overall deviation from the most homologous germline sequence was found to be 2.4 amino acid residues (s.d. = 2.7), ensuring that the "artificial" consensus sequences can still be considered as truly human sequences as far as immunogenicity is concerned.

### 1.3 Structural analysis

So far, only sequence information was used to design the consensus sequences. Since it was possible that during the calculation certain artificial combinations of amino acid residues have been created, which are located far away in the sequence but have contacts to each other in the three dimensional structure, leading to destabilized or even misfolded frameworks, the 14 consensus sequences were analyzed according to their structural properties.
It was rationalized that all rearranged sequences present in the database correspond to functional and therefore correctly folded antibody molecules. Hence, the most homologous rearranged sequence was calculated for each consensus sequence. The positions where the consensus differed from the rearranged sequence were identified as potential "artificial residues" and inspected.
The inspection itself was done in two directions. First, the local sequence stretch around each potentially "artificial residue" was compared with the corresponding stretch of all the rearranged sequences. If this stretch was found to be truly artificial, i.e. never occurred in any of the rearranged sequences, the critical residue was converted into the second most common amino acid found at this position and analyzed again. Second, the potentially "artificial residues" were analyzed for their long range interactions. This was done by collecting all available structures of human antibody variable domains from the corresponding PDB files and calculating for every structure the number and type of interactions each amino acid residue established to each side-chain. These "interaction maps" were used to analyze the probable side-chain/side-chain interactions of the potentially "artificial residues". As a result of this analysis, the following residues were exchanged (given is the name of the gene, the position according to Kabat's numbering scheme, the amino acid found at this position as the most abundant one and the amino acid which was used instead):

| | |
|---|---|
| VH2: | S₆₅T |
| Vk1: | N₃₄A, |
| Vk3: | G₉A, D₆₀A, R₇₇S |
| Vλ3: | V₇₈T |

### 1.4 Design of CDR sequences

The process described above provided the complete consensus sequences derived solely from the databases of rearranged sequences. It was rationalized that the CDR1 and CDR2 regions should be taken from the databases of used germline sequences, since the CDRs of rearranged and mutated sequences are biased towards their particular antigens. Moreover, the germline CDR sequences are known to allow binding to a variety of antigens in the primary immune response, where only CDR3 is varied. Therefore, the consensus CDRs obtained from the calculations described above were replaced by germline CDRs in the case of VH and Vk. In the case of Vλ, a few amino acid exchanges were introduced in some of the chosen germline CDRs in order to avoid possible protease cleavage sites as well as possible structural constraints.

The CDRs of following germline genes have been chosen:

| HuCAL gene | CDR1 | CDR2 |
|---|---|---|
| HuCAL-VH1A | VH1-12-1 | VH1-12-1 |
| HuCAL-VH1 B | VH1-13-16 | VH1-13-6,-7,-8,-9 |
| HuCAL-VH2 | VH2-31-10,-11,-12,-13 | VH2-31-3,-4 |
| HuCAL-VH3 | VH3-13-8,-9,-10 | VH3-13-8,-9,-10 |
| HuCAL-VH4 | VH4-11-7 to -14 | VH4-11-8,-9,-11,-12,-14,-16 |
| | | VH4-31-17,-18,-19,-20 |
| HuCAL-VH5 | VH5-12-1,-2 | VH5-12-1,-2 |
| HuCAL-VH6 | VH6-35-1 | VH6-35-1 |
| HuCAL-Vk1 | Vk1-14,-15 | Vk1-2,-3,-4,-5,-7,-8,-12,-13,-18,-19 |
| HuCAL-Vk2 | Vk2-6 | Vk2-6 |
| HuCAL-Vk3 | Vk3-1,-4 | Vk3-4 |
| HuCAL-Vk4 | Vk4-1 | Vk4-1 |
| HuCAL-Vλ1 | HUMLV117,DPL5 | DPL5 |
| HuCAL-Vλ2 | DPL11,DPL12 | DPL12 |
| HuCAL-Vλ3 | DPL23 | HUMLV318 |

In the case of the CDR3s, any sequence could be chosen since these CDRs were planned to be the first to be replaced by oligonucleotide libraries. In order to study the expression and folding behavior of the consensus sequences in *E*. *coli,* it would be useful to have all sequences with the same CDR3, since the influence of the CDR3s on the folding behavior would then be identical in all cases. The dummy sequences QQHYTTPP and ARWGGDGFYAMDY were selected for the VL chains (kappa and lambda) and for the VH chains, respectively. These sequences are known to be compatible with antibody folding in *E*. *coli* (Carter et al., 1992).

### 1.5 Gene design

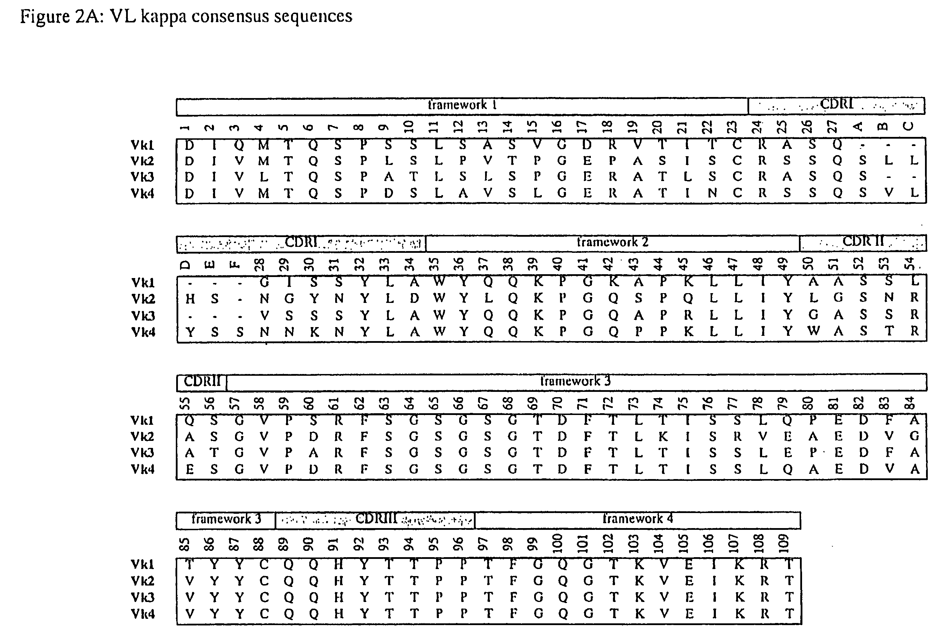

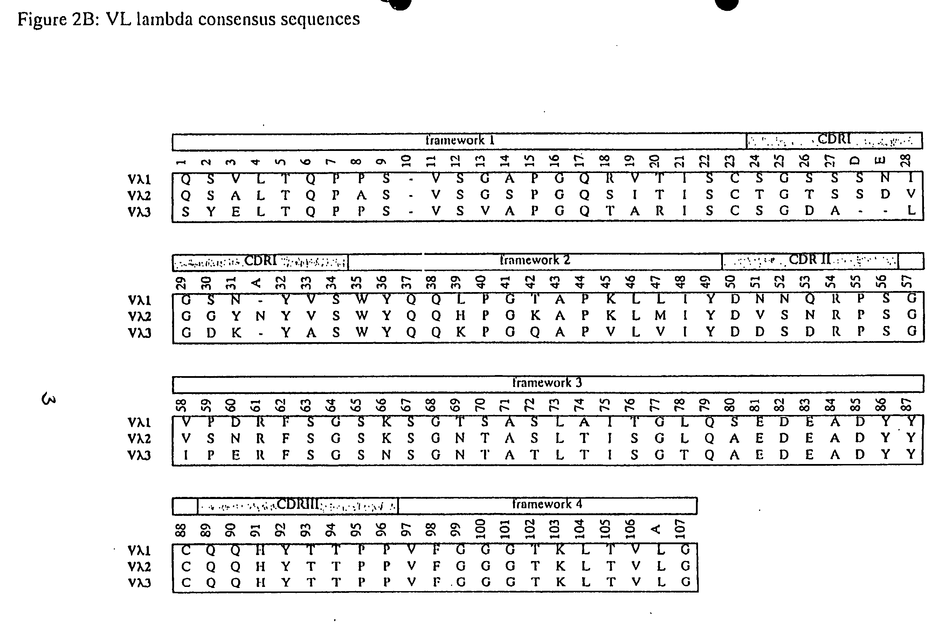

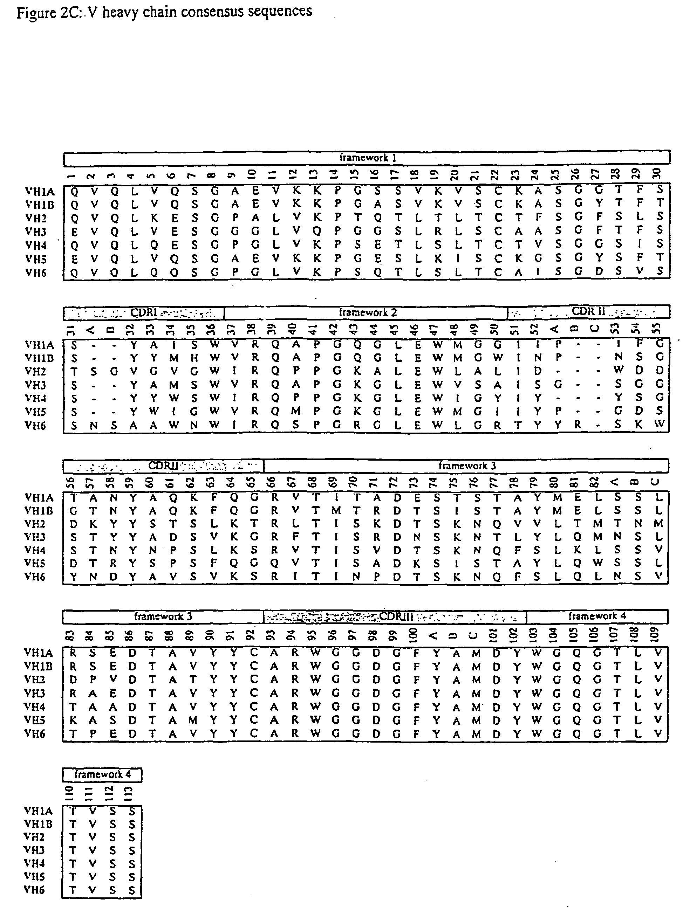

The final outcome of the process described above was a collection of 14 HuCAL amino acid sequences, which represent the frequently used structural antibody repertoire of the human immune system (see Figure 2). These sequences were back-translated into DNA sequences. In a first step, the back-translation was done using only codons which are known to be frequently used in *E. coli.* These gene sequences were then used for creating a database of all possible restriction endonuclease sites, which could be introduced without changing the corresponding amino acid sequences. Using this database, cleavage sites were selected which were located at the flanking regions of all sub-elements of the genes (CDRs and framework regions) and which could be introduced in all HuCAL VH, Vk or Vλ genes simultaneously at the same position. In a few cases it was not possible to find cleavage sites for all genes of a subgroup. When this happened, the amino acid sequence was changed, if this was possible according to the available sequence and structural information. This exchange was then analyzed again as described above. In total, the following 6 amino acid residues were exchanged during this design (given is the name of the gene, the position according to Kabat's numbering scheme, the amino acid found at this position as the most abundant one and the amino acid which was used instead):

| | |
|---|---|
| VH2: | T₃Q |
| VH6: | S₄₂G |
| Vk3: | E₁D, I₅₈V |
| Vk4: | K₂₄R |
| Vλ3: | T₂₂S |

In one case (5'-end of VH framework 3) it was not possible to identify a single cleavage site for all 7 VH genes. Two different type of cleavage sites were used instead: BstEII for HuCAL VH1A, VH1 B, VH4 and VH5, and NspV for HuCAL VH2, VH3, VH4 and VH6.

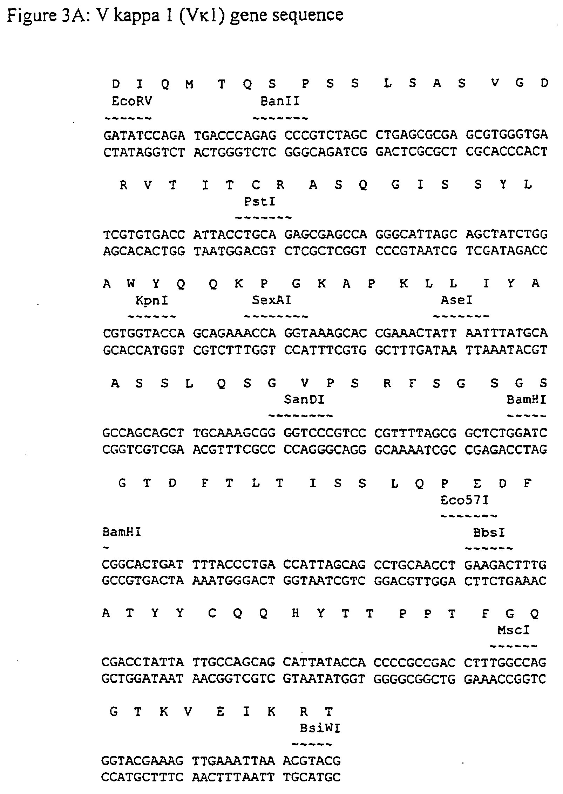

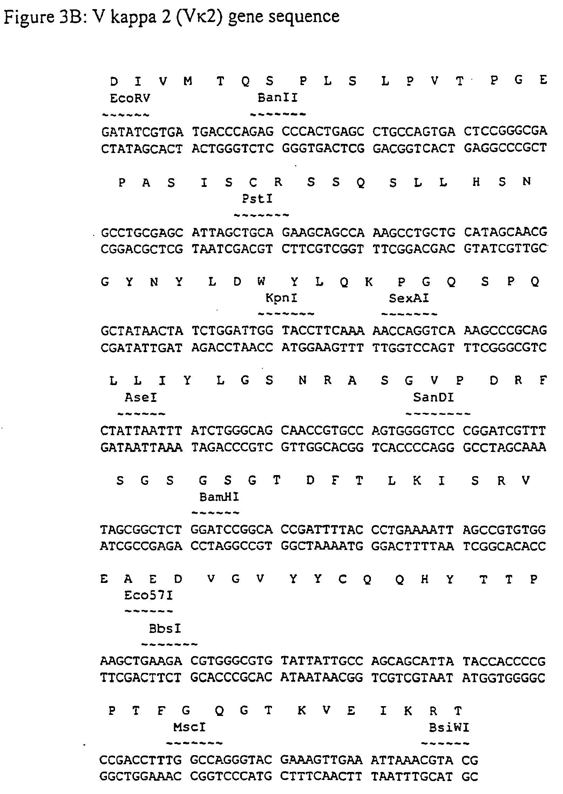

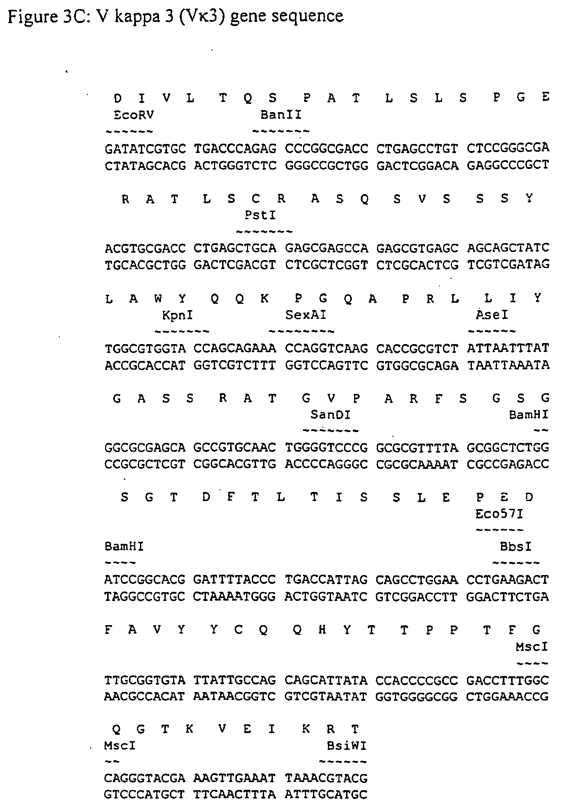

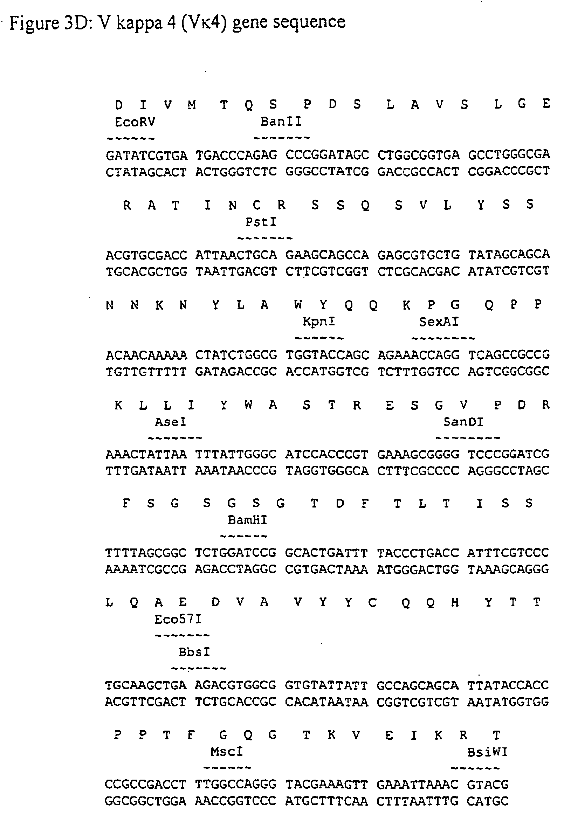

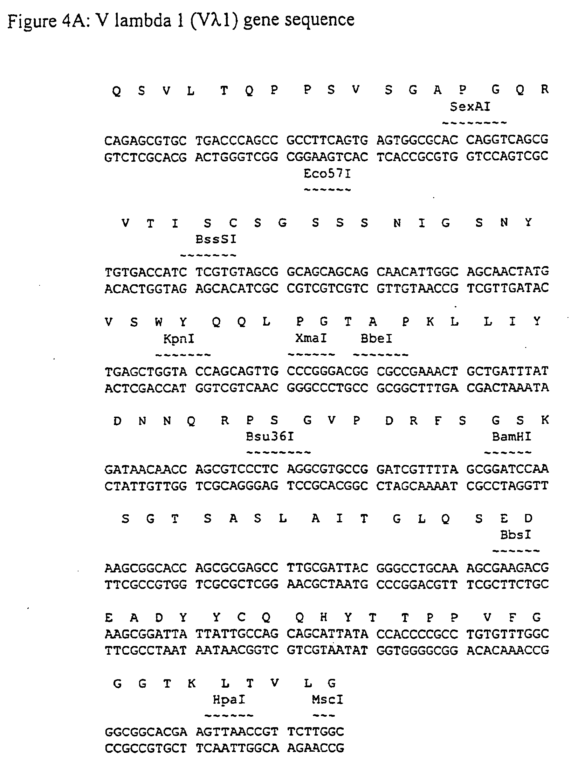

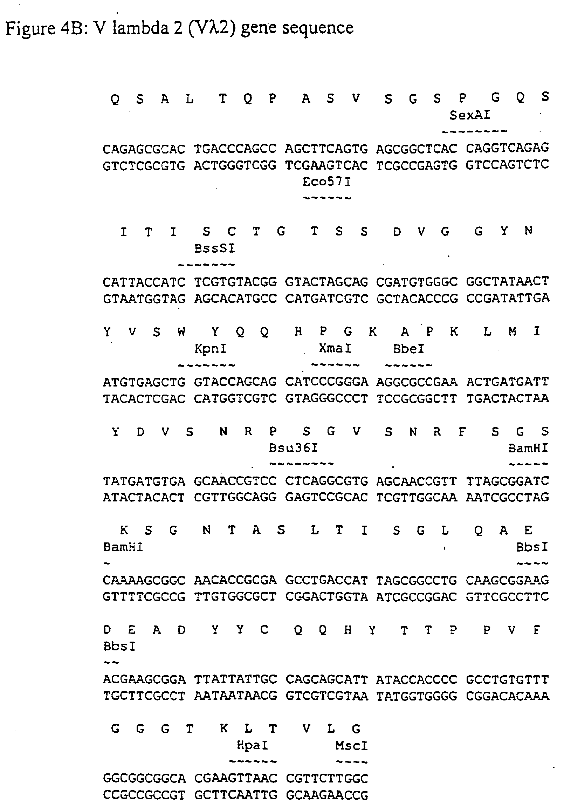

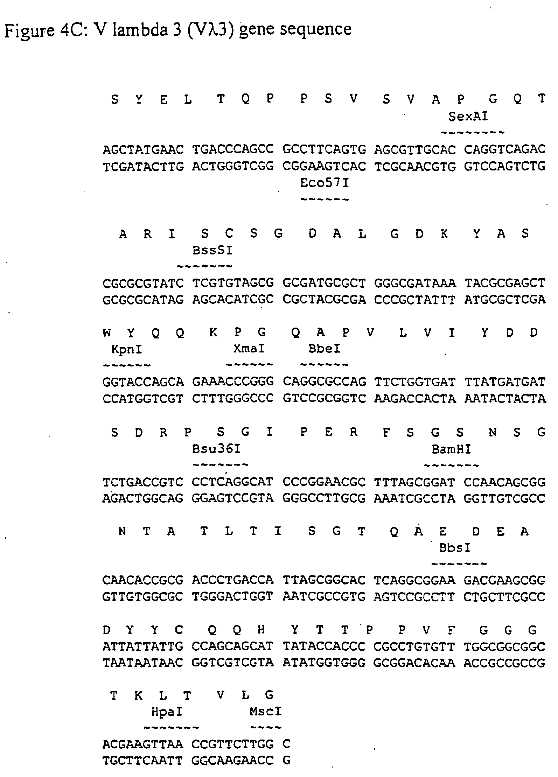

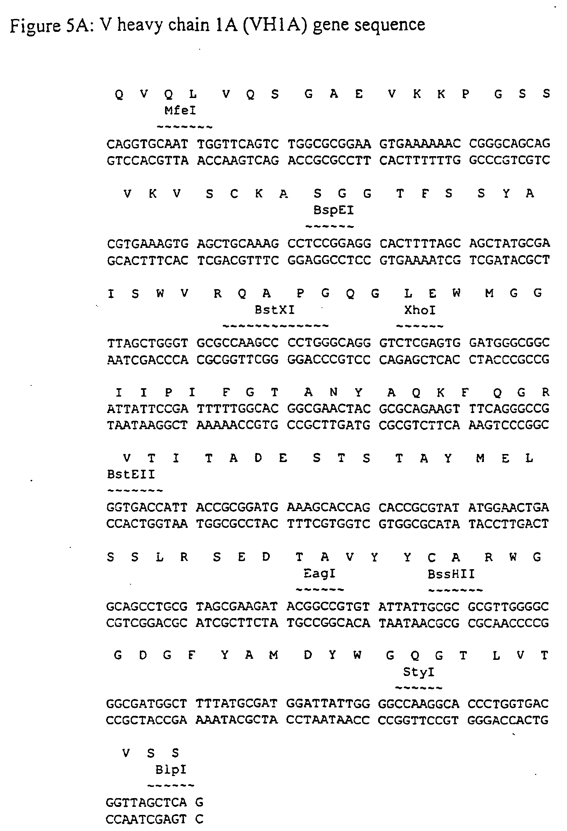

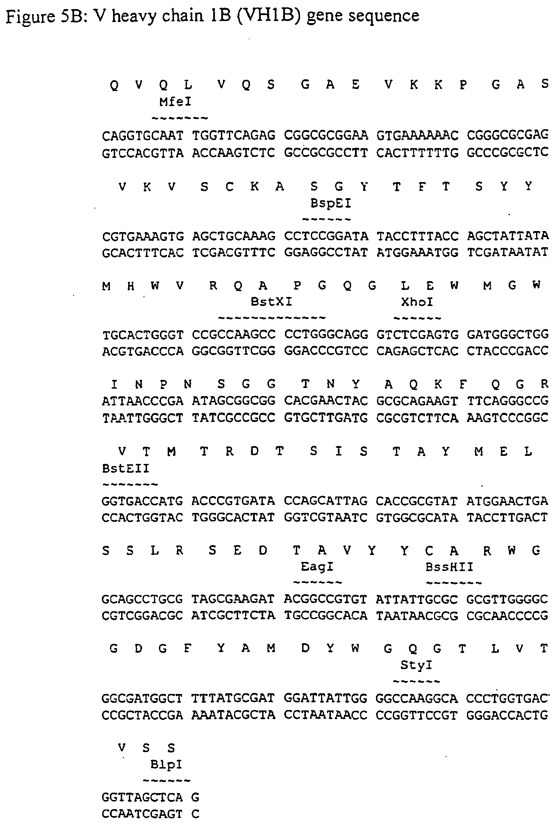

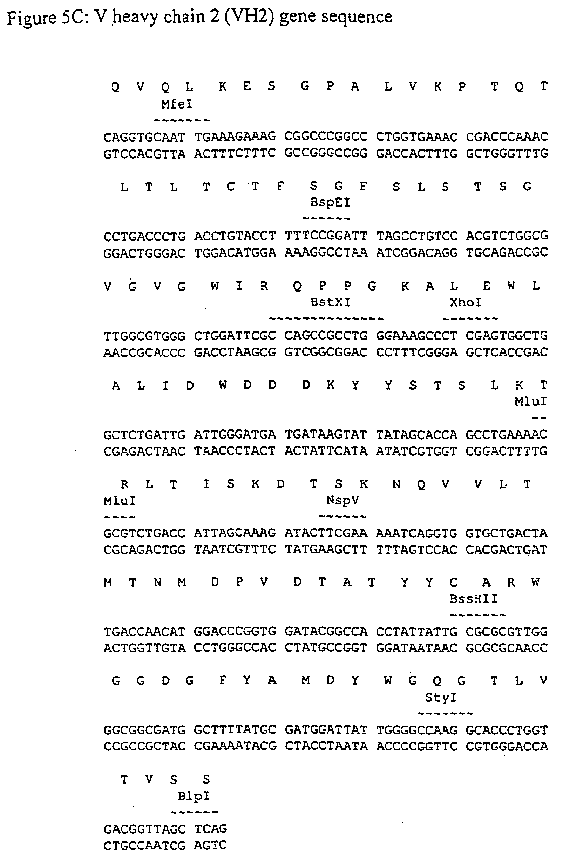

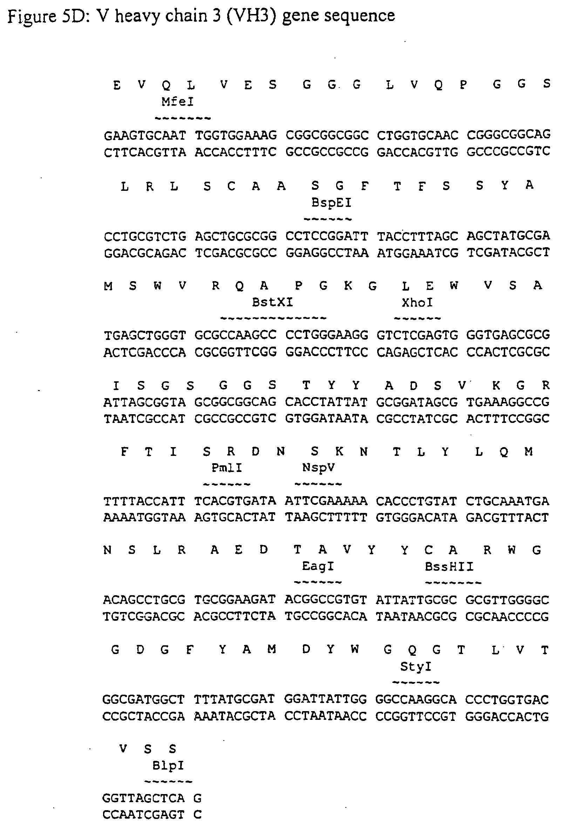

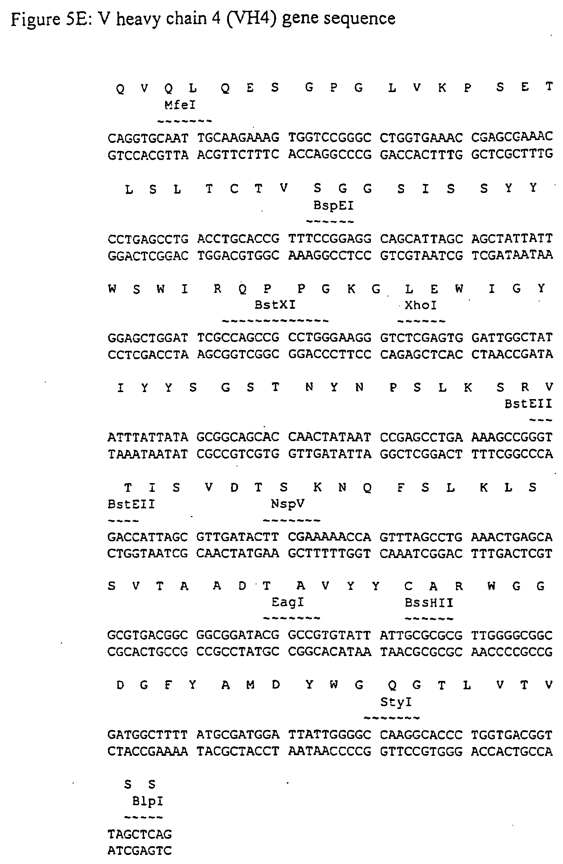

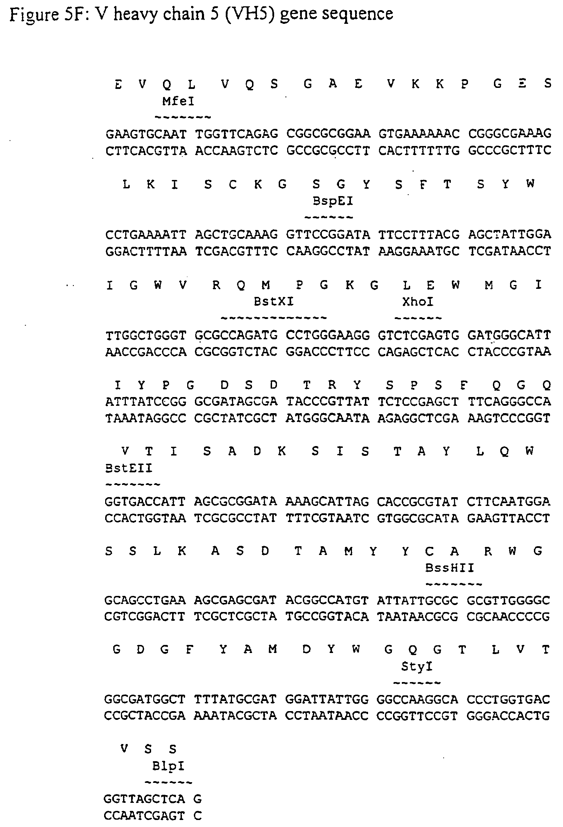

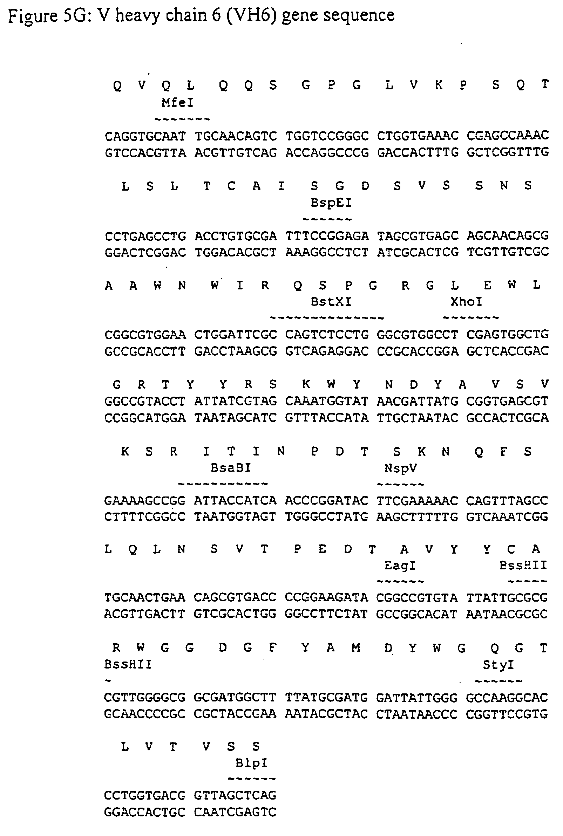

Several restriction endonuclease sites were identified, which were not located at the flanking regions of the sub-elements but which could be introduced in every gene of a given group without changing the amino acid sequence. These cleavage sites were also introduced in order to make the system more flexible for further improvements. Finally, all but one remaining restriction endonuclease sites were removed in every gene sequence. The single cleavage site, which was not removed was different in all genes of a subgroup and could be therefore used as a "fingerprint" site to ease the identification of the different genes by restriction digest. The designed genes, together with the corresponding amino acid sequences and the group-specific restriction endonuclease sites are shown in Figure 3, 4 and 5, respectively.

### 1.6 Gene synthesis and cloning

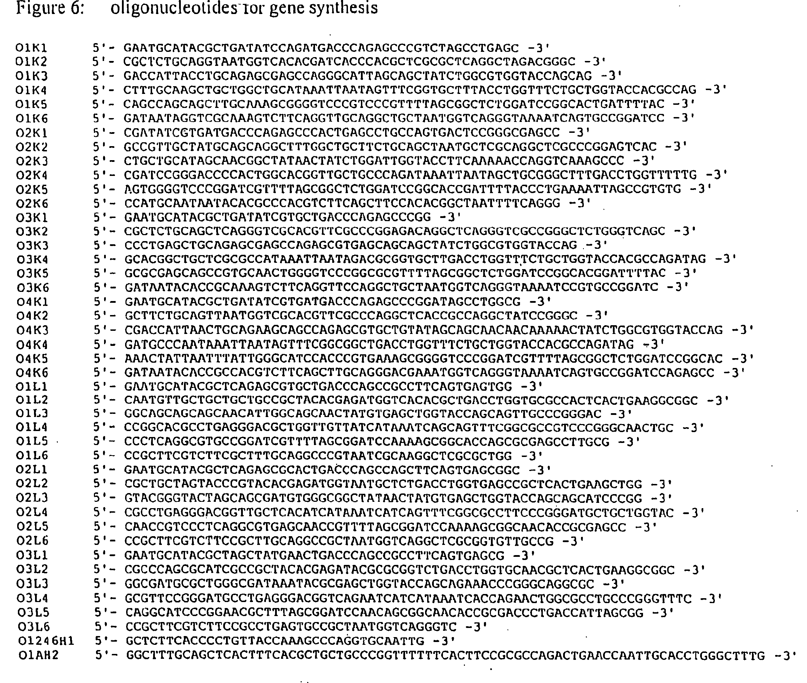

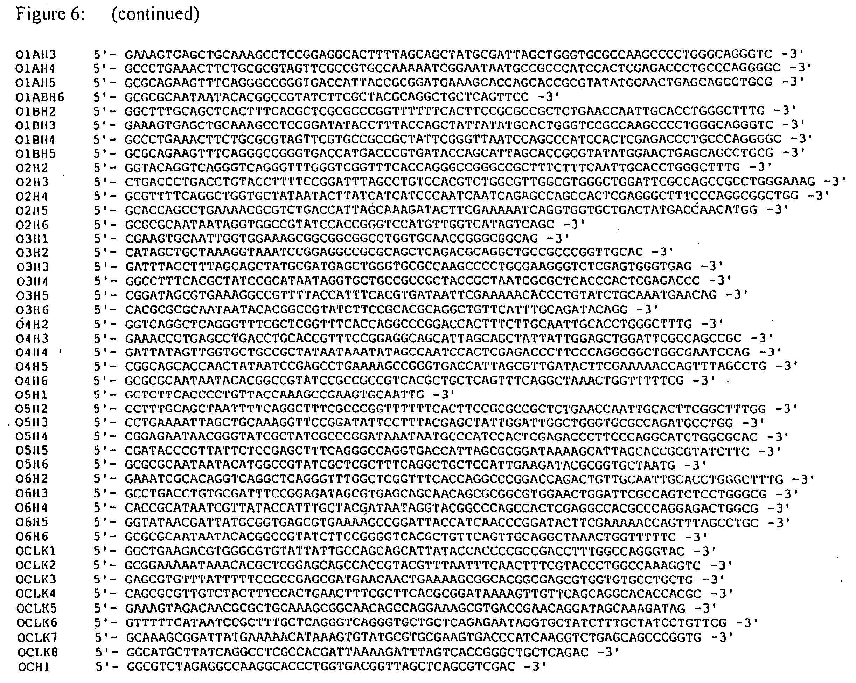

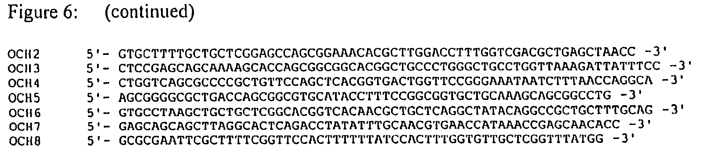

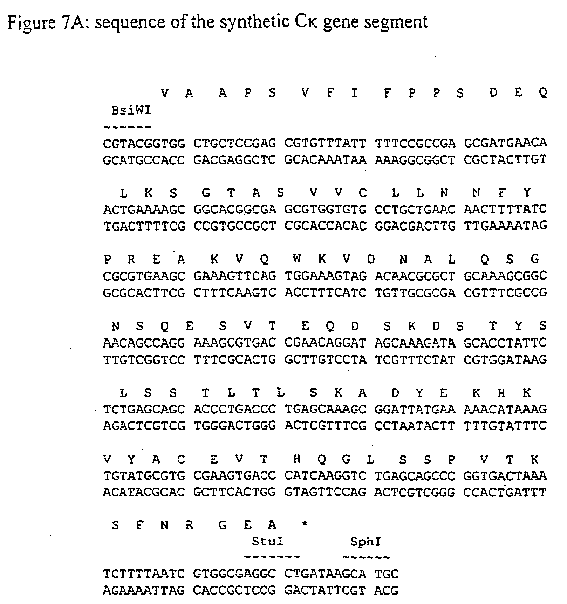

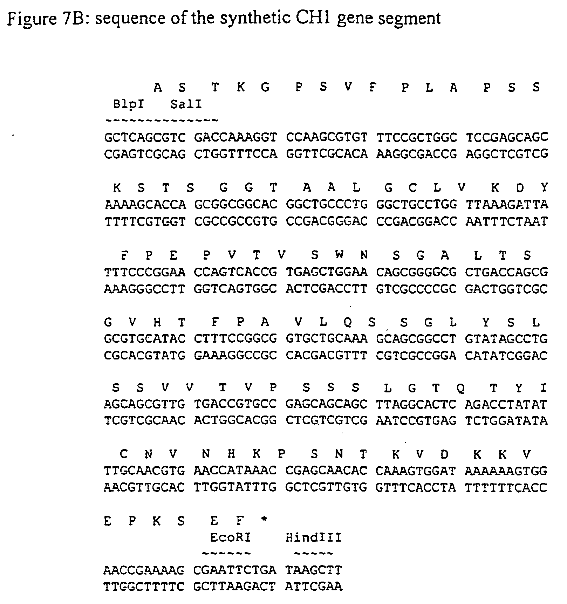

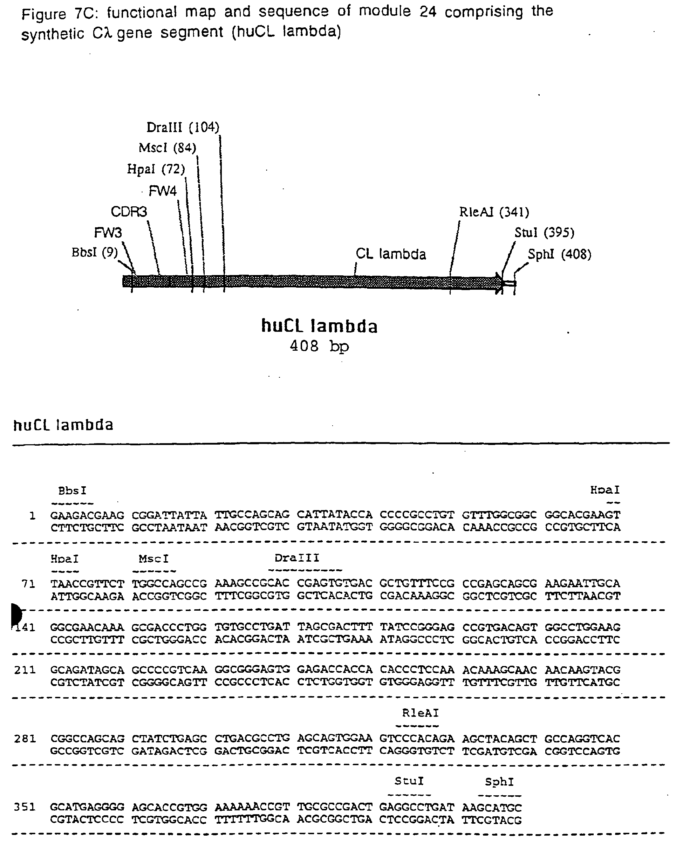

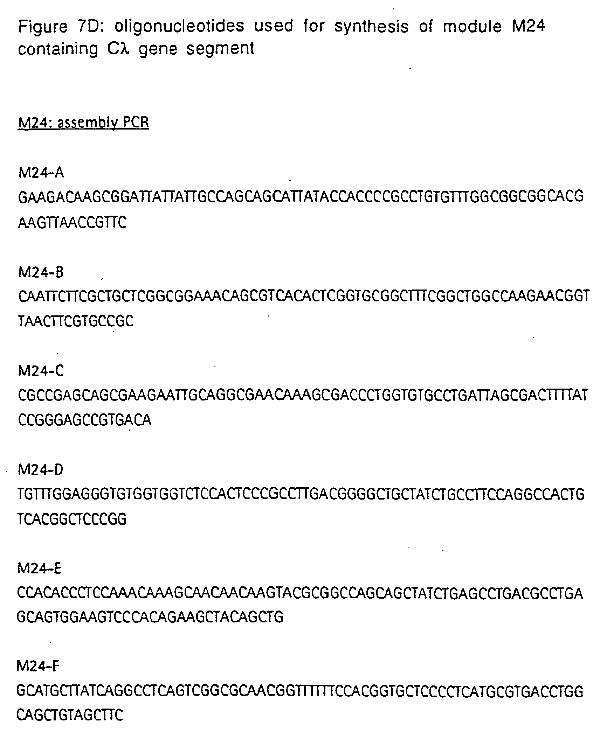

The consensus genes were synthesized using the method described by Prodromou & Pearl, 1992, using the oligonucleotides shown in Fig. 6. Gene segments encoding the human constant domains Ck, Cλ and CH1 were also synthesized, based on sequence information given by Kabat et al., 1991 (see Fig. 6 and Fig. 7). Since for both the CDR3 and the framework 4 gene segments identical sequences were chosen in all HuCAL Vk, Vλ and VH genes, respectively, this part was constructed only once, together with the corresponding gene segments encoding the constant domains. The PCR products were cloned into pCR-Script KS(+) (Stratagene, Inc.) or pZErO-1 (Invitrogen, Inc.) and verified by sequencing.

### Example 2: Cloning and Testing of a HuCAL-Based Antibody Library

A combination of two of the synthetic consensus genes was chosen after construction to test whether binding antibody fragments can be isolated from a library based on these two consensus frameworks. The two genes were cloned as a single-chain Fv (scFv) fragment, and a VH-CDR3 library was inserted. In order to test the library for the presence of functional antibody molecules, a selection procedure was carried out using the small hapten fluorescein bound to BSA (FITC-BSA) as antigen.

### 2.1 Cloning of the HuCAL VH3-Vk2 scFv fragment

In order to test the design of the consensus genes, one randomly chosen combination of synthetic light and heavy gene (HuCAL-Vk2 and HuCAL-VH3) was used for the construction of a single-chain antibody (scFv) fragment. Briefly, the gene segments encoding the VH3 consensus gene and the CH1 gene segment including the CDR3 - framework 4 region, as well as the Vk2 consensus gene and the Ck gene segment including the CDR3 - framework 4 region were assembled yielding the gene for the VH3-CH1 Fd fragment and the gene encoding the Vk2-Ck light chain, respectively. The CH1 gene segment was then replaced by an oligonucleotide cassette encoding a 20-mer peptide linker with the sequence AGGGSGGGGSGGGGSGGGGS. The two oligonucleotides encoding this linker were 5'- TCAGCGGGTGGCGGTTCTGGCGGCG GTGGGAGCGGTGGCG GTGGTTCTGGCGGTGGTGGTTCCGATATCGGTCCACGTACGG-3' and 5'-AATTCCGTACGTGGACCGATATCGGAACCACCACCGCCAGA ACCACCGCCACCGCTCCCACCGCCGCCAGAACCGCCACCCGC-3', respectively. Finally, the HuCAL-Vk2 gene was inserted *via* EcoRV and BsiWI into the plasmid encoding the HuCAL-VH3-linker fusion, leading to the final gene HuCAL-VH3-Vk2, which encoded the two consensus sequences in the single-chain format VH-linker-VL. The complete coding sequence is shown in Fig. 8.

### 2.2 Construction of a monovalent phage-display phagemid vector pIG10.3

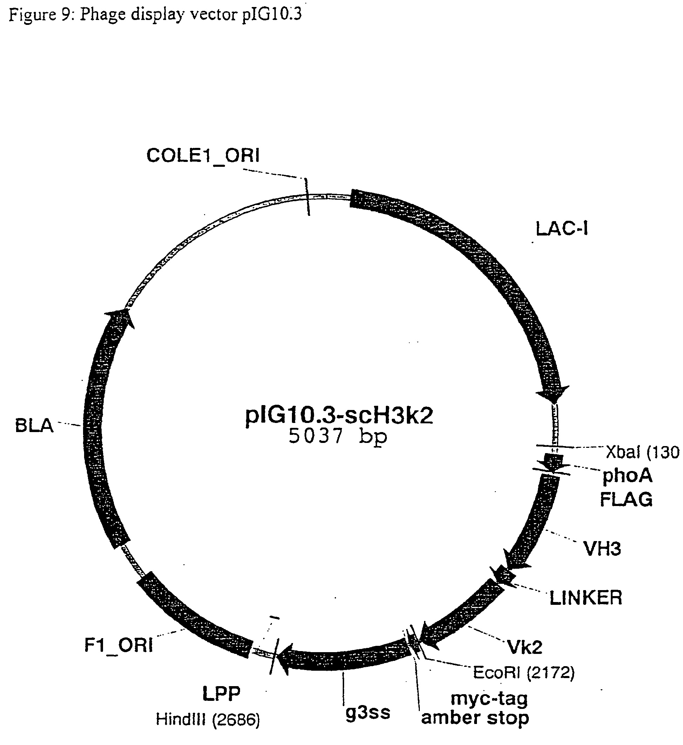

Phagemid pIG10.3 (Fig. 9) was constructed in order to create a phage-display system (Winter et al., 1994) for the H3k2 scFv gene. Briefly, the EcoRI/HindIII restriction fragment in the phagemid vector pIG10 (Ge et al., 1995) was replaced by the c-myc followed by an amber codon (which encodes an glutamate in the amber-suppresser strain XL1 Blue and a stop codon in the non-suppresser strain JM83) and a truncated version of the gene III (fusion junction at codon 249, see Lowman et al., 1991) through PCR mutagenesis.

### 2.3 Construction of H-CDR3 libraries

Heavy chain CDR3 libraries of two lengths (10 and 15 amino acids) were constructed using trinucleotide codon containing oligonucleotides (Virnekäs et al., 1994) as templates and the oligonucleotides complementing the flanking regions as primers. To concentrate only on the CDR3 structures that appear most often in functional antibodies, we kept the salt-bridge of R_{H94} and D_{H101} in the CDR3 loop. For the 15-mer library, both phenylalanine and methionine were introduced at position 100 since these two residues were found to occur quite often in human CDR3s of this length (not shown). For the same reason, valine and tyrosine were introduced at position 102. All other randomized positions contained codons for all amino acids except cystein, which was not used in the trinucleotide mixture.
The CDR3 libraries of lengths 10 and 15 were generated from the PCR fragments using oligonucleotide templates O3HCDR103T (5'- GATACGGCCGTGTATTATTGC GCGCGT(TRI)₆GATTATTGGGGCCAAGGCACC CTG-3') and O3HCDR153T (5'-GATACGGCCGTGTATTATTGCGCGCGT(TRI)₁₀(TTT/ATG)GAT(GTT/TAT)TGGGG CCAAGGCACCCTG-3'), and primers O3HCDR35 (5'-GATACGGCC GTGTATTATTGC-3') and O3HCDR33 (5'-CAGGGTGCCTTGGCCCC-3'), where TRI are trinucleotide mixtures representing all amino acids without cystein, (TTT/ATG) and (GTT/TAT) are trinucleotide mixtures encoding the amino acids phenylalanine/methionine and valine/tyrosine, respectively. The potential diversity of these libraries was 4.7 x 10⁷ and 3.4 x 10¹⁰ for 10-mer and 15-mer library, respectively. The library cassettes were first synthesized from PCR amplification of the oligo templates in the presence of both primers: 25 pmol of the oligo template O3HCDR103T or O3HCDR153T, 50 pmol each of the primers O3HCDR35 and O3HCDR33, 20 nmol of dNTP, 10x buffer and 2.5 units of Pfu DNA polymerase (Stratagene) in a total volume of 100 ml for 30 cycles (1 minute at 92°C, 1 minute at 62°C and 1 minute at 72°C). A hot-start procedure w as used. The resulting mixtures were phenol-extracted, ethanol-precipitated and digested overnight with EagI and Styl. The vector pIG10.3-scH3k2cat, where the Eagl-Styl fragment in the vector pIG10.3-scH3k2 encoding the H-CDR3 was replaced by the chloramphenicol acetyltransferase gene (cat) flanked with these two sites, was similarly digested. The digested vector (35 µg) was gel-purified and ligated with 100 µg of the library cassette overnight at 16°C. The ligation mixtures were isopropanol precipitated, air-dried and the pellets were redissolved in 100 ml of ddH2O. The ligation was mixed with 1 ml of freshly prepared electrocompetent XL1 Blue on ice. 20 rounds of electroporation were performed and the transformants were diluted in SOC medium, shaken at 37°C for 30 minutes and plated out on large LB plates (Amp/Tet/Glucose) at 37°C for 6-9 hrs. The number of transformants (library size) was 3.2x10⁷ and 2.3x10⁷ for the 10-mer and the 15-mer library, respectively. The colonies were suspended in 2xYT medium (Amp/Tet/Glucose) and stored as glycerol culture.

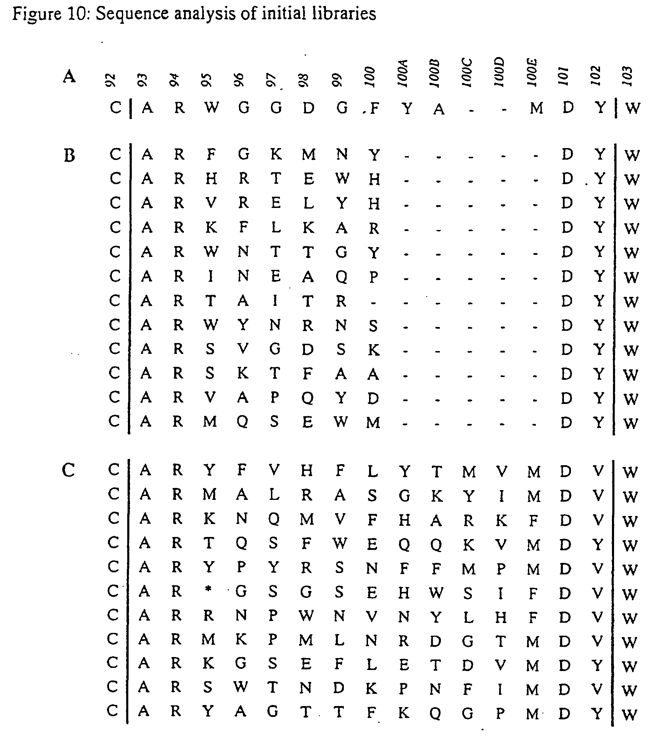

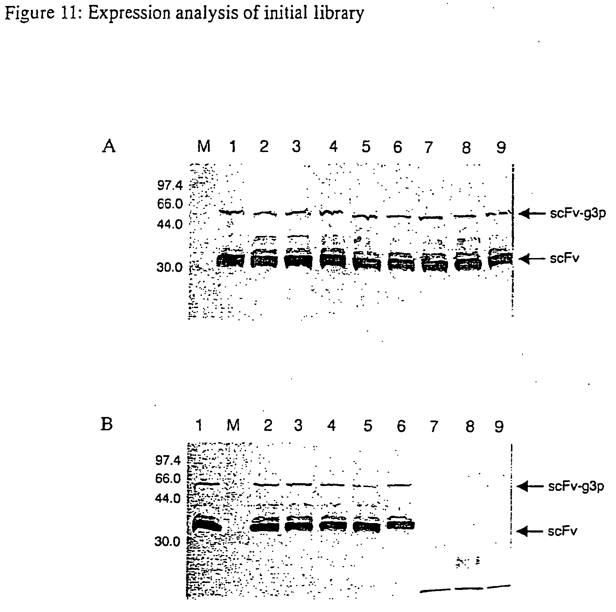

In order to test the quality of the initial library, phagemids from 24 independent colonies (12 from the 10-mer and 12 from the 15-mer library, respectively) were isolated and analyzed by restriction digestion and sequencing. The restriction analysis of the 24 phagemids indicated the presence of intact vector in all cases. Sequence analysis of these clones (see Fig. 10) indicated that 22 out of 24 contained a functional sequence in their heavy chain CDR3 regions. 1 out of 12 clones of the 10-mer library had a CDR3 of length 9 instead of 10, and 2 out of 12 clones of the 15-mer library had no open reading frame, thereby leading to a non-functional scFv; one of these two clones contained two consecutive inserts, but out of frame (data not shown). All codons introduced were presented in an even distribution.
Expression levels of individual library members were also measured. Briefly, 9 clones from each library were grown in 2xYT medium containing Amp/Tet/0.5% glucose at 37°C overnight. Next day, the cultures were diluted into fresh medium with Amp/Tet. At an OD₆₀₀nm of 0.4, the cultures were induced with 1 mM of IPTG and shaken at RT overnight. Then the cell pellets were suspended in 1 ml of PBS buffer + 1 mM of EDTA. The suspensions were sonicated and the supernatants were separated on an SDS-PAGE under reducing conditions, blotted on nylon membrane and detected with anti-FLAG M1 antibody (see Fig. 11). From the nine clones of the 10-mer library, all express the scFv fragments. Moreover, the gene III / scFv fusion proteins were present in all cases. Among the nine clones from the 15-mer library analyzed, 6/9 (67%) led to the expression of both scFv and the gene III/scFv fusion proteins. More importantly, all clones expressing the scFvs and gene III/scFv fusions gave rise to about the same level of expression.

### 2.4 Biopanning

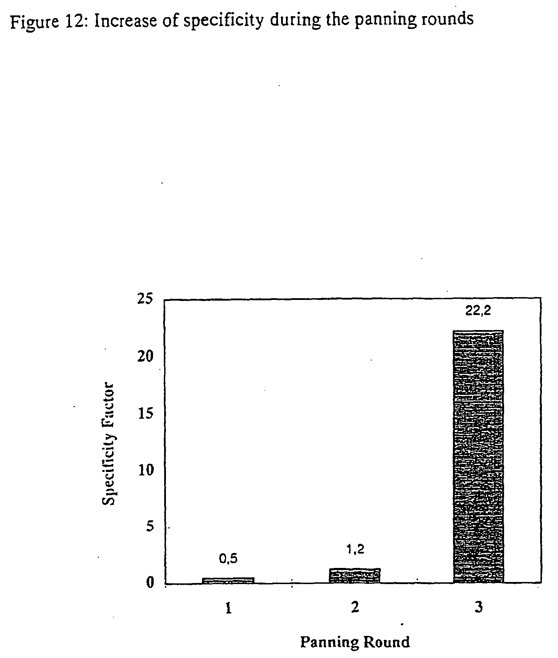

Phages displaying the antibody libraries were prepared using standard protocols. Phages derived from the 10-mer library were mixed with phages from the 15-mer library in a ratio of 20:1 (1x10¹⁰ cfu/well of the 10-mer and 5x10⁸ cfu/well of the 15-mer phages, respectively). Subsequently, the phage solution was used for panning in ELISA plates (Maxisorp, Nunc) coated with FITC-BSA (Sigma) at concentration of 100 µg/ml in PBS at 4°C overnight. The antigen-coated wells were blocked with 3% powder milk in PBS and the phage solutions in 1% powder milk were added to each well and the plate was shaken at RT for 1 hr. The wells were then washed with PBST and PBS (4 times each with shaking at RT for 5 minutes). The bound phages were eluted with 0.1 M triethylamine (TEA) at RT for 10 minutes. The eluted phage solutions were immediately neutralized with 1/2 the volume of 1 M Tris·Cl, pH 7.6. Eluted phage solutions (ca. 450 µl) were used to infect 5 ml of XL1 Blue cells at 37°C for 30 min. The infected cultures were then plated out on large LB plates (Amp/Tet/Glucose) and allowed to grow at 37°C until the colonies were visible. The colonies were suspended in 2xYT medium and the glycerol cultures were made as above described. This panning round was repeated twice, and in the third round elution was carried out with addition of fluorescein in a concentration of 100 µg/ml in PBS. The enrichment of specific phage antibodies was monitored by panning the initial as well as the subsequent fluorescein-specific sub-libraries against the blocking buffer (Fig. 12). Antibodies with specificity against fluorescein were isolated after 3 rounds of panning.

### 2.5 ELISA measurements

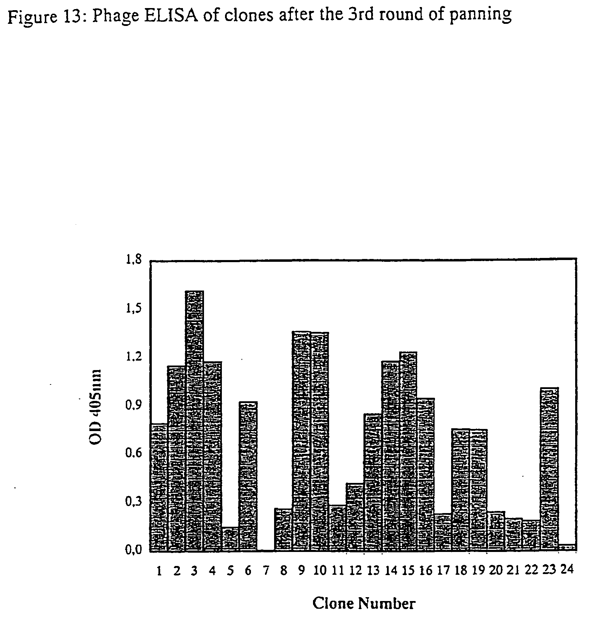

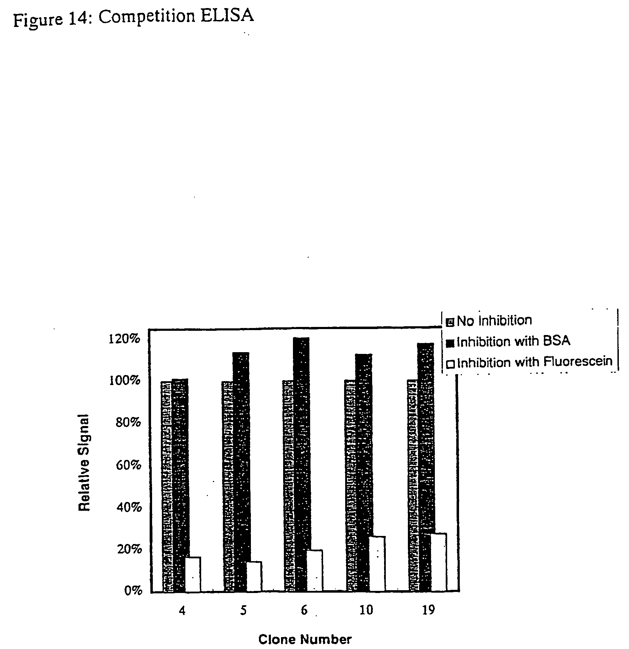

One of the criteria for the successful biopanning is the isolation of individual phage clones that bind to the targeted antigen or hapten. We undertook the isolation of anti-FITC phage antibody clones and characterized them first in a phage ELISA format. After the 3rd round of biopanning (see above), 24 phagemid containing clones were used to inoculate 100 µl of 2xYT medium (Amp/Tet/Glucose) in an ELISA plate (Nunc), which was subsequently shaken at 37°C for 5 hrs. 100 µl of 2xYT medium (Amp/Tet/1 mM IPTG) were added and shaking was continued for 30 minutes. A further 100 µl of 2xYT medium (Amp/Tet) containing the helper phage (1 x 10⁹ cfu/well) was added and shaking was done at RT for 3 hrs. After addition of kanamycin to select for successful helper phage infection, the shaking was continued overnight. The plates were then centrifuged and the supernatants were pipetted directly into ELISA wells coated with 100 µl FITC-BSA (100µg/ml) and blocked with milk powder. Washing was performed similarly as during the panning procedure and the bound phages were detected with anti-M13 antibody-POD conjugate (Pharmacia) using soluble POD substrate (Boehringer-Mannheim). Of the 24 clones screened against FITC-BSA, 22 were active in the ELISA (Fig. 13). The initial libraries of similar titer gave rise to no detectable signal.
Specificity for fluorescein was measured in a competitive ELISA. Periplasmic fractions of five FITC specific scFvs were prepared as described above. Western blotting indicated that all clones expressed about the same amount of scFv fragment (data not shown). ELISA was performed as described above, but additionally, the periplasmic fractions were incubated 30 min at RT either with buffer (no inhibition), with 10 mg/ml BSA (inhibition with BSA) or with 10 mg/ml fluorescein (inhibition with fluorescein) before adding to the well. Binding scFv fragment was detected using the anti-FLAG antibody M1. The ELISA signal could only be inhibited, when soluble fluorescein was added, indicating binding of the scFvs was specific for fluorescein (Fig. 14).

### 2.6 Sequence analysis

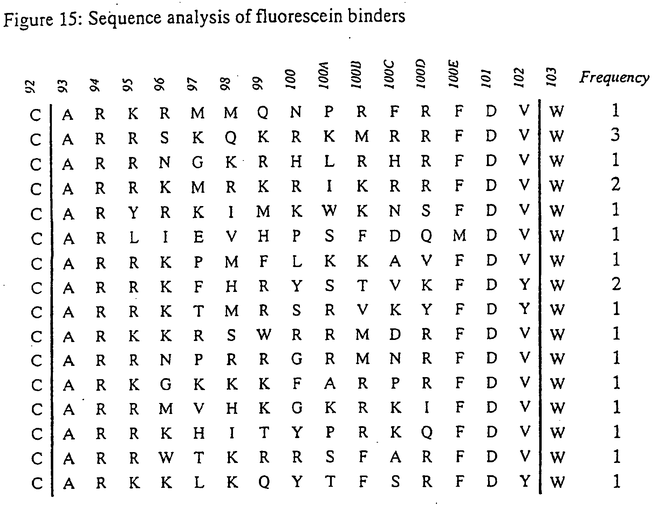

The heavy chain CDR3 region of 20 clones were sequenced in order to estimate the sequence diversity of fluorescein binding antibodies in the library (Fig. 15). In total, 16 of 20 sequences (80%) were different, showing that the constructed library contained a highly diverse repertoire of fluorescein binders. The CDR3s showed no particular sequence homology, but contained on average 4 arginine residues. This bias towards arginine in fluorescein binding antibodies had already been described by Barbas et al., 1992.

### 2.7 Production

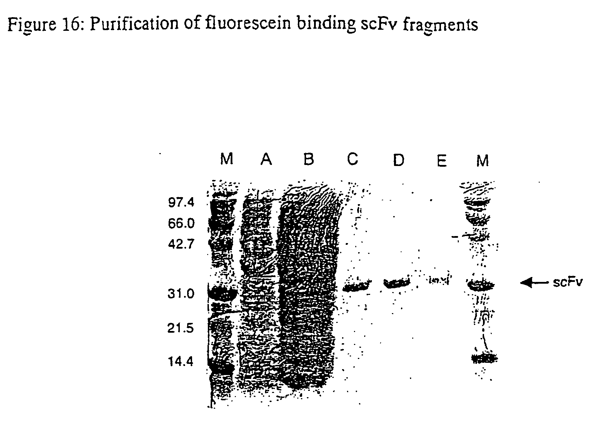

*E. coli* JM83 was transformed with phagemid DNA of 3 selected clones and cultured in 0.5 L 2xYT medium. Induction was carried out with 1 mM IPTG at OD₆₀₀nm = 0.4 and growth was continued with vigorous shaking at RT overnight. The cells were harvested and pellets were suspended in PBS buffer and sonicated. The supernatants were separated from the cell debris via centrifugation and purified via the BioLogic system (Bio-Rad) by with a POROS^{®}MC 20 column (IMAC, PerSeptive Biosystems, Inc.) coupled with an ion-exchange chromatography column. The ion-exchange column was one of the POROS^{®}HS, CM or HQ or PI 20 (PerSeptive Biosystems, Inc.) depended on the theoretical pi of the scFv being purified. The pH of all the buffers was adjusted to one unit lower or higher than the µl of the scFv being purified throughout. The sample was loaded onto the first IMAC column, washed with 7 column volumes of 20 mM sodium phosphate, 1 M NaCl and 10 mM imidazole. This washing was followed by 7 column volumes of 20 mM sodium phosphate and 10 mM imidazole. Then 3 column volumes of an imidazole gradient (10 to 250 mM) were applied and the eluent was connected directly to the ion-exchanger. Nine column volumes of isocratic washing with 250 mM imidazole was followed by 15 column volumes of 250 mM to 100 mM and 7 column volumes of an imidazole / NaCl gradient (100 to 10 mM imidazole, 0 to 1 M NaCl). The flow rate was 5 ml/min. The purity of scFv fragments was checked by SDS-PAGE Coomassie staining (Fig. 16). The concentration of the fragments was determined from the absorbance at 280 nm using the theoretically determined extinction coefficient (Gill & von Hippel, 1989). The scFv fragments could be purified to homogeneity (see Fig. 16). The yield of purified fragments ranged from 5 to 10 mg/L/OD.

### Example 3: HuCAL H3k2 Library Against a Collection of Antigens

In order to test the library used in Example 2 further, a new selection procedure was carried out using a variety of antigens comprising β-estradiol, testosterone, Lewis-Y epitope (LeY), interleukin-2 (IL-2), lymphotoxin-β (LT-β), E-selectin ligand-1 (ESL-1), and BSA.

### 3.1 Biopanning

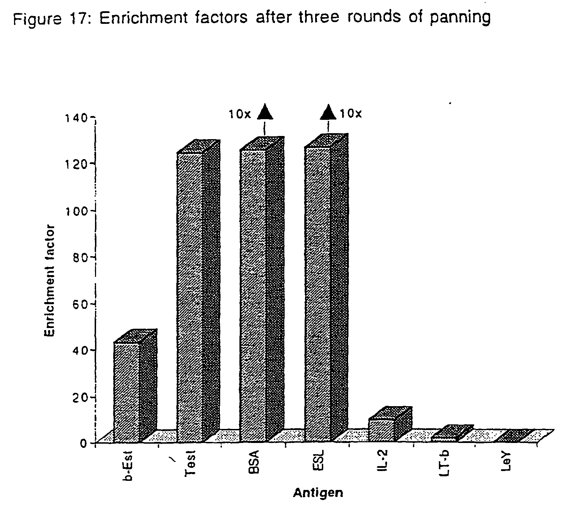

The library and all procedures were identical to those described in Example 2. The ELISA plates were coated with β-estradiol-BSA (100 µg/ml), testosterone-BSA (100 µg/ml), LeY-BSA (20 µg/ml) IL-2 (20 µg/ml), ESL-1 (20 µg/ml) and BSA (100 µg/ml), LT-β (denatured protein, 20 µg/ml). In the first two rounds, bound phages were eluted with 0.1 M triethylamine (TEA) at RT for 10 minutes. In the case of BSA, elution after three rounds of panning was carried out with addition of BSA in a concentration of 100 µg/ml in PBS. In the case of the other antigens, third round elution was done with 0.1 M triethylamine. In all cases except LeY, enrichment of binding phages could be seen (Figure 17). Moreover, a repetition of the biopanning experiment using only the 15-mer library resulted in the enrichment of LeY-binding phages as well (data not shown).

### 3.2. ELISA measurements

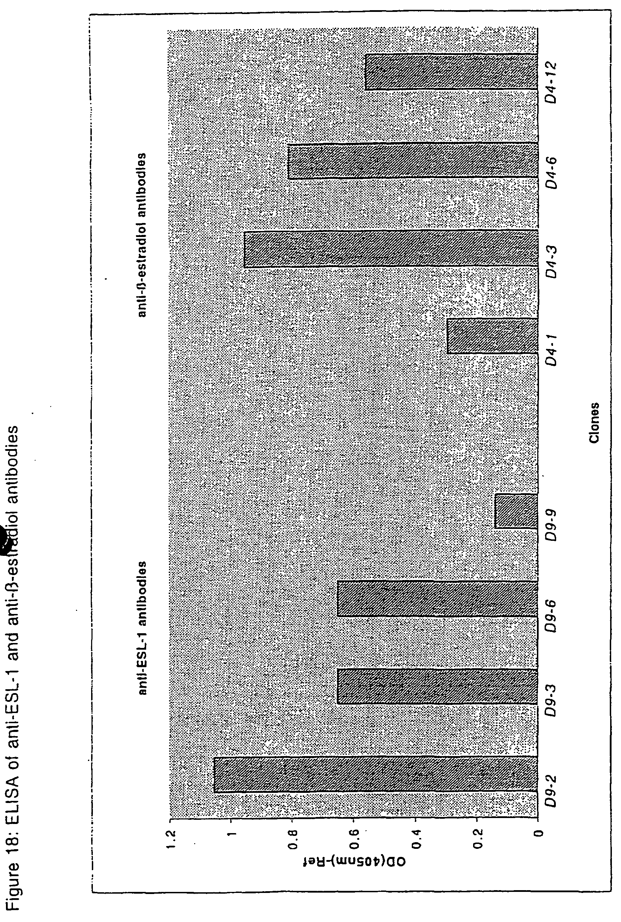

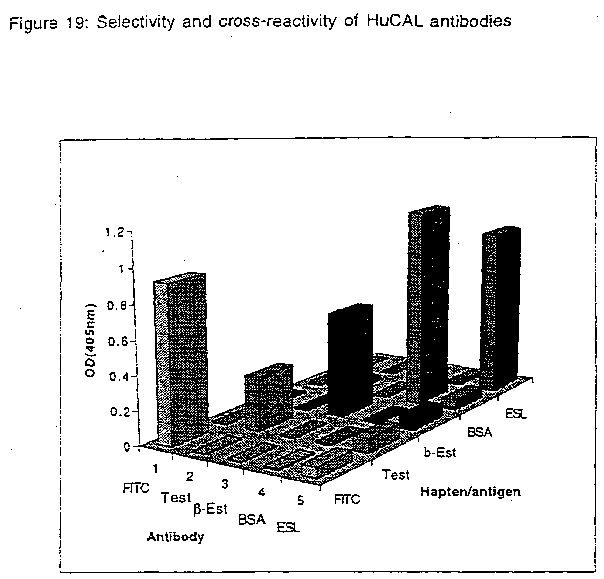

Clones binding to β-estradiol, testosterone, LeY, LT-β, ESL-1 and BSA were further analyzed and characterized as described in Example 2 for FITC. ELISA data for anti-β-estradiol and anti-ESL-1 antibodies are shown in Fig. 18. In one experiment, selectivity and cross-reactivity of binding scFv fragments were tested. For this purpose, an ELISA plate was coated with FITC, testosterone, β-estradiol, BSA, and ESL-1, with 5 wells for each antigen arranged in 5 rows, and 5 antibodies, one against each of the antigens, were screened against each of the antigens. Fig. 19 shows the specific binding of the antibodies to the antigen it was selected for, and the low cross-reactivity with the other four antigens.

### 3.3 Sequence analysis

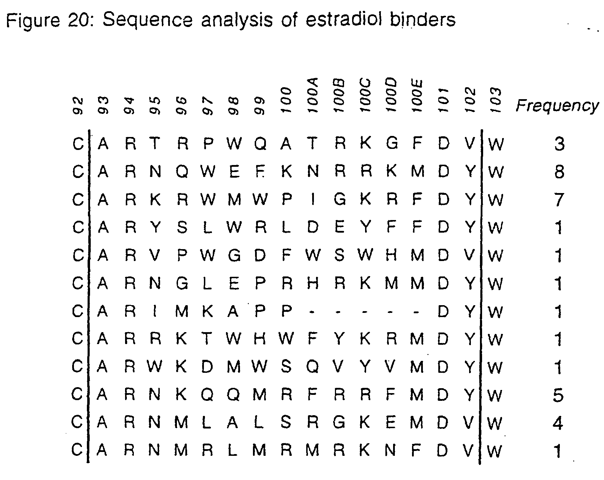

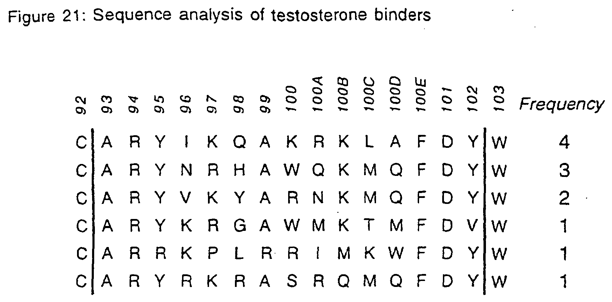

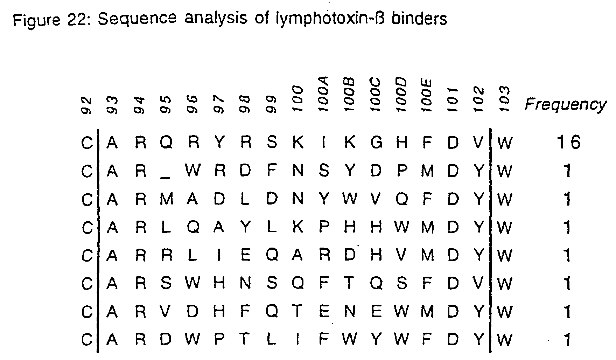

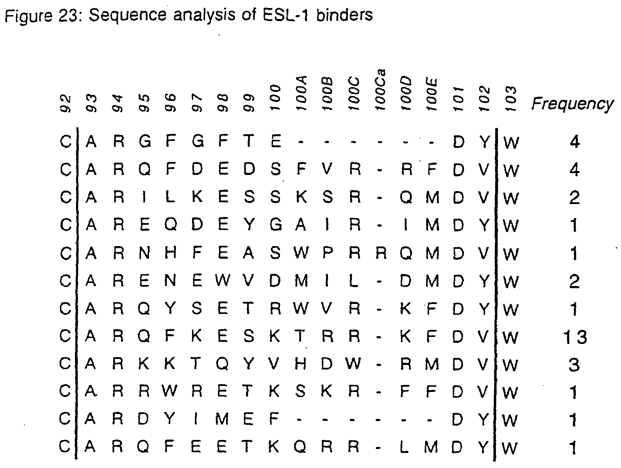

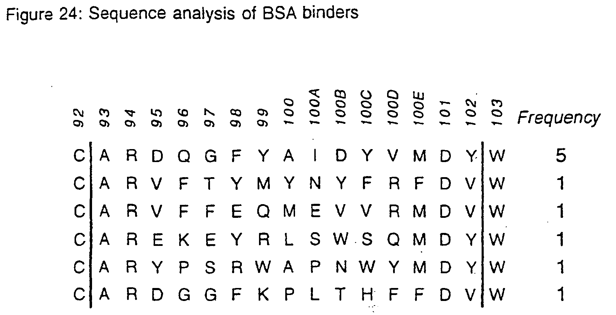

The sequencing data of several clones against β-estradiol (34 clones), testosterone (12 clones), LT-β (23 clones), ESL-1 (34 clones), and BSA (10 clones) are given in Figures 20 to 24.

### Example 4: Vector Construction

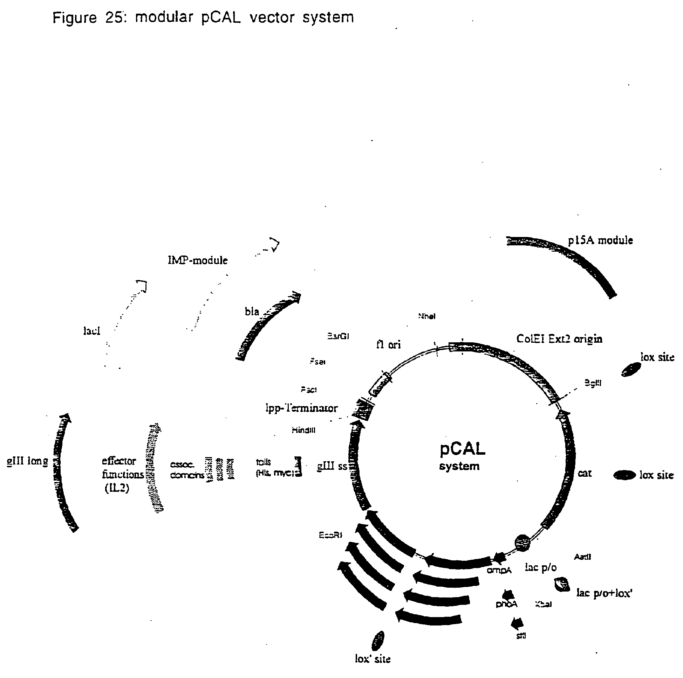

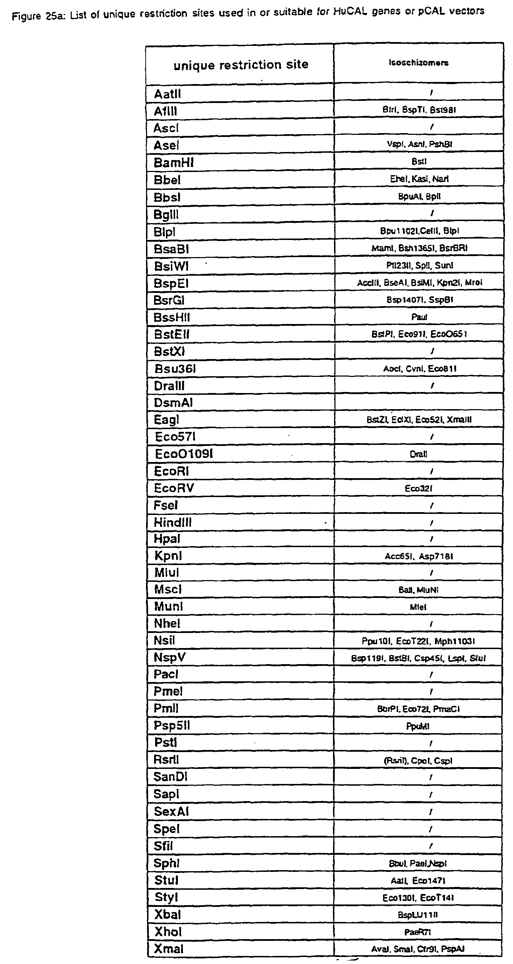

To be able to take advantage of the modularity of the consensus gene repertoire, a vector system had to be constructed which could be used in phage display screening of HuCAL libraries and subsequent optimization procedures. Therefore, all necessary vector elements such as origins of single-stranded or double-stranded replication, promotor/operator, repressor or terminator elements, resistance genes, potential recombination sites, gene III for display on filamentous phages, signal sequences, or detection tags had to be made compatible with the restriction site pattern of the modular consensus genes. Figure 25 shows a schematic representation of the pCAL vector system and the arrangement of vector modules and restriction sites therein. Figure 25a shows a list of all restriction sites which are already incorporated into the consensus genes or the vector elements as part of the modular system or which are not yet present in the whole system. The latter could be used in a later stage for the introduction of or within new modules.

### 4.1 Vector modules

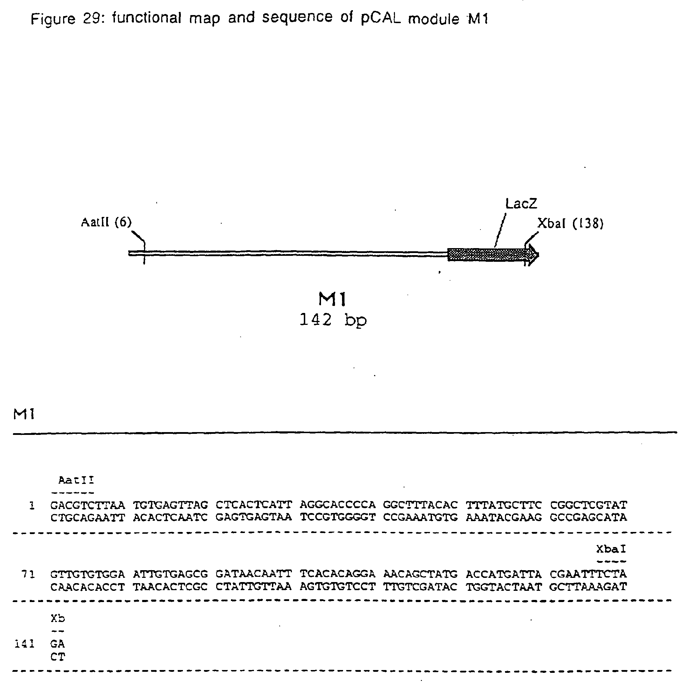

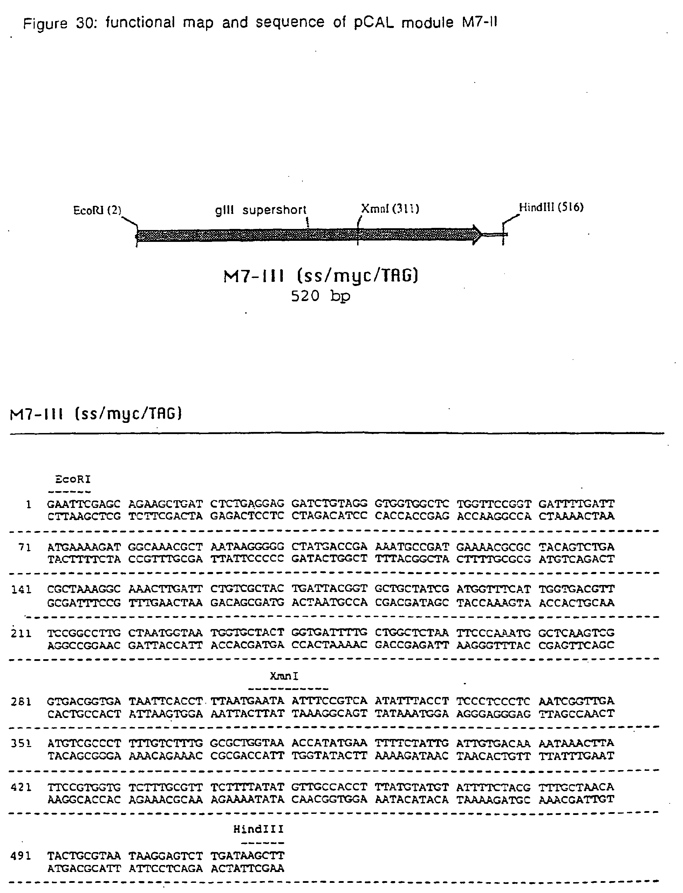

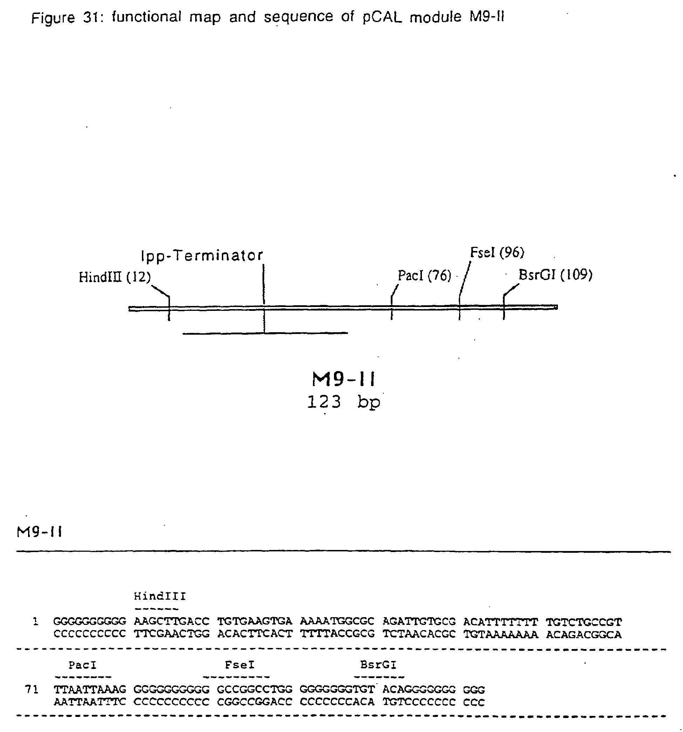

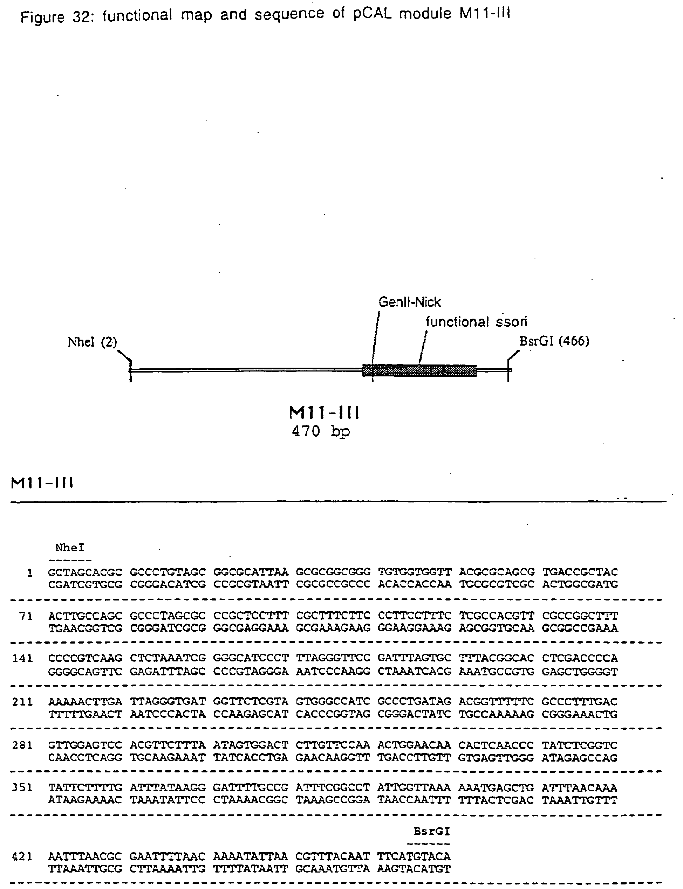

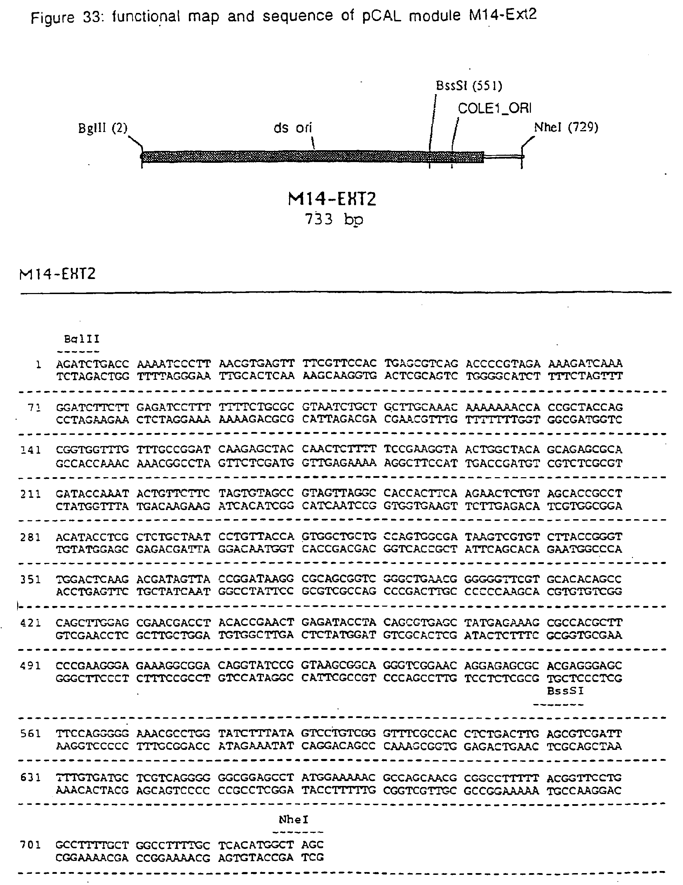

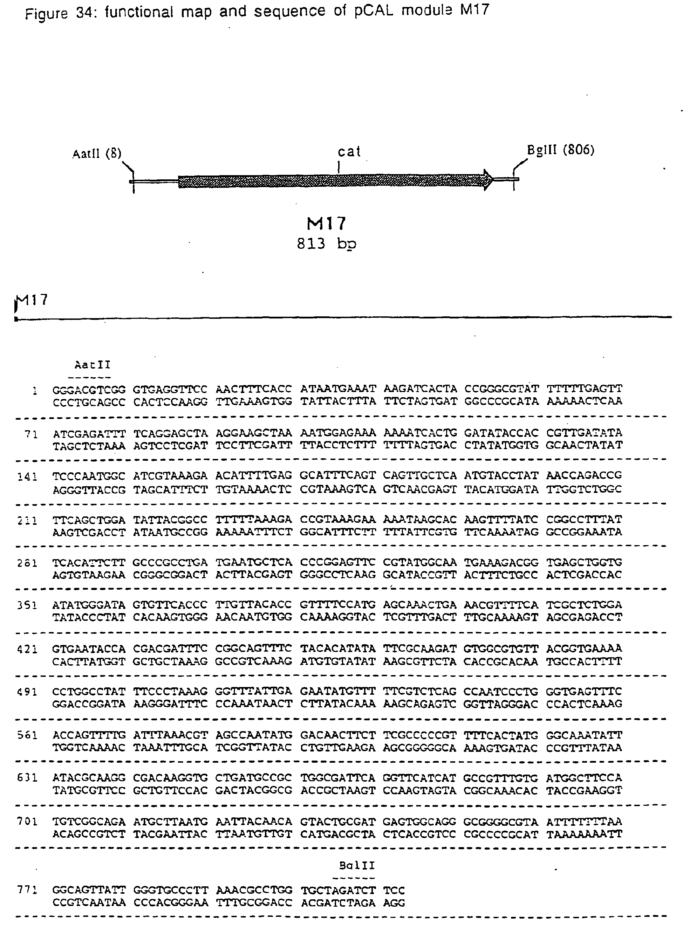

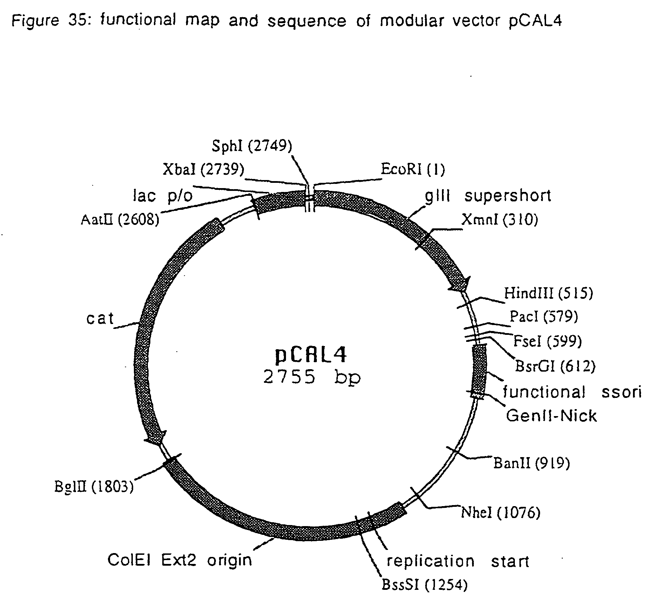

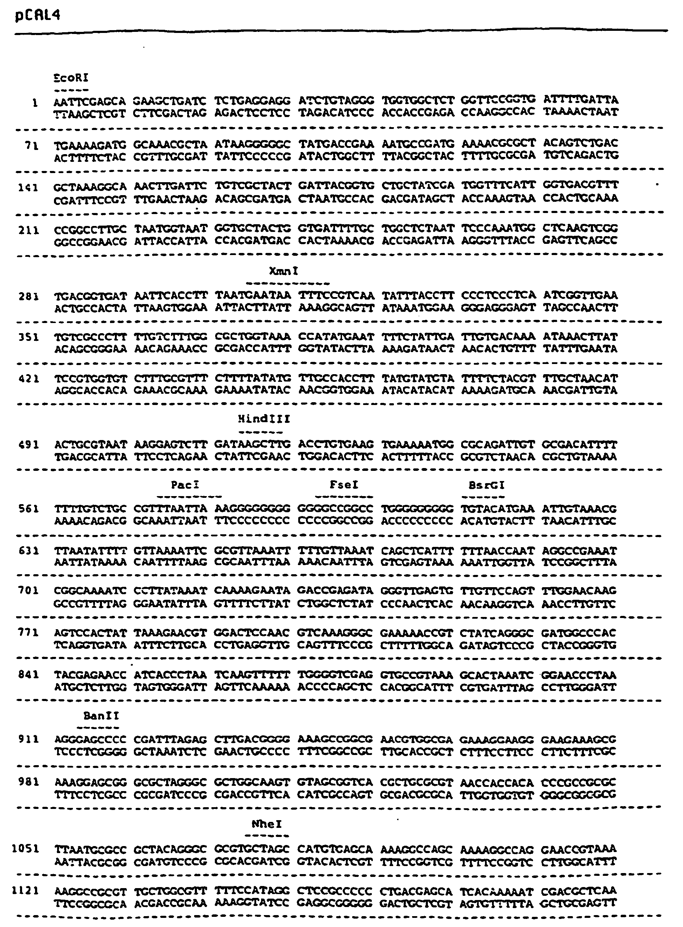

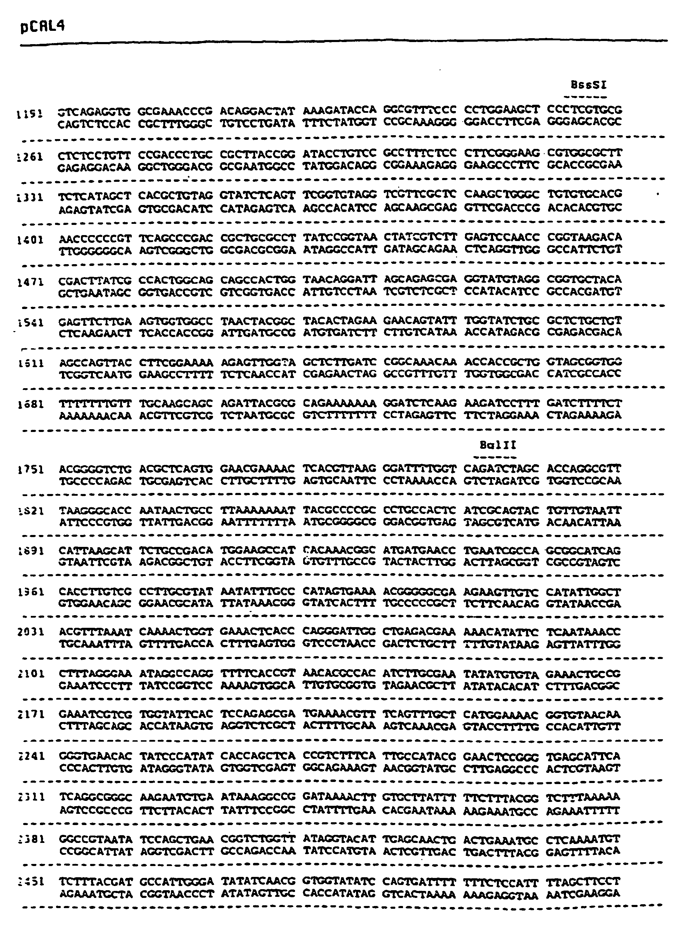

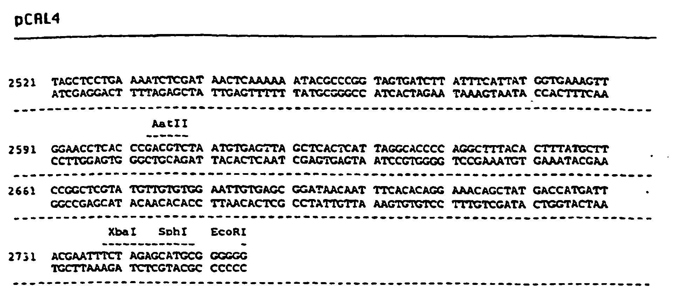

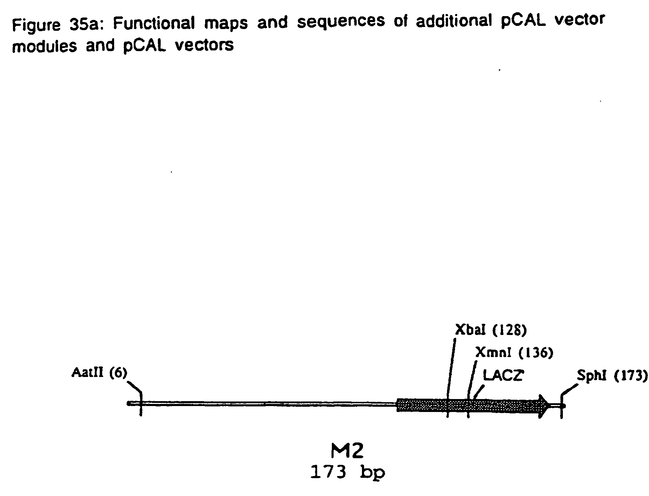

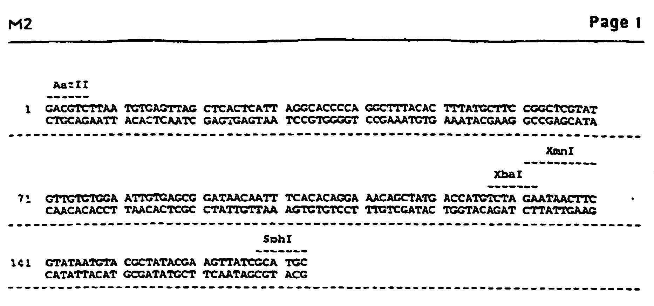

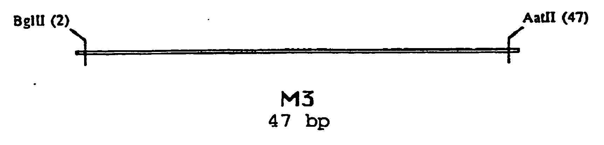

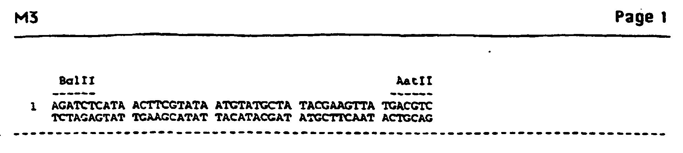

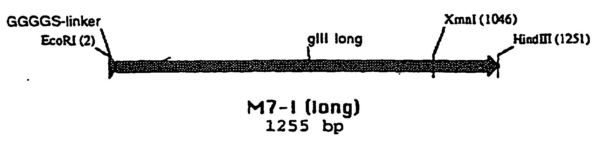

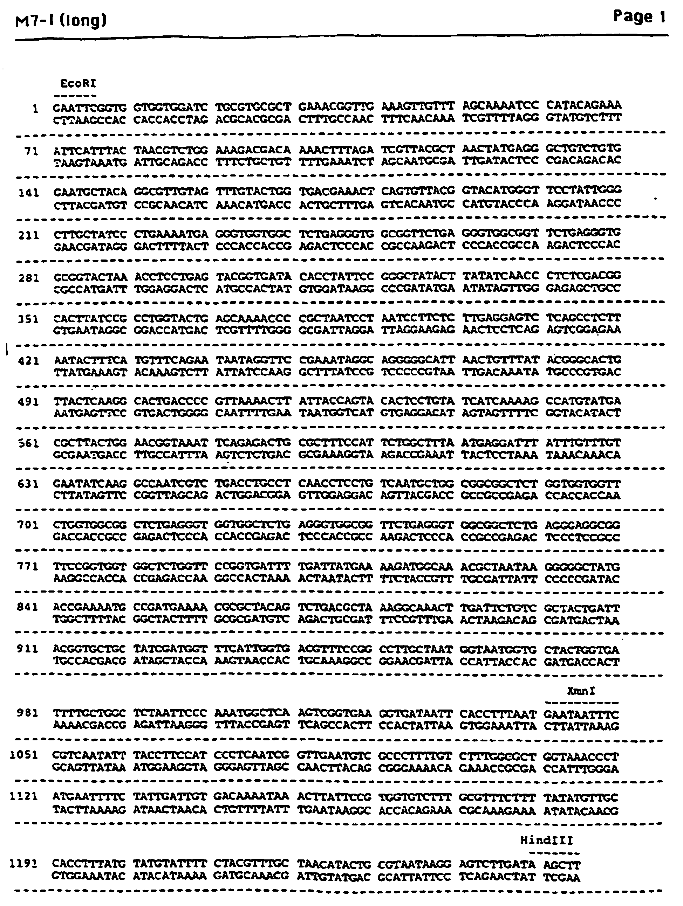

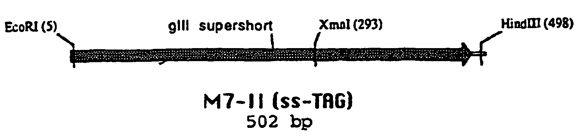

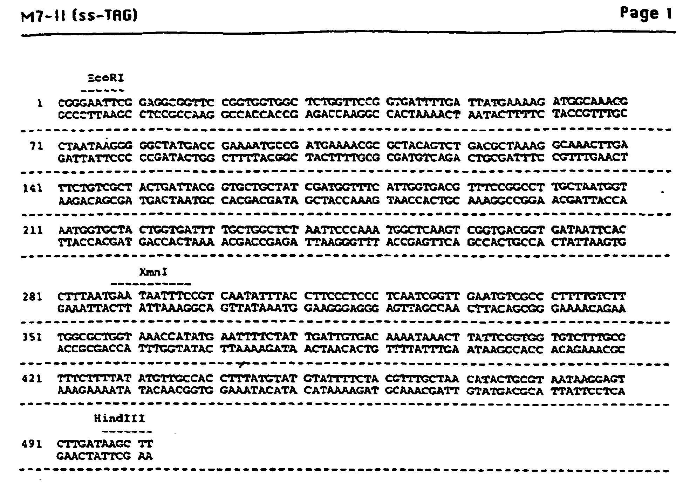

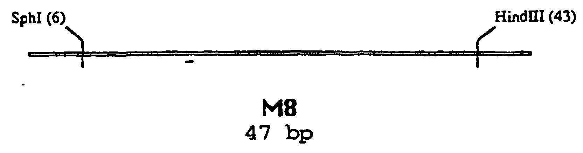

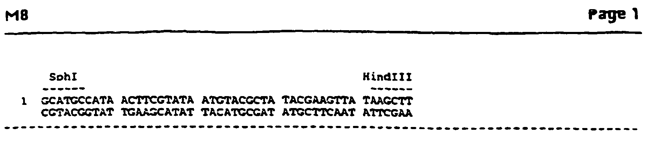

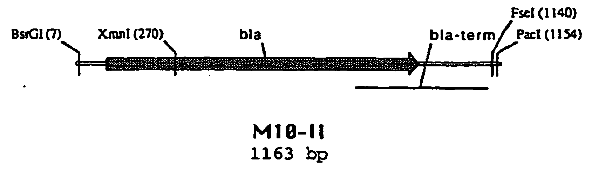

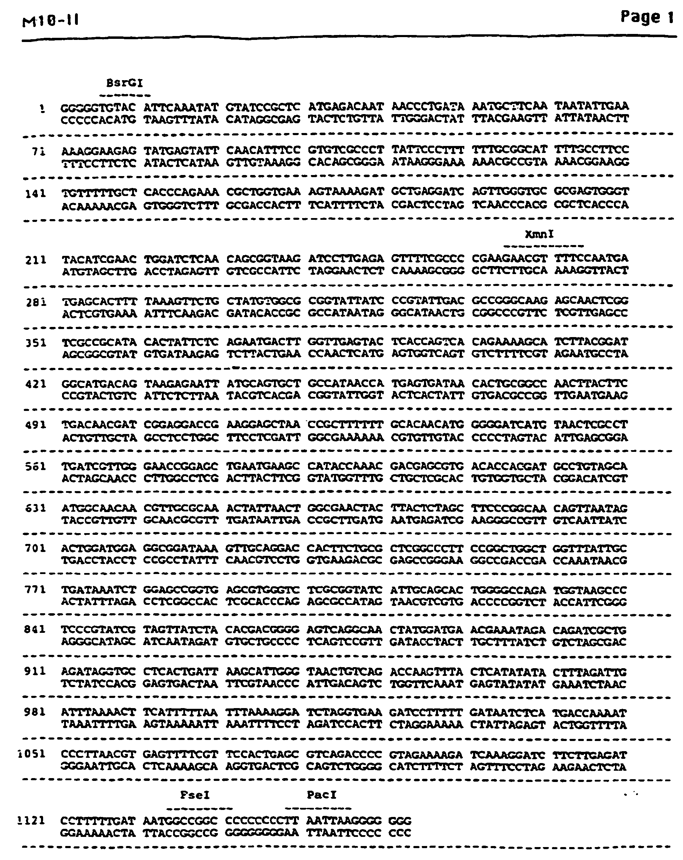

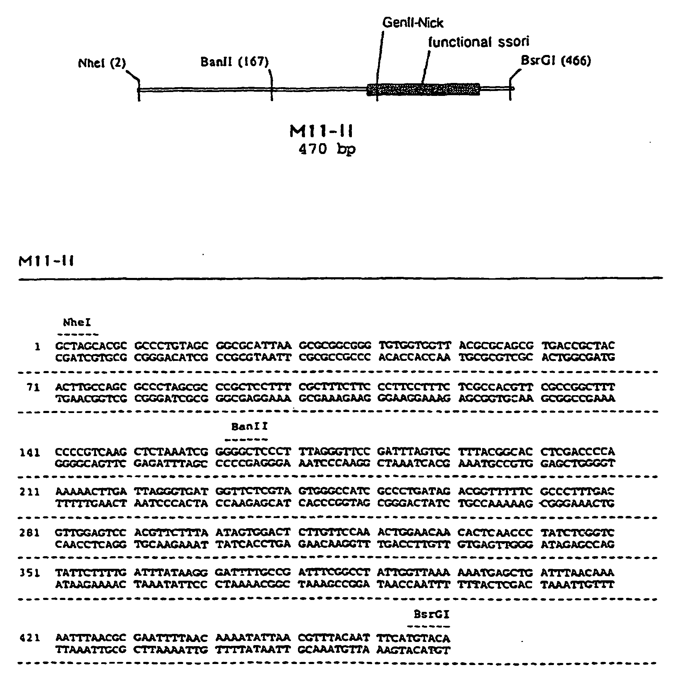

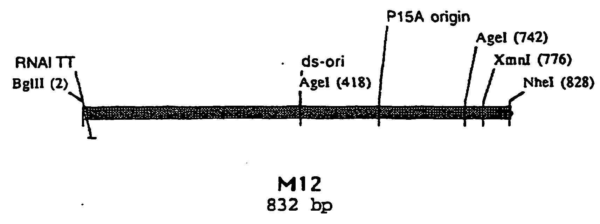

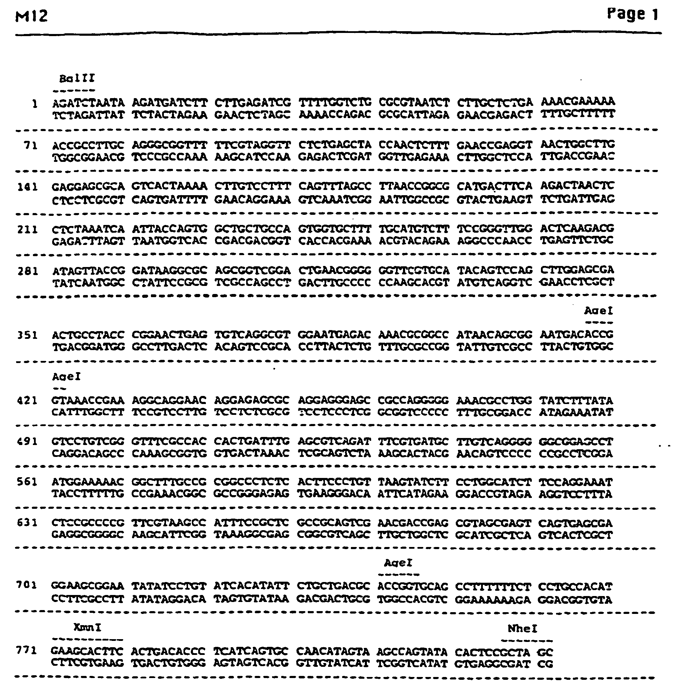

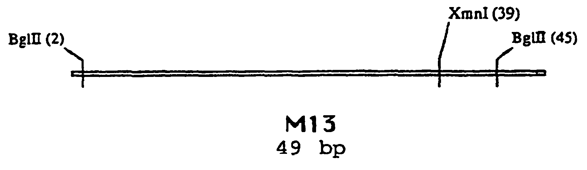

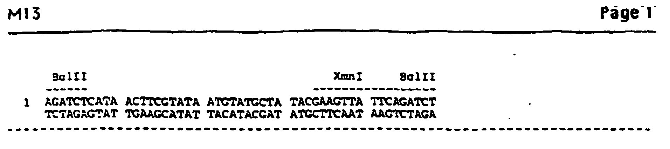

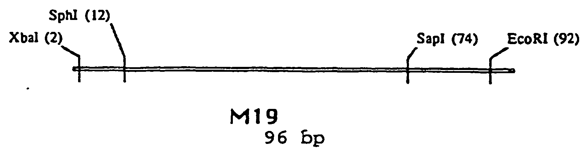

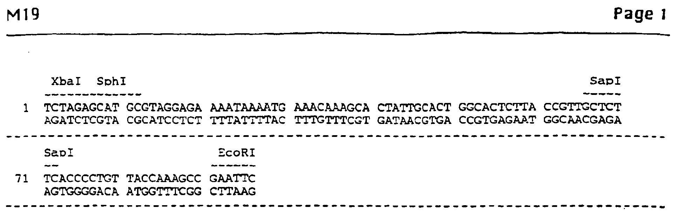

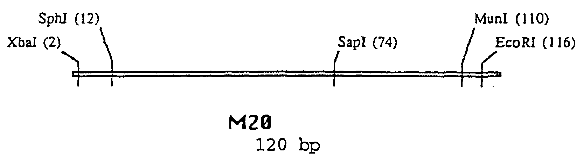

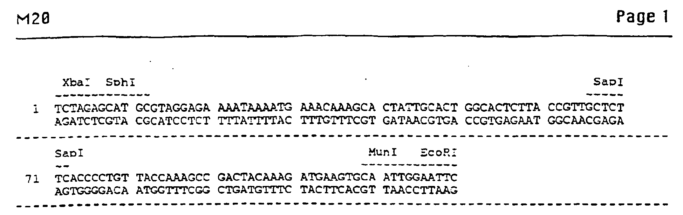

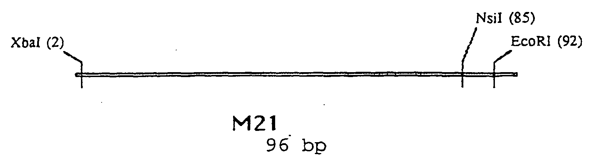

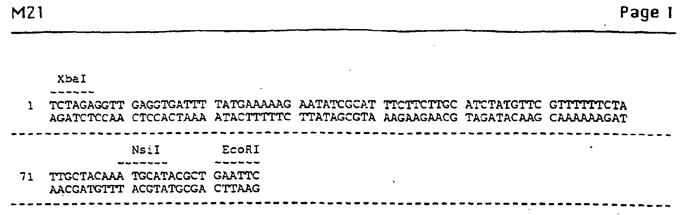

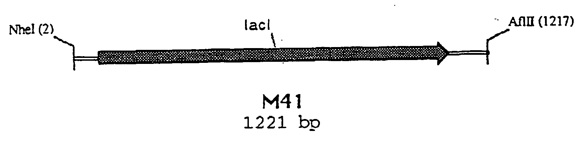

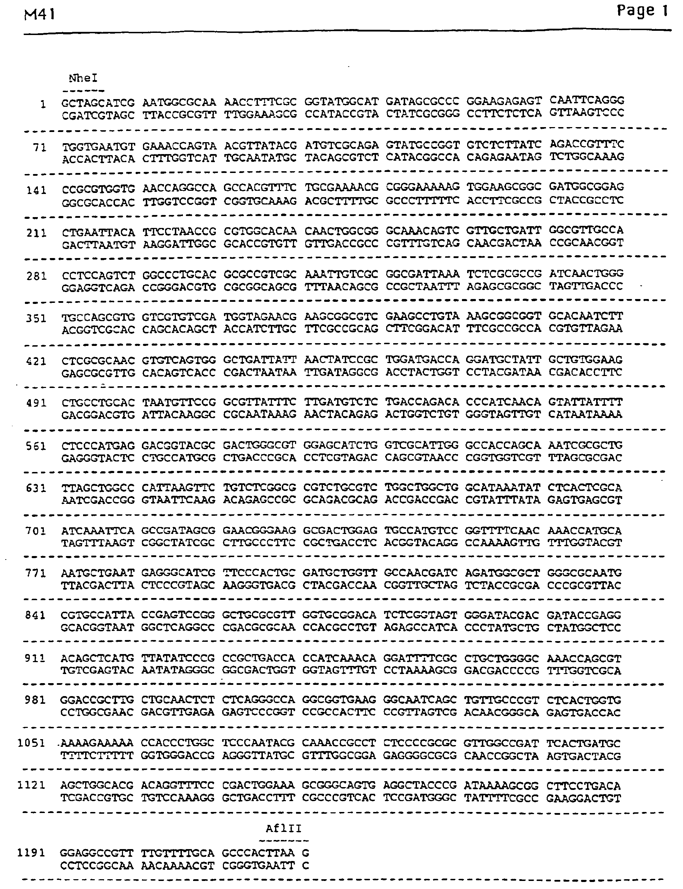

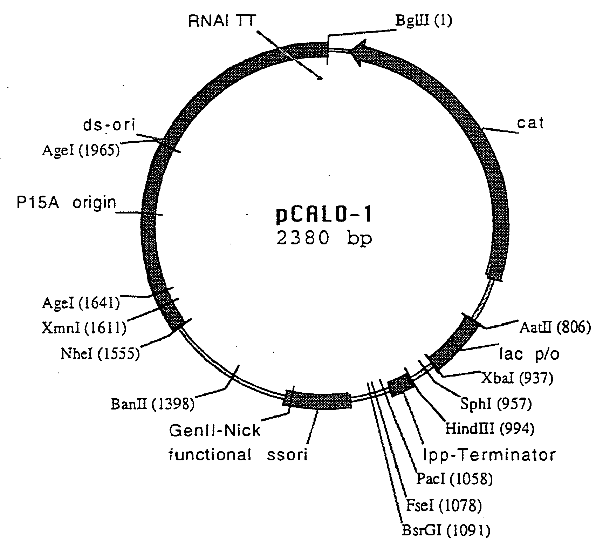

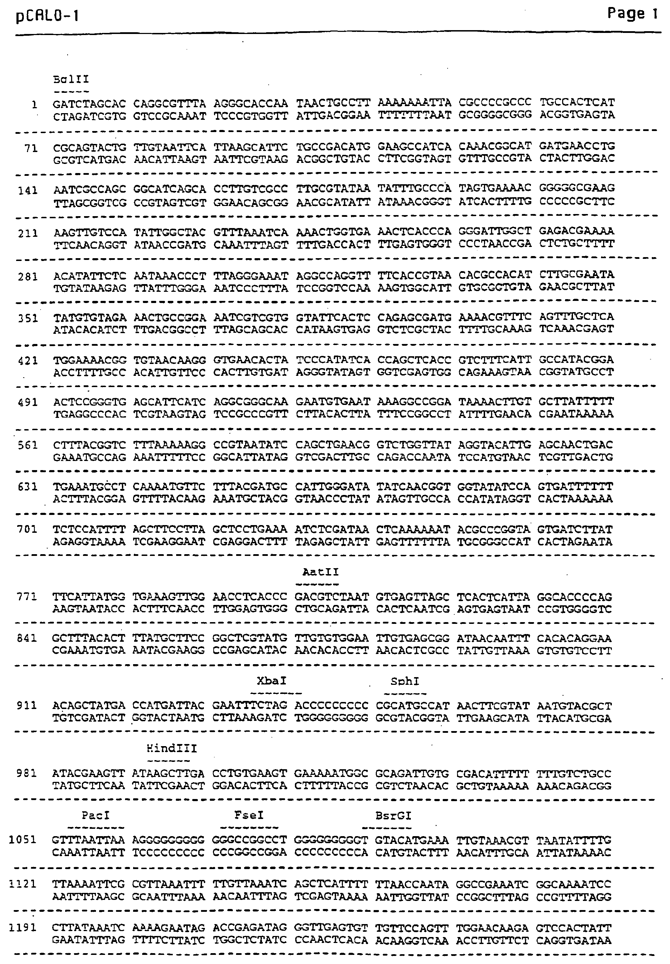

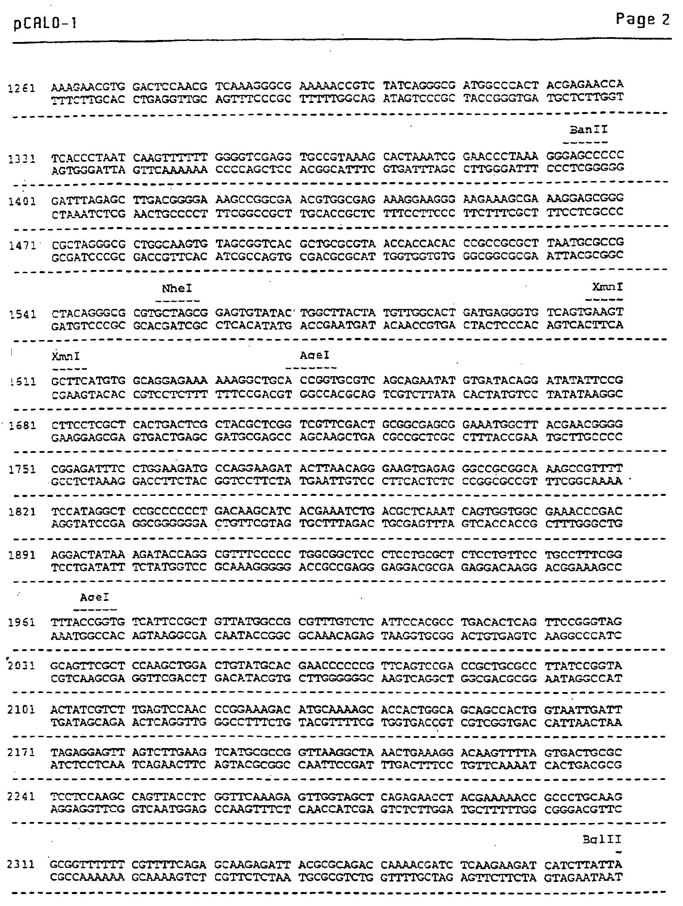

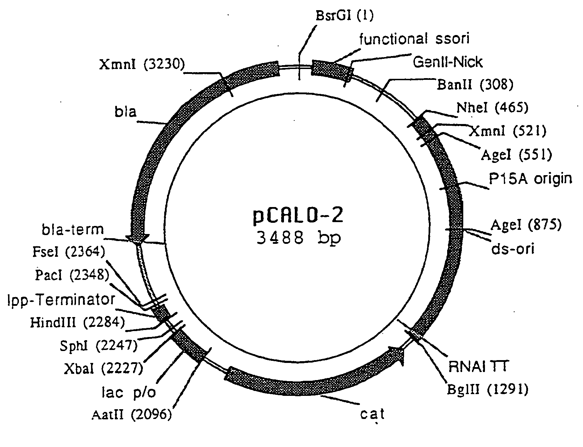

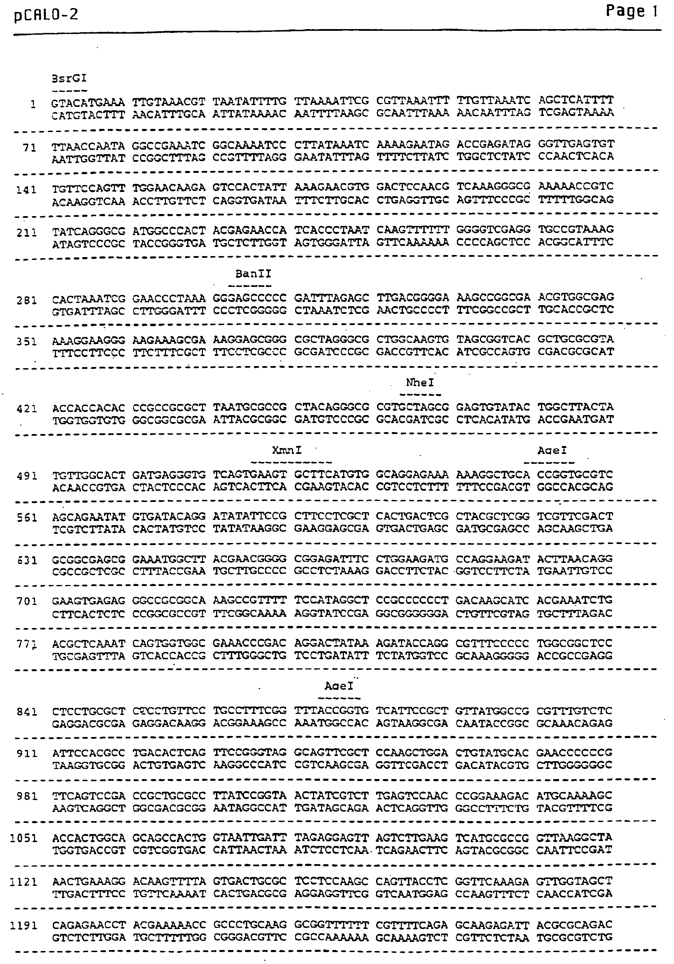

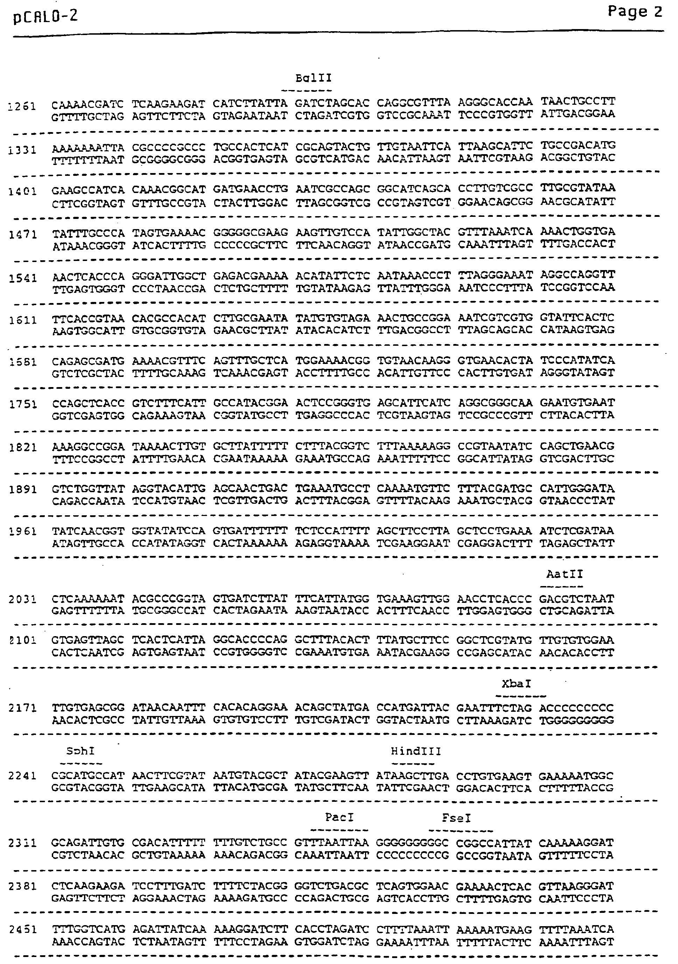

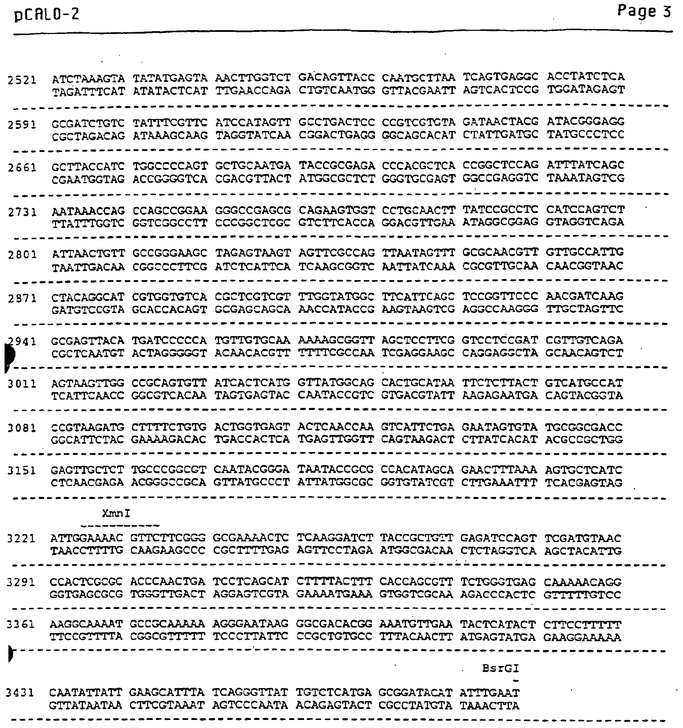

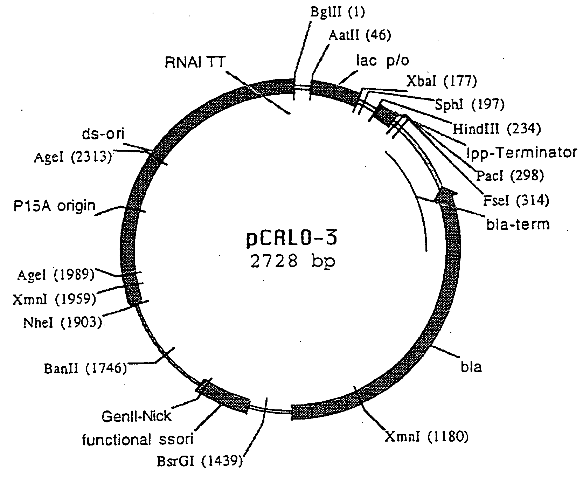

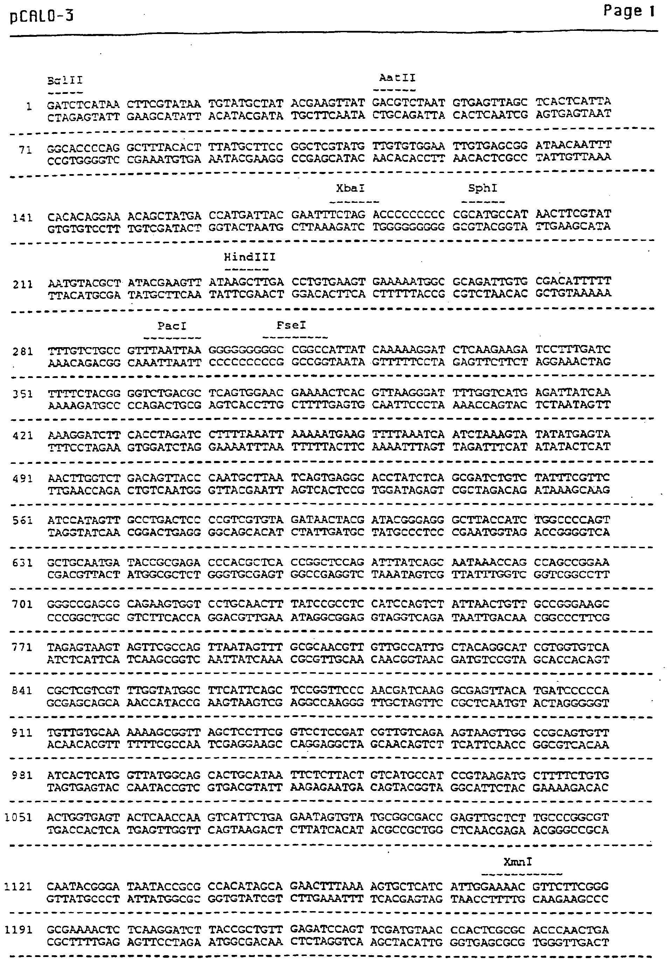

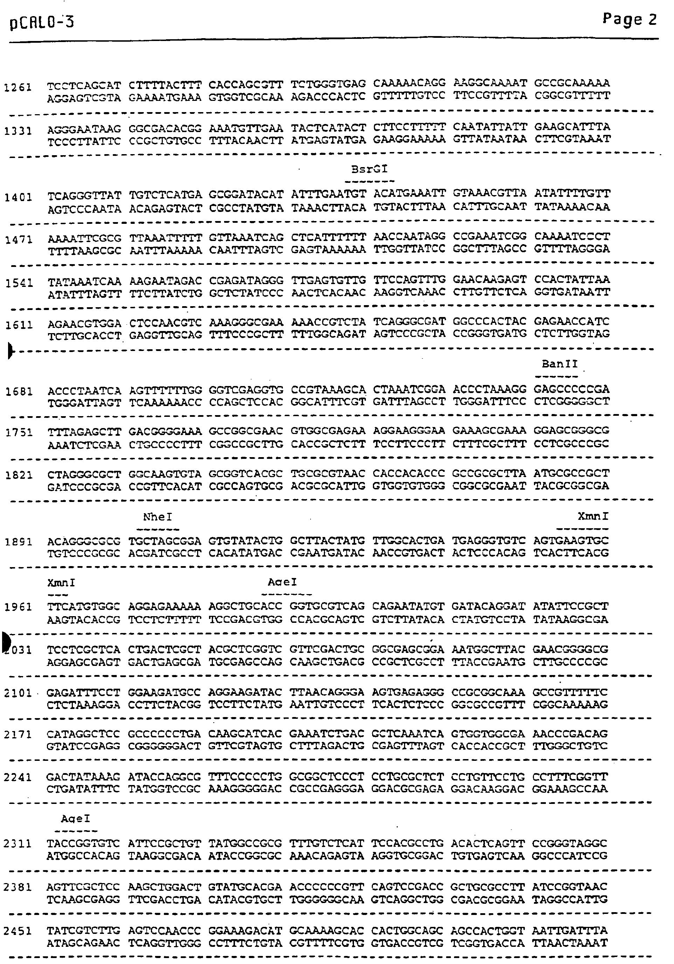

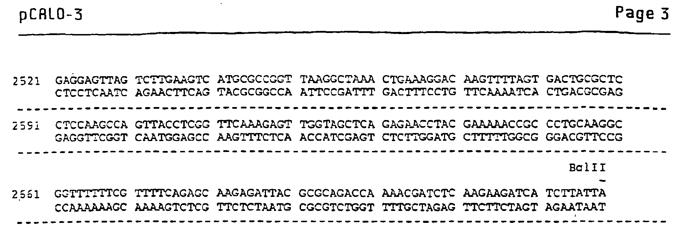

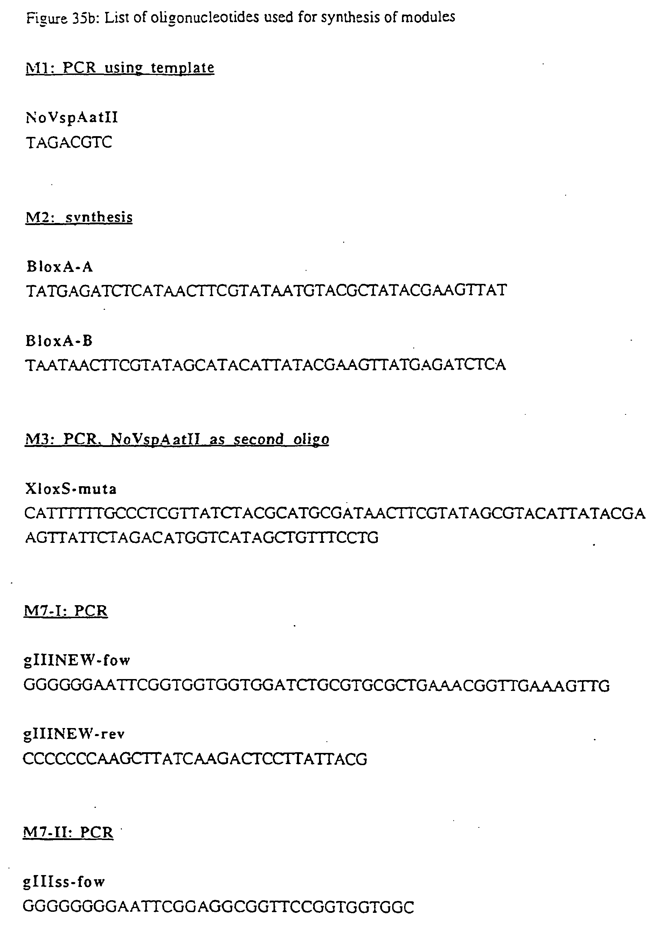

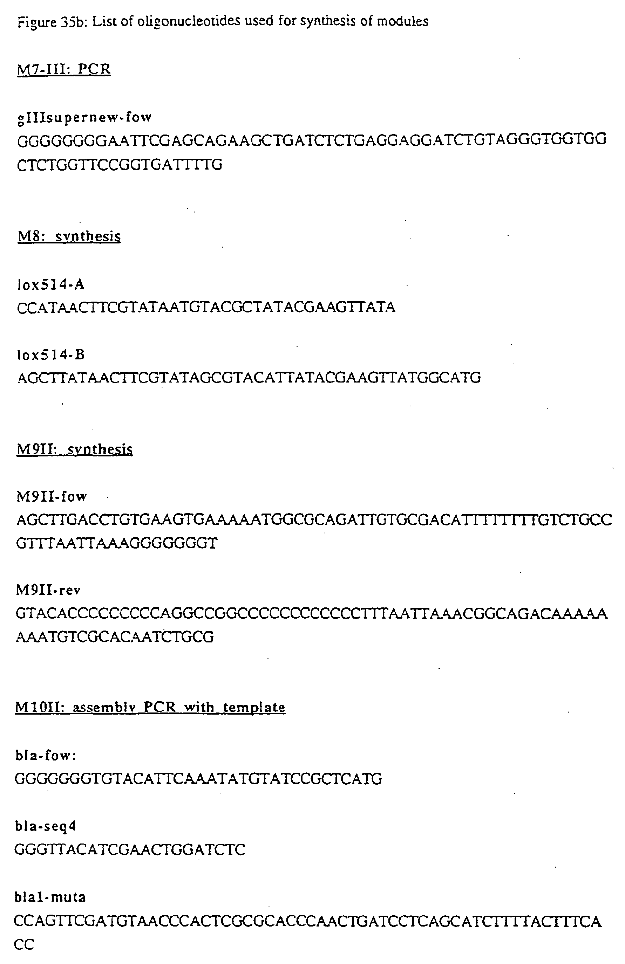

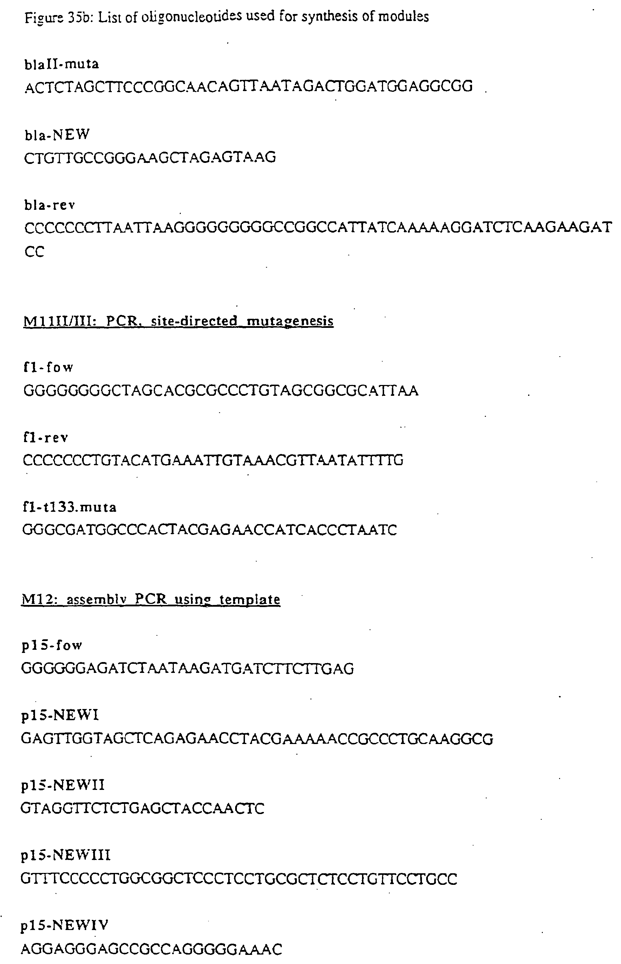

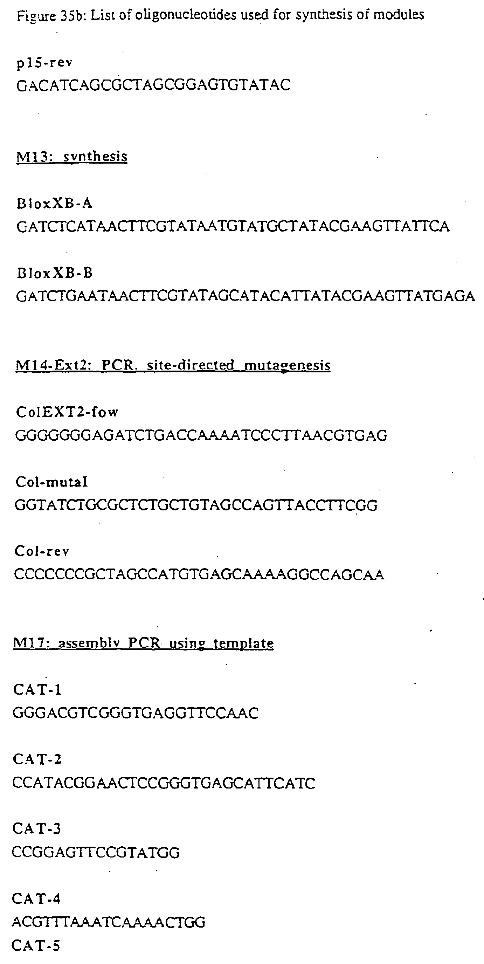

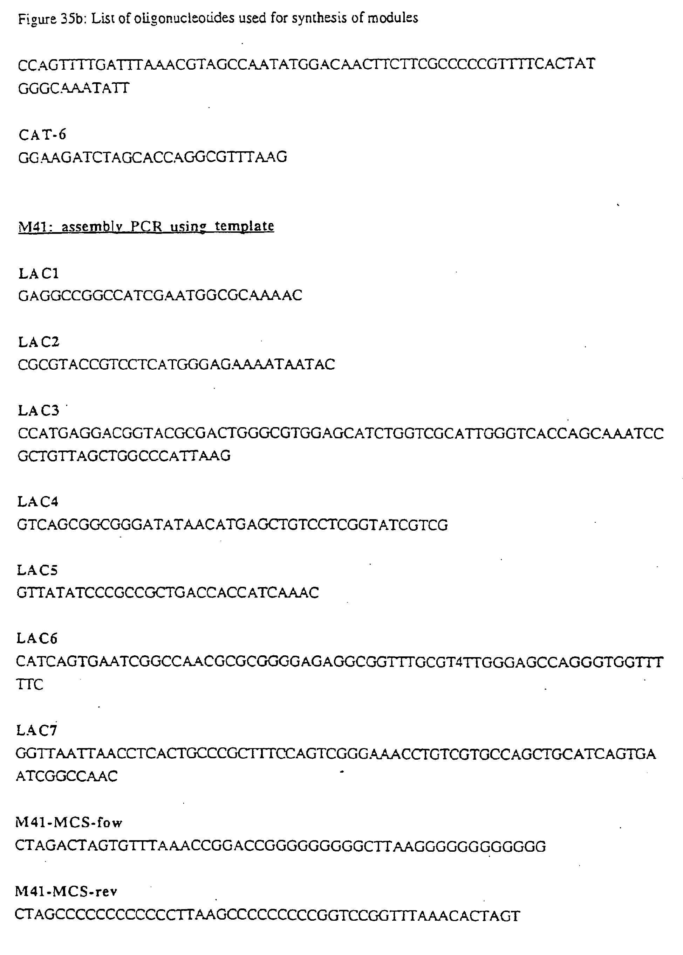

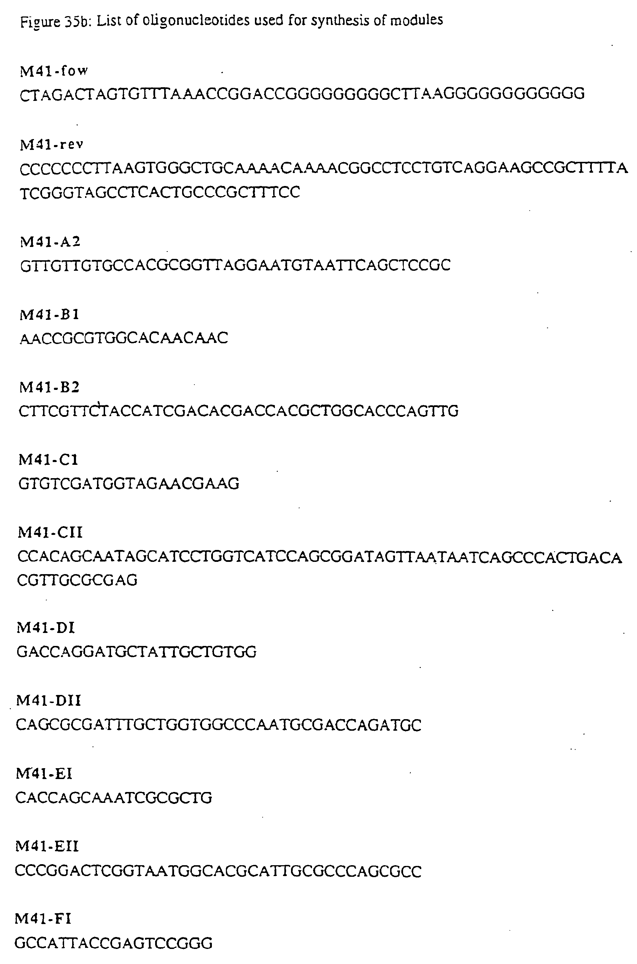

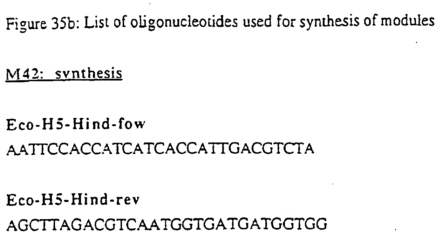

A series of vector modules was constructed where the restriction sites flanking the gene sub-elements of the HuCAL genes were removed, the vector modules themselves being flanked by unique restriction sites. These modules were constructed either by gene synthesis or by mutagenesis of templates. Mutagenesis was done by add-on PCR, by site-directed mutagenesis (Kunkel et al., 1991) or multisite oligonucleotide-mediated mutagenesis (Sutherland et al., 1995; Perlak, 1990) using a PCR-based assembly method.

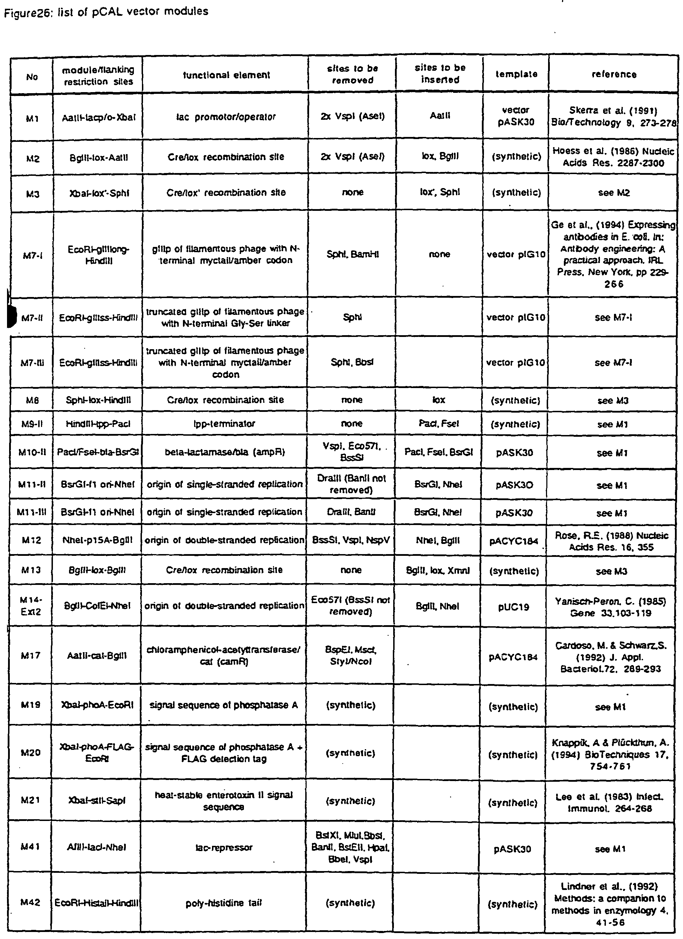

Figure 26 contains a list of the modules constructed. Instead of the terminator module M9 (HindIII-Ipp-Pacl), a larger cassette M9II was prepared to introduce Fsel as additional restriction site. M9II can be cloned via HindIII/BsrGI.
All vector modules were characterized by restriction analysis and sequencing. In the case of module M11-II, sequencing of the module revealed a two-base difference in positions 164/65 compared to the sequence database of the template. These two different bases (CA → GC) created an additional BanII site. Since the same two-base difference occurs in the f1 origin of other bacteriophages, it can be assumed that the two-base difference was present in the template and not created by mutagenesis during cloning. This BanII site was removed by site-directed mutagenesis, leading to module M11-III. The BssSI site of module M14 could initially not be removed without impact on the function of the ColE1 origin, therefore M14-Ext2 was used for cloning of the first pCAL vector series. Figures 29 to 34 are showing the functional maps and sequences of the modules used for assembly of the modular vector pCAL4 (see below). The functional maps and sequences of additional modules can be found in Figure 35a. Figure 35b contains a list of oligonucleotides and primers used for the synthesis of the modules.

### 4.2 Cloning vector pMCS

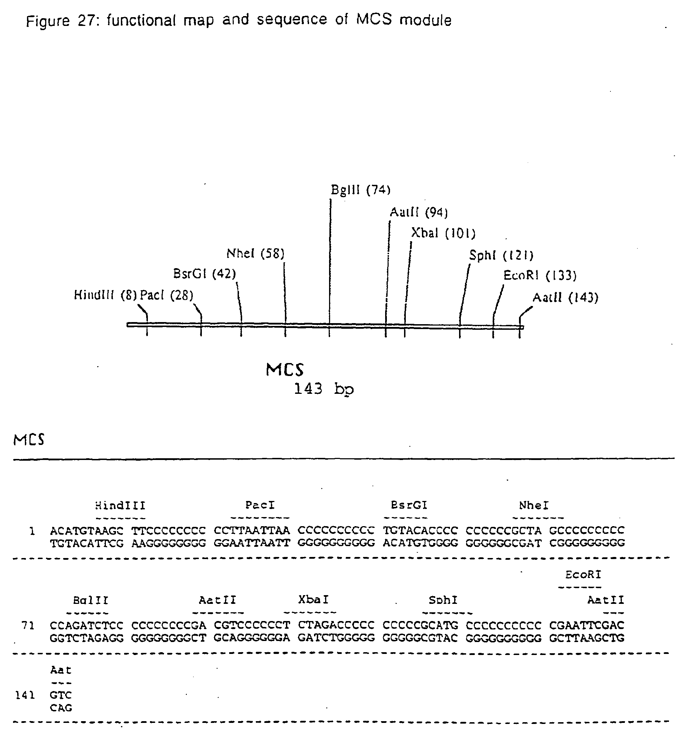

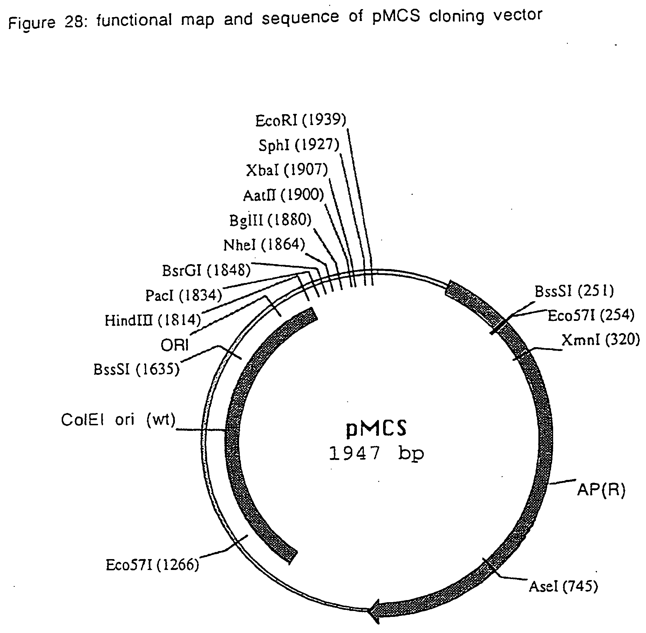

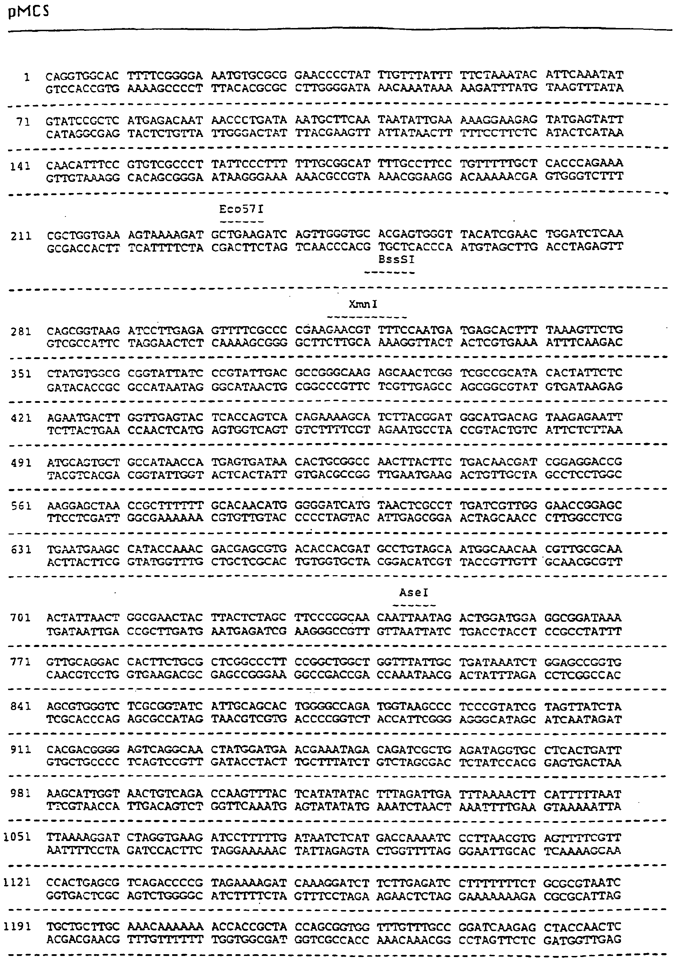

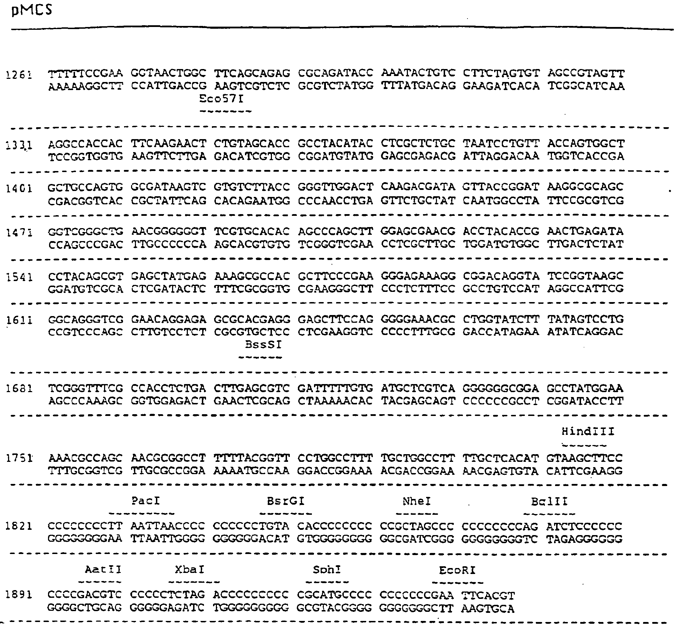

To be able to assemble the individual vector modules, a cloning vector pMCS containing a specific multi-cloning site (MCS) was constructed. First, an MCS cassette (Fig. 27) was made by gene synthesis. This cassette contains all those restriction sites in the order necessary for the sequential introduction of all vector modules and can be cloned via the 5'-Hindlll site and a four base overhang at the 3'-end compatible with an Aatll site. The vector pMCS (Figure 28) was constructed by digesting pUC19 with AatII and Hindlll, isolating the 2174 base pair fragment containing the bla gene and the ColE1 origin, and ligating the MCS cassette.

### 4.3 Cloning of modular vector pCAL4

This was cloned step by step by restriction digest of pMCS and subsequent ligation of the modules M1 (via Aatll/Xbal), M7III (via EcoRI/HindIII), and M9II (via HindIII/BsrGI), and M11-II (via BsrGI/NheI). Finally, the bla gene was replaced by the cat gene module M17 (via AatII/BgIII), and the wild type ColE1 origin by module M14-Ext2 (via BgIII/NheI). Figure 35 is showing the functional map and the sequence of pCAL4.

### 4.4 Cloning of low-copy number plasmid vectors pCALO

A series of low-copy number plasmid vectors was constructed in a similar way using the p15A module M12 instead of the ColE1 module M14-Ext2. Figure 35a is showing the functional maps and sequences of the vectors pCALO1 to pCALO3.

### Example 5: Construction of a HuCAL scFv Library

### 5.1. Cloning of all 49 HuCAL scFv fragments

All 49 combinations of the 7 HuCAL-VH and 7 HuCAL-VL consensus genes were assembled as described for the *HuCAL* VH3-Vk2 scFv in Example 2 and inserted into the vector pBS12, a modified version of the pLisc series of antibody expression vectors (Skerra et al., 1991).

### 5.2 Construction of a CDR cloning cassette

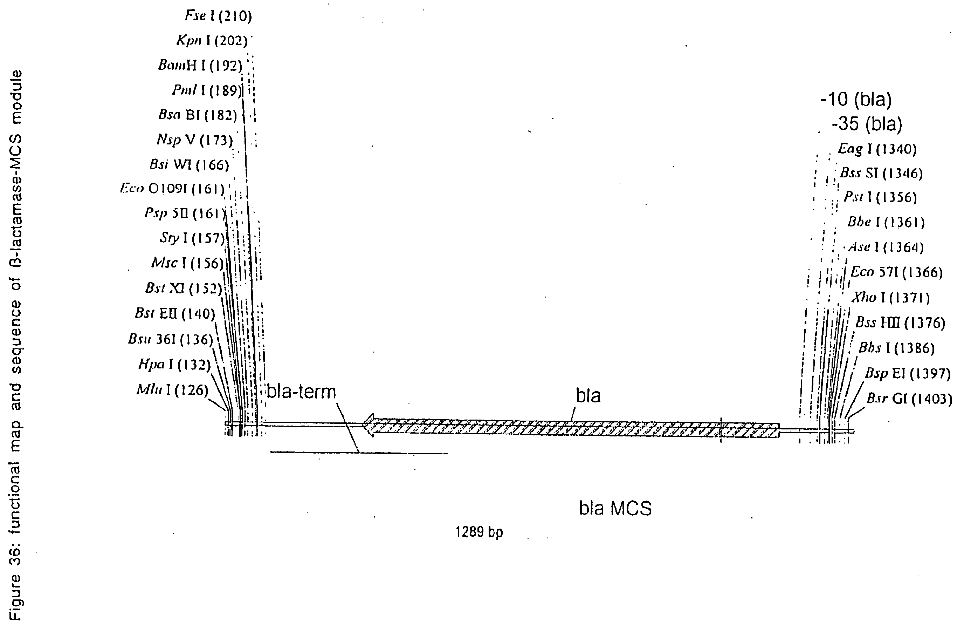

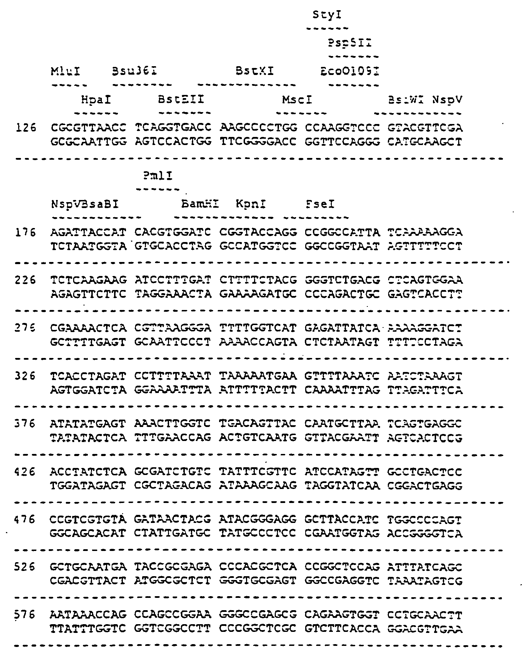

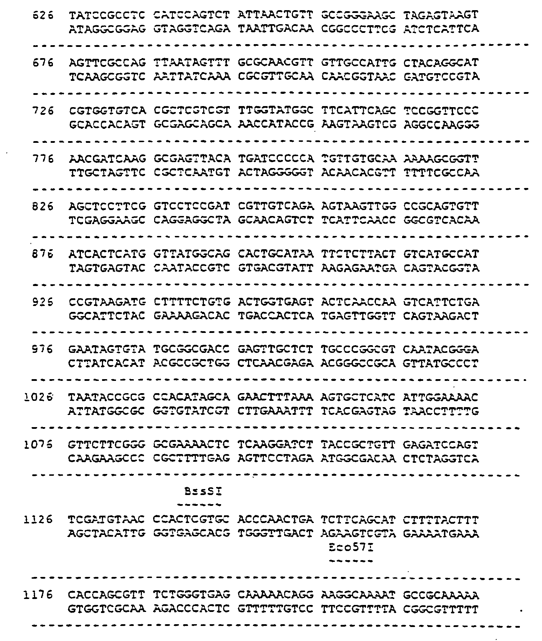

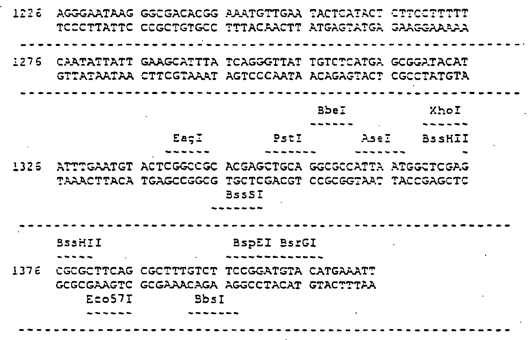

For replacement of CDRs, a universal β-lactamase cloning cassette was constructed having a multi-cloning site at the 5'-end as well as at the 3'-end. The 5'-multi-cloning site comprises all restriction sites adjacent to the 5'-end of the *HuCAL* VH and VL CDRs, the 3'-multi-cloning site comprises all restriction sites adjacent to the 3' end of the HuCAL VH and VL CDRs. Both 5'- and 3'-multi-cloning site were prepared as cassettes via add-on PCR using synthetic oligonucleotides as 5'- and 3'-primers using wild type β-lactamase gene as template. Figure 36 shows the functional map and the sequence of the cassette bla-MCS.

### 5.3. Preparation of VL-CDR3 library cassettes

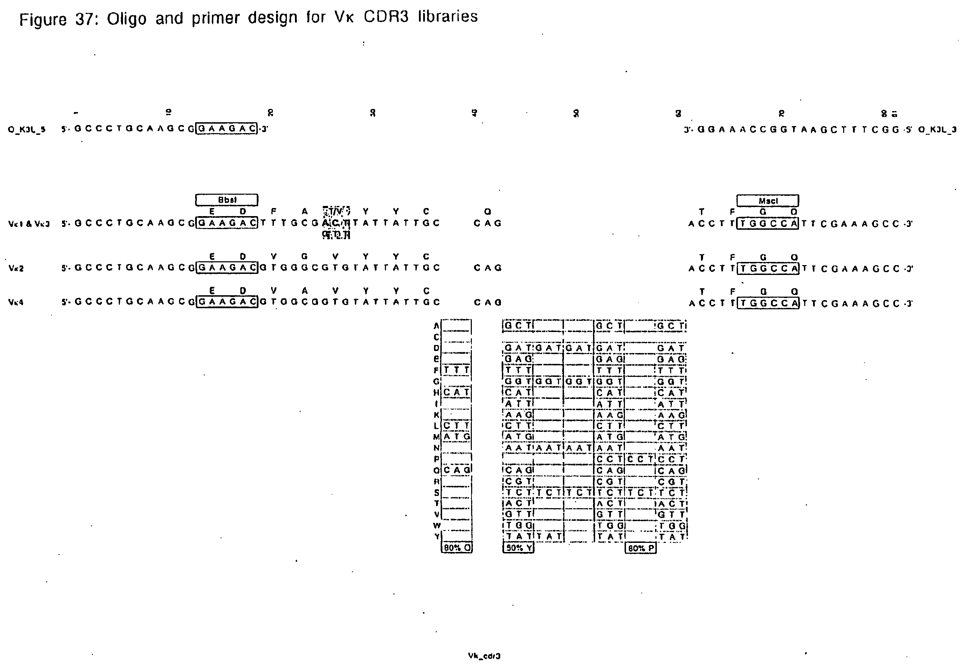

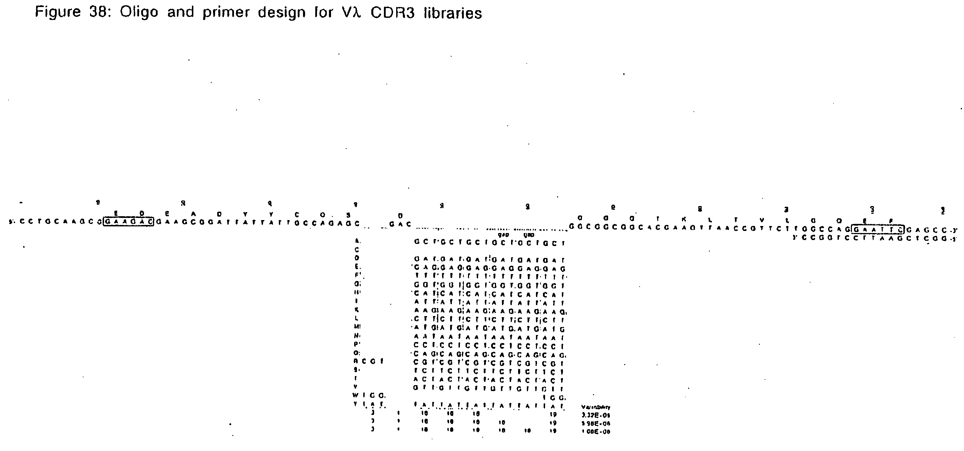

The VL-CDR3 libraries comprising 7 random positions were generated from the PCR fragments using oligonucleotide templates Vk1&Vk3, Vk2 and Vk4 and primers O_K3L_5 and O_K3L_3 (Fig. 37) for the Vk genes, and Vλ and primers O_L3L_5 (5'-GCAGAAGGCGAACGTCC-3') and O_L3LA_3 (Fig. 38) for the Vλ genes. Construction of the cassettes was performed as described in Example 2.3.

### 5.4 Cloning of HuCAL scFv genes with VL-CDR3 libraries

Each of the 49 single-chains was subcloned into pCAL4 via XbaI/EcoRI and the VL-CDR3 replaced by the ß-lactamase cloning cassette via Bbsl/Mscl, which was then replaced by the corresponding VL-CDR3 library cassette synthesized as described above. This CDR replacement is described in detail in Example 2.3 where the cat gene was used.

### 5.5 Preparation of VH-CDR3 library cassette

The VH-CDR3 libraries were designed and synthesized as described in Example 2.3.

### 5.6 Cloning of HuCAL scFv genes with VL- and VH-CDR3 libraries

Each of the 49 single-chain VL-CDR3 libraries was digested with BssHII/Styl to replace VH-CDR3. The "dummy" cassette digested with BssHII/Styl was inserted, and was then replaced by a corresponding VH-CDR3 library cassette synthesized as described above.

### Example 6: Expression tests

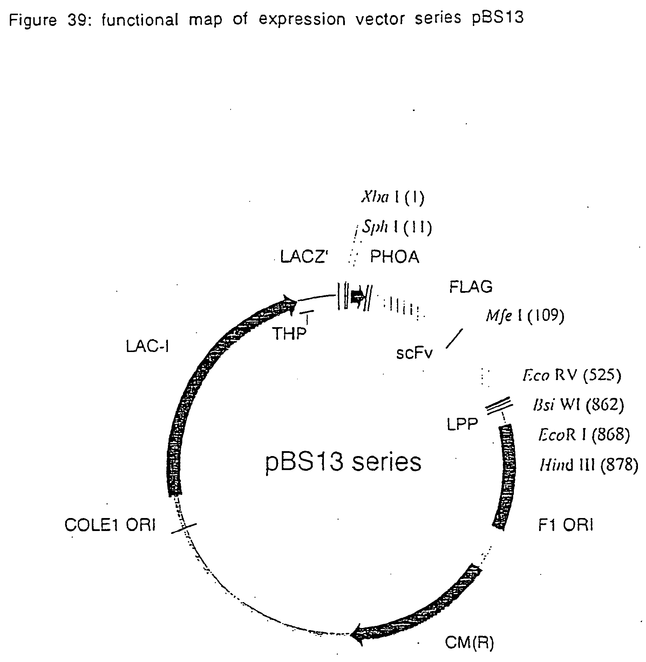

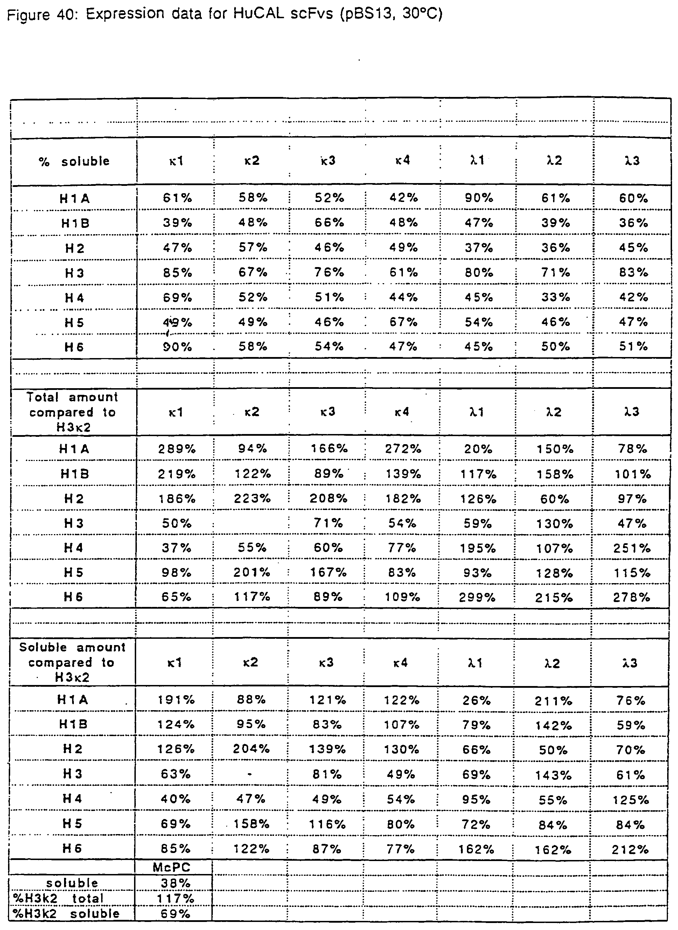

Expression and toxicity studies were performed using the scFv format VH-linker-VL. All 49 combinations of the 7 HuCAL-VH and 7 HuCAL-VL consensus genes assembled as described in Example 5 were inserted into the vector pBS13, a modified version of the pLisc series of antibody expression vectors (Skerra et al., 1991). A map of this vector is shown in Fig. 39.
*E. coli* JM83 was transformed 49 times with each of the vectors and stored as glycerol stock. Between 4 and 6 clones were tested simultaneously, always including the clone H3k2, which was used as internal control throughout. As additional control, the McPC603 scFv fragment (Knappik & Plückthun, 1995) in pBS13 was expressed under identical conditions. Two days before the expression test was performed, the clones were cultivated on LB plates containing 30 µg/ml chloramphenicol and 60 mM glucose. Using this plates an 3 ml culture (LB medium containing 90 µg chloramphenicol and 60 mM glucose) was inoculated overnight at 37 °C. Next day the overnight culture was used to i noculate 30 ml LB medium containing chloramphenicol (30 µg/ml). The starting OD₆₀₀ₙₘ was adjusted to 0.2 and a growth temperature of 30 °C was used. The physiology of the cells was monitored by measuring every 30 minutes for 8 to 9 hours the optical density at 600 nm. After the culture reached an OD₆₀₀ₙₘ of 0.5, antibody expression was induced by adding IPTG to a final concentration of 1 mM. A 5 ml aliquot of the culture was removed after 2 h of induction in order to analyze the antibody expression. The cells were lysed and the soluble and insoluble fractions of the crude extract were separated as described in Knappik & Plückthun, 1995. The fractions were assayed by reducing SDS-PAGE with the samples normalized to identical optical densities. After blotting and immunostaining using the α-FLAG antibody M1 as the first antibody (see Ge et al., 1994) and an Fc-specific anti-mouse antiserum conjugated to alkaline phosphatase as the second antibody, the lanes were scanned and the intensities of the bands of the expected size (appr. 30 kDa) were quantified densitometrically and tabulated relative to the control antibody (see Fig. 40).

### Example 7: Optimization of Fluorescein Binders

### 7.1. Construction of L-CDR3 and H-CDR2 library cassettes

A L-CDR3 library cassette was prepared from the oligonucleotide template CDR3L (5'-TGGAAGCTGAAGACGTGGGCGTGTATTATTGCCAGCAG(TR5)(TRI)₄CCG(TRI)TT TGGCCAGGGTACGAAAGTT-3') and primer 5'-AACTTTCGTACCCTGGCC-3' for synthesis of the complementary strand, where (TRI) was a trinucleotide mixture representing all amino acids except Cys, (TR5) comprised a trinucleotide mixture representing the 5 codons for Ala, Arg, His, Ser, and Tyr.
A H-CDR2 library cassette was prepared from the oligonucleotide template CDRsH (5'-AGGGTCTCGAGTGGGTGAGC(TRI)ATT(TRI)₂₋₃(6)₂(TRI)ACC(TRI)TATGC GGATAGCGTGAAAGGCCGTTTTACCATTTCACGTGATAATTCGAAAAACACCA-3'), and primer 5'-TGGTGTTTTTCGAATTATCA-3' for synthesis of the complementary strand, where (TRI) was a trinucleotide mixture representing all amino acids except Cys, (6) comprised the incorporation of (A/G) (A/C/G) T, resulting in the formation of 6 codons for Ala, Asn, Asp, Gly, Ser, and Thr, and the length distribution being obtained by performing one substoichiometric coupling of the (TRI) mixture during synthesis, omitting the capping step normally used in DNA synthesis. DNA synthesis was performed on a 40 nmole scale, oligos were dissolved in TE buffer, purified via gel filtration using spin columns (S-200), and the DNA concentration determined by OD measurement at 260 nm (OD 1.0 = 40 µg/ml). 10 nmole of the oligonucleotide templates and 12 nmole of the corresponding primers were mixed and annealed at 80°C for 1 min, and slowly cooled down to 37°C within 20 to 30 min. The fill-in reaction was performed for 2 h at 37°C using Klenow polymerase (2.0 µl) and 250 nmole of each dNTP. The excess of dNTPs was removed by gel filtration using Nick-Spin columns (Pharmacia), and the double-stranded DNA digested with Bbsl/Mscl (L-CDR3), or Xhol/Sful (H-CDR2) over night at 37°C. The cassettes were purified via Nick-Spin columns (Pharmacia), the concentration determined by OD measurement, and the cassettes aliquoted (15 pmole) for being stored at -80°C.

### 7.2 Library cloning:

DNA was prepared from the collection of FITC binding clones obtained in Example 2 (approx. 10⁴ to clones). The collection of scFv fragments was isolated via Xbal/EcoRI digest. The vector pCAL4 (100 fmole, 10 µg) described in Example 4.3 was similarly digested with Xbal/EcoRI, gel-purified and ligated with 300 fmole of the scFv fragment collection over night at 16°C. The ligation mixture was isopropanol precipitated, air-dried, and the pellets were redissolved in 100 III of dd H₂O. The ligation mixture was mixed with 1 ml of freshly prepared electrocompetent SCS 101 cells (for optimization of L-CDR3), or XL1 Blue cells (for optimization of H-CDR2) on ice. One round of electroporation was performed and the transformants were eluted in SOC medium, shaken at 37°C for 30 minutes, and a n aliquot plated out on LB plates (Amp/Tet/Glucose) at 37°C for 6-9 hrs. The n umber of transformants was 5 x 10⁴.
Vector DNA (100 µg) was isolated and digested (sequence and restriction map of scH3k2 see Figure 8) with Bbsl/Mscl for optimization of L-CDR3, or XhoI/NspV for optimization of H-CDR2. 10 µg of purified vector fragments (5 pmole) were ligated with 15 pmole of the L-CDR3 or H-CDR2 library cassettes over night at 16°C. The ligation mixtures were isopropanol precipitated, air-dried, and the pellets were redissolved in 100 µl of dd H₂O. The ligation mixtures were mixed with 1 ml of freshly prepared electrocompetent XL1 Blue cells on ice. Electroporation was performed and the transformants were eluted in SOC medium and shaken at 37°C for 30 minutes. An aliquot was plated out on LB plates (Amp/Tet/Glucose) at 37°C for 6-9 hrs. The number of transformants (library size) was greater than 10⁸ for both libraries. The libraries were stored as glycerol cultures.

### 7.3. Biopanning

This was performed as described for the initial H3k2 H-CDR3 library in Example 2.1. Optimized scFvs binding to FITC could be characterized and analyzed as described in Example 2.2 and 2.3, and further rounds of optimization could be made if necessary.

### References

Barbas III, C.F., Bain, J.D., Hoekstra, D.M. & Lerner, R.A., PNAS 89, 4457-4461 (1992).
Better, M., Chang, P., Robinson, R. & Horwitz, A.H., Science 240, 1041-1043 (1988).
Blake, M.S., Johnston, K.H., Russel-Jones, G.J. & Gotschlich, E.C., Anal. Biochem. 136, 175-179 (1984).
Carter, P., Kelly, R.F., Rodrigues, M.L., Snedecor, B., Covrrubias, M., Velligan, M.D., Wong, W.L.T., Rowland, A.M., Kotts, C.E., Carver, M.E., Yang, M., Bourell, J.H., Shepard, H.M. & Henner, D., Bio/Technology 10, 163-167 (1992).
Chothia, C. & Lesk, A.M., J. Biol. Chem. 196, 910-917 (1987).
Chothia, C., Lesk, A.M., Gherardi, E., Tomlinson, I.A., Walter, G., Marks, J.D., Llewelyn, M.B. & Winter, G., J. Mol. Biol. 227, 799-817 (1992).
Chothia, C., Lesk, A.M., Tramontano, A., Levitt, M., Smith-Gill, S.J., Air, G., Sheriff, S., Padlan, E.A., Davies, D., Tulip, W.R., Colman, P.M., Spinelli, S., Alzari, P.M. & Poljak, R.J., Nature 342, 877-883 (1989).
Chuchana, P., Blancher, A., Brockly, F., Alexandre, D., Lefranc, G & Lefranc, M.-P., Eur. J. Immunol. 20, 1317-1325 (1990).
Cox, J.P.L., Tomlinson, I.M. & Winter, G., Eur. J. Immunol. 24, 827-836 (1994).
Ge, L., Knappik, A., Pack, P., Freund, C. & Plückthun, A., In: Antibody Engineering. Borrebaeck, C.A.K. (Ed.). p.229-266 (1995), Oxford University Press, New York, Oxford.)
Gill, S.C. & von Hippel, P.H., Anal. Biochem. 182, 319.326 (1989).
Hochuli, E., Bannwarth, W., Dobeli, H., Gentz, R. & Stüber, D., Bio/Technology 6, 1321-1325 (1988).
Hopp, T.P., Prickett, K.S., Price, V.L., Libby, R.T., March, C.J., Cerretti, D.P., Urdal, D.L. & Conlon, P.J. Bio/Technology 6, 1204-1210 (1988).
Kabat, E.A., Wu, T.T., Perry, H.M., Gottesmann, K.S. & Foeller, C., Sequences of proteins of immunological interest, NIH publication 91-3242 (1991).
Knappik, A. & Plückthun, A., Biotechniques 17, 754-761 (1994).
Knappik, A. & Plückthun, A., Protein Engineering 8, 81-89 (1995).
Kunkel, T.A., Bebenek, K. & McClary, J., Methods in Enzymol. 204, 125-39 (1991).
Lindner, P., Guth, B., Wolfing, C., Krebber, C., Steipe, B., Müller, F. & Plückthun, A., Methods: A Companion to Methods Enzymol. 4, 41-56 (1992).
Lowman, H.B., Bass, S.H., Simpson, N. and Wells, J.A., Biochemistry 30, 10832-10838 (1991).
Pack, P. & Plückthun, A., Biochemistry 31, 1579-1584 (1992).
Pack, P., Kujau, M., Schroeckh, V., Knüpfer, U., Wenderoth, R., Riesenberg D. & Plückthun, A., Bio/Technology 11, 1271-1277 (1993).
Pack, P., Ph.D. thesis, Ludwig-Maximilians-Universität München (1994).
Perlak, F. J., Nuc. Acids Res. 18, 7457-7458 (1990).
Plückthun, A., Krebber, A., Krebber, C., Horn, U., Knüpfer, U., Wenderoth, R., Nieba, L., Proba, K. & Riesenberg, D., A practical approach. Antibody Engineering (Ed. J. McCafferty). IRL Press, Oxford, pp. 203-252 (1996).
Prodromou, C. & Pearl, L.H., Protein Engineering 5, 827-829 (1992).
Rosenberg, S.A. & Lotze, M.T., Ann. Rev. Immunol. 4, 681-709 (1986).
Skerra, A. & Plückthun, A., Science 240, 1038-1041 (1988).
Skerra, A., Pfitzinger, I. & Plückthun, A., Bio/Technology 9, 273-278 (1991).
Sutherland, L., Davidson, J., Glass, L.L., & Jacobs, H.T., BioTechniques 18, 458-464, 1995.
Tomlinson, I.M., Walter, G., Marks, J.D., Llewelyn, M.B. & Winter, G., J. Mol. Biol. 227, 776-798 (1992).
Ullrich, H.D., Patten, P.A., Yang, P.L., Romesberg, F.E. & Schultz, P.G., Proc. Natl. Acad. Sci. USA 92, 11907-11911 (1995).
Van Dijk, K.W., Mortari, F., Kirkham, P.M., Schroeder Jr., H.W. & Milner, E.C.B., Eur. J. Immunol. 23, 832-839 (1993).
Virnekäs, B., Ge, L., Plückthun, A., Schneider, K.C., Wellnhofer, G. & Moroney, S.E., Nucleic Acids Research 22, 5600-5607 (1994).
Vitetta, E.S., Thorpe, P.E. & Uhr, J., Immunol. Today 14, 253-259 (1993).
Williams, S.C. & Winter, G., Eur. J. Immunol. 23, 1456-1461 (1993).
Winter, G., Griffiths, A.D., Hawkins, R.E. & Hoogenboom, H.R., Ann. Rev. Immunol. 12, 433-455 (1994).

**Table 1A: Human kappa germline gene segments**

| **Used Name¹** | **Reference²** | **Family³** | **Germline genes⁴** |
|---|---|---|---|
| Vk1-1 | 9 | 1 | 08; 018; DPK1 |
| Vk1-2 | 1 | 1 | L14; DPK2 |
| Vk1-3 | 2 | 1 | L15(1); HK101; HK146; HK189 |
| Vk1-4 | 9 | 1 | L11 |
| Vk1-5 | 2 | 1 | A30 |
| Vk1-6 | 1 | 1 | LFVK5 |
| Vk1-7 | 1 | 1 | LFVK431 |
| Vk1-8 | 1 | 1 | L1; HK137 |
| Vk1-9 | 1 | 1 | A20; DPK4 |
| Vk1-10 | 1 | 1 | L18; Va" |
| Vk1-11 | 1 | 1 | L4; L18; Va'; V4a |
| Vk1-12 | 2 | 1 | L5; L19(1); Vb; Vb4; DPK5; L19(2); Vb"; DPK6 |
| Vk1-13 | 2 | 1 | L15(2); HK134; HK166; DPK7 |
| Vk1-14 | 8 | 1 | L8; Vd; DPK8 |
| Vk1-15 | 8 | 1 | L9; Ve |
| Vk1-16 | 1 | 1 | L12(1); HK102; V1 |
| Vk1-17 | 2 | 1 | L12(2) |
| Vk1-18 | 1 | 1 | O12a (V3b) |
| Vk1-19 | 6 | 1 | 02; 012; DPK9 |
| Vk1-20 | 2 | 1 | L24; Ve"; V13; DPK10 |
| Vk1-21 | 1 | 1 | 04; 014 |
| Vk1-22 | 2 | 1 | L22 |
| Vk1-23 | 2 | 1 | L23 |
| Vk2-1 | 1 | 2 | A2; DPK12 |
| Vk2-2 | 6 | 2 | O1; O11(1); DPK13 |
| Vk2-3 | 6 | 2 | 012(2); V3a |
| Vk2-4 | 2 | 2 | L13 |
| Vk2-5 | 1 | 2 | DPK14 |
| Vk2-6 | 4 | 2 | A3; A19; DPK15 |
| Vk2-7 | 4 | 2 | A29; DPK27 |
| Vk2-8 | 4 | 2 | A13 |
| Vk2-9 | 1 | 2 | A23 |
| Vk2-10 | 4 | 2 | A7; DPK17 |
| Vk2-11 | 4 | 2 | A17; DPK18 |
| Vk2-12 | 4 | 2 | A1; DPK19 |
| Vk3-1 | 11 | 3 | A11; humkv305; DPK20 |
| Vk3-2 | 1 | 3 | L20; Vg" |
| Vk3-3 | 2 | 3 | L2; L16; humkv328; humkv328h2; humkv328h5; DPK21 |
| Vk3-4 | 11 | 3 | A27; humkv325; VkRF; DPK22 |
| Vk3-5 | 2 | 3 | L25; DPK23 |
| Vk3-6 | 2 | 3 | L10(1) |
| Vk3-7 | 7 | 3 | L10(2) |
| Vk3-8 | 7 | 3 | L6; Vg |
| Vk4-1 | 3 | 4 | B3; VkIV; DPK24 |
| Vk5-1 | 10 | 5 | B2; EV15 |
| Vk6-1 | 12 | 6 | A14; DPK25 |
| Vk6-2 | 12 | 6 | A10; A26; DPK26 |
| Vk7-1 | 5 | 7 | B1 |

**Table 1B: Human lambda germline gene segments**

| **Used Name¹** | **Reference²** | **Family³** | **Germline genes⁴** |
|---|---|---|---|
| DPL1 | 1 | 1 | |
| DPL2 | 1 | 1 | HUMLV1L1 |
| DPL3 | 1 | 1 | HUMLV122 |
| DPL4 | 1 | 1 | VLAMBDA1.1 |
| HUMLV117 | 2 | 1 | |
| DPL5 | 1 | 1 | HUMLV117D |
| DPL6 | 1 | 1 | |
| DPL7 | 1 | 1 | IGLV1S2 |
| DPL8 | 1 | 1 | HUMLV1042 |
| DPL9 | 1 | 1 | HUMLV101 |
| DPL10 | 1 | 2 | |
| VLAMBDA 2.1 | 3 | 2 | |
| DPL11 | 1 | 2 | |
| DPL12 | 1 | 2 | |
| DPL13 | 1 | 2 | |
| DPL14 | 1 | 2 | |
| DPL16 | 1 | 3 | Humlv418; IGLV3S1 |
| DPL23 | 1 | 3 | V1 III.1 |
| Humlv318 | 4 | 3 | |
| DPL18 | 1 | 7 | 4A; HUMIGLVA |
| DPL19 | 1 | 7 | |
| DPL21 | 1 | 8 | VL8.1 |
| HUMLV801 | 5 | 8 | |
| DPL22 | 1 | 9 | |
| DPL24 | 1 | unassigned | VLAMBDA N.2 |
| gVLX-4.4 | 6 | 10 | |

**Table 1C: Human heavy chain germline gene segments**

| **Used Name¹** | **Reference²** | **Family³** | **Germline genes⁴** |
|---|---|---|---|
| VH1-12-1 | 19 | 1 | DP10; DA-2; DA-6 |
| VH1-12-8 | 22 | 1 | RR.VH1.2 |
| VH1-12-2 | 6 | 1 | hv1263 |
| VH1-12-9 | 7 | 1 | YAC-7; RR.VH1.1; 1-69 |
| VH1-12-3 | 19 | 1 | DP3 |
| VH1-12-4 | 19 | 1 | DP21; 4d275a; VH7a |
| VH1-12-5 | 18 | 1 | I-4.1b; V1-4.1b |
| VH1-12-6 | 21 | 1 | 1D37; VH7b; 7-81; YAC-10 |
| VH1-12-7 | 19 | 1 | DP14; VH1GRR, V1-18 |
| VH1-13-1 | 10 | 1 | 71-5; DP2 |
| VH1-13-2 | 10 | 1 | E3-10 |
| VH1-13-3 | 19 | 1 | DP1 |
| VH1-13-4 | 12 | 1 | V35 |
| VH1-13-5 | 8 | 1 | V1-2b |
| VH1-13-6 | 18 | 1 | I-2; DP75 |
| VH1-13-7 | 21 | 1 | V1-2 |
| VH1-13-8 | 19 | 1 | DP8 |
| VH1-13-9 | 3 | 1 | 1-1 |
| VH1-13-10 | 19 | 1 | DP12 |
| VH1-13-11 | 15 | 1 | V13C |
| VH1-13-12 | 18 | 1 | I-3b; DP25; V1-3b |
| VH1-13-13 | 3 | 1 | 1-92 |
| VH1-13-14 | 18 | 1 | I-3; V1-3 |
| VH1-13-15 | 19 | 1 | DP15; V1-8 |
| VH1-13-16 | 3 | 1 | 21-2; 3-1; DP7; V1-46 |
| VH1-13-17 | 16 | 1 | HG3 |
| VH1-13-18 | 19 | 1 | DP4; 7-2; V1-45 |
| VH1-13-19 | 27 | 1 | COS 5 |
| VH1-1X-1 | 19 | 1 | DP5:1-24P |
| VH2-21-1 | 18 | 2 | II-5b |
| VH2-31-1 | 2 | 2 | VH2S12-1 |
| VH2-31-2 | 2 | 2 | VH2S12-7 |
| VH2-31-3 | 2 | 2 | VH2S12-9; DP27 |
| VH2-31-4 | 2 | 2 | VH2S12-10 |
| VH2-31-5 | 14 | 2 | V2-26; DP26; 2-26 |
| VH2-31-6 | 15 | 2 | VF2-26 |
| VH2-31-7 | 19 | 2 | DP28; DA-7 |
| VH2-31-14 | 7 | 2 | YAC-3; 2-70 |
| VH2-31-8 | 2 | 2 | VH2S12-5 |
| VH2-31-9 | 2 | 2 | VH2S12-12 |
| VH2-31-10 | 18 | 2 | II-5: V2-5 |
| VH2-31-11 | 2 | 2 | VH2S12-2; VH2S12-8 |
| VH2-31-12 | 2 | 2 | VH2S12-4; VH2S12-6 |
| VH2-31-13 | 2 | 2 | VH2S12-14 |
| VH3-11-1 | 13 | 3 | v65-2; DP44 |
| VH3-11-2 | 19 | 3 | DP45 |
| VH3-11-3 | 3 | 3 | 13-2; DP48 |
| VH3-11-4 | 19 | 3 | DP52 |
| VH3-11-5 | 14 | 3 | v3-13 |
| VH3-11-6 | 19 | 3 | DP42 |
| VH3-11-7 | 3 | 3 | 8-1B: YAC-5; 3-66 |
| VH3-11-8 | 14 | 3 | V3-53 |
| VH3-13-1 | 3 | 3 | 22-2B; DP35; V3-11 |
| VH3-13-5 | 19 | 3 | DP59; VH19; V3-35 |
| VH3-13-6 | 25 | 3 | fl-pl; DP61 |
| VH3-13-7 | 19 | 3 | DP46; GL-SJ2; COS 8; hv3005; hv3005f3; 3d21b; 56pl |
| VH3-13-8 | 24 | 3 | VH26 |
| VH3-13-9 | 5 | 3 | vh26c |
| VH3-13-10 | 19 | 3 | DP47; VH26; 3-23 |
| VH3-13-11 | 3 | 3 | 1-91 |
| VH3-13-12 | 19 | 3 | DP58 |
| VH3-13-13 | 3 | 3 | 1-9III; DP49; 3-30; 3d28.1 |
| VH3-13-14 | 24 | 3 | 3019B9; DP50; 3-33; 3d277 |
| VH3-13-15 | 27 | 3 | COS 3 |
| VH3-13-16 | 19 | 3 | DP51 |
| VH3-13-17 | 16 | 3 | H11 |
| VH3-13-18 | 19 | 3 | DP53; COS 6; 3-74; DA-8 |
| VH3-13-19 | 19 | 3 | DP54: VH3-11; V3-7 |
| VH3-13-20 | 14 | 3 | V3-64; YAC-6 |
| VH3-13-21 | 14 | 3 | V3-48 |
| VH3-13-22 | 14 | 3 | V3-43; DP33 |
| VH3-13-23 | 14 | 3 | V3-33 |
| VH3-13-24 | 14 | 3 | V3-21; DP77 |
| VH3-13-25 | 14 | 3 | V3-20; DP32 |
| VH3-13-26 | 14 | 3 | V3-9; DP31 |
| VH3-14-1 | 3 | 3 | 12-2; DP29; 3-72; DA-3 |
| VH3-14-4 | 7 | 3 | YAC-9; 3-73; MTGL |
| VH3-14-2 | 4 | 3 | VHD26 |
| VH3-14-3 | 19 | 3 | DP30 |
| VH3-1X-1 | 1 | 3 | LSG8.1; LSG9.1; LSG10.1; HUM121GVH; HUM13IGVH |
| VH3-1X-2 | 1 | 3 | LSG11.1; HUM41GVH |
| VH3-1X-3 | 3 | 3 | 9-1; DP38; LSG7.1; RCG1.1; LSG1.1; LSG3.1; LSG5.1; HUM15IGVH; HUM2IGVH; HUM91GVH |
| VH3-1X-4 | 1 | 3 | LSG4.1 |
| VH3-1X-5 | 1 | 3 | LSG2.1 |
| VH3-1X-6 | 1 | 3 | LSG6.1; HUM10IGVH |
| VH3-1X-7 | 18 | 3 | 3-15; V3-15 |
| VH3-1X-8 | 1 | 3 | LSG12.1; HUM5IGVH |
| VH3-1X-9 | 14 | 3 | V3-49 |
| VH4-11-1 | 22 | 4 | Tou-VH4.21 |
| VH4-11-2 | 17 | 4 | VH4.21; DP63; VH5; 4d76; V4-34 |
| VH4-11-3 | 23 | 4 | 4.44 |
| VH4-11-4 | 23 | 4 | 4.44.3 |
| VH4-11-5 | 23 | 4 | 4.36 |
| VH4-11-6 | 23 | 4 | 4.37 |
| VH4-11-7 | 18 | 4 | IV-4; 435: V4-4 |
| VH4-11-8 | 17 | 4 | VH4.11; 3d197d; DP71; 58p2 |
| VH4-11-9 | 20 | 4 | H7 |
| VH4-11-10 | 20 | 4 | H8 |
| VH4-11-11 | 20 | 4 | H9 |
| VH4-11-12 | 17 | 4 | VH4.16 |
| VH4-11-13 | 23 | 4 | 4.38 |
| VH4-11-14 | 17 | 4 | VH4.15 |
| VH4-11-15 | 11 | 4 | 58 |
| VH4-11-16 | 10 | 4 | 71-4; V4-59 |
| VH4-21-1 | 11 | 4 | 11 |
| VH4-21-2 | 17 | 4 | VH4.17; VH4.23; 4d255; 4.40; DP69 |
| VH4-21-3 | 17 | 4 | VH4.19; 79; V4-4b |
| VH4-21-4 | 19 | 4 | DP70; 4d68; 4.41 |
| VH4-21-5 | 19 | 4 | DP67; VH4-4B |
| VH4-21-6 | 17 | 4 | VH4.22; VHSP; VH-JA |
| VH4-21-7 | 17 | 4 | VH4.13; 1-9II; 12G-1; 3d28d; 4.42; DP68; 4-28 |
| VH4-21-8 | 26 | 4 | hv4005;3d24d |
| VH4-21-9 | 17 | 4 | VH4.14 |
| VH4-31-1 | 23 | 4 | 434; 3d230d; DP78 |
| VH4-31-2 | 23 | 4 | 4.34.2 |
| VH4-31-3 | 19 | 4 | DP64; 3d216d |
| VH4-31-4 | 19 | 4 | DP65; 4-31; 3d277d |
| VH4-31-5 | 23 | 4 | 4.33; 3d75d |
| VH4-31-6 | 20 | 4 | H10 |
| VH4-31-7 | 20 | 4 | H11 |
| VH4-31-8 | 23 | 4 | 4.31 |
| VH4-31-9 | 23 | 4 | 4.32 |
| VH4-31-10 | 20 | 4 | 3d277d |
| VH4-31-11 | 20 | 4 | 3d216d |
| VH4-31-12 | 20 | 4 | 3d279d |
| VH4-31-13 | 17 | 4 | VH4.18; 4d154; DP79 |
| VH4-31-14 | 8 | 4 | V4-39 |
| VH4-31-15 | 11 | 4 | 2-1; DP79 |
| VH4-31-16 | 23 | 4 | 4.30 |
| VH4-31-17 | 17 | 4 | VH4.12 |
| VH4-31-18 | 10 | 4 | 71-2; DP66 |
| VH4-31-19 | 23 | 4 | 439 |
| VH4-31-20 | 8 | 4 | V4-61 |
| VH5-12-1 | 9 | 5 | VH251; DP73; VHVCW; 51-R1; VHVLB; VHVCH; VHVTT; VHVAU; VHVBLK; VhAU; V5-51 |
| VH5-12-2 | 17 | 5 | VHVJB |
| VH5-12-3 | 3 | 5 | 1-v; DP80; 5-78 |
| VH5-12-4 | 9 | 5 | VH32; VHVRG; VHVMW; 5-2R1 |
| VH6-35-1 | 4 | 6 | VHV1; VH6; VHVIIS; VHVITE; VHVIJB; VHVICH; VHVICW; VHVIBLK; VHVIMW; DP74; 6-1G1; V6-1 |

**Table 2A: rearranged human kappa sequences**

| **Name¹** | **aa²** | **Computed family³** | **Germline gene⁴** | **Diff. to germline⁵** | **% diff. to germline⁶** | **Reference⁷** |
|---|---|---|---|---|---|---|
| III-3R | 108 | 1 | O8 | 1 | 1,1% | 70 |
| No.86 | 109 | 1 | O8 | 3 | 3,2% | 80 |
| AU | 108 | 1 | O8 | 6 | 6,3% | 103 |
| ROY | 108 | 1 | O8 | 6 | 6,3% | 43 |
| IC4 | 108 | 1 | O8 | 6 | 6,3% | 70 |
| HIV-B26 | 106 | 1 | O8 | 3 | 3,2% | 8 |
| GRI | 108 | 1 | O8 | 8 | 8,4% | 30 |
| AG | 106 | 1 | O8 | 8 | 8,6% | 116 |
| REI | 108 | 1 | O8 | 9 | 9,5% | 86 |
| CLL PATIENT 16 | 88 | 1 | O8 | 2 | 2,3% | 122 |
| CLL PATIENT 14 | 87 | 1 | O8 | 2 | 2,3% | 122 |
| CLL PATIENT 15 | 88 | 1 | O8 | 2 | 2,3% | 122 |
| GM4672 | 108 | 1 | O8 | 11 | 11,6% | 24 |
| HUM. YFC51.1 | 108 | 1 | O8 | 12 | 12,6% | 110 |
| LAY | 108 | 1 | O8 | 12 | 12,6% | 48 |
| HIV-b13 | 106 | 1 | O8 | 9 | 9,7% | 8 |
| MAL-NaCl | 108 | 1 | O8 | 13 | 13,7% | 102 |
| STRAb SA-1A | 108 | 1 | O2 | 0 | 0,0% | 120 |
| HuVHCAMP | 108 | 1 | O8 | 13 | 13,7% | 100 |
| CRO | 108 | 1 | O2 | 10 | 10,5% | 30 |
| Am107 | 108 | 1 | O2 | 12 | 12,6% | 108 |
| WALKER | 107 | 1 | O2 | 4 | 4,2% | 57 |
| III-2R | 109 | 1 | A20 | 0 | 0,0% | 70 |
| FOGI-A4 | 107 | 1 | A20 | 4 | 4,2% | 41 |
| HK137 | 95 | 1 | L1 | 0 | 0,0% | 10 |
| CEA4-8A | 107 | 1 | O2 | 7 | 7,4% | 41 |
| Va' | 95 | 1 | L4 | 0 | 0,0% | 90 |
| TR1.21 | 108 | 1 | O2 | 4 | 4,2% | 92 |
| HAU | 108 | 1 | O2 | 6 | 6,3% | 123 |
| HK102 | 95 | 1 | L12(1) | 0 | 0,0% | 9 |
| H20C3K | 108 | 1 | L12(2) | 3 | 3,2% | 125 |
| CHEB | 108 | 1 | O2 | 7 | 7,4% | 5 |
| HK134 | 95 | 1 | L15(2) | 0 | 0,0% | 10 |
| TEL9 | 108 | 1 | O2 | 9 | 9,5% | 73 |
| TR1.32 | 103 | 1 | O2 | 3 | 3,2% | 92 |
| RF-KES1 | 97 | 1 | A20 | 4 | 4,2% | 121 |
| WES | 108 | 1 | L5 | 10 | 10,5% | 61 |
| DILp1 | 95 | 1 | O4 | 1 | 1,1% | 70 |
| SA-4B | 107 | 1 | L12(2) | 8 | 8,4% | 120 |
| HK101 | 95 | 1 | L15(1) | 0 | 0,0% | 9 |
| TR1.23 | 108 | 1 | O2 | 5 | 5,3% | 92 |
| HF2-1/17 | 108 | 1 | A30 | 0 | 0,0% | 4 |
| 2E7 | 108 | 1 | A30 | 1 | 1,1% | 62 |
| 33.C9 | 107 | 1 | L12(2) | 7 | 7,4% | 126 |
| 3D6 | 105 | 1 | L12(2) | 2 | 2,1% | 34 |
| I-2a | 108 | 1 | L8 | 8 | 8,4% | 70 |
| RF-KL1 | 97 | 1 | L8 | 4 | 4,2% | 121 |
| TNF-E7 | 108 | 1 | A30 | 9 | 9,5% | 41 |
| TR1.22 | 108 | 1 | O2 | 7 | 7,4% | 92 |
| HIV-B35 | 106 | 1 | O2 | 2 | 2,2% | 8 |
| HIV-b22 | 106 | 1 | O2 | 2 | 2,2% | 8 |
| HIV-b27 | 106 | 1 | O2 | 2 | 2,2% | 8 |
| HIV-B8 | 107 | 1 | O2 | 10 | 10,8% | 8 |
| HIV-b8 | 107 | 1 | O2 | 10 | 10,8% | 8 |
| RF-SJ5 | 95 | 1 | A30 | 5 | 5,3% | 113 |
| GAL(I) | 108 | 1 | A30 | 6 | 6,3% | 64 |
| R3.5H5G | 108 | 1 | O2 | 6 | 6,3% | 70 |
| HIV-b14 | 106 | 1 | A20 | 2 | 2,2% | 8 |
| TNF-E1 | 105 | 1 | L5 | 8 | 8,4% | 41 |
| WEA | 108 | 1 | A30 | 8 | 8,4% | 37 |
| EU | 108 | 1 | L12(2) | 5 | 5,3% | 40 |
| FOG1-G8 | 108 | 1 | L8 | 11 | 11,6% | 41 |
| X7RG1 | 108 | 1 | L1 | 8 | 8,4% | 70 |
| BLI | 108 | 1 | L8 | 3 | 3,2% | 72 |
| KUE | 108 | 1 | L12(2) | 11 | 11,6% | 32 |
| LUNm01 | 108 | 1 | L12(2) | 10 | 10,5% | 6 |
| HIV-b1 | 106 | 1 | A20 | 4 | 4,3% | 8 |
| HIV-s4 | 103 | 1 | O2 | 2 | 2,2% | 8 |
| CAR | 107 | 1 | L12(2) | 11 | 11,7% | 79 |
| BR | 107 | 1 | L12(2) | 11 | 11,6% | 50 |
| CLL PATIENT 10 | 88 | 1 | O2 | 0 | 0,0% | 122 |
| CLL PATIENT 12 | 88 | 1 | O2 | 0 | 0,0% | 122 |
| KING | 108 | 1 | L12(2) | 12 | 12,6% | 30 |
| V13 | 95 | 1 | L24 | 0 | 0,0% | 46 |
| CLL PATIENT 11 | 87 | 1 | O2 | 0 | 0,0% | 122 |
| CLL PATIENT 13 | 87 | 1 | O2 | 0 | 0,0% | 122 |
| CLL PATIENT 9 | 88 | 1 | O12 | 1 | 1,1% | 122 |
| HIV-B2 | 106 | 1 | A20 | 9 | 9,7% | 8 |
| HIV-b2 | 106 | 1 | A20 | 9 | 9,7% | 8 |
| LL PATIENT 5 | 88 | 1 | A20 | 1 | 1,1% | 122 |
| CLL PATIENT 1 | 88 | 1 | L8 | 2 | 2,3% | 122 |
| CLL PATIENT 2 | 88 | 1 | L8 | 0 | 0,0% | 122 |
| CLL PATIENT 7 | 88 | 1 | L5 | 0 | 0,0% | 122 |
| CLL PATIENT 8 | 88 | 1 | L5 | 0 | 0,0% | 122 |
| HIV-b5 | 105 | 1 | L5 | 11 | 12,0% | 8 |
| CLL PATIENT 3 | 87 | 1 | L8 | 1 | 1,1% | 122 |
| CLL PATIENT 4 | 88 | 1 | L9 | 0 | 0,0% | 122 |
| CLL PATIENT 18 | 85 | 1 | L9 | 6 | 7,1% | 122 |
| CLL PATIENT 17 | 86 | 1 | L12(2) | 7 | 8,1% | 122 |
| HIV-b20 | 107 | 3 | A27 | 11 | 11,7% | 8 |
| 2C12 | 108 | 1 | L12(2) | 20 | 21,1% | 68 |
| IB11 | 108 | 1 | L12(2) | 20 | 21,1% | 68 |
| 1H1 | 108 | 1 | L12(2) | 21 | 22,1% | 68 |
| 2AI2 | 108 | 1 | L12(2) | 21 | 22,1% | 68 |
| CUR | 109 | 3 | A27 | 0 | 0,0% | 66 |
| GLO | 109 | 3 | A27 | 0 | 0,0% | 16 |
| RF-TS1 | 96 | 3 | A27 | 0 | 0,0% | 121 |
| GAR' | 109 | 3 | A27 | 0 | 0,0% | 67 |
| FLO | 109 | 3 | A27 | 0 | 0,0% | 66 |
| PIE | 109 | 3 | A27 | 0 | 0,0% | 91 |
| HAH 14.1 | 109 | 3 | A27 | 1 | 1,0% | 51 |
| HAH 14.2 | 109 | 3 | A27 | 1 | 1,0% | 51 |
| HAH 16.1 | 109 | 3 | A27 | 1 | 1,0% | 51 |
| NOV | 109 | 3 | A27 | 1 | 1,0% | 52 |
| 33.F12 | 108 | 3 | A27 | 1 | 1,0% | 126 |
| 8E10 | 110 | 3 | A27 | 1 | 1,0% | 25 |
| TH3 | 109 | 3 | A27 | 1 | 1,0% | 25 |
| HIC (R) | 108 | 3 | A27 | 0 | 0,0% | 51 |
| SON | 110 | 3 | A27 | 1 | 1,0% | 67 |
| PAY | 109 | 3 | A27 | 1 | 1,0% | 66 |
| GOT | 109 | 3 | A27 | 1 | 1,0% | 67 |
| mAbA6H4C5 | 109 | 3 | A27 | 1 | 1,0% | 12 |
| BOR' | 109 | 3 | A27 | 2 | 2,1% | 84 |
| RF-SJ3 | 96 | 3 | A27 | 2 | 2,1% | 121 |
| SIE | 109 | 3 | A27 | 2 | 2,1% | 15 |
| ESC | 109 | 3 | A27 | 2 | 2,1% | 98 |
| HEW' | 110 | 3 | A27 | 2 | 2,1% | 98 |
| YES8c | 109 | 3 | A27 | 3 | 3,1% | 33 |
| TI | 109 | 3 | A27 | 3 | 3,1% | 114 |
| mAb113 | 109 | 3 | A27 | 3 | 3,1% | 71 |
| HEW | 107 | 3 | A27 | 0 | 0,0% | 94 |
| BRO | 106 | 3 | A27 | 0 | 0,0% | 94 |
| ROB | 106 | 3 | A27 | 0 | 0,0% | 94 |
| NG9 | 96 | 3 | A27 | 4 | 4,2% | 11 |
| NEU | 109 | 3 | A27 | 4 | 4,2% | 66 |
| WOL | 109 | 3 | A27 | 4 | 4,2% | 2 |
| 35G6 | 109 | 3 | A27 | 4 | 4,2% | 59 |
| RF-SJ4 | 109 | 3 | A11 | 0 | 0,0% | 88 |
| KAS | 109 | 3 | A27 | 4 | 4,2% | 84 |
| BRA | 106 | 3 | A27 | 1 | 1,1% | 94 |
| HAH | 106 | 3 | A27 | 1 | 1,1% | 94 |
| HIC | 105 | 3 | A27 | 0 | 0,0% | 94 |
| FS-2 | 109 | 3 | A27 | 6 | 6,3% | 87 |
| JH' | 107 | 3 | A27 | 6 | 6,3% | 38 |
| EV1-15 | 109 | 3 | A27 | 6 | 6,3% | 83 |
| SCA | 108 | 3 | A27 | 6 | 6,3% | 65 |
| mab112 | 109 | 3 | A27 | 6 | 6,3% | 71 |
| SIC | 103 | 3 | A27 | 3 | 3,3% | 94 |
| SA-4A | 109 | 3 | A27 | 6 | 6,3% | 120 |
| SER | 108 | 3 | A27 | 6 | 6,3% | 98 |
| GOL' | 109 | 3 | A27 | 7 | 7,3% | 82 |
| B5G10K | 105 | 3 | A27 | 9 | 9,7% | 125 |
| HG2B10K | 110 | 3 | A27 | 9 | 9,4% | 125 |
| Taykv322 | 105 | 3 | A27 | 5 | 5,4% | 52 |
| CLL PATIENT 24 | 89 | 3 | A27 | 1 | 1,1% | 122 |
| HIV-b24 | 107 | 3 | A27 | 7 | 7,4% | 8 |
| RIV-b6 | 107 | 3 | A27 | 7 | 7,4% | 8 |
| Taykv310 | 99 | 3 | A27 | 1 | 1,1% | 52 |
| KA3D1 | 108 | 3 | L6 | 0 | 0,0% | 85 |
| 19.E7 | 107 | 3 | L6 | 0 | 0,0% | 126 |
| rsv6L | 109 | 3 | A27 | 12 | 12,5% | **7** |
| Taykv320 | 98 | 3 | A27 | 1 | 1,2% | 52 |
| Vh | 96 | 3 | L10(2) | 0 | 0,0% | 89 |
| LS8 | 108 | 3 | L6 | 1 | 1,1% | 109 |
| LS1 | 108 | 3 | L6 | 1 | 1,1% | 109 |
| LS2S3-3 | 107 | 3 | L6 | 2 | 2,1% | 99 |
| LS2 | 108 | 3 | L6 | 1 | 1,1% | 109 |
| LS7 | 108 | 3 | L6 | 1 | 1,1% | 109 |
| LS2S3-4d | 107 | 3 | L6 | 2 | 2,1% | 99 |
| LS2S3-4a | 107 | 3 | L6 | 2 | 2,1% | 99 |
| LS4 | 108 | 3 | L6 | 1 | 1,1% | 109 |
| LS6 | 108 | 3 | L6 | 1 | 1,1% | 109 |
| LS2S3-10a | 107 | 3 | L6 | 2 | 2,1% | 99 |
| LS2S3-8c | 107 | 3 | L6 | 2 | 2,1% | 99 |
| LS5 | 108 | 3 | L6 | 1 | 1,1% | 109 |
| LS2S3-5 | 107 | 3 | L6 | 3 | 3,2% | 99 |
| LUNm03 | 109 | 3 | A27 | 13 | 13,5% | 6 |
| IARC/BL41 | 108 | 3 | A27 | 13 | 13,7% | 55 |
| slkv22 | 99 | 3 | A27 | 3 | 3,5% | 13 |
| POP | 108 | 3 | L6 | 4 | 4,2% | 111 |
| LS2S3-10b | 107 | 3 | L6 | 3 | 3,2% | 99 |
| LS2S3-8f | 107 | 3 | L6 | 3 | 3,2% | 99 |
| LS2S3-12 | 107 | 3 | L6 | 3 | 3,2% | 99 |
| HIV-B30 | 107 | 3 | A27 | 11 | 11,7% | 8 |
| HIV-B20 | 107 | 3 | A27 | 11 | 11,7% | 8 |
| HIV-b3 | 108 | 3 | A27 | 11 | 11,7% | 8 |
| HIV-s6 | 104 | 3 | A27 | 9 | 9,9% | 8 |
| YSE | 107 | 3 | L2/L16 | 1 | 1,1% | 72 |
| POM | 109 | 3 | L2/L16 | 9 | 9,4% | 53 |
| Humkv328 | 95 | 3 | L2/L16 | 1 | 1,1% | 19 |
| CLL | 109 | 3 | L2/L16 | 3 | 3,2% | 47 |
| LES | 96 | 3 | L2/L16 | 3 | 3,2% | 38 |
| HIV-s5 | 104 | 3 | A27 | 11 | 12,1%. | 8 |
| HIV-s7 | 104 | 3 | A27 | 11 | 12,1% | 8 |
| slkv1 | 99 | 3 | A27 | 7 | 8,1% | 13 |
| Humka31 es | 95 | 3 | L2/L16 | 4 | 4,2% | 18 |
| slkv12 | 101 | 3 | A27 | 8 | 9,2% | 13 |
| RF-TS2 | 95 | 3 | L2/L16 | 3 | 3,2% | 121 |
| II-1 | 109 | 3 | L2/L16 | 4 | 4,2% | 70 |
| HIV-s3 | 105 | 3 | A27 | 13 | 14,3% | 8 |
| RF-TMCI | 96 | 3 | L6 | 10 | 10,5% | 121 |
| GER | 109 | 3 | L2/L16 | 7 | 7,4% | 75 |
| GF4/1.1 | 109 | 3 | L2/L16 | 8 | 8,4% | 36 |
| mAb114 | 109 | 3 | L2/L16 | 6 | 6,3% | 71 |
| HIV-loop13 | 109 | 3 | L2/L16 | 7 | 7,4% | 8 |
| bkv16 | 86 | 3 | L6 | 1 | 1,2% | 13 |
| CLL PATIENT 29 | 86 | 3 | L6 | 1 | 1,2% | 122 |
| slkv9 | 98 | 3 | L6 | 3 | 3,5% | 13 |
| bkv17 | 99 | 3 | L6 | 1 | 1,2% | 13 |
| slkv14 | 99 | 3 | L6 | 1 | 1,2% | 13 |
| slkv16 | 101 | 3 | L6 | 2 | 2,3% | 13 |
| bkv33 | 101 | 3 | L6 | 4 | 4,7% | 13 |
| slkv15 | 99 | 3 | L6 | 2 | 2,3% | 13 |
| bkv6 | 100 | 3 | L6 | 3 | 3,5% | 13 |
| R6B8K | 108 | 3 | L2/L16 | 12 | 12,6% | 125 |
| AL 700 | 107 | 3 | L2/L16 | 9 | 9,5% | 117 |
| slkv11 | 100 | 3 | L2/L16 | 3 | 3,5% | 13 |
| slkv4 | 97 | 3 | L6 | 4 | 4,8% | 13 |
| CLL PATIENT 26 | 87 | 3 | L2/L16 | 1 | 1,1% | 122 |
| AL Se124 | 103 | 3 | L2/L16 | 9 | 9,5% | 117 |
| slkv13 | 100 | 3 | L2/L16 | 6 | 7,0% | 13 |
| bkv7 | 100 | 3 | L2/L16 | 5 | 5,8% | 13 |
| bkv22 | 100 | 3 | L2/L16 | 6 | 7,0% | 13 |
| CLL PATIENT 27 | 84 | 3 | L2/L16 | 0 | 0,0% | 122 |
| bkv35 | 100 | 3 | L6 | 8 | 9,3% | 13 |
| CLL PATIENT 25 | 87 | 3 | L2/L16 | 4 | 4,6% | 122 |
| slkv3 | 86 | 3 | L2/L16 | 7 | 8,1% | 13 |
| slkv7 | 99 | 1 | O2 | 7 | 8,1% | 13 |
| HuFd79 | 111 | 3 | L2/L16 | 24 | 24,2% | 21 |
| RAD | 99 | 3 | A27 | 9 | 10,3% | 78 |
| CLL PATIENT 28 | 83 | 3 | L2/L16 | 4 | 4,8% | 122 |
| REE | 104 | 3 | L2/L16 | 25 | 27,2% | 95 |
| FR4 | 99 | 3 | A27 | 8 | 9,2% | 77 |
| MD3.3 | 92 | 3 | L6 | 1 | 1,3% | 54 |
| MD3.1 | 92 | 3 | L6 | 0 | 0,0% | 54 |
| GA3.6 | 92 | 3 | L6 | 2 | 2,6% | 54 |
| M3.5N | 92 | 3 | L6 | 3 | 3,8% | 54 |
| WEI' | 82 | 3 | A27 | 0 | 0,0% | 65 |
| MD3.4 | 92 | 3 | L2/L16 | 1 | 1,3% | 54 |
| MD3.2 | 91 | 3 | L6 | 3 | 3,8% | 54 |
| VER | 97 | 3 | A27 | 19 | 22,4% | 20 |
| CLL PATIENT 30 | 78 | 3 | L6 | 3 | 3,8% | 122 |
| M3.IN | 92 | 3 | L2/L16 | 1 | 1,3% | 54 |
| MD3.6 | 91 | 3 | L2/L16 | 0 | 0,0% | 54 |
| MD3.8 | 91 | 3 | L2/L16 | 0 | 0,0% | 54 |
| GA3.4 | 92 | 3 | L6 | 7 | 9,0% | 54 |
| M3.6N | 92 | 3 | A27 | 0 | 0,0% | 54 |
| MD3.10 | 92 | 3 | A27 | 0 | 0,0% | 54 |
| MD3.13 | 91 | 3 | A27 | 0 | 0,0% | .54 |
| MD3.7 | 93 | 3 | A27 | 0 | 0,0% | 54 |
| MD3.9 | 93 | 3 | A27 | 0 | 0,0% | 54 |
| GA3.1 | 93 | 3 | A27 | 6 | 7,6% | 54 |
| bkv32 | 101 | 3 | A27 | 5 | 5,7% | 13 |
| GA3.5 | 93 | 3 | A27 | 5 | 6,3% | 54 |
| GA3.7 | 92 | 3 | A27 | 7 | 8,9% | 54 |
| MD3.12 | 92 | 3 | A27 | 2 | 2,5% | 54 |
| M3.2N | 90 | 3 | L6 | 6 | 7,8% | 54 |
| MD3.5 | 92 | 3 | A27 | 1 | 1,3% | 54 |
| M3.4N | 91 | 3 | L2/L16 | 8 | 10,3% | 54 |
| M3.8N | 91 | 3 | L2/L16 | 7 | 9,0% | 54 |
| M3.7N | 92 | 3 | A27 | 3 | 3,8% | 54 |
| GA3.2 | 92 | 3 | A27 | 9 | 11,4% | 54 |
| GA3.8 | 93 | 3 | A27 | 4 | 5,1% | 54 |
| GA3.3 | 92 | 3 | A27 | 8 | 10,1% | 54 |
| M3.3N | 92 | 3 | A27 | 5 | 6,3% | 54 |
| B6 | 83 | 3 | A27 | 8 | 11,3% | 78 |
| E29.1 KAPPA | 78 | 3 | L2/L16 | 0 | 0,0% | 22 |
| SCW | 108 | 1 | O8 | 12 | 12,6% | 31 |
| REI-based | 107 | 1 | O8 | 14 | 14,7% | 39 |
| CAMPATH-9 | | | | | | |
| RZ | 107 | 1 | O8 | 14 | 14,7% | 50 |
| BI | 108 | 1 | O8 | 14 | 14,7% | 14 |
| AND | 107 | 1 | O2 | 13 | 13,7% | 69 |
| 2A4 | 109 | 1 | O2 | 12 | 12,6% | 23 |
| KA | 108 | 1 | O8 | 19 | 20,0% | 107 |
| MEV | 109 | 1 | O2 | 14 | 14,7% | 29 |
| DEE | 106 | 1 | O2 | 13 | 14,0% | 76 |
| OU(IOC) | 108 | 1 | O2 | 18 | 18,9% | 60 |
| HuRSV19VK | 111 | 1 | O8 | 21 | 21,0% | 115 |
| SP2 | 108 | 1 | O2 | 17 | 17,9% | 93 |
| BJ26 | 99 | 1 | O8 | 21 | 24,1% | 1 |
| NI | 112 | 1 | O8 | 24 | 24,2% | 106 |
| BMA 0310EUCIV2 | 106 | 1 | L12(1) | 21 | 22,3% | 105 |
| CLL PATIENT 6 | 71 | 1 | A20 | 0 | 0,0% | 122 |
| BJ19 | 85 | 1 | O8 | 16 | 21,9% | 1 |
| GM 607 | 113 | 2 | A3 | 0 | 0,0% | 58 |
| R5A3K | 114 | 2 | A3 | 1 | 1,0% | 125 |
| R1C8K | 114 | 2 | A3 | 1 | 1,0% | 125 |
| VK2.R149 | 113 | 2 | A3 | 2 | 2,0% | 118 |
| TR1.6 | 109 | 2 | A3 | 4 | 4,0% | 92 |
| TR1.37 | 104 | 2 | A3 | 5 | 5,0% | 92 |
| FS-1 | 113 | 2 | A3 | 6 | 6,0% | 87 |
| TR1.8 | 110 | 2 | A3 | 6 | 6,0% | 92 |
| NIM | 113 | 2 | A3 | 8 | 8,0% | 28 |
| Inc | 112 | 2 | A3 | 11 | 11,0% | 35 |
| TEW | 107 | 2 | A3 | 6 | 6,4% | 96 |
| CUM | 114 | 2 | O1 | 7 | 6,9% | 44 |
| HRF1 | 71 | 2 | A3 | 4 | 5,6% | 124 |
| CLL PATIENT 19 | 87 | 2 | A3 | 0 | 0,0% | 122 |
| CLL PATIENT 20 | 87 | 2 | A3 | 0 | 0,0% | 122 |
| MIL | 112 | 2 | A3 | 16 | 16,2% | 26 |
| FR | 113 | 2 | A3 | 20 | 20,0% | 101 |
| MAL-Urine | 83 | 1 | O2 | 6 | 8,6% | 102 |
| Taykv306 | 73 | 3 | A27 | 1 | 1,6% | 52 |
| Taykv312 | 75 | 3 | A27 | 1 | 1,6% | 52 |
| HIV-b29 | 93 | 3 | A27 | 14 | 17,5% | 8 |
| 1-185-37 | 110 | 3 | A27 | 0 | 0,0% | 119 |
| 1-187-29 | 110 | 3 | A27 | 0 | 0,0% | 119 |
| TT117 | 110 | 3 | A27 | 9 | 9,4% | 63 |
| HIV-loop8 | 108 | 3 | A27 | 16 | 16,8% | 8 |
| rsv23L | 108 | 3 | A27 | 16 | 16,8% | 7 |
| HIV-b7 | 107 | 3 | A27 | 14 | 14,9% | 8 |
| HIV-b11 | 107 | 3 | A27 | 15 | 16,0% | 8 |
| HIV-LC1 | 107 | 3 | A27 | 19 | 20,2% | 8 |
| HIV-LC7 | 107 | 3 | A27 | 20 | 21,3% | 8 |
| HIV-LC22 | 107 | 3 | A27 | 21 | 22,3% | 8 |
| HIV-LC13 | 107 | 3 | A27 | 21 | 22,3% | 8 |
| HIV-LC3 | 107 | 3 | A27 | 21 | 22,3% | 8 |
| HIV-LC5 | 107 | 3 | A27 | 21 | 22,3% | 8 |
| HIV-LC28 | 107 | 3 | A27 | 21 | 22,3% | 8 |
| HIV-b4 | 107 | 3 | A27 | 22 | 23,4% | 8 |
| CLL PATIENT 31 | 87 | 3 | A27 | 15 | 17,2% | 122 |
| HIV-loop2 | 108 | 3 | L2/L16 | 17 | 17,9% | 8 |
| HIV-loop35 | 108 | 3 | L2/L16 | 17 | 17,9% | 8 |
| HIV-LC11 | 107 | 3 | A27 | 23 | 24,5% | 8 |
| HIV-LC24 | 107 | 3 | A27 | 23 | 24,5% | 8 |
| HIV-b12 | 107 | 3 | A27 | 24 | 25,5% | 8 |
| HIV-LC25 | 107 | 3 | A27 | 24 | 25,5% | 8 |
| HIV-b21 | 107 | 3 | A27 | 24 | 25,5% | 8 |
| HIV-LC26 | 107 | 3 | A27 | 26 | 27,7% | 8 |
| G3D10K | 108 | 1 | L12(2) | 12 | 12,6% | 125 |
| TT125 | 108 | 1 | L5 | 8 | 8,4% | 63 |
| HIV-s2 | 103 | 3 | A27 | 28 | 31,1% | 8 |
| 265-695 | 108 | 1 | L5 | 7 | 7,4% | 3 |
| 2-115-19 | 108 | 1 | A30 | 2 | 2,1% | 119 |
| rsv13L | 107 | 1 | O2 | 20 | 21,1% | 7 |
| HIV-b18 | 106 | 1 | O2 | 14 | 15,1% | 8 |
| RF-KL5 | 98 | 3 | L6 | 36 | 36,7% | 97 |
| ZM1-1 | 113 | 2 | A17 | 7 | 7,0% | 3 |
| HIV-s8 | 103 | 1 | O8 | 16 | 17,8% | 8 |
| K-EV15 | 95 | 5 | B2 | 0 | 0,0% | 112 |
| RF-TS3 | 100 | 2 | A23 | 0 | 0,0% | 121 |
| HF-21/28 | 111 | 2 | A17 | 1 | 1,0% | 17 |
| RPMI6410 | 113 | 2 | A17 | 1 | 1,0% | 42 |
| JC11 | 113 | 2 | A17 | 1 | 1,0% | 49 |
| O-81 | 114 | 2 | A17 | 5 | 5,0% | 45 |
| FK-001 | 113 | 4 | B3 | 0 | 0,0% | 81 |
| CD5+.28 | 101 | 4 | B3 | 1 | 1,0% | 27 |
| LEN | 114 | 4 | B3 | 1 | 1,0% | 104 |
| UC | 114 | 4 | B3 | 1 | 1,0% | 111 |
| CD5+.5 | 101 | 4 | B3 | 1 | 1,0% | 27 |
| CD5+.26 | 101 | 4 | B3 | 1 | 1,0% | 27 |
| CD5+.12 | 101 | 4 | B3 | 2 | 2,0% | 27 |
| CD5+.23 | 101 | 4 | B3 | 2 | 2,0% | 27 |
| CD5+.7 | 101 | 4 | B3 | 2 | 2,0% | 27 |
| VJI | 113 | 4 | B3 | 3 | 3,0% | 56 |
| LOC | 113 | 4 | B3 | 3 | 3,0% | 72 |
| MAL | 113 | 4 | B3 | 3 | 3,0% | 72 |
| CD5+.6 | 101 | 4 | B3 | 3 | 3,0% | 27 |
| H2F | 113 | 4 | B3 | 3 | 3,0% | 70 |
| PB17IV | 114 | 4 | B3 | 4 | 4,0% | 74 |
| CD5+.27 | 101 | 4 | B3 | 4 | 4,0% | 27 |
| CD5+.9 | 101 | 4 | B3 | 4 | 4,0% | 27 |
| CD5-.28 | 101 | 4 | B3 | 5 | 5,0% | 27 |
| CD5-.26 | 101 | 4 | B3 | 6 | 5,9% | 27 |
| CD5+.24 | 101 | 4 | B3 | 6 | 5,9% | 27 |
| CD5+.10 | 101 | 4 | B3 | 6 | 5,9% | 27 |
| CD5-.19 | 101 | 4 | B3 | 6 | 5,9% | 27 |
| CD5-.18 | 101 | 4 | B3 | 7 | 6,9% | 27 |
| CD5-.16 | 101 | 4 | B3 | 8 | 7,9% | 27 |
| CD5-.24 | 101 | 4 | B3 | 8 | 7,9% | 27 |
| CD5-.17 | 101 | 4 | B3 | 10 | 9,9% | 27 |
| MD4.1 | 92 | 4 | B3 | 0 | 0,0% | 54 |
| MD4.4 | 92 | 4 | B3 | 0 | 0,0% | 54 |
| MD4.5 | 92 | 4 | B3 | 0 | 0,0% | 54 |
| MD4.6 | 92 | 4 | B3 | 0 | 0,0% | 54 |
| MD4.7 | 92 | 4 | B3 | 0 | 0,0% | 54 |
| MD4.2 | 92 | 4 | B3 | 1 | 1,3% | 54 |
| MD4.3 | 92 | 4 | B3 | 5 | 6,3% | 54 |
| CLL PATIENT 22 | 87 | 2 | A17 | 2 | 2,3% | 122 |
| CLL PATIENT 23 | 84 | 2 | A17 | 2 | 2,4% | 122 |

**Table 2B: rearranged human lambda sequences**

| **Name¹** | **aa²** | **Computed family³** | **Germline gene⁴** | **Diff. to germline⁵** | **% diff. to germline⁶** | **Reference⁷** |
|---|---|---|---|---|---|---|
| WAH | 110 | 1 | DPL3 | 7 | 7% | 68 |
| 1B9/F2 | 112 | 1 | DPL3 | 7 | 7% | 9 |
| DIA | 112 | 1 | DPL2 | 7 | 7% | 36 |
| mAb67 | 89 | 1 | DPL3 | 0 | 0% | 29 |
| HiH2 | 110 | 1 | DPL3 | 12 | 11% | 3 |
| NIG-77 | 112 | 1 | DPL2 | 9 | 9% | 72 |
| OKA | 112 | 1 | DPL2 | 7 | 7% | 84 |
| KOL | 112 | 1 | DPL2 | 12 | 11% | 40 |
| T2:C5 | 111 | 1 | DPL5 | 0 | 0% | 6 |
| T2:C14 | 110 | 1 | DPL5 | 0 | 0% | 6 |
| PR-TS1 | 110 | 1 | DPL5 | 0 | 0% | 55 |
| 4G12 | 111 | 1 | DPL5 | 1 | 1% | 35 |
| KIM46L | 112 | 1 | HUMLV117 | 0 | 0% | 8 |
| Fog-B | 111 | 1 | DPL5 | 3 | 3% | 31 |
| 9F2L | 111 | 1 | DPL5 | 3 | 3% | 79 |
| mAb111 | 110 | 1 | DPL5 | 3 | 3% | 48 |
| PHOX15 | 111 | 1 | DPL5 | 4 | 4% | 49 |
| BL2 | 111 | 1 | DPL5 | 4 | 4% | 74 |
| NIG-64 | 111 | 1 | DPL5 | 4 | 4% | 72 |
| RF-SJ2 | 100 | 1 | DPL5 | 6 | 6% | 78 |
| ALEZI | 112 | 1 | DPL5 | 7 | 7% | 41 |
| ZIM | 112 | 1 | HUMLV117 | 7 | 7% | 18 |
| RF-SJ1 | 100 | 1 | DPL5 | 9 | 9% | 78 |
| IGLV1.1 | 98 | 1 | DPL4 | 0 | 0% | 1 |
| NEW | 112 | 1 | HUMLV117 | 11 | 10% | 42 |
| CB-201 | 87 | 1 | DPL2 | 1 | 1% | 62 |
| MEM | 109 | 1 | DPL2 | 6 | 6% | 50 |
| H210 | 111 | 2 | DPL10 | 4 | 4% | 45 |
| NOV | 110 | 2 | DPL10 | 8 | 8% | 25 |
| IEI | 111 | 2 | DPL10 | 8 | 8% | 24 |
| AL MC | 110 | 2 | DPL11 | 6 | 6% | 28 |
| MES | 112 | 2 | DPL11 | 8 | 8% | 84 |
| FOG1-A3 | 111 | 2 | DPL11 | 9 | 9% | 27 |
| AL NOV | 112 | 2 | DPL11 | 7 | 7% | 28 |
| HMST-1 | 110 | 2 | DPL11 | 4 | 4% | 82 |
| HBW4-1 | 108 | 2 | DPL12 | 9 | 9% | 52 |
| WH | 110 | 2 | DPL11 | 11 | 11% | 34 |
| 11-50 | 110 | 2 | DPL11 | 7 | 7% | 82 |
| HBp2 | 110 | 2 | DPL12 | 8 | 8% | 3 |
| NIG-84 | 113 | 2 | DPL11 | 12 | 11% | 73 |
| VIL | 112 | 2 | DPL11 | 9 | 9% | 58 |
| TRO | 111 | 2 | DPL12 | 10 | 10% | 61 |
| ES492 | 108 | 2 | DPL11 | 15 | 15% | 76 |
| mAb216 | 89 | 2 | DPL12 | 1 | 1% | 7 |
| BSA3 | 109 | 3 | DPL16 | 0 | 0% | 49 |
| THY-29 | 110 | 3 | DPL16 | 0 | 0% | 27 |
| PR-TS2 | 108 | 3 | DPL16 | 0 | 0% | 55 |
| E29.1 LAMBDA | 107 | 3 | DPL16 | 1 | 1% | 13 |
| mAb63 | 109 | 3 | DPL16 | 2 | 2% | 29 |
| TEL14 | 110 | 3 | DPL16 | 6 | 6% | 49 |
| 6H-3C4 | 108 | 3 | DPL16 | 7 | 7% | 39 |
| SH | 109 | 3 | DPL16 | 7 | 7% | 70 |
| AL GIL | 109 | 3 | DPL16 | 8 | 8% | 23 |
| H6-3C4 | 108 | 3 | DPL16 | 8 | 8% | 83 |
| V-lambda-2.DS | 111 | 2 | DPL11 | 3 | 3% | 15 |
| 8.12 ID | 110 | 2 | DPL11 | 3 | 3% | 81 |
| DSC | 111 | 2 | DPL11 | 3 | 3% | 56 |
| PV11 | 110 | 2 | DPL11 | 1 | 1% | 56 |
| 33.H11 | 110 | 2 | DPL11 | 4 | 4% | 81 |
| AS17 | 111 | 2 | DPL11 | 7 | 7% | 56 |
| SD6 | 110 | 2 | DPL11 | 7 | 7% | 56 |
| KS3 | 110 | 2 | DPL11 | 9 | 9% | 56 |
| V6 | 110 | 2 | DPL12 | 5 | 5% | 56 |
| NGD9 | 110 | 2 | DPL11 | 7 | 7% | 56 |
| MUC1-1 | 111 | 2 | DPL11 | 11 | 10% | 27 |
| A30c | 111 | 2 | DPL10 | 6 | 6% | 56 |
| KS6 | 110 | 2 | DPL12 | 6 | 6% | 56 |
| TEL13 | 111 | 2 | DPL11 | 11 | 10% | 49 |
| AS7 | 110 | 2 | DPL12 | 6 | 6% | 56 |
| MCG | 112 | 2 | DPL12 | 12 | 11% | 20 |
| U266L | 110 | 2 | DPL12 | 13 | 12% | 77 |
| PR-SJ2 | 110 | 2 | DPL12 | 14 | 13% | 55 |
| BOH | 112 | 2 | DPL12 | 11 | 10% | 37 |
| TOG | 111 | 2 | DPL11 | 19 | 18% | 53 |
| TEL16 | 111 | 2 | DPL11 | 19 | 18% | 49 |
| No.13 | 110 | 2 | DPL10 | 14 | 13% | 52 |
| BO | 112 | 2 | DPL12 | 18 | 17% | 80 |
| WIN | 112 | 2 | DPL12 | 17 | 16% | 11 |
| BUR | 104 | 2 | DPL12 | 15 | 15% | 46 |
| NIG-58 | 110 | 2 | DPL12 | 20 | 19% | 69 |
| EIR | 112 | 2 | DPL11 | 26 | 25% | 21 |
| THY-32 | 111 | 1 | DPL8 | 8 | 8% | 27 |
| TNF-H9G1 | 111 | 1 | DPL8 | 9 | 9% | 27 |
| mAb61 | 111 | 1 | DPL3 | 1 | 1% | 29 |
| LV1L1 | 98 | 1 | DPL2 | 0 | 0% | 54 |
| HA | 113 | 1 | DPL3 | 14 | 13% | 63 |
| LA1L1 | 111 | 1 | DPL2 | 3 | 3% | 54 |
| RHE | 112 | 1 | DPL1 | 17 | 16% | 22 |
| K1B12L | 113 | 1 | DPL8 | 17 | 16% | 79 |
| LOC | 113 | 1 | DPL2 | 15 | 14% | 84 |
| NIG-51 | 112 | 1 | DPL2 | 12 | 11% | 67 |
| NEWM | 104 | 1 | DPL8 | 23 | 22% | 10 |
| MD3-4 | 106 | 3 | DPL23 | 14 | 13% | 4 |
| COX | 112 | 1 | DPL2 | 13 | 12% | 84 |
| HiH10 | 106 | 3 | DPL23 | 13 | 12% | 3 |
| VOR | 112 | 1 | DPL2 | 16 | 15% | 16 |
| AL POL | 113 | 1 | DPL2 | 16 | 15% | 57 |
| CD4-74 | 111 | 1 | DPL2 | 19 | 18% | 27 |
| AMYLOID MOL | 102 | 3 | DPL23 | 15 | 15% | 30 |
| OST577 | 108 | 3 | Humlv318 | 10 | 10% | 4 |
| NIG-48 | 113 | 1 | DPL3 | 42 | 40% | 66 |
| CARR | 108 | 3 | DPL23 | 18 | 17% | 19 |
| mAb60 | 108 | 3 | DPL23 | 14 | 13% | 29 |
| NIG-68 | 99 | 3 | DPL23 | 25 | 26% | 32 |
| KERN | 107 | 3 | DPL23 | 26 | 25% | 59 |
| ANT | 106 | 3 | DPL23 | 17 | 16% | 19 |
| LEE | 110 | 3 | DPL23 | 18 | 17% | 85 |
| CLE | 94 | 3 | DPL23 | 17 | 17% | 19 |
| VL8 | 98 | 8 | DPL21 | 0 | 0% | 81 |
| MOT | 110 | 3 | Humlv318 | 23 | 22% | 38 |
| GAR | 108 | 3 | DPL23 | 26 | 25% | 33 |
| 32.B9 | 98 | 8 | DPL21 | 5 | 5% | 81 |
| UG | 108 | 3 | Humlv318 | 24 | 23% | 19 |
| T1 | 115 | 8 | HUMLV801 | 52 | 50% | 6 |
| RF-TS7 | 96 | 7 | DPL18 | 4 | 4% | 60 |
| YM-1 | 116 | 8 | HUMLV801 | 51 | 49% | 75 |
| K6H6 | 112 | 8 | HUMLV801 | 20 | 19% | 44 |
| K5C7 | 112 | 8 | HUMLV801 | 20 | 19% | 44 |
| K5B8 | 112 | 8 | HUMLV801 | 20 | 19% | 44 |
| K5G5 | 112 | 8 | HUMLV801 | 20 | 19% | 44 |
| K4B8 | 112 | 8 | HUMLV801 | 19 | 18% | 44 |
| K6F5 | 112 | 8 | HUMLV801 | 17 | 16% | 44 |
| HIL | 108 | 3 | DPL23 | 22 | 21% | 47 |
| KIR | 109 | 3 | DPL23 | 20 | 19% | 19 |
| CAP | 109 | 3 | DPL23 | 19 | 18% | 84 |
| IB8 | 110 | 3 | DPL23 | 22 | 21% | 43 |
| SHO | 108 | 3 | DPL23 | 19 | 18% | 19 |
| HAN | 108 | 3 | DPL23 | 20 | 19% | 19 |
| cML23 | 96 | 3 | DPL23 | 3 | 3% | 12 |
| PR-SJ1 | 96 | 3 | DPL23 | 7 | 7% | 55 |
| AU | 107 | 3 | DPL23 | 9 | 9% | 5 |
| TEX | 99 | 3 | DPL23 | 8 | 8% | 19 |
| X(PET) | 107 | 3 | DPL23 | 9 | 9% | 51 |
| DOY | 106 | 3 | DPL23 | 9 | 9% | 19 |
| COT | 106 | 3 | DPL23 | 13 | 12% | 19 |
| Pag-1 | 111 | 3 | Humlv318 | 5 | 5% | 31 |
| DIS | 107 | 3 | Humlv318 | 2 | 2% | 19 |
| WIT | 108 | 3 | Humlv318 | 7 | 7% | 19 |
| I.RH | 108 | 3 | Humlv318 | 12 | 11% | 19 |
| S1-1 | 108 | 3 | Humlv318 | 12 | 11% | 52 |
| DEL | 108 | 3 | Humlv318 | 14 | 13% | 17 |
| TYR | 108 | 3 | Humlv318 | 11 | 10% | 19 |
| J.RH | 109 | 3 | Humlv318 | 13 | 12% | 19 |
| THO | 112 | 2 | DPL13 | 38 | 36% | 26 |
| LBV | 113 | 1 | DPL3 | 38 | 36% | 2 |
| WLT | 112 | 1 | DPL3 | 33 | 31% | 14 |
| SUT | 112 | 2 | DPL12 | 37 | 35% | 65 |
| 21/28 | 119 | 1 | VH1-13-12 | 0 | 0,0% | 31 |
| 8E10 | 123 | 1 | VH1-13-12 | 0 | 0,0% | 31 |
| MUC1-1 | 118 | 1 | VH1-13-6 | 4 | 4,1% | 42 |
| gF1 | 98 | 1 | VH1-13-12 | 10 | 10,2% | 75 |
| VHGL1.2 | 98 | 1 | VH1-13-6 | 2 | 2,0% | 26 |
| HV1L1 | 98 | 1 | VH1-13-6 | 0 | 0,0% | 81 |
| RF-TS7 | 104 | 1 | VH1-13-6 | 3 | 3,1% | 96 |
| E55 1.A15 | 106 | 1 | VH1-13-15 | 1 | 1,0% | 26 |
| HA1L1 | 126 | 1 | VH1-13-6 | 7 | 7,1% | 81 |
| UC | 123 | 1 | VH1-13-6 | 5 | 5,1% | 115 |
| WIL2 | 123 | 1 | VH1-13-6 | 6 | 6,1% | 55 |
| R3.5H5G | 122 | 1 | VH1-13-6 | 10 | 10,2% | 70 |
| N89P2 | 123 | 1 | VH1-13-16 | 11 | 11,2% | 77 |
| mAb113 | 126 | 1 | VH1-13-6 | 10 | 10,2% | 71 |
| LS2S3-3 | 125 | 1 | VH1-12-7 | 5 | 5,1% | 98 |
| LS2S3-12a | 125 | 1 | VH1-12-7 | 5 | 5,1% | 98 |
| LS2S3-5 | 125 | 1 | VH1-12-7 | 5 | 5,1% | 98 |
| LS2S3-12e | 125 | 1 | VH1-12-7 | 5 | 5,1% | 98 |
| LS2S3-4 | 125 | 1 | VH1-12-7 | 5 | 5,1% | 98 |
| LS2S3-10 | 125 | 1 | VH1-12-7 | 5 | 5,1% | 98 |
| LS2S3-12d | 125 | 1 | VH1-12-7 | 6 | 6,1% | 98 |
| LS2S3-8 | 125 | 1 | VH1-12-7 | 5 | 5,1% | 98 |
| LS2 | 125 | 1 | VH1-12-7 | 6 | 6,1% | 113 |
| LS4 | 105 | 1 | VH1-12-7 | 6 | 6,1% | 113 |
| LS5 | 125 | 1 | VH1-12-7 | 6 | 6,1% | 113 |
| LS1 | 125 | 1 | VH1-12-7 | 6 | 6,1% | 113 |
| LS6 | 125 | 1 | VH1-12-7 | 6 | 6,1% | 113 |
| LS8 | 125 | 1 | VH1-12-7 | 7 | 7,1% | 113 |
| THY-29 | 122 | 1 | VH1-12-7 | 0 | 0,0% | 42 |
| TB9/F2 | 122 | 1 | VH1-12-7 | 10 | 10,2% | 21 |
| 51P1 | 122 | 1 | VH1-12-1 | 0 | 0,0% | 105 |
| NEI | 127 | 1 | VH1-12-1 | 0 | 0,0% | 55 |
| AND | 127 | 1 | VH1-12-1 | 0 | 0,0% | 55 |
| L7 | 127 | 1 | VH1-12-1 | 0 | 0,0% | 54 |
| L22 | 124 | 1 | VH1-12-1 | 0 | 0,0% | 54 |
| L24 | 127 | 1 | VH1-12-1 | 0 | 0,0% | 54 |
| L26 | 116 | 1 | VH1-12-1 | 0 | 0,0% | 54 |
| L33 | 119 | 1 | VH1-12-1 | 0 | 0,0% | 54 |
| L34 | 117 | 1 | VH1-12-1 | 0 | 0,0% | 54 |
| L36 | 118 | 1 | VH1-12-1 | 0 | 0,0% | 54 |
| L39 | 120 | 1 | VH1-12-1 | 0 | 0,0% | 54 |
| L41 | 120 | 1 | VH1-12-1 | 0 | 0,0% | 54 |
| L42 | 125 | 1 | VH1-12-1 | 0 | 0,0% | 54 |
| VHGL 1.8 | 101 | 1 | VH1-12-1 | 0 | 0,0% | 26 |
| 783c | 127 | 1 | VH1-12-1 | 0 | 0,0% | 22 |
| X17115 | 127 | 1 | VH1-12-1 | 0 | 0,0% | 37 |
| L25 | 124 | 1 | VH1-12-1 | 0 | 0,0% | 54 |
| L17 | 120 | 1 | VH1-12-1 | 1 | 1,0% | 54 |
| L30 | 127 | 1 | VH1-12-1 | 1 | 1,0% | 54 |
| L37 | 120 | 1 | VH1-12-1 | 1 | 1,0% | 54 |
| TNF-E7 | 116 | 1 | VH1-12-1 | 2 | 2,0% | 42 |
| mAb111 | 122 | 1 | VH1-12-1 | 7 | 7,1% | 71 |
| III-2R | 122 | 1 | VH1-12-9 | 3 | 3,1% | 70 |
| KAS | 121 | 1 | VH1-12-1 | 7 | 7,1% | 79 |
| YES8c | 122 | 1 | VH1-12-1 | 8 | 8,2% | 34 |
| RF-TS1 | 123 | 1 | VH1-12-1 | 8 | 8,2% | 82 |
| BOR' | 121 | 1 | VH1-12-8 | 7 | 7,1% | 79 |
| VHGL1.9 | 101 | 1 | VH1-12-1 | 8 | 8,2% | 26 |
| mAb410.30F305 | 117 | 1 | VH1-12-9 | 5 | 5,1% | 52 |
| EV1-15 | 127 | 1 | VH1-12-8 | 10 | 10,2% | 78 |
| mAb112 | 122 | 1 | VH1-12-1 | 11 | 11,2% | 71 |
| U | 117 | 1 | VH1-12-1 | 11 | 11,2% | 28 |
| H210 | 127 | 1 | VH1-12-1 | 12 | 12,2% | 66 |
| TRANSGENE | 104 | 1 | VH1-12-1 | 0 | 0,0% | 111 |
| CLL2-1 | 93 | 1 | VH1-12-1 | 0 | 0,0% | 30 |
| CLL10 13-3 | 97 | 1 | VH1-12-1 | 0 | 0,0% | 29 |
| LS7 | 99 | 1 | VH1-12-7 | 4 | 4,1% | 113 |
| ALL7-1 | 87 | 1 | VH1-12-7 | 0 | 0,0% | 30 |
| CLL3-1 | 91 | 1 | VH1-12-7 | 1 | 1,0% | 30 |
| ALL56-1 | 85 | 1 | VH1-13-8 | 0 | 0,0% | 30 |
| ALL1-1 | 87 | 1 | VH1-13-6 | 1 | 1,0% | 30 |
| ALL4-1 | 94 | 1 | VH1-13-8 | 0 | 0,0% | 30 |
| ALL5615-4 | 85 | 1 | VH1-13-8 | 5 | 5,1% | 29 |
| CLL4-1 | 88 | 1 | VH1-13-1 | 1 | 1,0% | 30 |
| Au92.1 | 98 | 1 | VH1-12-5 | 0 | 0,0% | 49 |
| RF-TS3 | 120 | 1 | VH1-12-5 | 1 | 1,0% | 82 |
| Au4.1 | 98 | 1 | VH1-12-5 | 1 | 1,0% | 49 |
| HP1 | 121 | 1 | VH1-13-6 | 13 | 13,3% | 110 |
| RL1 | 127 | 1 | VH1-13-15 | 5 | 5,1% | 72 |
| No.13 | 127 | 1 | VH1-12-2 | 19 | 19,4% | 76 |
| TR1.23 | 122 | 1 | VH1-13-2 | 23 | 23,5% | 88 |
| S1-1 | 125 | 1 | VH1-12-2 | 18 | 18,4% | 76 |
| TR1.10 | 119 | 1 | VH1-13-12 | 14 | 14,3% | 88 |
| E55 1.A2 | 102 | 1 | VH1-13-15 | 3 | 3,1% | 26 |
| SP2 | 119 | 1 | VH1-13-6 | 15 | 15,3% | 89 |
| TNF-H9G1 | 111 | 1 | VH1-13-18 | 2 | 2,0% | 42 |
| G3D10H | 127 | 1 | VH1-13-16 | 19 | 19,4% | 127 |
| TR1.9 | 118 | 1 | VH1-13-12 | 14 | 14,3% | 88 |
| TR1.8 | 121 | 1 | VH1-12-1 | 24 | 24,5% | 88 |
| LUNm01 | 127 | 1 | VH1-13-6 | 22 | 22,4% | 9 |
| K1B12H | 127 | 1 | VH1-12-7 | 23 | 23,5% | 127 |
| L3B2 | 99 | 1 | VH1-13-6 | 2 | 2,0% | 46 |
| ss2 | 100 | 1 | VH1-13-6 | 2 | 2,0% | 46 |
| No.86 | 124 | 1 | VH1-12-1 | 20 | 20,4% | 76 |
| TR1.6 | 124 | 1 | VH1-12-1 | 19 | 19,4% | 88 |
| ss7 | 99 | 1 | VH1-12-7 | 3 | 3,1% | 46 |
| s5B7 | 102 | 1 | VH1-12-1 | 0 | 0,0% | 46 |
| s6A3 | 97 | 1 | VH1-12-1 | 0 | 0,0% | 46 |
| ss6 | 99 | 1 | VH1-12-1 | 0 | 0,0% | 46 |
| L2H7 | 103 | 1 | VH1-13-12 | 0 | 0,0% | 46 |
| s6BG8 | 93 | 1 | VH1-13-12 | 0 | 0,0% | 46 |
| s6C9 | 107 | 1 | VH1-13-12 | 0 | 0,0% | 46 |
| HIV-b4 | 124 | 1 | VH1-13-12 | 21 | 21,4% | 12 |
| HIV-b12 | 124 | 1 | VH1-13-12 | 21 | 21,4% | 12 |
| L3G5 | 98 | I | VH1-13-6 | 1 | 1,0% | 46 |
| 22 | 115 | 1 | VH1-13-6 | 11 | 11,2% | 118 |
| L2A12 | 99 | 1 | VH1-13-15 | 3 | 3,1% | 46 |
| PHOX15 | 124 | 1 | VH1-12-7 | 20 | 20,4% | 73 |
| LUNm03 | 127 | 1 | VH1-1X-1 | 18 | 18,4% | 9 |
| CEA4-8A | 129 | 1 | VH1-12-7 | 1 | 1,0% | 42 |
| M60 | 121 | 2 | VH2-31-3 | 3 | 3,0% | 103 |
| HiH10 | 127 | 2 | VH2-31-5 | 9 | 9,0% | 4 |
| COR | 119 | 2 | VH2-31-2 | 11 | 11,0% | 91 |
| 2-115-19 | 124 | 2 | VH2-31-11 | 8 | 8,1% | 124 |
| OU | 125 | 2 | VH2-31-14 | 20 | 25,6% | 92 |
| HE | 120 | 2 | VH2-31-13 | 19 | 19,0% | 27 |
| CLL33 40-1 | 78 | 2 | VH2-31-5 | 2 | 2,0% | 29 |
| E55 3.9 | 88 | 3 | VH3-11-5 | 7 | 7,2% | 26 |
| MTFC3 | 125 | 3 | VH3-14-4 | 21 | 21,0% | 131 |
| MTFC11 | 125 | 3 | VH3-14-4 | 21 | 21,0% | 131 |
| MTFJ1 | 114 | 3 | VH3-14-4 | 21 | 21,0% | 131 |
| MTFJ2 | 114 | 3 | VH3-14-4 | 21 | 21,0% | 131 |
| MTFUJ4 | 100 | 3 | VH3-14-4 | 21 | 21,0% | 131 |
| MTFUJ5 | 100 | 3 | VH3-14-4 | 21 | 21,0% | 131 |
| MTFUJ2 | 100 | 3 | VH3-14-4 | 22 | 22,0% | 131 |
| MTFC8 | 125 | 3 | VH3-14-4 | 23 | 23,0% | 131 |
| TD e Vq | 113 | 3 | VH3-14-4 | 0 | 0,0% | 16 |
| rMTF | 114 | 3 | VH3-14-4 | 5 | 5,0% | 131 |
| MTFUJ6 | 100 | 3 | VH3-14-4 | 10 | 10,0% | 131 |
| RF-KES | 107 | 3 | VH3-14-4 | 9 | 9,0% | 85 |
| N51P8 | 126 | 3 | VH3-14-1 | 9 | 9,0% | 77 |
| TEI | 119 | 3 | VH3-13-8 | 21 | 21,4% | 20 |
| 33.H11 | 115 | 3 | VH3-13-19 | 10 | 10,2% | 129 |
| SB1/D8 | 101 | 3 | VH3-1X-8 | 14 | 14,0% | 2 |
| 38P1 | 119 | 3 | VH3-11-3 | 0 | 0,0% | 104 |
| BRO'IGM | 119 | 3 | VH3-11-3 | 13 | 13,4% | 19 |
| NIE | 119 | 3 | VH3-13-7 | 15 | 15,3% | 87 |
| 3D6 | 126 | 3 | VH3-13-26 | 5 | 5,1% | 35 |
| ZM1-1 | 112 | 3 | VH3-11-3 | 8 | 8,2% | 5 |
| E55 3.15 | 110 | 3 | VH3-13-26 | 0 | 0,0% | 26 |
| gF9 | 108 | 3 | VH3-13-8 | 15 | 15,3% | 75 |
| THY-32 | 120 | 3 | VH3-13-26 | 3 | 3,1% | 42 |
| RF-KL5 | 100 | 3 | VH3-13-26 | 5 | 5,1% | 96 |
| OST577 | 122 | 3 | VH3-13-13 | 6 | 6,1% | 5 |
| BO | 113 | 3 | VH3-13-19 | 15 | 15,3% | 10 |
| TT125 | 121 | 3 | VH3-13-10 | 15 | 15,3% | 64 |
| 2-115-58 | 127 | 3 | VH3-13-10 | 11 | 11,2% | 124 |
| KOL | 126 | 3 | VH3-13-14 | 16 | 16,3% | 102 |
| mAb60 | 118 | 3 | VH3-13-17 | 14 | 14,3% | 45 |
| RF-AN | 106 | 3 | VH3-13-26 | 8 | 8,2% | 85 |
| BUT | 115 | 3 | VH3-11-6 | 13 | 13,4% | 119 |
| KOL-based | | | | | | |
| CAMPATH-9 | 118 | 3 | VH3-13-13 | 16 | 16,3% | 41 |
| B1 | 119 | 3 | VH3-13-19 | 13 | 13,3% | 53 |
| N98P1 | 127 | 3 | VH3-13-1 | 13 | 13,3% | 77 |
| TT117 | 107 | 3 | VH3-13-10 | 12 | 12,2% | 64 |
| WEA | 114 | 3 | VH3-13-12 | 15 | 15,3% | 40 |
| HIL | 120 | 3 | VH3-13-14 | 14 | 14,3% | 23 |
| s5A10 | 97 | 3 | VH3-13-14 | 0 | 0,0% | 46 |
| s5D11 | 98 | 3 | VH3-13-7 | 0 | 0,0% | 46 |
| s6C8 | 100 | 3 | VH3-13-7 | 0 | 0,0% | 46 |
| 6H12 | 98 | 3 | VH3-13-7 | 0 | 0,0% | 46 |
| VH10.7 | 119 | 3 | VH3-13-14 | 16 | 16,3% | 128 |
| HIV-loop2 | 126 | 3 | VH3-13-7 | 16 | 16,3% | 12 |
| HIV-loop35 | 126 | 3 | VH3-13-7 | 16 | 16.3% | 12 |
| TRO | 122 | 3 | VH3-13-1 | 13 | 13,3% | 61 |
| SA-4B | 123 | 3 | VH3-13-1 | 15 | 15,3% | 125 |
| L2B5 | 98 | 3 | VH3-13-13 | 0 | 0,0% | 46 |
| s6E11 | 95 | 3 | VH3-13-13 | 0 | 0,0% | 46 |
| s6H7 | 100 | 3 | VH3-13-13 | 0 | 0,0% | 46 |
| ss1 | 102 | 3 | VH3-13-13 | 0 | 0,0% | 46 |
| ss8 | 94 | 3 | VH3-13-13 | 0 | 0,0% | 46 |
| DOB | 120 | 3 | VH3-13-26 | 21 | 21,4% | 116 |
| THY-33 | 115 | 3 | VH3-13-15 | 20 | 20,4% | 42 |
| NOV | 118 | 3 | VH3-13-19 | 14 | 14,3% | 38 |
| rsv13H | 120 | 3 | VH3-13-24 | 20 | 20,4% | 11 |
| L3G11 | 98 | 3 | VH3-13-20 | 2 | 2,0% | 46 |
| L2E8 | 99 | 3 | VH3-13-19 | 0 | 0,0% | 46 |
| L2D10 | 101 | 3 | VH3-13-10 | 1 | 1,0% | 46 |
| L2E7 | 98 | 3 | VH3-13-10 | 1 | 1,0% | 46 |
| L3A10 | 100 | 3 | VH3-13-24 | 0 | 0,0% | 46 |
| L2E5 | 97 | 3 | VH3-13-2 | 1 | 1,0% | 46 |
| BUR | 119 | 3 | VH3-13-7 | 21 | 21,4% | 67 |
| s4D5 | 107 | 3 | VH3-11-3 | 1 | 1,0% | 46 |
| 19 | 116 | 3 | VH3-13-16 | 4 | 4,1% | 118 |
| s5D4 | 99 | 3 | VH3-13-1 | 0 | 0,0% | 46 |
| s6A8 | 100 | 3 | VH3-13-1 | 0 | 0,0% | 46 |
| HIV-loop13 | 123 | 3 | VH3-13-12 | 17 | 17,3% | 12 |
| TR1.32 | 112 | 3 | VH3-11-8 | 18 | 18,6% | 88 |
| L2B10 | 97 | 3 | VH3-11-3 | 1 | 1,0% | 46 |
| TR1.5 | 114 | 3 | VH3-11-8 | 21 | 21,6% | 88 |
| s6H9 | 101 | 3 | VH3-13-25 | 0 | 0,0% | 46 |
| 8 | 112 | 3 | VH3-13-1 | 6 | 6,1% | 118 |
| 23 | 115 | 3 | VH3-13-1 | 6 | 6,1% | 118 |
| 7 | 115 | 3 | VH3-13-1 | 4 | 4,1% | 118 |
| TR1.3 | 120 | 3 | VH3-11-8 | 20 | 20,6% | 88 |
| 18/2 | 125 | 3 | VH3-13-10 | 0 | 0,0% | 32 |
| 18/9 | 125 | 3 | VH3-13-10 | 0 | 0,0% | 31 |
| 30P1 | 119 | 3 | VH3-13-10 | 0 | 0,0% | 106 |
| HF2-1/17 | 125 | 3 | VH3-13-10 | 0 | 0,0% | 8 |
| A77 | 109 | 3 | VH3-13-10 | 0 | 0,0% | 44 |
| B19.7 | 108 | 3 | VH3-13-10 | 0 | 0,0% | 44 |
| M43 | 119 | 3 | VH3-13-10 | 0 | 0,0% | 103 |
| 1/17 | 125 | 3 | VH3-13-10 | 0 | 0,0% | 31 |
| 18/17 | 125 | 3 | VH3-13-10 | 0 | 0,0% | 31 |
| E54 3.4 | 109 | 3 | VH3-13-10 | 0 | 0,0% | 26 |
| LAMBDA-VH26 | 98 | 3 | VH3-13-10 | 1 | 1,0% | 95 |
| E54 3.8 | 111 | 3 | VH3-13-10 | 1 | 1,0% | 26 |
| GL16 | 106 | 3 | VH3-13-10 | 1 | 1,0% | 44 |
| 4G12 | 125 | 3 | VH3-13-10 | 1 | 1,0% | 56 |
| A73 | 106 | 3 | VH3-13-10 | 2 | 2,0% | 44 |
| AL1.3 | 111 | 3 | VH3-13-10 | 3 | 3,1% | 117 |
| 3.A290 | 118 | 3 | VH3-13-10 | 2 | 2,0% | 108 |
| Ab18 | 127 | 3 | VH3-13-8 | 2 | 2,0% | 100 |
| E54 3.3 | 105 | 3 | VH3-13-10 | 3 | 3,1% | 26 |
| 35G6 | 121 | 3 | VH3-13-10 | 3 | 3,1% | 57 |
| A95 | 107 | 3 | VH3-13-10 | 5 | 5,1% | 44 |
| Ab25 | 128 | 3 | VH3-13-10 | 5 | 5,1% | 100 |
| N87 | 126 | 3 | VH3-13-10 | 4 | 4,1% | 77 |
| ED8.4 | 99 | 3 | VH3-13-10 | 6 | 6,1% | 2 |
| RF-KL1 | 122 | 3 | VH3-13-10 | 6 | 6,1% | 82 |
| AL1.1 | 112 | 3 | VH3-13-10 | 2 | 2,0% | 117 |
| AL3.11 | 102 | 3 | VH3-13-10 | 1 | 1,0% | 117 |
| 32.B9 | 127 | 3 | VH3-13-8 | 6 | 6,1% | 129 |
| TK1 | 109 | 3 | VH3-13-10 | 2 | 2,0% | 117 |
| POP | 123 | 3 | VH3-13-10 | 8 | 8,2% | 115 |
| 9F2H | 127 | 3 | VH3-13-10 | 9 | 9,2% | 127 |
| VD | 115 | 3 | VH3-13-10 | 9 | 9,2% | 10 |
| Vh38C1.10 | 121 | 3 | VH3-13-10 | 8 | 8,2% | 74 |
| Vh38C1.9 | 121 | 3 | VH3-13-10 | 8 | 8,2% | 74 |
| Vh38C1.8 | 121 | 3 | VH3-13-10 | 8 | 8,2% | 74 |
| 63P1 | 120 | 3 | VH3-11-8 | 0 | 0,0% | 104 |
| 60P2 | 117 | 3 | VH3-11-8 | 0 | 0,0% | 104 |
| AL3.5 | 90 | 3 | VH3-13-10 | 2 | 2,0% | 117 |
| GF4/1.1 | 123 | 3 | VH3-13-10 | 10 | 10,2% | 39 |
| Ab21 | 126 | 3 | VH3-13-10 | 12 | 12,2% | 100 |
| TDdVp | 118 | 3 | VH3-13-17 | 2 | 2,0% | 16 |
| Vh38C1.4 | 119 | 3 | VH3-13-10 | 8 | 8,2% | 74 |
| Vh38C1.5 | 119 | 3 | VH3-13-10 | 8 | 8,2% | 74 |
| AL3.4 | 104 | 3 | VH3-13-10 | 1 | 1,0% | 117 |
| FOG1-A3 | 115 | 3 | VH3-13-19 | 2 | 2,0% | 42 |
| HA3D1 | 117 | 3 | VH3-13-21 | 1 | 1,0% | 81 |
| E54 3.2 | 112 | 3 | VH3-13-24 | 0 | 0,0% | 26 |
| mAb52 | 128 | 3 | VH3-13-12 | 2 | 2,0% | 51 |
| mAb53 | 128 | 3 | VH3-13-12 | 2 | 2,0% | 51 |
| mAb56 | 128 | 3 | VH3-13-12 | 2 | 2,0% | 51 |
| mAb57 | 128 | 3 | VH3-13-12 | 2 | 2,0% | 51 |
| mAb58 | 128 | 3 | VH3-13-12 | 2 | 2,0% | 51 |
| mAb59 | 128 | 3 | VH3-13-12 | 2 | 2,0% | 51 |
| mAb105 | 128 | 3 | VH3-13-12 | 2 | 2,0% | 51 |
| mAb107 | 128 | 3 | VH3-13-12 | 2 | 2,0% | 51 |
| E55 3.14 | 110 | 3 | VH3-13-19 | 0 | 0,0% | 26 |
| F13-28 | 106 | 3 | VH3-13-19 | 1 | 1,0% | 94 |
| mAb55 | 127 | 3 | VH3-13-18 | 4 | 4,1% | 51 |
| YSE | 117 | 3 | VH3-13-24 | 6 | 6,1% | 72 |
| E55 3.23 | 106 | 3 | VH3-13-19 | 2 | 2,0% | 26 |
| RF-TS5 | 101 | 3 | VH3-13-1 | 3 | 3,1% | 85 |
| N42P5 | 124 | 3 | VH3-13-2 | 7 | 7,1% | 77 |
| FOG1-H6 | 110 | 3 | VH3-13-16 | 7 | 7,1% | 42 |
| O-81 | 115 | 3 | VH3-13-19 | 11 | 11,2% | 47 |
| HIV-s8 | 122 | 3 | VH3-13-12 | 11 | 11,2% | 12 |
| nAb114 | 125 | 3 | VH3-13-19 | 12 | 12,2% | 71 |
| 33.F12 | 116 | 3 | VH3-13-2 | 4 | 4,1% | 129 |
| 4B4 | 119 | 3 | VH3-1X-3 | 0 | 0,0% | 101 |
| M26 | 123 | 3 | VH3-1X-3 | 0 | 0,0% | 103 |
| VHGL 3.1 | 100 | 3 | VH3-1X-3 | 0 | 0,0% | 26 |
| E55 3.13 | 113 | 3 | VH3-1X-3 | 1 | 1,0% | 26 |
| SB5/D6 | 101 | 3 | VH3-1X-6 | 3 | 3,0% | 2 |
| RAY4 | 101 | 3 | VH3-1X-6 | 3 | 3,0% | 2 |
| 82-D V-D | 106 | 3 | VH3-1X-3 | 5 | 5,0% | 112 |
| MAL | 129 | 3 | VH3-1X-3 | 5 | 5,0% | 72 |
| LOC | 123 | 3 | VH3-1X-6 | 5 | 5,0% | 72 |
| LSF2 | 101 | 3 | VH3-1X-6 | 11 | 11,0% | 2 |
| HIBRC3 | 100 | 3 | VH3-1X-6 | 11 | 11,0% | 1 |
| 56P1 | 119 | 3 | VH3-13-7 | 0 | 0,0% | 104 |
| M72 | 122 | 3 | VH3-13-7 | 0 | 0,0% | 103 |
| M74 | 121 | 3 | VH3-13-7 | 0 | 0,0% | 103 |
| E54 3.5 | 105 | 3 | VH3-13-7 | 0 | 0,0% | 26 |
| E7 | 123 | 3 | VH3-13-7 | 0 | 0,0% | 63 |
| 2P1 | 117 | 3 | VH3-13-7 | 0 | 0,0% | 104 |
| RF-SJ2 | 127 | 3 | VH3-13-7 | 1 | 1,0% | 83 |
| PR-TS1 | 114 | 3 | VH3-13-7 | 1 | 1,0% | 85 |
| KIM46H | 127 | 3 | VH3-13-13 | 0 | 0,0% | 18 |
| E55 3.6 108 | 108 | 3 | VH3-13-7 | 2 | 2,0% | 26 |
| E55 3.10 | 107 | 3 | VH3-13-13 | 1 | 1,0% | 26 |
| 3.B6 | 114 | 3 | VH3-13-13 | 1 | 1,0% | 108 |
| E54 3.6 | 110 | 3 | VH3-13-13 | 1 | 1,0% | 26 |
| FL2-2 | 114 | 3 | VH3-13-13 | 1 | 1,0% | 80 |
| RF-SJ3 | 112 | 3 | VH3-13-7 | 2 | 2,0% | 85 |
| E55 3.5 | 105 | 3 | VH3-13-14 | 1 | 1,0% | 26 |
| BSA3 | 121 | 3 | VH3-13-13 | 1 | 1,0% | 73 |
| HMST-1 | 119 | 3 | VH3-13-7 | 3 | 3,1% | 130 |
| RF-TS2 | 126 | 3 | VH3-13-13 | 4 | 4,1% | 82 |
| E55 3.12 | 109 | 3 | VH3-13-15 | 0 | 0,0% | 26 |
| 19.E7 | 126 | 3 | VH3-13-14 | 3 | 3,1% | 129 |
| 11-50 | 119 | 3 | VH3-13-13 | 6 | 6,1% | 130 |
| E29.1 | 120 | 3 | VH3-13-15 | 2 | 2,0% | 25 |
| E55 3.16 | 108 | 3 | VH3-13-7 | 6 | 6,1% | 26 |
| TNF-E1 | 117 | 3 | VH3-13-7 | 7 | 7,1% | 42 |
| RF-SJ1 | 127 | 3 | VH3-13-13 | 6 | 6,1% | 83 |
| FOG1-A4 | 116 | 3 | VH3-13-7 | 8 | 8,2% | 42 |
| TNF-A1 | 117 | 3 | VH3-13-15 | 4 | 4,1% | 42 |
| PR-SJ2 | 107 | 3 | VH3-13-14 | 8 | 8,2% | 85 |
| HN.14 | 124 | 3 | VH3-13-13 | 10 | 10,2% | 33 |
| CAM' | 121 | 3 | VH3-13-7 | 12 | 12,2% | 65 |
| HIV-B8 | 125 | 3 | VH3-13-7 | 9 | 9,2% | 12 |
| HIV-b27 | 125 | 3 | VH3-13-7 | 9 | 9,2% | 12 |
| HIV-b8 | 125 | 3 | VH3-13-7 | 9 | 9,2% | 12 |
| HIV-s4 | 125 | 3 | VH3-13-7 | 9 | 9,2% | 12 |
| HIV-B26 | 125 | 3 | VH3-13-7 | 9 | 9,2% | 12 |
| HIV-B35 | 125 | 3 | VH3-13-7 | 10 | 10,2% | 12 |
| HIV-b18 | 125 | 3 | VH3-13-7 | 10 | 10,2% | 12 |
| HIV-b22 | 125 | 3 | VH3-13-7 | 11 | 11,2% | 12 |
| HIV-b13 | 125 | 3 | VH3-13-7 | 12 | 12,2% | 12 |
| 333 | 117 | 3 | VH3-14-4 | 24 | 24,0% | 24 |
| 1H1 | 120 | 3 | VH3-14-4 | 24 | 24,0% | 24 |
| 1B11 | 120 | 3 | VH3-14-4 | 23 | 23,0% | 24 |
| CLL30 2-3 | 86 | 3 | VH3-13-19 | 1 | 1,0% | 29 |
| GA | 110 | 3 | VH3-13-7 | 19 | 19,4% | 36 |
| JeB | 99 | 3 | VH3-13-14 | 3 | 3,1% | 7 |
| GAL | 110 | 3 | VH3-13-19 | 10 | 10,2% | 126 |
| K6H6 | 119 | 3 | VH3-1X-6 | 18 | 18,0% | 60 |
| K4B8 | 119 | 3 | VH3-1X-6 | 18 | 18,0% | 60 |
| K5B8 | 119 | 3 | VH3-1X-6 | 18 | 18,0% | 60 |
| K5C7 | 119 | 3 | VH3-1X-6 | 19 | 19,0% | 60 |
| K5G5 | 119 | 3 | VH3-1X-6 | 19 | 19,0% | 60 |
| K6F5 | 119 | 3 | VH3-1X-6 | 19 | 19,0% | 60 |
| AL3.16 | 98 | 3 | VH3-13-10 | 1 | 1,0% | 117 |
| 86P2 | 98 | 3 | VH3-13-10 | 3 | 3,1% | 77 |
| N54P6 | 95 | 3 | VH3-13-16 | 7 | 7,1% | 77 |
| LAMBDA HT112-1 | 126 | 4 | VH4-11-2 | 0 | 0,0% | 3 |
| HY18 | 121 | 4 | VH4-11-2 | 0 | 0,0% | 43 |
| mAb63 | 126 | 4 | VH4-11-2 | 0 | 0,0% | 45 |
| FS-3 | 105 | 4 | VH4-11-2 | 0 | 0,0% | 86 |
| FS-5 | 111 | 4 | VH4-11-2 | 0 | 0,0% | 86 |
| FS-7 | 107 | 4 | VH4-11-2 | 0 | 0,0% | 86 |
| FS-8 | 110 | 4 | VH4-11-2 | 0 | 0,0% | 86 |
| PR-TS2 | 105 | 4 | VH4-11-2 | 0 | 0,0% | 85 |
| RF-TMC | 102 | 4 | VH4-11-2 | 0 | 0,0% | 85 |
| mAb216 | 122 | 4 | VH4-11-2 | 1 | 1,0% | 15 |
| mAb410.7.F91 | 122 | 4 | VH4-11-2 | 1 | 1,0% | 52 |
| mAbA6H4C5 | 124 | 4 | VH4-11-2 | 1 | 1,0% | 15 |
| Ab44 | 127 | 4 | VH4-11-2 | 2 | 2,1% | 100 |
| 6H-3C4 | 124 | 4 | VH4-11-2 | 3 | 3,1% | 59 |
| FS-6 | 108 | 4 | VH4-11-2 | 6 | 6,2% | 86 |
| FS-2 | 114 | 4 | VH4-11-2 | 6 | 6,2% | 84 |
| HIG1 | 126 | 4 | VH4-11-2 | 7 | 7,2% | 62 |
| FS-4 | 105 | 4 | VH4-11-2 | 8 | 8,2% | 86 |
| SA-4A | 123 | 4 | VH4-11-2 | 9 | 9,3% | 125 |
| LES-C | 119 | 4 | VH4-11-2 | 10 | 10,3% | 99 |
| DI | 78 | 4 | VH4-11-9 | 16 | 16,5% | 58 |
| Ab26 | 126 | 4 | VH4-31-4 | 8 | 8,1% | 100 |
| TS2 | 124 | 4 | VH4-31-12 | 15 | 15,2% | 110 |
| 265-695 | 115 | 4 | VH4-11-7 | 16 | 16,5% | 5 |
| WAH | 129 | 4 | VH4-31-13 | 19 | 19,2% | 93 |
| 268-D | 122 | 4 | VH4-11-8 | 22 | 22,7% | 6 |
| 58P2 | 118 | 4 | VH4-11-8 | 0 | 0,0% | 104 |
| mAb67 | 128 | 4 | VH4-21-4 | 1 | 1,0% | 45 |
| 4.L39 | 115 | 4 | VH4-11-8 | 2 | 2,1% | 108 |
| mF7 | 111 | 4 | VH4-31-13 | 3 | 3,0% | 75 |
| 33.C9 | 122 | 4 | VH4-21-5 | 7 | 7,1% | 129 |
| Pag-1 | 124 | 4 | VH4-11-16 | 5 | 5,2% | 50 |
| B3 | 123 | 4 | VH4-21-3 | 8 | 8,2% | 53 |
| IC4 | 120 | 4 | VH4-11-8 | 6 | 6,2% | 70 |
| C6B2 | 127 | 4 | VH4-31-12 | 4 | 4,0% | 48 |
| N78 | 118 | 4 | VH4-11-9 | 11 | 11,3% | 77 |
| B2 | 109 | 4 | VH4-11-8 | 12 | 12,4% | 53 |
| WRD2 | 123 | 4 | VH4-11-12 | 6 | 6,2% | 90 |
| mAb426.4.2F20 | 126 | 4 | VH4-11-8 | 2 | 2,1% | 52 |
| E54 4.58 | 115 | 4 | VH4-11-8 | 1 | 1,0% | 26 |
| WRD6 | 123 | 4 | VH4-11-12 | 10 | 10,3% | 90 |
| mAb426.12.3F1.4 | 122 | 4 | VH4-11-9 | 4 | 4,1% | 52 |
| E54 4.2 | 108 | 4 | VH4-21-6 | 2 | 2,0% | 26 |
| WIL | 127 | 4 | VH4-31-13 | 0 | 0,0% | 90 |
| COF | 126 | 4 | VH4-31-13 | 0 | 0,0% | 90 |
| LAR | 122 | 4 | VH4-31-13 | 2 | 2,0% | 90 |
| WAT | 125 | 4 | VH4-31-13 | 4 | 4,0% | 90 |
| mAb61 | 123 | 4 | VH4-31-13 | 5 | 5,1% | 45 |
| WAG | 127 | 4 | VH4-31-4 | 0 | 0,0% | 90 |
| RF-SJ4 | 108 | 4 | VH4-31-12 | 2 | 2,0% | 85 |
| E54 4.4 | 110 | 4 | VH4-11-7 | 0 | 0,0% | 26 |
| E55 4.A1 | 108 | 4 | VH4-11-7 | 0 | 0,0% | 26 |
| PR-SJ1 | 103 | 4 | VH4-11-7 | 1 | 1,0% | 85 |
| E54 4.23 | 111 | 4 | VH4-11-7 | 1 | 1,0% | 26 |
| CLL7 7-2 | 97 | 4 | VH4-11-12 | 0 | 0,0% | 29 |
| 37P1 | 95 | 4 | VH4-11-12 | 0 | 0,0% | 104 |
| ALL52 30-2 | 91 | 4 | VH4-31-12 | 4 | 4,0% | 29 |
| EBV-21 | 98 | 5 | VH5-12-1 | 0 | 0,0% | 13 |
| CB-4 | 98 | 5 | VH5-12-1 | 0 | 0,0% | 13 |
| CLL-12 | 98 | 5 | VH5-12-1 | 0 | 0,0% | 13 |
| L3-4 | 98 | 5 | VH5-12-1 | 0 | 0,0% | 13 |
| CLL11 | 98 | 5 | VH5-12-1 | 0 | 0,0% | 17 |
| CORD3 | 98 | 5 | VH5-12-1 | 0 | 0,0% | 17 |
| CORD4 | 98 | 5 | VH5-12-1 | 0 | 0,0% | 17 |
| CORD8 | 98 | 5 | VH5-12-1 | 0 | 0,0% | 17 |
| CORD9 | 98 | 5 | VH5-12-1 | 0 | 0,0% | 17 |
| CD+1 | 98 | 5 | VH5-12-1 | 0 | 0,0% | 17 |
| CD+3 | 98 | 5 | VH5-12-1 | 0 | 0,0% | 17 |
| CD+4 | 98 | 5 | VH5-12-1 | 0 | 0,0% | 17 |
| CD-1 | 98 | 5 | VH5-12-1 | 0 | 0,0% | 17 |
| CD-5 | 98 | 5 | VH5-12-1 | 0 | 0,0% | 17 |
| VERG14 | 98 | 5 | VH5-12-1 | 0 | 0,0% | 17 |
| PBL1 | 98 | 5 | VH5-12-1 | 0 | 0,0% | 17 |
| PBL10 | 98 | 5 | VH5-12-1 | 0 | 0,0% | 17 |
| STRAb SA-1A | 127 | 5 | VH5-12-1 | 0 | 0,0% | 125 |
| DOB' | 122 | 5 | VH5-12-1 | 0 | 0,0% | 97 |
| VERG5 | 98 | 5 | VH5-12-1 | 0 | 0,0% | 17 |
| PBL2 | 98 | 5 | VH5-12-1 | 1 | 1,0% | 17 |
| Tu16 | 119 | 5 | VH5-12-1 | 1 | 1,0% | 49 |
| PBL12 | 98 | 5 | VH5-12-1 | 1 | 1,0% | 17 |
| CD+2 | 98 | 5 | VH5-12-1 | 1 | 1,0% | 17 |
| ORD10 | 98 | 5 | VH5-12-1 | 1 | 1,0% | 17 |
| PBL9 | 98 | 5 | VH5-12-1 | 1 | 1,0% | 17 |
| CORD2 | 98 | 5 | VH5-12-1 | 2 | 2,0% | 17 |
| PBL6 | 98 | 5 | VH5-12-1 | 2 | 2,0% | 17 |
| CORD5 | 98 | 5 | VH5-12-1 | 2 | 2,0% | 17 |
| CD-2 | 98 | 5 | VH5-12-1 | 2 | 2,0% | 17 |
| CORD1 | 98 | 5 | VH5-12-1 | 2 | 2,0% | 17 |
| CD-3 | 98 | 5 | VH5-12-1 | 3 | 3,1% | 17 |
| VERG4 | 98 | 5 | VH5-12-1 | 3 | 3,1% | 17 |
| PBL13 | 98 | 5 | VH5-12-1 | 3 | 3,1% | 17 |
| PBL7 | 98 | 5 | VH5-12-1 | 3 | 3,1% | 17 |
| HAN | 119 | 5 | VH5-12-1 | 3 | 3,1% | 97 |
| VERG3 | 98 | 5 | VH5-12-1 | 3 | 3,1% | 17 |
| PBL3 | 98 | 5 | VH5-12-1 | 3 | 3,1% | 17 |
| VERG7 | 98 | 5 | VH5-12-1 | 3 | 3,1% | 17 |
| PBL5 | 94 | 5 | VH5-12-1 | 0 | 0,0% | 17 |
| CD-4 | 98 | 5 | VH5-12-1 | 4 | 4,1% | 17 |
| CLL10 | 98 | 5 | VH5-12-1 | 4 | 4,1% | 17 |
| BL11 | 98 | 5 | VH5-12-1 | 4 | 4,1% | 17 |
| CORD6 | 98 | 5 | VH5-12-1 | 4 | 4,1% | 17 |
| VERG2 | 98 | 5 | VH5-12-1 | 5 | 5,1% | 17 |
| 83P2 | 119 | 5 | VH5-12-1 | 0 | 0,0% | 103 |
| VERG9 | 98 | 5 | VH5-12-1 | 6 | 6,1% | 17 |
| CLL6 | 98 | 5 | VH5-12-1 | 6 | 6,1% | 17 |
| PBL8 | 98 | 5 | VH5-12-1 | 7 | 7,1% | 17 |
| Ab2022 | 120 | 5 | VH5-12-1 | 3 | 3,1% | 100 |
| CAV | 127 | 5 | VH5-12-4 | 0 | 0,0% | 97 |
| HOW | 120 | 5 | VH5-12-4 | 0 | 0,0% | 97 |
| PET | 127 | 5 | VH5-12-4 | 0 | 0,0% | 97 |
| ANG | 121 | 5 | VH5-12-4 | 0 | 0,0% | 97 |
| KER | 121 | 5 | VH5-12-4 | 0 | 0,0% | 97 |
| 5.M13 | 118 | 5 | VH5-12-4 | 0 | 0,0% | 107 |
| Au2.1 | 118 | 5 | VH5-12-4 | 1 | 1,0% | 49 |
| WS1 | 126 | 5 | VH5-12-1 | 9 | 9,2% | 110 |
| TD Vn | 98 | 5 | VH5-12-4 | 1 | 1,0% | 16 |
| TEL13 | 116 | 5 | VH5-12-1 | 9 | 9,2% | 73 |
| E55 5.237 | 112 | 5 | VH5-12-4 | 2 | 2,0% | 26 |
| VERG1 | 98 | 5 | VH5-12-1 | 10 | 10,2% | 17 |
| CD4-74 | 117 | 5 | VH5-12-1 | 10 | 10,2% | 42 |
| 257-D | 125 | 5 | VH5-12-1 | 11 | 11,2% | 6 |
| CLL4 | 98 | 5 | VH5-12-1 | 11 | 11,2% | 17 |
| CLL8 | 98 | 5 | VH5-12-1 | 11 | 11,2% | 17 |
| Ab2 | 124 | 5 | VH5-12-1 | 12 | 12,2% | 120 |
| Vh383ex | 98 | 5 | VH5-12-1 | 12 | 12,2% | 120 |
| CLL3 | 98 | 5 | VH5-12-2 | 11 | 11,2% | 17 |
| Au59.1 | 122 | 5 | VH5-12-1 | 12 | 12,2% | 49 |
| TEL16 | 117 | 5 | VH5-12-1 | 12 | 12,2% | 73 |
| M61 | 104 | 5 | VH5-12-1 | 0 | 0,0% | 103 |
| Tu0 | 99 | 5 | VH5-12-1 | 5 | 5,1% | 49 |
| P2-51 | 122 | 5 | VH5-12-1 | 13 | 13,3% | 121 |
| 2-54 | 122 | 5 | VH5-12-1 | 11 | 11,2% | 121 |
| P1-56 | 119 | 5 | VH5-12-1 | 9 | 9,2% | 121 |
| P2-53 | 122 | 5 | VH5-12-1 | 10 | 10,2% | 121 |
| P1-51 | 123 | 5 | VH5-12-1 | 19 | 19,4% | 121 |
| P1-54 | 123 | 5 | VH5-12-1 | 3 | 3,1% | 121 |
| P3-69 | 127 | 5 | VH5-12-1 | 4 | 4,1% | 121 |
| P3-9 | 119 | 5 | VH5-12-1 | 4 | 4,1% | 121 |
| 1-185-37 | 125 | 5 | VH5-12-4 | 0 | 0,0% | 124 |
| 1-187-29 | 125 | 5 | VH5-12-4 | 0 | 0,0% | 124 |
| P1-58 | 128 | 5 | VH5-12-4 | 10 | 10,2% | 121 |
| P2-57 | 118 | 5 | VH5-12-4 | 3 | 3,1% | 121 |
| P2-55 | 123 | 5 | VH5-12-1 | 5 | 5,1% | 121 |
| P2-56 | 123 | 5 | VH5-12-1 | 20 | 20,4% | 121 |
| P2-52 | 122 | 5 | VH5-12-1 | 11 | 11,2% | 121 |
| P3-60 | 122 | 5 | VH5-12-1 | 8 | 8,2% | 121 |
| P1-57 | 123 | 5 | VH5-12-1 | 4 | 4,1% | 121 |
| P1-55 | 122 | 5 | VH5-12-1 | 14 | 14,3% | 121 |
| MD3-4 | 128 | 5 | VH5-12-4 | 12 | 12,2% | 5 |
| 11-52 | 121 | 5 | VH5-12-1 | 11 | 11,2% | 121 |
| CLL5 | 98 | 5 | VH5-12-1 | 13 | 13,3% | 17 |
| CLL7 | 98 | 5 | VH5-12-1 | 14 | 14,3% | 17 |
| L2F10 | 100 | 5 | VH5-12-1 | 1 | 1,0% | 46 |
| L3B6 | 98 | 5 | VH5-12-1 | 1 | 1,0% | 46 |
| VH6.A12 | 119 | 6 | VH6-35-1 | 13 | 12,9% | 122 |
| s5A9 | 102 | 6 | VH6-35-1 | 1 | 1,0% | 46 |
| s6G4 | 99 | 6 | VH6-35-1 | 1 | 1,0% | 46 |
| ss3 | 99 | 6 | VH6-35-1 | 1 | 1,0% | 46 |
| 6-1G1 | 101 | 6 | VH6-35-1 | 0 | 0,0% | 14 |
| F19L16 | 107 | 6 | VH6-35-1 | 0 | 0,0% | 68 |
| L16 | 120 | 6 | VH6-35-1 | 0 | 0,0% | 69 |
| M71 | 121 | 6 | VH6-35-1 | 0 | 0,0% | 103 |
| ML1 | 120 | 6 | VH6-35-1 | 0 | 0,0% | 69 |
| F19ML1 | 107 | 6 | VH6-35-1 | 0 | 0,0% | 68 |
| 15P1 | 127 | 6 | VH6-35-1 | 0 | 0,0% | 104 |
| VH6.N1 | 121 | 6 | VH6-35-1 | 0 | 0,0% | 122 |
| VH6.N11 | 123 | 6 | VH6-35-1 | 0 | 0,0% | 122 |
| VH6.N12 | 123 | 6 | VH6-35-1 | 0 | 0,0% | 122 |
| VH6.N2 | 125 | 6 | VH6-35-1 | 0 | 0,0% | 122 |
| VH6.N5 | 125 | 6 | VH6-35-1 | 0 | 0,0% | 122 |
| VH6.N6 | 127 | 6 | VH6-35-1 | 0 | 0,0% | 122 |
| VH6.N7 | 126 | 6 | VH6-35-1 | 0 | 0,0% | 122 |
| VH6.N8 | 123 | 6 | VH6-35-1 | 0 | 0,0% | 122 |
| VH6.N9 | 123 | 6 | VH6-35-1 | 0 | 0,0% | 122 |
| VH6.N10 | 123 | 6 | VH6-35-1 | 0 | 0,0% | 122 |
| VH6.A3 | 123 | 6 | VH6-35-1 | 0 | 0,0% | 122 |
| VH6.A1 | 124 | 6 | VH6-35-1 | 0 | 0,0% | 122 |
| VH6.A4 | 120 | 6 | VH6-35-1 | 0 | 0,0% | 122 |
| E55 6.16 | 116 | 6 | VH6-35-1 | 0 | 0,0% | 26 |
| E55 6.17 | 120 | 6 | VH6-35-1 | 0 | 0,0% | 26 |
| E55 6.6 | 120 | 6 | VH6-35-1 | 0 | 0,0% | 26 |
| VHGL 6.3 | 102 | 6 | VH6-35-1 | 0 | 0,0% | 26 |
| CB-201 | 118 | 6 | VH6-35-1 | 0 | 0,0% | 109 |
| VH6.N4 | 122 | 6 | VH6-35-1 | 0 | 0,0% | 122 |
| E54 6.4 | 109 | 6 | VH6-35-1 | 1 | 1,0% | 26 |
| VH6.A6 | 126 | 6 | VH6-35-1 | 1 | 1,0% | 122 |
| E55 6.14 | 120 | 6 | VH6-35-1 | 1 | 1,0% | 26 |
| E54 6.6 | 107 | 6 | VH6-35-1 | 1 | 1,0% | 26 |
| E55 6.10 | 112 | 6 | VH6-35-1 | 1 | 1,0% | 26 |
| E54 6.1 | 107 | 6 | VH6-35-1 | 2 | 2,0% | 26 |
| E55 6.13 | 120 | 6 | VH6-35-1 | 2 | 2,0% | 26 |
| E55 6.3 | 120 | 6 | VH6-35-1 | 2 | 2,0% | 26 |
| E55 6.7 | 116 | 6 | VH6-35-1 | 2 | 2,0% | 26 |
| E55 6.2 | 120 | 6 | VH6-35-1 | 2 | 2,0% | 26 |
| E55 6.X | 111 | 6 | VH6-35-1 | 2 | 2,0% | 26 |
| E55 6.11 | 111 | 6 | VH6-35-1 | 3 | 3,0% | 26 |
| VH6.A11 | 118 | 6 | VH6-35-1 | 3 | 3,0% | 122 |
| A10 | 107 | 6 | VH6-35-1 | 3 | 3,0% | 68 |
| F55 6.1 | 120 | 6 | VH6-35-1 | 4 | 4,0% | 26 |
| FK-001 | 124 | 6 | VH6-35-1 | 4 | 4,0% | 65 |
| VH6.A5 | 121 | 6 | VH6-35-1 | 4 | 4,0% | 122 |
| VH6.A7 | 123 | 6 | VH6-35-1 | 4 | 4,0% | 122 |
| HBp2 | 119 | 6 | VH6-35-1 | 4 | 4,0% | 4 |
| Au46.2 | 123 | 6 | VH6-35-1 | 5 | 5,0% | 49 |
| A431 | 106 | 6 | VH6-35-1 | 5 | 5,0% | 68 |
| VH6.A2 | 120 | 6 | VH6-35-1 | 5 | 5,0% | 122 |
| VH6.A9 | 125 | 6 | VH6-35-1 | 8 | 7,9% | 122 |
| VH6.A8 | 118 | 6 | VH6-35-1 | 10 | 9,9% | 122 |
| VH6-FF3 | 118 | 6 | VH6-35-1 | 2 | 2,0% | 123 |
| VH6.A10 | 126 | 6 | VH6-35-1 | 12 | 11,9% | 122 |
| VH6-EB10 | 117 | 6 | VH6-35-1 | 3 | 3,0% | 123 |
| VH6-E6 | 119 | 6 | VH6-35-1 | 6 | 5,9% | 123 |
| VH6-FE2 | 121 | 6 | VH6-35-1 | 6 | 5,9% | 123 |
| VH6-EE6 | 116 | 6 | VH6-35-1 | 6 | 5,9% | 123 |
| VH6-FD10 | 118 | 6 | VH6-35-1 | 6 | 5,9% | 123 |
| VH6-EX8 | 113 | 6 | VH6-35-1 | 6 | 5,9% | 123 |
| VH6-FG9 | 121 | 6 | VH6-35-1 | 8 | 7,9% | 123 |
| VH6-E5 | 116 | 6 | VH6-35-1 | 9 | 8,9% | 123 |
| VH6-EC8 | 122 | 6 | VH6-35-1 | 9 | 8,9% | 123 |
| VH6-E10 | 120 | 6 | VH6-35-1 | 10 | 9,9% | 123 |
| VH6-FF11 | 122 | 6 | VH6-35-1 | 11 | 10,9% | 123 |
| VH6-FD2 | 115 | 6 | VH6-35-1 | 11 | 10,9% | 123 |
| CLL10 17-2 | 88 | 6 | VH6-35-1 | 4 | 4,0% | 29 |
| VH6-BB11 | 94 | 6 | VH6-35-1 | 4 | 4,0% | 123 |
| VH6-B41 | 93 | 6 | VH6-35-1 | 7 | 6,9% | 123 |
| JU17 | 102 | 6 | VH6-35-1 | 3 | 3,0% | 114 |
| VH6-BD9 | 96 | 6 | VH6-35-1 | 11 | 10,9% | 123 |
| VH6-BB9 | 94 | 6 | VH6-35-1 | 12 | 11,9% | 123 |

**Table 3A: assignment of rearranged V kappa sequences to their germline counterparts**

| **Family¹** | **Name** | **Rearranged²** | **Sum** |
|---|---|---|---|
| 1 | Vk1-1 | 28 | |
| 1 | Vk1-2 | 0 | |
| 1 | Vk1-3 | 1 | |
| 1 | Vk1-4 | 0 | |
| 1 | Vk1-5 | 7 | |
| 1 | Vk1-6 | 0 | |
| 1 | Vk1-7 | 0 | |
| 1 | Vk1-8 | 2 | |
| 1 | Vk1-9 | 9 | |
| 1 | Vk1-10 | 0 | |
| 1 | Vk1-11 | 1 | |
| 1 | Vk1-12 | 7 | |
| 1 | Vk1-13 | 1 | |
| 1 | Vk1-14 | 7 | |
| 1 | Vk1-15 | 2 | |
| 1 | Vk1-16 | 2 | |
| 1 | Vk1-17 | 16 | |
| 1 | Vk1-18 | 1 | |
| 1 | Vk1-19 | 33 | |
| 1 | Vk1-20 | 1 | |
| 1 | Vk1-21 | 1 | |
| 1 | Vk1-22 | 0 | |
| 1 | Vk1-23 | 0 | ***119 entries*** |
| 2 | Vk2-1 | 0 | |
| 2 | Vk2-2 | 1 | |
| 2 | Vk2-3 | 0 | |
| 2 | Vk2-4 | 0 | |
| 2 | Vk2-5 | 0 | |
| 2 | Vk2-6 | 16 | |
| 2 | Vk2-7 | 0 | |
| 2 | Vk2-8 | 0 | |
| 2 | Vk2-9 | 1 | |
| 2 | Vk2-10 | 0 | |
| 2 | Vk2-11 | 7 | |
| 2 | Vk2-12 | 0 | ***25 entries*** |
| 3 | Vk3-1 | 1 | |
| 3 | Vk3-2 | 0 | |
| 3 | Vk3-3 | 35 | |
| 3 | Vk3-4 | 115 | |
| 3 | Vk3-5 | 0 | |
| 3 | Vk3-6 | 0 | |
| 3 | Vk3-7 | 1 | |
| 3 | Vk3-8 | 40 | ***192 entries*** |
| 4 | Vk4-1 | 33 | ***33 entries*** |
| 5 | Vk5-1 | 1 | ***1 entry*** |
| 6 | Vk6-1 | 0 | |
| 6 | Vk6-2 | 0 | ***0 entries*** |
| 7 | Vk7-1 | 0 | ***0 entries*** |

**Table 3B: assignment of rearranged V lambda sequences to their germline counterparts**

| **Family¹** | **Name** | **Rearranged²** | **Sum** |
|---|---|---|---|
| 1 | DPL1 | 1 | |
| 1 | DPL2 | 14 | |
| 1 | DPL3 | 6 | |
| 1 | DPL4 | 1 | |
| 1 | HUMLV117 | 4 | |
| 1 | DPL5 | 13 | |
| 1 | DPL6 | 0 | |
| 1 | DPL7 | 0 | |
| 1 | DPL8 | 3 | |
| 1 | DPL9 | 0 | ***42 entries*** |
| 2 | DPL10 | 5 | |
| 2 | VLAMBDA 2.1 | 0 | |
| 2 | DPL11 | 23 | |
| 2 | DPL12 | 15 | |
| 2 | DPL13 | 0 | |
| 2 | DPL14 | 0 | ***43 entries*** |
| 3 | DPL16 | 10 | |
| 3 | DPL23 | 19 | |
| 3 | Humlv318 | 9 | ***38 entries*** |
| 7 | DPL18 | 1 | |
| 7 | DPL19 | 0 | ***1 entries*** |
| 8 | DPL21 | 2 | |
| 8 | HUMLV801 | 6 | ***8 entries*** |
| 9 | DPL22 | 0 | ***0 entries*** |
| unassigned | DPL24 | 0 | ***0 entries*** |
| 10 | gVLX-4.4 | 0 | ***0 entries*** |

**Table 3C: assignment of rearranged V heavy chain sequences to their germline counterparts**

| **Family¹** | **Name** | **Rearranged²** | **Sum** |
|---|---|---|---|
| 1 | VH1-12-1 | 38 | |
| 1 | VH1-12-8 | 2 | |
| 1 | VH1-12-2 | 2 | |
| 1 | VH1-12-9 | 2 | |
| 1 | VH1-12-3 | 0 | |
| 1 | VH1-12-4 | 0 | |
| 1 | VH1-12-5 | 3 | |
| 1 | VH1-12-6 | 0 | |
| 1 | VH1-12-7 | 23 | |
| 1 | VH1-13-1 | 1 | |
| 1 | VH1-13-2 | 1 | |
| 1 | VH1-13-3 | 0 | |
| 1 | VH1-13-4 | 0 | |
| 1 | VH1-13-5 | 0 | |
| 1 | VH1-13-6 | 17 | |
| 1 | VH1-13-7 | 0 | |
| 1 | VH1-13-8 | 3 | |
| 1 | VH1-13-9 | 0 | |
| 1 | VH1-13-10 | 0 | |
| 1 | VH113-11 | 0 | |
| 1 | VH1-13-12 | 10 | |
| 1 | VH1-13-13 | 0 | |
| 1 | VH1-13-14 | 0 | |
| 1 | VH1-13-15 | 4 | |
| 1 | VH1-13-16 | 2 | |
| 1 | VH1-13-17 | 0 | |
| 1 | VH1-13-18 | 1 | |
| 1 | VH1-13-19 | 0 | |
| 1 | VH1-1X-1 | 1 | ***110 entries*** |
| 2 | VH2-21-1 | 0 | |
| 2 | VH2-31-1 | 0 | |
| 2 | VH2-31-2 | 1 | |
| 2 | VH2-31-3 | 1 | |
| 2 | VH2-31-4 | 0 | |
| 2 | VH2-31-5 | 2 | |
| 2 | VH2-31-6 | 0 | |
| 2 | VH2-31-7 | 0 | |
| 2 | VH2-31-14 | 1 | |
| 2 | VH2-31-8 | 0 | |
| 2 | VH2-31-9 | 0 | |
| 2 | VH2-31-10 | 0 | |
| 2 | VH2-31-11 | 1 | |
| 2 | VH2-31-12 | 0 | |
| 2 | VH2-31-13 | 1 | **7 *entries*** |
| 3 | VH3-11-1 | 0 | |
| 3 | VH3-11-2 | 0 | |
| 3 | VH3-11-3 | 5 | |
| 3 | VH3-11-4 | 0 | |
| 3 | VH3-11-5 | 1 | |
| 3 | VH3-11-6 | 1 | |
| 3 | VH3-11-7 | 0 | |
| 3 | VH3-11-8 | 5 | |
| 3 | VH3-13-1 | 9 | |
| 3 | VH3-13-2 | 3 | |
| 3 | VH3-13-3 | 0 | |
| 3 | VH3-13-4 | 0 | |
| 3 | VH3-13-5 | 0 | |
| 3 | VH3-13-6 | 0 | |
| 3 | VH3-13-7 | 32 | |
| 3 | VH3-13-8 | 4 | |
| 3 | VH3-13-9 | 0 | |
| 3 | VH3-13-10 | 46 | |
| 3 | VH3-13-11 | 0 | |
| 3 | VH3-13-12 | 11 | |
| 3 | VH3-13-13 | 17 | |
| 3 | VH3-13-14 | 8 | |
| 3 | VH3-13-15 | 4 | |
| 3 | VH3-13-16 | 3 | |
| 3 | VH3-13-17 | 2 | |
| 3 | VH3-13-18 | 1 | |
| 3 | VH3-13-19 | 13 | |
| 3 | VH3-13-20 | 1 | |
| 3 | VH3-13-21 | 1 | |
| 3 | VH3-13-22 | 0 | |
| 3 | VH3-13-23 | 0 | |
| 3 | VH3-13-24 | 4 | |
| 3 | VH3-13-25 | 1 | |
| 3 | VH3-13-26 | 6 | |
| 3 | VH3-14-1 | 1 | |
| 3 | VH3-14-4 | 15 | |
| 3 | VH3-14-2 | 0 | |
| 3 | VH3-14-3 | 0 | |
| 3 | VH3-1X-1 | 0 | |
| 3 | VH3-1X-2 | 0 | |
| 3 | VH3-1X-3 | 6 | |
| 3 | VH3-1X-4 | 0 | |
| 3 | VH3-1X-5 | 0 | |
| 3 | VH3-1X-6 | 11 | |
| 3 | VH3-1X-7 | 0 | |
| 3 | VH3-1X-8 | 1 | |
| 3 | VH3-1X-9 | 0 | ***212 entries*** |
| 4 | VH4-11-1 | 0 | |
| 4 | VH4-11-2 | 20 | |
| 4 | VH4-11-3 | 0 | |
| 4 | VH4-11-4 | 0 | |
| 4 | VH4-11-5 | 0 | |
| 4 | VH4-11-6 | 0 | |
| 4 | VH4-11-7 | 5 | |
| 4 | VH4-11-8 | 7 | |
| 4 | VH4-11-9 | 3 | |
| 4 | VH4-11-10 | 0 | |
| 4 | VH4-11-11 | 0 | |
| 4 | VH4-11-12 | 4 | |
| 4 | VH4-11-13 | 0 | |
| 4 | VH4-11-14 | 0 | |
| 4 | VH4-11-15 | 0 | |
| 4 | VH4-11-16 | 1 | |
| 4 | VH4-21-1 | 0 | |
| 4 | VH4-21-2 | 0 | |
| 4 | VH4-21-3 | 1 | |
| 4 | VH4-21-4 | 1 | |
| 4 | VH4-21-5 | 1 | |
| 4 | VH4-21-6 | 1 | |
| 4 | VH4-21-7 | 0 | |
| 4 | VH4-21-8 | 0 | |
| 4 | VH4-21-9 | 0 | |
| 4 | VH4-31-1 | 0 | |
| 4 | VH4-31-2 | 0 | |
| 4 | VH4-31-3 | 0 | |
| 4 | VH4-31-4 | 2 | |
| 4 | VH4-31-5 | 0 | |
| 4 | VH4-31-6 | 0 | |
| 4 | VH4-31-7 | 0 | |
| 4 | VH4-31-8 | 0 | |
| 4 | VH4-31-9 | 0 | |
| 4 | VH4-31-10 | 0 | |
| 4 | VH4-31-11 | 0 | |
| 4 | VH4-31-12 | 4 | |
| 4 | VH4-31-13 | 7 | |
| 4 | VH4-31-14 | 0 | |
| 4 | VH4-31-15 | 0 | |
| 4 | VH4-31-16 | 0 | |
| 4 | VH4-31-17 | 0 | |
| 4 | VH4-31-18 | 0 | |
| 4 | VH4-31-19 | 0 | |
| 4 | VH4-31-20 | 0 | ***57 entries*** |
| 5 | VH5-12-1 | 82 | |
| 5 | VH5-12-2 | 1 | |
| 5 | VH5-12-3 | 0 | |
| 5 | VH5-12-4 | 14 | ***97 entries*** |
| 6 | VH6-35-1 | 74 | ***74 entries*** |

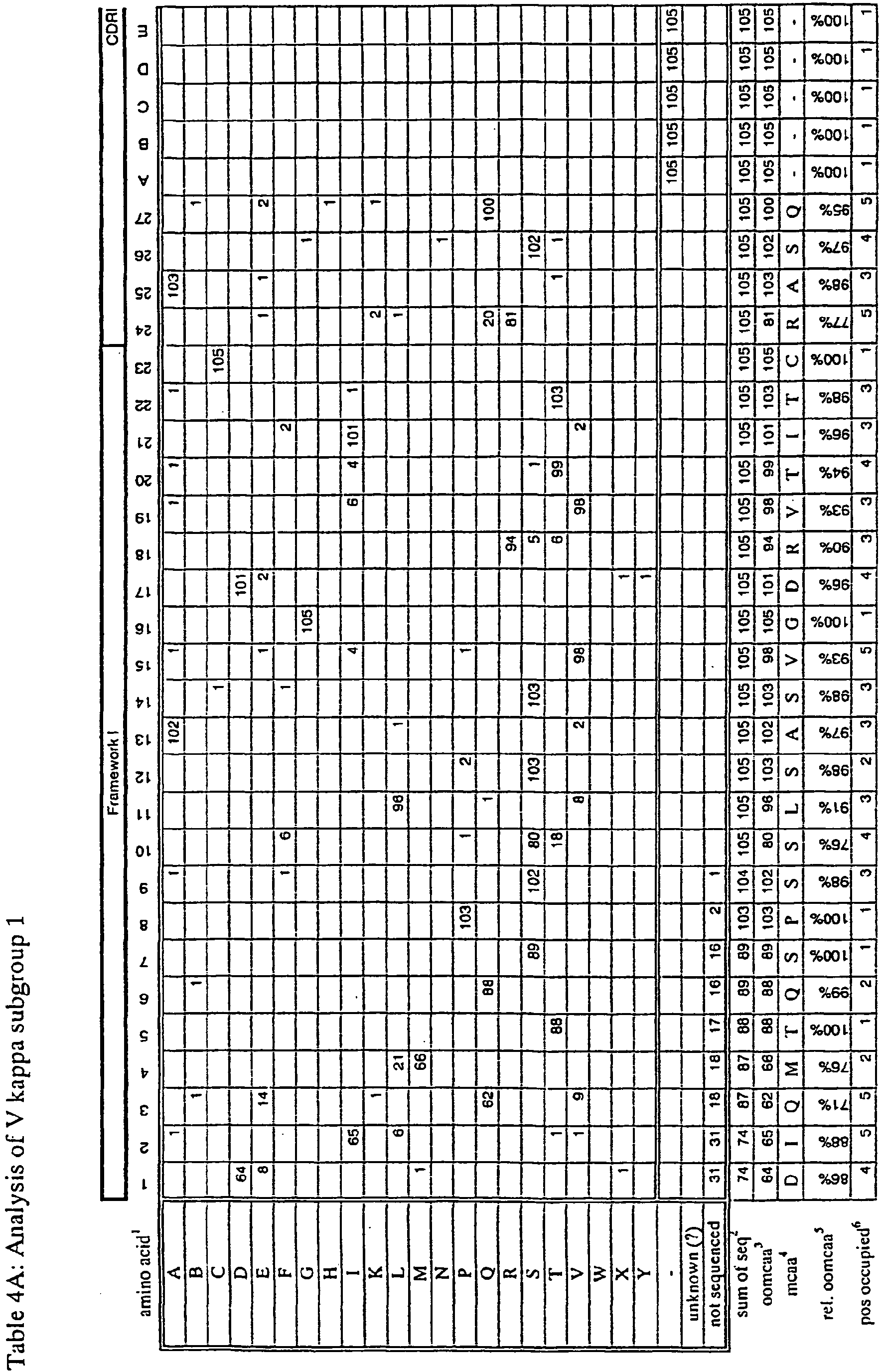

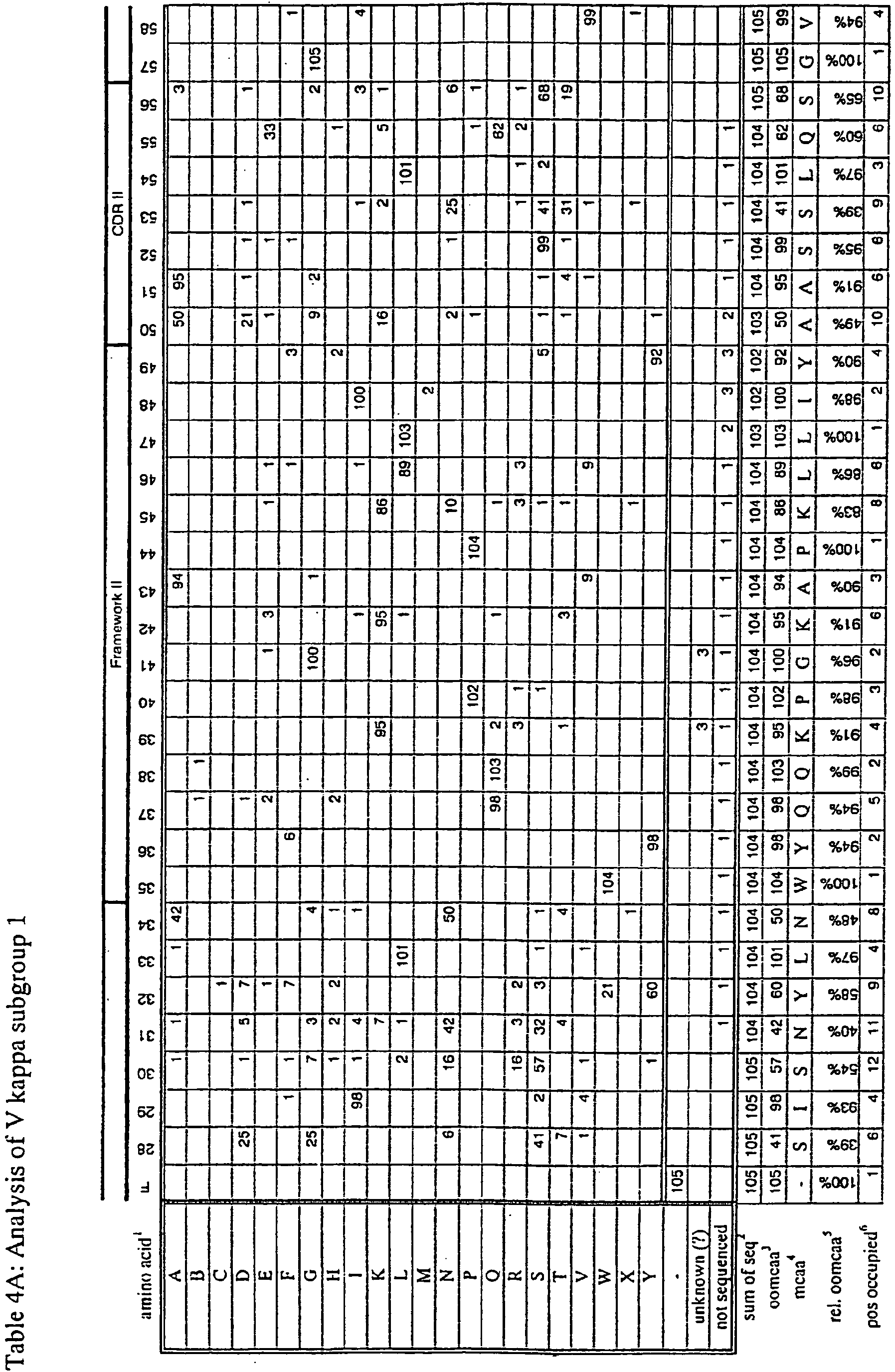

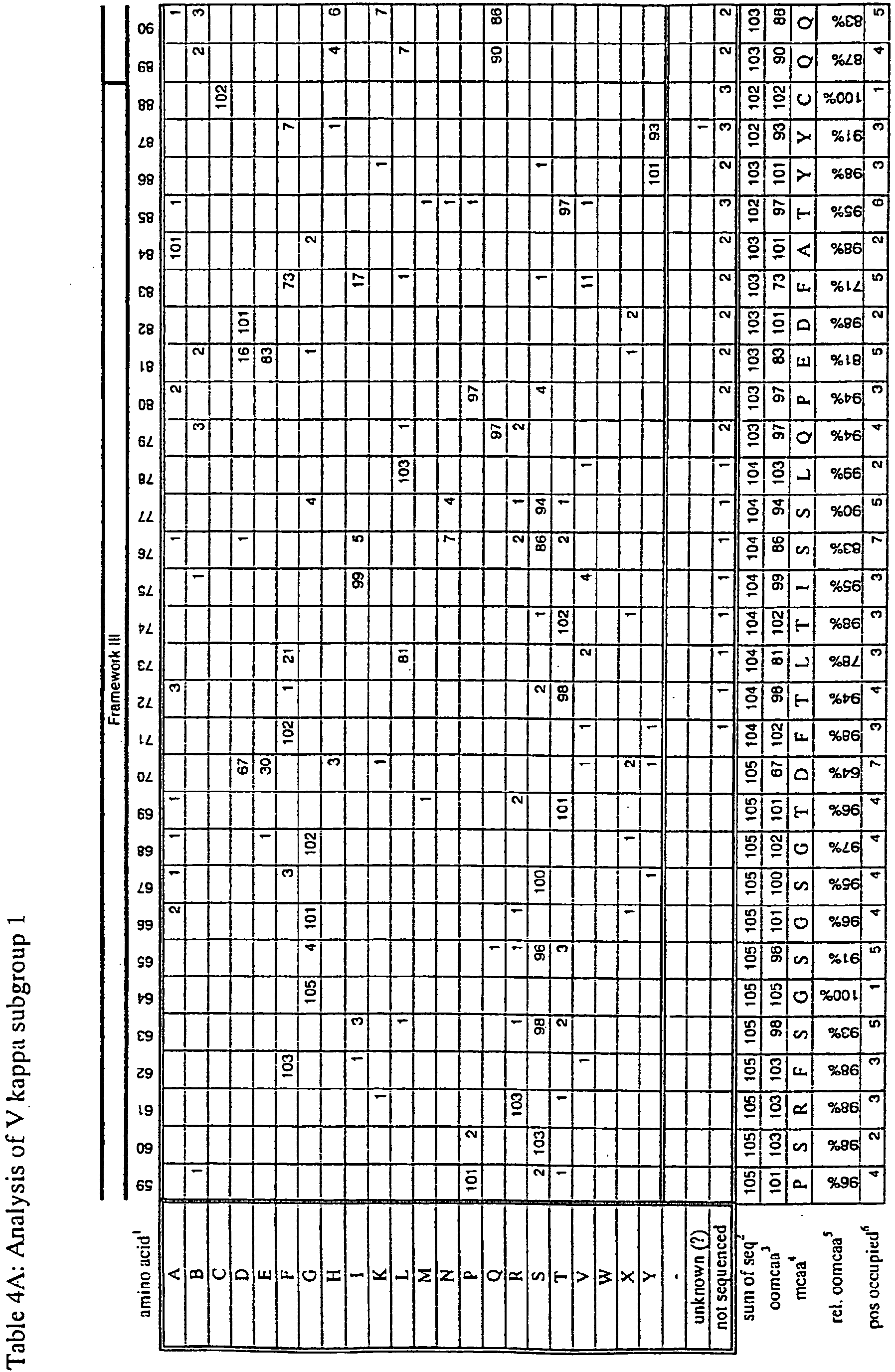

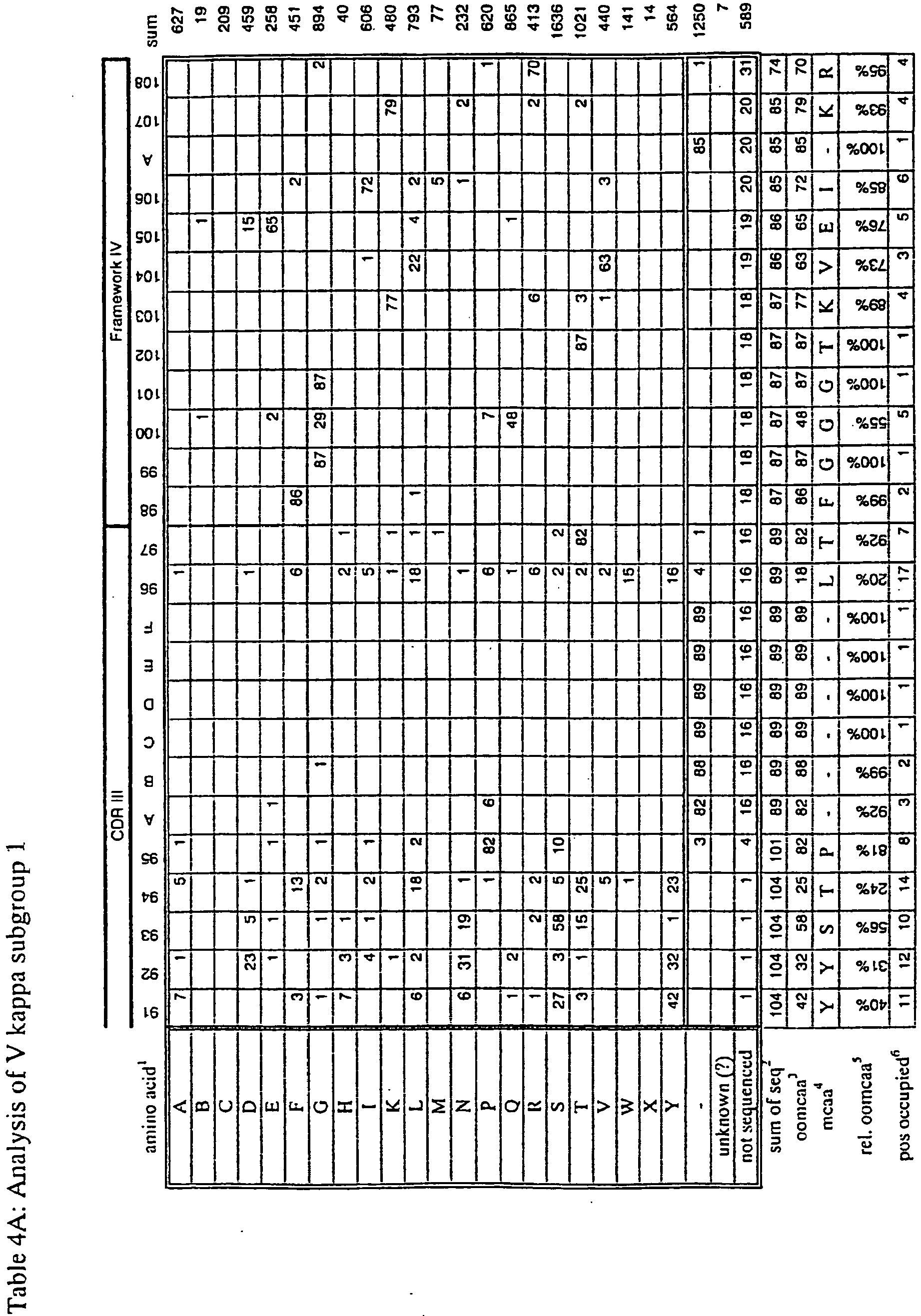

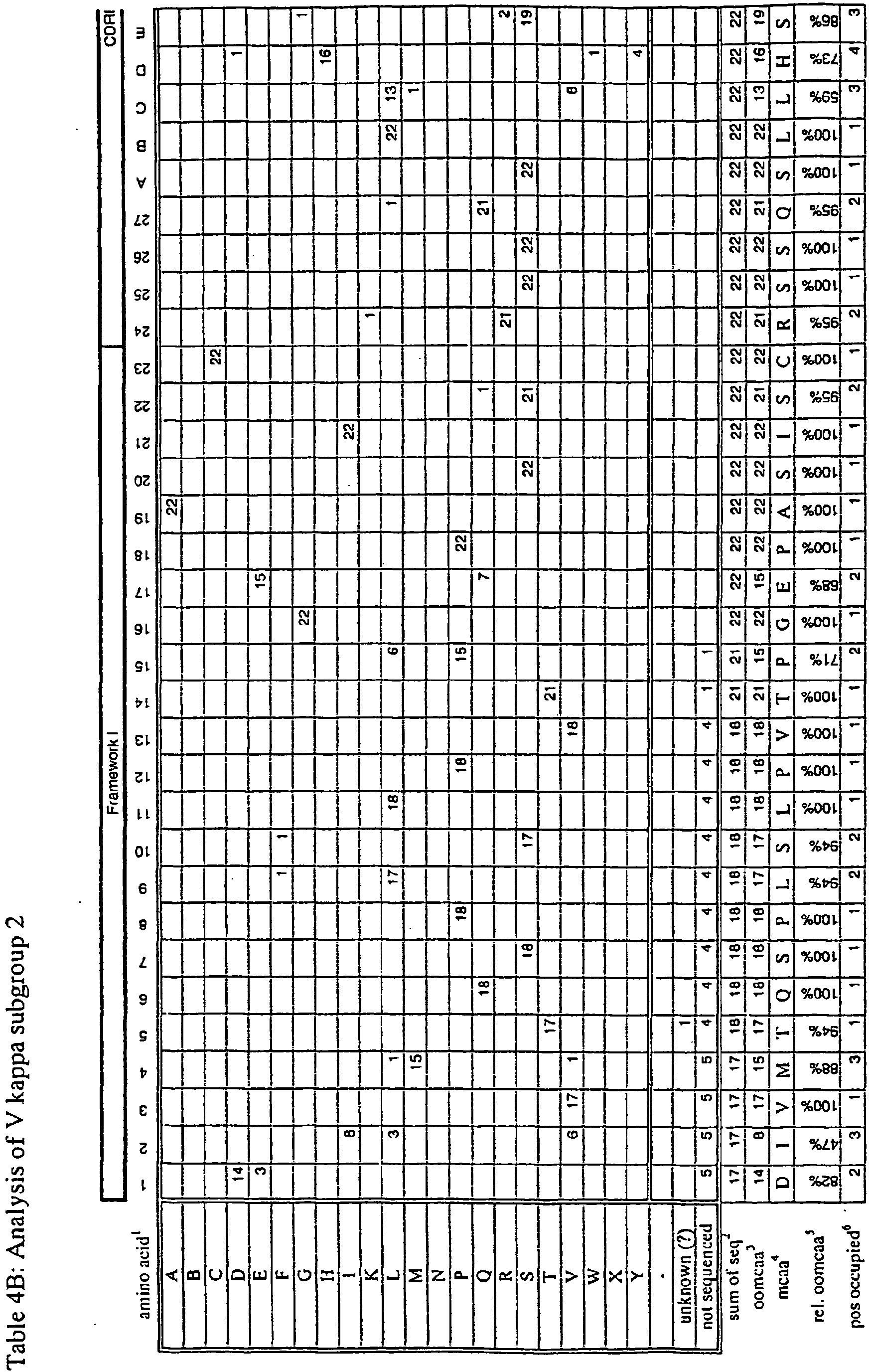

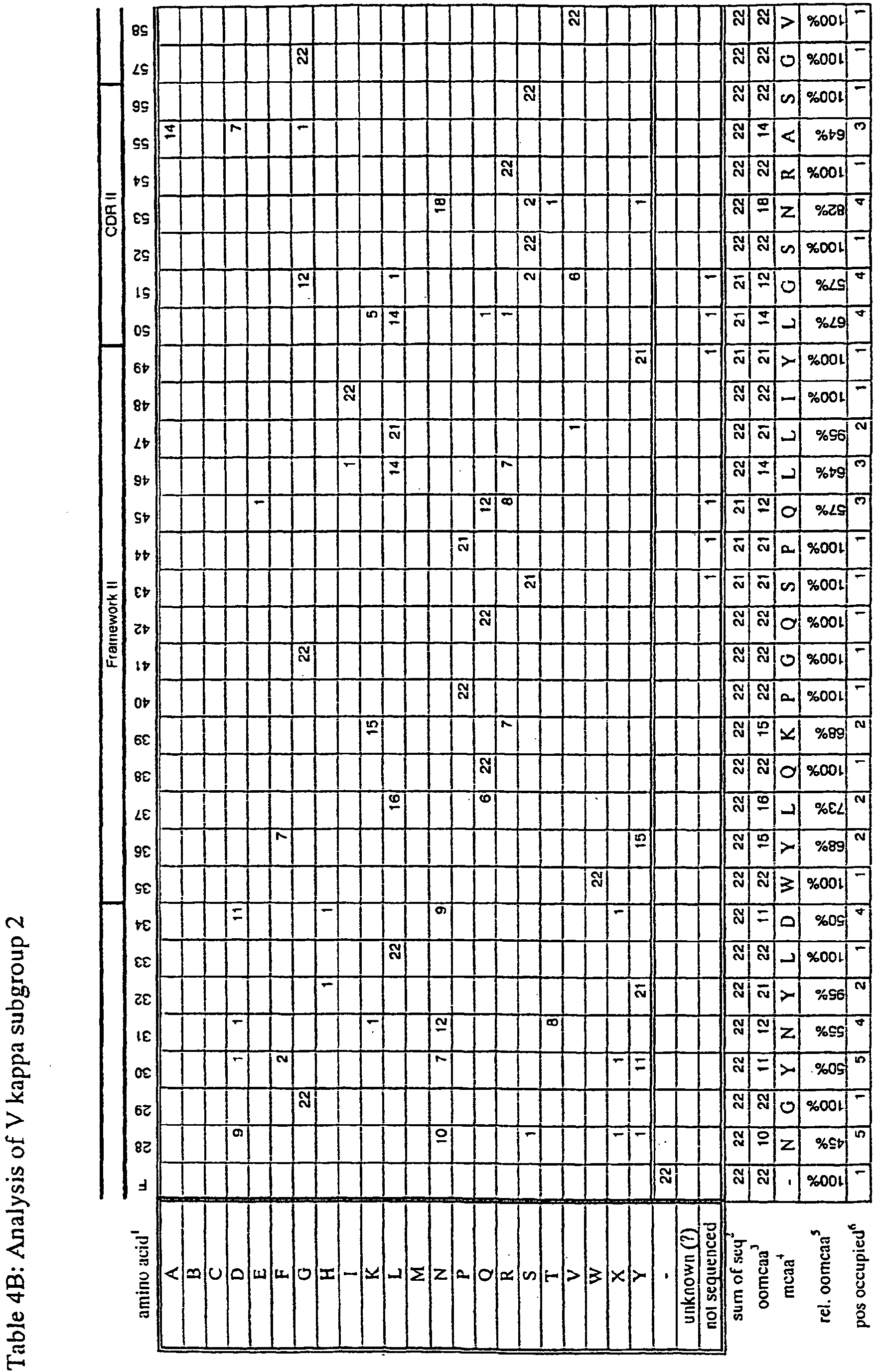

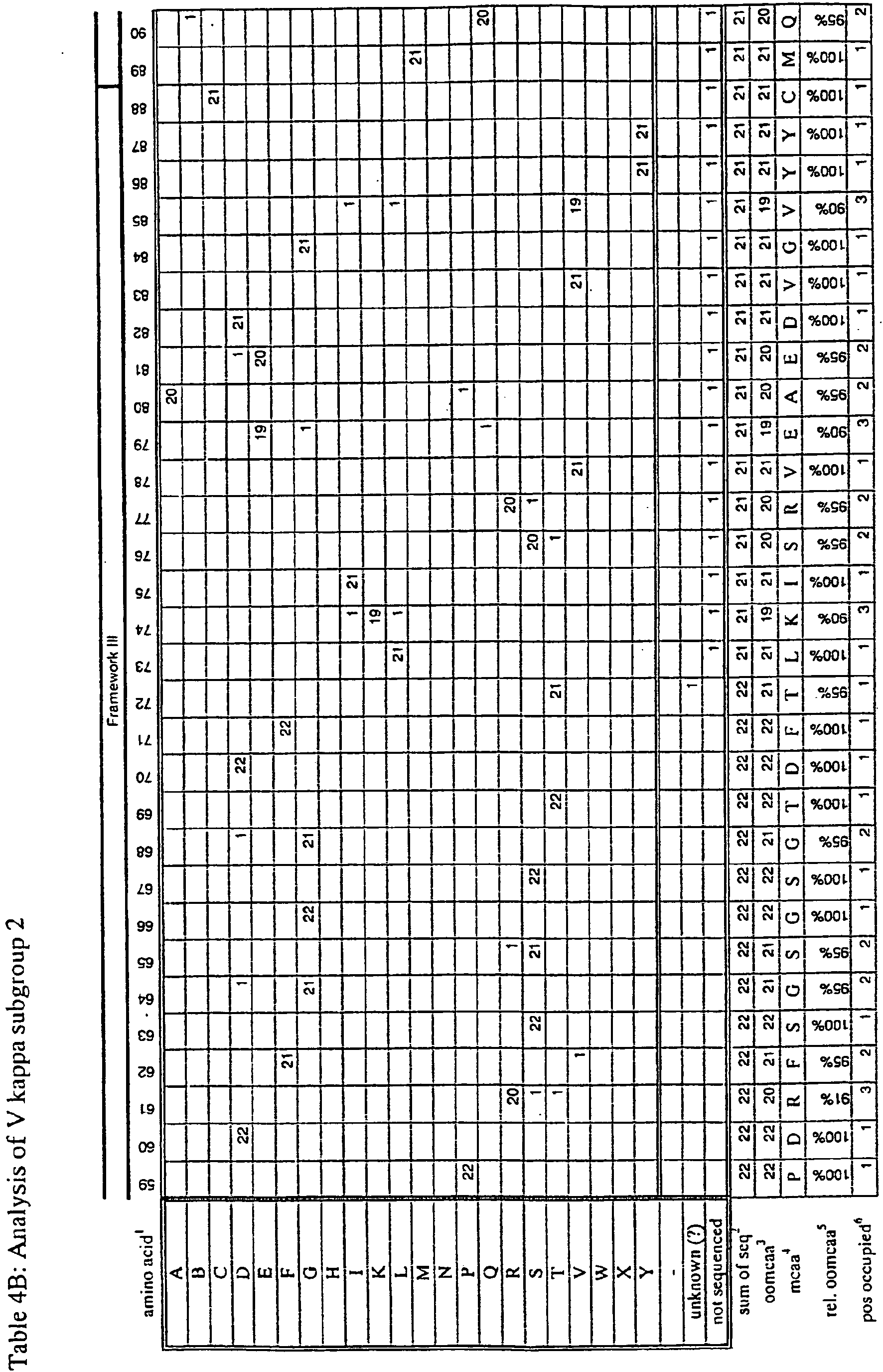

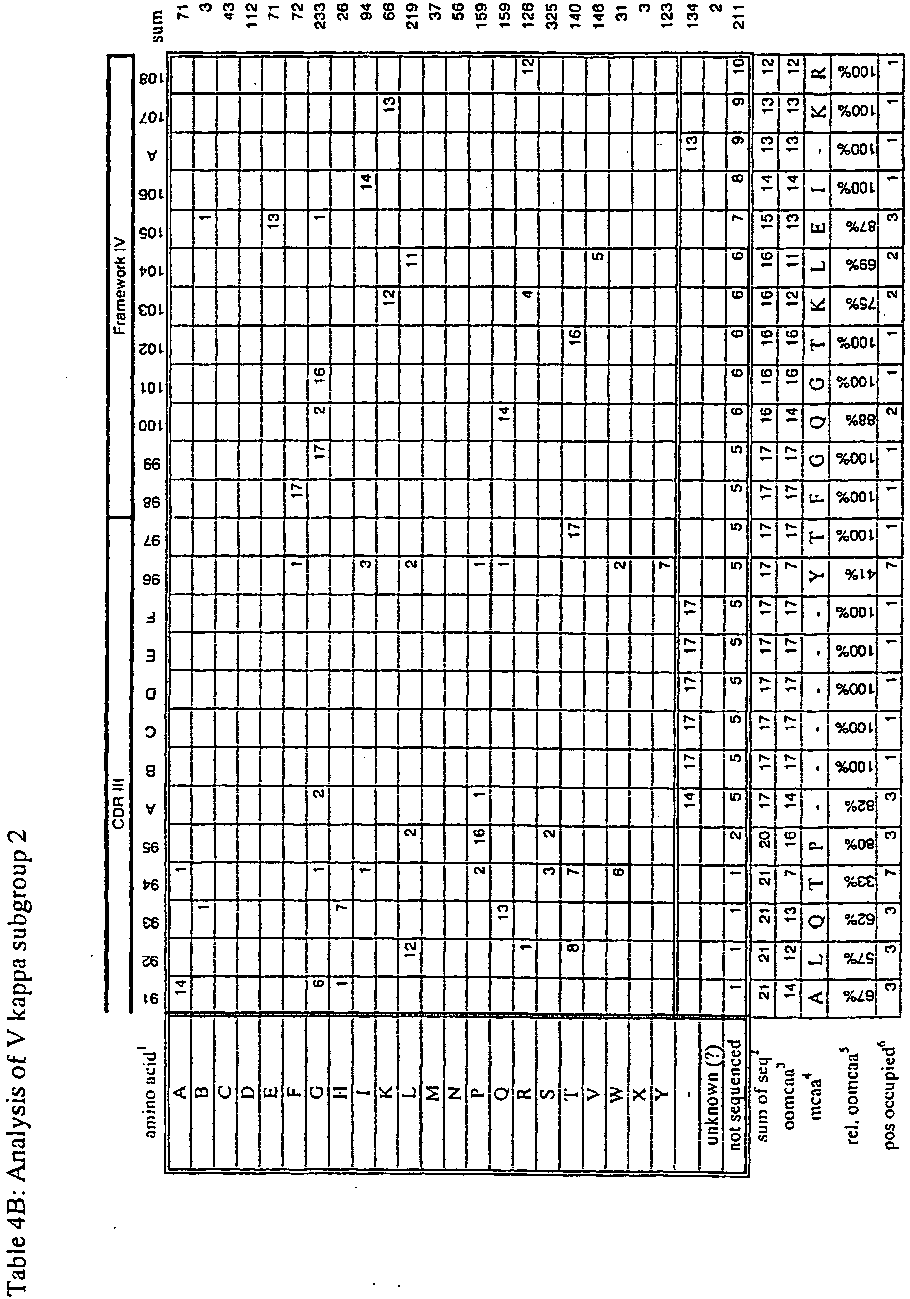

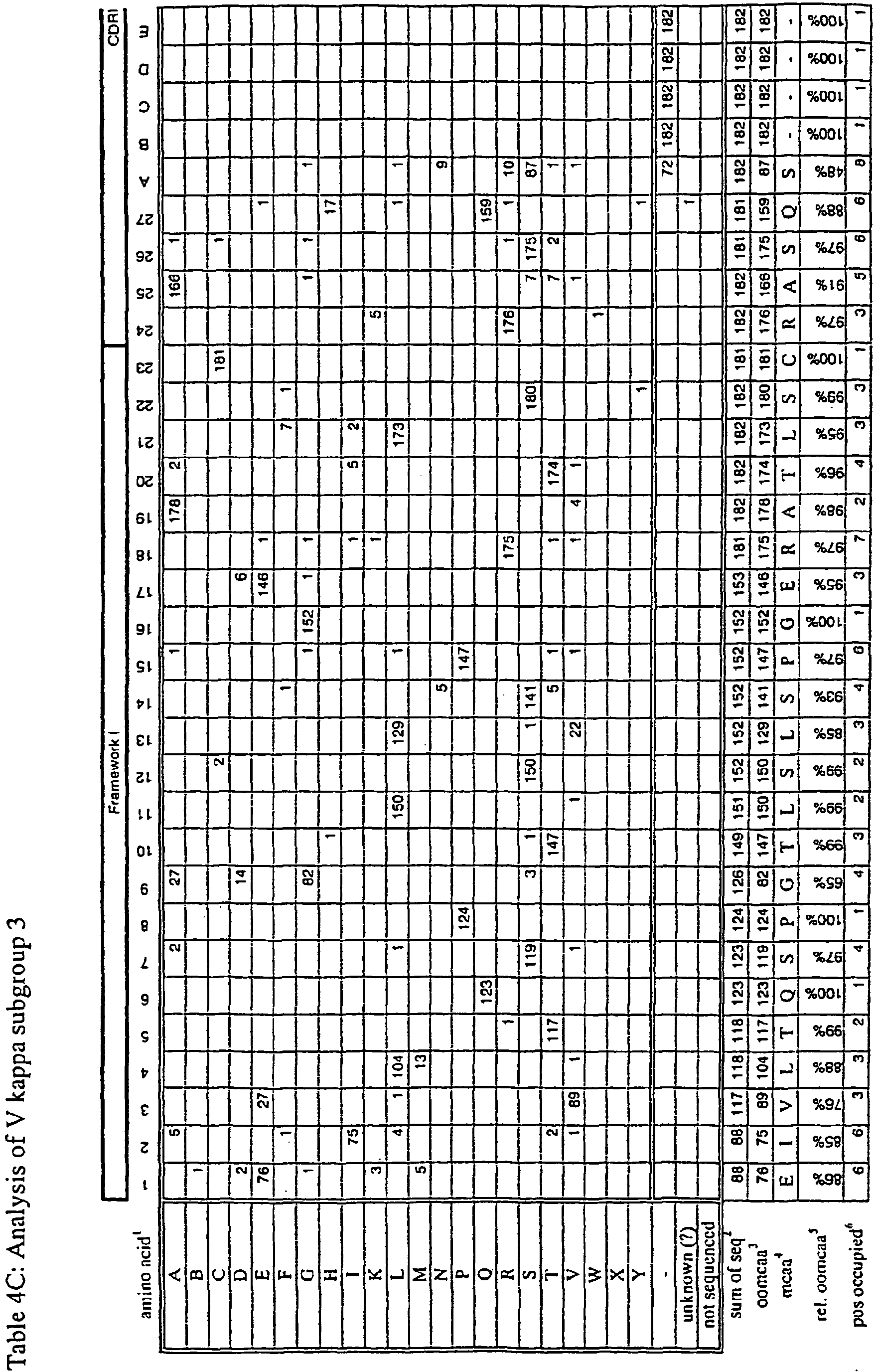

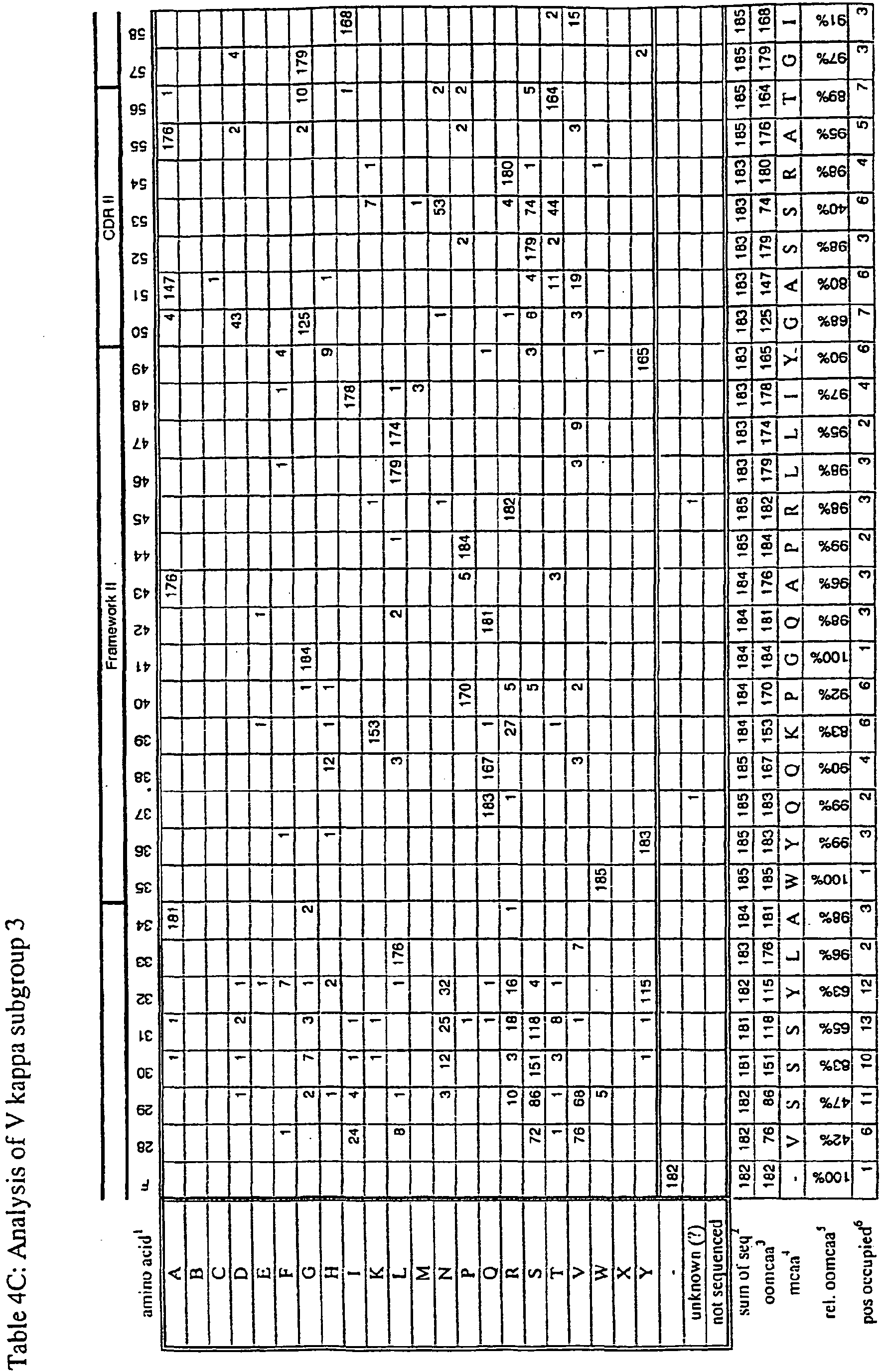

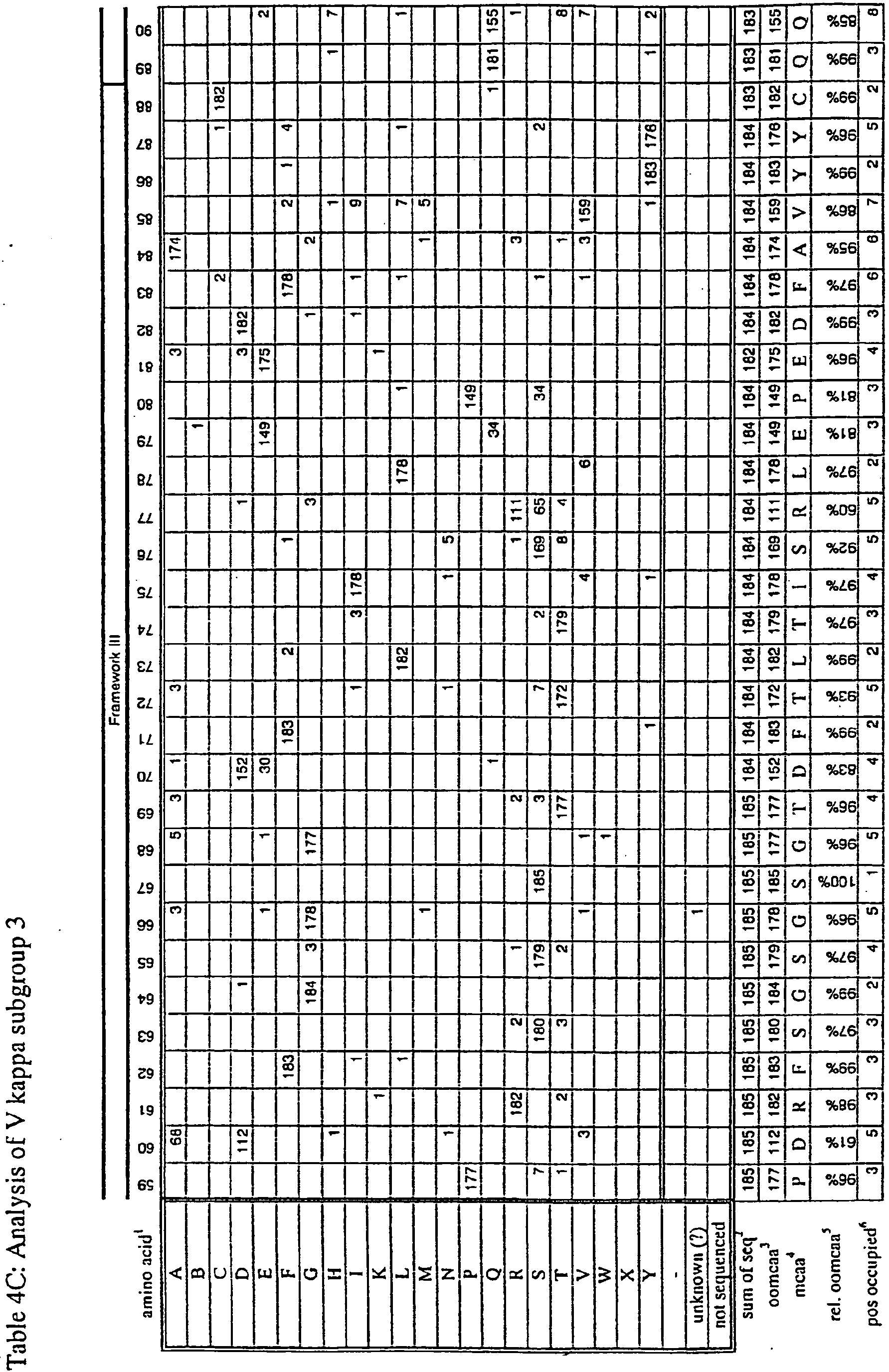

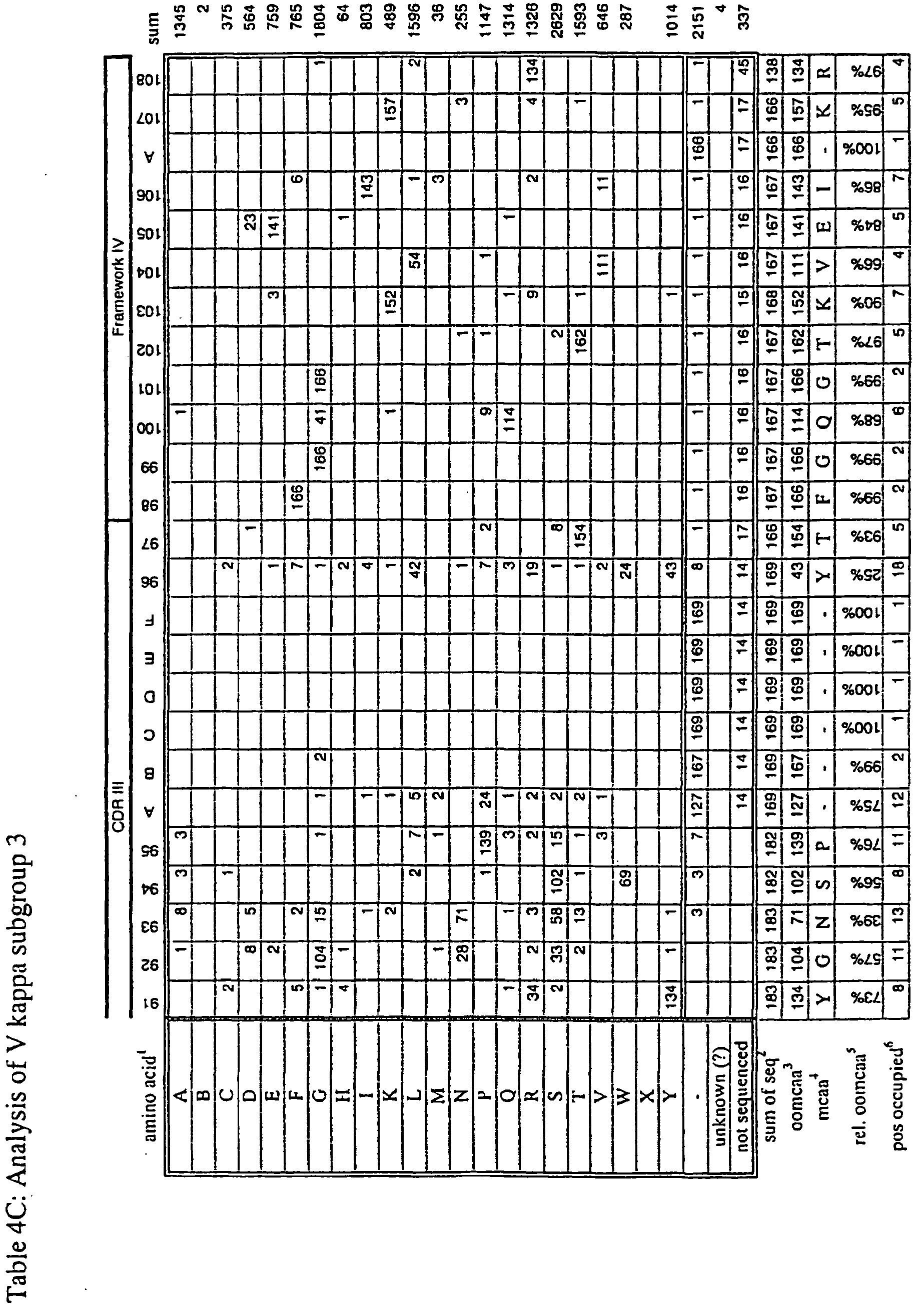

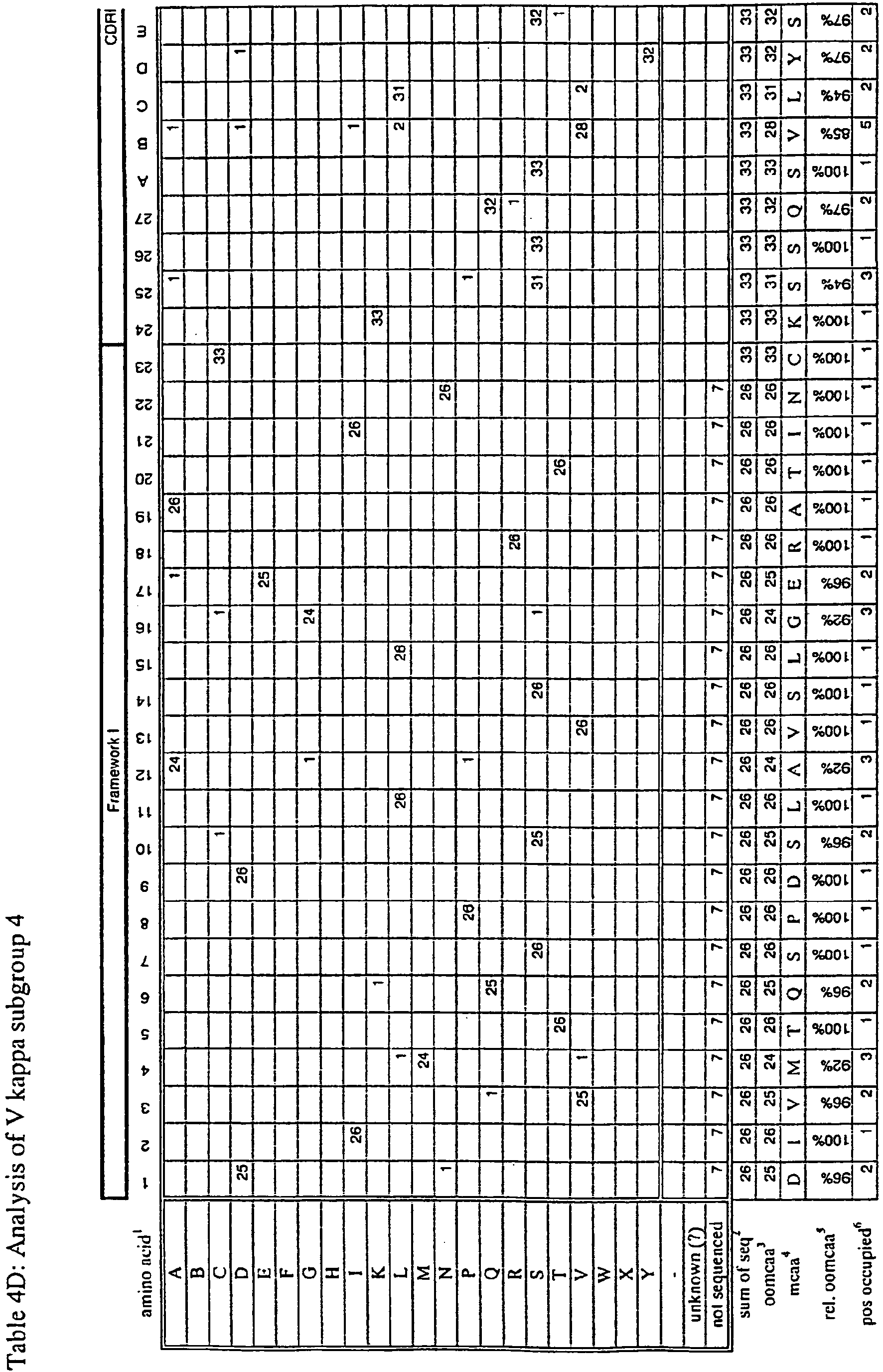

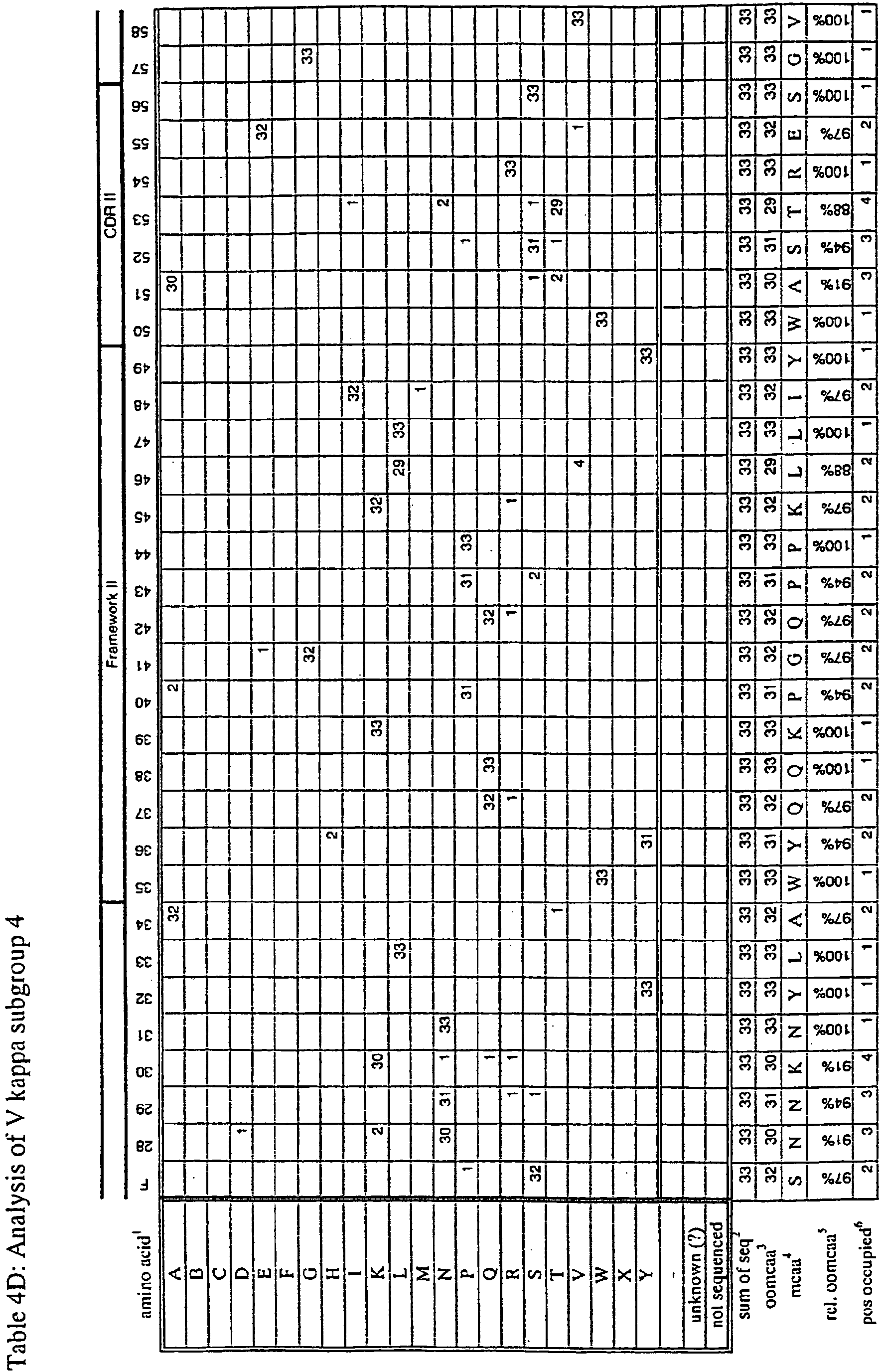

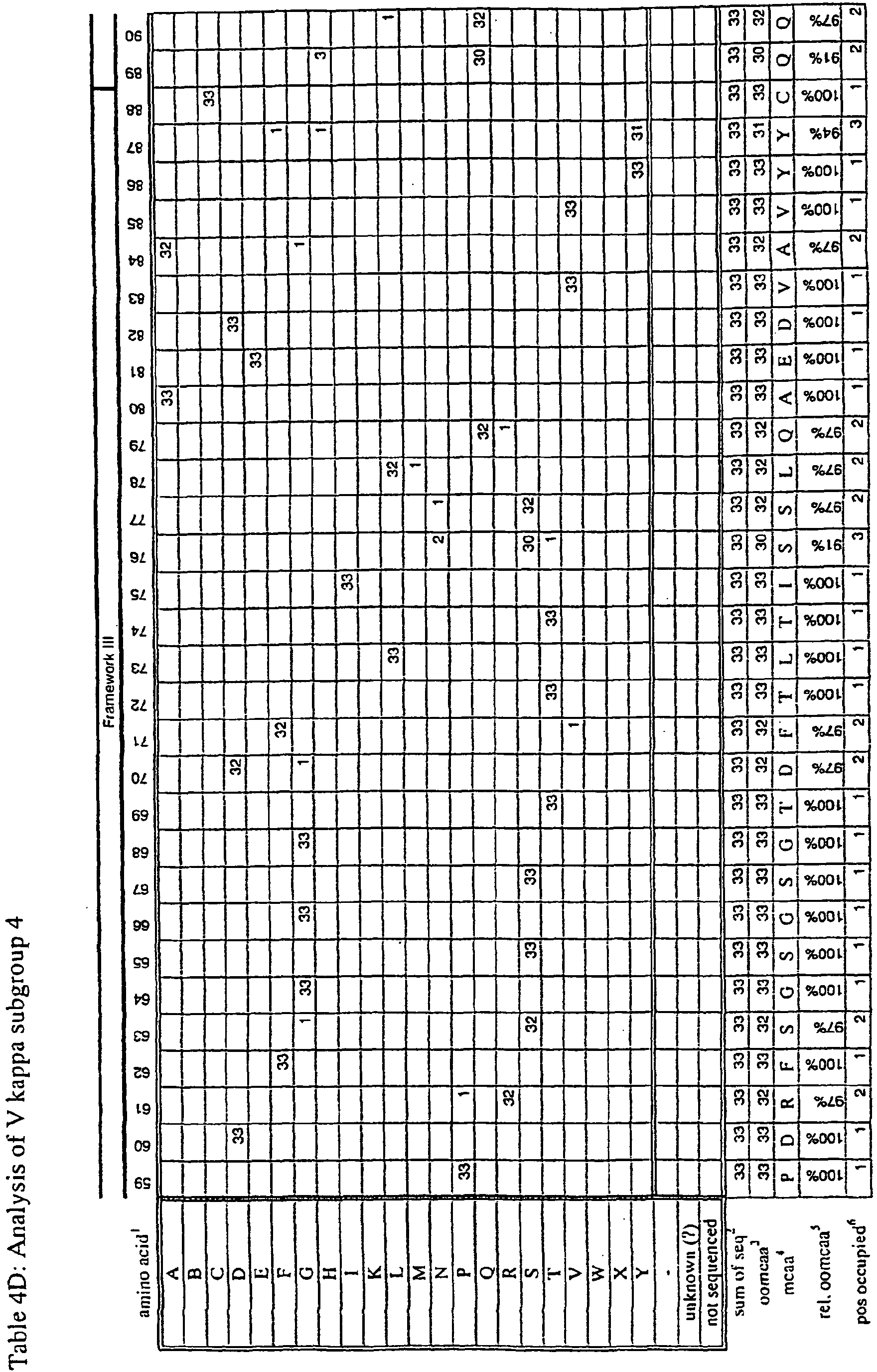

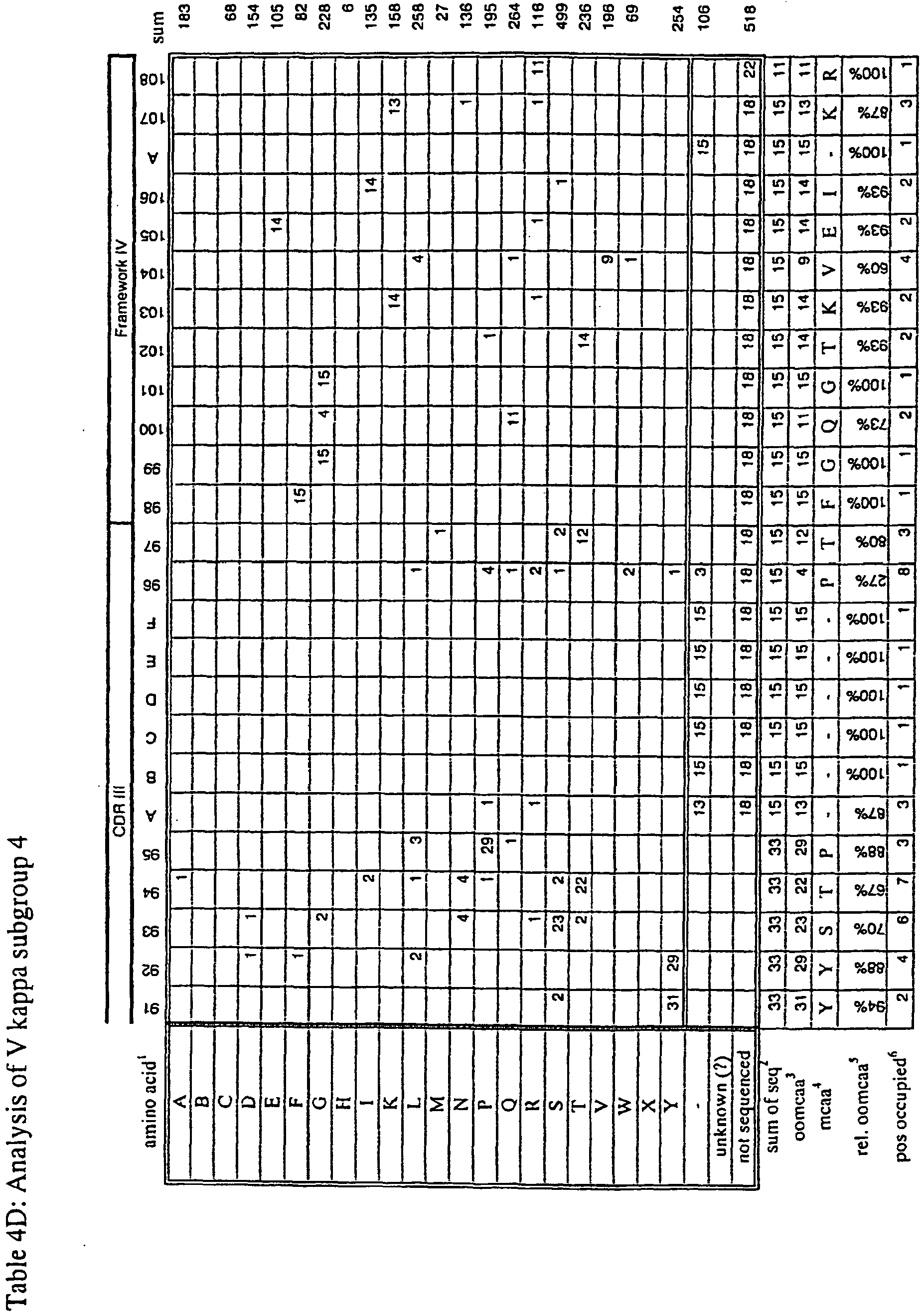

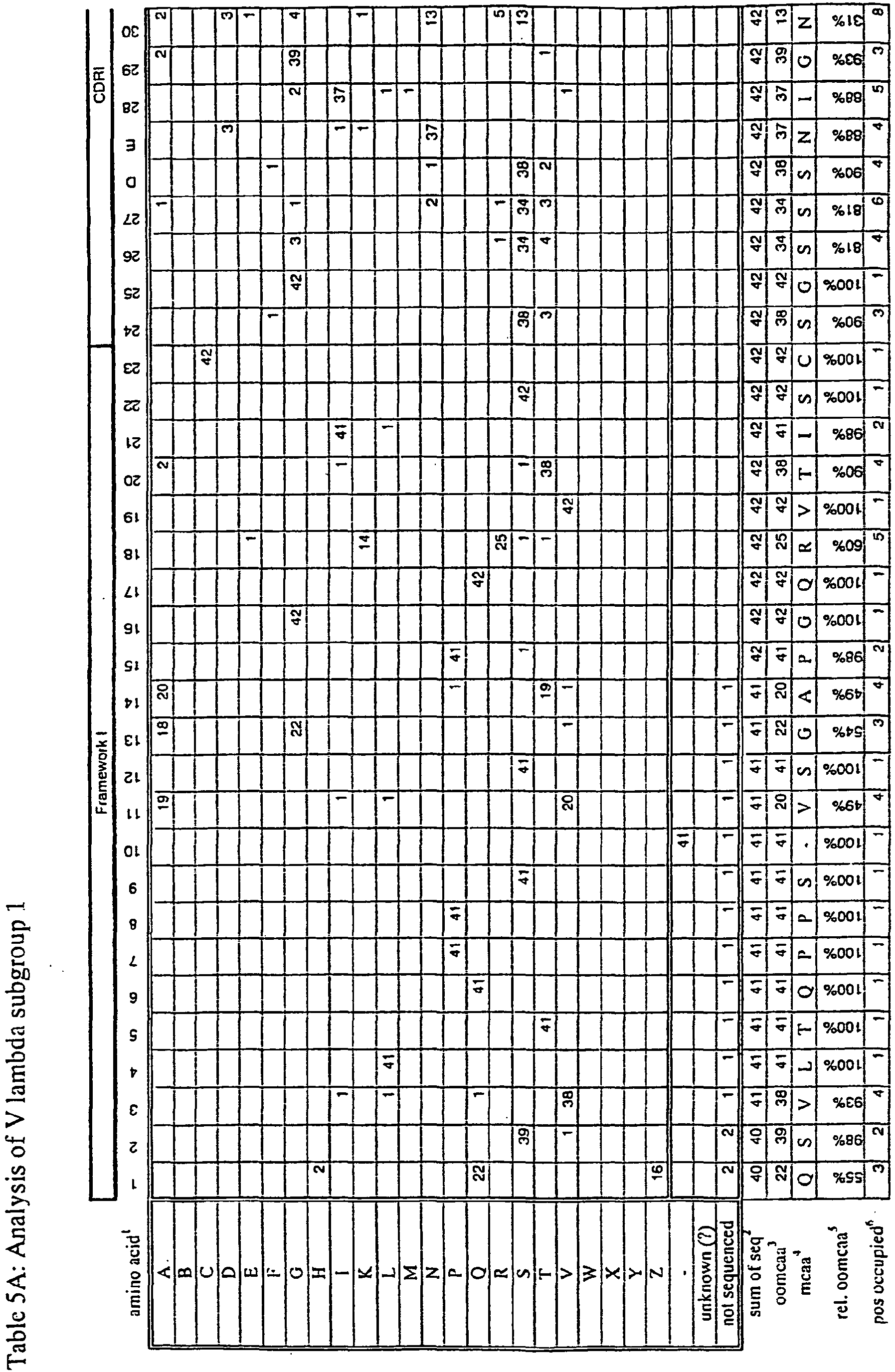

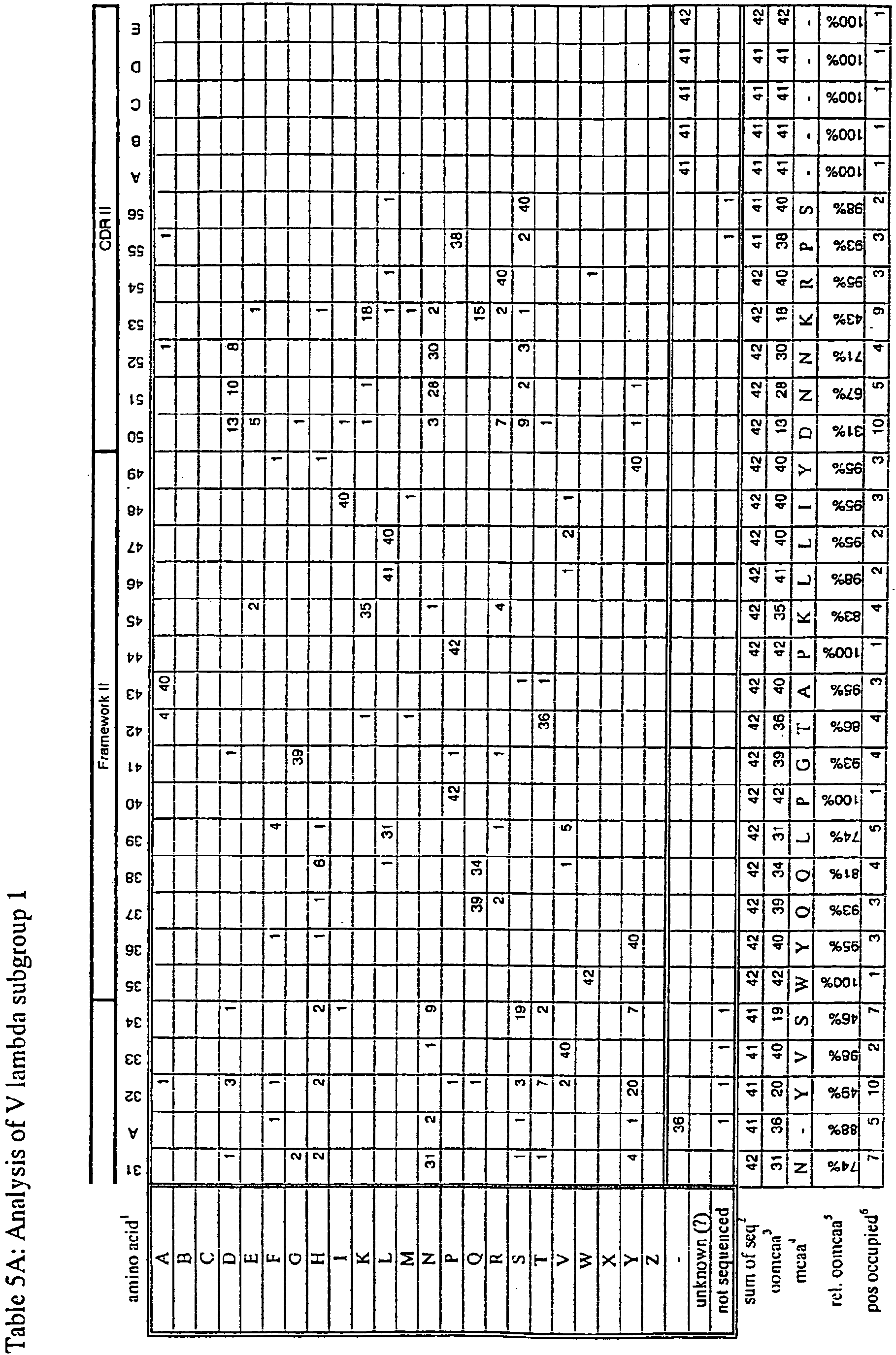

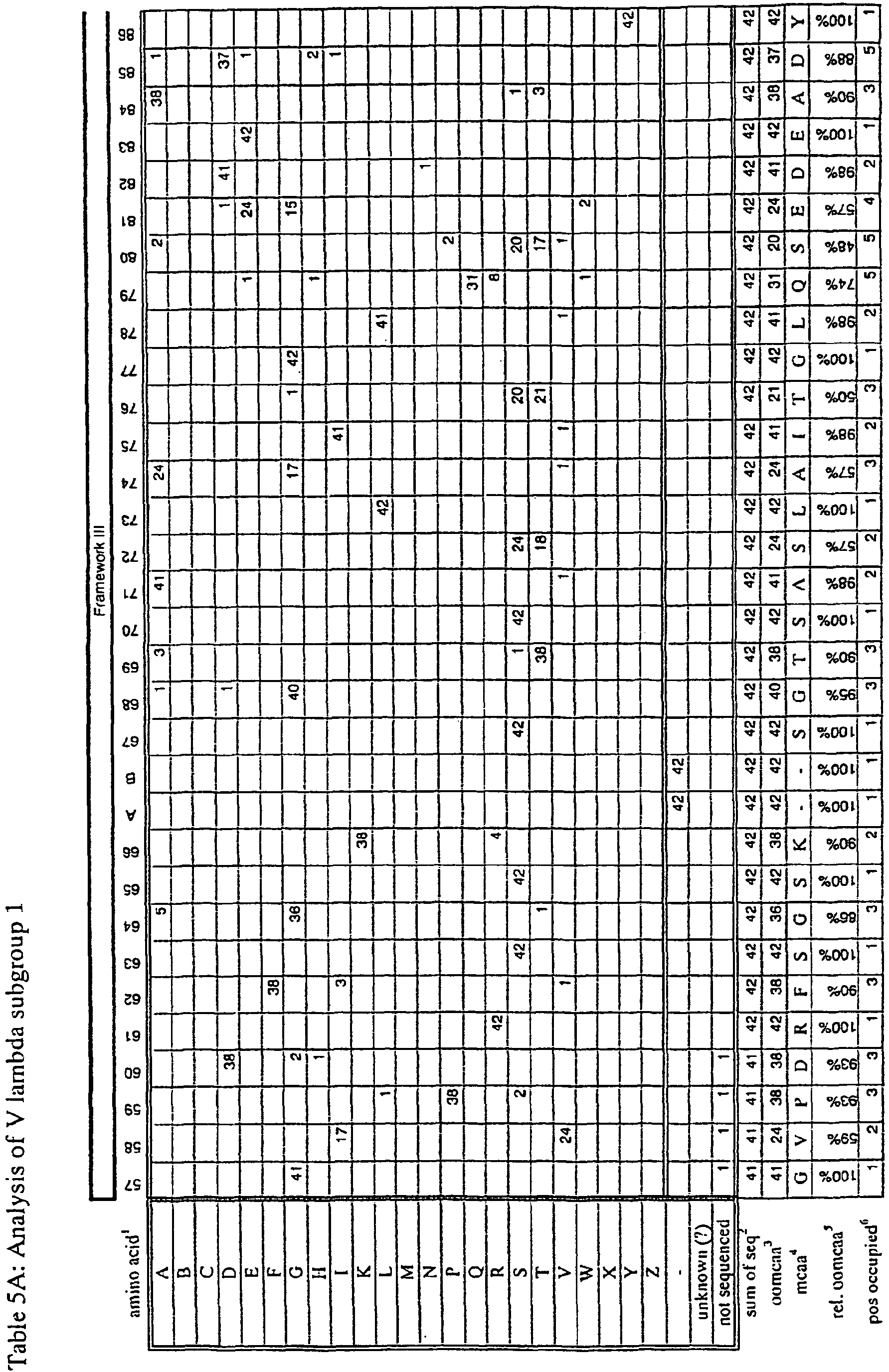

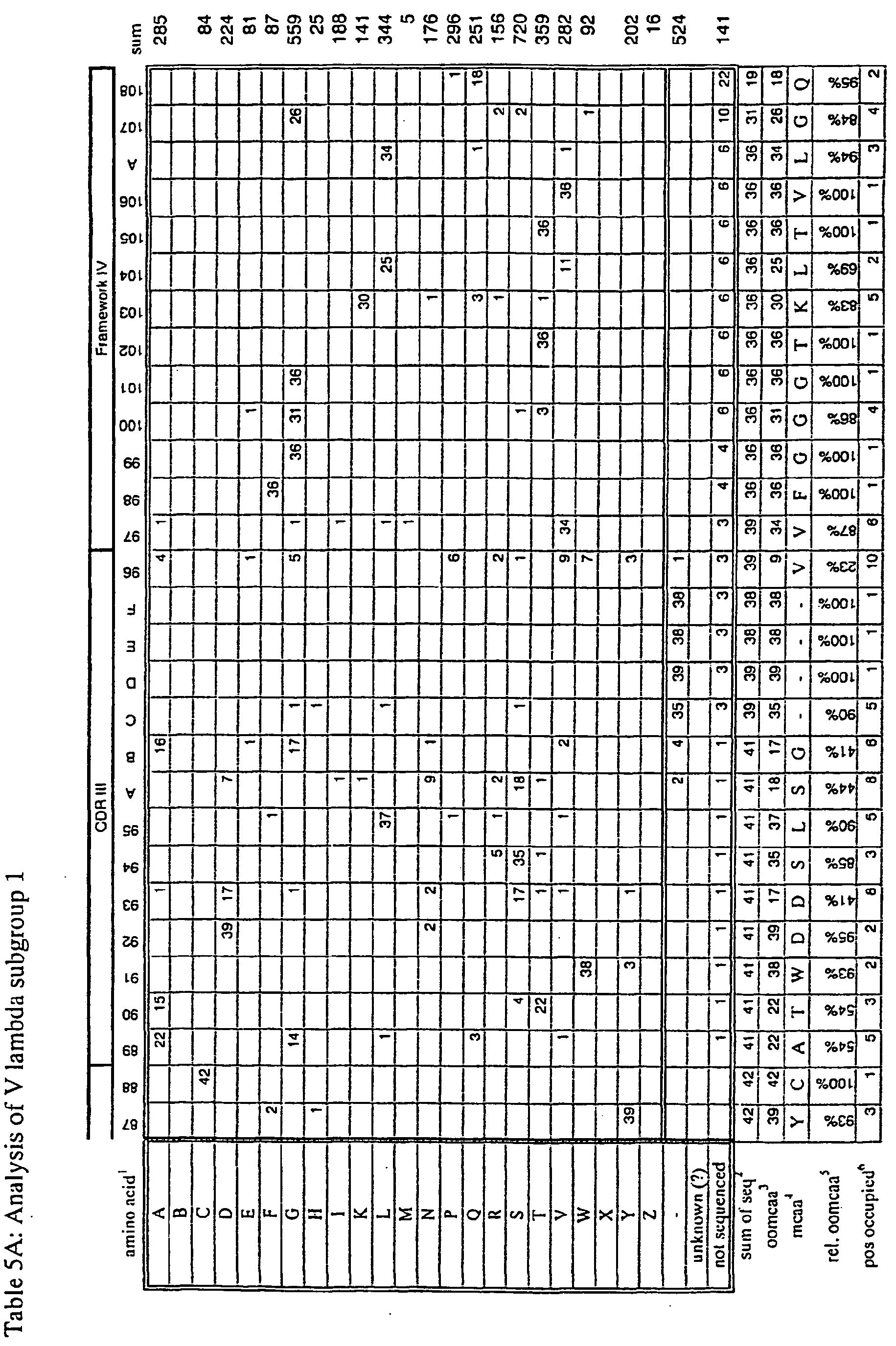

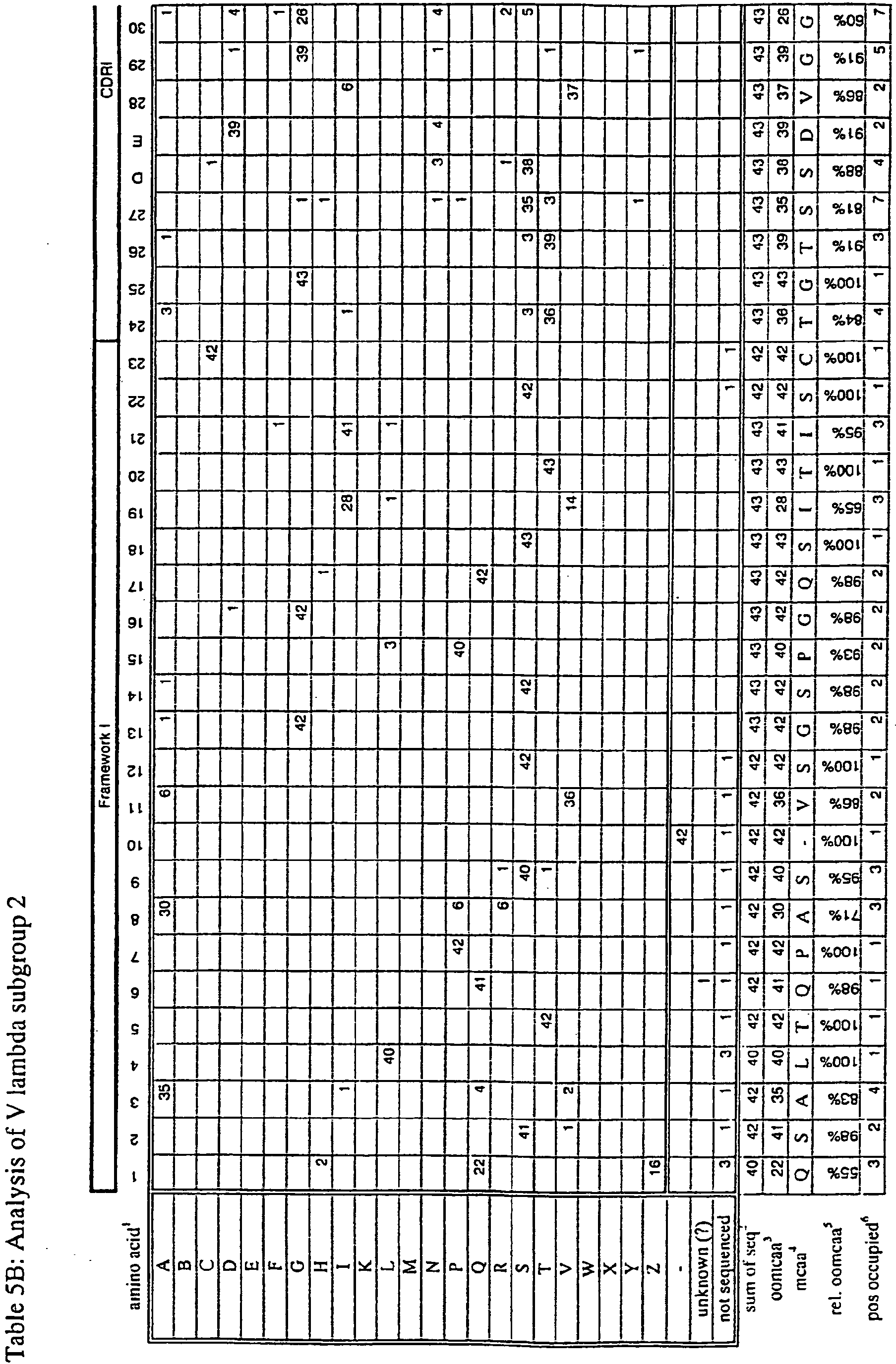

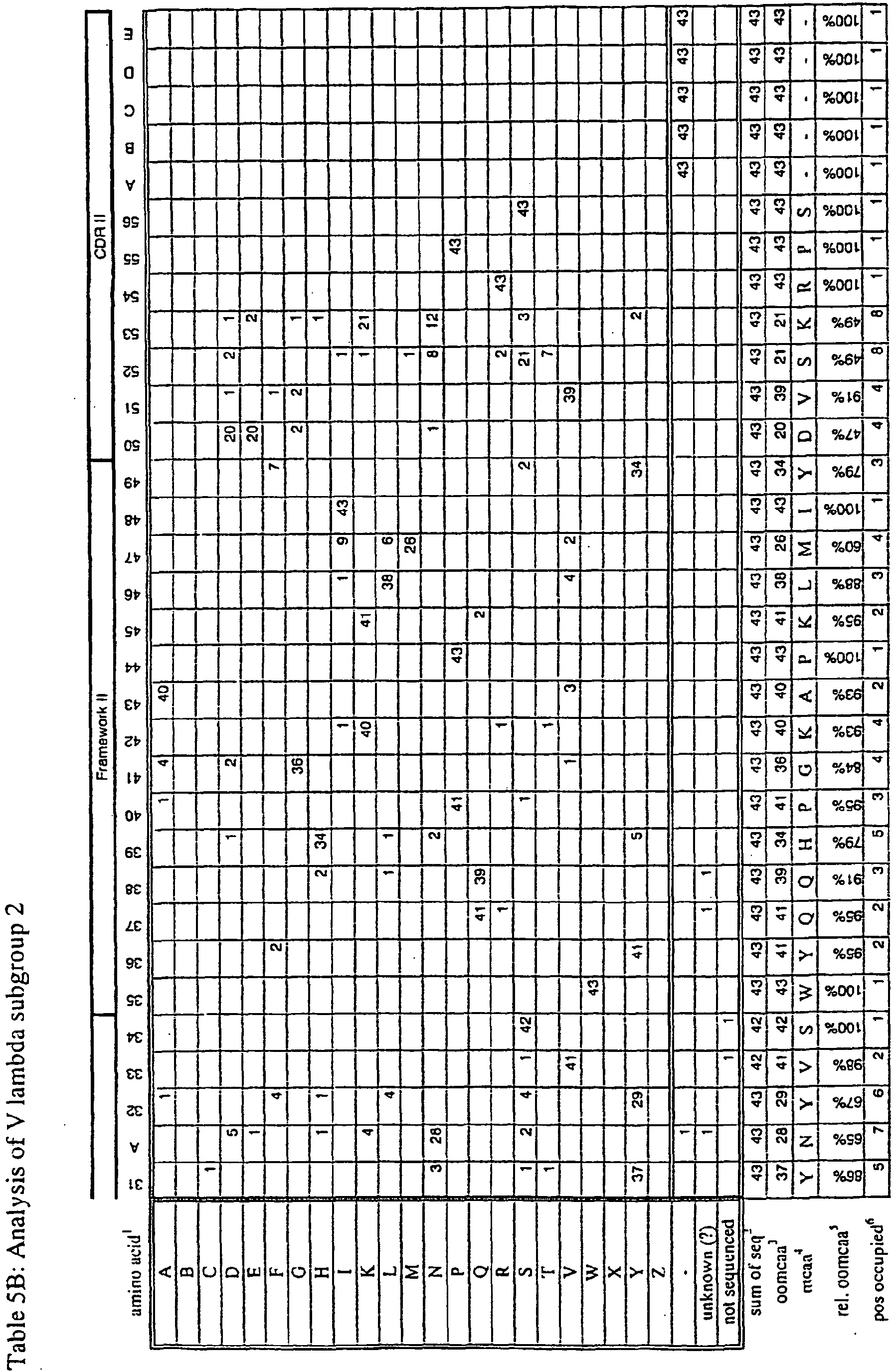

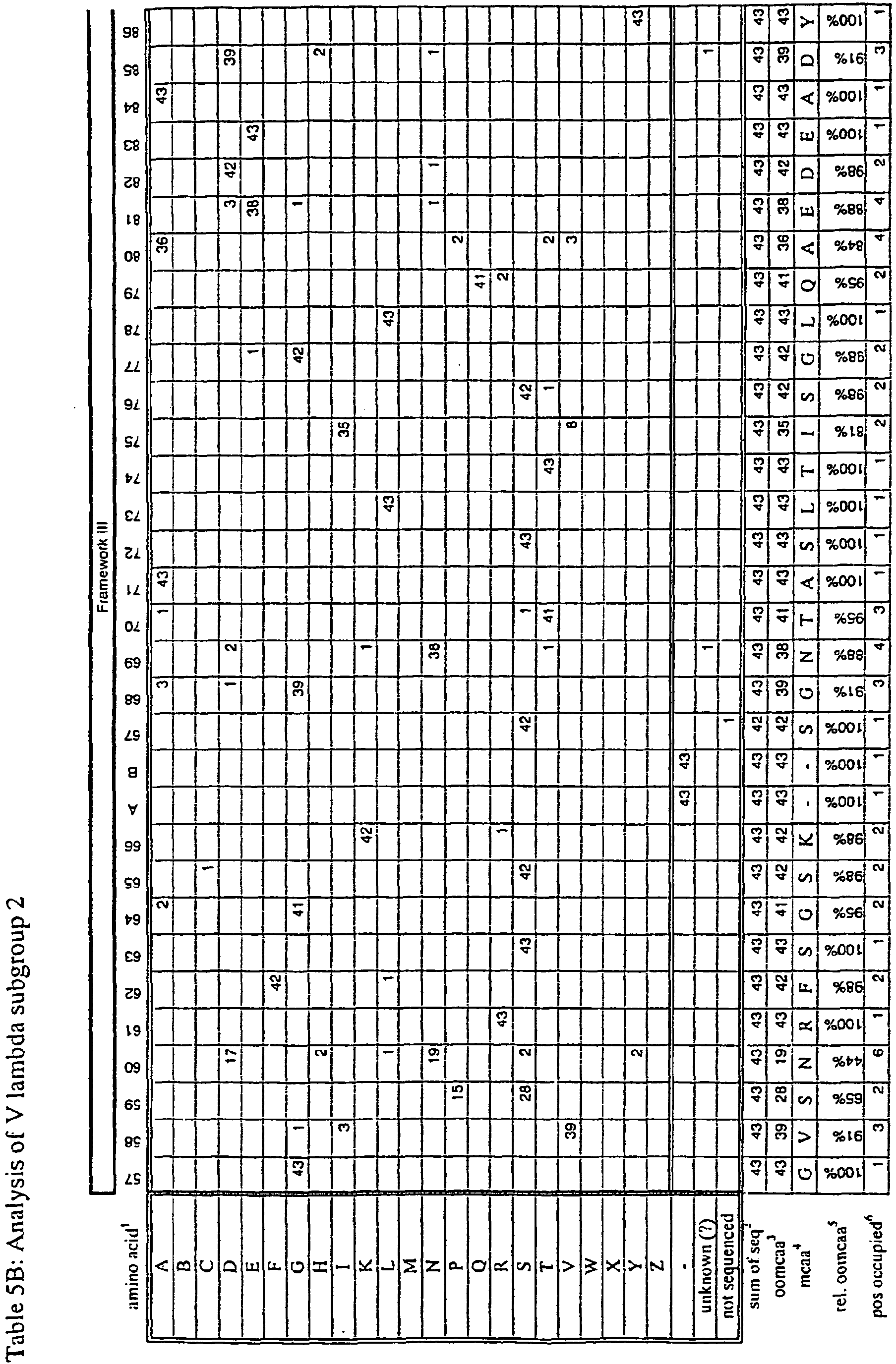

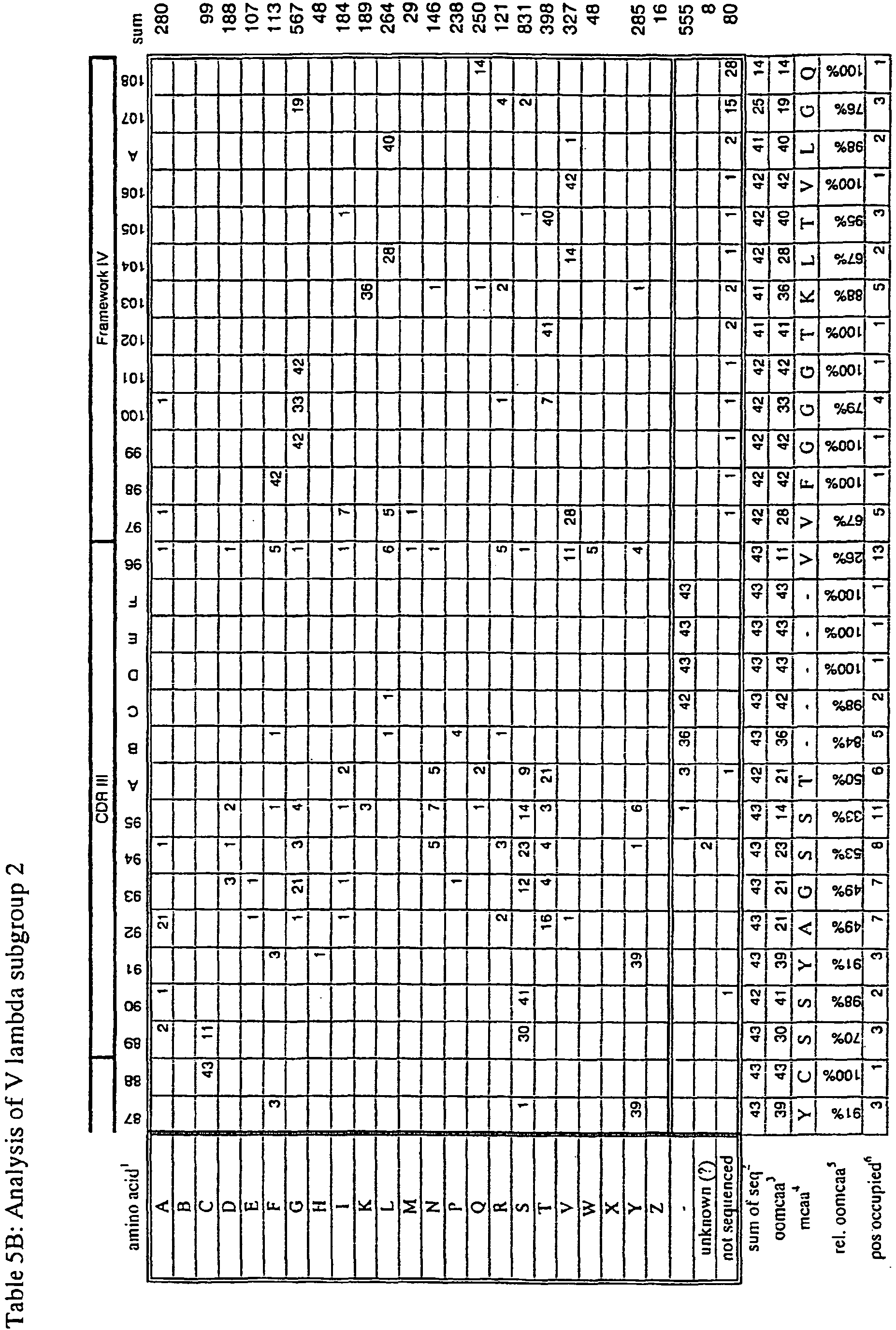

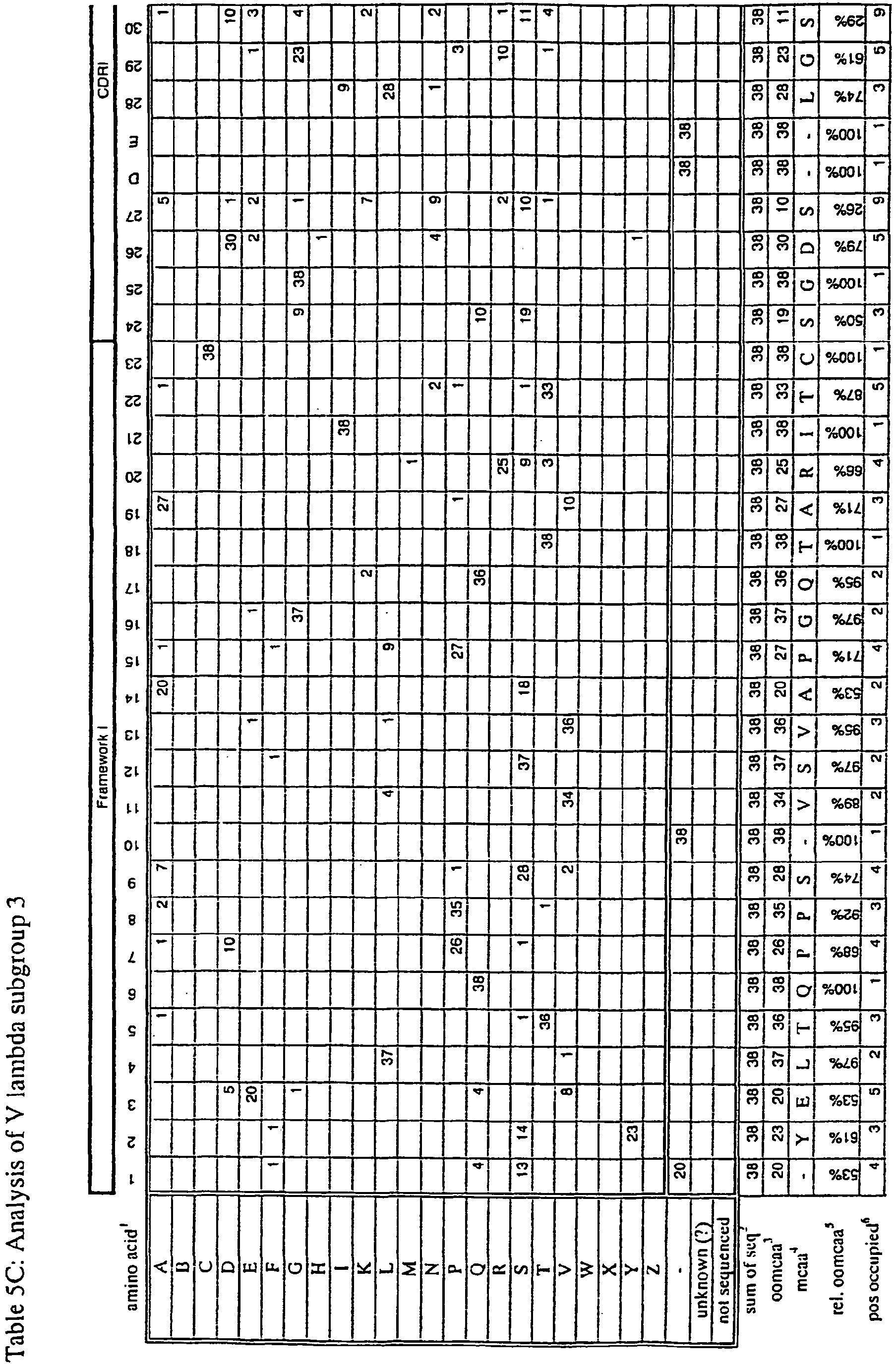

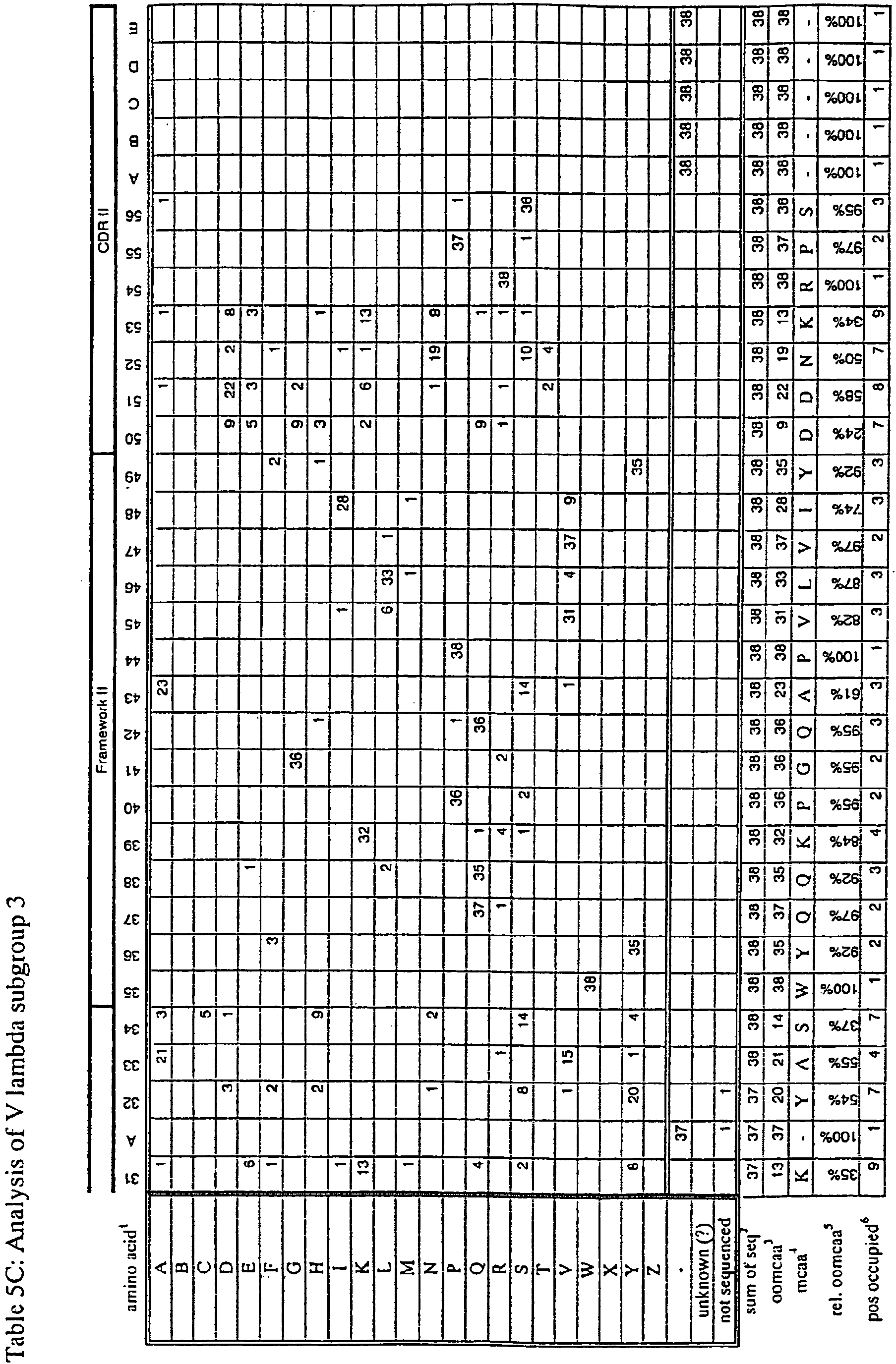

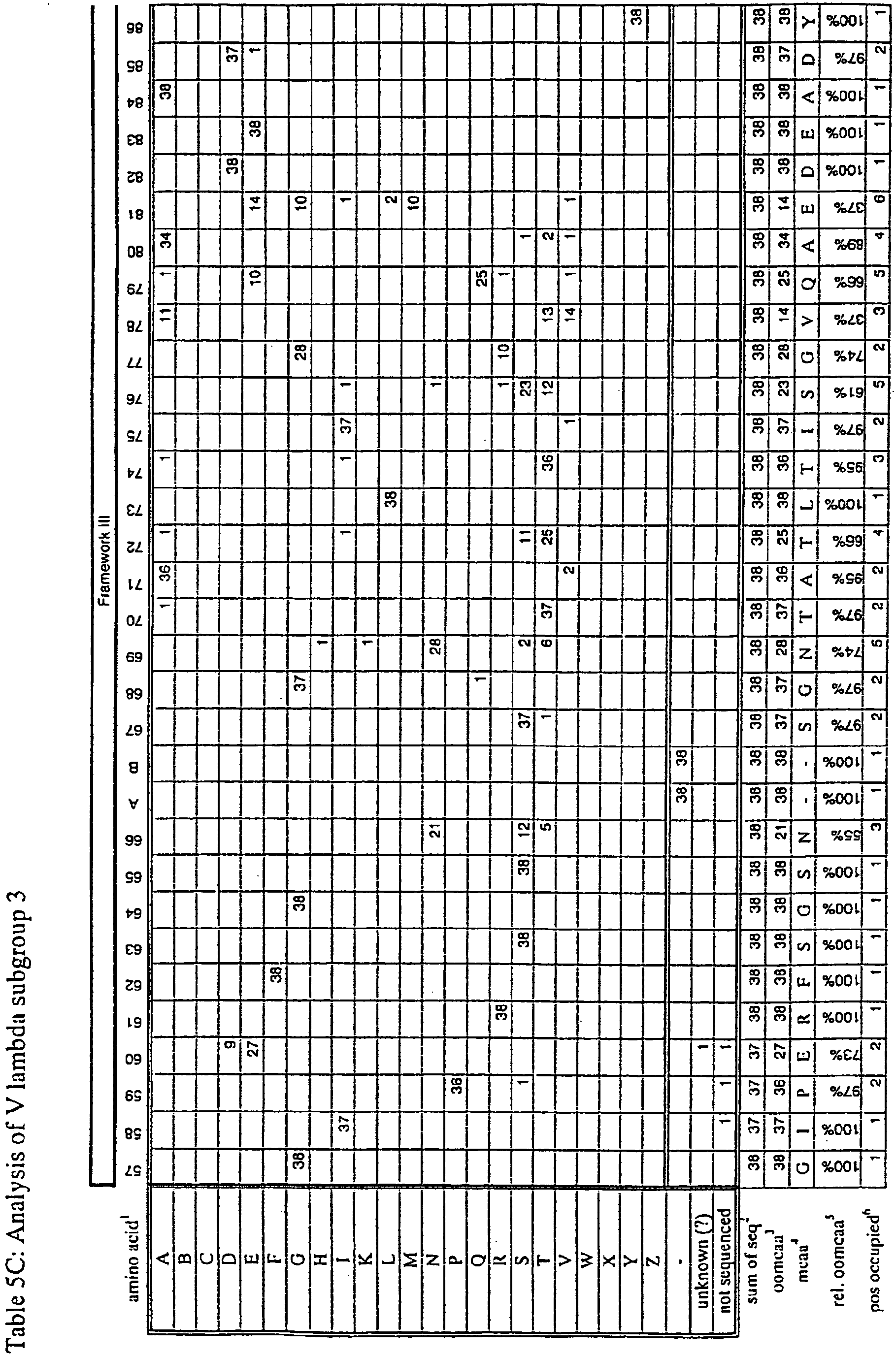

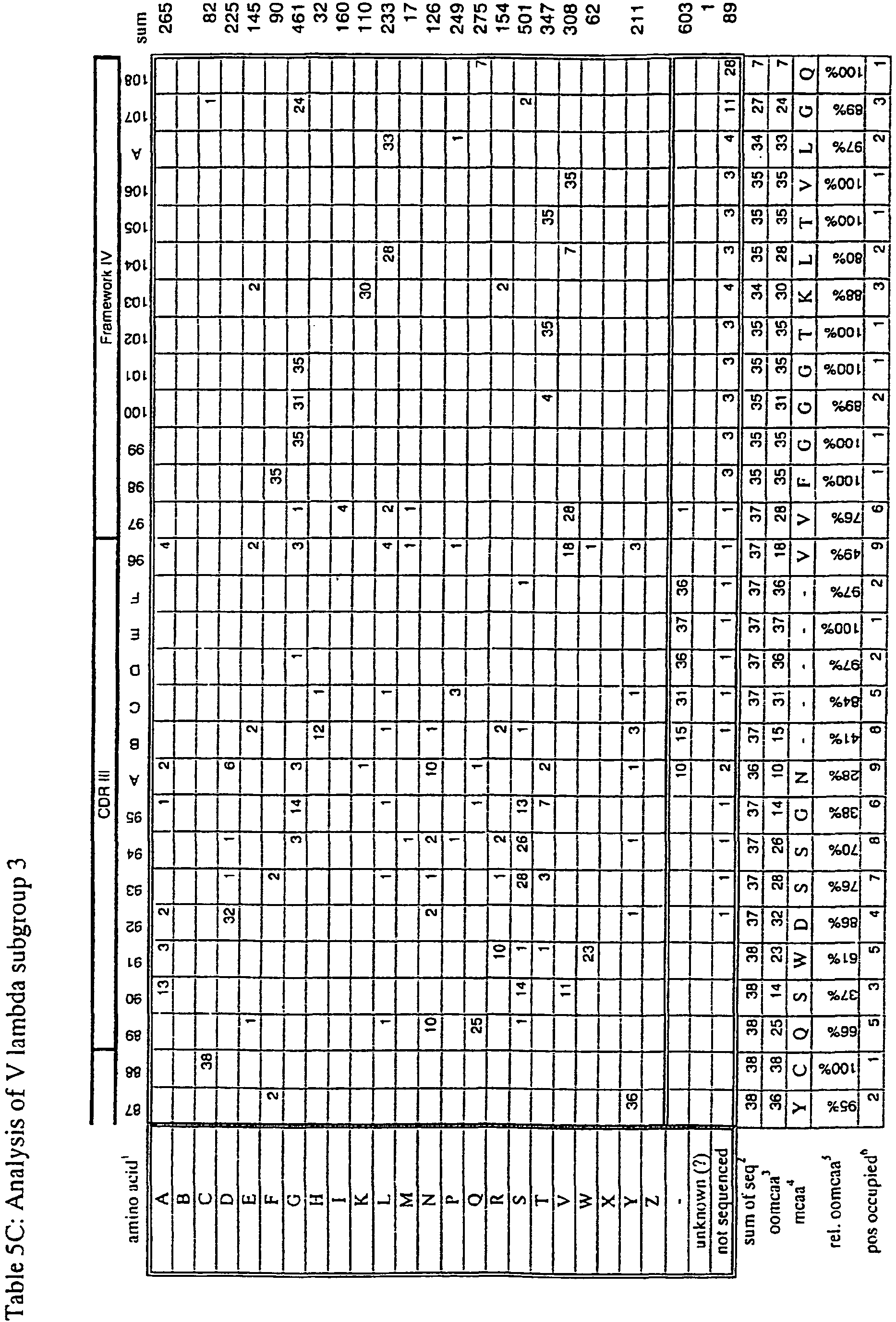

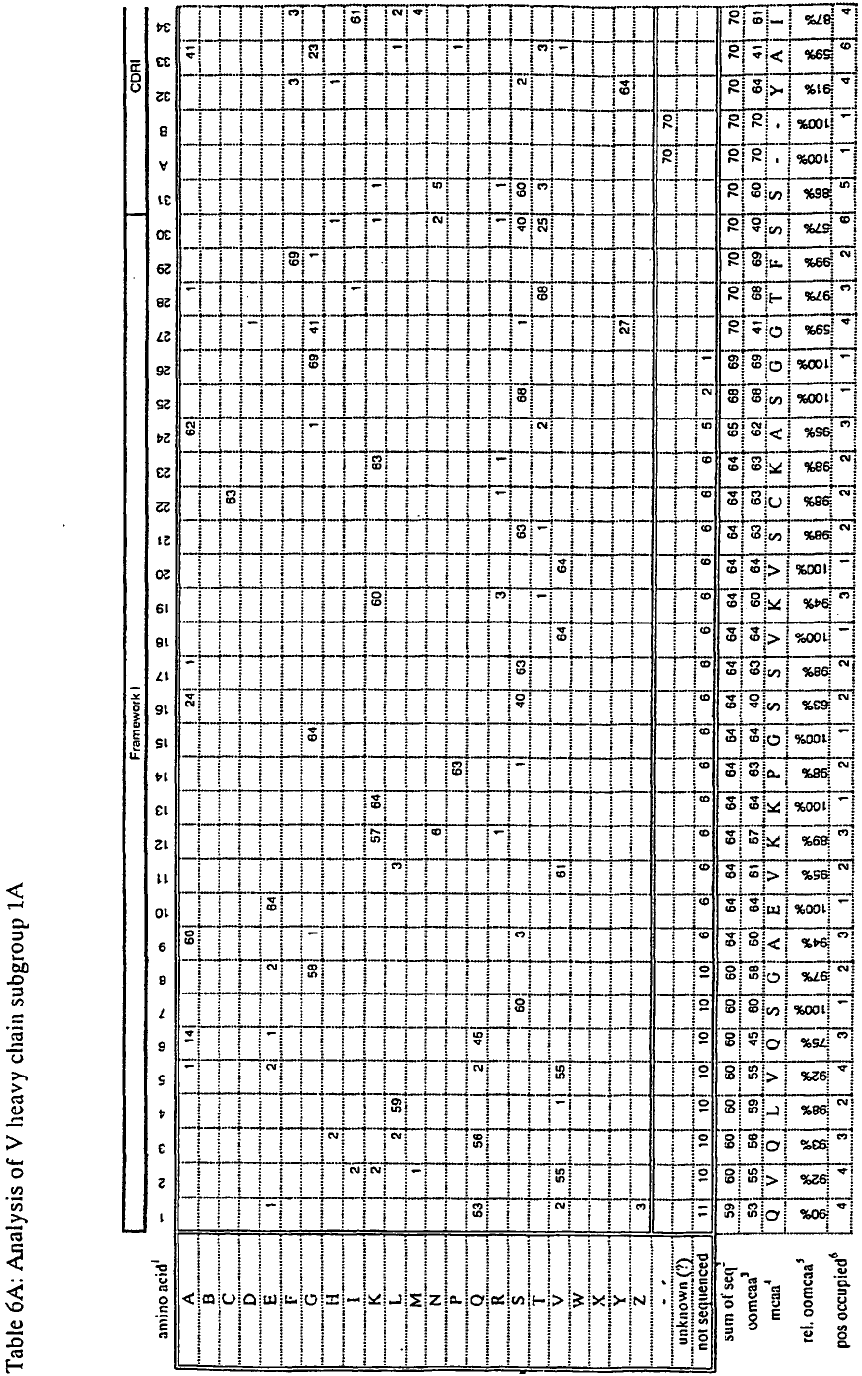

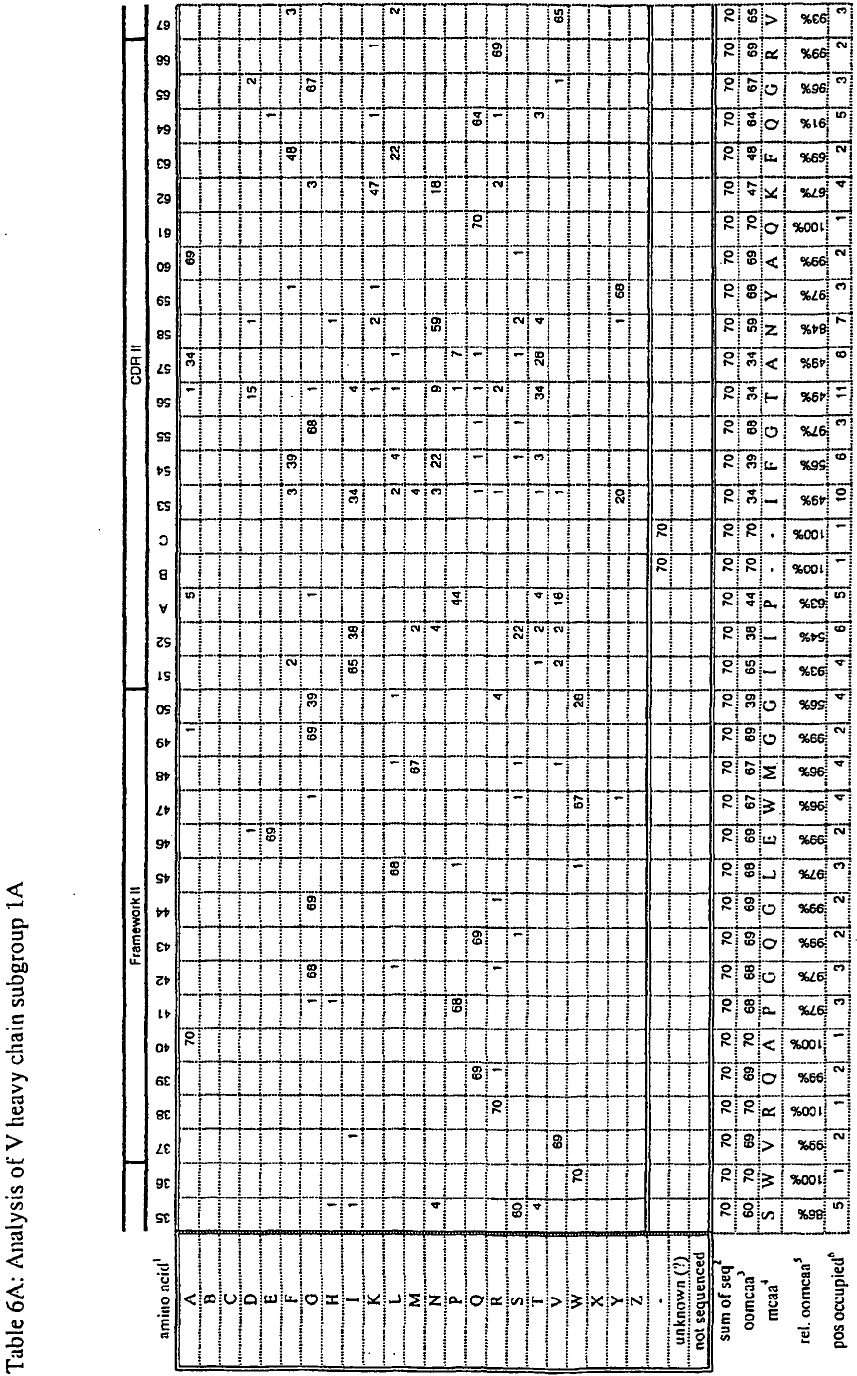

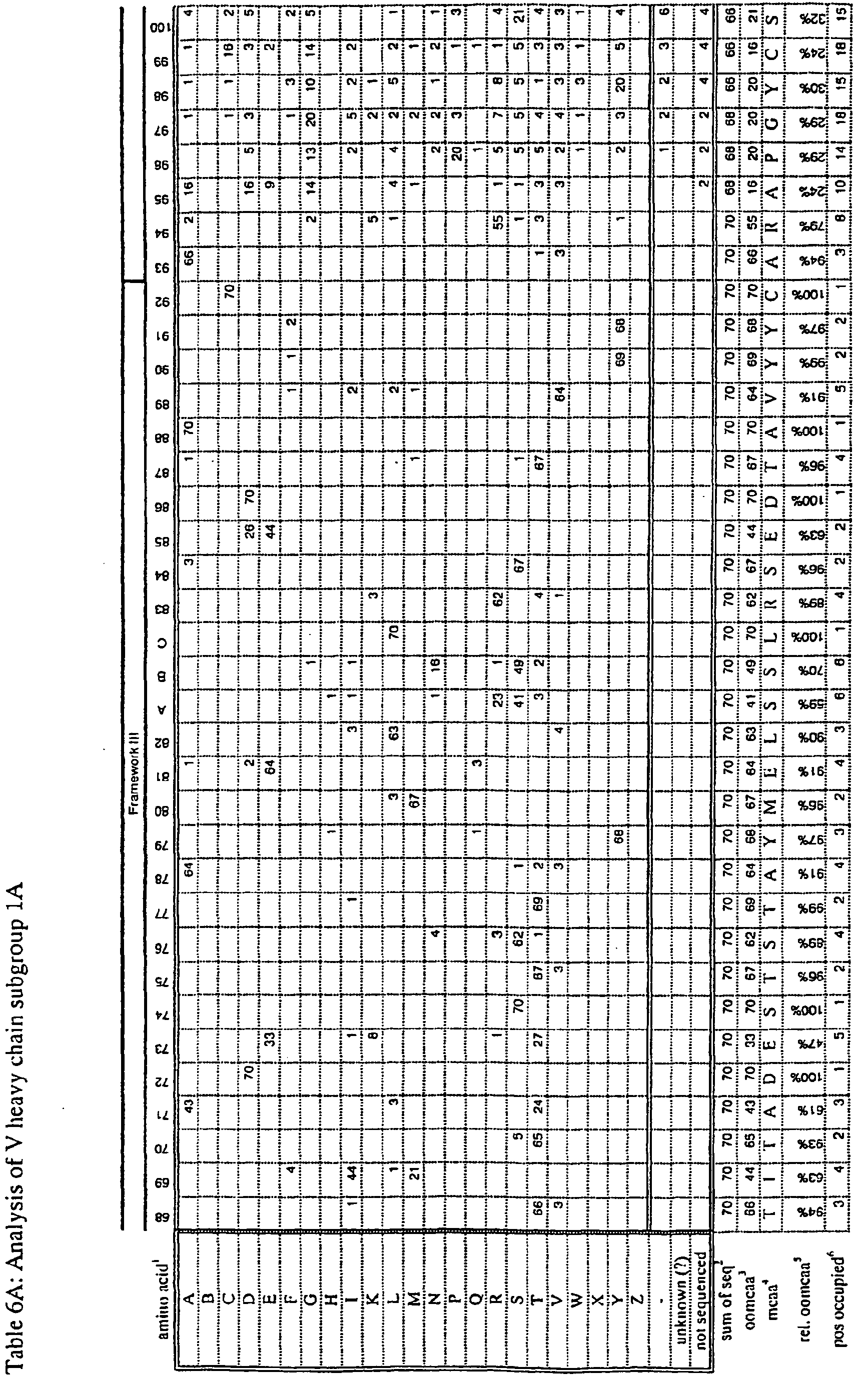

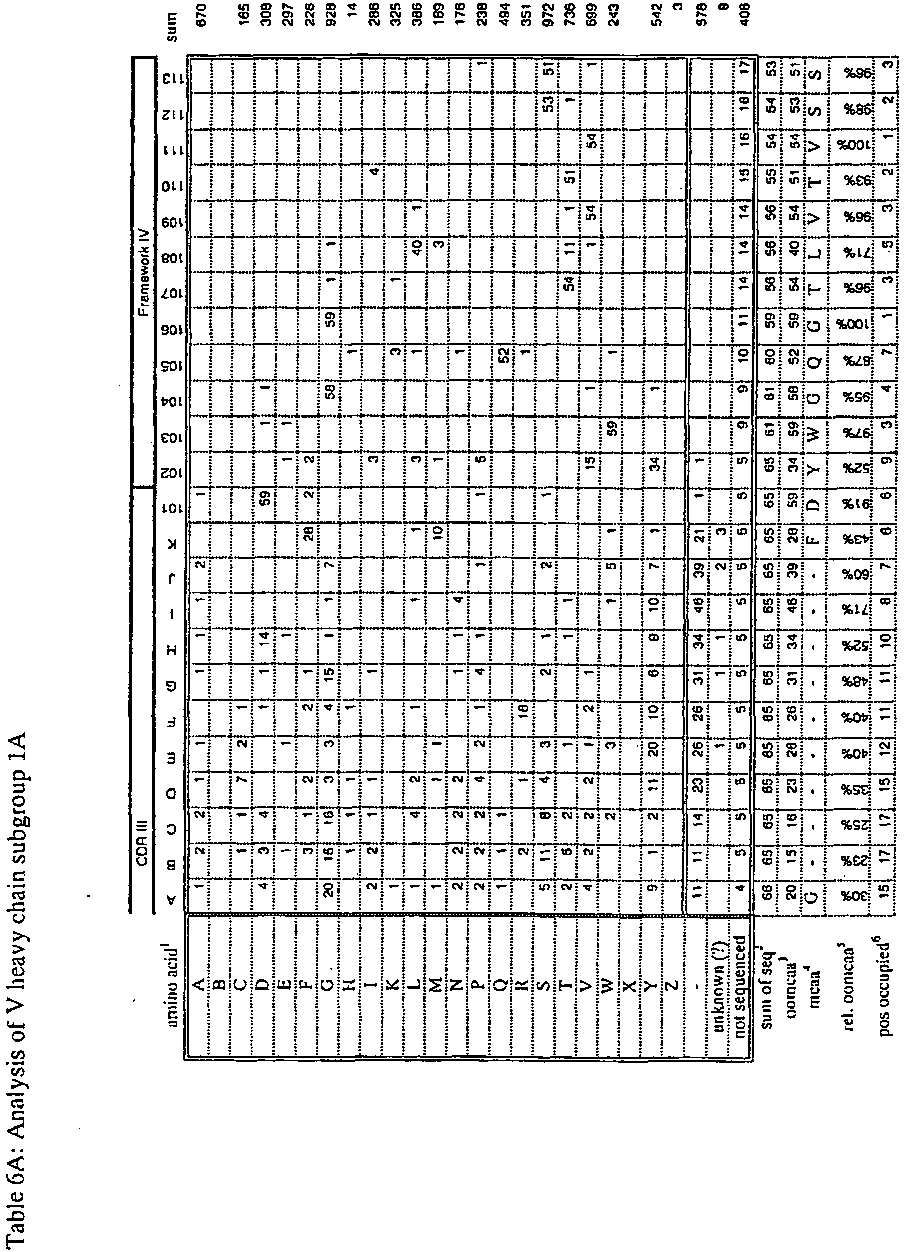

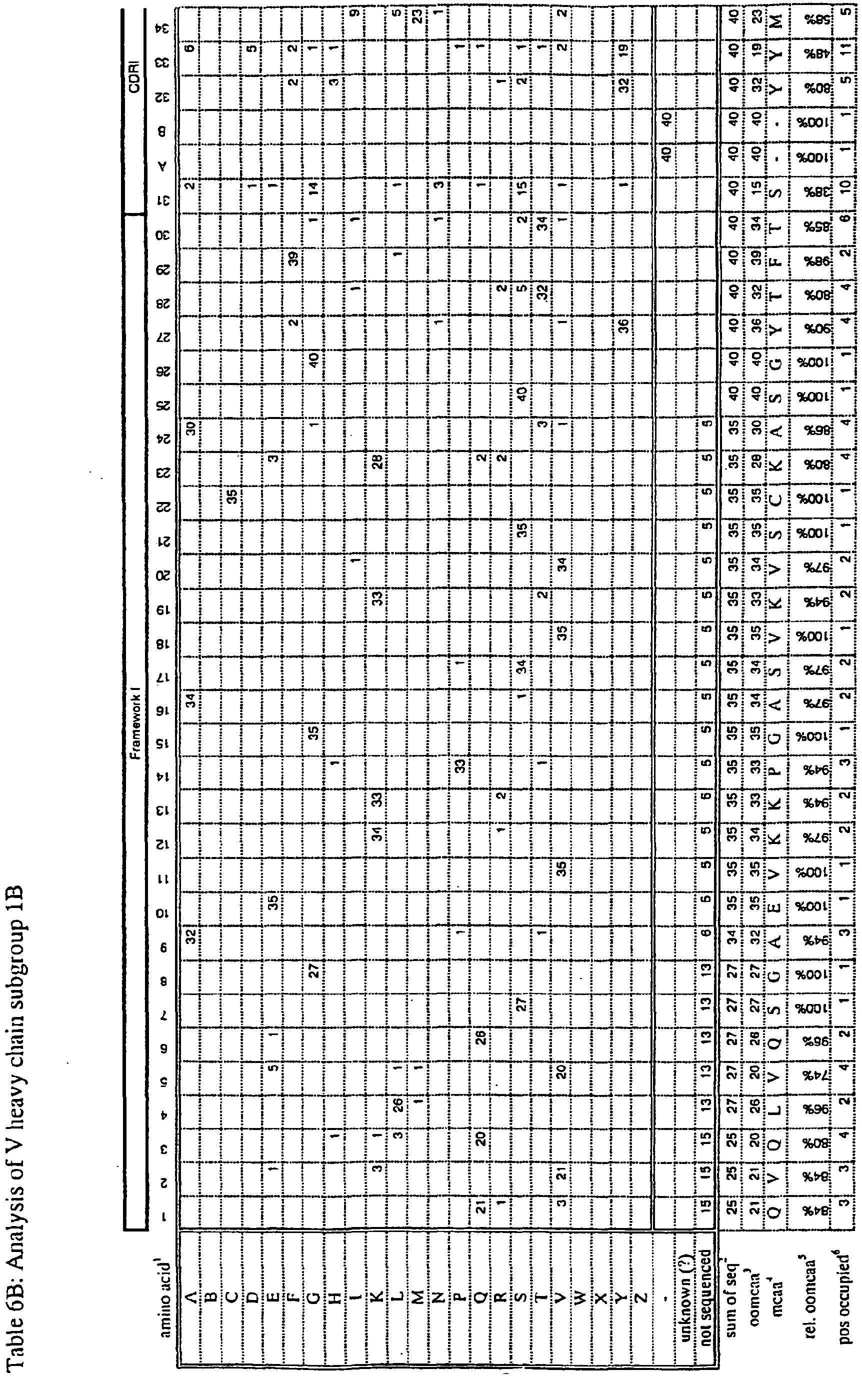

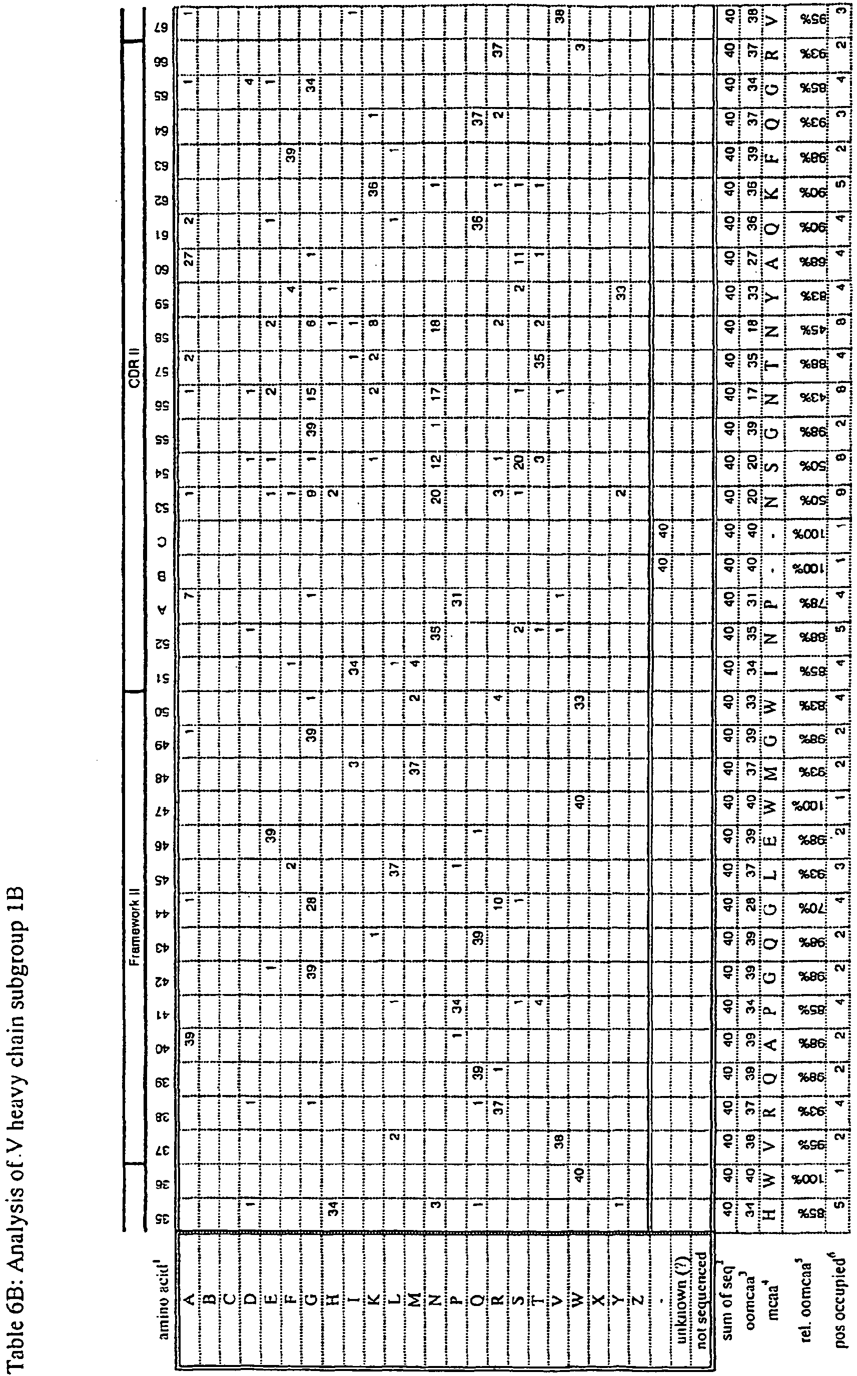

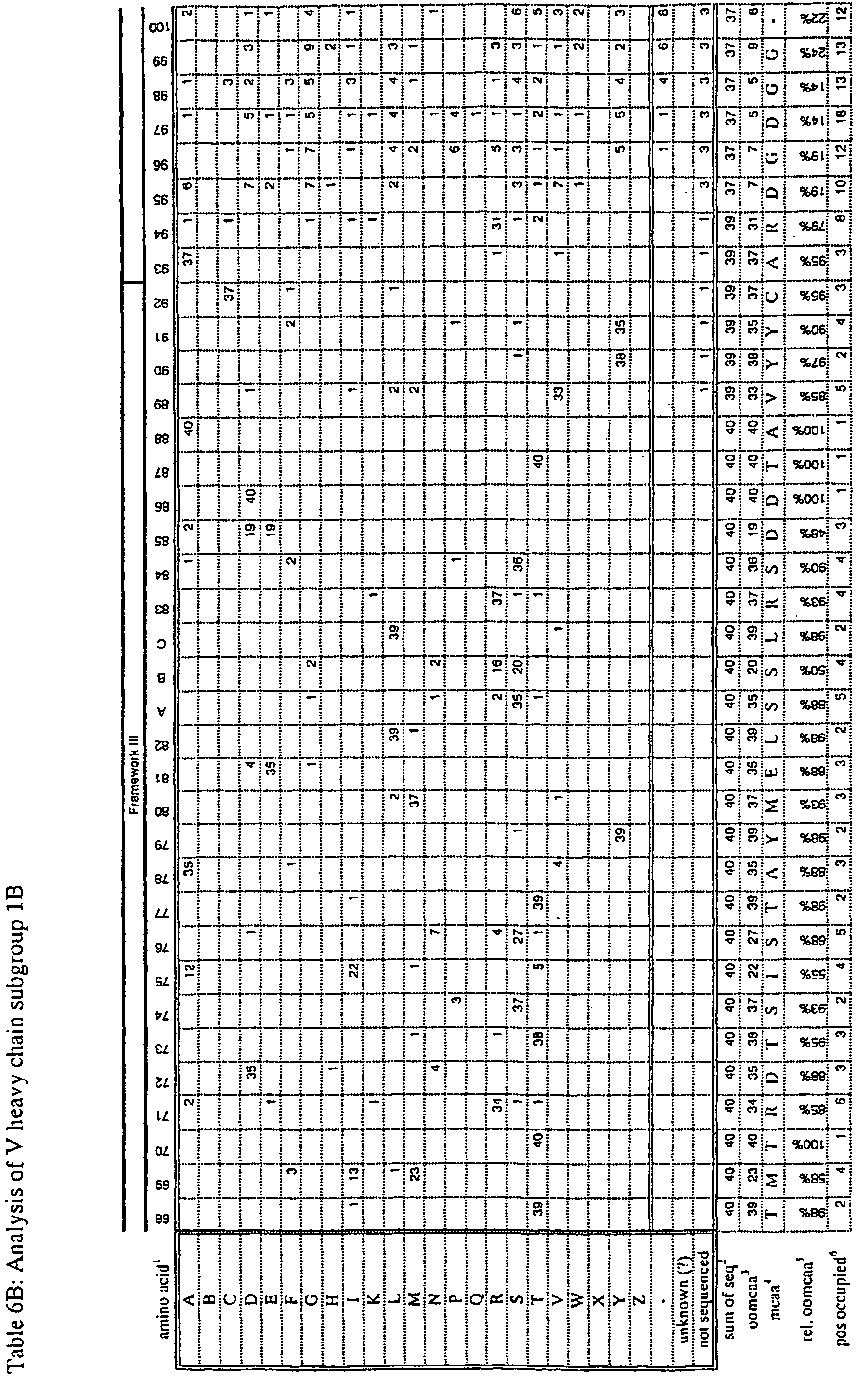

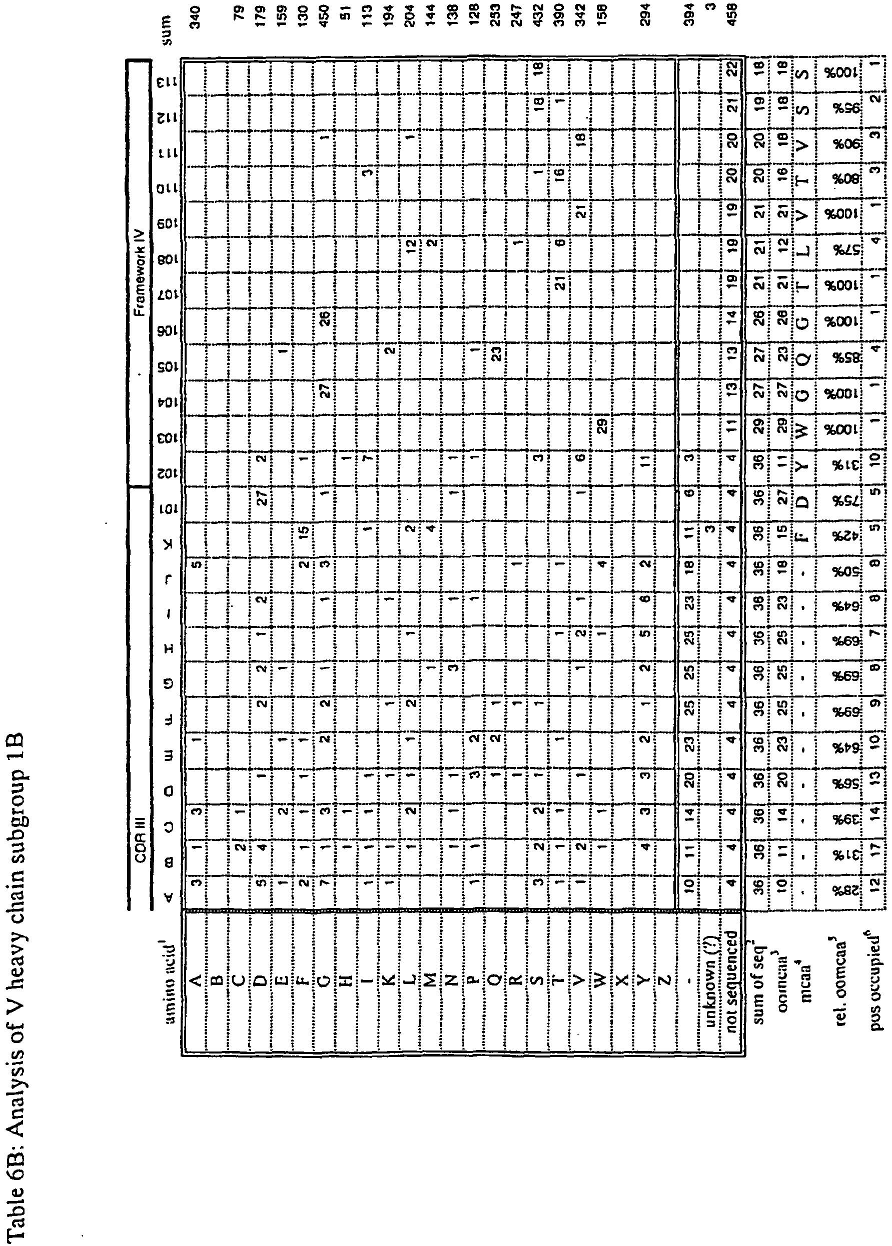

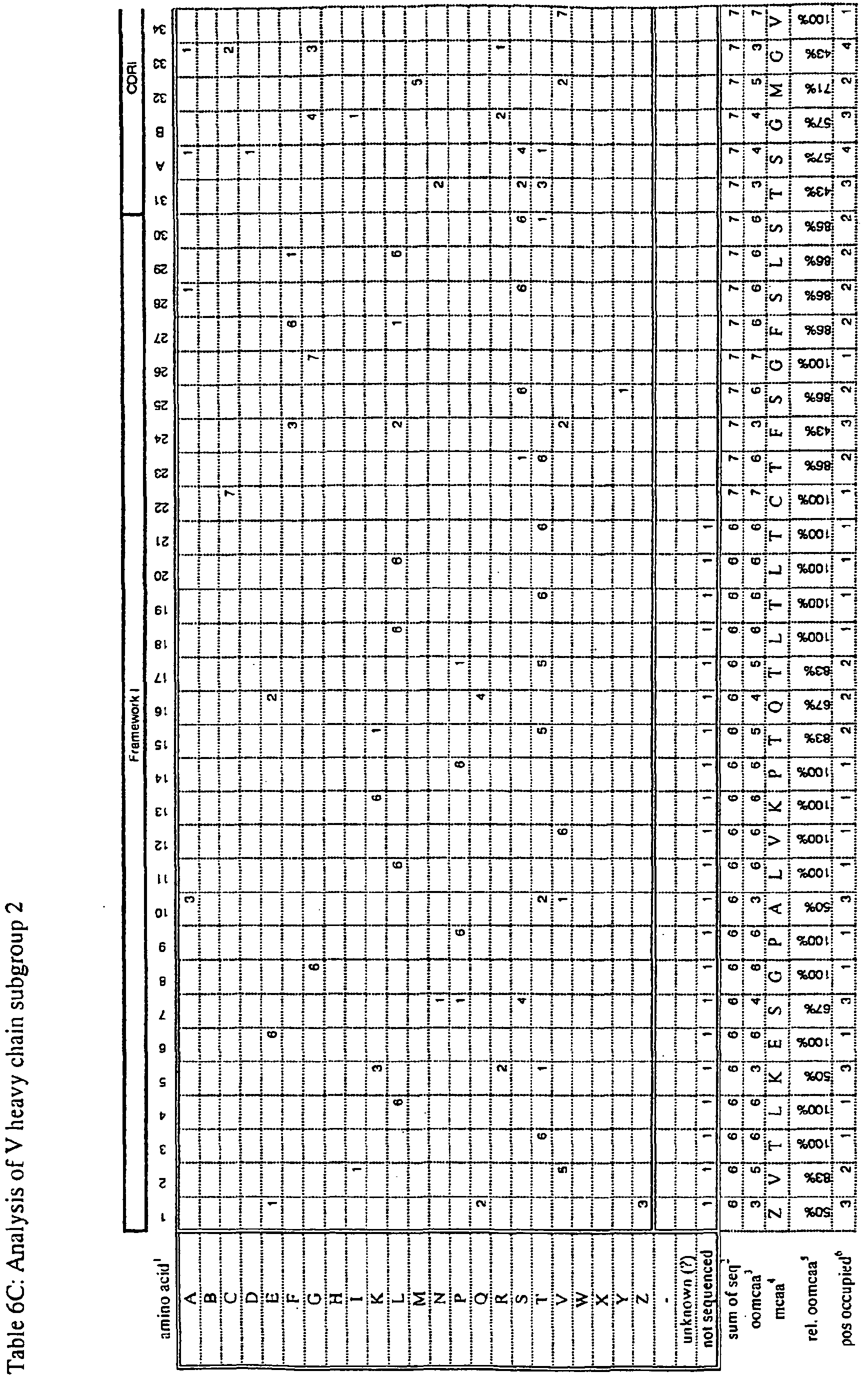

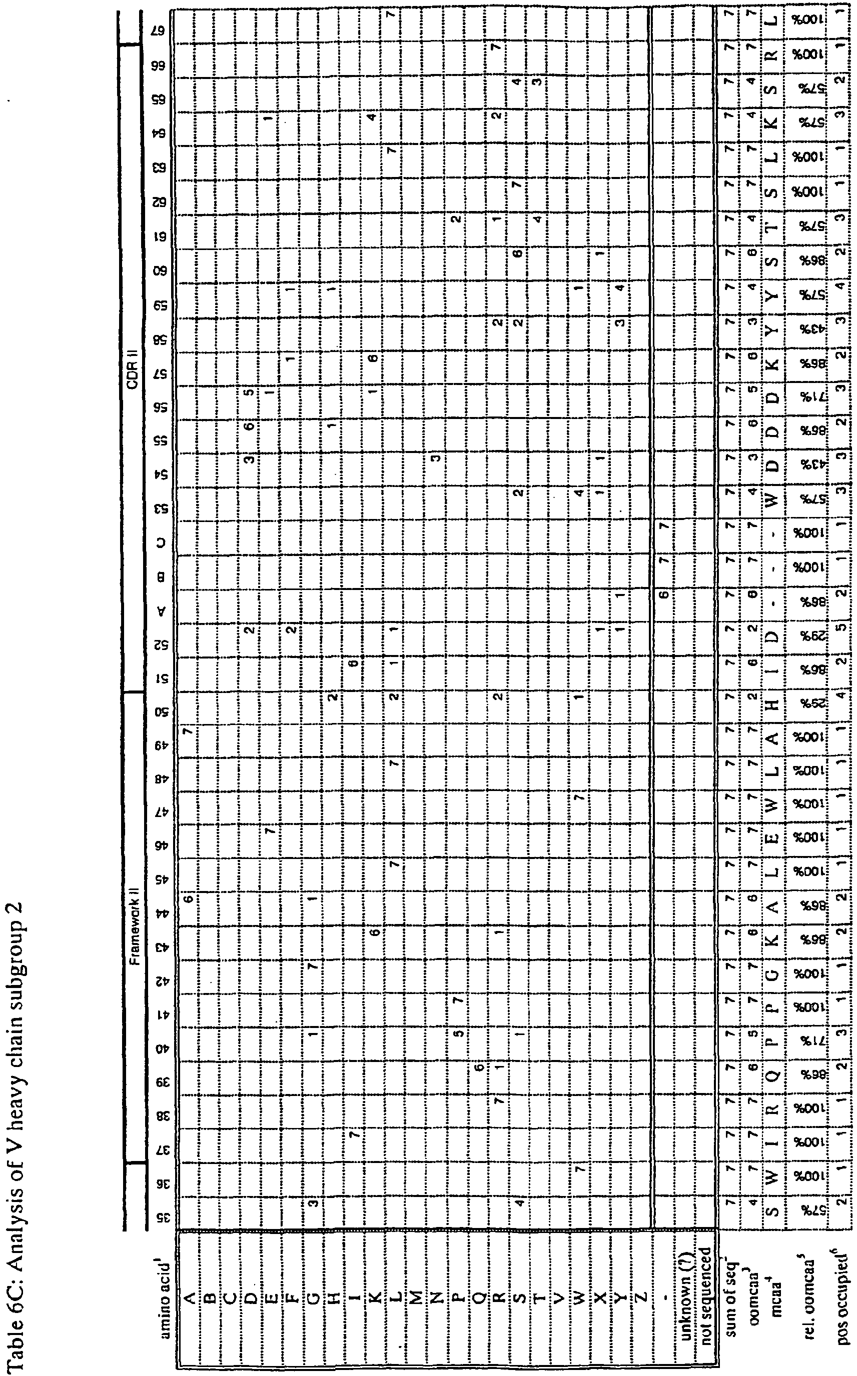

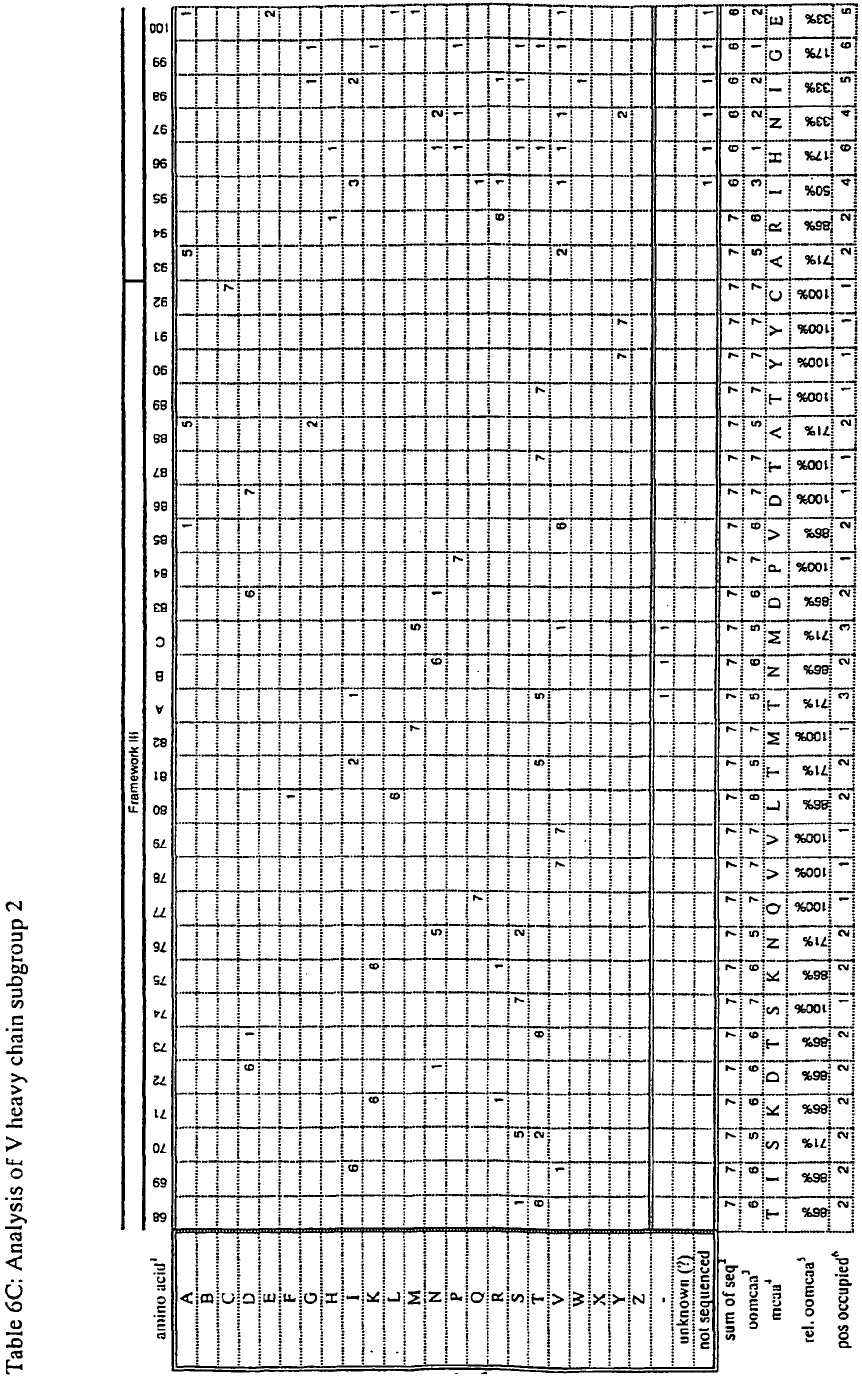

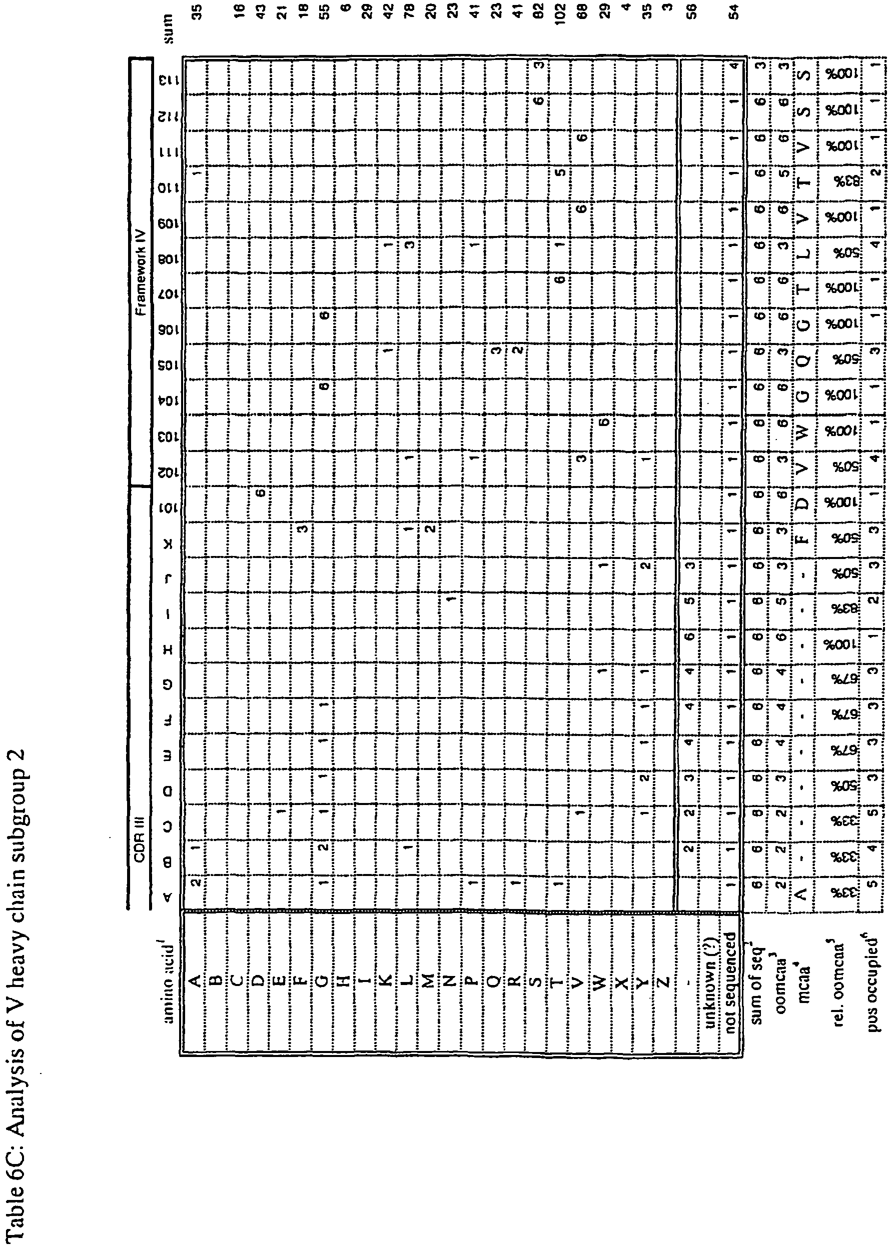

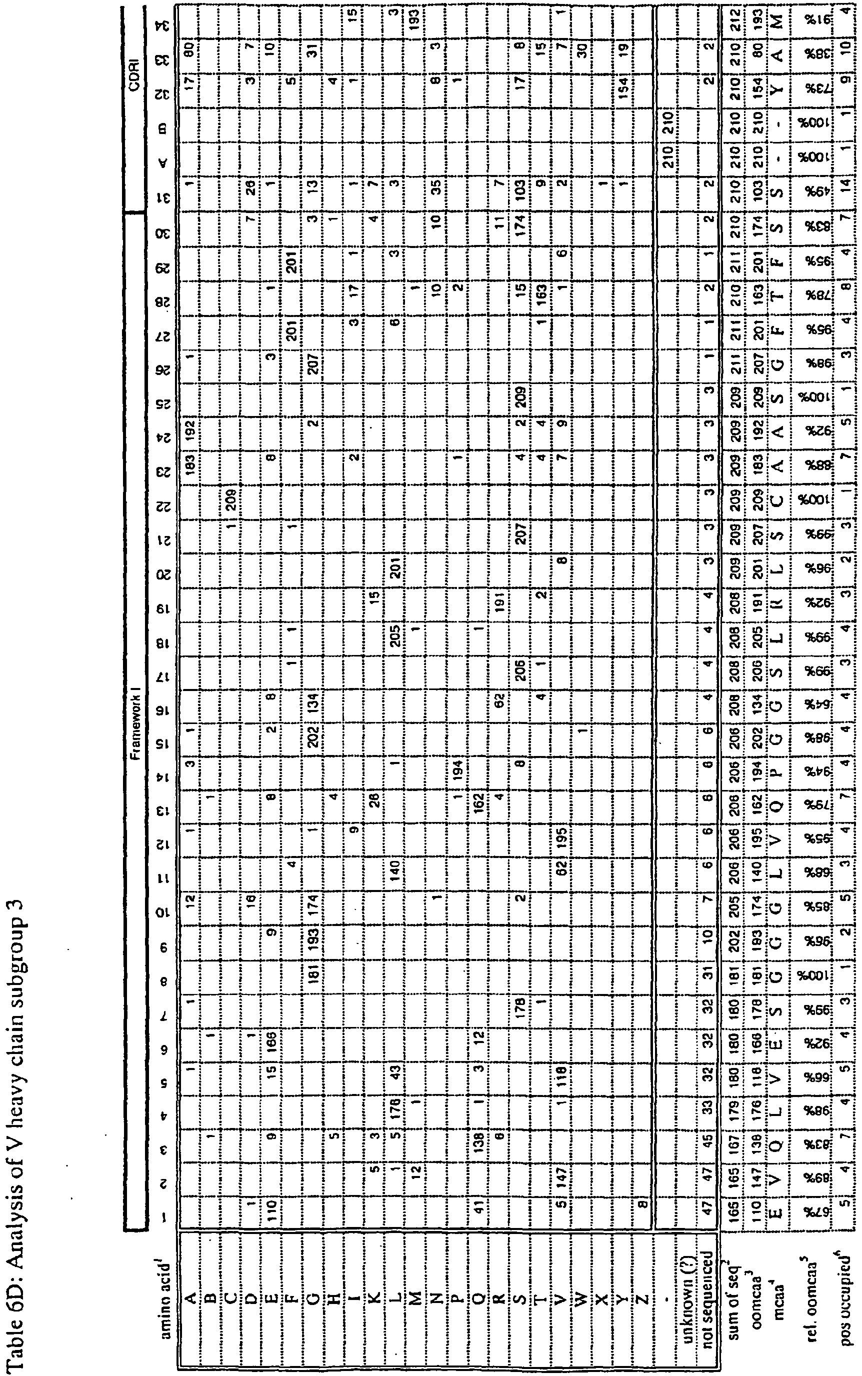

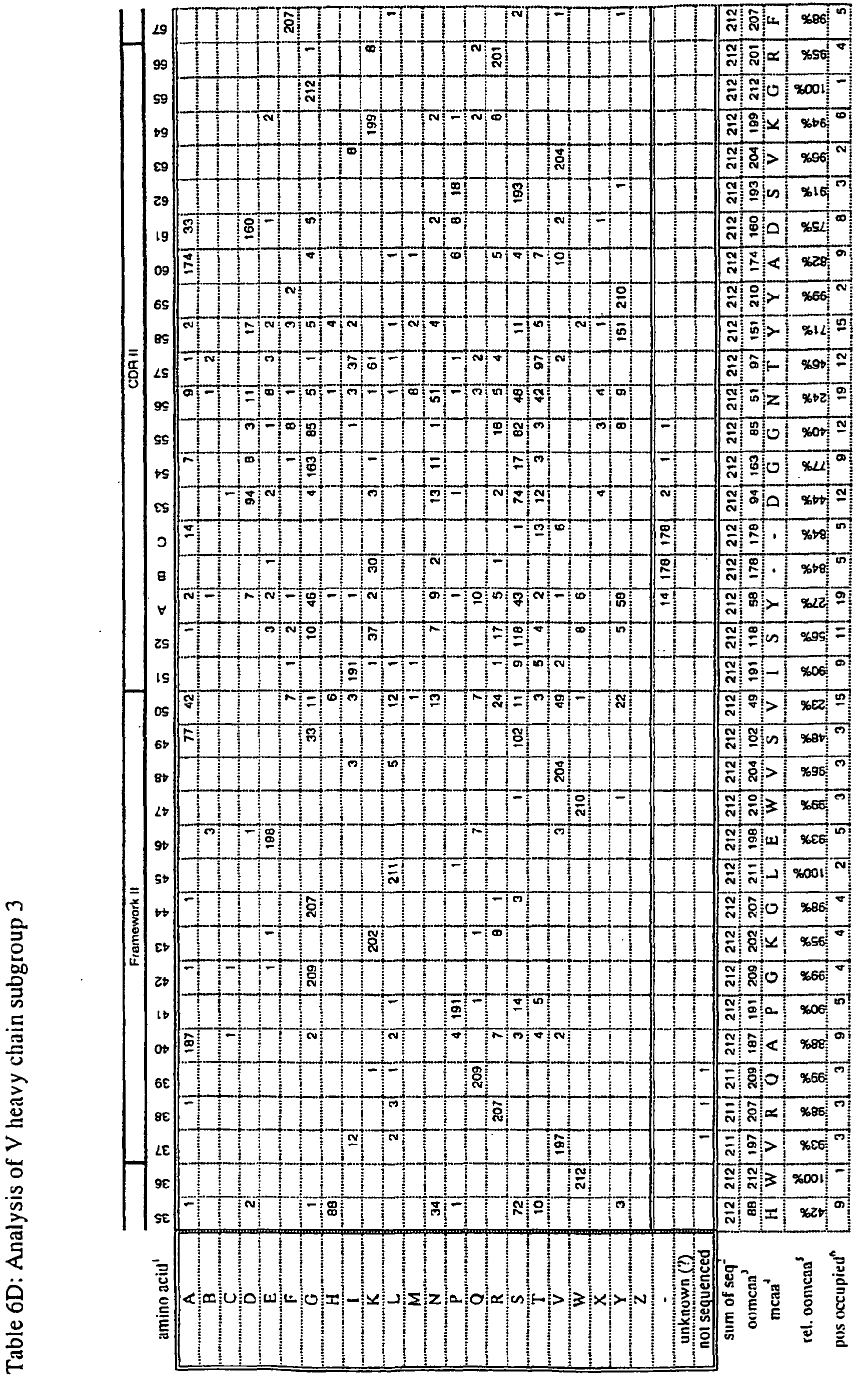

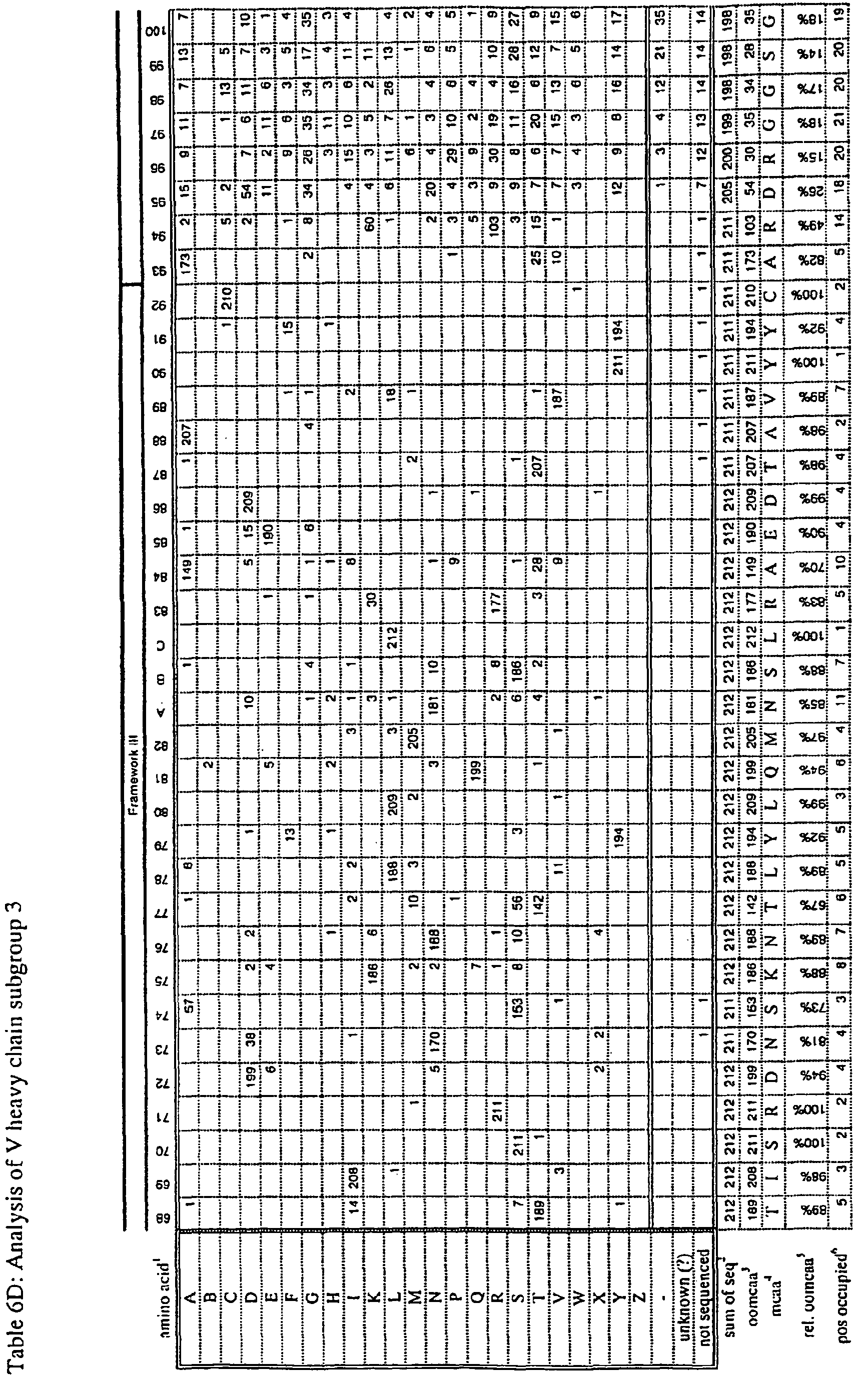

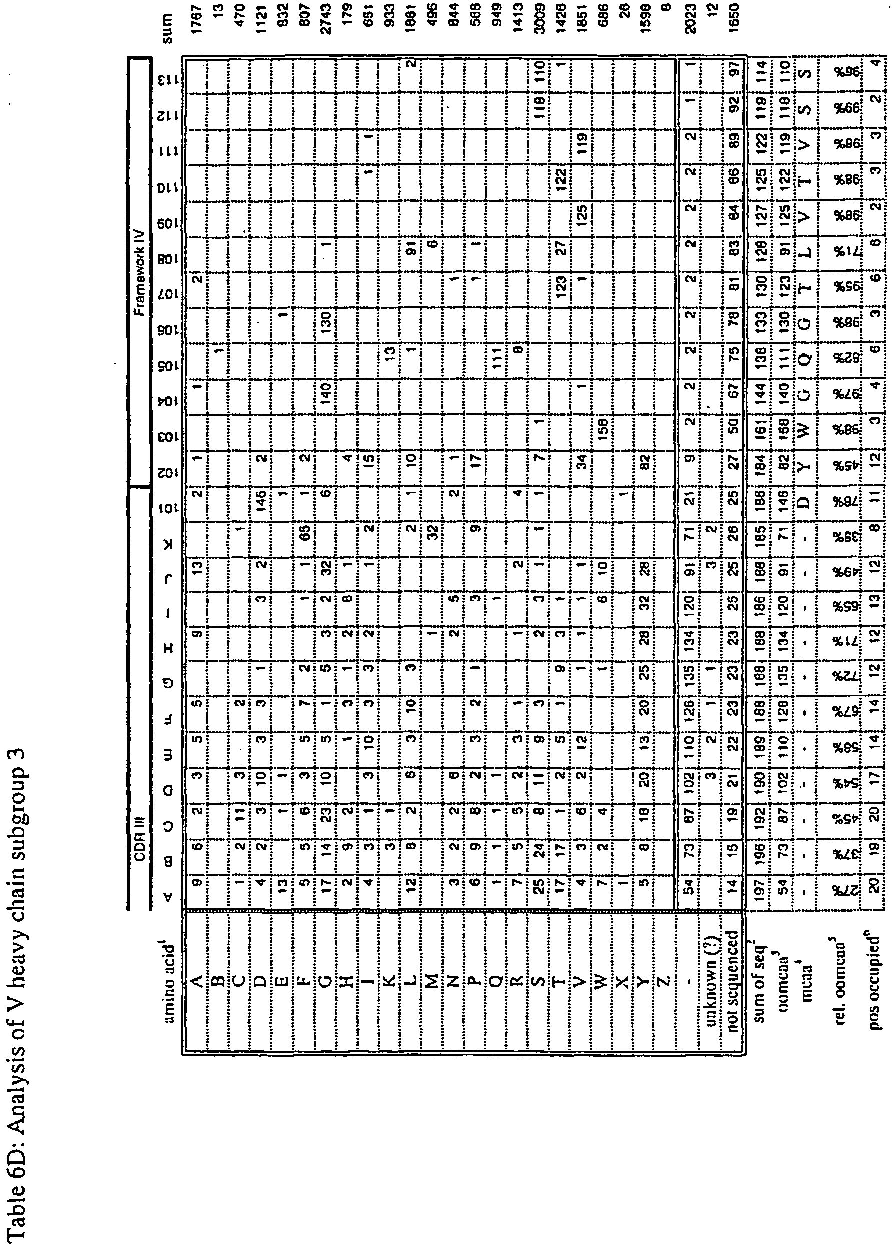

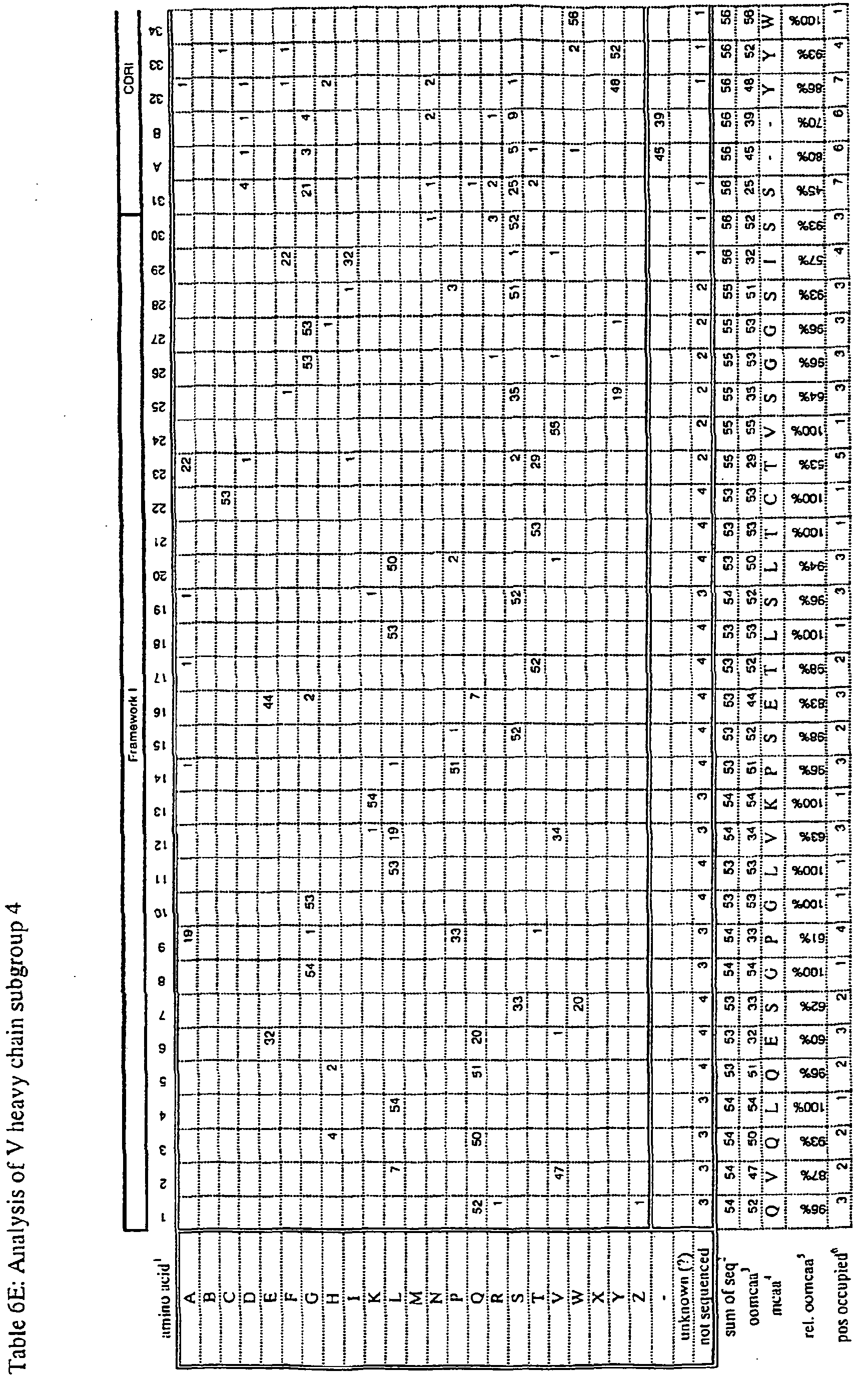

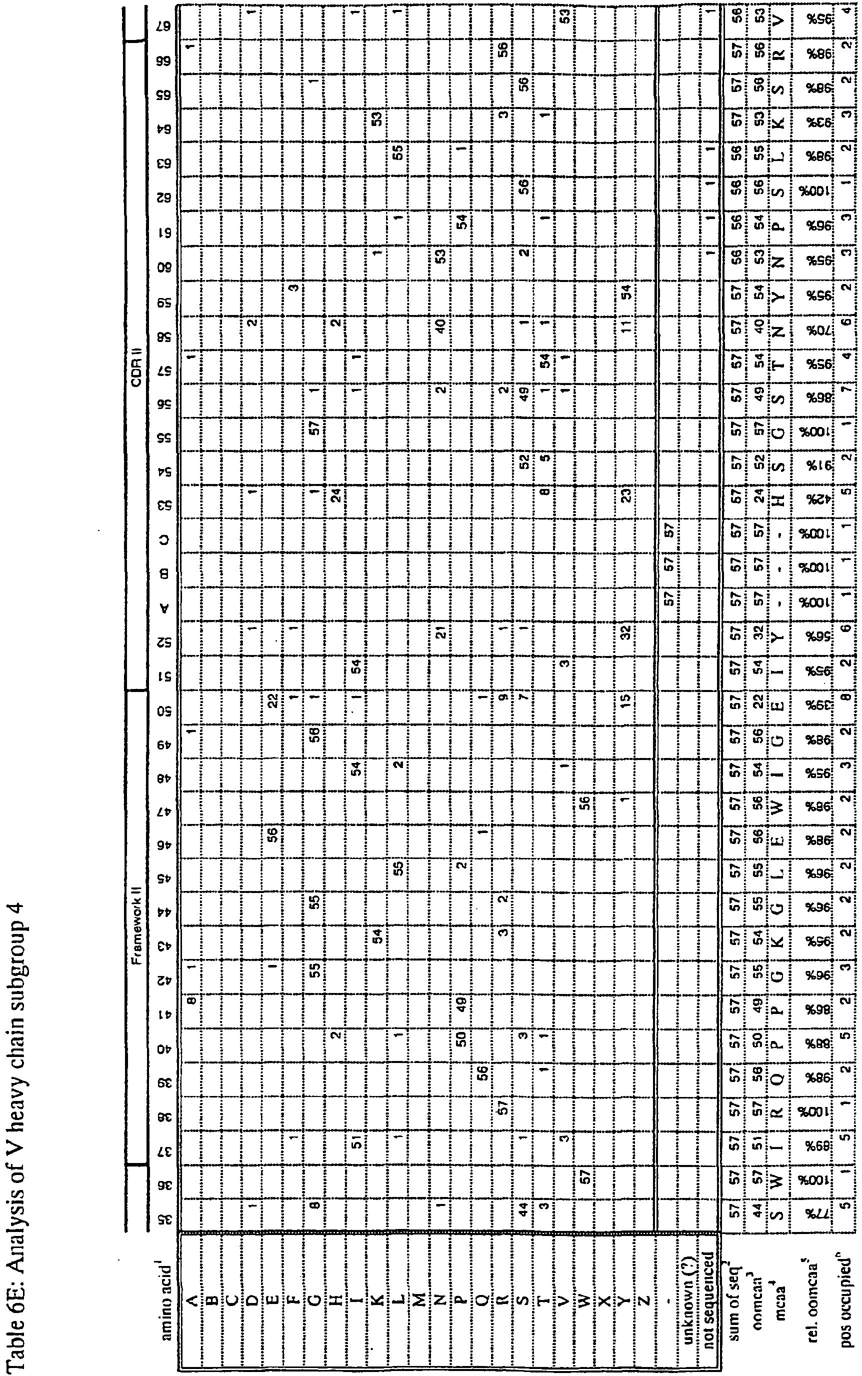

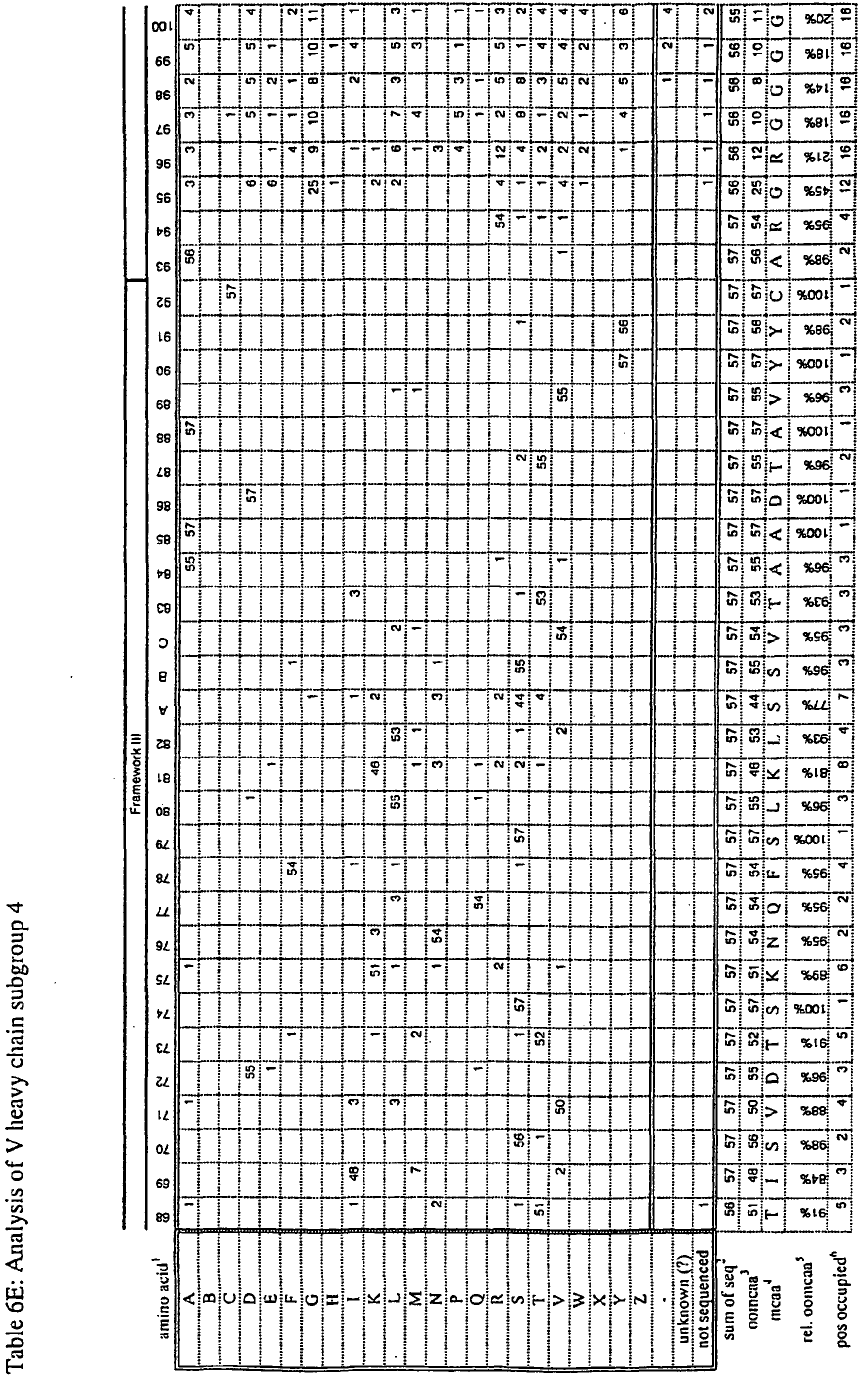

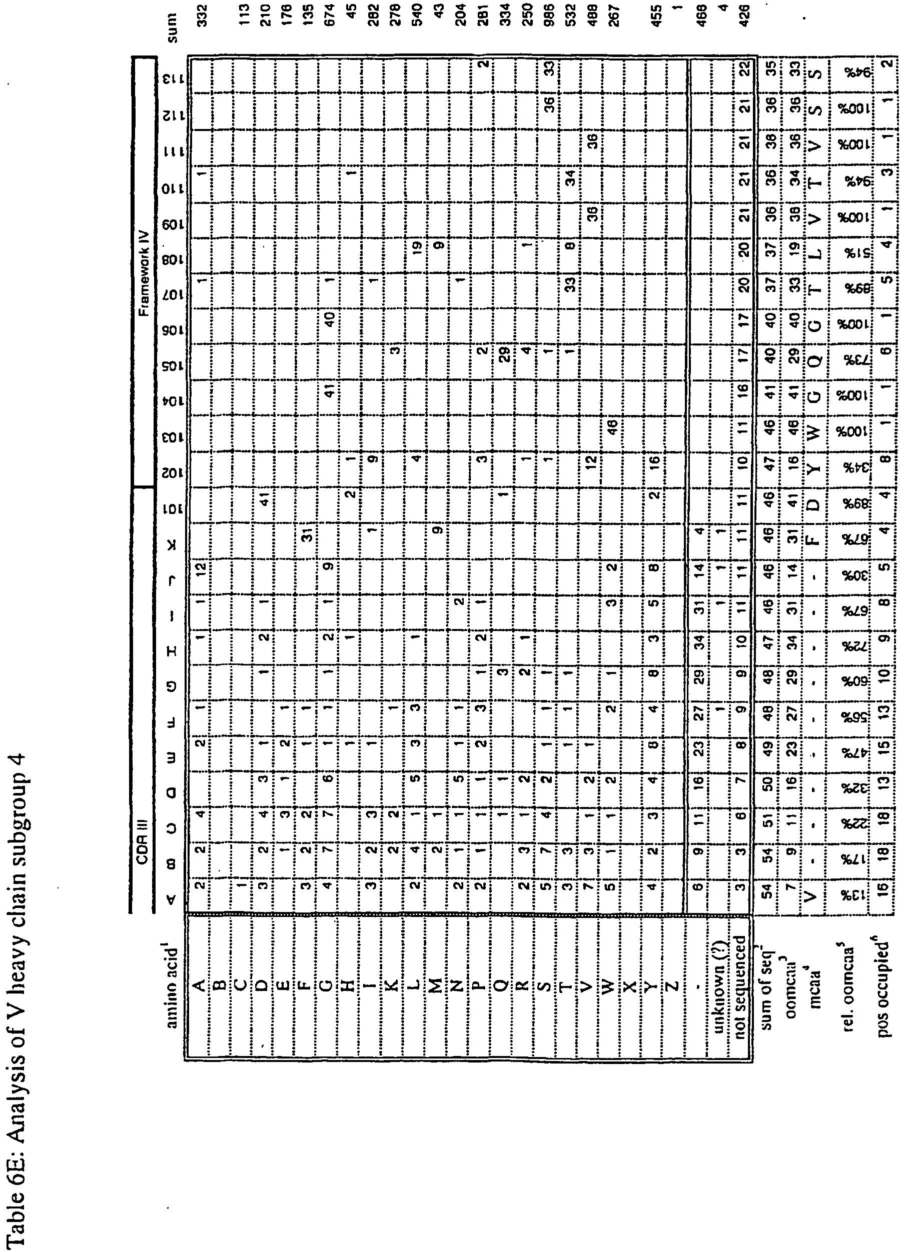

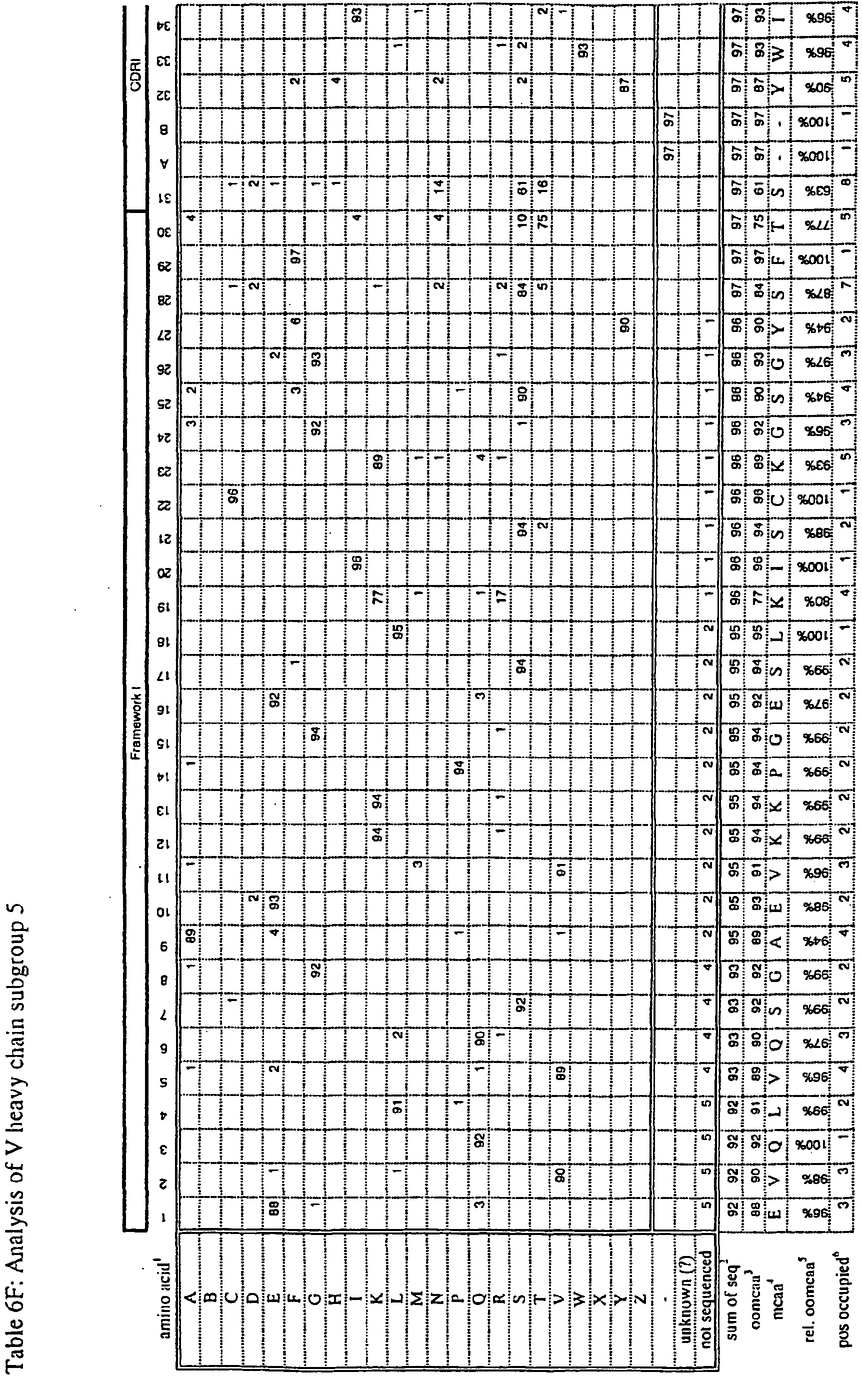

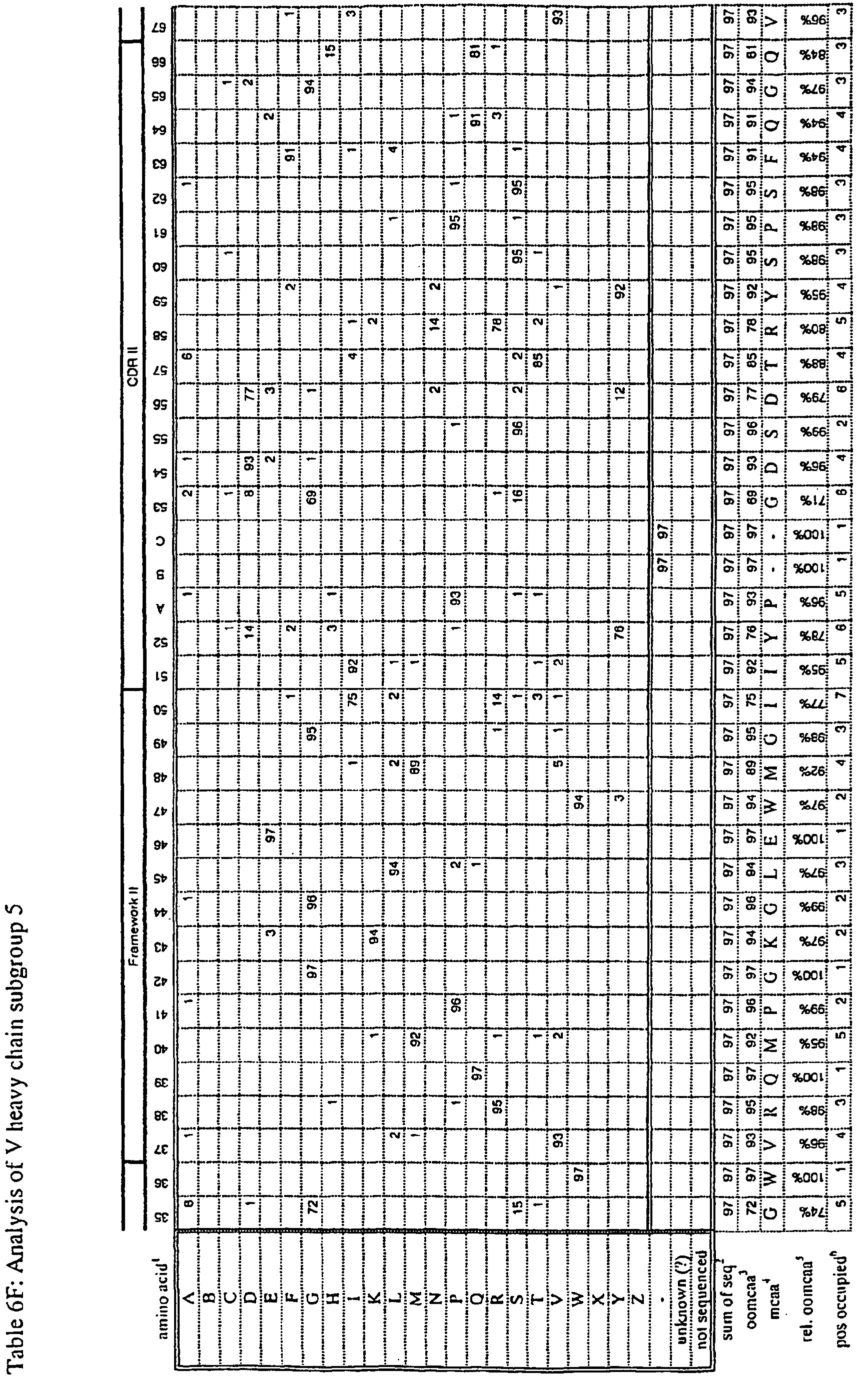

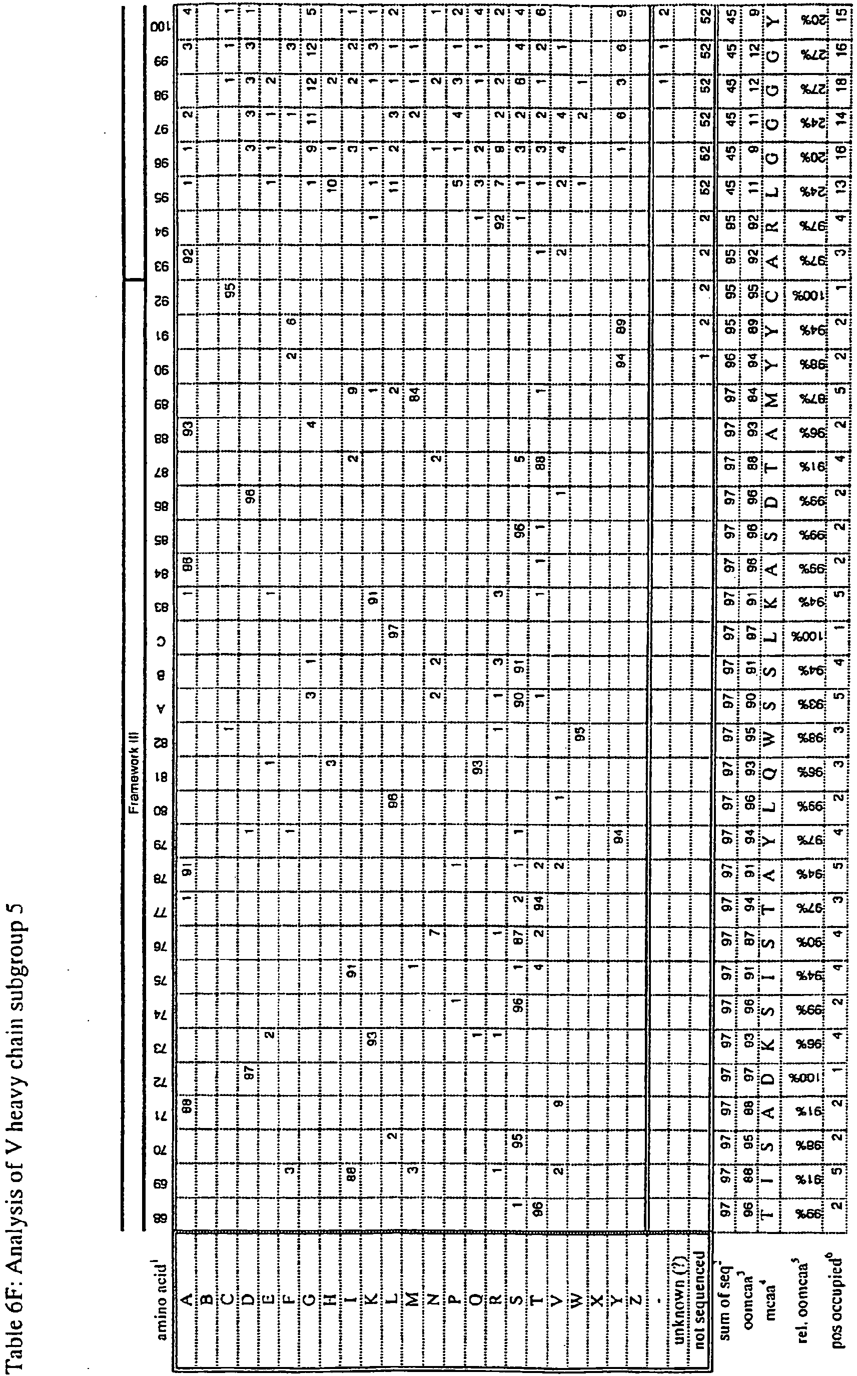

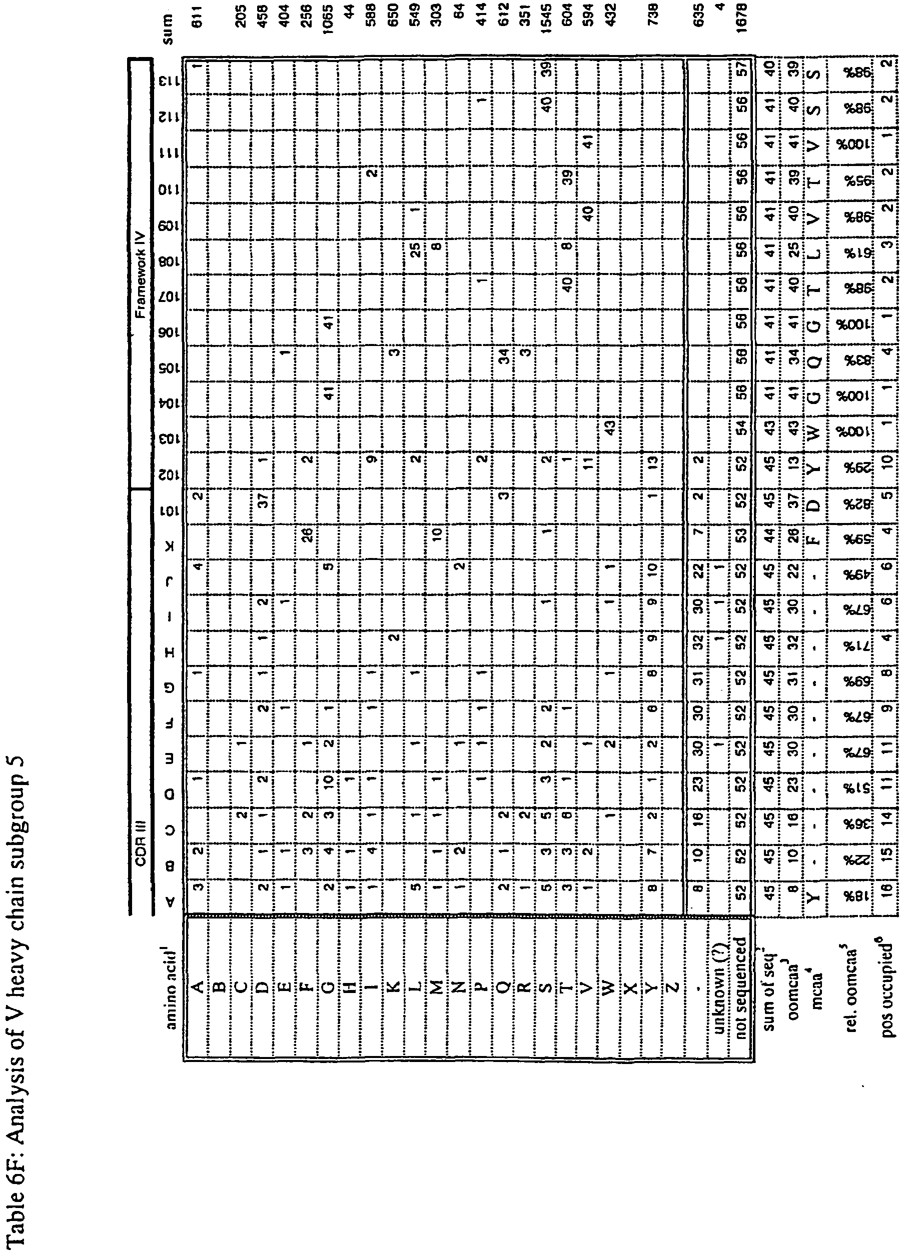

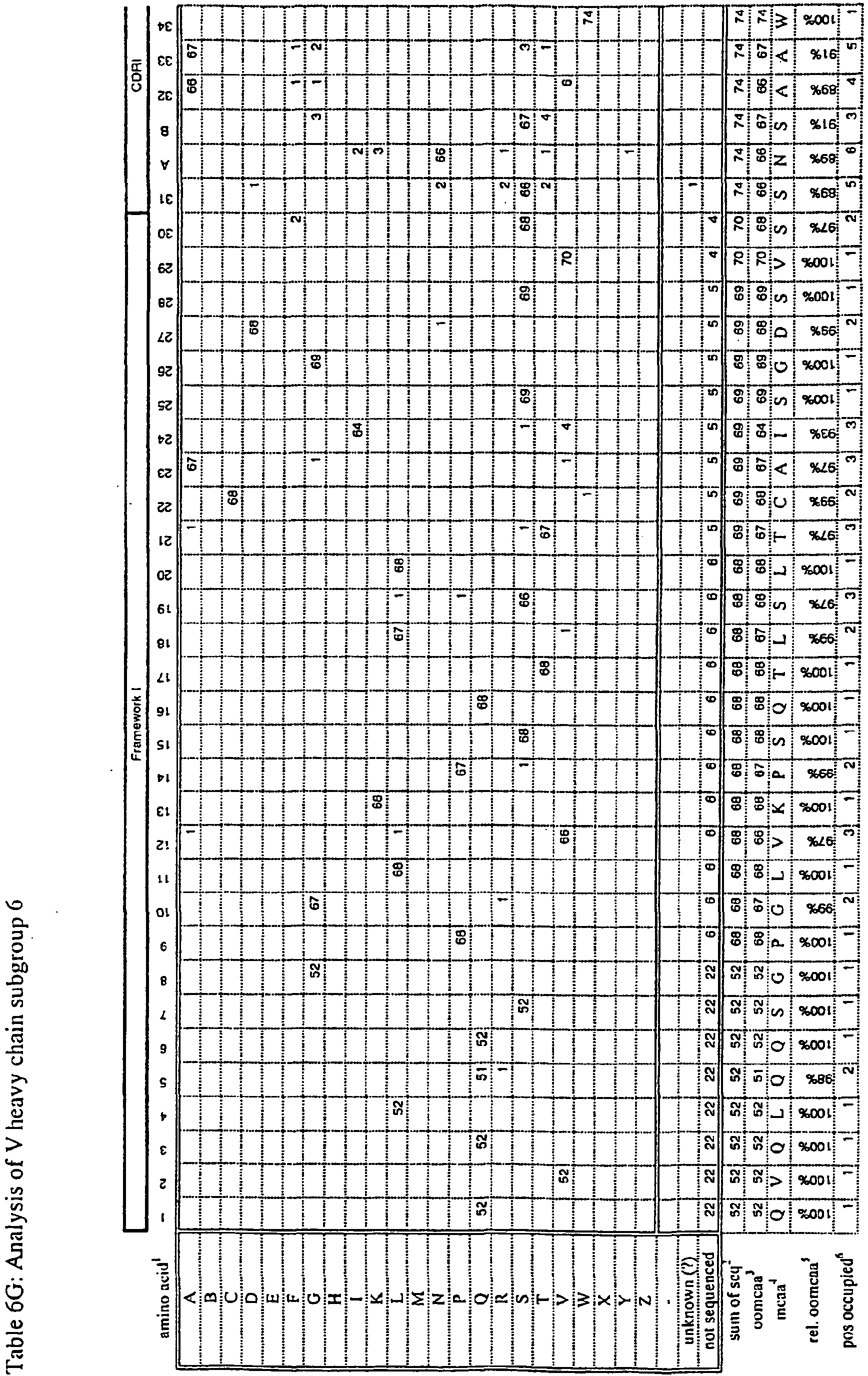

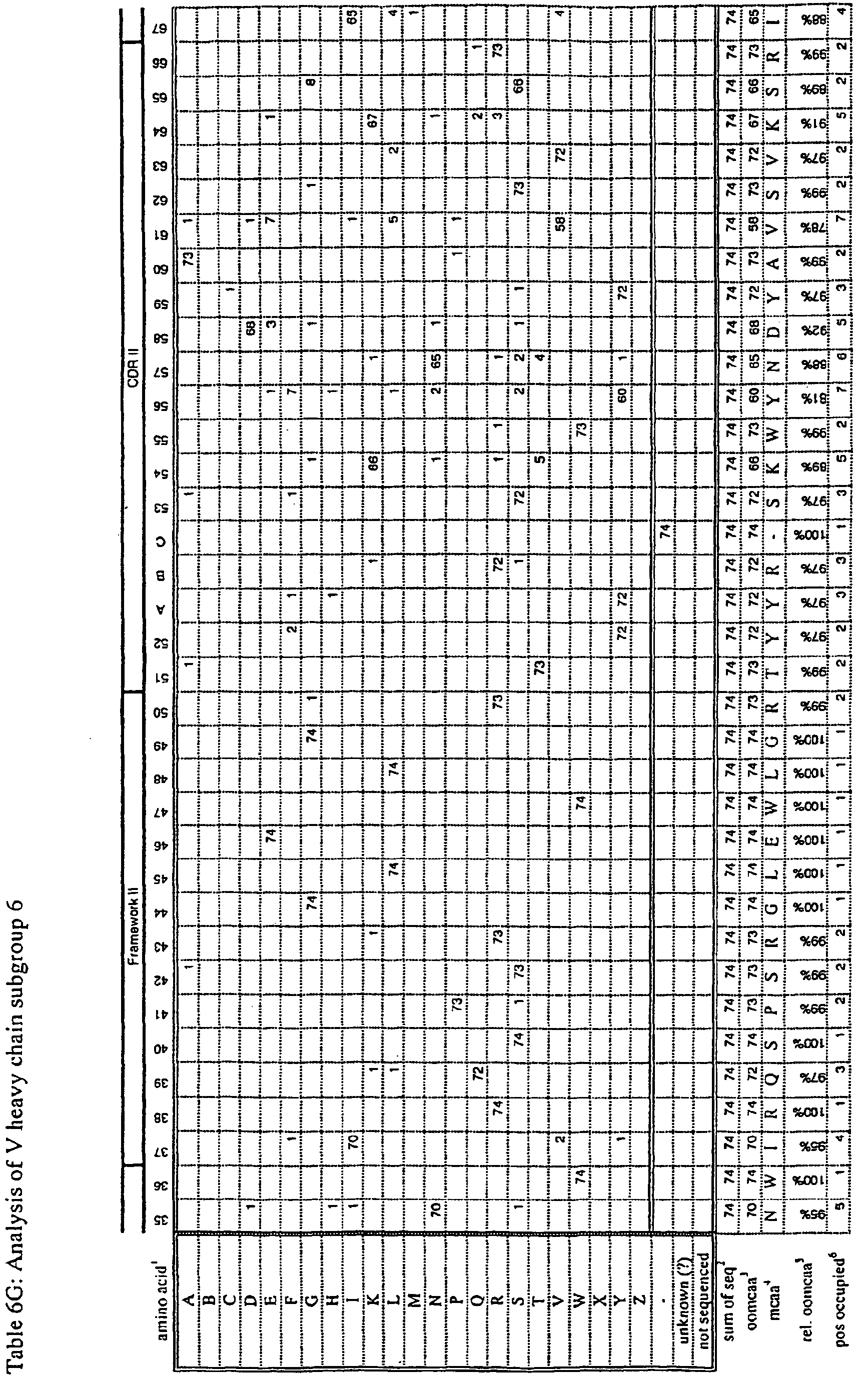

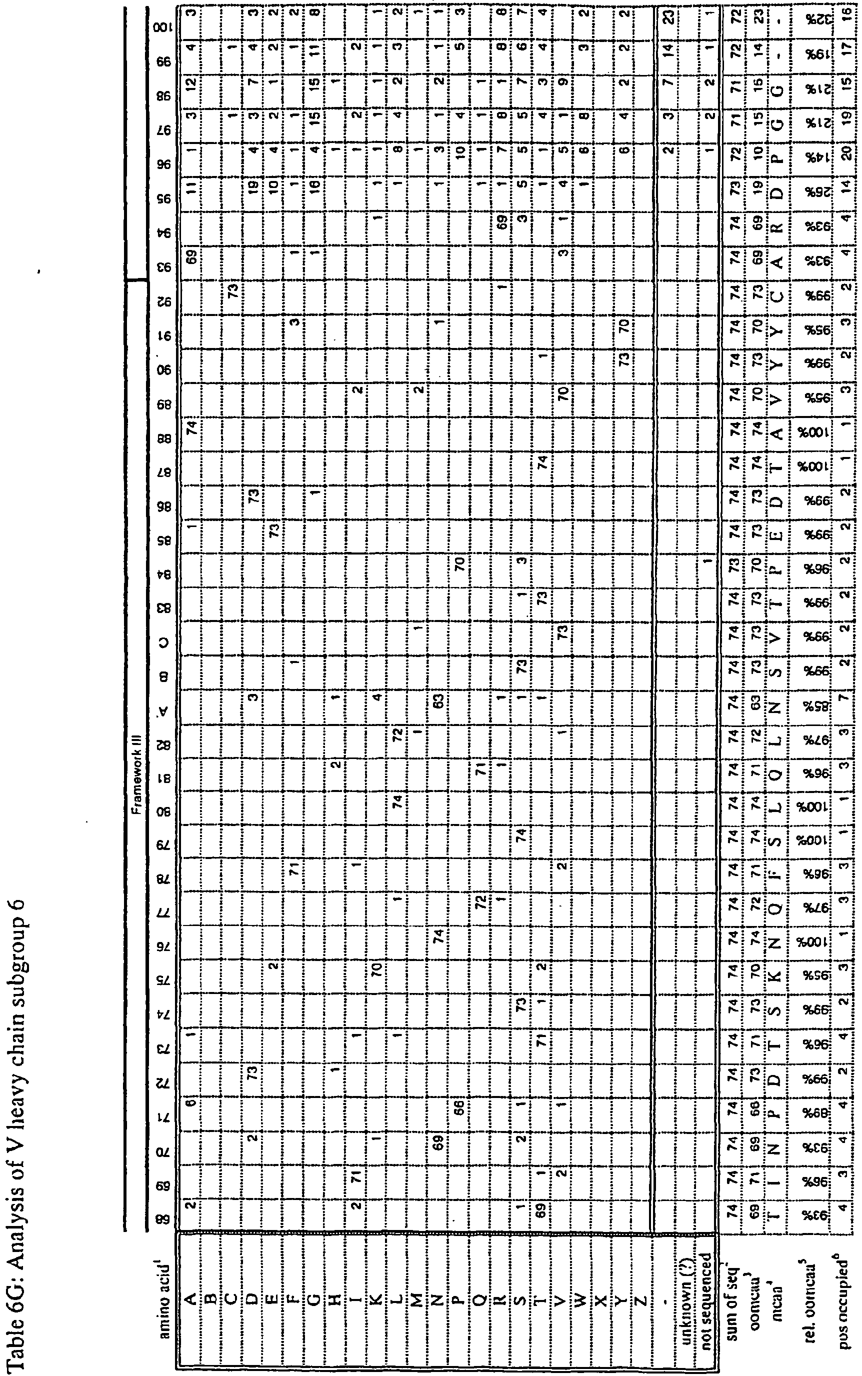

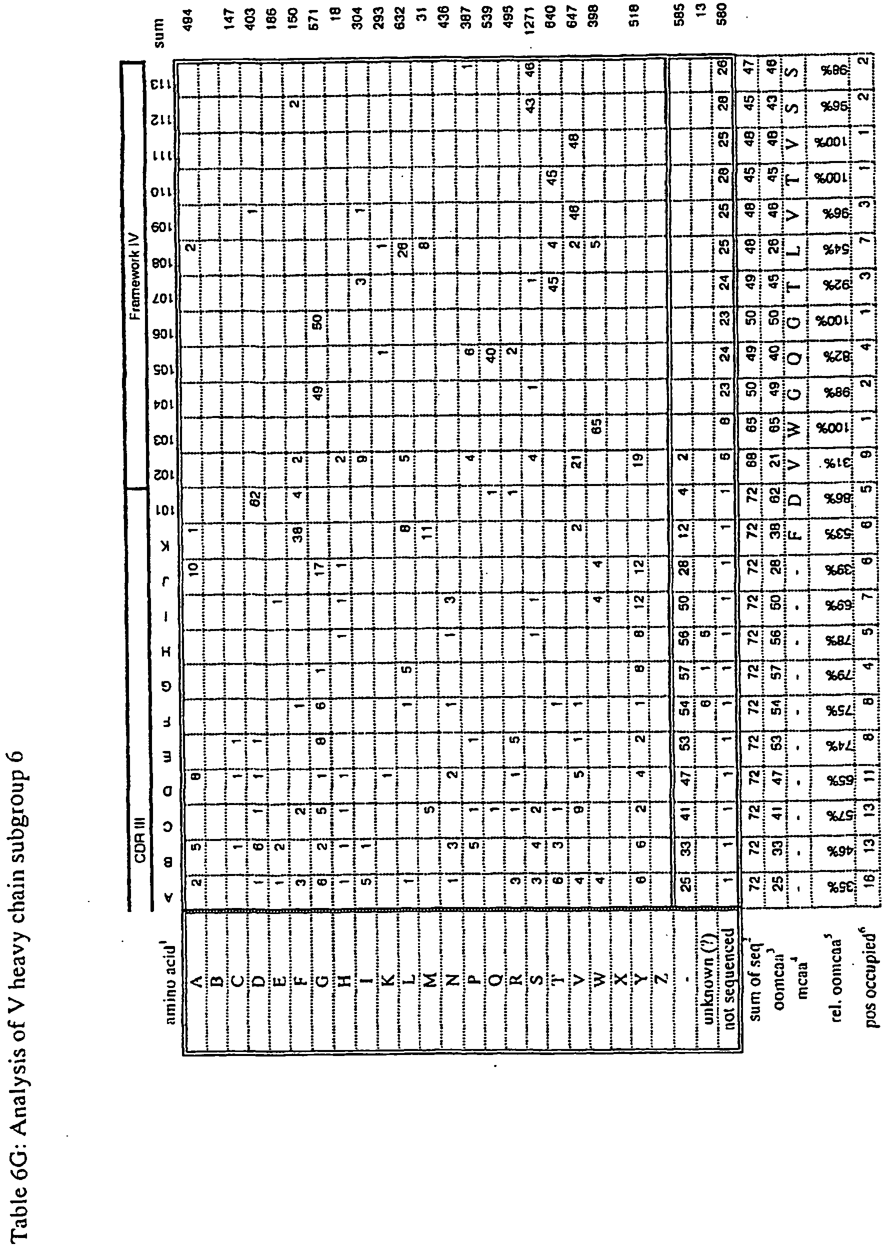

### Appendix to Tables 1A-C

### A. References of rearranged sequences

### Reference of rearranged human kappa sequences used for alignment

1 Alescio-Zonta, L. & Baglioni, C. (1970) Eur.J.Biochem., 15, 450-463.
2 Andrews, D.W. & Capra, J.D. (1981) Biochemistry, 20, 5816-5822.
3 Andris, J.S., Ehrfich, P.H., Ostberg, L. & Capra, J.D. (1992) J.Immunol., 149, 4053-4059.
4 Atkinson, P.M., Lampman, G.W., Furie, B.C., Naparstek, Y., Schwartz, R.S., Stollar, B.D. & Furie, B. (1985) J.Clin.Invest., 75, 1138-1143.
5 Aucouturier, P., Bauwens, M., Khamlichi, A.A., Denoroy, L, Spinelli, S., Touchard, G., Preud'homme, J.-L. & Cogne, M. (1993) J.Immunol., 150, 3561-3568.
6 Avila, M.A., Vazques, J., Danielsson, L., Fernandez De Cossio, M.E. & Borrebaeck, C.A.K. (1993) Gene, 127, 273-274.
7 Barbas, C.F., III, Crowe, Jr., J.E., Cababa, D., Jones, T.M.. Zebedee, S.L., Murphy, B.R., Chanock, R.M. & Burton, D.R. (1992) Proc.Natl.Acad.Sci.USA, 89, 10164-10168.
8 Barbas, C.F., III, et al. (1993) J-Mol-Biol., 230, 812-23.
9 Bentley, D.L. & Rabbitts, T.H. (1980) Nature, 288, 730-733.
10 Bentley, D.L. & Rabbitts, T.H. (1983) Cell, 32, 181-189.
11 Bentley, D.L. (1984) Nature, 307, 77-80.
12 Bhat, N.M., Bieber, M.M., Chapman, C.J., Stevenson. F.K. & Teng, N.N.H. (1993) J.Immunol., 151, 5011-5021.
13 Blaison, G., Kuntz, J.-L. & Pasquali, J.-L. (1991) Eur.J.Immunol., 21, 1221-1227.
14 Braun, H., Leibold, W., Barnikol, H.U. & Hilschmann, N. (1971) Z.Physiol.Chem., 352, 647-651; (1972) Z.Physiol.Chem., 353, 1284-1306.
15 Capra, J.D. & Kehoe, J.M. (1975) Adv.Immunology, 20, 1-40. ; Andrews, D.W. & Capra, J.D. (1981) Proc.Nat.Acad.Sci.USA, 78, 3799-3803.
16 Capra, J.D. & Kehoe, J.M. (1975) Adv.Immunology, 20, 1-40. ; Ledford, D.K., Goni, F., Pizzolato, M., Franklin, E.C., Solomon, A. & Frangione, B. (1983) J.Immunol.. 131, 1322-1325.
17 Chastagner, P., Theze, J. & Zouali, M. (1991) Gene, 101, 305-306.
18 Chen, P.P., Robbins, D.L., Jirik, F.R., Kipps. T.J. & Carson, D.A. (1987) J.Exp.Med, 166, 1900-1905.
19 Chen, P.P., Robbins, D.L., Jirik, F.R., Kipps, T.J. & Carson, D.A. (1987) J.Exp.Med, 166, 1900-1905; Liu, M.-F., Robbins, D.L., Crowley, J.J., Sinha, S., Kozin, F., Kipps, T.J., Carson, D.A. & Chen.P.P. (1989) J.Immunol., 142, 688-694.
20 Chersi, A. & Natali, P.G. (1978) Irnmunochemistry, 15, 585-589.
21 Co, M.S., Deschamps, M., Whitley, R.J. & Queen, C. (1991) Proc.Natl.Acad.Sci.USA, 88, 2869-2873.
22 Cuisinier, A.-M., Fumoux, F., Fougereau, M. & Tonnelle, C. (1992) Mol.Immunol., 29, 1363-1373.
23 Davidson, A., Manheimer-Lory, A., Aranow, C., Peterson, R., Hannigan, N. & Diamond, B. (1990) J.Clin.Invest., 85, 1401-1409.
24 Denomme, G.A., Mahmoudi, M., Edwards, J.Y., Massicotte, H., Cairns, E. & Bell, D.A. (1993) Hum.Antibod.Hybridomas, 4, 98-103.
25 Dersimonian, H., Mcadam, K.P.W.J., Mackworth-Young, C. & Stollar. B.D. (1989) J.Immunol., 142, 4027-4033.
26 Dreyer, W.J., Gray, W.R. & Hood, L. (1967) Cold Spring Harbor Symp. Quantitative Biol., 32, 353-367.
27 Ebeling, S.B., Schutte, M.E.M. & Logtenberg, T. (1993) Eur.J.Immunol., 23, 1405-1408.
28 Eulitz, M. & Kley, H.-P. (1977) Immunochem., 14, 289-297.
29 Eulitz, M. & Linke, R.P. (1982) Z.Physiol.Chem., 363, 1347-1358.
30 Eulitz, M., Breuer, M., Eblen, A., Weiss, D.T. & Solomon, A. (1990) In Amyloid And Anryloidosis, Eds. J.B.Natvig, O.Forre, G.Husby, A.Husebekk, B.Skogen, K.Sletten & P.Westermarh Kluwer Academic .
31 Eulitz, M., Gotze, D. & Hilschmann, N. (1972) Z.Physiol.Chem., 353, 487-491; Eulitz, M. & Hilschmann, N. (1974) Z.Physiol.Chem., 355, 842-866.
32 Eulitz, M., Kley, H.P. & Zeitler, H.J. (1979) Z. Physiol. Chem., 360, 725-734.
33 Ezaki, I., Kanda, H., Sakai, K., Fukui, N., Shingu, M., Nobunaga, M. & Watanabe, T. (1991) Arthritis And Rheumatism, 34, 343-350.
34 Felgenhauer, M., Kohl, J. & Ruker, F. (1990) Nucl.Acids Res., 18, 4927.
35 Ferri, G., Stoppini, M., Iadarola, P., Bellotti, V. & Merlini, G. (1989) Biochim.Biophys.Acta, 995, 103-108.
36 Gillies, S.D., Dorai, H., Wesolowski, J., Majeau, G., Young, D., Boyd, J., Gardner, J. & James, K. (1989) Bio/Tech., 7, 799-804.
37 Goni, F: & Frangione, B. (1983) Proc.Nat.Acad.Sci.USA, 80, 4837-4841.
38 Goni, F.R., Chen, P.P., Mcginnis, D., Arjonilla, M.L., Fernandez, J., Carson, D., Solomon, A., Mendez, E. & Frangione, B. (1989) J.Immunol., 142, 3158-3163.
39 Gorman, S.D., Clark, M.R., Routledge, E.G., Cobbold, S.P. & Waldmann, H. (1991) Proc.Natl.Acad.Sci.USA, 88, 4181-4185.
40 Gottlieb, P.D., Cunningham, B.A., Rutishauser, U. & Edelman, G.M. (1970) Biochemistry, 9.3155-3161.
41 Griffiths. A.D., Malmqvist, M., Marks, J.D., Bye, J.M., Embleton, M.J., Mccafferty, J., Baier, M.. Holliger, K.P., Gorick, B.D., Hughes-Jones, N.C., Hoogenboom, H.R. & Winter, G. (1993) Embo J., 12, 725-734.
42 Hieter, P.A, Max, E.E., Seidman, J.G., Maizel, J.V., Jr. & Leder, P. (1980) Cell, 22, 197-207; Klobeck, H.G, Meindl, A., Combriato, G., Solomon, A. & Zachau, H.G. (1985) Nucl.Acids Res., 13, 6499-6513; Weir, L. & Leder, P. (1986)
43 Hilschmann, N. & Craig, L.C. (1965) Proc.Nat.Acad.Sci.USA, 53, 1403-1409; Hilschmann, N. (1967) Z.Physiol.Chem., 348, 1077-1080.
44 Hilschmann, N. & Craig, L.C. (1965) Proc.Nat.Acad.Sci.USA, 53, 1403-1409; Hilschmann, N. (1967) Z.Physiol.Chem., 348, 1718-1722; Hilschmann, N. (1969) Naturwissenschaften, 56, 195-205.
45 Hirabayashi, Y., Munakata, Y., Sasaki, T. & Sano, H. (1992) Nucl.Acids Res., 20, 2601.
46 Jaenichen, H.-R., Pech, M., Lindenmaier, W., Wildgruber, N. & Zachau, H.G. (1984) Nuc.Acids Res., 12, 5249-5263.
47 Jirik, F.R., Sorge, J., Fong, S., Heitzmann, J.G., Curd, J.G., Chen, P.P., Goldfien, R. & Carson, D.A. (1986) Proc.Nat.Acad.Sci.USA, 83, 2195-2199.
48 Kaplan, A.P. & Metzger, H. (1969) Biochemistry, 8, 3944-3951. ; Klapper, D.G. & Capra, J.D. (1976) Ann.Immunol.(Inst.Pasteur), 127c, 261-271.
49 Kennedy, M.A (1991) J.Exp.Med., 173, 1033-1036.
50 Kim, H.S. & Deutsch, H.F. (1988) Immunol., 64, 573-579.
51 Kipps, T.J., Tomhave, E., Chen, P.P. & Carson, D.A. (1988) J.Exp.Med.., 167, 840-852.
52 Kipps, T.J., Tomhave, E., Chen, P.P. & Fox, R.I. (1989) J.Immunol., 142, 4261-4268.
53 Klapper, D.G. & Capra, J.D. (1976) Ann. Immunol. (Inst.Pasteur), 127c, 261-271.
54 Klein, U., Kuppers, R. & Rajewsky, K. (1993) Eur.J. Immunol., 23, 3272-3277.
55 Klobeck. H.G, Meindl, A., Combriato, G.. Solomon, A. & Zachau, H.G. (1985) Nucl.Acids Res., 13, 6499-6513.
56 Klobeck, H.G., Bornkammm, G.W., Combriato, G., Mocikat, R., Pohlenz, H.D. & Zachau, H.G. (1985) Nucl.Acids Res., 13, 6515-6529.
57 Klobeck, H.G., Combriato, G. & Zachau, H.G. (1984) Nuc.Acids Res., 12, 6995-7006.
58 Klobeck, H.G., Solomon, A. & Zachau, H.G. (1984) Nature, 309, 73-76.
59 Knight, G.B., Agnello, V., Bonagura, V., Barnes, J.L., Panka, D.J. & Zhang, Q.-X. (1993) J.Exp.Med., 178, 1903-1911.
60 Kohler, H., Shimizu, A., Paul, C. & Putnam, F.W. (1970) Science, 169, 56-59. (Kaplan, A.P. & Metzger, H. (1969) Biochemistry, 8, 3944-3951.)
61 Kratzin, H., Yang, C.Y., Krusche, J.U. & Hilschmann, N. (1980) Z.Physiol. Chem., 361, 1591-1598.
62 Kunicki, T.J., Annis, D.S., Gorski, J. & Nugent, D.J. (1991) J.Autoimmunity, 4, 433-446.
63 Larrick, J.W., Wallace, E.F., Coloma, M.J., Bruderer, U., Lang, A.B. & Fry, K.E. (1992) Immunological Reviews, 130, 69-85.
64 Laure, C.J., Watanabe, S. & Hilschmann, N. (1973) Z.Physiol. Chem., 354. 1503-1504.
65 Ledford, D.K., Goni, F., Pizzolato, M., Franklin, E.C., Solomon, A. & Frangione, B. (1983) J.Immunol., 131, 1322-1325.
66 Ledford, D.K., Goni, F., Pizzolato, M., Franklin, E.C., Solomon, A. & Frangione, B. (1983) J.Immunol., 131, 1322-1325.
67 Ledford, D.K., Goni, F., Pizzolato, M., Franklin, E.C., Solomon, A. & Frangione, B. (1983) J.Immunol., 131, 1322-1325. Pons-Estel, B., Goni, F., Solomon, A. & Frangione, B. (1984) J.Exp.Med., 160, 893.
68 Levy, S., Mendel, E., Kon, S., Avnur, Z. & Levy, R. (1988) J.Exp.Med., 168, 475-489.
69 Liepnieks, J.J., Dwulet, F.E. & Benson, M.D. (1990) Mol.Immunol., 27, 481-485.
70 Manheimer-Lory, A., Katz, J.B., Pillinger, M., Ghossein, C., Smith, A. & Diamond, B. (1991)J.Exp.Med., 174, 1639-1652.
71 Mantovani, L., Wilder, R.L. & Casali, P. (1993) J.Immunol., 151, 473-488.
72 Mariette, X., Tsapis, A. & Brouet, J.-C. (1993) Eur.J.Immunol., 23, 846-851.
73 Marks, J.D., Hoogenboom, H.R., Bonnert, T.P., Mccafferty, J., Griffiths, A.D. & Winter, G. (1991) J.Mol.Biol., 222, 581-597.
74 Marsh, P., Mills, F. & Gould, H. (1985) Nuc.Acids Res., 13, 6531-6544.
75 Middaugh, C.R. & Litman, G.W. (1987) J.Biol.Chem.. 262,3671-3673.
76 Milstein, C. & Deverson, E.V. (1971) Biochem.J., 123, 945-958.
77 Milstein, C. (1969) Febs Letters, 2, 301-304.
78 Milstein, C. (1969) Febs Letters, 2, 301-304.
79 Milstein, C.P. & Deverson, E.V. (1974) Eur.J.Biochem., 49, 377-391.
80 Moran, M.J., Andris, J.S., Matsumato, Y.-I., Capra, J.D. & Hersh, E.M. (1993) Mol.Immunol., 30, 1543-1551.
81 Nakatani, T., Nomura, N., Horigome, K., Ohtsuka, H. & Noguchi, H. (1989) Bio/Tech., 7, 805-810.
82 Newkirk, M., Chen, P.P., Carson, D., Posnett, D. & Capra, J.D. (1986) Mol.Immunol., 23, 239-244.
83 Newkirk, M.M., Gram, H., Heinrich, G.F., Ostberg, L., Capra, J.D. & Wasserman, R.L. (1988) J.Clin.Invest., 81, 1511-1518.
84 Newkirk, M.M., Mageed, R.A., Jefferis, R., Chen, P.P. & Capra, J.D. (1987) J.Exp.Med., 166, 550-564.
85 Olee, B.T., Lu, E.W., Huang, D.-F., Soto-Gil, R. W., Deftos, M., Kozin, F., Carson, D.A. & Chen, P.P. (1992) J.Exp.Med., 175, 831-842.
86 Palm. W. & Hilschmann, N. (1973) Z.Physiol.Chem., 354, 1651-1654; (1975) Z.Physiol.Chem., 356, 167-191.
87 Pascual, V., Victor, K., Lelsz, D., Spellerberg, M.B., Hamblin, T.J., Thompson, K.M., Randen, I., Natvig, J., Capra, J.D. & Stevenson, F.K. (1991) J.Immunol., 146, 4385-4391.
88 Pascual, V., Victor, K., Randen, I., Thompson, K., Steinitz, M., Forre, O., Fu, S.-M., Natvig, J.B. & Capra, J.D. (1992) Scand.J.Immunol., 36, 349-362.
89 Pech, M. & Zachau, H.G. (1984) Nuc.Acids Res., 12, 9229-9236.
90 Pech, M., Jaenichen, H.-R., Pohlenz, H.-D., Neumaier, P.S., Klobeck, H.-G. & Zachau, H.G. (1984) J.Mol.Biol., 176, 189-204.
91 Pons-Estel, B., Goni, F., Solomon, A. & Frangione, B. (1984) J.Exp.Med., 160, 893-904.
92 Portolano, S., Mclachlan, S.M. & Rapoport, B. (1993) J.Immunol., 151, 2839-2851.
93 Portolano, S., Seto, P., Chazenbalk, G.D., Nagayama, Y., Mclachlan, S.M. & Rapoport, B. (1991) Biochem.Biophys.Res.Commun., 179, 372-377.
94 Pratt, L.F., Rassenti, L., Larrick, J., Robbins, B., Banks, P.M. & Kipps, T.J. (1989) J.Immunol., 143, 699-705.
95 Prelli, F., Tummolo, D., Solomon, A. & Frangione, B. (1986) J.Immunol., 136, 4169-4173.
96 Putnam, F.W., Whitley, E.J., Jr., Paul, C.& Davidson, J.N. (1973) Biochemistry, 12, 3763-3780.
97 Randen, I., Pascual, V., Victor, K., Thompson, K.M., Forre, O., Capra, J.D. & Natvig, J.B. (1993) Eur.J.Immunol., 23, 1220-1225.
98 Rassenti, L.Z., Pratt, L.F., Chen, P.P., Carson, D.A. & Kipps, T.J. (1991) J.Immunol., 147, 1060-1066.
99 Reidl, L.S., Friedman. D.F., Goldman, J., Hardy, R.R., Jefferies, L.C. & Silberstein, L.E. (1991) J.Immunol., 147, 3623-3631.
100 Riechmany L., Clark, M., Waldmann, H. & Winter, G. (1988) Nature, 332, 323-327.
101 Riesen, W., Rudikoff, S., Oriol, R. & Potter, M. (1975) Biochemistry, 14, 1052-1057; Riesen. W.F., Braun, D.G. & Jaton, J.C. (1976) Proc.Nat.Acad.Sci.USA, 73, 2096-2100; Riesen, W.F. & Jaton, J.C. (1976) Biochemistry, 15, 3829.
102 Rodilla Sala, E., Kratzin, D.H., Pick, A.I. & Hilschmann, N. (1990) In Amyloid And Amyloidosis, Eds. J.B.Natvig, O.Forre, G.Husby, A.Husebekk, B.Skogen, K.Sletten & P.Westermark, Kluwer Academic
103 Schiechl, H. & Hilschmann, N. (1971) Z.Physiol.Chem., 352, 111-115; (1972) Z.Physiol.Chem., 353, 345-370.
104 Schneider, M. & Hilschmann, N. (1974) Z.Physiol.Chem., 355, 1164-1168.
105 Shearman, C.W., Pollock, D., White, G., Hehir, K., Moore, G.P., Kanzy, E.J. & Kurrle, R. (1991) J.Immunol., 147,4366-4373.
106 Shinoda, T. (1973) J.Biochem., 73, 433-446.
107 Shinoda, T. (1975) J.Biochem., 77, 1277-1296.
108 Shinoda, T., Takenawa, T., Hoshi, A. & Isobe, T. (1990) In Amyloid And Amyloidosis, Eds. J.B.Natvig, O.Forre, G.Husby, A.Husebekk, B.Skogen, K.Sletten & P.Westermark, Kluwer Academic Publishers, Dordrecht/Boston/London, Pp.157-
109 Silberstein, L.E., Litwin, S. & Carmack, C.E. (1989) J.Exp.Med., 169, 1631-1643.
110 Sims, M.J., Hassal, D.G., Brett, S., Rowan, W., Lockyer, M.J., Angel, A., Lewis, Asp., Hale, G., Waldmann, H. & Crowe, J.S. (1993) J.Immunol., 151, 2296-2308.
111 Spatz, L.A., Wong, K.K., Williams, M., Desai, R., Golier, J., Berman, J.E., Alt, F.W. & Latov, N. (1990) J.Immunol., 144, 2821-2828.
112 Stavnezer, J., Kekish, O., Batter, D., Grenier, J., Balazs, I, Henderson, E. & Zegers, B.J.M. (1985) Nucl.Acids Res., 13, 3495-3514.
113 Straubinger, B., Thiebe, R., Pech, M. & Zachau, H.G. (1988) Gene, 69, 209-214.
114 Suter, L., Barnikol, H.U., Watanabe, S. & Hilschmann, N. (1969) Z.Physiol.Chem., 350, 275-278; (1972) Z.Physiol.Chem., 353, 189-208.
115 Tempest, P.R., Bremner, P., Lambert, M., Taylor, G., Furze, J.M., Carr, F.J. & Harris, W.J. (1991) Bio/Tech., 9, 266-271.
116 Titani, K., Shinoda, T. & Putnam, F.W. (1969) J.Biol.Chem., 244, 3550-3560.
117 Toft, K.G., Olstad, O.K., Sletten, K. & Westermark, P. (1990) In Amyloid And Amyloidosis, Eds. J.B.Natvig, O.Forre, G.Husby, A.Husebekk, B.Skogen, K.Sletten & P. Westermark, Kluwer Academic
118 Van Es, J.H., Aanstoot, H., Gmelig-Meyling, F.H.J., Derksen, R.H.W.M. & Logtenberg, T. (1992) J.Immunol., 149, 2234-2240.
119 Victor, K.D., Pascual, V., Lefvert, A.K. & Capra, J.D. (1992) Mol.Immunol., 29, 1501-1506.
120 Victor, K.D., Pascual, V., Williams, C.L., Lennon, V.A. & Capra, J.D. (1992) Eur.J.Immunol., 22, 2231-2236.
121 Victor, K.D., Randen, I, Thompson, K., Forre, O., Natvig, J.B., Fu, S.M. & Capra, J.D. (1991) J.Clin.Invest., 87, 1603-1613.
122 Wagner, S.D. & Luzzatto, L. (1993) Eur.J.Immunol., 23, 391-397.
123 Watanabe, S. & Hilschmann, N. (1970) Z.Physiol.Chem., 351, 1291-1295.
124 Weisbart, R.H., Wong, A.L., Noritake, D., Kacena, A., Chan, G., Ruland, C., Chin, E., Chen, I.S.Y. & Rosenblatt, J.D. (1991) J.Immunol., 147, 2795-2801.
125 Weng, N.-P., Yu-Lee, L.-Y., Sanz, I., Patten, B.M. & Marcus, D.M. (1992) J.Immunol., 149, 2518-2529.
126 Winkler, T.H., Fehr, H. & Kalden, J.R. (1992) Eur.J.Immunol., 22, 1719-1728.

### References of rearranged human lambda sequences used for alignment

1 Alexandre, D., Chuchana, P., Brockly, F., Blancher, A., Lefranc, G. & Lefranc, M.-P. (1989) Nuc.Acids Res., 17, 3975.
2 Anderson, M.L.M., Brown, L., Mckenzie, E., Kellow, J.E. & Young, B.D. (1985) Nuc.Acids Res., 13, 2931-2941.
3 Andris, J.S., Brodeur, B.R. & Capra, J.D. (1993) Mol.Immunol., 30, 1601-1616.
4 Andris, J.S., Ehrlich, P.H., Ostberg, L. & Capra, J.D. (1992) J.Immunol., 149, 4053-4059.
5 Baczko, K., Braun, D.G., Hess, M. & Hilschmann, N. (1970) Z.Physiol Chem., 351, 763-767; Baczko, K., Braun, D.G. & Hilschmann, N. (1974) Z.Physiol.Chem., 355, 131-154.
6 Berinstein, N., Levy, S. & Levy, R. (1989) Science, 244, 337-339.
7 Bhat, N.M., Bieber, M.M., Chapman, C.J., Stevenson, F.K. & Teng, N.N.H. (1993) J.Immunol., 151, 5011-5021.
8 Cairns, E., Kwong, P.C., Misener, V., Ip, P., Bell, D.A. & Siminovitch, K.A. (1989) J.Immunol., 143, 685-691.
9 Carroll, W.L., Yu, M., Link, M.P. & Korsmeyer, S.J. (1989) J.Immunol., 143, 692-698.
10 Chen, B.L. & Poljak, R.J. (1974) Biochemistry, 13, 1295-1302.
11 Chen, B.L., Chiu, Y.Y.H., Humphrey, RL. & Poljak, R.J. (1978) Biochim.Biophys.Acta, 537,9-21.
12 Combriato, G. & Klobeck, H.G. (1991) Eur.J.Immunol., 21, 1513-1522.
13 Cuisinier, A.-M., Fumoux, F., Fougereau, M. & Tonnelle, C. (1992) Mol.Immunol., 29, 1363-1373.
14 Dwulet, F.E., Strako, K. & Benson, M.D. (1985) Scand.J.Immunol., 22, 653-660.
15 Elahna, P., Livneh, A., Manheimer-Lory, A.J. & Diamond, B. (1991) J.Immunol., 147, 2771-2776.
16 Engelhard, M., Hess, M. & Hilschmann, N. (1974) Z.Physiol.Chem., 355, 85-88; Engelhard, M. & Hilschmann, N. (1975) Z.Physiol.Chem., 356, 1413-1444.
17 Eulitz, M. (1974) Eur.J.Biochem., 50, 49-69.
18 Eulitz, M., Breuer, M. & Linke, R.P. (1987) Biol.Che.Hoppe-Seyler, 368, 863-870.
19 Eulitz, M., Murphy, C., Weiss, D.T. & Solomon, A. (1991) J.Immunol., 146, 3091-3096.
20 Fett, J.W. & Deutsch, H.F. (1974) Biochemistry, 13, 4102-4114.
21 Fett, J.W. & Deutsch, H.F. (1976) Immunochem., 13, 149-155.; Jabusch, J.R. & Deutsch, H.F. (1982) Mol.Immunol., 19, 901-906.
22 Furey, W. Jr., Wang, B.C., Yoo, C.S. & Sax, M. (1983) J.Mol.Biol., 167, 661-692.
23 Fykse, E.-M., Sletten, K., Husby, G. & Cornwell, G.G., III (1988) Biochem.J., 256, 973-980.
24 Garver, F.A. & Hilschmann, N. (1971) Febs Letters, 16, 128-132; (1972) Eur.J.Biochem., 26, 10-32.
25 Gawinowicz, M.A., Merlin, G., Birken, S., Osserman, E.F. & Kabat, E.A. (1991) J.Immunol., 147, 915-920.
26 Ghiso, J., Solomon, A. & Frangione, B. (1986) J.Immunol., 136, 716-719.
27 Griffiths, A.D., Malmqvist, M., Marks, J.D., Bye, J.M., Embleton, M.J., Mccafferty, J., Baier, M., Holliger, K.P., Gorick, B.D., Hughes-Jones, N.C., Hoogenboom, H.R. & Winter, G. (1993) Embo J., 12, 725-734.
28 Gullasken, N., Idso, H., Nilsen, R., Sletten, K., Husby, G. & Cornwell, G.G. (1990) In Amyloid And Amyloidosis, Eds. J.B.Natvig, O.Forre, G.Husby, A.Husebekk, B.Skogen, K.Sletten & P.Westermark, Kluwer Academic
29 Harindranath, N., Goldfarb, I.S., Ikematsu, H., Burastero, S.E., Wilder, R.L., Notkins, A.L. & Casali, P. (1991) Int.Immunol., 3, 865-875.
30 Holm, E., Sletten, K. & Husby, G. (1986) Biochem.J., 239, 545-551.
31 Hughes-Jones, N.C., Bye, J.M., Beale, D. & Coadwell, J. (1990) Biochem.J., 268, 135-140.
32 Kametani, F., Yoshimura, K., Tonoike, H., Hoshi, A., Shinoda, T. & Isobe, T. (1985) Biochem.Biophys.Res.Commun., 126, 848-852.
33 Kiefer, C.R., Mcguire, B.S., Jr., Osserman, E.F. & Garver, F.A. (1983) J.Immunol., 131, 1871-1875.
34 Kiefer, C.R., Patton, H.M., Jr., Mcquire, B.S., Jr. & Garver, F.A. (1980) J.Immunol., 124, 301-306.
35 Kishimoto, T., Okajima, H., Okumoto, T. & Taniguchi, M. (1989) Nucl.Acids Res., 17, 4385.
36 Klafki, H.-W., Kratzin, H.D., Pick, A.I., Eckart, K. & Hilschmann, N. (1990) In Amyloid And Amyloidosis, Eds. J.B.Natvig, O.Forre, G.Husby, A.Husebekk, B.Skogen, K.Sletten & P.Westermark, Kluwer Academic
37 Kohler, H., Rudofsky, S. & Kluskens, L. (1975) J.Iminunology, 114.415-421.
38 Kojima, M., Odani, S. & Ikenaka, T. (1980) Mol.Immunol., 17, 1407-1414.
39 Komori, S., Yamasaki, N., Shigeta, M., Isojima, S. & Watanabe, T. (1988) Clin.Exp.Immunol., 71, 508-516.
40 Kratzin, H.D., Palm, W., Stangel, M., Schmidt, W.E., Friedrich, J. & Hitschmann. N. (1989) Biol.Chem.Hoppe-Seyler, 370, 263-272.
41 Kratzin, H.D., Pick, A.I., Stangel, M. & Hilschmann, N. (1990) In Amyloid And Amyloidosis, Eds. J.B.Natvig, O.Forre, G.Husby, A.Husebekk, B.Skogen, K.Sletten & P.Westermark, Kluwer Academic Publishers, Dordrecht/Boston/London, Pp.181-
42 Langer, B., Steinmetz-Kayne, M. & Hilschmann, N. (1968) Z.Physiol. Chem., 349, 945-951.
43 Larrick, J.W., Danielsson, L., Brenner, C.A., Wallace, E.F., Abrahamson, M., Fry, K.E. & Borrebaeck. C.A.K. (1989) Bio/Tech., 7, 934-938.
44 Levy, S., Mendel, E., Kon, S., Avnur, Z. & Levy, R. (1988) J.Exp.Med., 168,475-489.
45 Lewis, A.P., Lemon, S.M., Barber, K.A., Murphy, P., Parry, N.R., Peakman, T.C., Sims, M.J., Worden, J. & Crowe, J.S. (1993) J.Immunol., 151, 2829-2838.
46 Liu, V.Y.S., Low, T.L.K., Infante, A. & Putnam, F.W. (1976) Science, 193, 1017-1020; Infante, A. & Putnam, F.W. (1979) J.Biol.Chem., 254, 9006-9016.
47 Lopez De Castro, J.A., Chiu, Y.Y.H. & Poljak, R.J. (1978) Biochemistry, 17, 1718-1723.
48 Mantovani, L., Wilder, R.L. & Casali, P.(1993) J.mmunol., 151, 473-488.
49 Marks, J.D., Hoogenboom, H.R., Bonnert, T.P., Mccafferty, J., Griffiths, A.D. & Winter, G. (1991) J.Mol.Biol., 222, 581-597.
50 Mihaesco, E., Roy, J.-P., Congy, N., Peran-Rivat, L. & Mihaesco, C. (1985) Eur.J.Biochem., 150, 349-357.
51 Milstein, C., Clegg, J.B. & Jarvis, J.M. (1968) Biochem.J., 110, 631-652.
52 Moran, M.J., Andris, J.S., Matsumato, Y.-I., Capra, J.D. & Hersh, E.M. (1993) Mol.Immunol., 30, 1543-1551.
53 Nabeshima, Y. & Ikenaka, T. (1979) Mol.Immunol., 16, 439-444.
54 Olee, B.T., Lu, E.W., Huang, D.-F., Soto-Gil, R.W., Deftos, M., Kozin, F., Carson, D.A. & Chen, P.P. (1992) J.Exp.Med., 175, 831-842.
55 Pascual, V., Victor, K., Randen, I., Thompson, K., Steinitz, M., Forre. O., Fu, S.-M., Natvig, J.B. & Capra, J.D. (1992) Scand.J.Immunol., 36, 349-362.
56 Paul, E., Iliev, A.A., Livneh, A. & Diamond, B. (1992) J.Immunol., 149, 3588-3595.
57 Pick, A.I., Kratzin, H.D., Barnikol-Watanabe, S. & Hilschmann, N. (1990) In Amyloid And Amyloidosis, Eds. J.B.Natvig, O.Forre, G.Husby, A.Husebekk, B.Skogen, K.Sletten & P.Westennark, Kluwer Academic
58 Ponstingl, H. & Hilschmann, N. (1969) Z.Physiol.Chem., 350, 1148-1152; (1971) Z.Physiol.Chem., 352, 859-877.
59 Ponstingl, H., Hess, M. & Hilschmann, N. (1968) Z.Physiol.Chem., 349, 867-871; (1971) Z.Physiol.Chem., 352, 247-266.
60 Randen, I., Pascual, V., Victor, K., Thompson, K.M., Forre, O., Capra, J.D. & Natvig, J.B. (1993) Eur.J.Immunol., 23, 1220-1225.
61 Scholz, R. & Hilschmann, N. (1975) Z.Physiol.Chem., 356, 1333-1335.
62 Settmacher, U, Jahn, S., Siegel, P., Von Baehr, R. & Hansen, A (1993) Mol.Immunol., 30, 953-954.
63 Shinoda, T., Titani, K. & Putnam, F.W. (1970) J.Biol.Chem., 245, 4475-4487.
64 Sletten, K., Husby, G. & Natvig, J.B. (1974) Scand.J.Immunol., 3, 833-836.; Sletten, K., Natvig, J.B., Husby, G. & Juul, J. (1981) Biochem., 195, 561-572.
65 Solomon, A., Frangione, B. & Franklin, E.C. (1982) J.Clin.Invest., 70, 453-460.; Frangione, B., Moloshok, T. & Solomon, A. (1933) J.Immunol., 131, 2490-2493.
66 Takahashi, N., Takayasu, T., Isobe, T., Shinoda, T., Okuyama, T. & Shimizu, A. (1979) J.Biochem., 86, 1523-1535.
67 Takahashi, N., Takayasu, T., Shinoda, T., Ito, S., Okuyama, T. & Shimizu. A. (1980) Biomed.Res., 1, 321-333.
68 Takahashi, Y., Takahashi, N., Tetaert, D. & Putnam, F.W. (1983) Proc.Nat.Acad.Sci.USA, 80, 3686-3690.
69 Takayasu, T., Takahashi, N., Shinoda, T., Okuyama, T. & Tomioka, H. (1980) J.Biochem., 89, 421-436.
70 Titani. K., Wikler, M., Shinoda, T. & Putnam, F.W. (1970) 1.Biol.Chem., 245, 2171-2176.
71 Toft, K.G., Sletten, K. & Husby, G. (1985) Biol.Chem.Hoppe-Seyler, 366, 617-625.
72 Tonoike, H., Kametani, F., Hoshi, A., Shinoda, T. & Isobe, T. (1985) Biochem.Biophys.Res.Commun., 126, 1228-1234.
73 Tonoike, H., Kametani, F., Hoshi, A., Shinoda, T. & Isobe. T. (1985) Febs Letters, 185, 139-141.
74 Tsujimoto, Y. & Croce, C.M. (1984) Nucl.Acids Res., 12, 8407-8414.
75 Tsunetsugu-Yokota, Y., Minekawa, T., Shigemoto, K., Shirasawa, T. & Takemori, T. (1992) Mol.Immunol., 29, 723-728.
76 Tveteraas, T., Sletten, K. & Westermark, P. (1985) Biochem.J., 232, 183-190.
77 Vasicek, T.J. & Leder, P. (1990) J.Exp.Med., 172, 609-620.
78 Victor, K.D., Randen, I., Thompson, K., Forre, O., Natvig, J.B., Fu, S.M. & Capra, J.D. (1991) J.Clin.Invest., 87, 1603-1613.
79 Weng, N.-P., Yu-Lee, L.-Y., Sanz, I., Patten, B.M. & Marcus, D.M. (1992) J.Immunol., 149, 2518-2529.
80 Wikler, M. & Putnam, F.W. (1970) J.Biol.Chem., 245, 4488-4507.
81 Winkler, T.H., Fehr, H. & Kalden, J.R. (1992) Eur.J.Immunol., 22, 1719-1728.
82 Yago, K., Zenita, K., Ohwaki, I., Harada, Y., Nozawa, S., Tsukazaki, K., Iwamori, M., Endo, N., Yasuda, N., Okuma, M. & Kannagi, R. (1993) Mol.Immunol., 30, 1481-1489.
83 Yamasaki, N., Komori, S. & Watanabe, T. (1987) Mol.Immunol., 24, 981-985.
84 Zhu, D., Kim, H.S. & Deutsch, H.F. (1983) Mol.Immunol., 20,1107-1116.
85 Zhu, D., Zhang, H., Zhu, N. & Luo, X. (1986) Scientia Sinica, 29, 746-755.

### References of rearranged human heavy chain sequences used for alignment

1 Adderson. E.E., Azmi, F.H., Wilson, P.M., Shackelford, P.G. & Carroll, W.L. (1993) J.Immunol., 151, 800-809.
2 Adderson, E.E., Shackelford, P.G., Quinn, A. & Carroll, W.L. (1991) J.Imnunol., 147, 1667-1674.
3 Akahori, Y., Kurosawa. Y., Kamachi, Y., Torii, S. & Matsuoka, H. (1990) J.Clin.Invest., 85, 1722-1727.
4 Andris, J.S., Brodeur, B.R. & Capra, J.D. (1993) Mol.Immunol., 30, 1601-1616.
5 Andris, J.S., Ehrlich, P.H., Ostberg, L. & Capra, J.D. (1992) J.Immunol., 149, 4053-4059.
6 Andris, J.S., Johnson, S., Zolla-Pazner, S. & Capra, J.D. (1991) Proc.Natl.Acad.Sci.USA, 88, 7783-7787.
7 Anker, R., Conley, M.E. & Pollok, B.A. (1989) J.Exp.Med., 169, 2109-2119.
8 Atkinson, P.M., Lampman, G.W., Furie, B.C., Naparstek, Y., Schwartz, R.S., Stollar, B.D. & Furie. B. (1985) J.Clin.Invest., 75, 1138-1143.;Lampman, G.W., Furie, B., Schwartz, R.S., Stollar, B.D. & Furie. B.C. (1989)
9 Avila, M.A., Vazques, J., Danielsson, L., Fernandez De Cossio, M.E. & Borrebaeck, C.A.K. (1993) Gene, 127, 273-274.
10 Bakkus. M.H.C., Heirman, C., Van Riet, I., Van Camp, B. & Thielemans, K. (1992) Blood, 80, 2326-2335.
11 Barbas, C.F., III, Crowe, Jr., J.E., Cababa, D., Jones, T.M., Zebedee, S.L., Murphy, B.R., Chanock, R.M. & Burton, D.R. (1992) Proc.Natl.Acad.Sci.USA, 89, 10164-10168.
12 Barbas, C.F., III, Collet, T.A., Amberg, W., Roben, P., Binley, J.M., Hoekstra, D., Cababa, D., Jones, T.M., Williamson, R.A., Pilkington, G.R., Haigwood, N.L., Cabezas, E., Satterthwait, A.C., Sanz, I. & Burton, D.R. (1993) J.Mol.Biol., 230, 812-823.
13 Berman, J.E., Humphries, C.G., Barth, J., Alt, F.W. & Tucker, P.W. (1991) J.Exp.Med., 173, 1529-1535.
14 Berman, J.E., Mellis, S.J., Pollock, R., Smith, C.L., Suh, H., Heinke, B., Kowal, C., Surti, U., Chess, L., Cantor, C.R & Alt, F.W. (1988) Embo J., 7, 727-738.
15 Bhat, N.M., Bieber, M.M., Chapman, C.J., Stevenson, F.K. & Teng, N.N.H. (1993) J.Immunol., 151, 5011-5021.
16 Bird. J., Galili, N., Link, M., Stites, D. & Sklar, J. (1988) J.Exp.Med., 168, 229-245.
17 Cai, J., Humphries, C., Richardson, A. & Tucker, P.W. (1992) J.Exp.Med., 176, 1073-1081.
18 Cairns, E., Kwong, P.C., Misener, V., Ip, P., Bell, D.A. & Siminovitch, K.A..(1989) J.Immunol., 143, 685-691.
19 Capra, J.D. & Hopper, J.E. (1976) Immunochemistry, 13, 995-999; Hopper, J.E., Noyes, C., Heinrikson, R. & Kessel, J.W. (1976) J.Immunol., 116, 743-746.
20 Capra, J.D. & Kehoe, J.M. (1974) Proc.Nat.Acad.Sci.USA, 71, 845-848.
21 Carroll W.L., Yu, M., Link, M.P. & Korsmeyer, S.J. (1989) J.Immunol., 143, 692-698.
22 Chen, P.P., Liu, M.-F., Glass, C.A., Sinha, S., Kipps, T.J. & Carson, D.A. (1989) Arthritis & Rheumatism, 32, 72-76; Kipps, T.J., Tomhave, E., Pratt, L.F., Duffy, S., Chen, P.P. & Carson, D.A. (1989) Proc.Natl.Acad.Sci.USA, 86, 5913-5917.
23 Chiu, Y.Y.H., Lopez De Castro, J.A. & Poljak, R.J. (1979) Biochemistry, 18, 553-560.
24 Cleary, M.L., Meeker, T.C., Levy, S., Lee, E., Trela, M., Sklar, J. & Levy. R. (1986) Cell, 44, 97-106.
25 Cuisinier. A.-M., Fumoux. F., Fougereau, M. & Tonnelle, C. (1992) Mol.Immunol., 29, 1363-1373.
26 Cuisinier, A.-M., Gauthier, L., Boubli, L., Fougereau, M. & Tonnelle, C. (1993) Eur.J.Immunol., 23, 110-118.
27 Cunningham, B.A., Gottlieb.P.D., Pflumm, MN. & Edelman, G.M. (1971) Progress In Immunology (B.Amos, Ed.), Academic Press, N.Y., Pp.3-24.
28 Cunningham, B.A., Rutishauser, U., Gall, W.E., Gottlieb, P.D., Waxdal, M.J. & Edelman, G.M. (1970) Biochemistry, 9, 3161-3170.
29 Deane, M. & Norton, J.D. (1990) Eur.J.Immunol., 20, 2209-2217.
31 Dersimonian, H., Schwartz, R.S., Barrett, K.J. & Stollar, B.D. (1987) J.Immunol., 139, 2496-2501.
32 Dersimonian, H., Schwartz, R.S., Barrett, K.J. & Stollar, B.D. (1987) J.Immunol., 139, 2496-2501; Chen, P.P., Liu, M.-F., Sinha, S. & Carson, D.A. (1988) Arth.Rheum., 31, 1429-1431.
33 Desai, R., Spatz, L., Matsuda, T., Ilyas, A.A., Berman, J.E., Alt, F.W., Kabat, E.A. & Latov, N. (1990) J.Neuroimmunol., 26, 35-41.
34 Ezaki, I., Kanda, H., Sakai, K., Fukui, N., Shingu, M., Nobunaga, M. & Watanabe, T. (1991) Arthritis And Rheumatism, 34, 343-350.
35 Felgenhauer, M., Kohl, J. & Ruker, F. (1990) Nucl.Acids Res., 18, 4927.
36 Florent, G., Lehman, D. & Putnam, F.W. (1974) Biochemistry, 13, 2482-2498.
37 Friedlander, R.M., Nussenzweig, M.C. & Leder, P. (1990) Nucl.Acids Res., 18, 4278.
38 Gawinowicz, M.A., Merlini, G., Birken, S., Osserman, E.F. & Kabat, E.A. (1991) J.Immunol., 147, 915-920.
39 Gillies, S.D., Dorai, H., Wesolowski, J., Majeau, G., Young, D., Boyd, J., Gardner, J. & James, K. (1989) Bio/Tech., 7, 799-804.
40 Goni, F. & Frangione, B. (1983) Proc.Nat.Acad.Sci.USA, 80, 4837-4841.
41 German, S.D., Clark, M.R., Routledge, E.G., Cobbold, S.P. & Waldmann, H. (1991) Proc.Natl.Acad.Sci.USA, 88, 181-4185.
42 Griffiths, A.D., Malmqvist, M., Marks, J.D., Bye, J.M., Embleton, M.J., Mccafferty, J., Baier, M., Holliger, K.P., Gorick, B.D., Hughes-Jones, N.C., Hoogenboom, H.R. & Winter, G. (1993) Embo J., 12, 725-734.
43 Grillot-Courvalin, C., Brouet, J.-C., Piller, F., Rassenti, L.Z., Labaume, S., Silverman, G.J., Silberstein, L. & Kipps, T.J. (1992) Eur.J.Immunol., 22, 1781-1788.
44 Guillaume, T., Rubinstein, D.B., Young, F., Tucker, L., Logtenberg, T., Schwartz, R.S. & Barrett, K.L. (1990) J.Immunol., 145, 1934-1945;Young, F., Tucker, L., Rubinstein, D., Guillaume, T., Andre-Schwartz, J., Barrett, K.J., Schwartz, R.S. & Logtenberg, T. (1990)
45 Harindranath, N., Goldfarb, I.S., Ikematsu, H., Burastero, S.E., Wilder, R.L., Notkins, A.L. & Casali, P. (1991) Int.Immunol., 3, 865-875.
46 Hillson. J.L., Oppliger, I.R., Sasso, E.H., Milner, E.C.B. & Wener, M.H. (1992) J.Immunol., 149, 3741-3752.
47 Hirabayashi, Y., Munakata, Y., Sasaki, T. & Sano, H. (1992) Nucl.Acids Res., 20, 2601.
48 Hoch, S. & Schwaber, J. (1987) J.Immunol., 139, 1689-1693.
49 Huang. C., Stewart, A.K., Schwartz, R.S. & Stollar, B.D. (1992) J:Clin.Invest., 89, 1331-1343.
50 Hughes-Jones, N.C., Bye, J.M., Beale, D. & Coadwell, J. (1990) Biochem.J. 268, 135-140.
51 Ikematsu, H., Harindranath, N., Ueki, Y., Notkins, A.L. & Casali, P. (1993) J.Immunol., 150, 1325-1337.
52 Ikematsu, H.. Kasaian, M.T., Schettino, E.W. & Casali, P. (1993) J.Immunol., 151, 3604-3616.
53 Kelly, P.J., Pascual, V., Capra, J.D. & Lipsky, P.E. (1992) J.Immunol., 148, 1294-1301.
54 Kipps, T.J. & Duffy, S.F. (1991) J.Clin.Invest., 87, 2087-2096.
55 Kipps, T.J., Tomhave, E., Pratt, L.F., Duffy, S., Chen, P.P. & Carson, D.A. (1989) Proc.Natl.Acad.Sci.USA, 86, 5913-5917.
56 Kishimoto, T., Okajima, H., Okumoto, T. & Taniguchi, M. (1989) Nucl.Acids Res., 17, 4385.
57 Knight, G.B., Agnello, V., Bonagura, V., Barnes, J.L., Panka, D.J. & Zhang, Q.-X. (1993) J.Exp.Med., 178, 1903-1911.
58 Kohler, H., Shimizu, A., Paul, C., Moore, V. & Putnam, F.W. (1970) Nature, 227, 1318-1320; Florent, G., Lehman, D. & Putnam, F.W. (1974) Biochemistry, 13, 2482-2498
59 Komori, S., Yamasaki, N., Shigeta, M., Isojima, S. & Watanabe, T. (1988) Clin.Exp.Immunol., 71, 508-516.
60 Kon. S., Levy, S. & Levy, R. (1987) Proc.Natl.Acad.Sci.USA, 84, 5053-5057.
61 Kratzin, H., Altevogt, P., Ruban, E., Kortt, A., Staroscik, K. & Hilschmann, N. (1975) Z.Physiol.Chem., 356, 1337-1342; Kratzin, H., Altevogt, P., Kortt, A., Ruban, E. & Hilschmann, N. (1978) Z.Physiol.Chem., 359, 1717-1745.
62 Kudo, A., Ishihara, T., Nishimura, Y. & Watanabe, T. (1985) Gene, 33, 181-189.
63 Kunicki, T.J., Annis, D.S., Gorski, J. & Nugent, D.J. (1991) J.Autoimmunity, 4, 433-446.
64 Larrick, J.W., Wallace, E.F., Coloma, M.J., Bruderer, U., Lang, A.B. & Fry, K.E. (1992) Immunological Reviews, 130, 69-85.
65 Lehman, D.W. & Putnam, F.W. (1980) Proc.Nat.Acad.Sci.USA. 77, 3239-3243.
66 Lewis, A.P., Lemon, S.M., Barber, K.A., Murphy, P., Parry, N.R., Peakman, T.C., Sims, M.J., Worden, J. & Crowe, J.S. (1993) J.Immunol., 151, 2829-2838.
67 Liu, V.Y.S., Low, T.L.K., Infante, A. & Putnam, F.W. (1976) Science, 193, 1017-1020.
68 Logtenberg, T., Young, F.M., Van Es, J., Gmelig-Meyling, F.H.J., Berman, J.E. & Alt, F.W. (1989) J.Autoimmunity, 2, 203-213.
69 Logtenberg, T., Young, F.M., Van Es, J.H., Gmelig-Meyling, F.H.J. & Alt, F.W. (1989) J.Exp.Med., 170, 1347-1355.
70 Manheimer-Lory, A., Katz, J.B., Pillinger, M., Ghossein, C., Smith, A. & Diamond, B. (1991)J.Exp.Med., 174, 1639-1652.
71 Mantovani, L., Wilder, R.L. & Casali, P. (1993) J.Immunol., 151, 473-488.
72 Mariette, X., Tsapis, A. & Brouet, J.-C. (1993) Eur. J.Immunol., 23, 846-851.
73 Marks, J.D., Hoogenboom, H.R., Bonnert, T.P., Mccafferty, J., Griffiths, A.D. & Winter, G. (1991) J.Mol.Biol., 222, 581-597.
74 Meeker, T.C., Grimaldi, J., O'rourke, R., Loeb, J.Juliusson, G. & Einhorn, S. (1988) J.Immol., 141, 3994-3998.
75 Milili, M., Fougereau, M., Guglielmi, P. & Schiff, C. (1991) Mol.Immunol., 28, 753-761.
76 Moran, M.J., Andris, J.S., Matsumato, Y.-I., Capra, J.D. & Hersh, E.M. (1993) Mol.Immunol., 30, 1543-1551.
77 Mortari, F., Wang, J-Y. & Schroeder, Jr., H.W. (1993) J.Immunol., 150, 1348-1357.
78 Newkirk, M.M., Gram, H., Heinrich, G.F., Ostberg, L., Capra, J.D. & Wasserman, R.L. (1988) J.Clin.Invest., 81, 1511-1518.
79 Newkirk, M.M., Mageed, R.A., Jefferis, R., Chen, P.P. & Capra, J.D. (1987) J.Exp.Med., 166, 550-564.
80 Nickerson, K.G., Berman, J., Glickman, E., Chess, L. & Alt, F.W. (1989) J.Exp.Med., 169, 1391-1403.
81 Olee, B.T., Lu, E. W., Huang, D.-F., Soto-Gil, R.W., Deftos, M., Kozin, F., Carson, D.A. & Chen, P.P. (1992) J.Exp.Med., 175, 831-842.
82 Pascual, V., Randen, I., Thompson, K., Sioud, M.Forre, O., Natvig, J. & Capra, J.D. (1990) J.Clin.Invest., 86, 1320-1328.
83 Pascual, V., Randen, I., Thompson, K., Sioud, M.Forre, O., Natvig, J. & Capra, J.D. (1990) J.Clin.Invest., 86, 1320-1328; Randen, I., Brown, D., Thompson, K.M., Hughes-Jones. N., Pascual, V., Victor, K., Capra, J.D., Forre, O. & Natvig, J.B. (1992)
84 Pascual, V., Victor, K., Lelsz, D., Spellerberg, M.B., Hamblin, T.J., Thompson. K.M., Randen, I., Natvig, J., Capra, J.D. & Stevenson, F.K. (1991) J.Immunol., 146, 4385-4391.
85 Pascual, V., Victor, K., Randen, I., Thompson, K., Steinitz, M., Forre, O., Fu, S.-M., Natvig, J.B. & Capra, J.D. (1992) Scand.J.Immunol., 36, 349-362.
86 Pascual, V., Victor, K., Spellerberg, M., Hamblin, T.J., Stevenson, F.K. & Capra, J.D. (1992) J.Immunol., 149, 2337-2344.
87 Ponstingl, H., Schwarz, J., Reichel, W. & Hilschmann, N. (1970) Z.Physiol.Chem., 351, 1591-1594.; Ponstingl, H. & Hilschmann, N. (1976) Z.Physiol.Chem., 357, 1571-1604.
88 Portolano, S., Mclachlan, S.M. & Rapoport, B. (1993) J.Immunol., 151, 2839-2851.
89 Portolano, S., Seto, P., Chazenbalk, G.D., Nagayama, Y., Mclachlan, S.M. & Rapoport, B. (1991) Biochem.Biophys.Res.Commun., 179, 372-377.
90 Pratt, L.F., Szubin, R., Carson, D.A. & Kipps, T.J. (1991) J.Immunol., 147, 2041-2046.
91 Press. E.M. & Hogg, N.M. (1970) Biochem.J., 117, 641-660.
92 Putnam, F.W., Shimizu, A., Paul., C., Shinoda, T. & Kofiler, H. (1971) Ann.N.Y.Acad.Sci., 190, 83-103.
93 Putnam, F.W., Takahashi, N., Tetaert, D., Debuire, B. & Lin, L.C. (1981) Proc.Nat.Acad.Sci.USA, 78, b168-6172.;Takahashi, N., Tetaert, D., Debuire, B., Lin, L. & Putnam, F.W. (1982) Proc.Nat.Acad.Sci.USA, 79, 2850-2854.
94 Raaphorst. F.M. Timmers, E., Kenter, M.J.H., Van Tol, M.J.D., Vossen, J.M. & Schuurman, R.K.B. (1992) Eur.J.Immunol., 22, 247-251.
95 Rabbitts, T.H., Bentley, D.L., Dunnick, W., Forster, A., Matthyssens, G. & Milstein, C. (1980) Cold Spring Harb.Symp.Quanti.Biol., 45, 867-878; Matthyssens, G. & Rabbitts, T.H. (1980) Proc.Nat.Acad.Sci.USA, 77, 6561-6565.
96 Randen. I., Pascual, V., Victor, K., Thompson, K.M., Forre, O., Capra, J.D. & Natvig, J.B. (1993) Eur.J.Immunol., 23, 1220-1225.
97 Rassenti. L.Z. & Kipps, T.J. (1993) J.Exp.Med., 177, 1039-1046.
98 Reidl, L.S., Friedman, D.F., Goldman, J., Hardy, R.R., Jefferies, L.C. & Silberstein, L.E. (1991) J.Immununol., 147, 3623-3631.
99 Roudier, J., Silverman, G.J., Chen, P.P., Carson, D.A. & Kipps, T.J. (1990) J.Immunol., 144, 1526-1530.
100 Sanz, I., Casali, P., Thomas, J.W., Notkins, A.L. & Capra, J.D. (1989) J.Immunol., 142, 4054-4061.
101 Sanz, I., Dang, H., Takei, M., Talal, N. & Capra, J.D. (1989) J.Immunol., 142, 883-887.
102 Schmidt, W.E., Jung, H-.D., Palm, W. & Hilschmann, N. (1983) Z.Physiol.Chem., 364, 713-747.
103 Schroeder, H.W., Jr. & Wang, J.Y. (1990) Proc.Natl.Acad.Sci.USA, 87, 6146-6150.
104 Schroeder, H.W., Jr., Hillson, J..L. & Perlmutter, R.M. (1987) Science, 238, 791-793.
105 Schroeder, H.W., Jr., Hillson, J.L. & Perlmutter, R.M. (1987) Science, 238, 791-793; Chen. P.P., Liu, M.-F., Glass. C.A., Sinha, S., Kipps, T.J. & Carson, D.A. (1989) Arthritis & Rheumatism, 32, 72-76.
106 Schroeder, H.W., Jr., Hillson, J.L. & Perlmutter, R.M. (1987) Science, 238, 791-793; Chen, P.P., Liu, M.-F., Sinha, S. & Carson, D.A. (1988) Arth.Rheum., 31, 1429-1431.
107 Schutte, M.E., Ebeling, S.B., Akkermans, K.E., Gmelig-Meyling. F.H. & Logtenberg, T. (1991) Eur.J.Immunol., 21, 1115-1121.
108 Schutte, M.E., Ebeling, S.B., Akkermans, K.E., Gmelig-Meyling, F.H.J. & Logtenberg, T. (1991) Eur.J.Immunol., 21, 1115-1121.
109 Settmacher, U., Jahn, S., Siegel, P., Von Baehr, R. & Hansen, A. (1993) Mol.Immunol., 30, 953-954.
110 Shen, A, Humphries, C., Tucker, P. & Blattner, F. (1987) Proc.Natl.Acad.Sci.USA, 84, 8563-8567.
111 Shimizu, A., Nussenzweig, M.C., Mizuta, T.-R., Leder, P. & Honjo, T. (1989) Proc.Natl.Acad.Sci.USA, 86, 8020-8023.
112 Shin, E.K., Matsuda, F., Fujikura. J.. Akamizu, T., Sugawa, H., Mori, T. & Honjo, T. (1993) Eur.J.Immunol., 23, 2365-2367.
113 Silberstein, L.E., Litwin, S. & Carmack, C.E. (1989) J.Exp.Med., 169, 1631-1643.
114 Singal, D.P., Frame, B., Joseph, S., Blajchman, M.A. & Leber, B.F. (1993) Immunogenet., 38, 242.
115 Spatz, L.A., Wong, K.K., Williams, M., Desai, R., Golier, J., Berman, J.E., Alt, F.W. & Latov, N. (1990) J.Immunol., 144, 2821-2828.
116 Steiner, L.A., Garcia-Pardo, A. & Margolies, M.N. (1979) Biochemistry, 18, 4068-4080.
117 Stewart, A.K., Huang, C., Stollar, B.D. & Schwartz, R.S. (1993) J.Exp.Med., 177, 409-418.
118 Thomas, J.W. (1993) J.Immunol., 150, 1375-1382.
119 Torano, A. & Putnam, F.W. (1978) Proc.Nat.Acad.Sci.USA, 75, 966-969.
120 Van Der Heijden, R. W.J., Bunschoten, H., Pascual, V., Uytdehaag, F.G.C.M., Osterhaus, A.D.M.E. & Capra, J.D. (1990) J.Immunol., 144, 2835-2839.
121 Van Der Stoep, N., Van Der Linden, J. & Logtenberg, T. (1993) J.Exp.Med., 177, 99-107.
122 Van Es, J.H., Gmelig-Meyling, F.H.J. & Logtenberg, T. (1992) Eur.J.Immunol., 22, 2761-2764.
123 Varade, W.S., Marin, E., Kittelberger, A.M. & Insel, R.A. (1993) J.Immunol., 150, 4985-4995.
124 Victor, K.D., Pascual, V., Lefvert, A.K. & Capra, J.D. (1992) Mol.Immunol., 29, 1501-1506.
125 Victor, K.D., Pascual, V., Williams, C.L., Lennon, V.A. & Capra, J.D. (1992) Eur.J.Immunol., 22, 2231-2236.
126 Watanabe, S., Barnikol, H.U., Horn, J., Bertram, J. & Hilschmann, N. (1973) Z.Physiol.Chem., 354, 1505-1509.
127 Weng, N.-P., Yu-Lee, L.-Y., Sanz, I., Patten, B.M. & Marcus, D.M. (1992) J.Immunol., 149, 2518-2529.
128 White, M.B., Word, C.J., Humphries, C.G., Blattner, F.R. & Tucker, P.W. (1990) Mol.Cell.Biol., 10, 3690-3699.
129 Winkler, T.H., Fehr, H. & Kalden, J.R (1992) Eur.J.Immunol., 22, 1719-1728.
130 Yago, K., Zenita, K., Ohwaki, I., Harada, Y., Nozawa, S., Tsukazaki, K., Iwamori, M., Endo, N., Yasuda, N., Okuma, M. & Kannagi, R. (1993) Mol.Immunol., 30, 1481-1489.
131 Zelenetz, A.D., Chen, T.T. & Levy, R. (1992) J.Exp.Med., 176, 1137-1148.

### B. References of germline sequences

### References of human germline kappa sequences

1 Cox, J.P.L., Tomlinson, I.M. & Winter, G. (1994) Eur.J.Immunol., 24, 827-836.
2 Huber, C., Et Al. (1993) Eur.J.Immunol., 23, 2868.
3 Klobeck, H.G., Bornkammm, G.W., Combriato, G., Mocikat, R., Pohlenz, H.D. & Zachau, H.G. (1985) Nucl.Acids Res., 13, 6515-6529.
4 Lautner-Rieske, A., Huber, C., Meindl, A., Pargent, W., Schäble, K.F., Thiebe, R., Zocher, I. & Zachau, H.G. (1992) Eur.J.Immunol. 22, 1023.
5 Lorenz, W., Schäble, K.F., Thiebe, R., Stavnezer, J. & Zachau, H.G. (1988) Mol.Immunol., 25, 479.
6 Pargent, W., Meindl, A., Thiebe, R., Mitzel, S. & Zachau, H.G. (1991) Eur.J.Immunol., 21, 1821-1827.
7 Pech, M. & Zachau, H.G. (1984) Nuc.Acids Res., 12, 9229-9236.
8 Pech, M., Jaenichen, H.-R., Pohlenz, H.-D., Neumaier, P.S., Klobeck, H.-G. & Zachau, H.G. (1984) J.Mol.Biol., 176, 189-204.
9 Scott, M.G., Crimmins, D.L., Mccourt, D.W., Chung, G., Schable, K.F., Thiebe, R., Quenzel, E.-M., Zachau, H.G. & Nahm, M.H. (1991) J.Immunol., 147, 4007-4013.
10 Stavnezer, J., Kekish, O., Batter, D., Grenier, J., Balazs, I., Henderson, E. & Zegers, B.J.M. (1985) Nucl.Acids Res., 13, 3495-3514.
11 Straubinger, B., Huber, E., Lorenz, W., Osterholzer, E., Pargent, W., Pech, M., Pohlenz, H.-D., Zimmer, F.-J. & Zachau, H.G. (1988) J.Mol.Biol., 199, 23-34.
12 Straubinger, B., Thiebe, R., Huber, C., Osterholzer, E. & Zachau, H.G. (1988) Biol.Chem.Hoppe-Seyer, 369, 601-607.

### References of human germline lambda sequences

1 Williams, S.C. & Winter, G. (1993) Eur.J.Immunol., 23, 1456-1461.
2 Siminovitch, K.A., Misener, V., Kwong, P.C., Song, Q.-L. & Chen, P.P. (1989) J.Clin.Invest., 84, 1675-1678.
3 Brockly, F., Alexandre, D., Chuchana, P., Huck, S., Lefranc, G. & Lefranc, M.-P. (1989) Nuc.Acids.Res., 17, 3976.
4 Daley, M.D., Peng, H.-Q., Misener, V., Liu, X.-Y., Chen, P.P. & Siminovitch, K.A. (1992) Mol.Immunol., 29, 1515-1518.
5 Deftos, M., Soto-Gil, R., Quan, M., Olee, T. & Chen, P.P. (1994) Scand. J. Immunol., 39, 95.
6 Stiernholm, N.B.J., Kuzniar, B. & Berinstein, N.L. (1994) J. Immunol., 152, 4969-4975.
7 Combriato, G. & Klobeck, H.G. (1991) Eur.J.Immunol., 21, 1513-1522.
8 Anderson, M.L.M., Szajnert, M.F., Kaplan, J.C., Mccoll, L. & Young, B.D. (1984) Nuc.Acids Res., 12, 6647-6661.

### Reference of human germiline heavy chain sequences

1 Adderson, E.E., Azmi, F.H., Wilson, P.M., Shackelford, P.G. & Carroll, W.L. (1993) J.Immunol., 151, 800-809.
2 Andris, J.S., Brodeur, B.R. & Capra, J.D. (1993) Mol.Immunol., 30, 1601-1616.
3 Berman, J.E., Mellis, S.J., Pollock, R., Smith, C.L., Suh, H., Heinke, B., Kowal, C., Surti, U., Chess, L., Cantor, C.R & Alt, F.W. (1988) Embo J., 7, 727-738.
4 Buluwela, L. & Rabbitts, T.H. (1988) Eur.J.Immunol., 18, 1843-1845.; Buluwela, L., Albertson, D.G., Sherrington, P., Rabbitts, P.H., Spurr, N. & Rabbitts, T.H. (1988) Embo J., 7, 2003-2010.
5 Chen, P.P., Liu, M.-F., Sinha, S. & Carson, D.A. (1988) Arth.Rheum., 31, 1429-1431.
6 Chen, P.P., Liu, M.-F., Glass, C.A., Sinha, S., Kipps. T.J. & Carson, D.A. (1989) Arthritis & Rheumatism, 32, 72-76.
7 Cook, G.P. et al. (1994) Nature Genetics 7, 162-168.
8 Haino, M. et al., (1994). J. Biol. Chem. 269, 2619-2626
9 Humphries, C.G., Shen, A., Kuziel, W.A., Capra, J.D., Blattner, F.R. & Tucker, P.W. (1988) Nature, 331, 446-449.
10 Kodaira, M., Kinashi, T., Umemura, I., Matsuda, F., Noma, T., Ono, Y. & Honjo, T. (1986) J.Mol.Biol., 190, 529-541.
11 Lee, K.H., Matsuda, F., Kinashi, T., Kodaira, M. & Honjo, T. (1987) J.Mol.Biol., 195, 761-768.
12 Matsuda, F., Lee, K.H., Nakai, S., Sato, T., Kodaira, M., Zong, S.Q., Ohno, H., Fukuhara, S. & Honjo, T. (1988) Embo J., 7, 1047-1051.
13 Matsuda, F., Shin, E.K., Hirabayashi, Y., Nagaoka, H., Yoshida, M.C., Zong, S.Q. & Honjo, T. (1990) Embo J., 9, 2501-2506.
14 Matsuda, F., Shin, E.K., Nagaoka, H., Matsumura, R., Haino, M., Fukita, Y., Taka-Ishi, S., Imai, T., Riley, J.H., Anand, R. Et, Al. (1993) Nature Genet. 3, 88-94
15 Nagaoka, H., Ozawa, K., Matsuda, F., Hayashida, H., Matsumura, R., Haino, M., Shin, E.K., Fukita, Y., Imai, T., Anand, R., Yokoyama, K., Eki, T., Soeda, E. & Honjo, T. (1993). (Temporal)
16 Rechavi, G., Bienz, B., Ram, D., Ben-Neriah, Y., Cohen, J.B., Zakut, R. & Givol, D. (1982) Proc.Nat.Acad.Sci.USA, 79, 4405-4409.
17 Sanz, I., Kelly, P., Williams, C., Scholl, S., Tucker, P. & Capra, J.D. (1989) Embo J., 8, 3741-3748.
18 Shin, E.K., Matsuda, F., Fujikura, J., Akamizu, T., Sugawa, H., Mori, T. & Honjo, T. (1993) Eur.J.Immunol., 23, 2365-2367.
19 Tomlinson, Im., Walter, G., Marks, Jd., Llewelyn, Mb. & Winter. G. (1992) J.Mol.Biol. 227, 776-798.
20 Van Der Maarel, S., Van Dijk, K.W., Alexander, C.M., Sasso, E.H., Bull, A. & Milner, E.C.B. (1993) J.Immunol., 150, 2858-2868.
21 Van Dijk, K.W., Mortari, F., Kirkham, P.M., Schroeder, Jr., H.W. & Milner, E.C.B. (1993) Eur.J.Immunol., 23, 832-839.
22 Van Es, J.H., Aanstoot, H., Gmelig-Meyling, F.H.J., Derksen, R.H.W.M. & Logtenberg, T. (1992) J.Immunol., 149, 2234-2240.
23 Weng. N.-P., Snyder, J.G., Yu-Lee, L.-Y. & Marcus, D.M. (1992) Eur.J.Immunol., 22, 1075-1082.
24 Winkler, T.H., Fehr, H. & Kalden, J.R. (1992) Eur.J.Immunol., 22, 1719-1728.
25 Olee, T., Yang, P.M., Sinunovitch, K.A., Olsen, N.J., Hillson, J.L., Wu, J., Kozin, F., Carson, D.A.&Chen, P.P. (1991) J. Clin. Invest. 88, 193-203.
26 Chen, P.P.& Yang, P.M. (1990) Scand. J. Immunol. 31, 593-599.
27 Tomlinson, M., Walter, G., Cook&Winter, G. (Unpublished)

## Claims

1. A method of identifying one or more genes encoding one or more proteins having an optimized property, comprising the steps of:
(a) expressing a collection of proteins from a collection of genes;
(b) screening said collection of proteins against at least one antigen to isolate proteins having a desired property;
(c) obtaining a plurality of genes, wherein each of said genes encodes a protein resulting from step (b);
(d) replacing one or more sub-sequence of each of said genes with a different, compatible genetic sub-sequence;
(e) screening the proteins encoded by the genes resulting from step (d) against said antigen(s), wherein the property of one or more proteins encoded by a gene resulting from step (d) is optimized compared to the property of the collection of proteins generated in step (b);
wherein one or more of said genes encode at least part of a member of the immunoglobulin superfamily, wherein the genetic sub-sequence encodes a complementarity determining region, and wherein the optimized property is selected from the group consisting of optimized affinity for a target molecule, optimized specificity for a target molecule, optimized expression yields, optimized stability and optimized solubility.

2. A method according to claim 1, wherein the replaced genetic sub-sequence is flanked by restriction sites that are unique within said gene.

3. A method according to claim 1 or 2, wherein the gene referred to in step (b) of claim 1 comprises multiple sub-sequences, wherein each sub-sequence is flanked by restriction sites that are unique within said gene.

4. A method according to claim 3 wherein the sub-sequences correspond to nucleic acids that encode three complementarity determining regions.

5. A method according to claim 1, wherein each sub-sequence encodes between 3 and 25 amino acids.

6. A method according to claim 1, wherein said compatible genetic sub-sequence comprises a nucleic acid encoding an antibody variable light chain.

7. A method according to claim 1, wherein said compatible genetic sub-sequence comprises a nucleic acid encoding a light chain CDR region.

8. A method according to claim 7, wherein said compatible genetic sub-sequence comprises a nucleic acid encoding a light chain CDR3 region.

9. A method according to claim 7, wherein said compatible genetic sub-sequence comprises a nucleic acid encoding a light chain CDR2 region.

10. A method according to claim 7, wherein said compatible genetic sub-sequence comprises a nucleic acid encoding a light chain CDR1 region.

11. A method according to claim 1, wherein said compatible genetic sub-sequence comprises a nucleic acid encoding an antibody variable heavy chain.

12. A method according to claim 1, wherein said compatible genetic sub-sequence comprises a nucleic acid encoding a heavy chain CDR region.

13. A method according to claim 12, wherein said compatible genetic sub-sequence comprises a nucleic acid encoding a heavy chain CDR3 region.

14. A method according to claim 12, wherein said compatible genetic sub-sequence comprises a nucleic acid encoding a heavy chain CDR2 region.

15. A method according to claim 12, wherein said compatible genetic sub-sequence comprises a nucleic acid encoding a heavy chain CDR1 region.

16. The method of claim 1, comprising screening said collection of proteins using a bacteriophage display systems.

## Patentansprüche

1. Verfahren zur Identifizierung eines oder mehrerer für ein oder mehrere Proteine mit einer optimierten Eigenschaft codierender Gene, das die Schritte:
(a) Exprimieren einer Sammlung von Proteinen von einer Sammlung von Genen;
(b) Screening der Sammlung von Proteinen gegen wenigstens ein Antigen, um Proteine mit einer gewünschten Eigenschaft zu isolieren;
(c) Erhalten mehrerer Gene, wobei die einzelnen Gene jeweils für ein aus Schritt (b) erhaltenes Protein codieren;
(d) Ersetzen jeweils einer oder mehrerer Teilsequenzen der einzelnen Gene durch eine unterschiedliche, kompatible genetische Teilsequenz;
(e) Screening der von den aus Schritt (d) erhaltenen Genen codierten Proteine gegen das bzw. die Antigen(e), wobei die Eigenschaft eines oder mehrerer von einem aus Schritt (d) erhaltenen Gen codierter Proteine verglichen mit der Eigenschaft der in Schritt (b) erzeugten Sammlung von Proteinen optimiert ist;
umfasst, wobei eines oder mehrere der Gene wenigstens einen Teil eines Mitglieds der Immunglobulin-Superfamilie codieren, wobei die genetische Teilsequenz eine Komplementarität bestimmende Region (CDR) codiert und wobei die optimierte Eigenschaft aus der aus optimierter Affinität für ein Zielmolekül, optimierter Spezifität für ein Zielmolekül, optimierten Expressionsausbeuten, optimierter Stabilität und optimierter Löslichkeit bestehenden Gruppe ausgewählt ist.

2. Verfahren nach Anspruch 1, wobei die ersetzte genetische Teilsequenz von Restriktionsstellen flankiert ist, die in dem Gen nur einmal vorkommen.

3. Verfahren nach Anspruch 1 oder 2, wobei das unter Schritt (b) von Anspruch 1 genannte Gen mehrere Teilsequenzen umfasst, wobei jede Teilsequenz von Restriktionsstellen flankiert ist, die in dem Gen nur einmal vorkommen.

4. Verfahren nach Anspruch 3, wobei die Teilsequenzen Nukleinsäuren entsprechen, die drei Komplementarität bestimmende Regionen (CDR) codieren.

5. Verfahren nach Anspruch 1, wobei jede Teilsequenz zwischen 3 und 25 Aminosäuren codiert.

6. Verfahren nach Anspruch 1, wobei die kompatible genetische Teilsequenz eine eine variable leichte Antikörperkette codierende Nukleinsäure umfasst.

7. Verfahren nach Anspruch 1, wobei die kompatible genetische Teilsequenz eine eine CDR-Region der leichten Kette codierende Nukleinsäure umfasst.

8. Verfahren nach Anspruch 7, wobei die kompatible genetische Teilsequenz eine eine CDR3-Region der leichten Kette codierende Nukleinsäure umfasst.

9. Verfahren nach Anspruch 7, wobei die kompatible genetische Teilsequenz eine eine CDR2-Region der leichten Kette codierende Nukleinsäure umfasst.

10. Verfahren nach Anspruch 7, wobei die kompatible genetische Teilsequenz eine eine CDR1-Region der leichten Kette codierende Nukleinsäure umfasst.

11. Verfahren nach Anspruch 1, wobei die kompatible genetische Teilsequenz eine eine variable schwere Antikörperkette codierende Nukleinsäure umfasst.

12. Verfahren nach Anspruch 1, wobei die kompatible genetische Teilsequenz eine eine CDR-Region der schweren Kette codierende Nukleinsäure umfasst.

13. Verfahren nach Anspruch 12, wobei die kompatible genetische Teilsequenz eine eine CDR3-Region der schweren Kette codierende Nukleinsäure umfasst.

14. Verfahren nach Anspruch 12, wobei die kompatible genetische Teilsequenz eine eine CDR2-Region der schweren Kette codierende Nukleinsäure umfasst.

15. Verfahren nach Anspruch 12, wobei die kompatible genetische Teilsequenz eine eine CDR1-Region der schweren Kette codierende Nukleinsäure umfasst.

16. Verfahren gemäß Anspruch 1, das ein Screening der Sammlung von Proteinen unter Verwendung eines Bakteriophagen-Display-Systems umfasst.

## Revendications

1. Procédé pour identifier un ou plusieurs gènes codant pour une ou plusieurs protéines ayant une propriété optimisée, comprenant les étapes de :
(a) expression d'une collection de protéines à partir d'une collection de gènes ;
(b) criblage de ladite collection de protéines contre au moins un antigène pour isoler les protéines ayant une propriété souhaitée ;
(c) obtention d'une pluralité de gènes, chacun desdits gènes codant pour une protéine résultant de l'étape (b) ;
(d) remplacement d'une ou plusieurs sous-séquences de chacun desdits gènes par une sous-séquence génétique compatible différente ;
(e) criblage des protéines codées par les gènes résultant de l'étape (d) contre ledit ou lesdits antigènes, la propriété d'une ou plusieurs protéines codées par un gène résultant de l'étape (d) étant optimisée par comparaison avec la propriété de la collection de protéines générée dans l'étape (b) ;
dans lequel un ou plusieurs desdits gènes codent pour au moins une partie d'un membre de la superfamille des immunoglobulines, la sous-séquence génétique codant pour une région déterminant la complémentarité (CDR), et la propriété optimisée étant choisie dans le groupe consistant en une affinité optimisée pour une molécule cible, une spécificité optimisée pour une molécule cible, des rendements d'expression optimisés, une stabilité optimisée et une solubilité optimisée.

2. Procédé selon la revendication 1, dans lequel la sous-séquence génétique remplacée est flanquée par des sites de restriction qui sont uniques à l'intérieur dudit gène.

3. Procédé selon la revendication 1 ou 2, dans lequel le gène auquel il est fait mention dans l'étape (b) de la revendication 1 comprend des sous-séquences multiples, chaque sous-séquence étant flanquée par des sites de restriction qui sont uniques à l'intérieur dudit gène.

4. Procédé selon la revendication 3, dans lequel les sous-séquences correspondent aux acides nucléiques qui codent pour trois régions déterminant la complémentarité (CDR).

5. Procédé selon la revendication 1, dans lequel chaque sous-séquence code pour 3 à 25 acides aminés.

6. Procédé selon la revendication 1, dans lequel ladite sous-séquence génétique compatible comprend un acide nucléique codant pour une chaîne légère variable d'un anticorps.

7. Procédé selon la revendication 1, dans lequel ladite sous-séquence génétique compatible comprend un acide nucléique codant pour une région CDR de la chaîne légère.

8. Procédé selon la revendication 7, dans lequel ladite sous-séquence génétique compatible comprend un acide nucléique codant pour une région CDR3 de la chaîne légère.

9. Procédé selon la revendication 7, dans lequel ladite sous-séquence génétique compatible comprend un acide nucléique codant pour une région CDR2 de la chaîne légère.

10. Procédé selon la revendication 7, dans lequel ladite sous-séquence génétique compatible comprend un acide nucléique codant pour une région CDR1 de la chaîne légère.

11. Procédé selon la revendication 1, dans lequel ladite sous-séquence génétique compatible comprend un acide nucléique codant pour une chaîne lourde variable d'un anticorps.

12. Procédé selon la revendication 1, dans lequel ladite sous-séquence génétique compatible comprend un acide nucléique codant pour une région CDR de la chaîne lourde.

13. Procédé selon la revendication 12, dans lequel ladite sous-séquence génétique compatible comprend un acide nucléique codant pour une région CDR3 de la chaîne lourde.

14. Procédé selon la revendication 12, dans lequel ladite sous-séquence génétique compatible comprend un acide nucléique codant pour une région CDR2 de la chaîne lourde.

15. Procédé selon la revendication 12, dans lequel ladite sous-séquence génétique compatible comprend un acide nucléique codant pour une région CDR1 de la chaîne lourde.

16. Procédé selon la revendication 1, comprenant le criblage de ladite collection de protéines par utilisation d'un système d'exposition sur bactériophage.
